# EUROPEAN PATENT APPLICATION

(11) **EP 4 393 922 A1**
(43) Date of publication of application: **03.07.2024**
(21) Application number: 22860505.1
(22) Date of filing: 23.08.2022
(51) Int. Cl.: C07D 471/10, C07D 403/12, C07D 401/14, A61K 31/513, A61P 35/00, A61P 35/02, A61P 25/08

(54) **IRAK4 DEGRADATION AGENT, AND PREPARATION METHOD THEREFOR AND USE THEREOF**

(30) Priority: 23.08.2021 CN 202110966608; 19.05.2022 CN 202210558158; 17.08.2022 CN 202210989448
(71) Applicant: Shanghai Leadingtac Pharmaceutical Co. Ltd., Shanghai 201203 (CN)
(72) Inventor: YE, Zhengqing, Shanghai 201203 (CN); FENG, Yan, Shanghai 201203 (CN); LI, Shiqiang, Shanghai 201203 (CN)
(74) Representative: Maiwald GmbH
(86) International application number: PCT/CN2022/114323
(87) International publication number: WO 2023/025159

(57) **Abstract**

An IRAK4 degradation agent as represented by structural formula PTM-L-ULM (I), and a preparation method therefor and the use thereof. The provided compound can effectively inhibit and/or degrade IRAK4 kinase protein in a cell and inhibit an immune cell from producing IL-6.

## Description

The present application claims priority to the Chinese Patent Application 202110966608X filed on Aug. 23, 2021, the Chinese Patent Application 2022105581585 filed on May 19, 2022, and the Chinese Patent Application 2022109894485 filed on Aug. 17, 2022, which are incorporated herein by reference in their entirety.

### TECHNICAL FIELD

The present invention relates to the field of pharmaceuticals and particularly to an IRAK4-targeting protein-degrading compound, a pharmaceutical composition, a preparation method therefor, and use thereof in the preparation of a medicament for treating and/or preventing IRAK4-mediated related diseases or disorders, such as cancer, immune diseases and inflammatory diseases.

### BACKGROUND

Interleukin-1 receptor kinase 4 (IRAK4) is a serine/threonine-specific protein kinase, which has biologically important kinase activity and plays an important role in activating the immune system. Studies have shown that IRAK4 is a key factor in the signaling pathways of the receptors of the interleukin (IL)-1β family (including IL-1R, IL-18R, IL-33R and IL-36R) and Toll-like receptors (TLRs), and that both IRAK4-deficient mice and IRAK4-deficient patients are non-responsive to stimulation by TLRs (except TLR3) and the IL-1β family.

According to whether MyD88 is involved or not, the TLR/IL-1β-mediated signaling pathways are classified into two categories: MyD88-dependent signaling pathways and MyD88-independent signaling pathways. The signaling pathways mediated by IL-1R and TLR2, TLR4, TLR7/8 and TLR9 all rely on MyD88 as a regulatory factor to activate downstream inflammatory signaling pathways. Upon binding to the ligand, TLR/IL-1β recruits MyD88 molecules. Further, MyD88 recruits IRAK4 into the TLR/IL-1β complex through its N-terminal death domain, and interacts with IRAK1 or IRAK2 and activates them, thereby signaling downstream to the E3 ubiquitin ligase TNF receptor associated factor (TRAF6), activating the serine/threonine kinase TAK1 and further the NF-κB and MAPK signaling pathways, resulting in the release of various inflammatory cytokines and anti-apoptotic molecules. The IRAK4-dependent TLR/IL-1β signaling pathway has been shown to be associated with a variety of diseases such as multiple sclerosis, atherosclerosis, myocardial infarction, myocarditis, Vogt-Koyanagi-Harada syndrome, systemic lupus erythematosus (SLE), obesity, type 1 diabetes, rheumatoid arthritis, spondyloarthritis (especially psoriatic spondyloarthritis and Bekhterev's disease), lupus erythematosus, psoriasis, vitiligo, giant cell arteritis, chronic inflammatory intestinal diseases and viral diseases, for example, HIV (human immunodeficiency virus), hepatitis viruses; skin diseases such as psoriasis, atopic dermatitis, Kindler's syndrome, bullous pemphigoid, allergic contact dermatitis, alopecia areata, acneinversa and acne vulgaris; other inflammatory diseases such as allergy, Bechet's disease, gout, adult-onset Still's disease, pericarditis and chronic inflammatory intestinal diseases such as ulcerative colitis and Crohn's disease, transplant rejection and graft-versus-host reactions; gynecological diseases such as adenomyosis, dysmenorrhea, dyspareunia and endometriosis, especially pain related to adenomyosis and other symptoms related to adenomyosis such as dysmenorrhea, dyspareunia, dysuria and difficulty defecating; eye diseases such as retinal ischemia, keratitis, allergic conjunctivitis, keratoconjunctivitis sicca, macular degeneration and ocular pigment layer inflammation; fibrotic diseases such as liver fibrosis, myocarditis, primary biliary cirrhosis, cystic fibrosis; chronic liver diseases such as fatty liver hepatitis, especially non-alcoholic fatty liver disease (NAFLD) and/or non-alcoholic steatohepatitis (NASH), alcoholic steatohepatitis (ASH); cardiovascular diseases and neurological disorders such as myocardial reperfusion injury, myocardial infarction, hypertension, and Alzheimer's disease, stroke, craniocerebral trauma, amyotrophic lateral sclerosis (ALS) and Parkinson's disease; pruritus and pain (including acute, chronic, inflammatory and neuropathic pain) such as hyperalgesia, allodynia, premenstrual pain, pain related to adenomyosis, postoperative pain, interstitial cystitis, CRPS (complex local pain syndrome), trigeminal neuralgia, prostatitis, pain caused by spinal cord injury, pain caused by inflammation, lower back pain, cancer pain, chemotherapy-related pain, HIV treatment-induced neuropathy, pain caused by burns and chronic pain; tumor diseases such as certain lymphomas: ABC-DLBCL (activated B-cell diffuse large cell B-cell lymphoma), mantle cell lymphoma and Waldenstrom disease, as well as chronic lymphocytic leukemia, melanoma, pancreatic tumors and hepatocellular carcinoma, ras-dependent tumors, breast cancer, ovarian cancer, colorectal cancer, head and neck cancer, lung cancer, and prostate cancer.

Modulation of IRAK4-mediated signaling pathways is primarily associated with its kinase function. However, there are also reports that signal modulation by IRAK4 of downstream processes is associated with the non-kinase function of IRAK4 in certain cell types. Cushing et al. have indicated that despite reduced levels of IRAK4 phosphorylation in IL-1β-stimulated human skin fibroblasts, pharmacological inhibition of IRAK4 does not result in inhibition of IL-6 and TNF-α. Supporting these results, the scaffolding function of IRAK4 is important for IL1 signaling, but its kinase activity is redundant in IRAK4-deficient fibroblasts compared to wild-type cells. Chiang et al. have also indicated that the kinase activity of IRAK4 is not essential in human B cells and T cells, dendritic cells and monocytes, and siRNA gene excision also shows that IRAK4 can function as a scaffold in these cells. A variety of potent selective inhibitors against IRAK4 have been reported, such as CA-4948, BAY-1834845, BMS-986126 and PF-06650833. These inhibitors can selectively inhibit the kinase activity of IRAK4 and are mainly used for the prevention and treatment of autoimmune diseases, inflammatory diseases and tumor diseases. However, since IRAK4 can function as a scaffold protein and an active kinase, and traditional small-molecule inhibitors easily lead to drug resistance, inhibiting only the kinase activity of IRAK4 may not be sufficient to produce a therapeutic effect.

Proteolysis targeting chimera (PROTAC) is a technology different from the traditional small-molecule inhibitors. The traditional small-molecule inhibitors usually need to act on the active site of a target protein to inhibit the activity of the target protein, while the PROTAC is a heterogeneous bifunctional molecule, one end of which is a small-molecule inhibitor capable of recognizing the target protein, and the other end of which is an E3 ubiquitin ligase ligand capable of recognizing E3 ubiquitin ligase via a linking chain. Such a bifunctional molecule recognizes the target protein and the E3 ubiquitin ligase *in vivo,* draws the target protein and the E3 ubiquitin ligase closer to form a ternary complex, and degrades the target protein *in vivo* through a ubiquitin-proteasome pathway after ubiquitinating the target protein. Compared with the traditional small-molecule inhibitors, in one aspect, the PROTAC only needs to draw the target protein and the E3 ubiquitin ligase closer to degrade the substrate, and the action mode enables the technology to be applied to some non-druggable targets; in another aspect, the PROTAC molecules can be released to continue to participate in the degradation process of the next protein after the target protein is degraded, so that the degradation effect with a catalytic effect can realize efficient degradation with less dosage of the PROTAC medicament; in yet another aspect, the traditional small-molecule inhibitors easily generate drug resistance often because a point mutation occurs, so that the small-molecule inhibitors lose the inhibition effect on the target point, while the PROTAC can directly degrade the target protein, so that the drug resistance generated by the point mutation can be avoided to a certain extent. Therefore, compared with the traditional small-molecule inhibitors, the PROTAC technology has high advantages and feasibility in the research and development of small-molecule new drugs, and these drugs are expected to become next-generation promising new drugs. PROTAC technology has also been applied to the modification of various target drugs, such as the androgen receptor, the estrogen protein receptor, and BTK. Several types of IRAK4-targeting degraders are disclosed in US2019/0151295, US2019/0192688, WO2019/160915 and WO2020/113233. There is an urgent need to develop more IRAK4-targeting degraders.

### SUMMARY

The present invention provides a compound of formula I, a stereoisomer, enantiomer, diastereomer, deuteride, hydrate, solvate or prodrug thereof or a pharmaceutically acceptable salt thereof;

PTM-L-ULM I

wherein:
the PTM has a structure of the following formula: 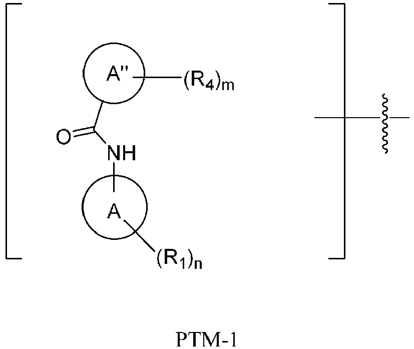
wherein in the PTM-1:
m is 0, 1, 2, 3 or 4;
n is 0, 1, 2, 3 or 4;
ring A" is 6-10 membered aryl or is 5-11 membered monocyclic or bicyclic heteroaryl containing 1, 2 or 3 heteroatoms selected from N, O or S;
ring A is 6-10 membered aryl or is 5-11 membered monocyclic or bicyclic heteroaryl containing 1, 2 or 3 heteroatoms selected from N, O or S;
each R₁ is independently selected from: C₁-C₄ alkyl, -O(C₁-C₄ alkyl), -N(C₁-C₄ alkyl)₁₋₂, C₃-C₈ cycloalkyl, -O(C₃-C₈ cycloalkyl), -NH(C₃-C₈ cycloalkyl), 3-8 membered heterocycloalkyl, -O(3-8 membered heterocycloalkyl), -NH(3-8 membered heterocycloalkyl), 6-10 membered aryl, -0(6-10 membered aryl), - NH(6-10 membered aryl), 5-6 membered heteroaryl, -O(5-6 membered heteroaryl), -NH(5-6 membered heteroaryl), CN, halogen, -OH, -NH₂, -NHC(O)(C₁-C₄ alkyl), -NHS(O)(C₁-C₄ alkyl), -NHS(O)₂(C₁-C₄ alkyl), -COOH, -C(O)NH₂, -S(O)₂H, -S(O)₂NH₂, -C(O)(C₁-C₄ alkyl), -C(O)NH(C₁-C₄ alkyl), -C(O)N(C₁-C₄ alkyl)₂, -C(O)O(C₁-C₄ alkyl), -S(O)(C₁-C₄ alkyl), -S(O)₂(C₁-C₄ alkyl), -S(O)₂NH(C₁-C₄ alkyl), -S(O)₂N(C₁-C₄ alkyl)₂ or -S(O)NH(C₁-C₄ alkyl), wherein the alkyl, cycloalkyl, heterocycloalkyl, aryl and heteroaryl are optionally substituted with groups selected from halogen, cyano, -OH, C₁-C₄ alkyl, -O(C₁-C₄ allcyl), methylpiperidine, - S(O)₂H, -S(O)₂(C₁-C₄ alkyl), -C(O)N(C₁-C₄ alkyl)₁₋₂ and C(O)O(C₁-C₄ alkyl);
or the R₁, together with the atom to which it is attached, forms cycloalkyl, heterocycloalkyl, aryl or heteroaryl;
each R₄ is independently hydrogen, C₁-C₄ alkyl, -O(C₁-C₄ alkyl), C₃-C₈ cycloalkyl, 3-8 membered heterocycloalkyl, 6-10 membered aryl, 5-6 membered heteroaryl, CN, halogen, -OH, -NHC(O)(C₁-C₄ alkyl), -NHS(O)(C₁-C₄ alkyl), -NHS(O)₂(C₁-C₄ alkyl), -COOH, -C(O)NH₂, -S(O)₂H, -S(O)₂NH₂, -C(O)(C₁-C₄ alkyl), -C(O)NH(C₁-C₄ alkyl), -C(O)N(C₁-C₄ alkyl)₂, -C(O)O(C₁-C₄ alkyl), -S(O)(C₁-C₄ alkyl), -S(O)₂(C₁-C₄ alkyl), -S(O)₂NH(C₁-C₄ alkyl), -S(O)₂N(C₁-C₄ alkyl)₂, -S(O)NH(C₁-C₄ alkyl) or X(R₂)(R₃), wherein the alkyl, cycloalkyl, heterocycloalkyl, aryl and heteroaryl are optionally substituted with groups selected from halogen, cyano, -OH, C₁-C₄ alkyl, -O(C₁-C₄ alkyl), methylpiperidine, -S(O)₂H, -S(O)₂(C₁-C₄ alkyl), -C(O)N(C₁-C₄ alkyl)₁₋₂, and -C(O)O(C₁-C₄ alkyl);
X is O, S, CH₂ or N;
R₂ and R₃ are independently selected from: hydrogen, C₁-C₆, alkyl, C₃-C₈ cycloalkyl, 3-8 membered heterocycloalkyl, 6-10 membered aryl or 5-6 membered heteroaryl, wherein the alkyl, cycloalkyl, heterocycloalkyl, aryl and heteroaryl are each independently optionally substituted with one or more substituents selected from halogen, OH, oxo, N(R_{b})₂, oxopyrrolidinyl and morpholinyl, or R₂ and R₃, together with the atom to which they are attached, form C₃-C₈ cycloalkyl or 3-8 membered heterocycloalkyl, wherein the cycloalkyl and heterocycloalkyl are optionally substituted with one or more substituents selected from Rₐ;
Rₐ is independently selected from oxo, C₁-C₄ alkyl, C₃-C₆ cycloalkyl, CF₃, CHF₂, OH, halogen or NH₂, wherein the alkyl is optionally substituted with C₃-C₆ cycloalkyl or CF₃; and
R_{b} is independently selected from H or C₁-C₄ alkyl;
L is a linking chain, which links PTM and ULM by covalent bonds;
the ULM has a structure of the following formula: 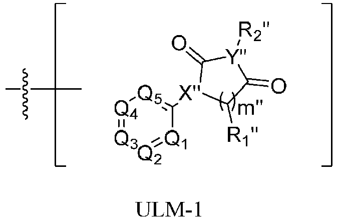
wherein in the ULM-1:
X" is C or N;
Y" is C, N, O or S;
Q₁, Q₂, Q₃, Q₄ and Q₅ are each independently CR₃" or N;
each R₃" is independently hydrogen, deuterium, hydroxy, amino, cyano, halogen, C₁-C₆ alkyl, C₃-C₈ cycloalkyl, 3-8 membered heterocycloalkyl, 6-10 membered aryl, 5-10 membered heteroaryl, -O(C₁-C₆ alkyl), -O-(C₃-C₈ cycloalkyl), -O-(3-8 membered heterocycloalkyl), N(C₁-C₆ alkyl)₁₋₂, NH(C₃-C₈ cycloalkyl), NH(3-8 membered heterocycloalkyl), -O-(6-10 aryl) or -0-(5-10 membered heteroaryl), wherein the alkyl, cycloalkyl, heterocycloalkyl, aryl and heteroaryl are optionally substituted with 1-3 groups independently selected from hydroxy, halogen, cyano and amino; or R₃", together with the atom to which it is attached, forms cycloalkyl, heterocycloalkyl, heteroaryl or aryl;
m" is 1, 2 or 3;
each R₁" is independently hydrogen, deuterium, hydroxy, amino, cyano, halogen, C₁-C₆ alkyl, C₃-C₈ cycloalkyl, 3-8 membered heterocycloalkyl, 6-10 membered aryl, 5-10 membered heteroaryl or -O(C₁-C₆ alkyl), wherein the alkyl, cycloalkyl, heterocycloalkyl, aryl and heteroaryl are optionally substituted with 1-3 groups independently selected from hydroxy, halogen, cyano and amino;
R₂" is hydrogen, deuterium, C₁-C₆ alkyl or C₃-C₆ cycloalkyl, wherein the C₁-C₆ alkyl and C₃-C₆ cycloalkyl are optionally substituted with 1-3 groups independently selected from hydroxy, halogen, cyano, amino and -OC(O)(C₁-C₆ alkyl).

Preferably, in certain embodiments of the present invention, each R₃" is independently hydrogen, deuterium, hydroxy, amino, cyano, halogen, C₁-C₆ alkyl, C₃-C₈ cycloalkyl, 3-8 membered heterocycloalkyl, 6-10 membered aryl, 5-10 membered heteroaryl, -O(C₁-C₆ alkyl), -O-(C₃-C₈ cycloalkyl), -O-(3-8 membered heterocycloalkyl), N(C₁-C₆ alkyl)₁₋₂, NH(C₃-C₈ cycloalkyl), NH(3-8 membered heterocycloalkyl), -0-(6-10 aryl) or -0-(5-10 membered heteroaryl), wherein the alkyl, cycloalkyl, heterocycloalkyl, aryl and heteroaryl are optionally substituted with 1-3 groups independently selected from hydroxy, halogen, cyano and amino; or R₃", together with the atom to which it is attached, forms cycloalkyl or heterocycloalkyl.

Preferably, in certain embodiments of the present invention, R₃", together with the atom to which it is attached, forms heteroaryl, which is not

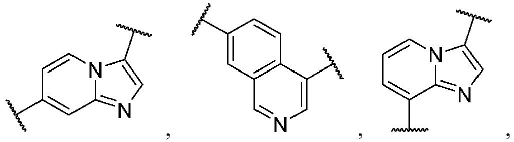

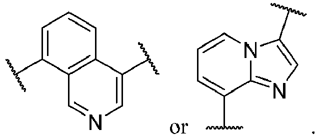

Preferably, in certain embodiments of the present invention,
in the PTM-1:
m is 0 or 1;
n is 0, 1 or 2;
ring A" is 6-10 membered aryl or is 5-11 membered monocyclic or bicyclic heteroaryl containing 1, 2 or 3 heteroatoms selected from N, O or S;
ring A is 6-10 membered aryl or 5-6 membered heteroaryl;
R₁ is independently selected from: C₁-C₄ alkyl, -O(C₁-C₄ alkyl), -N(C₁-C₄ alkyl)₁₋₂, C₃-C₈ cycloalkyl, -O(C₃-C₈ cycloalkyl), -NH(C₃-C₈ cycloalkyl), 3-8 membered heterocycloalkyl, -O(3-8 membered heterocycloalkyl), -NH(3-8 membered heterocycloalkyl), 6-10 membered aryl, -0(6-10 membered aryl), - NH(6-10 membered aryl), 5-6 membered heteroaryl, -O(5-6 membered heteroaryl), -NH(5-6 membered heteroaryl), CN, halogen, -OH, -NH₂, -NHC(O)(C₁-C₄ alkyl), -NHS(O)(Ci-Ca alkyl), -NHS(O)₂(C₁-C₄ alkyl), -COOH, -C(O)NH₂, -S(O)₂H, -S(O)₂NH₂, -C(O)(C₁-C₄ alkyl), -C(O)NH(C₁-C₄ alkyl), -C(O)N(C₁-C₄ alkyl)₂, -C(O)O(C₁-C₄ alkyl), -S(O)(C₁-C₄ alkyl), -S(O)₂(C₁-C₄ alkyl), -S(O)₂NH(C₁-C₄ alkyl), -S(O)₂N(C₁-C₄ alkyl)₂ or -S(O)NH(C₁-C₄ alkyl), wherein the alkyl, cycloalkyl, heterocycloalkyl, aryl and heteroaryl are optionally substituted with groups selected from halogen, cyano, -OH, C₁-C₄ alkyl, -O(C₁-C₄ alkyl), methylpiperidine, - S(O)₂H, -S(O)₂(C₁-C₄ alkyl), -C(O)N(C₁-C₄ alkyl)₁₋₂, or -C(O)O(C₁-C₄ alkyl);
or the R₁, together with the atom to which it is attached, forms cycloalkyl, heterocycloalkyl, aryl or heteroaryl;
R₄ is hydrogen, C₁-C₄ alkyl, -O(C₁-C₄ alkyl), C₃-C₈ cycloalkyl, 3-8 membered heterocycloalkyl, 6-10 membered aryl, 5-6 membered heteroaryl, CN, halogen, -OH, -NHC(O)(C₁-C₄ alkyl), -NHS(O)(C₁-C₄ alkyl), -NHS(O)₂(C₁-C₄ alkyl), -COOH, -C(O)NH₂, -S(O)₂H, -S(O)₂NH₂, -C(O)(C₁-C₄ alkyl), -C(O)NH(C₁-C₄ alkyl), -C(O)N(C₁-C₄ alkyl)₂, -C(O)O(C₁-C₄ alkyl), -S(O)(C₁-C₄ alkyl), -S(O)₂(C₁-C₄ alkyl), -S(O)₂NH(C₁-C₄ alkyl), -S(O)₂N(C₁-C₄ alkyl)₂, -S(O)NH(C₁-C₄ alkyl) or -X(R₂)(R₃), wherein the alkyl, cycloalkyl, heterocycloalkyl, aryl and heteroaryl are optionally substituted with groups selected from halogen, cyano, - OH, C₁-C₄ alkyl, -O(C₁-C₄ alkyl), methylpiperidine, -S(O)₂H, -S(O)₂(C₁-C₄ alkyl), -C(O)N(C₁-C₄ alkyl)₁₋₂, or -C(O)O(C₁-C₄ alkyl); or
X is O, S, CH₂ or N;
R₂ and R₃ are independently selected from: hydrogen, C₁-C₆ alkyl, C₃-C₈ cycloalkyl, 3-8 membered heterocycloalkyl, 6-10 aryl or 5-6 membered heteroaryl, wherein the alkyl, cycloalkyl, heterocycloalkyl, aryl and heteroaryl are each independently optionally substituted with one or more substituents selected from halogen, OH, oxo, N(R_{b})₂, oxopyrrolidinyl or morpholinyl, or R₂ and R₃, together with the atom to which they are attached, form C₃-C₈ cycloalkyl or 3-8 membered heterocycloalkyl, wherein the cycloalkyl or heterocycloalkyl is optionally substituted with one or more substituents selected from Rₐ;
Rₐ is independently selected from oxo, C₁-C₄ alkyl, C₃-C₆ cycloalkyl, CF₃, CHF₂, OH, halogen or NH₂, wherein the alkyl is optionally substituted with C₃-C₆ cycloalkyl or CF₃; and
R_{b} is independently selected from H or C₁-C₄ alkyl;
L is a linking chain, which links PTM and ULM by covalent bonds;
the ULM has a structure of the following formula: 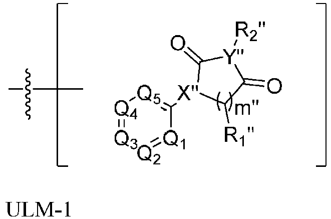
wherein in the ULM-1:
X" is C or N;
Y" is C, N, O or S;
Q₁, Q₂, Q₃, Q₄ and Q₅ are each independently CR₃" or N;
each R₃" is independently hydrogen, deuterium, hydroxy, amino, cyano, halogen, C₁-C₆ alkyl, C₃-C₈ cycloalkyl, 3-8 membered heterocycloalkyl, 6-10 membered aryl, 5-10 membered heteroaryl, -O(C₁-C₆ alkyl), -O-(C₃-C₈ cycloalkyl), -O-(3-8 membered heterocycloalkyl), N(C₁-C₆ alkyl)₁₋₂, NH(C₃-C₈ cycloalkyl), NH(3-8 membered heterocycloalkyl), -0-(6-10 aryl) or -0-(5-10 membered heteroaryl), wherein the alkyl, cycloalkyl, heterocycloalkyl, aryl and heteroaryl are optionally substituted with 1-3 groups independently selected from hydroxy, halogen, cyano and amino; or R₃", together with the atom to which it is attached, forms cycloalkyl, heterocycloalkyl, heteroaryl or aryl;
m" is 1, 2 or 3;
each R₁" is independently hydrogen, deuterium, hydroxy, amino, cyano, halogen, C₁-C₆ alkyl, C₃-C₈ cycloalkyl, 3-8 membered heterocycloalkyl, 6-10 membered aryl, 5-10 membered heteroaryl or -O(C1-C6 alkyl), wherein the alkyl, cycloalkyl, heterocycloalkyl, aryl and heteroaryl are optionally substituted with 1-3 groups independently selected from hydroxy, halogen, cyano and amino;
R₂" is hydrogen, deuterium, C₁-C₆ alkyl or C₃-C₆ cycloalkyl, wherein the C₁-C₆ alkyl and C₃-C₆ cycloalkyl are optionally substituted with 1-3 groups independently selected from hydroxy, halogen, cyano, amino or -OC(O)(C₁-C₆ alkyl).
Preferably, in certain embodiments of the present invention,
in the PTM-1:
m is 0, 1, 2, 3 or 4;
n is 0, 1, 2, 3 or 4;
ring A" is 6-10 membered aryl or is 5-11 membered monocyclic or bicyclic heteroaryl containing 1, 2 or 3 heteroatoms selected from N, O or S;
ring A is 6-10 membered aryl or is 5-11 membered monocyclic or bicyclic heteroaryl containing 1, 2 or 3 heteroatoms selected from N, O or S;
R₁ is independently selected from: C₁-C₄ alkyl, -O(C₁-C₄ alkyl), -N(C₁-C₄ alkyl)₁₋₂, C₃-C₈ cycloalkyl, -O(C₃-C₈ cycloalkyl), -NH(C₃-C₈ cycloalkyl), 3-8 membered heterocycloalkyl, -O(3-8 membered heterocycloalkyl), -NH(3-8 membered heterocycloalkyl), 6-10 membered aryl, -0(6-10 membered aryl), - NH(6-10 membered aryl), 5-6 membered heteroaryl, -O(5-6 membered heteroaryl), -NH(5-6 membered heteroaryl), CN, halogen, -OH, -NH₂, -NHC(O)(C₁-C₄ alkyl), -NHS(O)(C₁-C₄ alkyl), -NHS(O)₂(C₁-C₄ alkyl), -COOH, -C(O)NH₂, -S(O)₂H, -S(O)₂NH₂, -C(O)(C₁-C₄ alkyl), -C(O)NH(C₁-C₄ alkyl), -C(O)N(C₁-C₄ alkyl)₂, -C(O)O(C₁-C₄ alkyl), -S(O)(C₁-C₄ alkyl), -S(O)₂(C₁-C₄ alkyl), -S(O)₂NH(C₁-C₄ alkyl), -S(O)₂N(C₁-C₄ alkyl)₂ or -S(O)NH(C₁-C₄ alkyl), wherein the alkyl, cycloalkyl, heterocycloalkyl, aryl and heteroaryl are optionally substituted with groups selected from halogen, cyano, -OH, C₁-C₄ alkyl, -O(C₁-C₄ alkyl), methylpiperidine, - S(O)₂H, -S(O)₂(C₁-C₄ alkyl), -C(O)N(C₁-C₄ alkyl)₁₋₂, or -C(O)O(C₁-C₄ alkyl);
or the R₁, together with the atom to which it is attached, forms cycloalkyl, heterocycloalkyl, aryl or heteroaryl;
R₄ is hydrogen, C₁-C₄ alkyl, -O(C₁-C₄ alkyl), C₃-C₈ cycloalkyl, 3-8 membered heterocycloalkyl, 6-10 membered aryl, 5-6 membered heteroaryl, CN, halogen, -OH, -NHC(O)(C₁-C₄ alkyl), -NHS(O)(C₁-C₄ alkyl), -NHS(O)₂(C₁-C₄ alkyl), -COOH, -C(O)NH₂, -S(O)₂H, -S(O)₂NH₂, -C(O)(C₁-C₄ alkyl), -C(O)NH(C₁-C₄ alkyl), -C(O)N(C₁-C₄ alkyl)₂, -C(O)O(C₁-C₄ alkyl), -S(O)(C₁-C₄ alkyl), -S(O)₂(C₁-C₄ alkyl), -S(O)₂NH(C₁-C₄ alkyl), -S(O)₂N(C₁-C₄ alkyl)₂, -S(O)NH(C₁-C₄ alkyl) or -X(R₂)(R₂), wherein the alkyl, cycloalkyl, heterocycloalkyl, aryl and heteroaryl are optionally substituted with groups selected from halogen, cyano, - OH, C₁-C₄ alkyl, -O(C₁-C₄ alkyl), methylpiperidine, -S(O)₂H, -S(O)₂(C₁-C₄ alkyl), -C(O)N(C₁-C₄ alkyl)₁₋₂, or -C(O)O(C₁-C₄ alkyl);
X is O, S, CH₂ or N;
R₂ and R₃ are independently selected from: hydrogen, C₁-C₆ alkyl, C₃-C₈ cycloalkyl, 3-8 membered heterocycloalkyl, 6-10 membered aryl or 5-6 membered heteroaryl, wherein the alkyl, cycloalkyl, heterocycloalkyl, aryl and heteroaryl are each independently optionally substituted with one or more substituents selected from halogen, OH, oxo, N(R_{b})₂, oxopyrrolidinyl or morpholinyl, or R₂ and R₃, together with the atom to which they are attached, form C₃-C₈ cycloalkyl or 3-8 membered heterocycloalkyl, wherein the cycloalkyl or heterocycloalkyl is optionally substituted with one or more substituents selected from Rₐ;
Rₐ is independently selected from oxo, C₁-C₄ alkyl, C₃-C₆ cycloalkyl, CF₃, CHF₂, OH, halogen or NH₂, wherein the alkyl is optionally substituted with C₃-C₆ cycloalkyl or CF₃; and
R_{b} is independently selected from H or C₁-C₄ alkyl;
L is a linking chain, which links PTM and ULM by covalent bonds;
the ULM has a structure of the following formula: 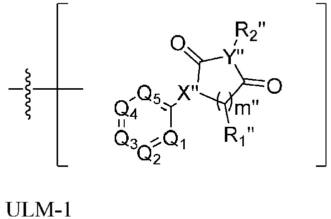
wherein in the ULM-1:
X" is C or N;
Y" is C, N, O or S;
Q₁, Q₂, Q₃, Q₄ and Q₅ are each independently CR₃" or N;
each R₃" is independently hydrogen, deuterium, hydroxy, amino, cyano, halogen, C₁-C₆ alkyl, C₃-C₈ cycloalkyl, 3-8 membered heterocycloalkyl, 6-10 membered aryl, 5-10 membered heteroaryl, -O(C₁-C₆ alkyl), -O-(C₃-C₈ cycloalkyl), -O-(3-8 membered heterocycloalkyl), N(C₁-C₆ alkyl)₁₋₂, NH(C₃-C₈ cycloalkyl), NH(3-8 membered heterocycloalkyl), -O-(6-10 aryl) or -O-(5-10 membered heteroaryl), wherein the alkyl, cycloalkyl, heterocycloalkyl, aryl and heteroaryl are optionally substituted with 1-3 groups independently selected from hydroxy, halogen, cyano and amino; or R₃", together with the atom to which it is attached, forms cycloalkyl, heterocycloalkyl, heteroaryl or aryl;
m" is 1, 2 or 3;
each R₁" is independently hydrogen, deuterium, hydroxy, amino, cyano, halogen, C₁-C₆ alkyl, C₃-C₈ cycloalkyl, 3-8 membered heterocycloalkyl, 6-10 membered aryl, 5-10 membered heteroaryl or -O(C₁-C₆ alkyl), wherein the alkyl, cycloalkyl, heterocycloalkyl, aryl and heteroaryl are optionally substituted with 1-3 groups independently selected from hydroxy, halogen, cyano and amino;
R₂" is hydrogen, deuterium, C₁-C₆ alkyl or C₃-C₆ cycloalkyl, wherein the C₁-C₆ alkyl and C₃-C₆ cycloalkyl are optionally substituted with 1-3 groups independently selected from hydroxy, halogen, cyano, amino or -OC(O)(C₁-C₆ alkyl).

Preferably, in certain embodiments of the present invention, in the PTM-1, ring A" is 9-10 membered bicyclic heteroaryl containing 1, 2 or 3 heteroatoms selected from N, O or S; preferably, ring A" is 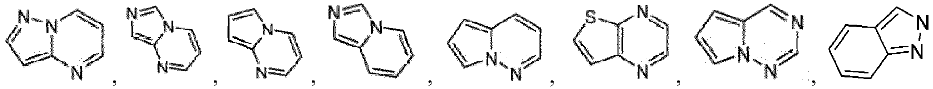 or

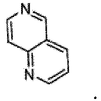

Preferably, in certain embodiments of the present invention, the PTM is 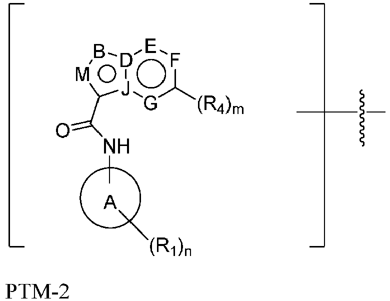
wherein in the PTM-2:
B is CH, N, O or S;
M is CH, N, O or S;
D is CH or N;
E is CH or N;
F is CH or N;
G is CH or N;
J is CH or N;
when B or M is S or O, D is CH, E is N, F is CH, G is N, and J is CH; and B and M are not simultaneously S and/or O;
the ring A, R₁, n, R₄ and m are as defined and described in PTM-1.

Preferably, in certain embodiments of the present invention, the 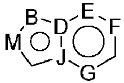 in the PTM-2 is

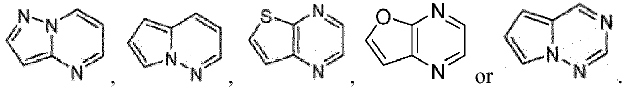

Preferably, in certain embodiments of the present invention, the PTM is 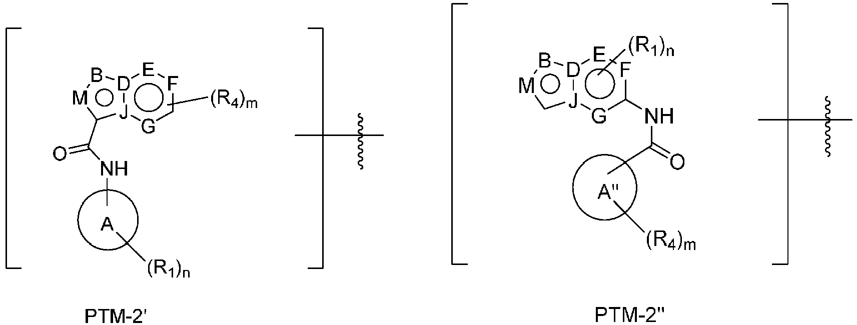
wherein in the PTM-2' or PTM-2":
B is CH, N, O or S;
M is CH, N, O or S;
D is CH or N;
E is CH or N;
F is CH or N;
G is CH or N;
J is CH or N;
when B or M is S or O, D is CH, E is N, F is CH, G is N, and J is CH; and B and M are not simultaneously S and/or O;
the ring A, ring A", R₁, n, R₄ and m are as defined and described in PTM-1.

Preferably, in certain embodiments of the present invention, the 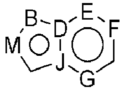 in the PTM-2' is

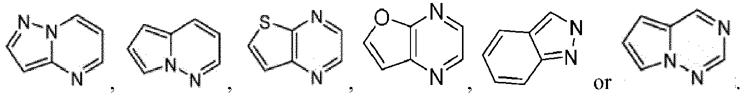

Preferably, in certain embodiments of the present invention, the PTM is: 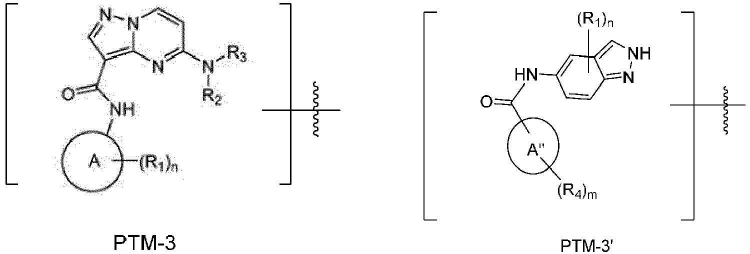 wherein the ring A, ring A", R₁, R₂, R₃, R₄, m and n are as defined and described in PTM-1.

Preferably, in certain embodiments of the present invention, in the PTM-1, PTM-2, PTM-2' and PTM-3:
ring A is phenyl or is 5-6 membered heteroaryl containing 1, 2 or 3 heteroatoms selected from N or O; preferably, ring A is phenyl, pyridyl, pyrrolyl, pyrazolyl, imidazolyl, pyrimidinyl, triazolyl, tetrazolyl, thienyl, thiazolyl, furanyl, oxazolyl, isoxazolyl or isothiazolyl; further preferably, ring A is pyrazolyl or thienyl.

Preferably, in certain embodiments of the present invention, in the PTM-1, PTM-2, PTM-2' and PTM-3, n is 1 or 2; preferably n is 2.

Preferably, in certain embodiments of the present invention, in the PTM-1, PTM-2, PTM-2' and PTM-3, R₁ is independently selected from: C₁-C₄ alkyl, C₃-C₆ cycloalkyl, 3-6 membered heterocycloalkyl, CN or halogen, wherein the alkyl, cycloalkyl and heterocycloalkyl are optionally substituted with halogen, OH, CH₃ or OCH₃; preferably, R₁ is independently selected from: C₁-C₄ alkyl, C₃-C₆ cycloalkyl, 3-6 membered heterocycloalkyl containing 1 or 2 heteroatoms selected from N, CN or halogen, wherein the alkyl, cycloalkyl and heterocycloalkyl are optionally substituted with halogen, OH, CH₃ or OCH₃; further preferably, the R₁ is CHF₂, CF₃, cyclohexyl, piperidinyl, methyl, cyano or piperazinyl.

Preferably, in certain embodiments of the present invention, in the PTM-1, PTM-2 and PTM-3, R₁ is independently selected from: C₁-C₄ alkyl, C₃-C₆ cycloalkyl, 3-6 membered heterocycloalkyl, CN or halogen, wherein the alkyl, cycloalkyl and heterocycloalkyl are optionally substituted with halogen, OH, CH₃ or OCH₃; preferably, R₁ is independently selected from: C₁-C₄ alkyl, C₃-C₆ cycloalkyl, 3-6 membered heterocycloalkyl containing 1 or 2 heteroatoms selected from N, CN or halogen, wherein the alkyl, cycloalkyl and heterocycloalkyl are optionally substituted with halogen, OH, CH₃ or OCH₃; further preferably, the R₁ is CHF₂, CF₃, cyclohexyl, piperidinyl or piperazinyl.

In certain embodiments of the present invention, R₁ is independently selected from: C₁-C₄ alkyl, C₃-C₆ cycloalkyl, 3-6 membered heterocycloalkyl containing 1 or 2 heteroatoms selected from N, CN or halogen, wherein the alkyl, cycloalkyl and heterocycloalkyl are optionally substituted with halogen, OH, C₁-C₄ alkyl or C₁-C₄ alkoxy.

Preferably, in certain embodiments of the present invention, in the PTM-1, PTM-2 and PTM-3, R₂ and R₃ are each independently selected from: hydrogen, C₁-C₆ alkyl, C₃-C₈ cycloalkyl or 3-8 membered heterocycloalkyl, and are each optionally substituted with one or more halogens, OH, N(R_{b})₂ or morpholinyl groups, or R₂ and R₃, together with the nitrogen atom to which they are attached, form 3-8 membered heterocycloalkyl, wherein the heterocycloalkyl is optionally substituted with one or more substituents selected from Rₐ; Rₐ is independently selected from C₁-C₄ alkyl, C₃-C₆ cycloalkyl, CF₃, CHF₂, OH, halogen or NH₂, wherein the alkyl is optionally substituted with C₁-C₄ alkyl, C₃-C₆ cycloalkyl or CF₃; and R_{b} is independently selected from H or C₁-C₄ alkyl; more preferably, R₂ and R₃, together with the nitrogen atom to which they are attached, form 3-8 membered heterocycloalkyl containing, in addition to the N atom to which they are attached, 1 additional heteroatom selected from N or O, wherein the heterocycloalkyl is optionally substituted with one or more substituents selected from Rₐ; the Rₐ is independently selected from C₁-C₄ alkyl, CF₃, CHF₂, OH, halogen or NH₂; further preferably, the R₂ and R₃, together with the nitrogen atom to which they are attached, form 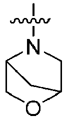 ; still further preferably, the R₂ and R₃, together with the nitrogen atom to which they are attached, form

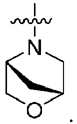

Preferably, in certain embodiments of the present invention, R₂ and R₃, together with the nitrogen atom to which they are attached, form 3-8 membered heterocycloalkyl containing, in addition to the N atom to which they are attached, 1 additional heteroatom selected from N, S or O, wherein the heterocycloalkyl is optionally substituted with one or more substituents selected from Rₐ; the Rₐ is independently selected from C₁-C₄ alkyl, C₁-C₄ alkyl substituted with 1, 2 or 3 halogens (preferably C₁-C₄ alkyl substituted with 1, 2 or 3 fluorines, and more preferably methyl substituted with 1, 2 or 3 fluorines), OH, halogen or NH₂; preferably, the R₂ and R₃, together with the nitrogen atom to which they are attached, form

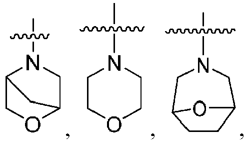

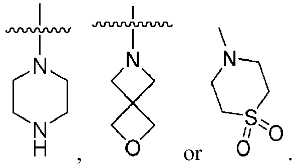

Preferably, in certain embodiments of the present invention, in the PTM-1, PTM-2' and PTM-3, R₂ and R₃ are each independently selected from: hydrogen, C₁-C₆ alkyl, C₃-C₈ cycloalkyl or 3-8 membered heterocycloalkyl, and are each optionally substituted with one or more halogens, OH, N(R_{b})₂ or morpholinyl groups, or R₂ and R₃, together with the nitrogen atom to which they are attached, form 3-8 membered heterocycloalkyl, wherein the heterocycloalkyl is optionally substituted with one or more substituents selected from Rₐ; Rₐ is independently selected from C₁-C₄ alkyl, C₃-C₆ cycloalkyl, CF₃, CHF₂, OH, halogen or NH₂, wherein the alkyl is optionally substituted with C₃-C₆ cycloalkyl or CF₃; and R_{b} is independently selected from H or C₁-C₄ alkyl; preferably, R₂ and R₃, together with the nitrogen atom to which they are attached, form 3-8 membered heterocycloalkyl containing, in addition to the N atom to which they are attached, 1 additional heteroatom selected from N or O, wherein the heterocycloalkyl is optionally substituted with one or more substituents selected from Rₐ; the Rₐ is independently selected from C₁-C₄ alkyl, CF₃, CHF₂, OH, halogen or NH₂; preferably, the R₂ and R₃, together with the nitrogen atom to which they are attached, form

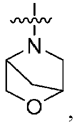

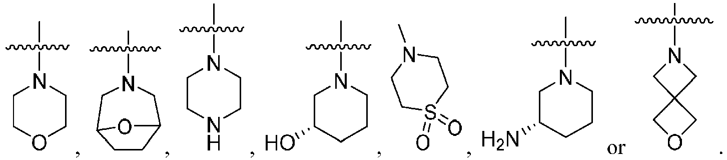

Preferably, in certain embodiments of the present invention, in the PTM-1, PTM-2' and PTM-3, R₂ and R₃ are each independently selected from: hydrogen, C₁-C₆ alkyl, C₃-C₈ cycloalkyl and 3-8 membered heterocycloalkyl, and are each optionally substituted with one or more halogens, OH, N(R_{b})₂ and morpholinyl groups, or R₂ and R₃, together with the nitrogen atom to which they are attached, form 3-8 membered heterocycloalkyl, wherein the heterocycloalkyl is optionally substituted with one or more substituents selected from Rₐ; Rₐ is independently selected from C₁-C₄ alkyl, C₃-C₆ cycloalkyl, CF₃, CHF₂, OH, halogen or NH₂, wherein the alkyl is optionally substituted with C₁-C₄ alkyl, C₃-C₆ cycloalkyl and CF₃; and R_{b} is independently selected from H and C₁-C₄ alkyl; preferably, R₂ and R₃, together with the nitrogen atom to which they are attached, form 3-8 membered heterocycloalkyl containing, in addition to the N atom to which they are attached, 1 additional heteroatom selected from N or O, wherein the heterocycloalkyl is optionally substituted with one or more substituents selected from Rₐ; the Rₐ is independently selected from C₁-C₄ alkyl, CF₃, CHF₂, OH, halogen and NH₂; preferably, the R₂ and R₃, together with the nitrogen atom to which they are attached, form

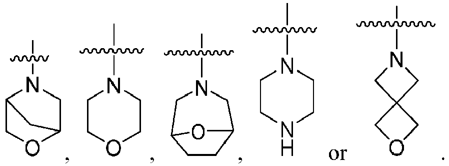

Preferably, in certain embodiments of the present invention, the PTM has a structure of the following formula: 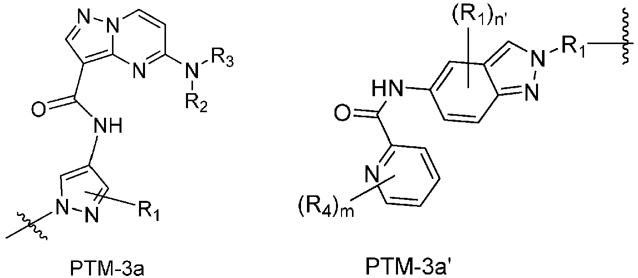 wherein,
n' is 0, 1, 2 or 3;
the R₁, R₂, R₃, R₄, m and n are as described and defined in PTM-1, PTM-2, PTM-2' and PTM-3.

Preferably, in certain embodiments of the present invention, the PTM has a structure of the following formula: 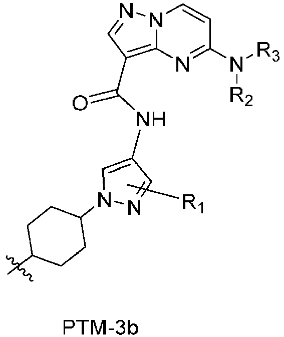 wherein R₁, R₂, R₃, R₄, m and n are as described and defined in PTM-1, PTM-2, PTM-2' and PTM-3.

Preferably, in certain embodiments of the present invention, the PTM has a structure of the following formula: 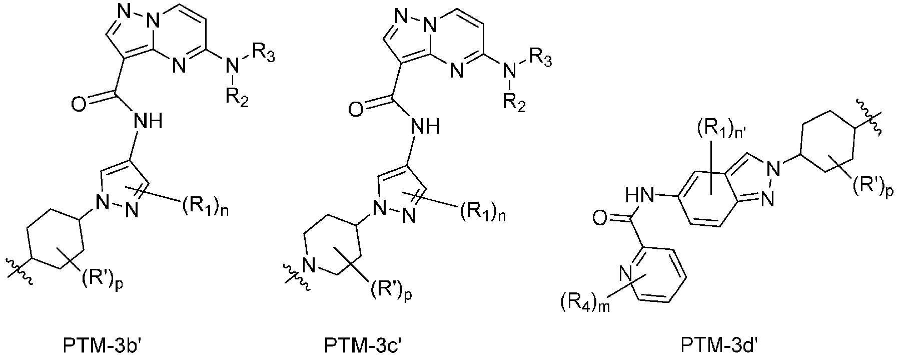 wherein,
n' is 0, 1, 2 or 3;
each R is independently hydrogen, deuterium, hydroxy, amino, cyano, halogen, C₁-C₆ alkyl or - O(C₁-C₆ alkyl), wherein the alkyl is optionally substituted with 1-3 groups independently selected from hydroxy, halogen, cyano and amino;
p is 0, 1, 2, 3, 4, 5, 6, 7, 8 or 9;
the R₁, R₂, R₃, R₄, m and n are as described and defined in PTM-1, PTM-2' and PTM-3.

Preferably, in certain embodiments of the present invention, the PTM has a structure of the following formula: 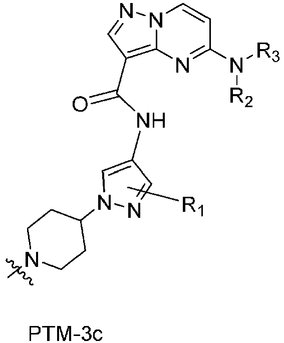 wherein the R₁, R₂ and R₃ are as described and defined in PTM-1, PTM-2' and PTM-3.

Preferably, in certain embodiments of the present invention, the PTM has a structure of the following formula: 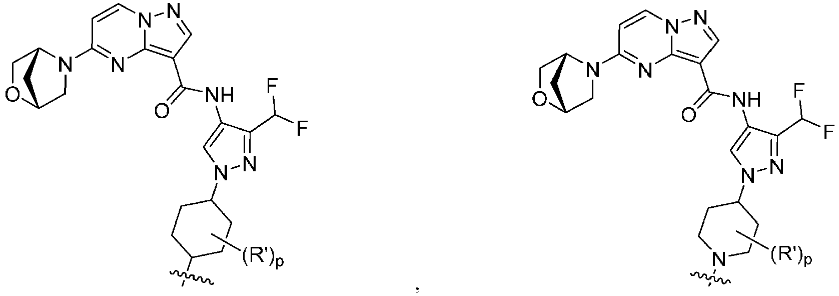 or 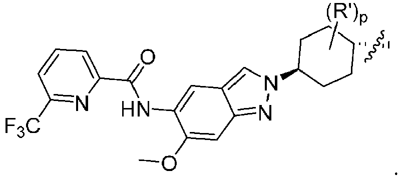 wherein the R' and p are as defined and described in PTM-3b', PTM-3c' and PTM-3d'.

Preferably, in certain embodiments of the present invention, the PTM has a structure of the following formula:

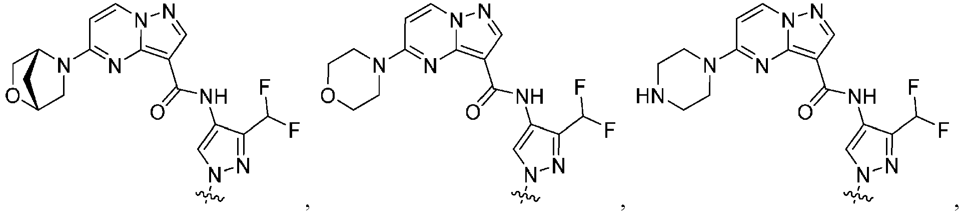

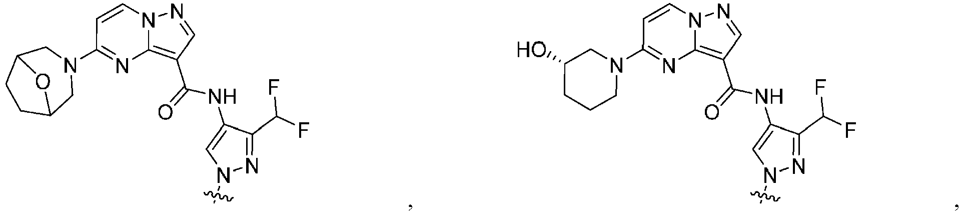

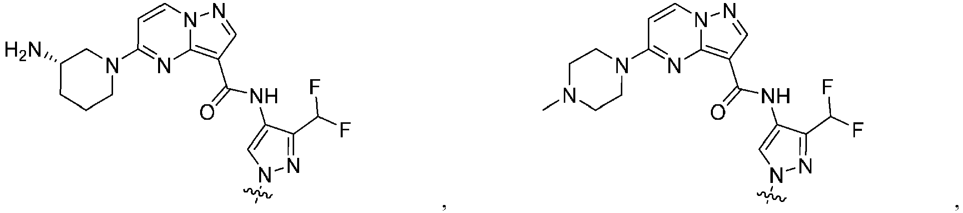

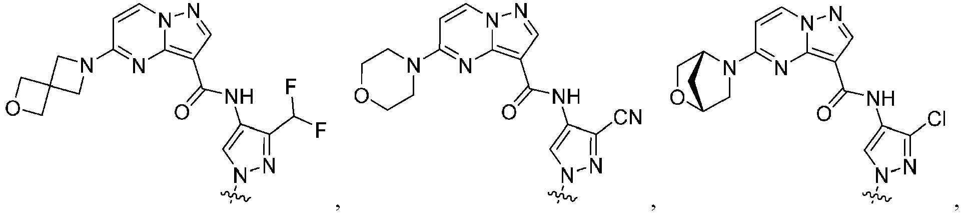

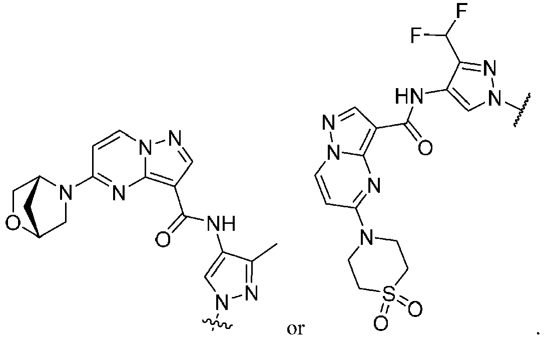

In certain embodiments of the present invention, 1 or 2 of Q₁, Q₂, Q₃, Q₄ and Q₅ in the ULM-1 are N, and the rest are each independently CR₃".

In certain embodiments of the present invention, Q₁, Q₂, Q₃, Q₄ and Q₅ in the ULM-1 are each independently CR₃".

In certain embodiments of the present invention, X" in the ULM-1 is N.

In certain embodiments of the present invention, X" in the ULM-1 is C.

In certain embodiments of the present invention, Y" in the ULM-1 is N.

In certain embodiments of the present invention, each R₁" in the ULM-1 is independently hydrogen, deuterium, -F, -Cl or C₁-C₆ alkyl, wherein the alkyl is optionally substituted with 1-3 halogens; preferably, R₁" is hydrogen.

In certain embodiments of the present invention, R₂" in the ULM-1 is hydrogen or C₁-C₆ alkyl, wherein the alkyl is optionally substituted with 1-3 halogens; preferably, R₂" is hydrogen.

In certain embodiments of the present invention, each R₃" in the ULM-1 is independently hydrogen, deuterium, halogen, -O(C₁-C₆ alkyl) or C₁-C₆ alkyl, wherein the alkyl is optionally substituted with 1-3 halogens; preferably, each R₃" is independently hydrogen, deuterium, F, Cl, methyl, methoxy, ethoxy, trifluoromethoxy, 2-hydroxypropan-2-yl or trifluoromethyl.

In certain embodiments of the present invention, each R₃" is independently hydrogen, deuterium, hydroxy, amino, cyano, halogen, C₁-C₆ alkyl or -O(C₁-C₆ alkyl); the alkyl is optionally substituted with 1-3 groups independently selected from hydroxy and halogen.

In certain embodiments of the present invention, the ULM has a structure of the following formula: 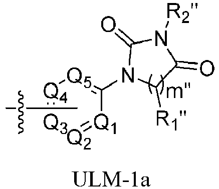 wherein the Q₁, Q₂, Q₃, Q₄, Q₅, R₁", R₂" and m" are as defined in the ULM-1 described above.

In certain embodiments of the present invention, the ULM has a structure of the following formula: 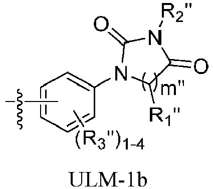 wherein the R₁", R₂", R₃" and m" are as defined in the ULM-1 described above.

In certain embodiments of the present invention, the ULM has a structure of the following formula: 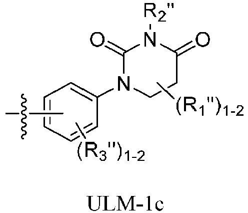 wherein the R₁", R₂" and R₃" are as defined in the ULM-1 described above.

In certain embodiments of the present invention, the ULM has a structure of the following formula: 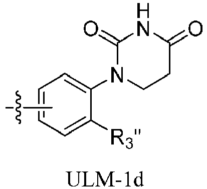 wherein the R₃" is as defined in the ULM-1 described above.

In certain embodiments of the present invention, the ULM has a structure of the following formula: 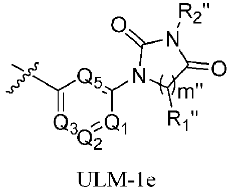 wherein the Q₁, Q₂, Q₃, Q₅, R₁", R₂" and m" are as defined in the ULM-1 described above.

In certain embodiments of the present invention, the ULM has a structure of the following formula: 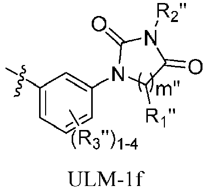 wherein the R₁", R₂", R₃" and m" are as defined in the ULM-1 described above.

In certain embodiments of the present invention, the ULM has a structure of the following formula: 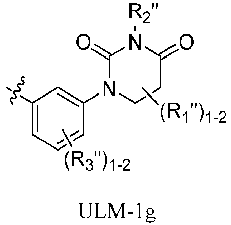 wherein the R₁", R₂" and R₃" are as defined in the ULM-1 described above. 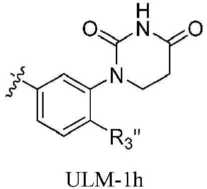 wherein the R₃" is as defined in the ULM-1 described above.

In certain embodiments of the present invention, the ULM has a structure of the following formula: 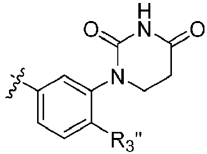 ; R₃" is halogen, C₁-C₆ alkyl or -O(C₁-C₆ alkyl).

In certain embodiments of the present invention, the ULM is selected from

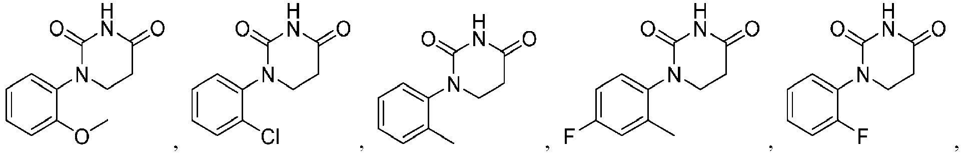

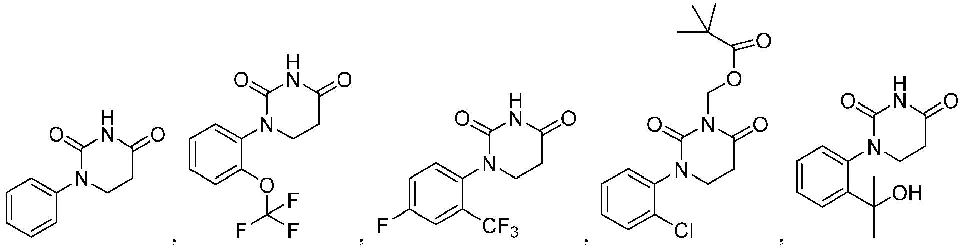

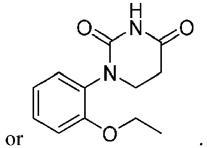

In certain embodiments of the present invention, the ULM is selected from 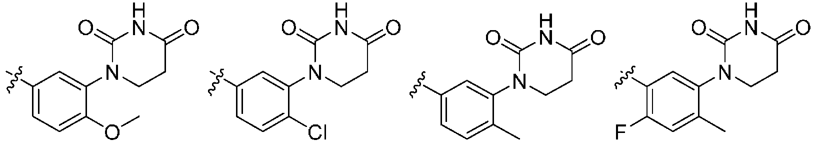 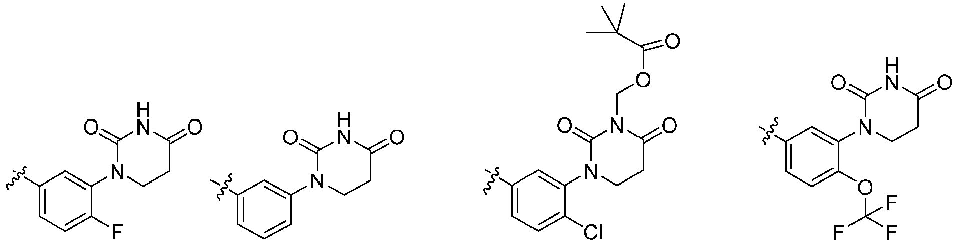 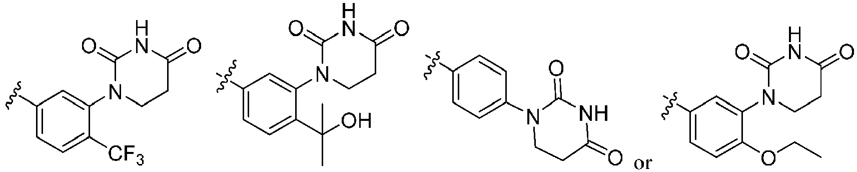 , preferably

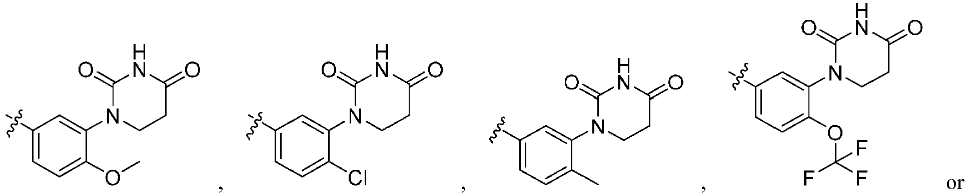

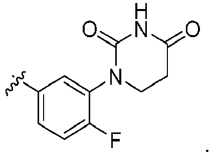

In certain embodiments of the present invention, the ULM has a structure of the following formula: 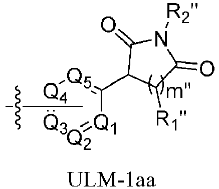 wherein the Q₁, Q₂, Q₃, Q₄, Q₅, R₁", R₂" and m" are as defined in the ULM-1 described above.

In certain embodiments of the present invention, the ULM has a structure of the following formula: 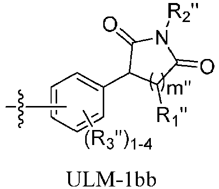 wherein the R₁", R₂", R₃" and m" are as defined in the ULM-1 described above.

In certain embodiments of the present invention, the ULM has a structure of the following formula: 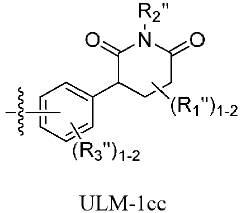 wherein the R₁", R₂" and R₃" are as defined in the ULM-1 described above.

In certain embodiments of the present invention, the ULM has a structure of the following formula: 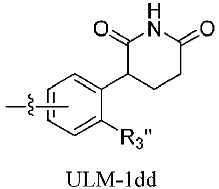 wherein the R₃" is as defined in the ULM-1 described above.

In certain embodiments of the present invention, the ULM has a structure of the following formula: 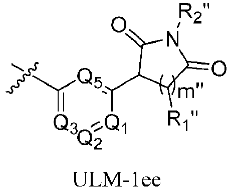 wherein the Q₁, Q₂, Q₃, Q₅, R₁", R₂" and m" are as defined in the ULM-1 described above.

In certain embodiments of the present invention, the ULM has a structure of the following formula: 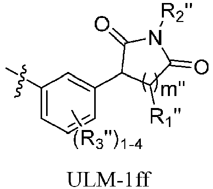 wherein the R₁", R₂", R₃" and m" are as defined in the ULM-1 described above.

In certain embodiments of the present invention, the ULM has a structure of the following formula: 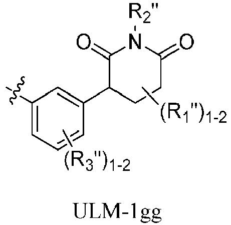 wherein the R₁", R₂" and R₃" are as defined in the ULM-1 described above. 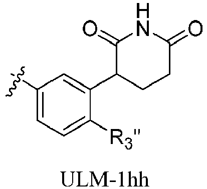 wherein the R₃" is as defined in the ULM-1 described above.

In certain embodiments of the present invention, the ULM is selected from

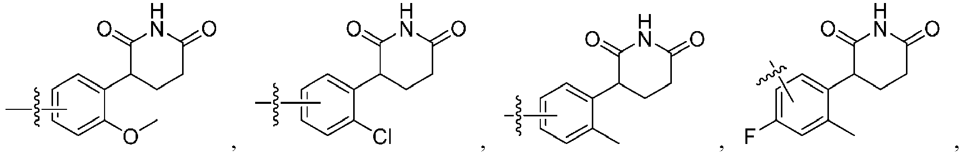

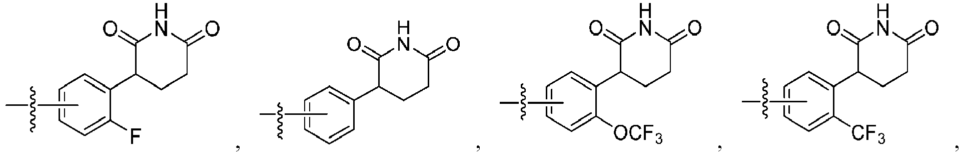

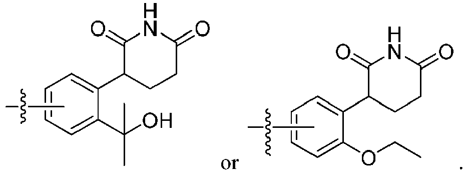

In certain embodiments of the present invention, the ULM is selected from

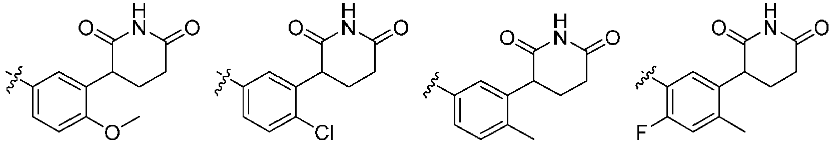

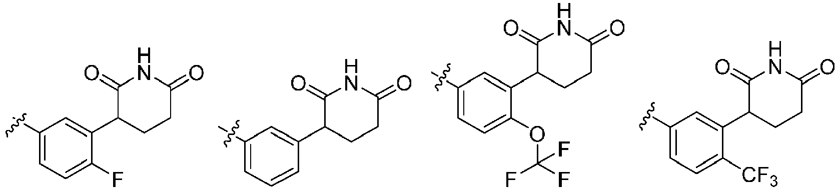

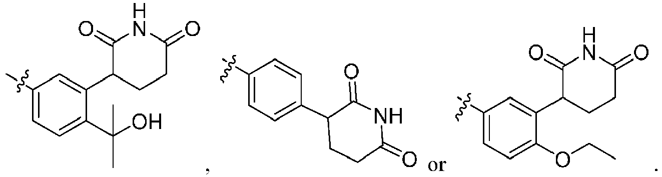

In certain embodiments of the present invention, L is a bond.

In certain embodiments of the present invention, L is -(CH₂)ⱼ-, wherein 1 or more methylene groups in the -(CH₂)ⱼ- are optionally substituted with groups selected from -NR^{3'}-, -O-, -S-, -S(O)-, -S(O)NR^{3'}-, - NR^{3'}S(O)-, -S(O)₂-, -S(O)₂NR^{3'}-, -NR^{3'}S(O)₂-, -NR^{4'}S(O)₂NR^{3'}-, -CR^{1'}R^{2'}-, -C(O)-, -C(O)O-, -OC(O)-, - NR^{3'}C(O)O-, -OC(O)NR^{3'}-, -C(O)NR^{3'}-, -NR^{3'}C(O)-, -NR^{4'}C(O)NR^{3'}-, -P(O)-, -P(O)O-, -OP(O)-, -OP(O)O-, ethenylene, ethynylene, C₃-C₁₂ cycloalkylene, 3-12 membered heterocycloalkylene containing 1 or more heteroatoms selected from N, O or S, 6-10 membered arylene or 5-10 membered heteroarylene, wherein the ethenylene, cycloalkylene, heterocycloalkylene, arylene and heteroarylene are each independently optionally substituted with 1 or more substituents selected from halogen, -OR^{3'}, -NR^{3'}R^{4'}, oxo, nitro, cyano, C1-C6 alkyl, -S(C1-C6 alkyl), C3-C10 cycloalkyl, 3-10 membered heterocycloalkyl, 6-10 membered aryl, 5-10 membered heteroaryl, -C(O)R^{1'}, -C(O)OR^{3'}, -OC(O)R^{1'}, -C(O)NR^{3'}, -NR^{3'}C(O)R^{1'}, -S(O)R^{1'}, -S(O)NR^{3'}, -S(O)₂R^{1'}, - S(O)₂NR^{3'}, -NR^{3'}S(O)₂R^{1'}, -NR^{4'}S(O)₂NR^{3'}, -OC(O)NR^{3'} and NR^{4'}C(O)NR^{3'}, wherein the alkyl, cycloalkyl, heterocycloalkyl, aryl and heteroaryl are each independently optionally substituted with 1 or more substituents selected from halogen, -OH, -NR^{3'}R^{4'}, oxo, nitro, cyano, C₁-C₆ alkyl, C₃-C₁₀ cycloalkyl, 3-10 membered heterocycloalkyl, 6-10 membered aryl, and 5-10 membered heteroaryl; R^{1'} and R^{2'} are each independently halogen, -OH, -NR^{3'}R^{4'}, C₁-C₆ alkyl, C₁-C₆ chloroalkyl, hydroxy C₁-C₆ alkyl, -O(C₁-C₆ alkyl), -NH(C₁-C₆ alkyl), -NH(C₁-C₆ alkyl)₂, C₃-C₁₀ cycloalkyl, -O(C₃-C₁₀ cycloalkyl), -NH(C₃-C₁₀ cycloalkyl), 3-10 membered heterocycloalkyl, -0(3-10 membered heterocycloalkyl), -NH(3-10 membered heterocycloalkyl), 6-10 membered aryl, -0(6-10 membered aryl), -NH(6-10 membered aryl), 5-10 membered heteroaryl, -0(5-10 membered heteroaryl) or -NH(5-10 membered heteroaryl), wherein R^{3'} and R^{4'} are each independently hydrogen, deuterium, C₁-C₆ alkyl, C₃-C₁₀ cycloalkyl, 3-10 membered heterocycloalkyl, 6-10 membered aryl or 5-10 membered heteroaryl; j is 1, 2, 3, 4, 5, 6, 7, 8, 9, 10, 11, 12, 13, 14, 15, 16, 17, 18, 19, 20, 21, 22, 23, 24 or 25.

In certain embodiments of the present invention, L is -(CH₂)ⱼ-, wherein 1 or more methylene groups in the -(CH₂)ⱼ- are optionally substituted with groups selected from -NR^{3'}-, -O-, -S-, -S(O)-, -S(O)NR^{3'}-, - NR^{3'}S(O)-, -S(O)₂-, -S(O)₂NR^{3'}-, -NR³'S(O)₂-, -NR^{4'}S(O)₂NR^{3'}-, -CR^{1'}R^{2'}-, -C(O)-, -C(O)O-, -OC(O)-, - NR^{3'}C(O)O-, -OC(O)NR^{3'}-, -C(O)NR^{3'}-, -NR^{3'}C(O)-, -NR^{4'}C(O)NR^{3'}-, -P(O)-, -P(O)O-, -OP(O)-, -OP(O)O-, ethenylene, ethynylene, C₃-C₁₂ cycloalkylene, 3-12 membered heterocycloalkylene containing 1 or more heteroatoms selected from N, O or S, 6-10 membered arylene or 5-10 membered heteroarylene, wherein the ethenylene, cycloalkylene, heterocycloalkylene, arylene and heteroarylene are each independently optionally substituted with 1 or more substituents selected from halogen, -OR^{3'}, -NR^{3'}R^{4'}, oxo, nitro, cyano, C1-C6 alkyl, -S(C1-C6 alkyl), C3-C10 cycloalkyl, 3-10 membered heterocycloalkyl, 6-10 membered aryl, 5-10 membered heteroaryl, -C(O)R^{1'}, -C(O)OR^{3'}, -OC(O)R^{1'}, -C(O)NR^{3'}, -NR^{3'}C(O)R^{1'}, -S(O)R^{1'}, -S(O)NR^{3'}, -S(O)₂R^{1'}, - S(O)₂NR^{3'}, -NR^{3'}S(O)₂R^{1'}, -NR^{4'}S(O)₂NR^{3'}, -OC(O)NR^{3'} and NR^{4'}C(O)NR^{3'}, wherein the alkyl, cycloalkyl, heterocycloalkyl, aryl and heteroaryl are each independently optionally substituted with 1 or more substituents selected from halogen, -OH, -NR^{3'}R^{4'}, oxo, nitro, cyano, C₁-C₆ alkyl, C₃-C₁₀ cycloalkyl, 3-10 membered heterocycloalkyl, 6-10 membered aryl, and 5-10 membered heteroaryl; R^{1'} and R^{2'} are each independently halogen, -OH, -NR^{3'}R^{4'}, C₁-C₆ alkyl, C₁-C₆ chloroalkyl, hydroxy C₁-C₆ alkyl, -O(C₁-C₆ alkyl), -NH(C₁-C₆ alkyl), -NH(C₁-C₆ alkyl)₂, C₃-C₁₀ cycloalkyl, -O(C₃-C₁₀ cycloalkyl), -NH(C₃-C₁₀ cycloalkyl), 3-10 membered heterocycloalkyl, -0(3-10 membered heterocycloalkyl), -NH(3-10 membered heterocycloalkyl), 6-10 membered aryl, -0(6-10 membered aryl), -NH(6-10 membered aryl), 5-10 membered heteroaryl, -0(5-10 membered heteroaryl) or -NH(5-10 membered heteroaryl), wherein R^{3'} and R^{4'} are each independently hydrogen, deuterium, C₁-C₆ alkyl, C₃-C₁₀ cycloalkyl, 3-10 membered heterocycloalkyl, 6-10 membered aryl or 5-10 membered heteroaryl; j is 1, 2, 3, 4, 5, 6, 7, 8, 9, 10, 11, 12, 13, 14, 15, 16, 17, 18, 19, 20, 21, 22, 23, 24 or 25.

Preferably, in certain embodiments of the present invention, L is -(CH₂)ⱼ-, wherein 1 or more methylene groups in the -(CH₂)ⱼ- are optionally replaced by groups selected from -NR^{3'}-, -O-, -CR^{1'}R^{2'}-, - C(O)-, -S(O)-, -S(O)₂-, -C(O)O-, -OC(O)-, -C(O)NR^{3'}-, -NR^{3'}C(O)-, -S(O)₂NR^{3'}-, -NR^{3'}S(O)₂-, ethenylene, ethynylene, phenyl, 8-10 membered bicyclic arylene, 3-7 membered saturated and/or partially unsaturated cycloalkylene, 4-11 membered saturated and/or partially unsaturated spirocycloalkylene, 4-11 membered saturated and/or partially unsaturated fused cycloalkylene, 8-10 membered bicyclic saturated and/or partially unsaturated cycloalkylene, 4-7 membered saturated and/or partially unsaturated heterocycloalkylene having 1-2 heteroatoms independently selected from nitrogen, oxygen or sulfur, 4-11 membered saturated and/or partially unsaturated spiroheterocycloalkylene having 1-2 heteroatoms independently selected from nitrogen, oxygen or sulfur, 4-11 membered saturated and/or partially unsaturated fused heterocycloalkylene having 1-2 heteroatoms independently selected from nitrogen, oxygen or sulfur, 8-10 membered bicyclic saturated and/or partially unsaturated heterocycloalkylene having 1-2 heteroatoms independently selected from nitrogen, oxygen or sulfur, 5-6 membered heteroarylene having 1-4 heteroatoms independently selected from nitrogen, oxygen or sulfur, or 8-10 membered bicyclic heteroaryl having 1-5 heteroatoms selected from nitrogen, oxygen or sulfur, wherein the ethenylene, ethynylene, cycloalkylene, heterocycloalkylene, phenyl, spiroheterocycloalkylene, fused heterocycloalkylene, spirocycloalkylene, fused cycloalkylene and heteroarylene are each independently optionally substituted with 1 or more substituents selected from halogen, oxo, -NR^{3'}R^{4'}, -OR^{3'}, nitro, -CN, C₁-C₆ alkyl, C₃-C₁₀ cycloalkyl and 3-10 membered heterocycloalkyl, wherein the alkyl, cycloalkyl and heterocycloalkyl are optionally substituted with 1 or more substituents selected from halogen, -OH, -NH₂, -CN, C₁-C₄ alkyl and C₃-C₆ cycloalkyl; R^{1'} and R^{2'} are each independently halogen, -OH, -NH₂, C₁-C₄ alkyl, C₁-C₄ chloroalkyl, C₁-C₄ hydroxyalkyl, -O(C₁-C₄ alkyl), -NH(C₁-C₄ alkyl), -NH(C₁-C₄ alkyl), C₃-C₆ cycloalkyl, -O(C₃-C₆ cycloalkyl), -NH(C₃-C₆ cycloalkyl), 3-6 membered heterocycloalkyl, -O(3-6 membered heterocycloalkyl) or -NH(C₃-C₆ cycloalkyl); R^{3'} and R^{4'} are each independently hydrogen, deuterium, C₁-C₄ alkyl, C₃-C₆ cycloalkyl or 3-6 membered heterocycloalkyl; j is 2, 3, 4, 5, 6, 7, 8, 9, 10, 11 or 12.

Preferably, in certain embodiments of the present invention, L is -(CH₂)ⱼ-, wherein 1 or more methylene groups in the -(CH₂)ⱼ- are optionally replaced by groups selected from -NR^{3'}-, -O-, -CR^{1'}R^{2'}-, - C(O)-, -S(O)-, -S(O)₂-, -C(O)O-, -OC(O)-, -C(O)NR^{3'}-, -NR^{3'}C(O)-, -S(O)₂NR^{3'}-, -NR^{3'}S(O)₂-, ethenylene, ethynylene, phenyl, 8-10 membered bicyclic arylene, 3-7 membered saturated and/or partially unsaturated monocyclic cycloalkylene, 4-11 membered saturated and/or partially unsaturated spirocycloalkylene, 4-11 membered saturated and/or partially unsaturated fused cycloalkylene, 8-10 membered bicyclic saturated and/or partially unsaturated cycloalkylene, 4-7 membered saturated and/or partially unsaturated heterocycloalkylene having 1-2 heteroatoms independently selected from nitrogen or oxygen, 4-11 membered saturated and/or partially unsaturated spiroheterocycloalkylene having 1-2 heteroatoms independently selected from nitrogen or oxygen, 4-11 membered saturated and/or partially unsaturated fused heterocycloalkylene having 1-2 heteroatoms independently selected from nitrogen or oxygen, 8-10 membered bicyclic saturated and/or partially unsaturated heterocycloalkylene having 1-2 heteroatoms independently selected from nitrogen or oxygen, 5-6 membered heteroarylene having 1-4 heteroatoms independently selected from nitrogen or oxygen, or 8-10 membered bicyclic heteroaryl having 1-5 heteroatoms selected from nitrogen or oxygen, wherein the ethenylene, ethynylene, cycloalkylene, heterocycloalkylene, phenyl, spiroheterocycloalkylene, fused heterocycloalkylene, fused cycloalkylene, spirocycloalkylene and heteroarylene are each independently optionally substituted with 1 or more substituents selected from halogen, oxo, -NR^{3'}R^{4'}, -OR^{3'}, nitro, -CN, C₁-C₆ alkyl, C₃-C₆ cycloalkyl and 3-6 membered heterocycloalkyl, wherein the alkyl, cycloalkyl and heterocycloalkyl are optionally substituted with 1 or more substituents selected from halogen, -OH, -NH₂, - CN, C₁-C₄ alkyl and C₃-C₆ cycloalkyl; R^{1'} and R^{2'} are each independently halogen, -OH, -NH₂, C₁-C₄ alkyl, C₁-C₄ chloroalkyl, C₁-C₄ hydroxyalkyl, -O(C₁-C₄ alkyl), -NH(C₁-C₄ alkyl), -NH(C₁-C₄ alkyl), C₃-C₆ cycloalkyl, -O(C₃-C₆ cycloalkyl), -NH(C₃-C₆ cycloalkyl), 3-6 membered heterocycloalkyl, -O(3-6 membered heterocycloalkyl) or -NH(C₃-C₆ cycloalkyl); R^{3'} and R^{4'} are each independently hydrogen, deuterium, C₁-C₄ alkyl, C₃-C₆ cycloalkyl or 3-6 membered heterocycloalkyl; j is 2, 3, 4, 5, 6, 7, 8, 9 or 10.

Preferably, in certain embodiments of the present invention, L is -(CH₂)ⱼ-, wherein 1, 2, 3 or 4 methylene groups in the -(CH₂)ⱼ- are optionally replaced by groups selected from -NH-, -NCH₃-, -NCH₂CH₃-, -O-, -C(CH₃)₂-, -CHF-, -CHCF₃-, -C(O)-, -C(O)O-, -OC(O)-, -C(O)NH-, -C(O)NCH₃-, -NHC(O)-, - NCH₃C(O)-, ethenylene, ethynylene, cyclopropylene, cyclobutylene, cyclopentylene, cyclohexylene, oxiranylidene, oxetanylidene, oxolanylidene, oxanylidene, aziridinylidene, azetidinylidene, azolidinylidene, piperidinylidene, piperazinylidene, morpholinylidene, homomorpholinylidene, phenylene, pyrrolylidene, thienylidene, furylidene, imidazolylidene, pyrazolylidene, triazolylidene, tetrazolylidene, oxazolylidene, isoxazolylidene, thiazolylidene, isothiazolylidene, pyridinylidene, pyrimidinylidene, pyridazinylidene, pyrazinylidene, 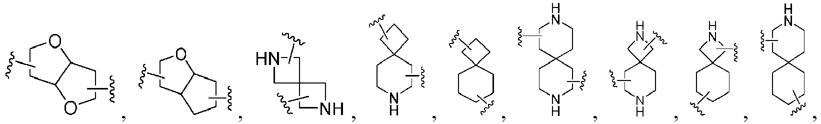 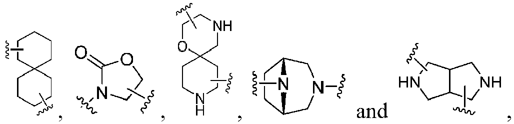 wherein the group for replacement is optionally substituted with 1 or more substituents selected from halogen, oxo, -NR^{3'}R^{4'}, -OR^{3'} and C₁-C₄ alkyl, wherein the alkyl is optionally substituted with 1 or more substituents selected from halogen, -OH and -NH₂; R^{3'} and R^{4'} are each independently hydrogen, deuterium or C₁-C₄ alkyl; j is 2, 3, 4, 5, 6, 7 or 8.

Preferably, in certain embodiments of the present invention, L is -(CH₂)ⱼ-, wherein 1, 2 or 3 methylene groups in the -(CH₂)ⱼ- are optionally replaced by groups selected from -NH-, -NCH₃-, -NCH₂CH₃-, -O-, -C(CH₃)₂-, -CHF-, -CHCF₃-, -C(O)-, -C(O)O-, -OC(O)-, -C(O)NH-, -C(O)NCH₃-, -NHC(O)-, - NCH₃C(O)-, cyclopropylene, cyclobutylene, cyclopentylene, cyclohexylene, oxiranylidene, oxetanylidene, oxolanylidene, oxanylidene, aziridinylidene, azetidinylidene, azolidinylidene, piperidinylidene, piperazinylidene, morpholinylidene, homomorpholinylidene, 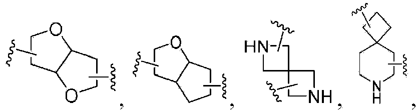 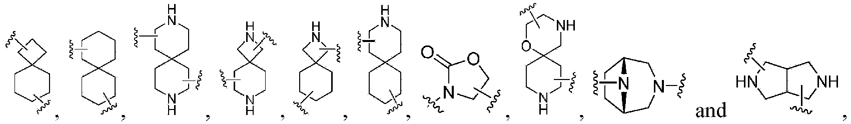 wherein the group for replacement is optionally substituted with 1 or more substituents selected from F, Cl, oxo, -NR^{3'}R^{4'}, -OR^{3'} and C₁-C₄ alkyl, wherein the alkyl is optionally substituted with 1 or more substituents selected from halogen, -OH and -NH₂; R^{3'} and R^{4'} are each independently hydrogen, deuterium, methyl, ethyl and propyl; j is 2, 3, 4, 5, 6, 7 or 8.

Preferably, in certain embodiments of the present invention, L is -(CH₂)ⱼ-, wherein 1, 2 or 3 methylene groups in the -(CH₂)ⱼ- are optionally replaced by groups selected from -O-, -NH-, -NCH₃-, - NCH₂CH₃-, -C(O)-, -C(O)NH-, -NHC(O)-, -NCH₃C(O)-, -C(O)NCH₃-, 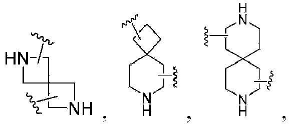 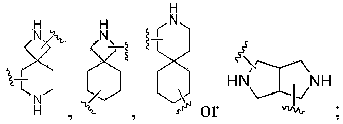 j is 2, 3, 4, 5, 6, 7 or 8.

Preferably, in certain embodiments of the present invention, L is selected from -(CH₂)ⱼ₋₁-C(O)-, wherein the methylene in the -(CH₂)ⱼ₋₁-C(O)- is as defined in the L described above, and is optionally replaced by 1 or more groups, and j is as defined in the L described above.

Preferably, in certain embodiments of the present invention, L is selected from

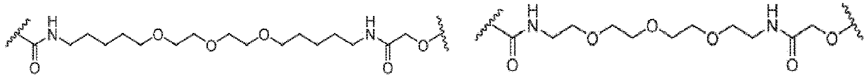

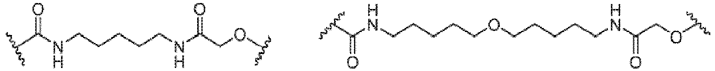

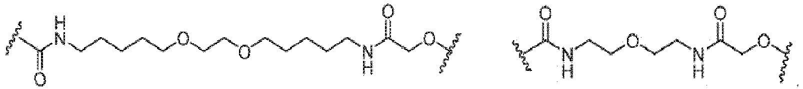

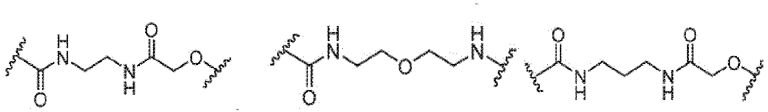

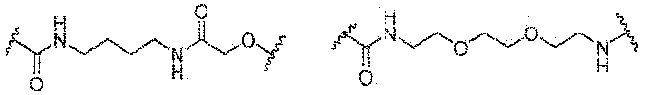

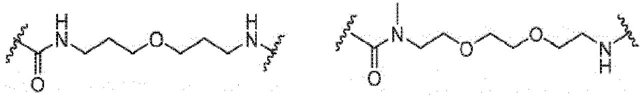

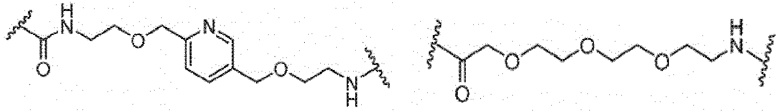

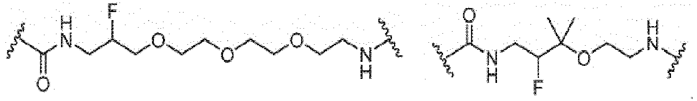

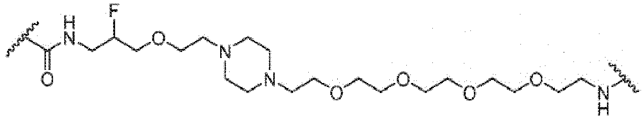

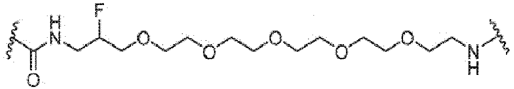

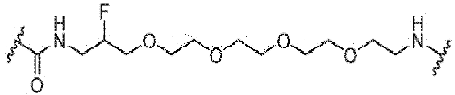

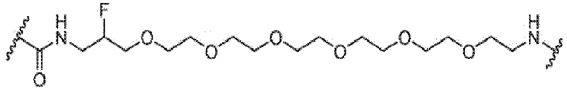

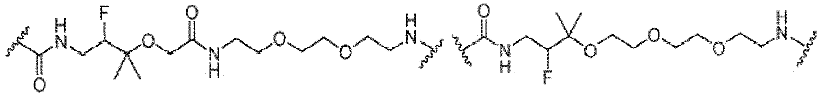

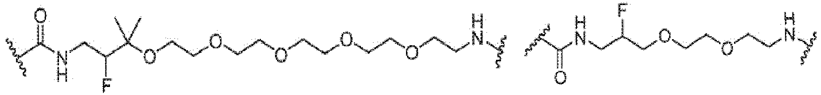

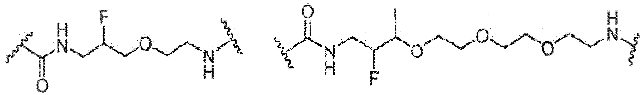

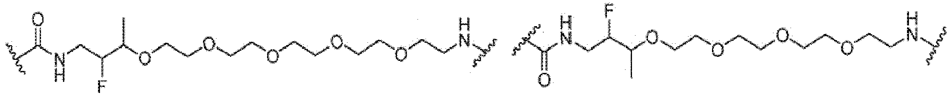

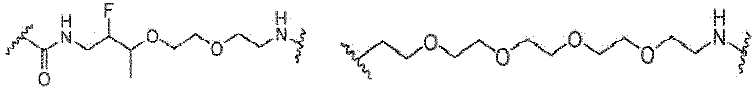

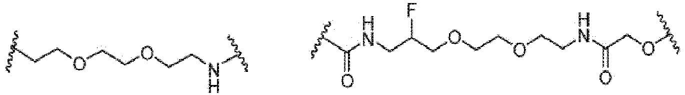

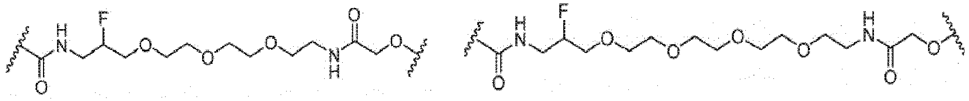

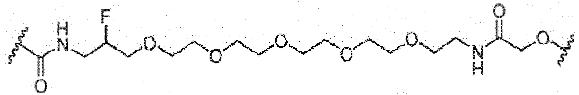

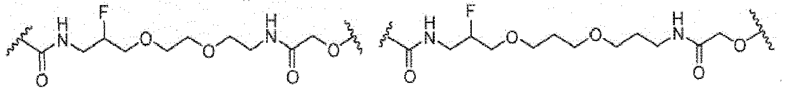

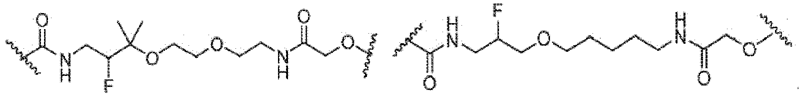

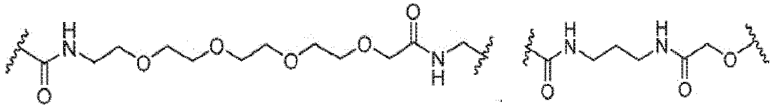

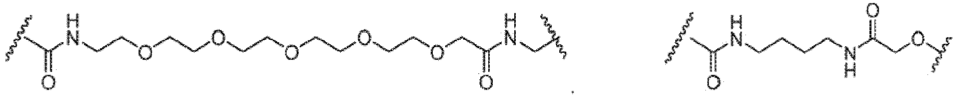

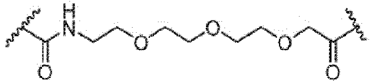

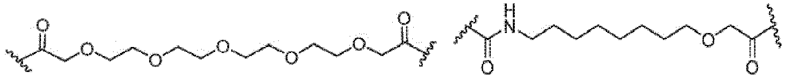

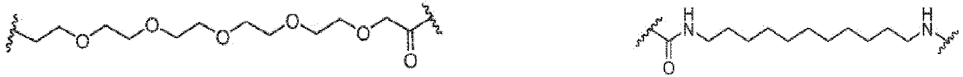

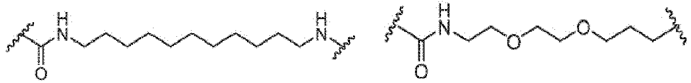

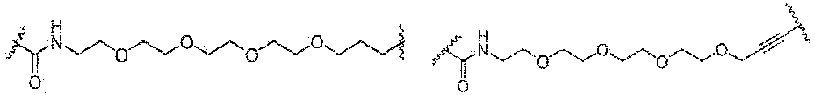

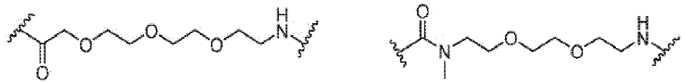

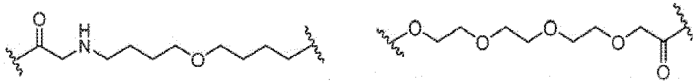

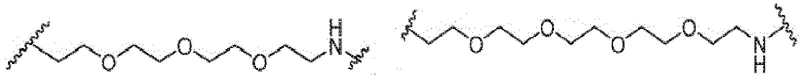

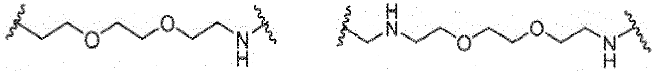

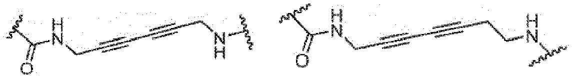

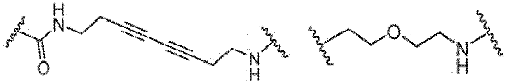

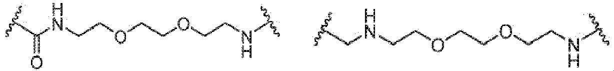

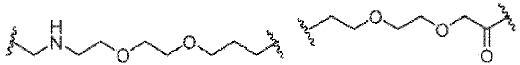

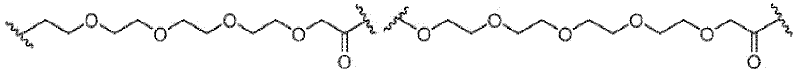

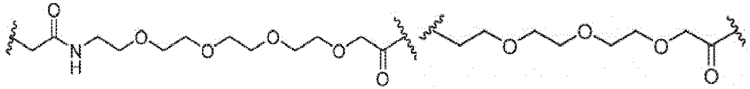

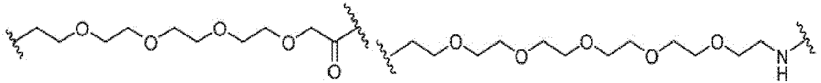

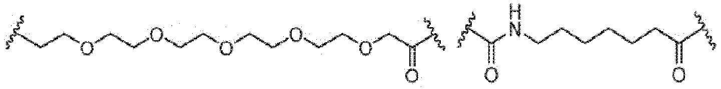

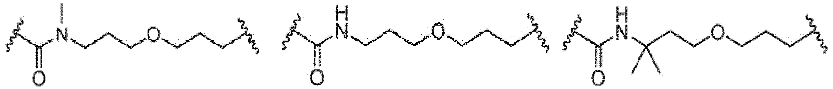

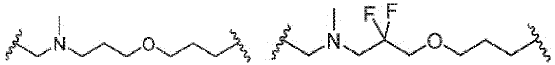

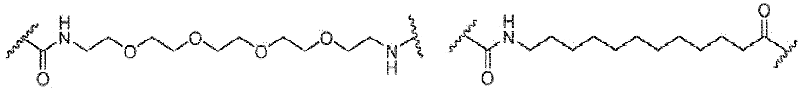

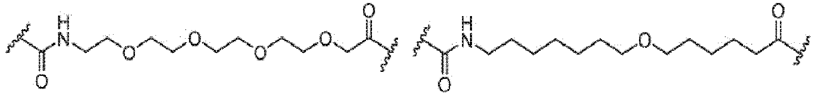

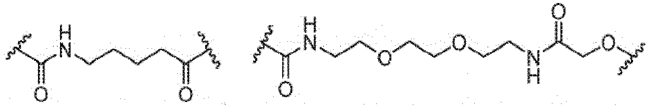

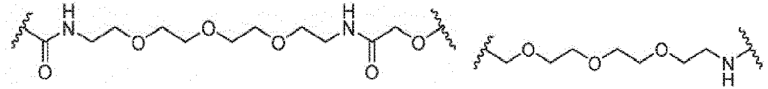

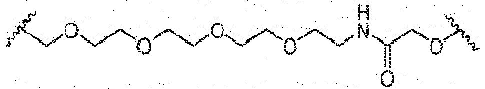

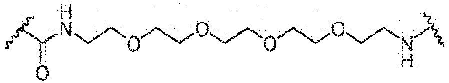

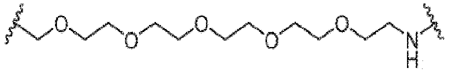

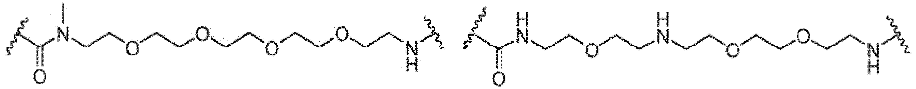

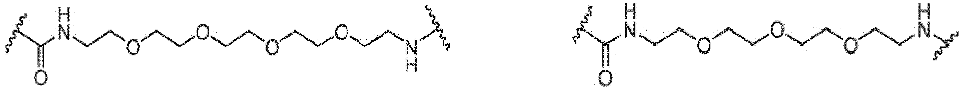

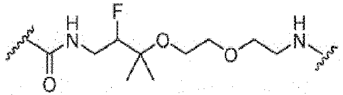

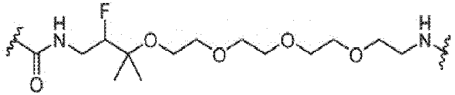

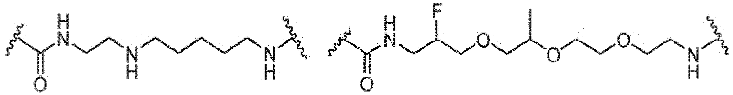

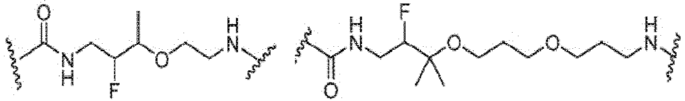

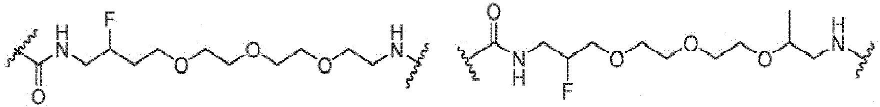

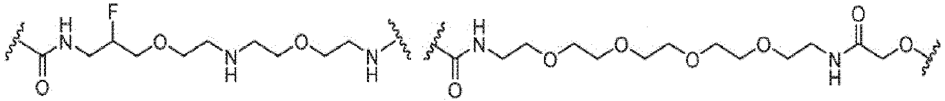

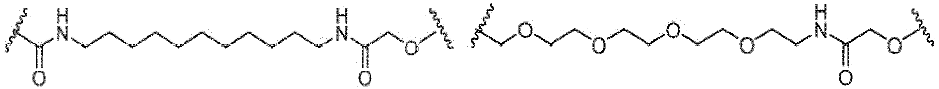

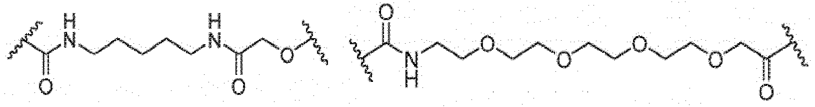

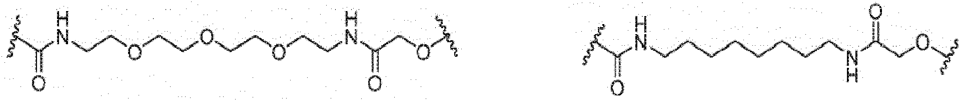

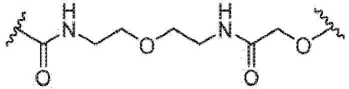

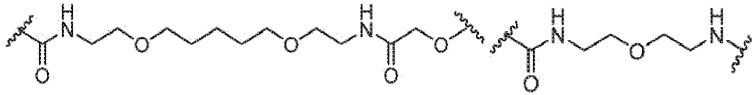

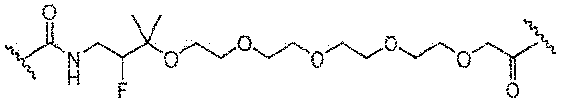

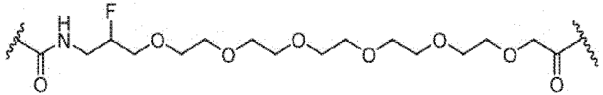

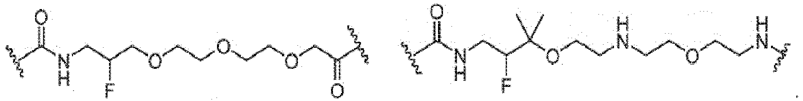

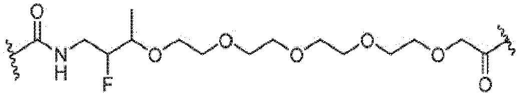

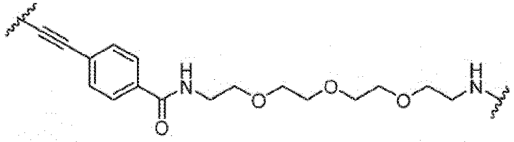

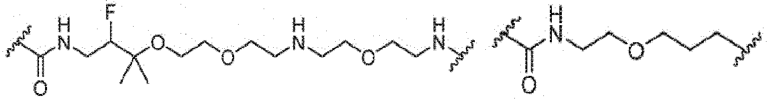

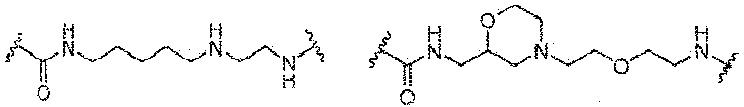

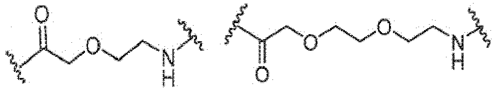

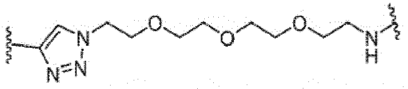

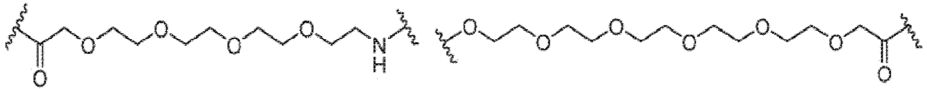

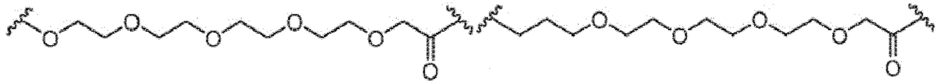

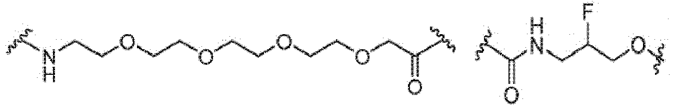

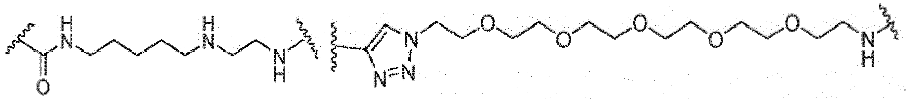

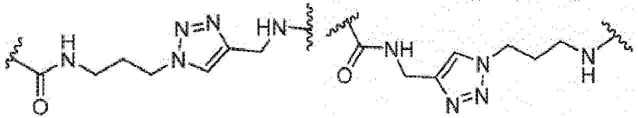

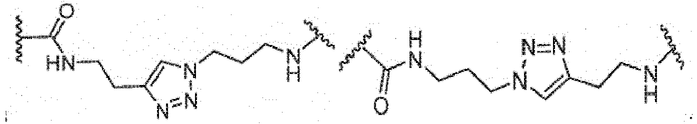

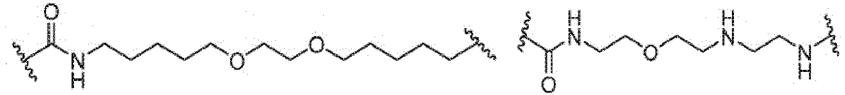

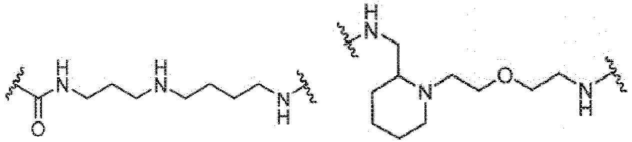

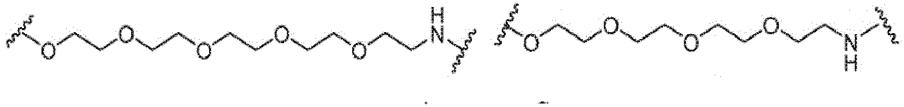

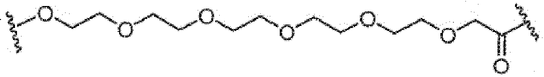

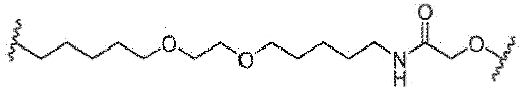

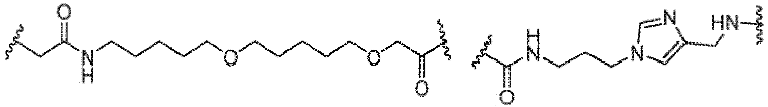

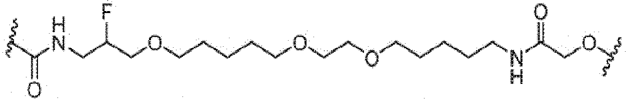

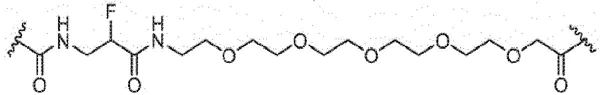

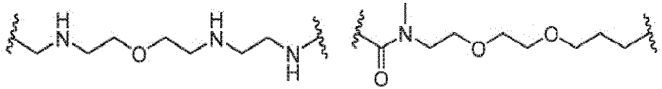

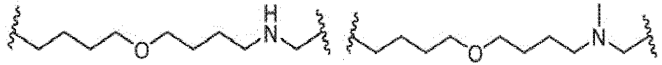

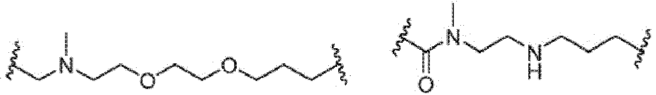

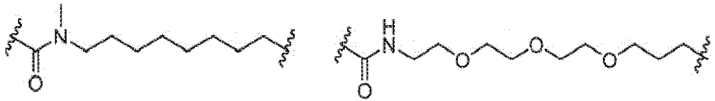

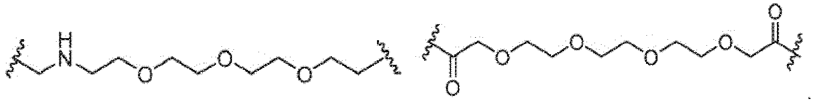

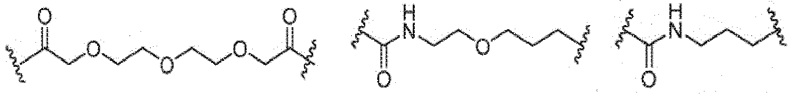

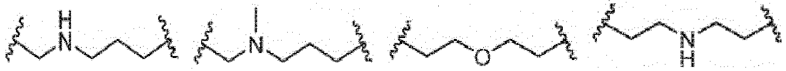

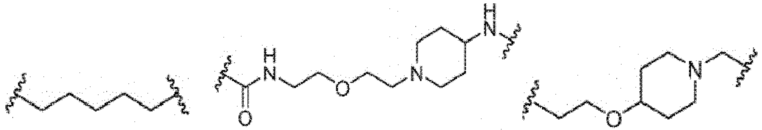

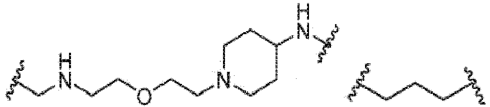

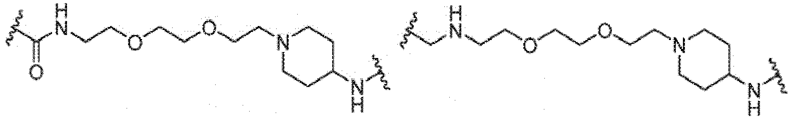

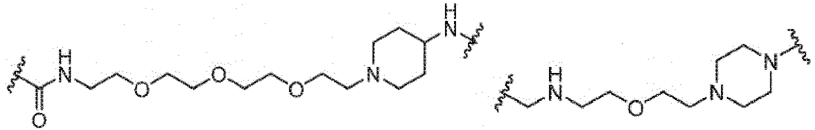

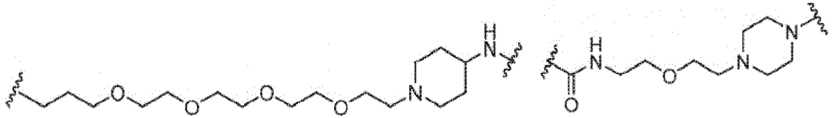

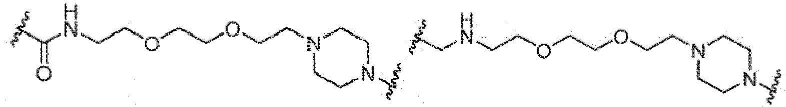

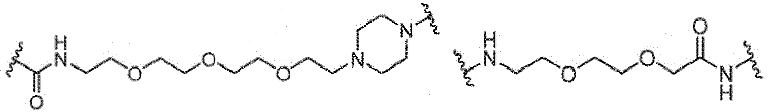

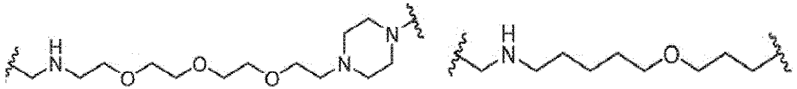

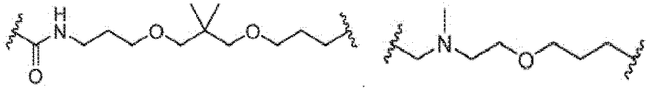

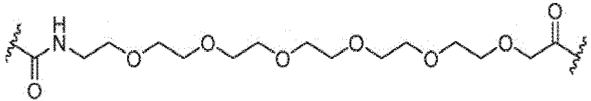

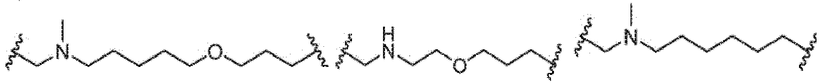

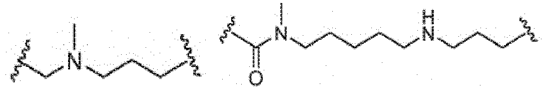

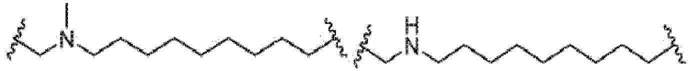

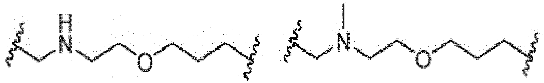

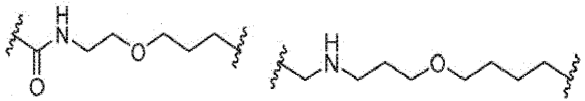

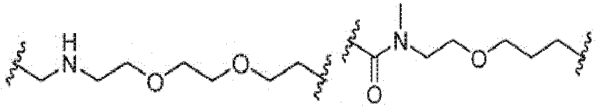

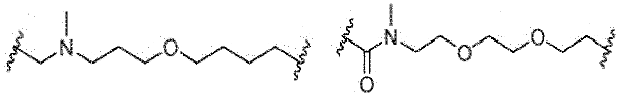

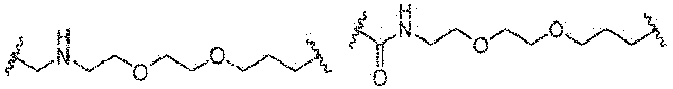

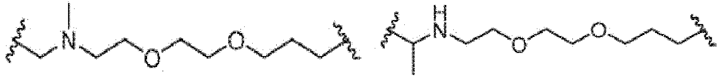

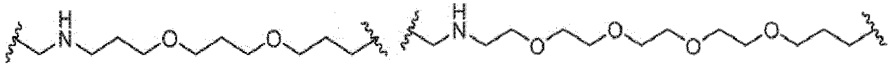

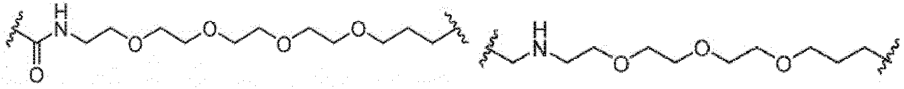

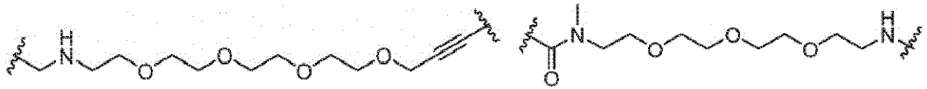

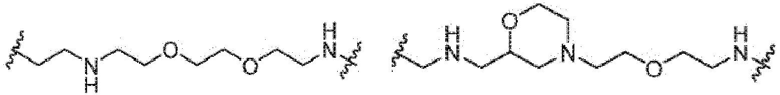

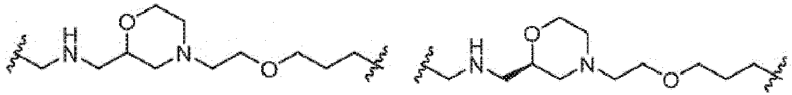

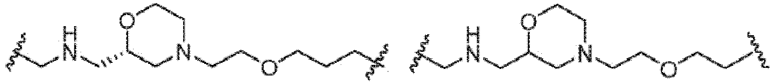

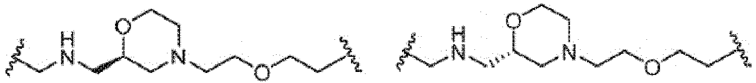

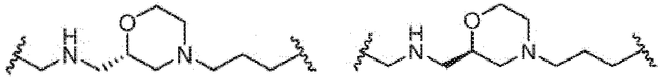

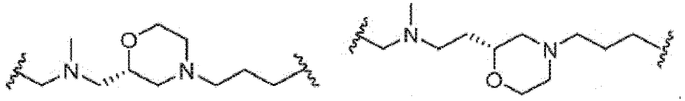

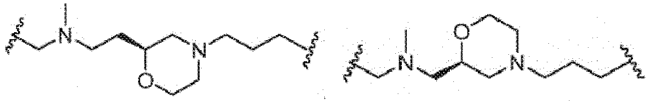

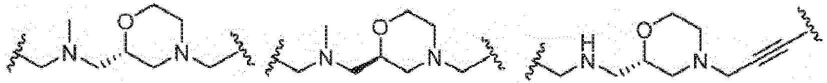

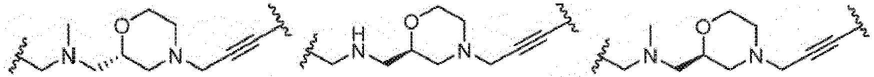

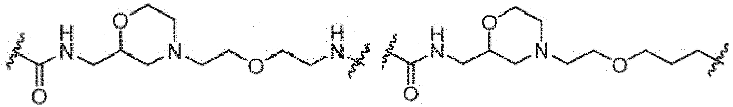

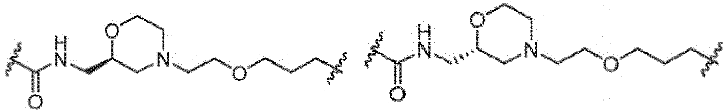

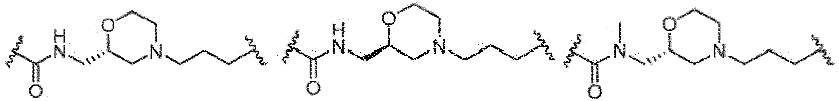

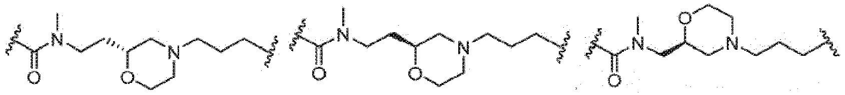

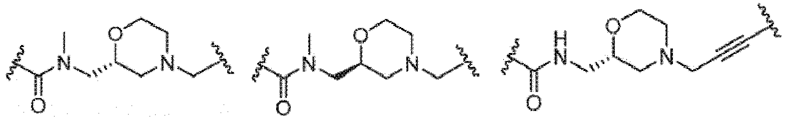

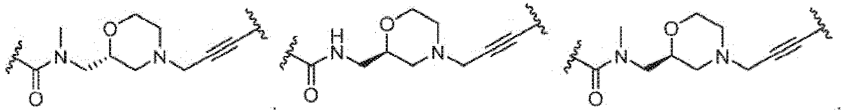

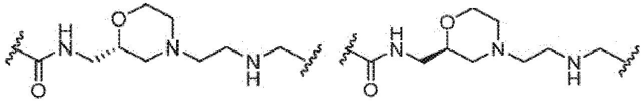

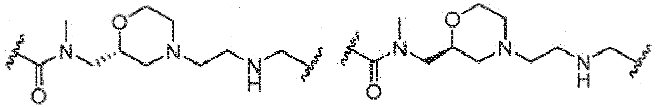

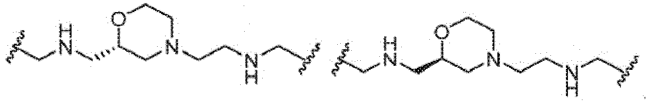

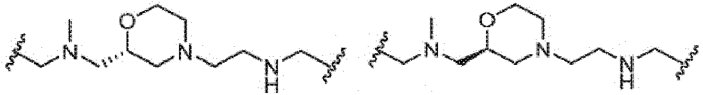

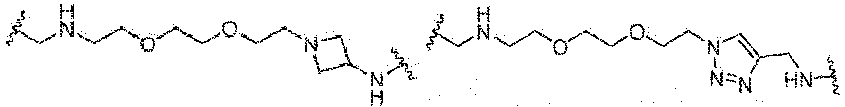

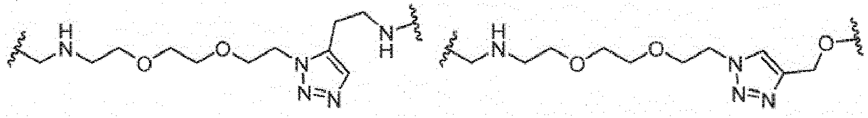

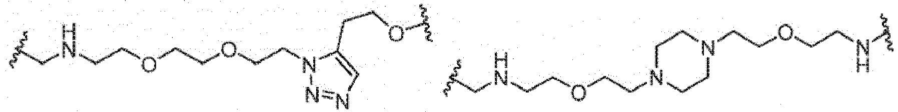

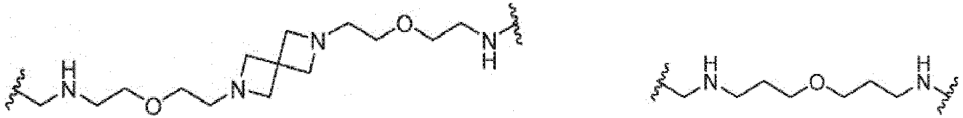

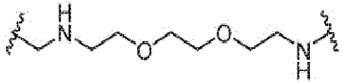

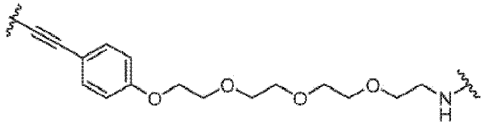

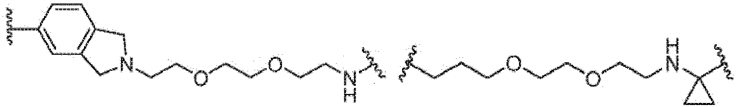

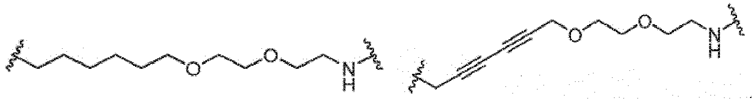

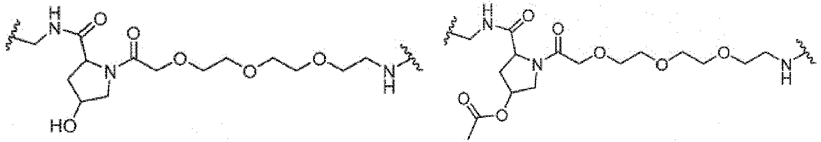

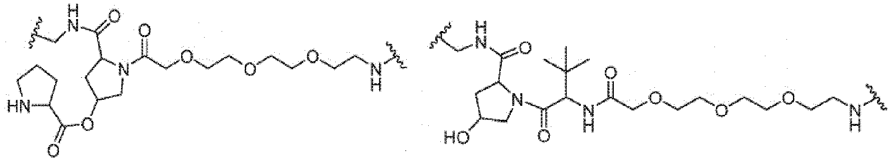

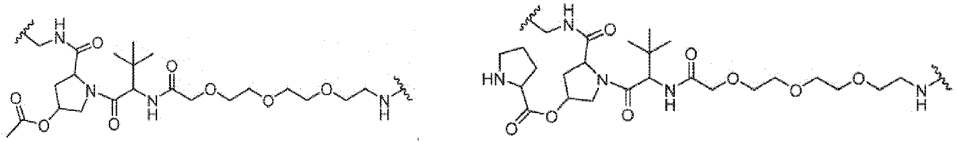

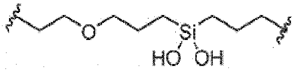

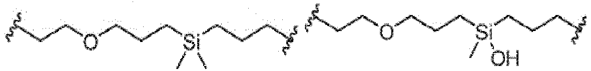

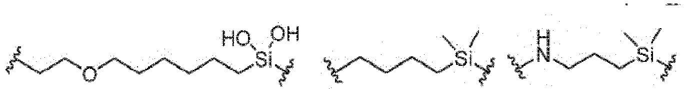

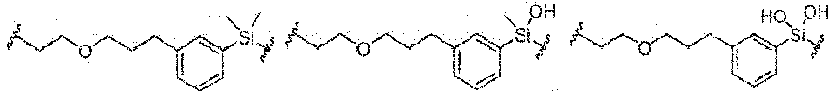

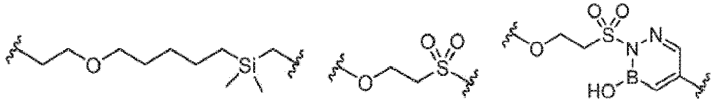

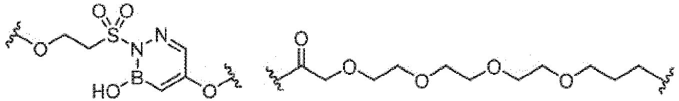

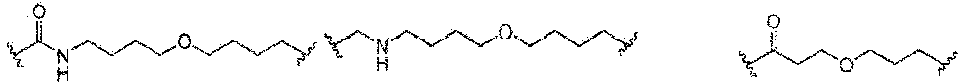

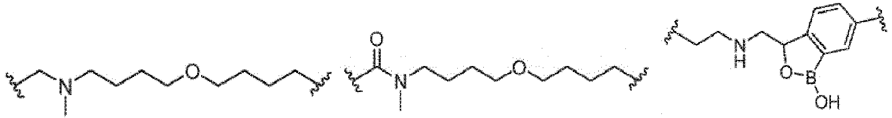

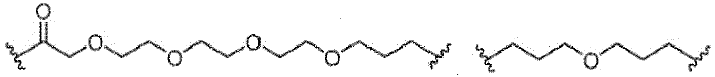

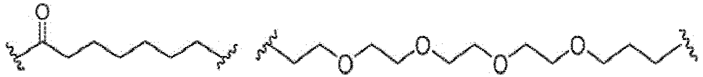

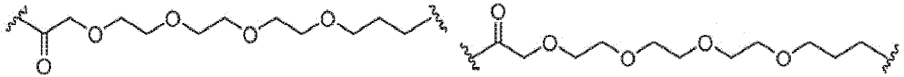

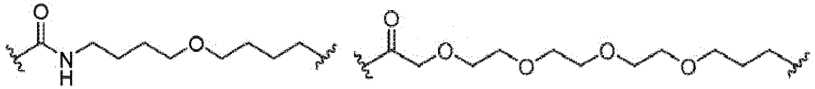

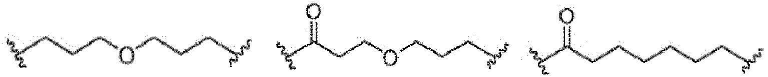

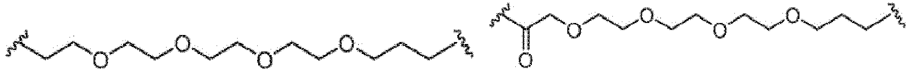

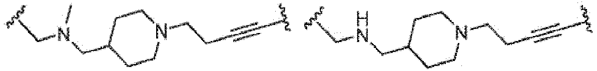

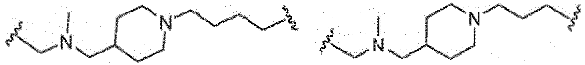

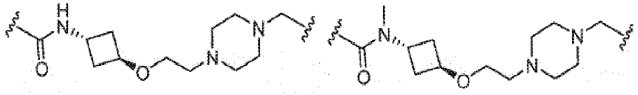

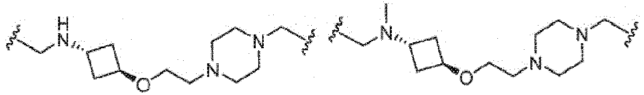

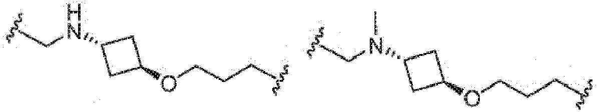

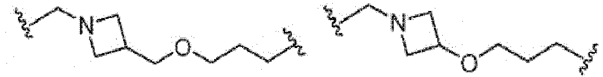

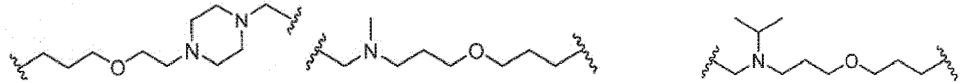

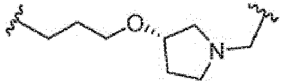

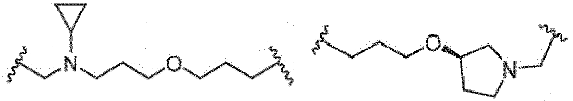

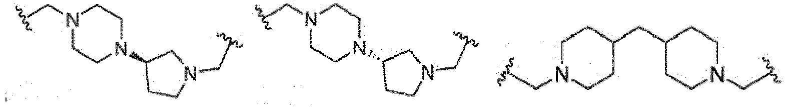

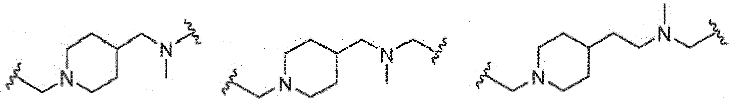

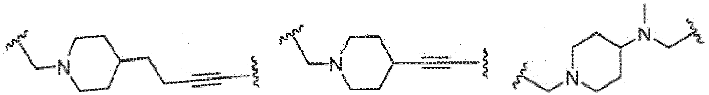

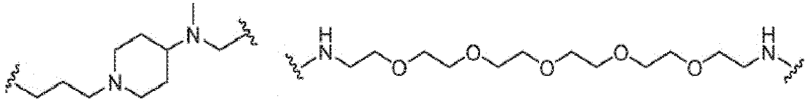

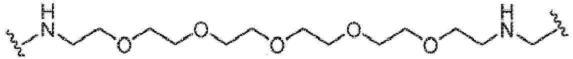

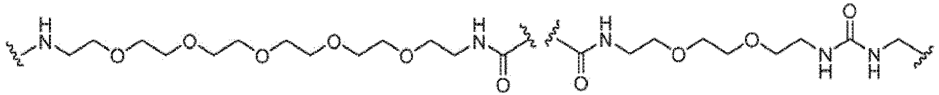

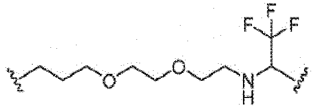

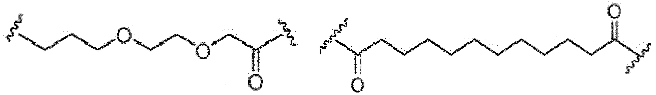

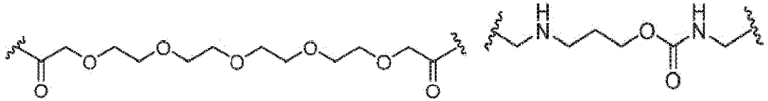

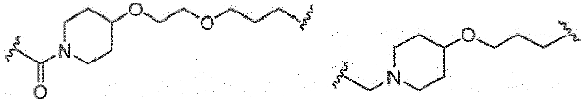

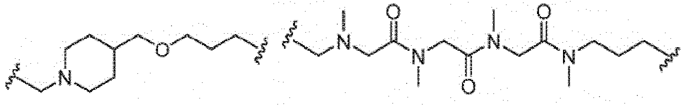

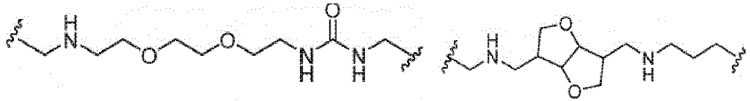

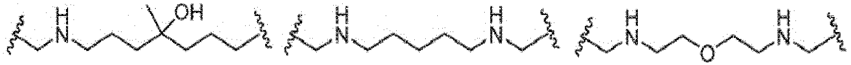

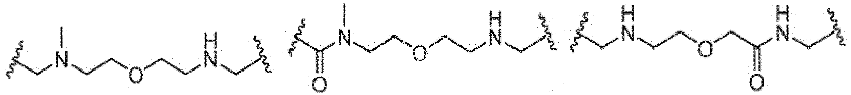

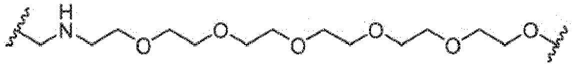

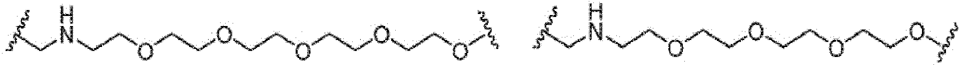

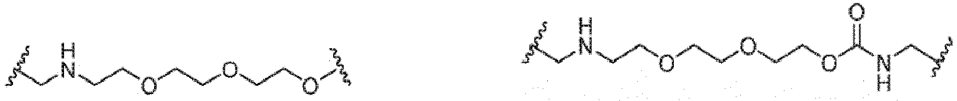

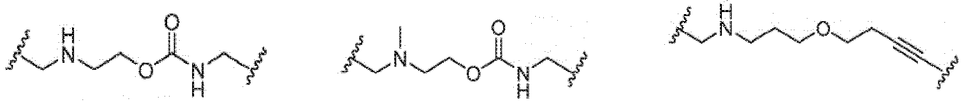

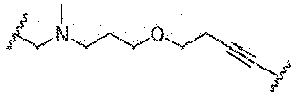

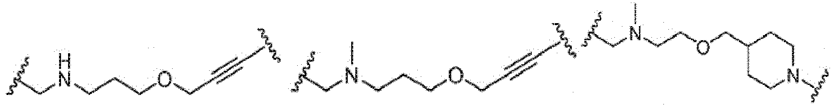

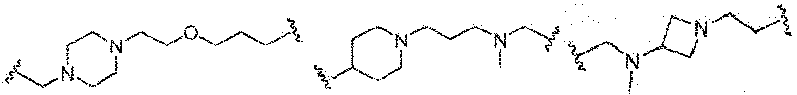

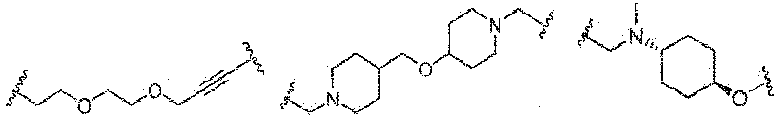

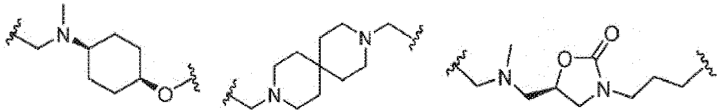

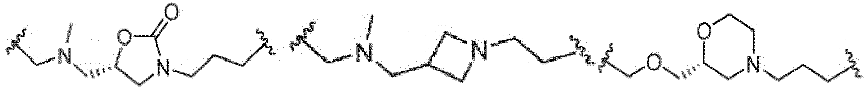

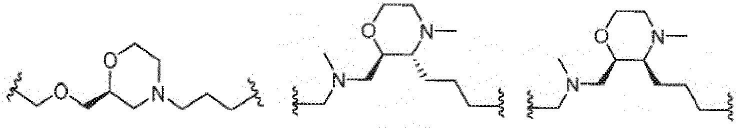

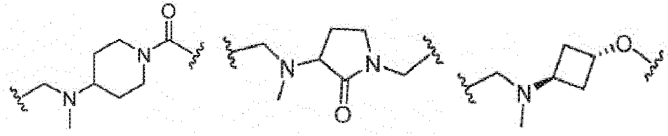

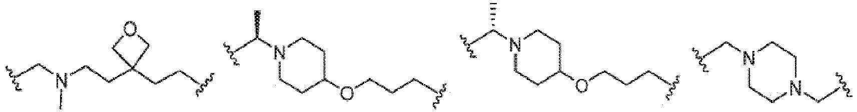

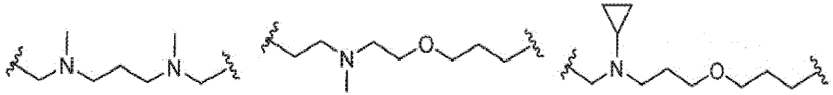

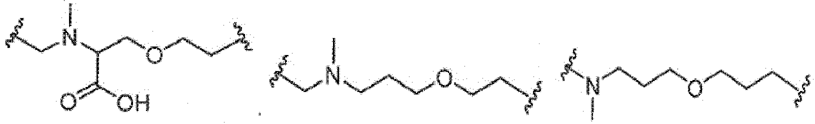

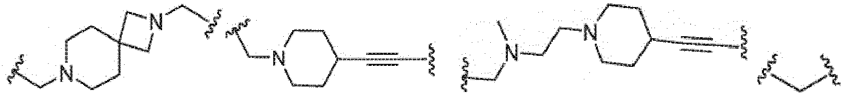

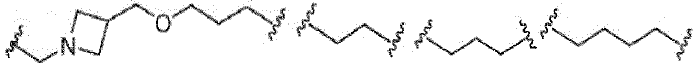

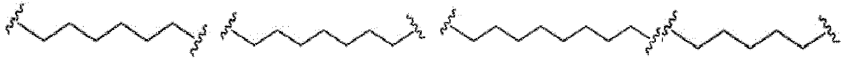

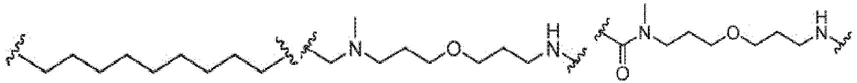

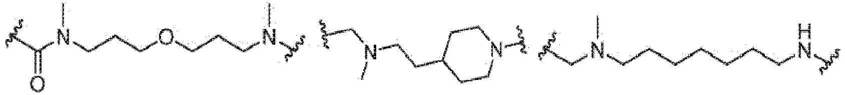

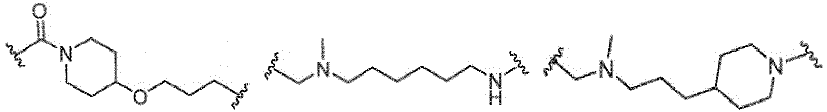

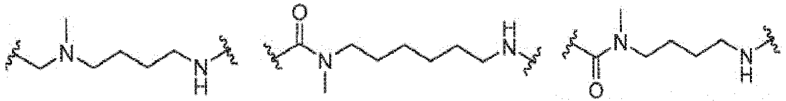

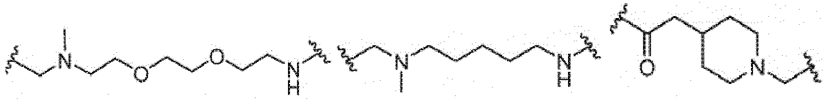

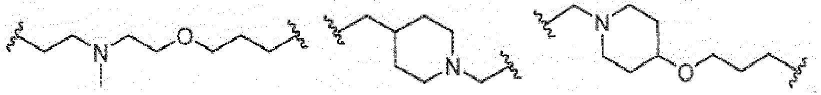

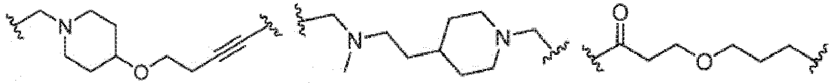

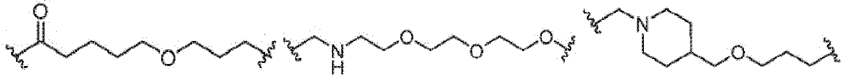

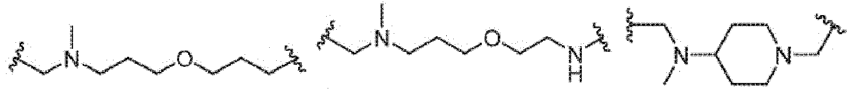

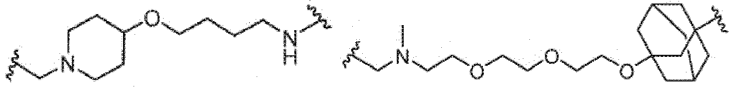

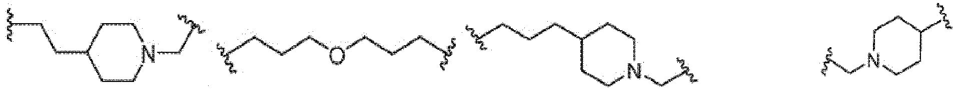

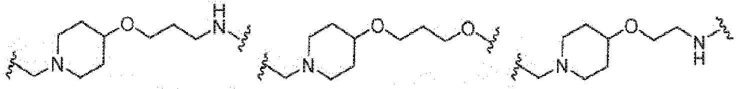

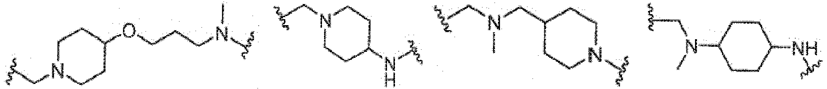

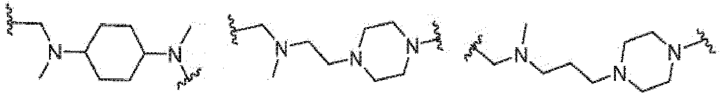

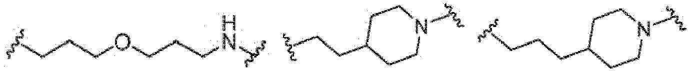

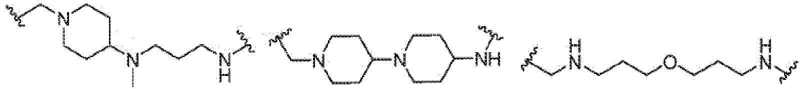

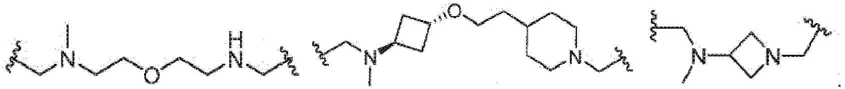

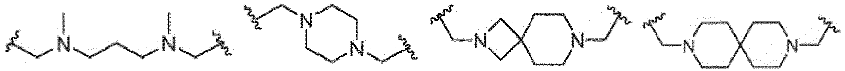

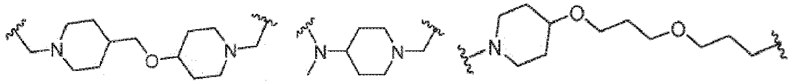

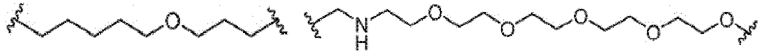

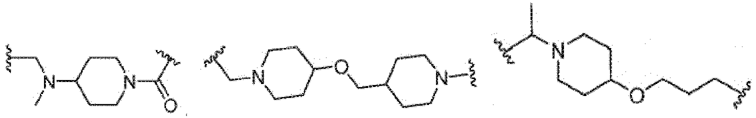

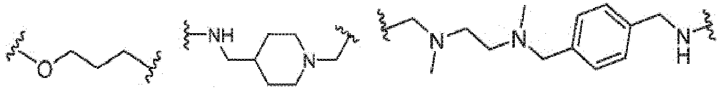

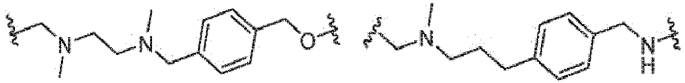

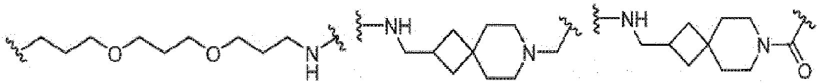

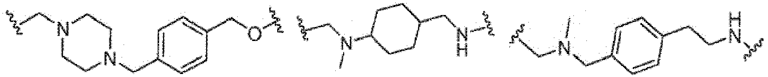

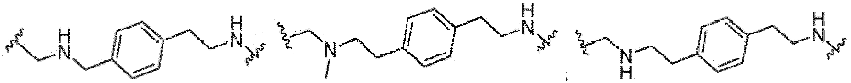

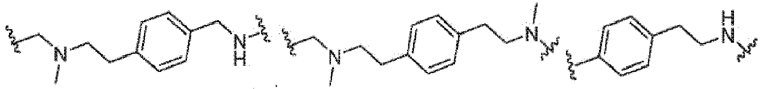

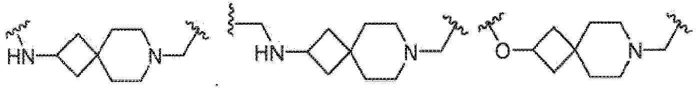

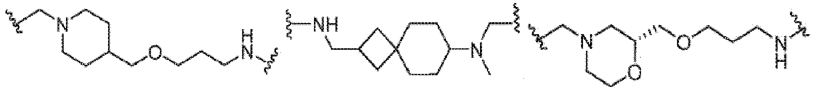

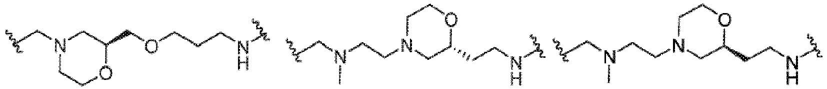

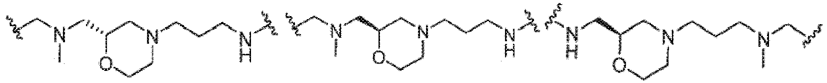

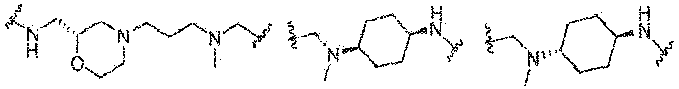

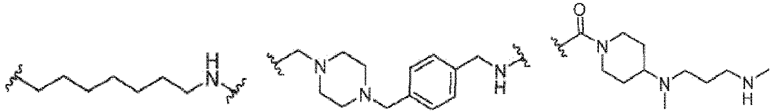

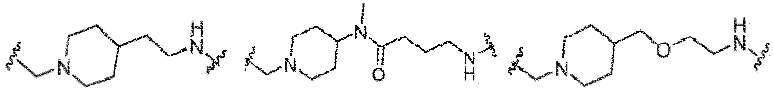

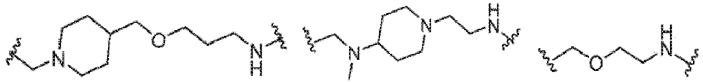

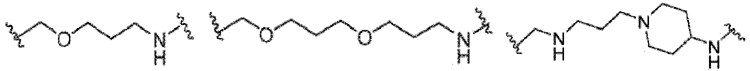

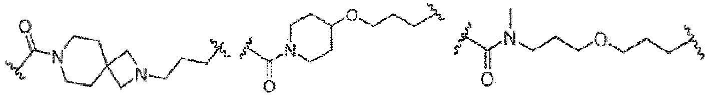

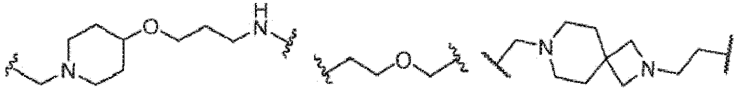

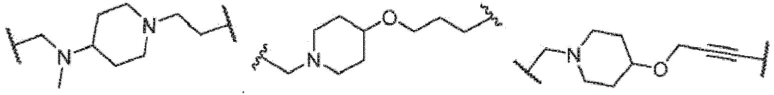

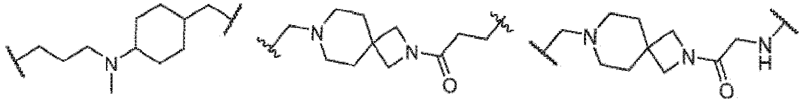

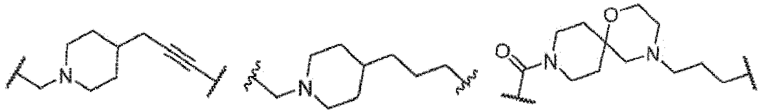

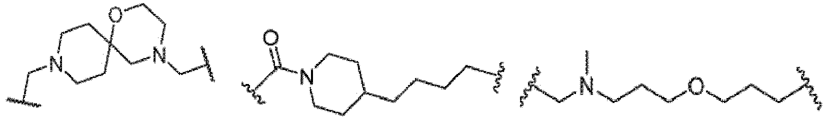

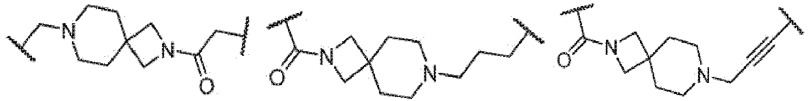

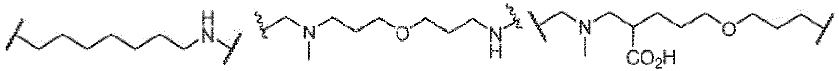

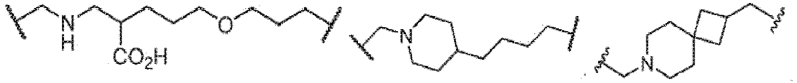

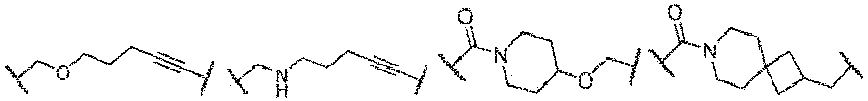

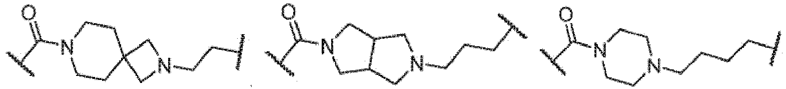

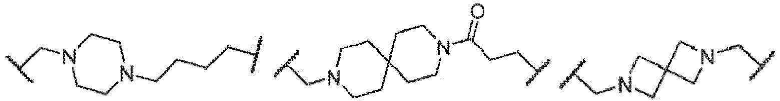

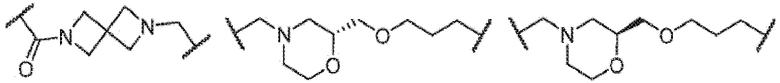

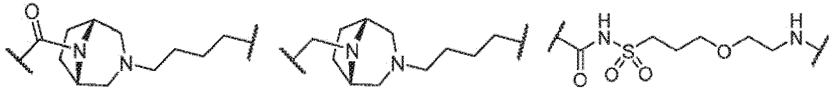

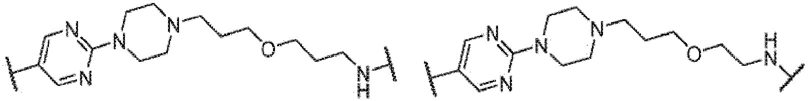

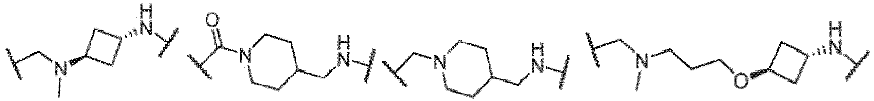

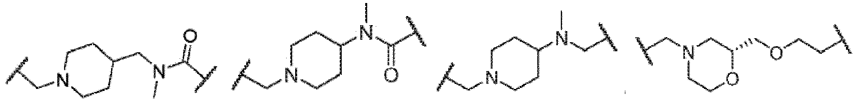

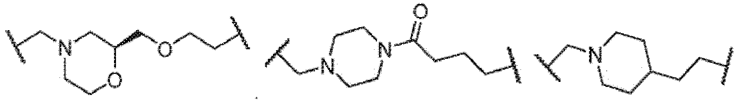

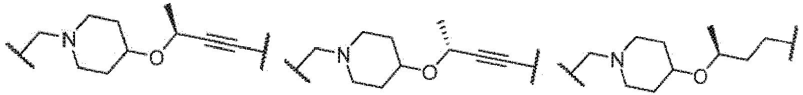

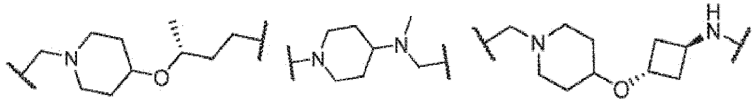

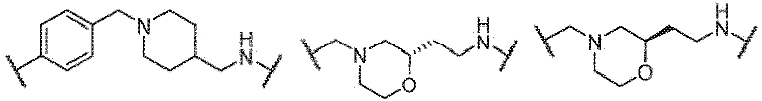

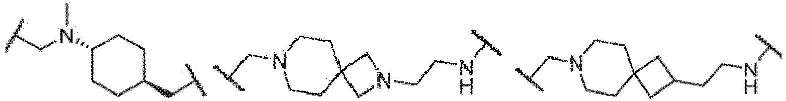

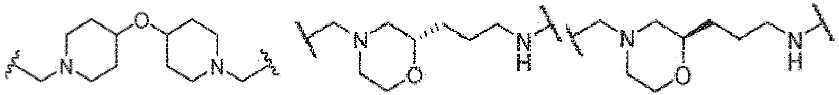

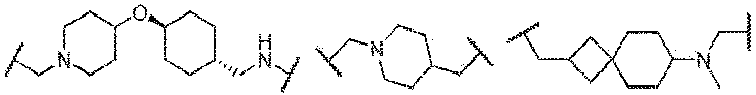

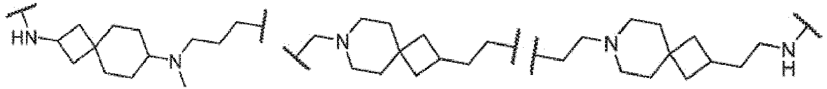

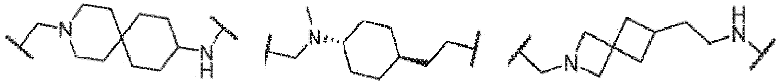

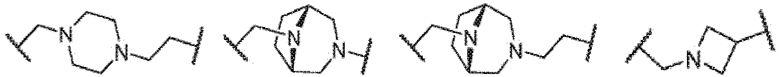

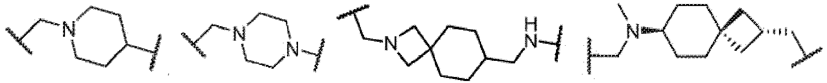

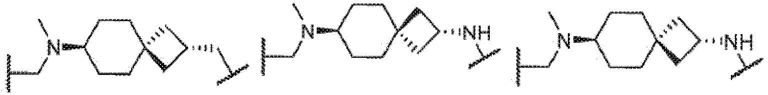

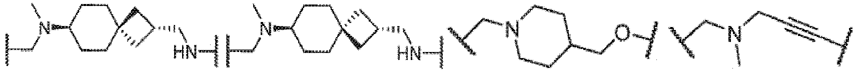

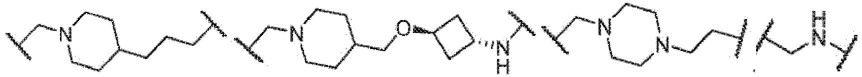

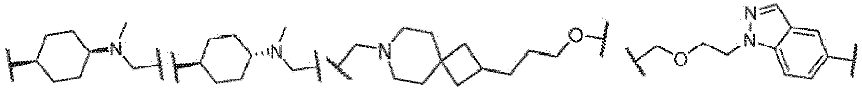

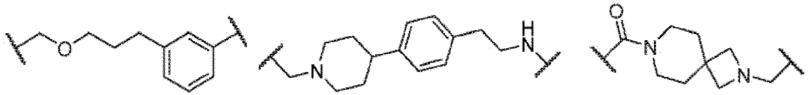

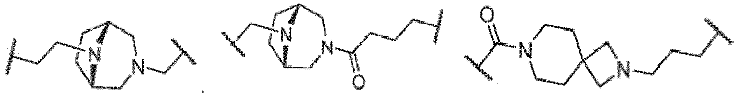

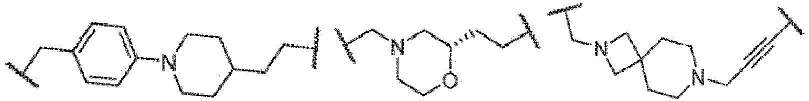

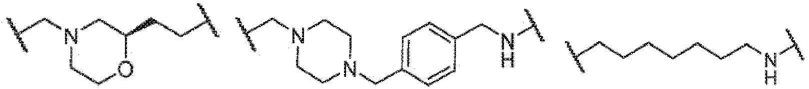

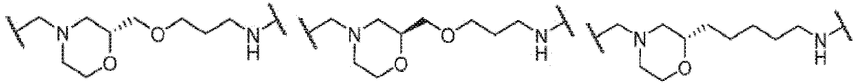

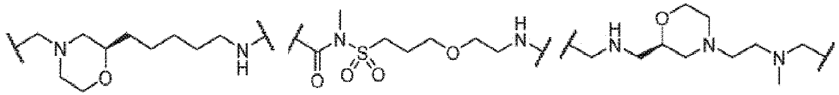

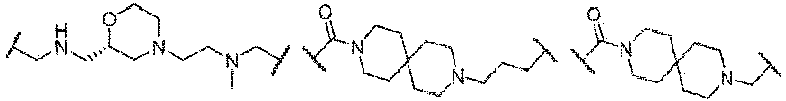

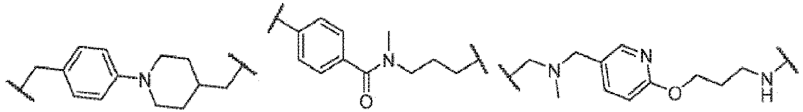

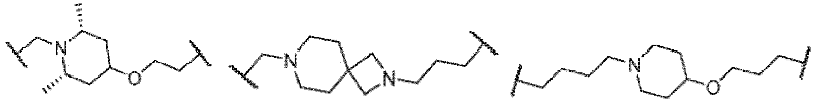

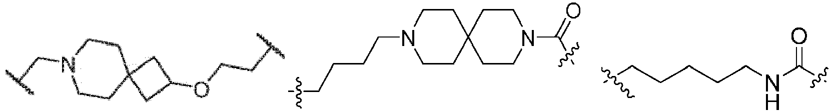

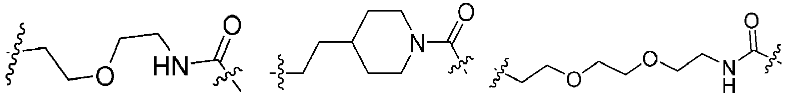

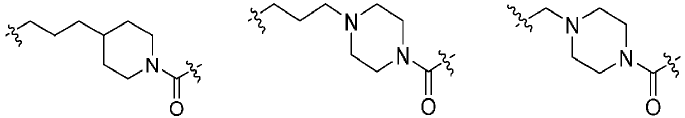

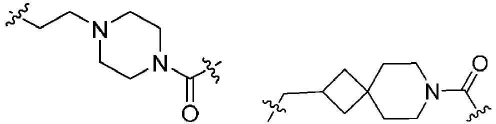

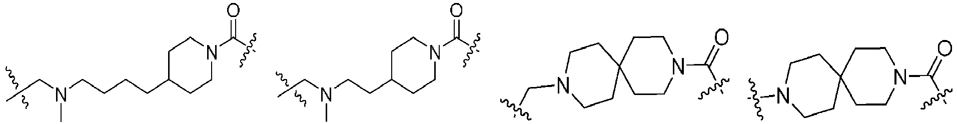

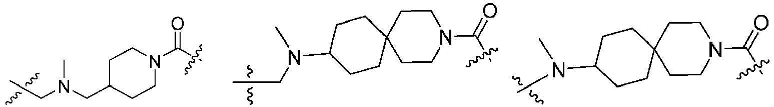

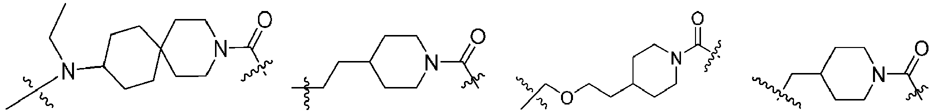

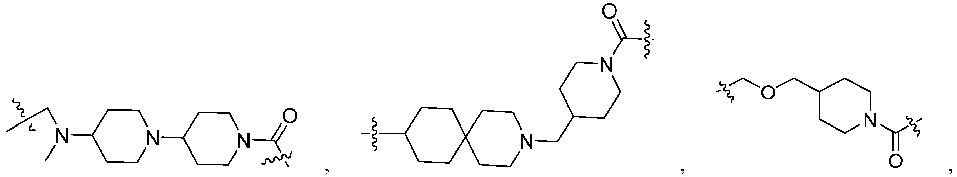

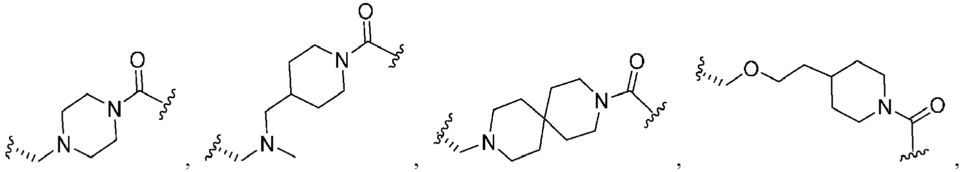

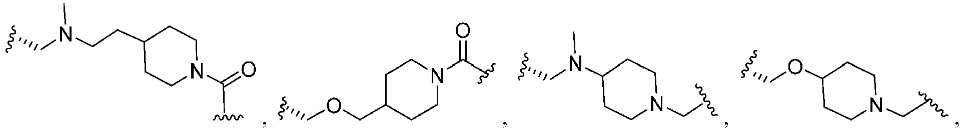

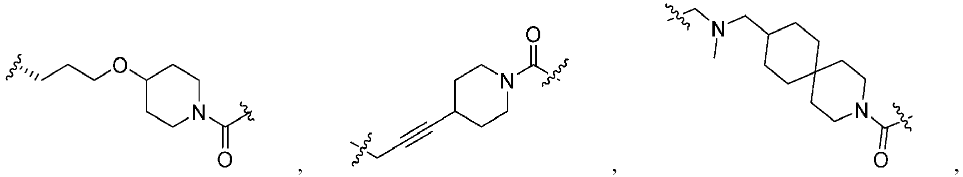

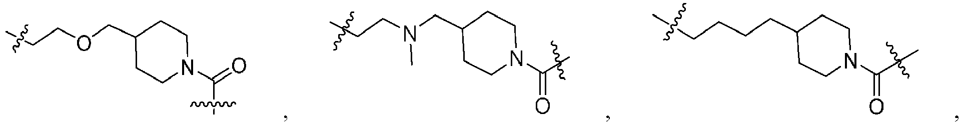

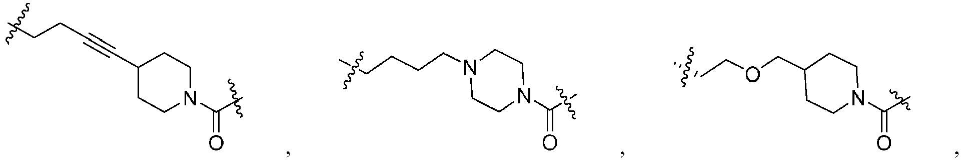

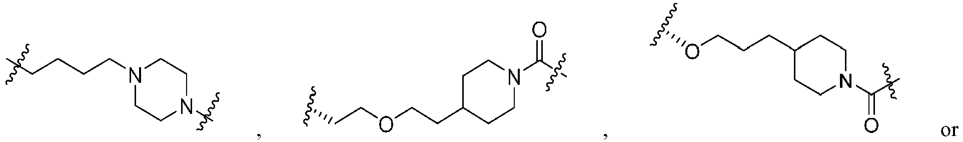

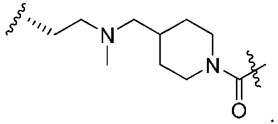

Preferably, in certain embodiments of the present invention, L is LA, 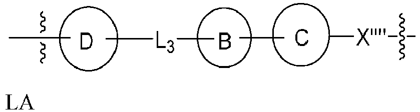 wherein in the LA:
ring D is absent or is C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from deuterium, halogen, oxo, cyano, amino, hydroxy, C₁-C₆ alkyl, C₁-C₆ haloalkyl, C₁-C₆ hydroxyalkyl or -O-(C₁-C₆ alkyl);
ring B is absent or is C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from deuterium, halogen, oxo, cyano, amino, hydroxy, C₁-C₆ alkyl, C₁-C₆ haloalkyl, C₁-C₆ hydroxyalkyl or -O-(C₁-C₆ alkyl);
ring C is absent, C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from deuterium, halogen, oxo, cyano, amino, hydroxy, C₁-C₆ alkyl, C₁-C₆ haloalkyl, C₁-C₆ hydroxyalkyl or -O-(C₁-C₆ alkyl);
Xʺʺ is a bond, -NH-, -NCH₃-, -O-, -C(CH₃)₂-, -S-, -C=C-, -C=C-, -CHF-, -CHCF₃-, -C(O)-, -S(O)-, -S(O)₂-, -C(O)O-, -OC(O)-, -C(O)NH-, -C(O)NCH₃-, -NHC(O)- or -NCH₃C(O)-;
L₃ is -(CH₂)ₖ-, wherein one or two methylene groups in the L₃ are optionally replaced by groups selected from deuterium, -O-, -NH-, -N(C₁-C₆ alkyl)-, -N(C₁-C₆ haloalkyl)-, -N(C₁-C₆ hydroxyalkyl)- or - N(C₃-C₈ cycloalkyl)-, and k is 0, 1, 2, 3, 4, 5, 6, 7, 8, 9 or 10.

Preferably, in certain embodiments of the present invention, L is LA, 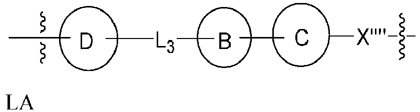 wherein in the LA:
ring D is absent or is C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
ring B is absent or is C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
ring C is absent, C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
Xʺʺ is a bond, -NH-, -NCH₃-, -O-, -C(CH₃)₂-, -S-, -C=C-, -C≡C-, -CHF-, -CHCF₃-, -C(O)-, -S(O)-, -S(O)₂-, -C(O)O-, -OC(O)-, -C(O)NH-, -C(O)NCH₃-, -NHC(O)- or -NCH₃C(O)-;
L₃ is -(CH₂)ₖ-, wherein one or two methylene groups in the L₃ are optionally replaced by groups selected from -O-, -NH-, -N(C₁-C₆ alkyl)-, -N(C₁-C₆ haloalkyl)-, -N(C₁-C₆ hydroxyalkyl)- or -N(C₃-C₈ cycloalkyl)-, and k is 0, 1, 2, 3, 4, 5, 6, 7, 8, 9 or 10.

Preferably, in certain embodiments of the present invention, L is LA, 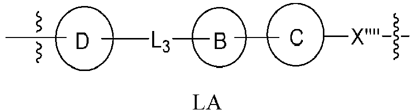 wherein in the LA:
ring D is absent, 6-10 membered aryl or 5-10 membered heteroaryl or is C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
ring B is absent or is C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
ring C is absent, C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
Xʺʺ is a bond, -NH-, -NCH₃-, -O-, -C(CH₃)₂-, -S-, -C=C-, -C≡C-, -CHF-, -CHCF₃-, -C(O)-, -S(O)-, -S(O)₂-, -C(O)O-, -OC(O)-, -C(O)NH-, -C(O)NCH₃-, -NHC(O)-, -(CH₂)ₜ- or -NCH₃C(O)-; one or more methylene groups in the -(CH₂)ₜ- are optionally replaced by groups selected from -O-, -NH-, -C≡C-, -N(C₁-C₆ alkyl)- and -C(O)-, and t is 0, 1, 2, 3, 4, 5, 6 or 7.

L₃ is -(CH₂)ₖ-, wherein one or two methylene groups in the L₃ are optionally replaced by groups selected from -O-, -NH-, -N(C₁-C₆ alkyl)-, -N(C₁-C₆ haloalkyl)-, -N(C₁-C₆ hydroxyalkyl)-, -C≡C-, -C(O)- or -N(C₃-C₈ cycloalkyl)-, and k is 0, 1, 2, 3, 4, 5, 6, 7, 8, 9 or 10. Preferably, in certain embodiments of the present invention, when ring A" is 5-11 membered bicyclic heteroaryl containing 1, 2 or 3 heteroatoms selected from N, O or S, ring D is 6-10 membered aryl, 5-10 membered heteroaryl, C₃-C₁₂ cycloalkylene or 3-12 membered heterocycloalkylene containing 1-2 heteroatoms selected from N, O or S, wherein the cycloalkylene and heterocycloalkylene are substituted with substituents selected from halogen, oxo, cyano, amino, hydroxy, C₁-C₆ alkyl, C₁-C₆ haloalkyl, C₁-C₆ hydroxyalkyl and -O-(C₁-C₆ alkyl).

Preferably, in certain embodiments of the present invention, when ring A" is 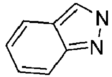 , the ring D is C₃-C₁₂ cycloalkylene or is 3-12 membered heterocycloalkylene containing 1-2 heteroatoms selected from N, O or S, wherein the cycloalkylene and heterocycloalkylene are independently substituted with 1, 2 or 3 hydroxy groups or with 1, 2 or 3 -O-(C₁-C₆ alkyl) groups.

In certain embodiments of the present invention, the L is LA, 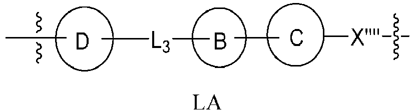 wherein the LA is scheme 1, scheme 2, scheme 3 or scheme 4;
scheme 1:
   ring D is C₃-C₁₂ cycloalkylene or is 3-12 membered heterocycloalkylene containing 1-2 N heteroatoms, wherein the cycloalkylene and heterocycloalkylene are optionally substituted with substituents selected from halogen, -O-(C₁-C₆ alkyl) or hydroxy;
   ring B is C₃-C₁₂ cycloalkylene or is 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
   ring C is absent;
   Xʺʺ is -C(O)-;
   L₃ is -(CH₂)ₖ-, wherein one or two methylene groups in the L₃ are optionally replaced by groups selected from -O-, -NH-, -N(C₁-C₆ alkyl)-, -N(C₁-C₆ haloalkyl)-, -C(O)-, -N(C₁-C₆ hydroxyalkyl)- or -N(C₃-C₈ cycloalkyl)-, and k is 0, 1, 2, 3, 4, 5, 6, 7, 8, 9 or 10;
scheme 2:
   ring D is C₃-C₁₂ cycloalkylene or is 3-12 membered heterocycloalkylene containing 1-2 N heteroatoms, wherein the cycloalkylene and heterocycloalkylene are optionally substituted with substituents selected from halogen, methoxy or hydroxy;
   ring B is 7-11 membered spiroheterocycloalkylene or fused heterocycloalkylene containing 1 or 2 nitrogen heteroatoms;
   ring C is absent;
   Xʺʺ is -C(O)-;
   L₃ is -(CH₂)ₖ-, wherein k is 1, 2, 3, 4, 5, 6, 7, 8, 9 or 10;
scheme 3:
   ring D is C₃-C₁₂ cycloalkylene or is 3-12 membered heterocycloalkylene containing 1-2 N heteroatoms, wherein the cycloalkylene and heterocycloalkylene are optionally substituted with substituents selected from halogen, hydroxy or methoxy;
   ring B is 3-12 membered heterocycloalkylene containing 1-2 heteroatoms selected from N, O or S;
   ring C is absent;
   Xʺʺ is -C(O)-;
   L₃ is -(CH₂)ₖ-, wherein k is 1, 2, 3 or 4;
scheme 4:
   ring D is cyclohexylene or is 6-membered heterocycloalkylene containing 1-2 N heteroatoms, wherein the cyclohexylene and 6-membered heterocycloalkylene are optionally substituted with substituents selected from halogen, hydroxy or methoxy;
   ring B is 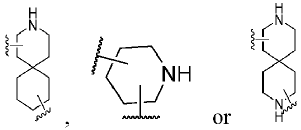 (e.g., 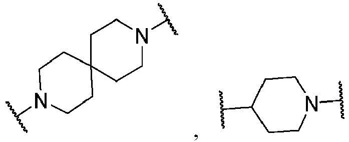 or 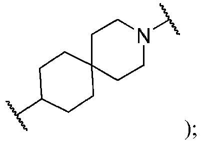
   ring C is absent;
   Xʺʺ is -C(O)-;
   L₃ is -(CH₂)ₖ-, wherein one or two methylene groups in the L₃ are replaced by groups selected from -O-, -NH-, -N(C₁-C₆ alkyl)-, -N(C₁-C₆ haloalkyl)-, -C(O)-, -N(C₁-C₆ hydroxyalkyl)- or -N(C₃-C₈ cycloalkyl)-, and k is 1, 2, 3 or 4.

Preferably, in certain embodiments of the present invention, in the LA, ring D is absent.

Preferably, in certain embodiments of the present invention, in the LA, ring D is 4-7 membered saturated monocyclic heterocycloalkylene containing 1 or 2 nitrogen heteroatoms, or 7-11 membered spiroheterocycloalkylene or fused heterocycloalkylene containing 1 or 2 nitrogen heteroatoms.

Preferably, in certain embodiments of the present invention, in the LA, ring D is 6-10 membered aryl, 4-7 membered saturated monocyclic heterocycloalkylene containing 1 or 2 nitrogen heteroatoms, or 7-11 membered spiroheterocycloalkylene or fused heterocycloalkylene containing 1 or 2 nitrogen heteroatoms.

Preferably, in certain embodiments of the present invention, in the LA, ring D is 4-7 membered saturated monocyclic heterocycloalkylene containing 1 or 2 nitrogen heteroatoms, or 7-11 membered spiroheterocycloalkylene or fused heterocycloalkylene containing 1 or 2 nitrogen heteroatoms.

Preferably, in certain embodiments of the present invention, in the LA, ring D is 7-11 membered spiroheterocycloalkylene containing 1 or 1 nitrogen heteroatom.

Preferably, in certain embodiments of the present invention, in the LA, ring D is 7-11 membered spiroheterocycloalkylene containing 1 or 2 nitrogen heteroatoms.

Preferably, in certain embodiments of the present invention, in the LA, ring D is

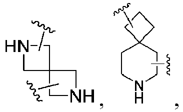

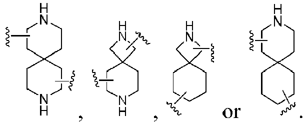

Preferably, in certain embodiments of the present invention, in the LA, ring B is absent.

Preferably, in certain embodiments of the present invention, in the LA, ring B is 4-7 membered saturated monocyclic heterocycloalkylene containing 1 or 2 nitrogen heteroatoms, or 7-11 membered spiroheterocycloalkylene or fused heterocycloalkylene containing 1 or 2 nitrogen heteroatoms.

Preferably, in certain embodiments of the present invention, in the LA, ring B is 4-7 membered saturated monocyclic heterocycloalkylene containing 1 or 2 nitrogen heteroatoms.

Preferably, in certain embodiments of the present invention, in the LA, ring B is piperidinylidene, piperazinylidene,

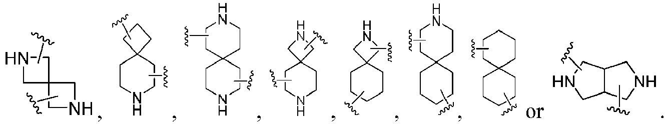

Preferably, in certain embodiments of the present invention, ring C is 4-11 membered saturated or partially unsaturated spirocycloalkylene, 4-11 membered saturated or partially unsaturated fused cycloalkylene, 8-10 membered bicyclic saturated or partially unsaturated cycloalkylene, 4-7 membered saturated or partially unsaturated heterocycloalkylene having 1-2 heteroatoms independently selected from nitrogen, oxygen or sulfur, 4-11 membered saturated or partially unsaturated spiroheterocycloalkylene having 1-2 heteroatoms independently selected from nitrogen, oxygen or sulfur, 4-11 membered saturated and/or partially unsaturated fused heterocycloalkylene having 1-2 heteroatoms independently selected from nitrogen, oxygen or sulfur, 8-10 membered bicyclic saturated and/or partially unsaturated heterocycloalkylene having 1-2 heteroatoms independently selected from nitrogen, oxygen or sulfur, wherein the cycloalkyl and heterocycloalkyl are optionally substituted with substituents selected from deuterium, halogen, oxo, cyano, amino, hydroxy, C₁-C₆ alkyl, C₁-C₆ haloalkyl, C₁-C₆ hydroxyalkyl and -O-(C₁-C₆ alkyl).

Preferably, in certain embodiments of the present invention, in the LA, ring C is 3-7 membered saturated or partially unsaturated cycloalkylene, 4-11 membered saturated or partially unsaturated spirocycloalkylene, 4-11 membered saturated or partially unsaturated fused cycloalkylene, 8-10 membered bicyclic saturated or partially unsaturated cycloalkylene, 4-7 membered saturated or partially unsaturated heterocycloalkylene having 1-2 heteroatoms independently selected from nitrogen, oxygen or sulfur, 4-11 membered saturated or partially unsaturated spiroheterocycloalkylene having 1-2 heteroatoms independently selected from nitrogen, oxygen or sulfur, 4-11 membered saturated and/or partially unsaturated fused heterocycloalkylene having 1-2 heteroatoms independently selected from nitrogen, oxygen or sulfur, or 8-10 membered bicyclic saturated and/or partially unsaturated heterocycloalkylene having 1-2 heteroatoms independently selected from nitrogen, oxygen or sulfur.

Preferably, in certain embodiments of the present invention, in the LA, ring C is 4-7 membered saturated monocyclic heterocycloalkylene containing 1 or 2 nitrogen heteroatoms, or 7-11 membered spiroheterocycloalkylene or fused heterocycloalkylene containing 1 or 2 nitrogen heteroatoms.

Preferably, in certain embodiments of the present invention, in the LA, ring C is 7-11 membered spiroheterocycloalkylene or fused heterocycloalkylene containing 1 or 2 nitrogen heteroatoms.

Preferably, in certain embodiments of the present invention, in the LA, ring C is

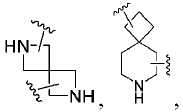

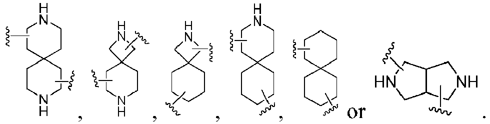

Preferably, in certain embodiments of the present invention, in the LA, ring C is

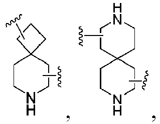

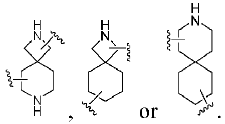

Preferably, in certain embodiments of the present invention, in the LA, Xʺʺ is a bond, -CH₂- or - C(O)-.

Preferably, in certain embodiments of the present invention, in the LA, Xʺʺ is a bond or -C(O)-.

Preferably, in certain embodiments of the present invention, in the LA, Xʺʺ is -C(O)-.

Preferably, in certain embodiments of the present invention, in the LA, L₃ is -(CH₂)ₖ-, wherein one methylene group in the L₃ is optionally replaced by a group selected from -O-, -NH-, -NCH₃- or -NCH₂CH₃-; k is 1, 2, 3 or 4.

Preferably, in certain embodiments of the present invention, in the LA, L₃ is -(CH₂)ₖ-, wherein one or two methylene groups in the L₃ are optionally replaced by groups selected from deuterium-C(O)-, -O-, - NH-, -NCH₃- or -NCH₂CH₃-; k is 1, 2, 3 or 4.

Preferably, in certain embodiments of the present invention, in the LA, ring D and ring B are absent, and ring C is 7-11 membered spiroheterocycloalkylene containing 1 or 2 nitrogen heteroatoms; Xʺʺ is -C(O)-; L₃ is -(CH₂)ₖ-; k is 1, 2, 3, 4 or 5.

Preferably, in certain embodiments of the present invention, formula L is selected from

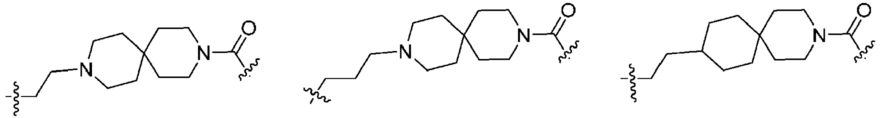

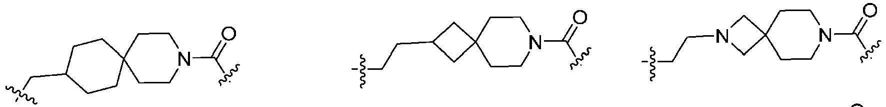

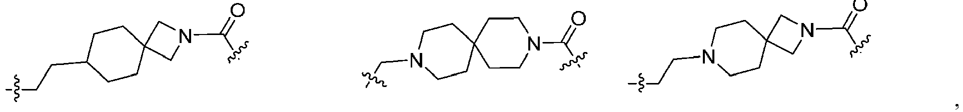

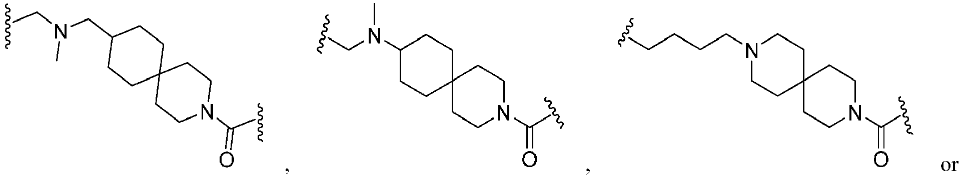

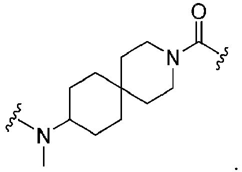

Preferably, in certain embodiments of the present invention, in the LA, ring D and ring B are absent, and ring C is 4-7 membered heterocycloalkyl containing 1 or 2 nitrogen heteroatoms; X"" is -C(O)-, a bond or -CH₂-; L₃ is -(CH₂)ₖ-; k is 1, 2, 3, 4 or 5.

Preferably, in certain embodiments of the present invention, formula L is selected from

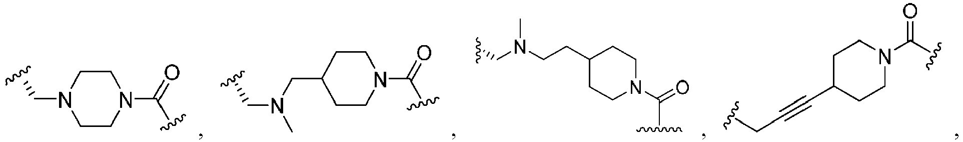

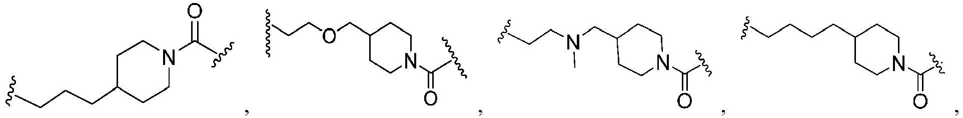

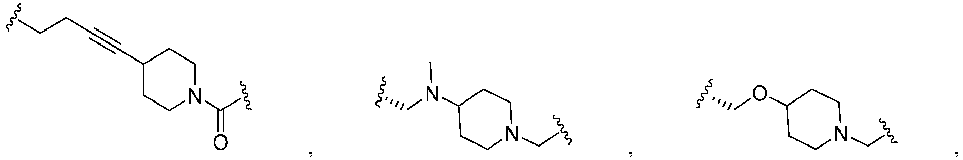

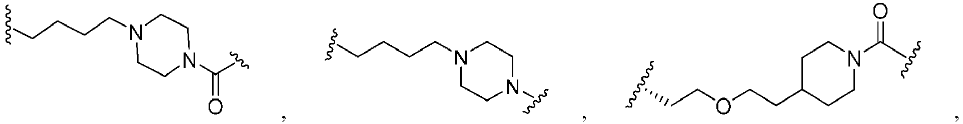

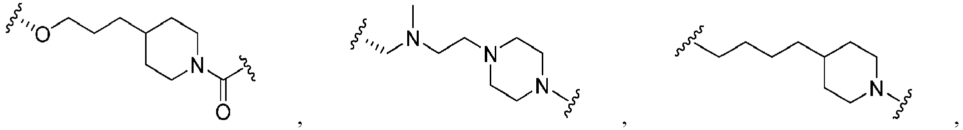

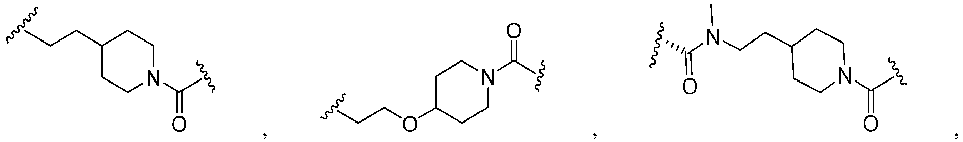

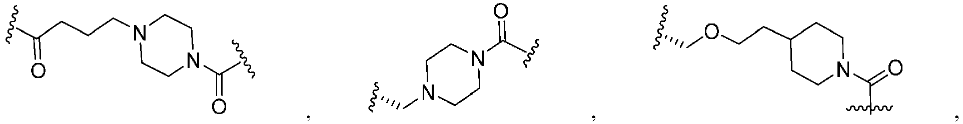

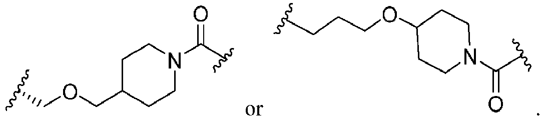

Preferably, in certain embodiments of the present invention, in the LA, ring D is C₄-C₇ membered cycloalkyl or 4-7 membered heterocycloalkyl; ring B and ring C are absent or are 5-11 membered heterocycloalkylene containing 1 or 2 nitrogen heteroatoms; Xʺʺ is -C(O)- or -(CH2)ₜ-, t is 0, 1, 2, 3, 4 or 5; L₃ is -(CH₂)ₖ-, wherein k is 1, 2, 3, 4 or 5.

Preferably, in certain embodiments of the present invention, formula L is selected from 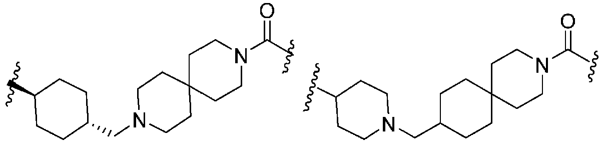 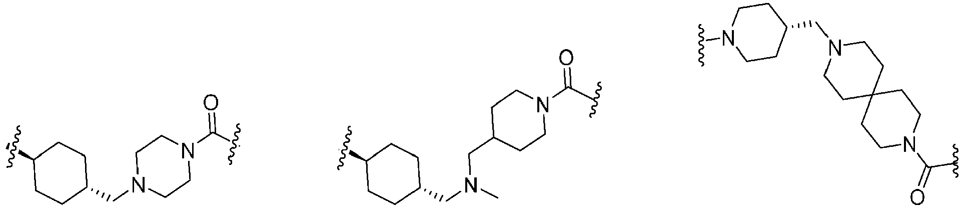 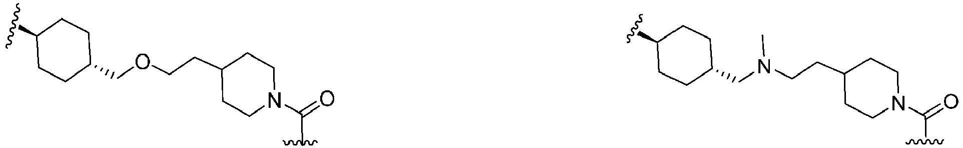 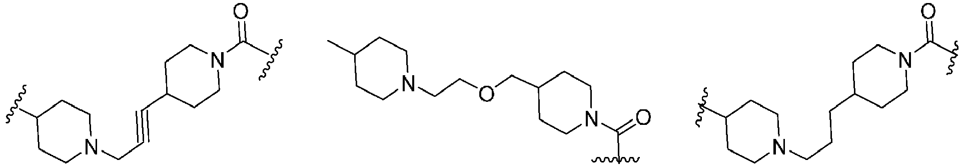 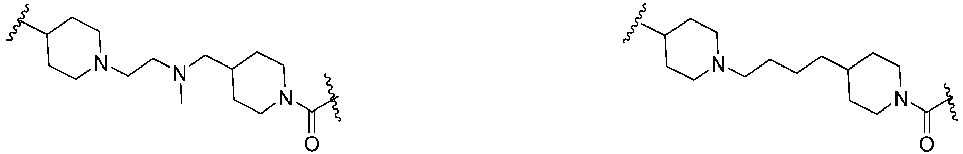 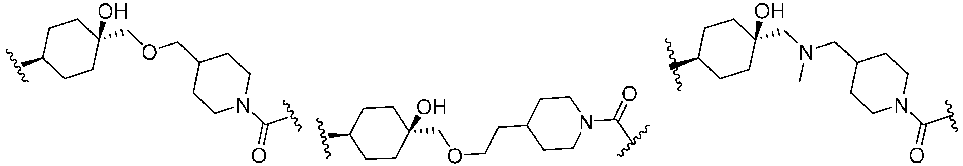 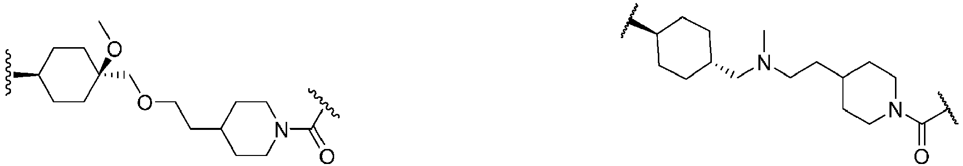 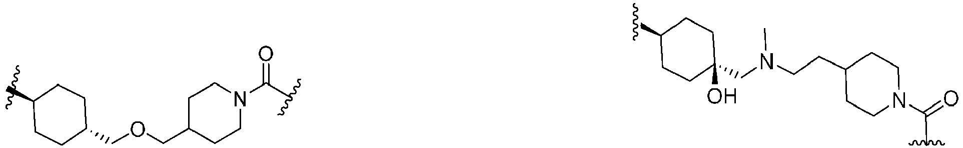 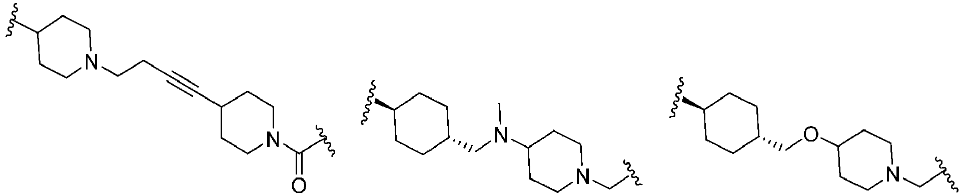 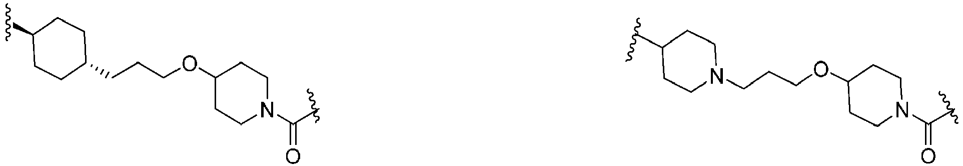 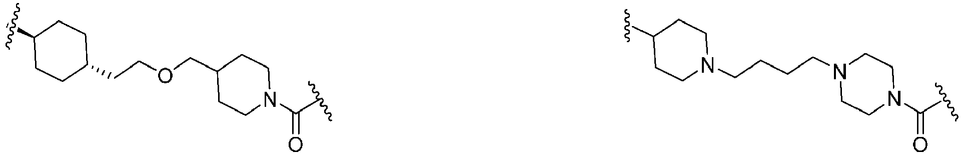 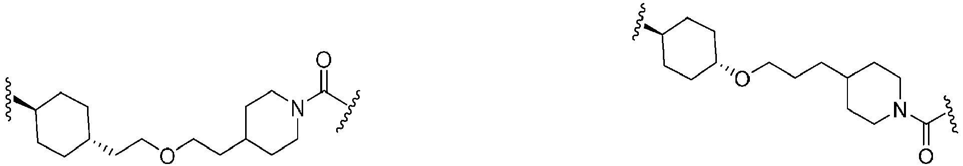 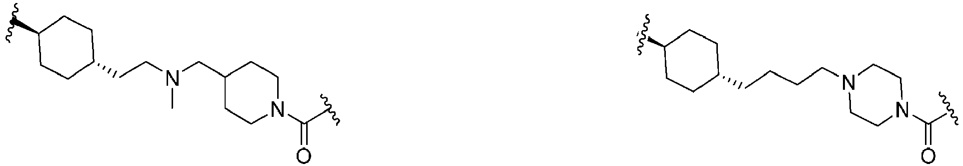 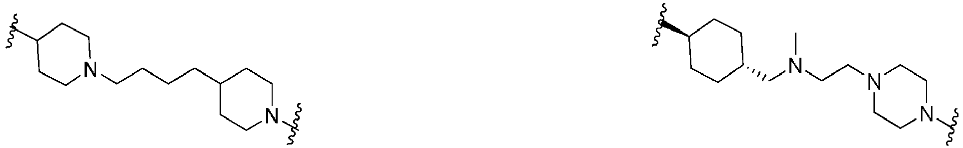 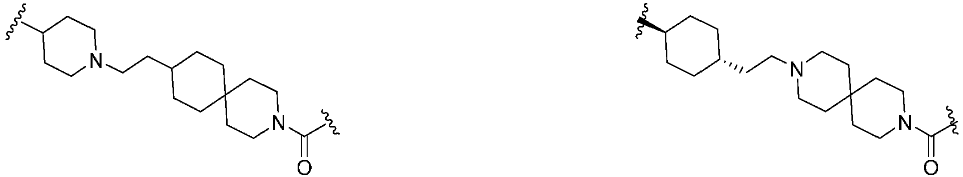 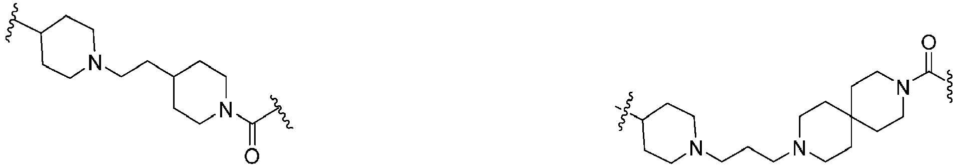 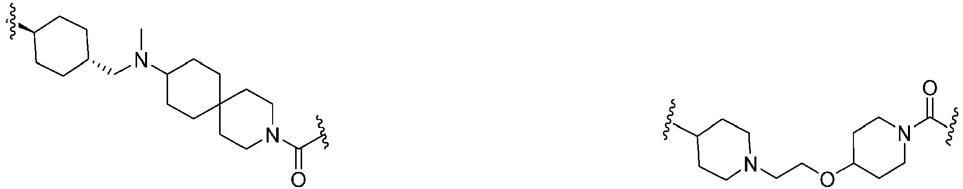 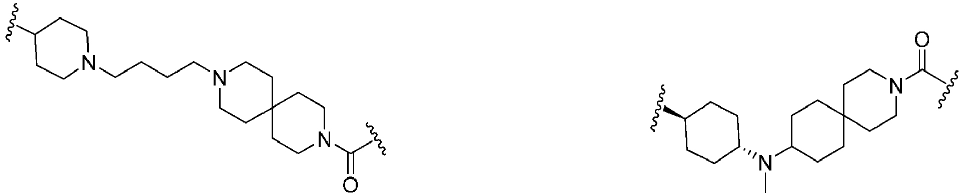 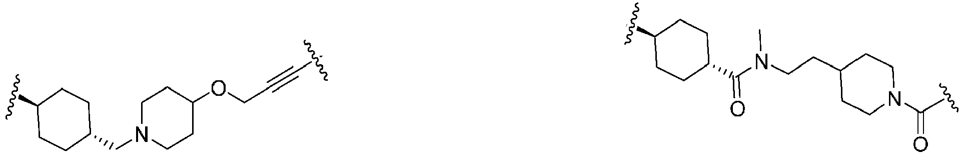 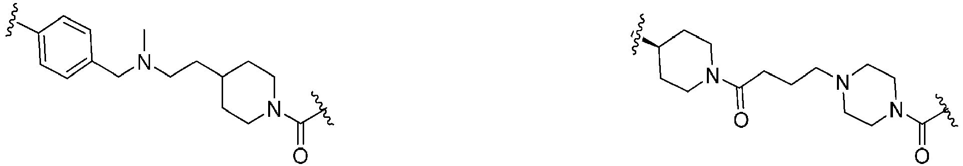 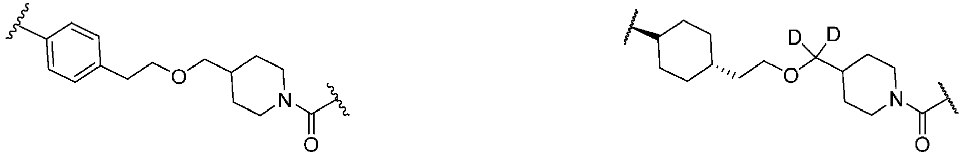 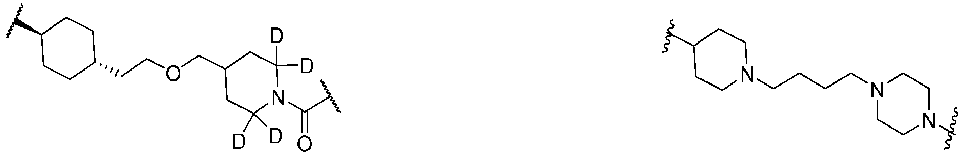 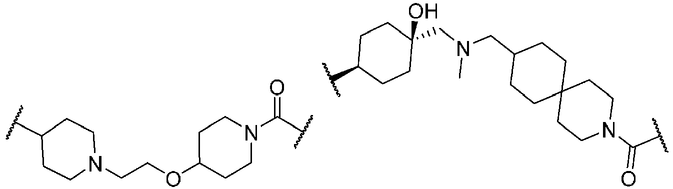 or 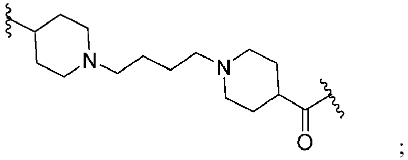 preferably,

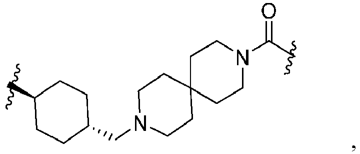

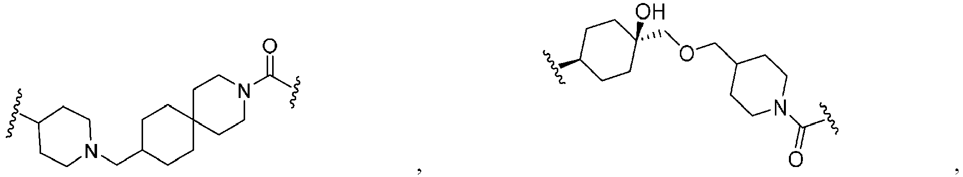

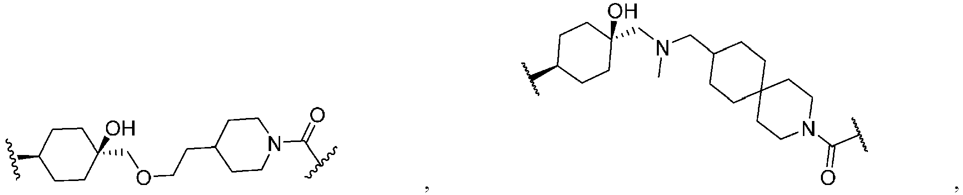

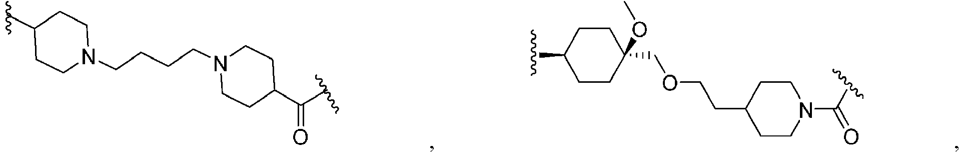

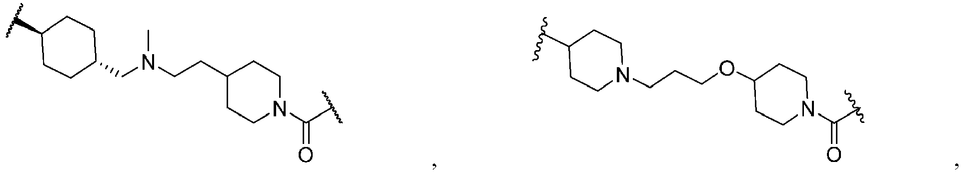

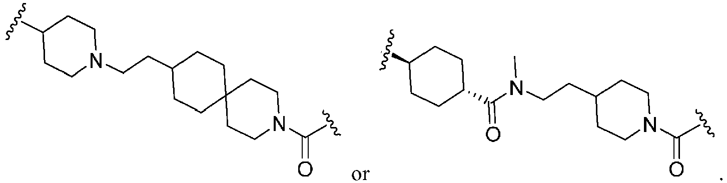

In certain embodiments of the present invention, the pharmaceutically acceptable salt may be a formate.

Preferably, in certain embodiments of the present invention, the compound of formula I, the stereoisomer, enantiomer, diastereomer, deuteride, metabolite, hydrate, solvate and/or prodrug thereof and/or the pharmaceutically acceptable salt thereof are/is: 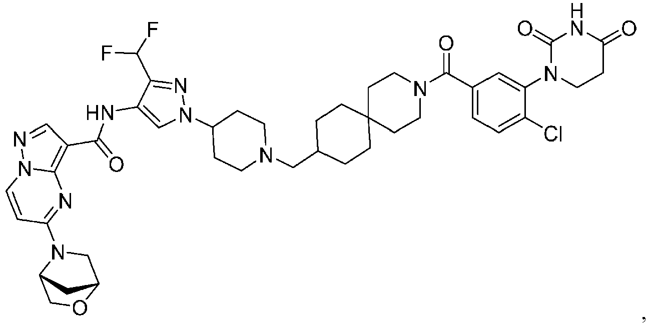 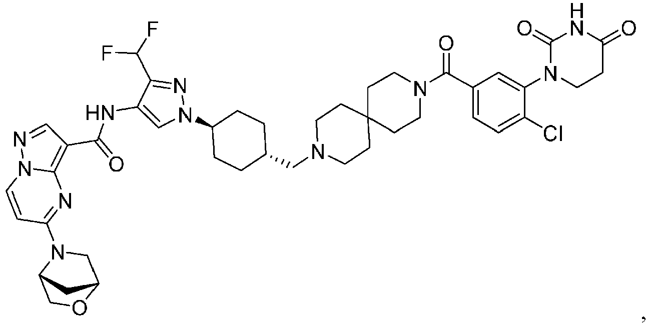 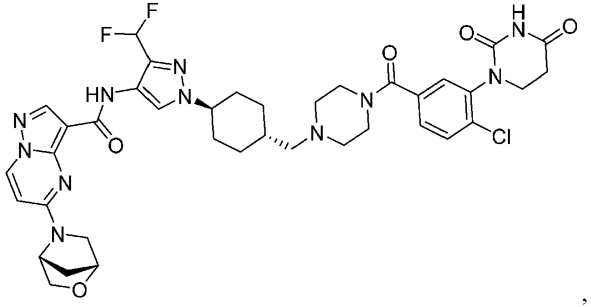 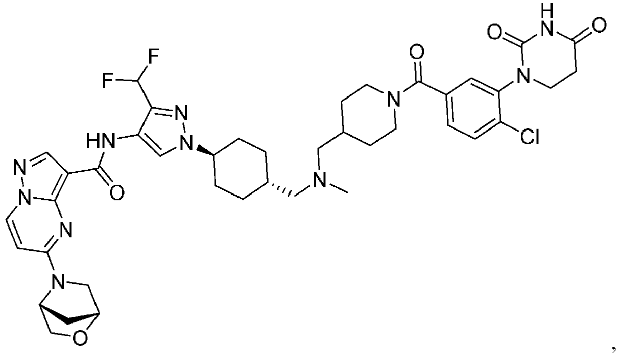 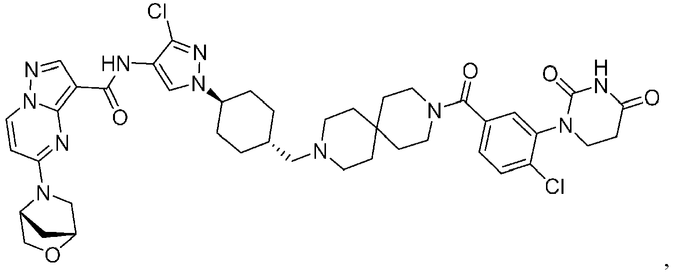 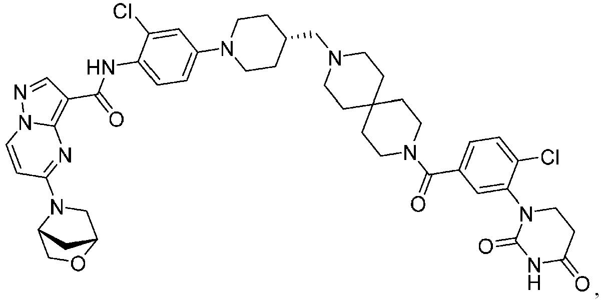 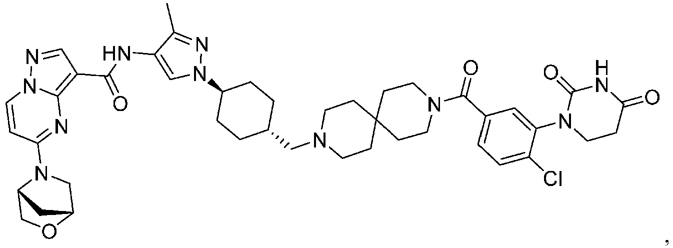 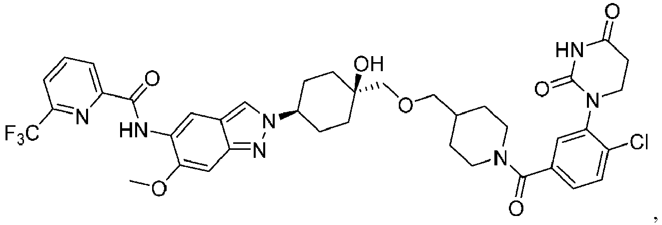 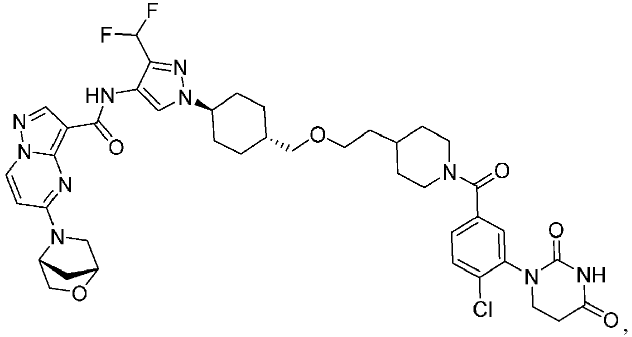 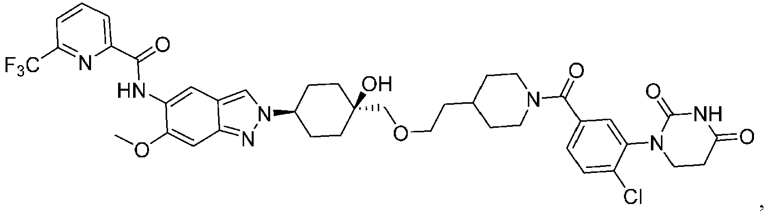 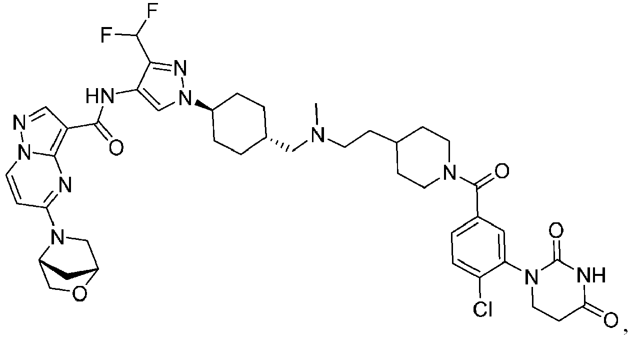 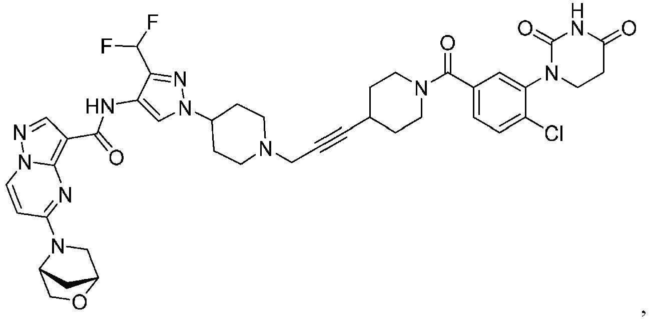 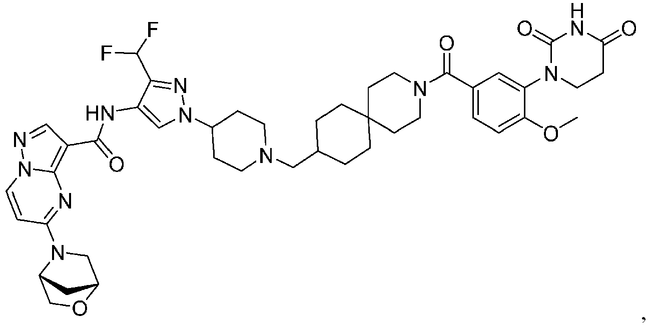 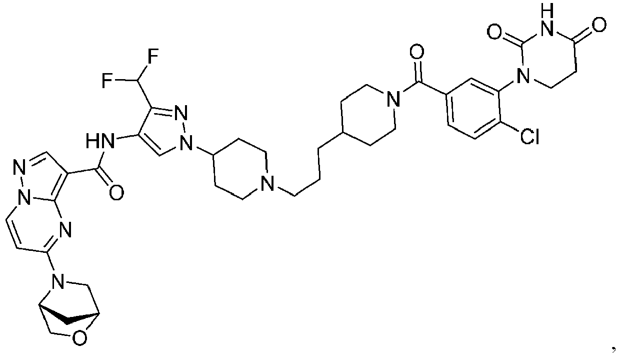 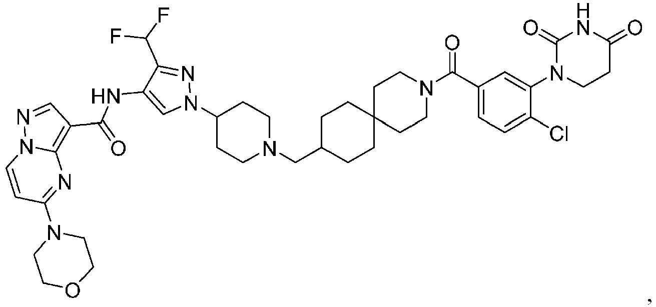 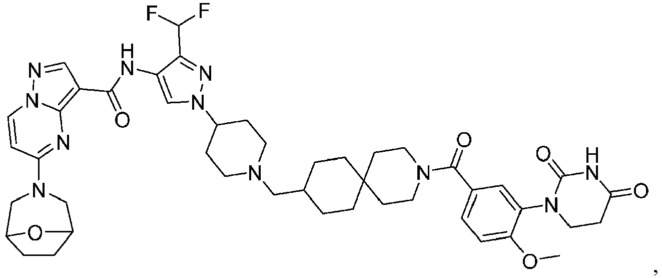 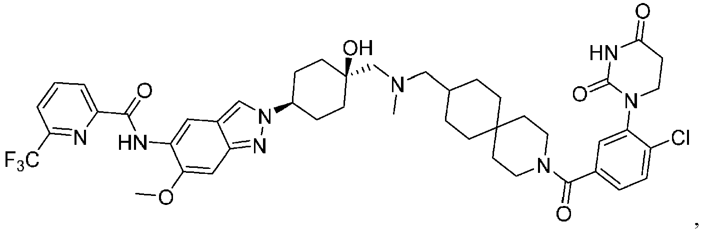 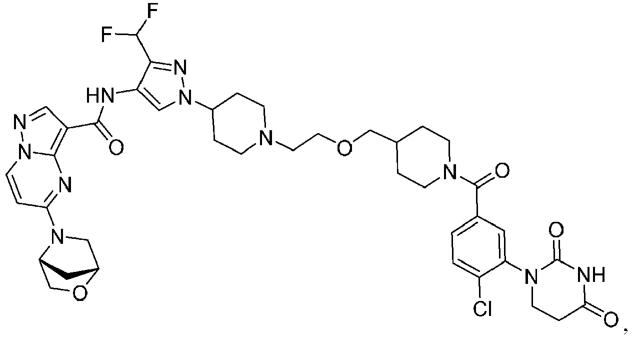 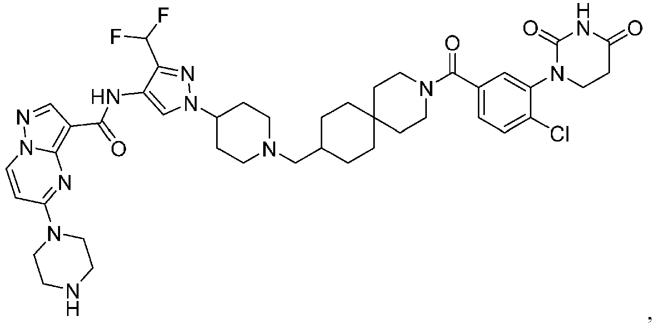 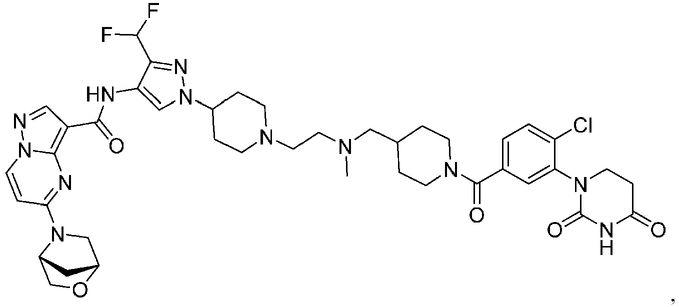 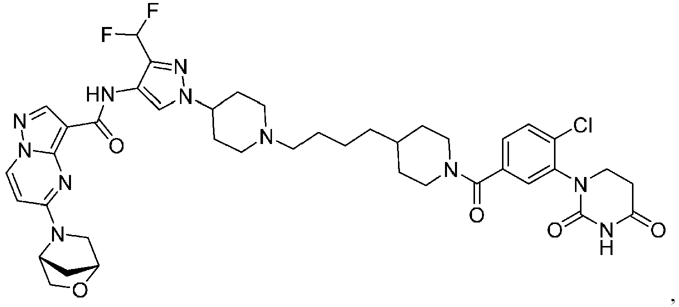 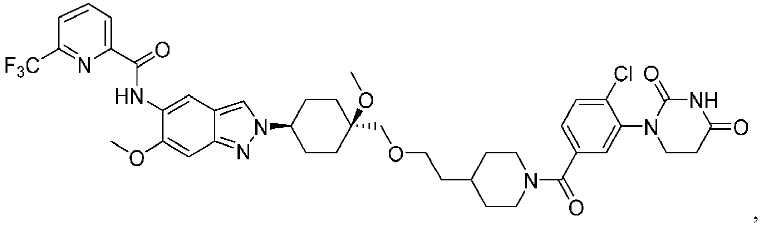 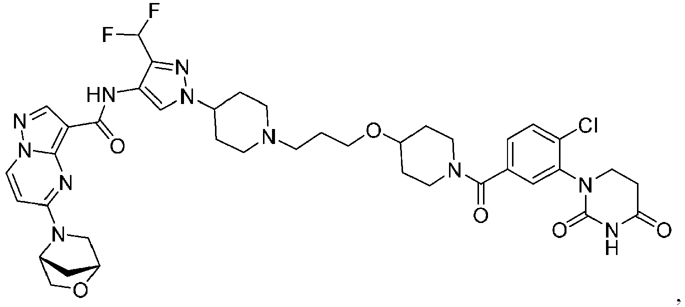 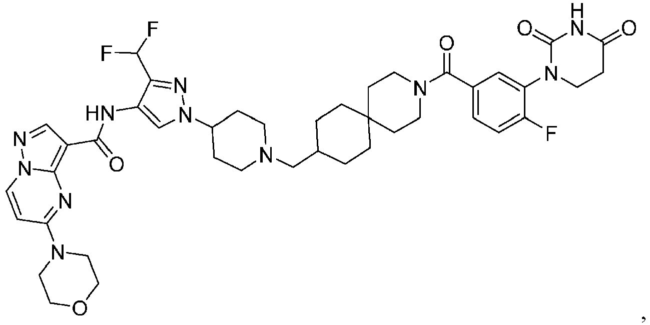 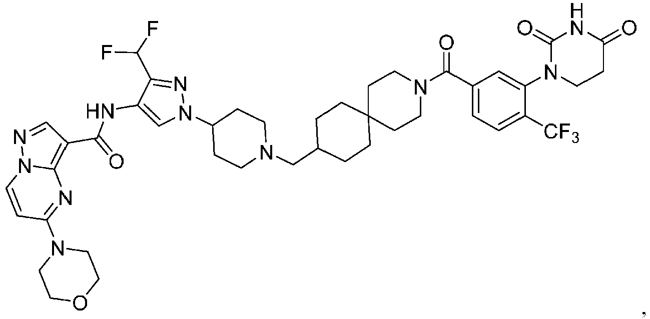 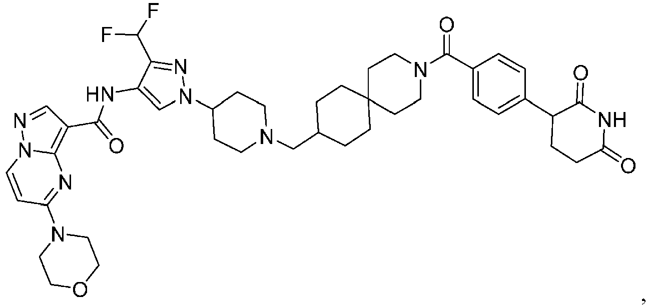 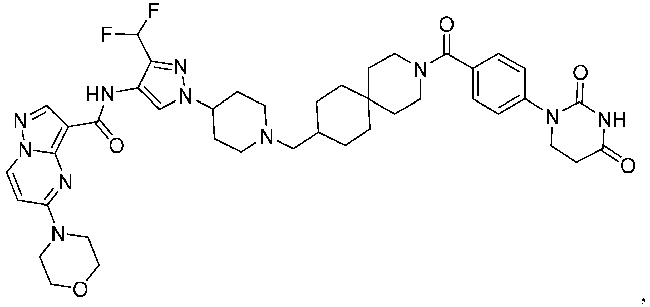 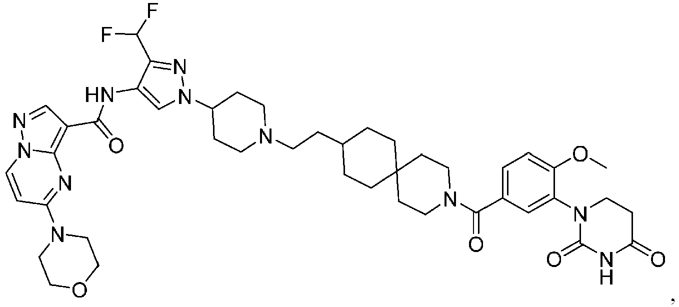 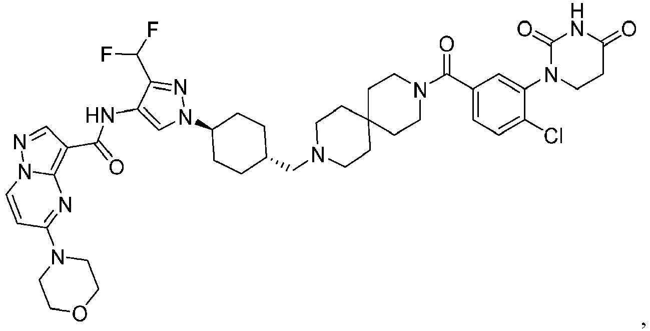 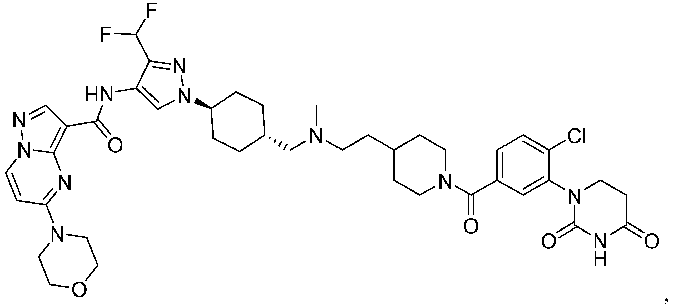 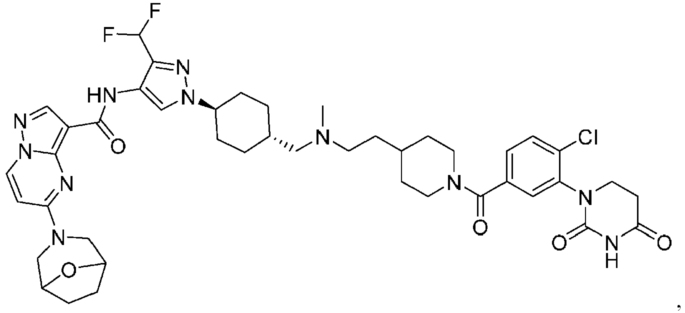 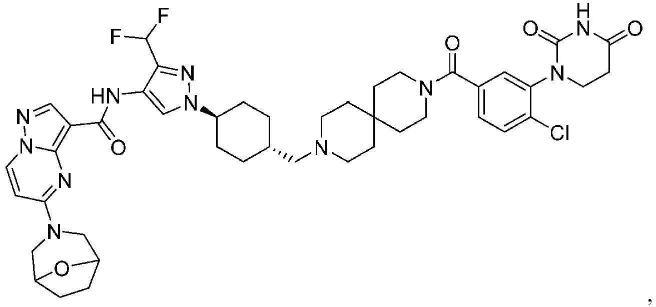 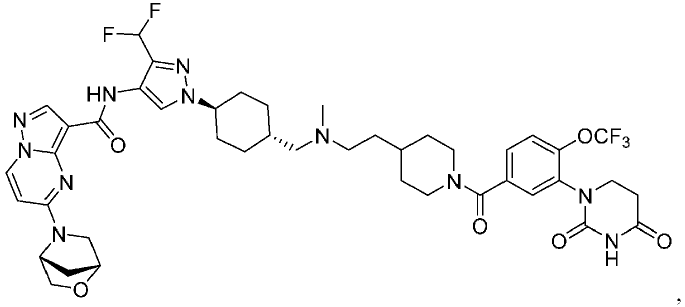 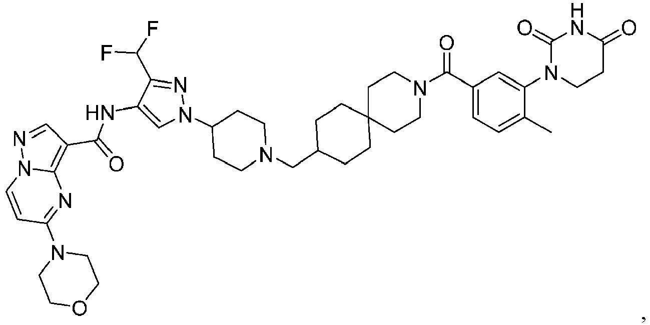 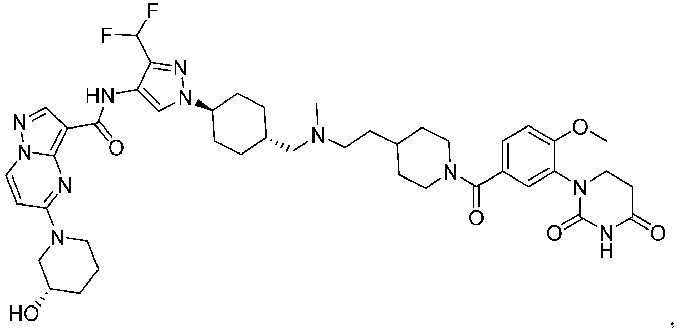 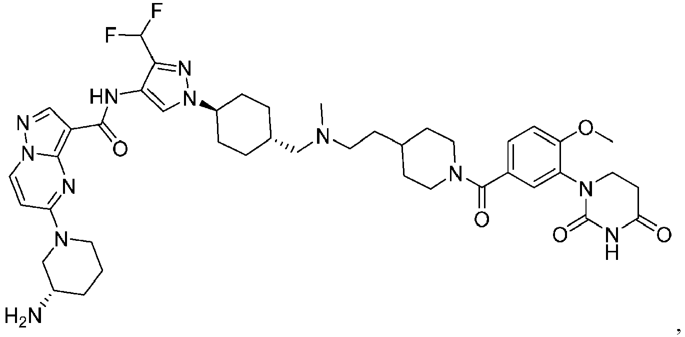 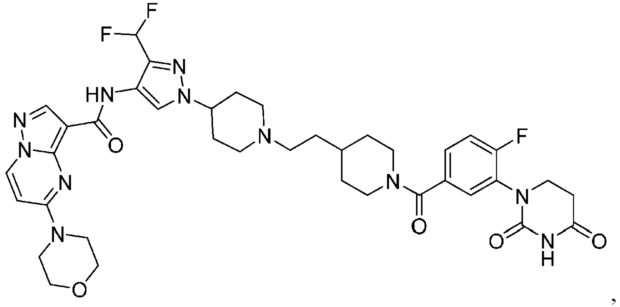 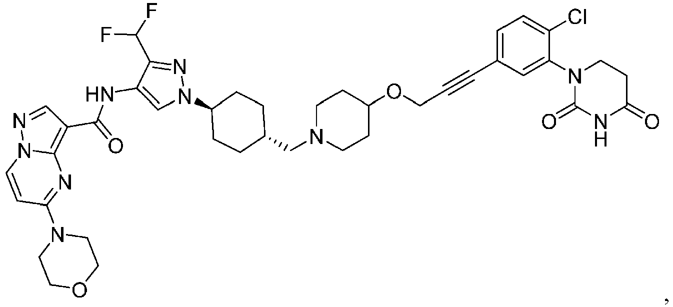 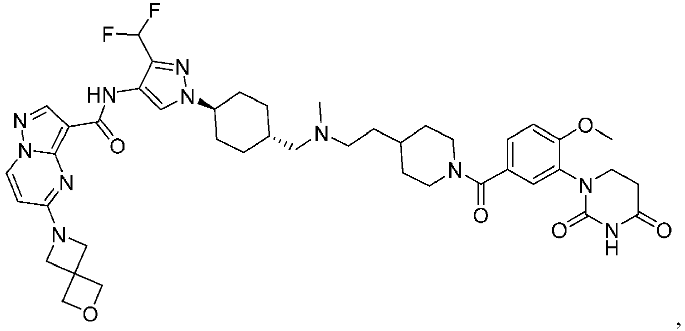 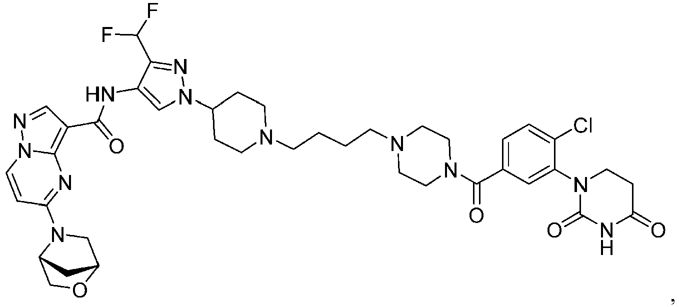 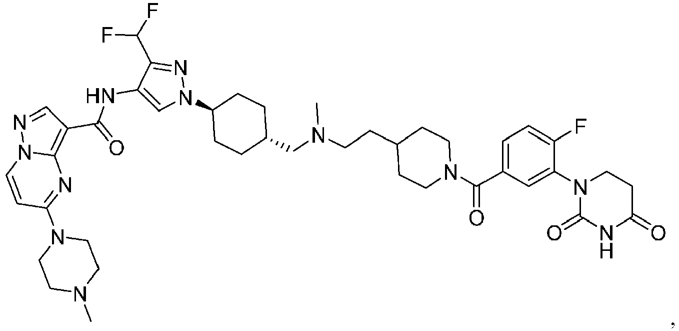 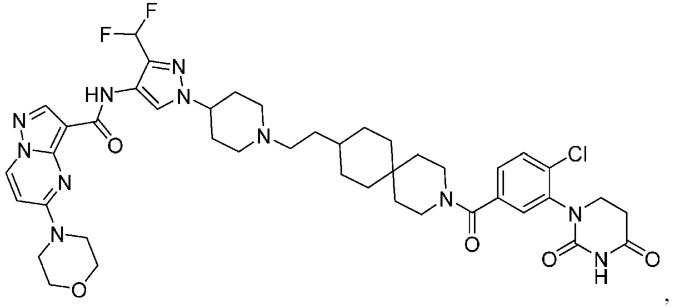 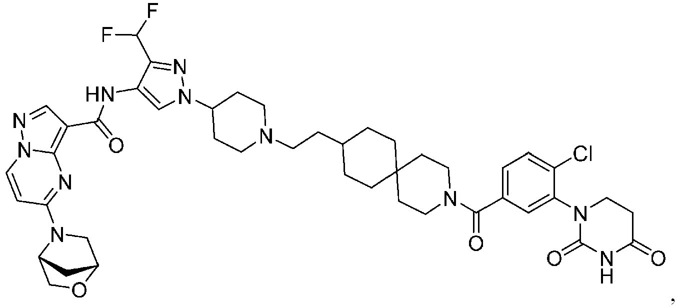 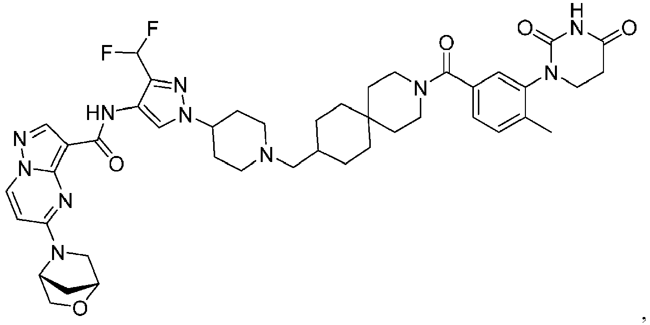 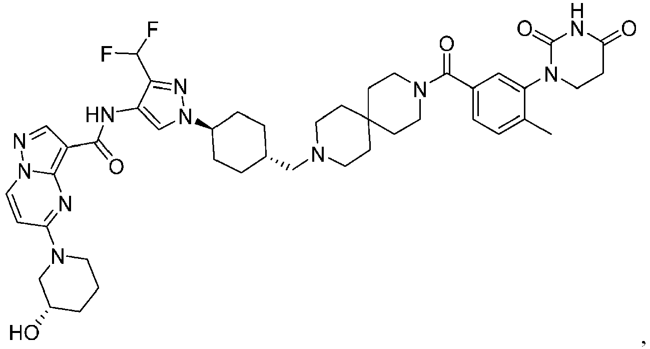 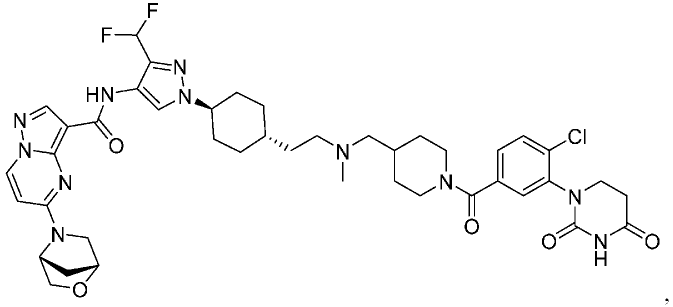 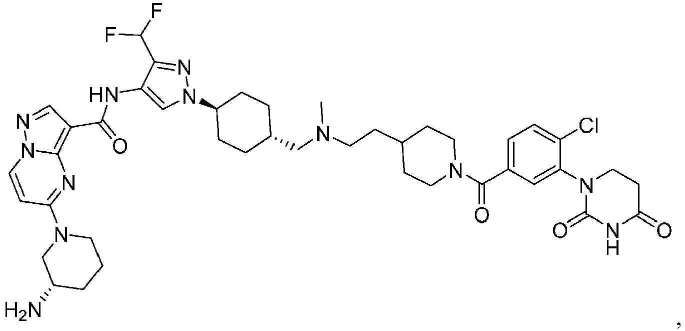 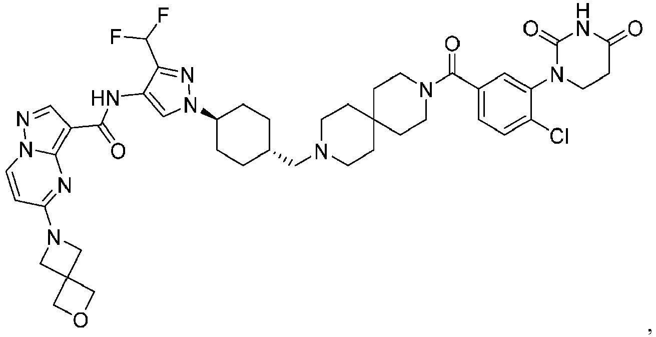 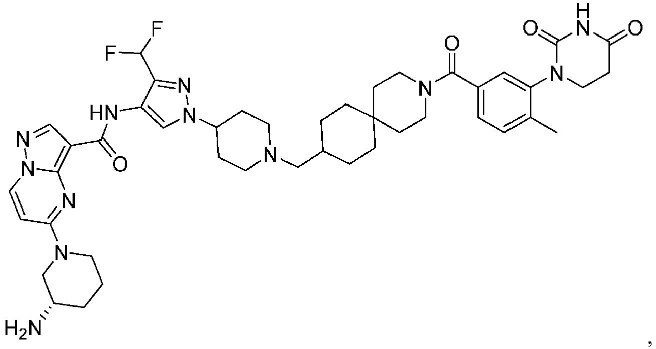 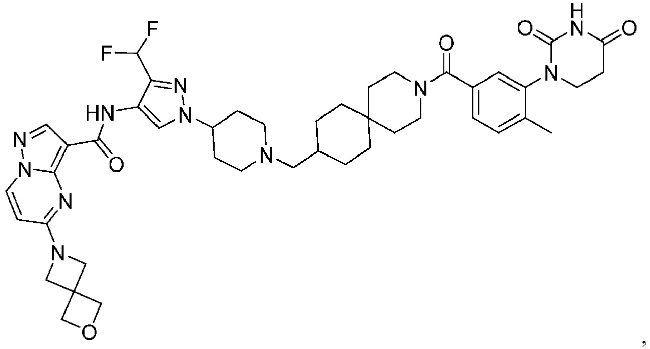 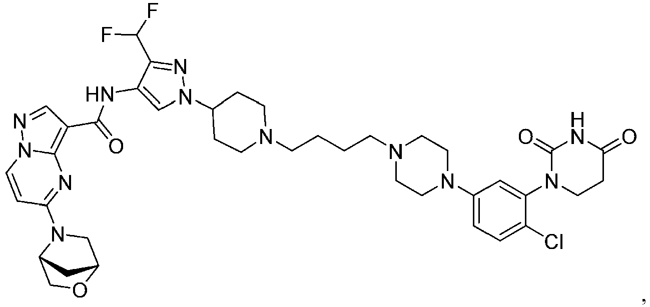 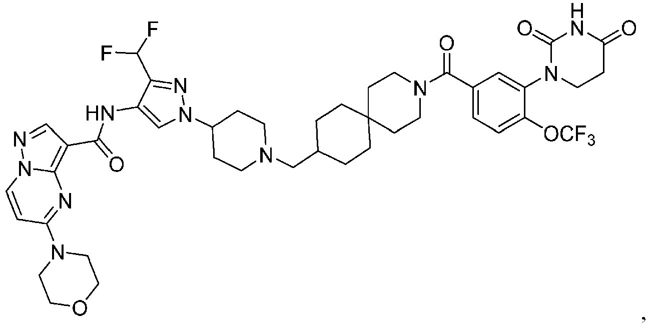 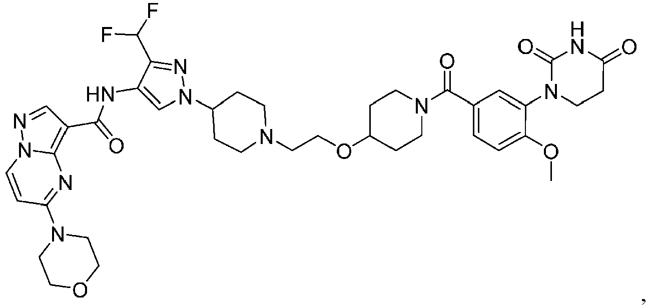 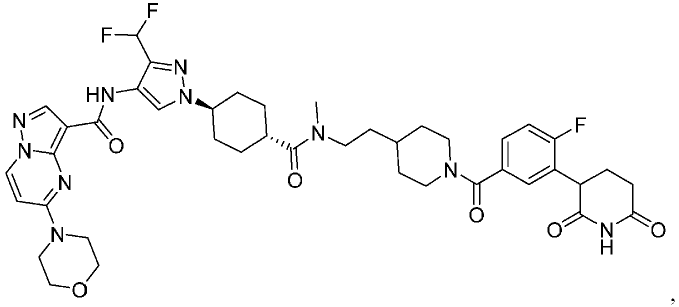 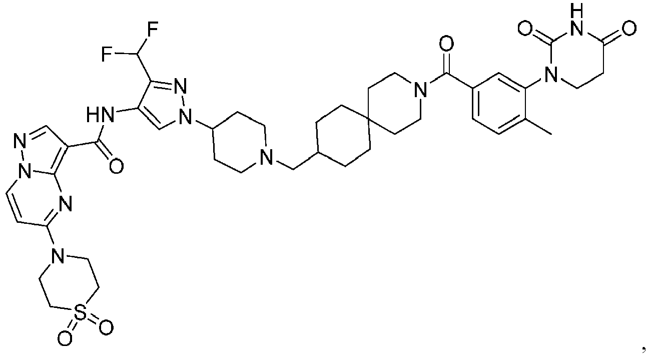 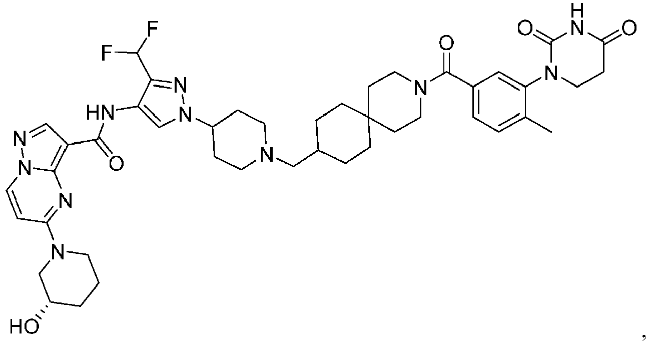 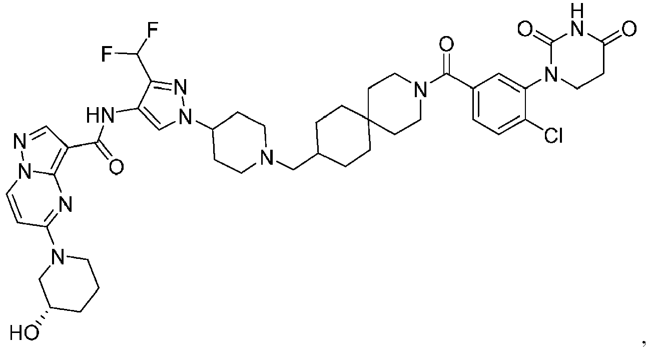 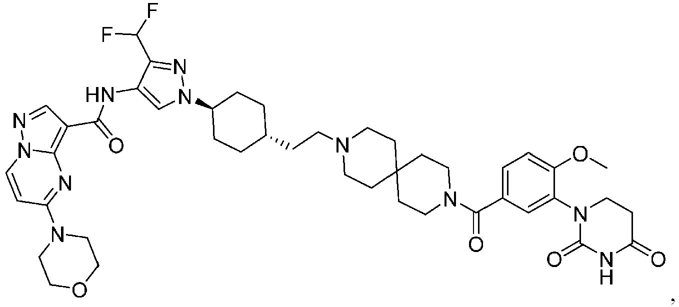 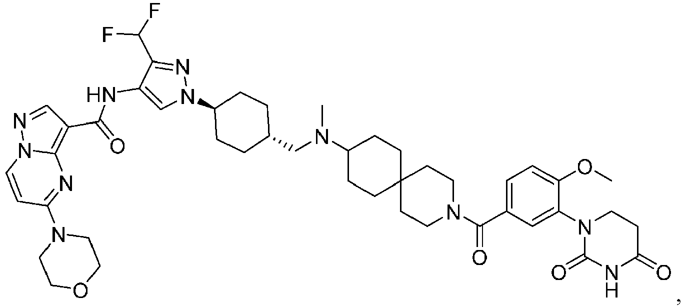 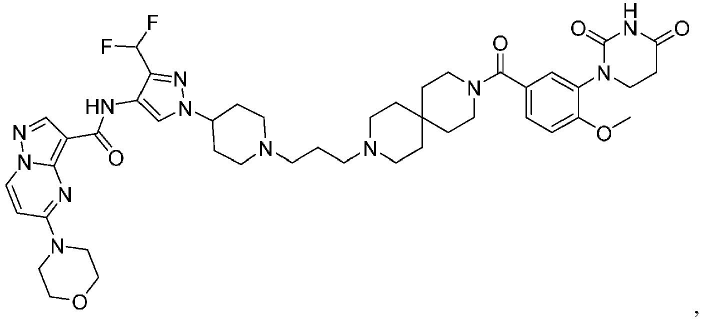 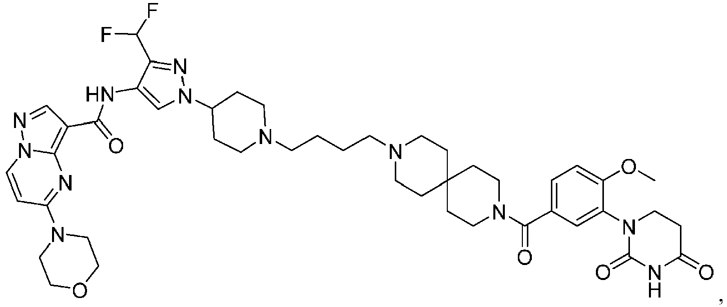 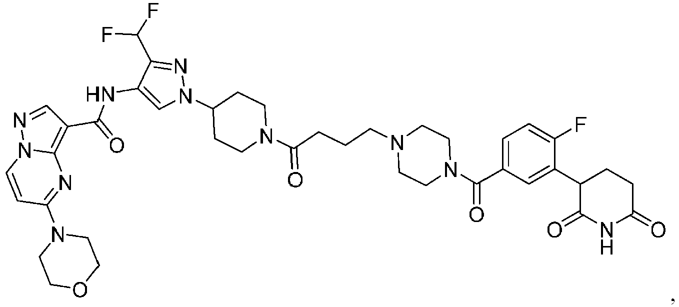 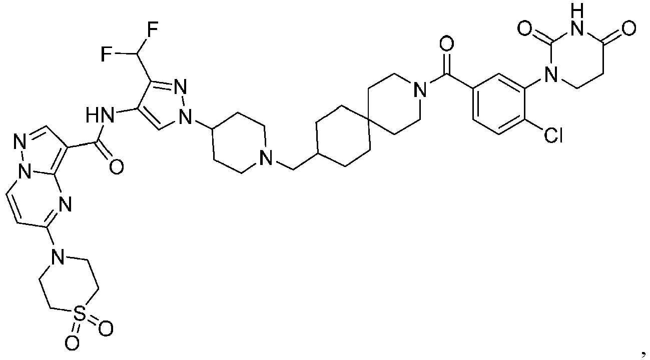 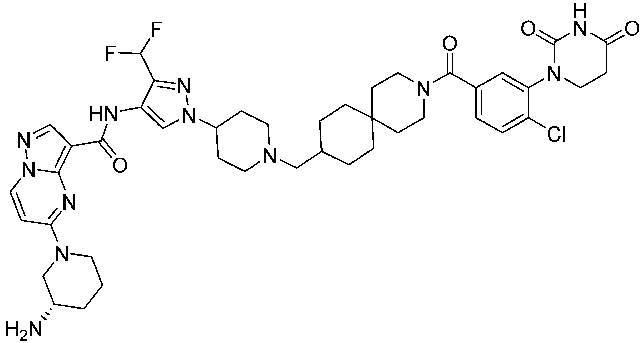 or

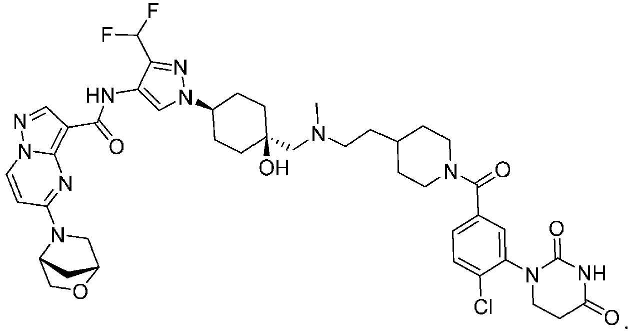

Preferably, in certain embodiments of the present invention, the compound of formula I, and/or the stereoisomer, enantiomer, diastereomer, deuteride, metabolite, hydrate, solvate and/or prodrug thereof and/or the pharmaceutically acceptable salt thereof are/is 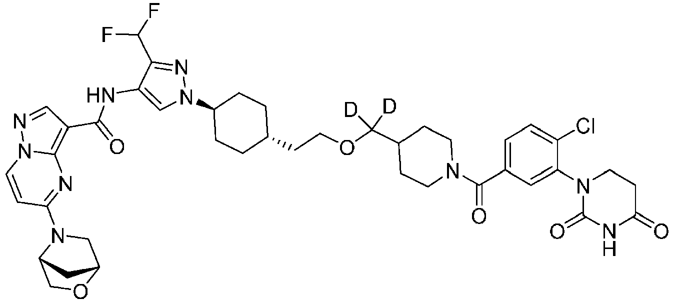 or

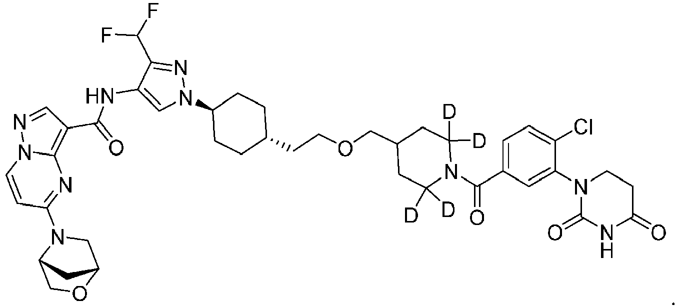

The present invention further discloses a compound of formula VII or VIII, a stereoisomer, enantiomer, diastereomer or deuteride thereof or a salt thereof: 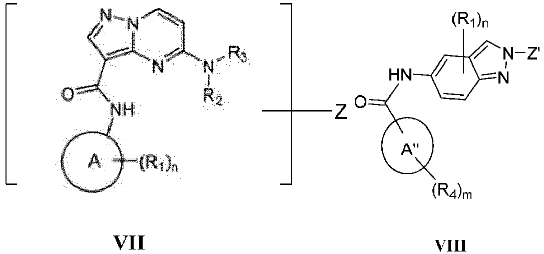
Z is 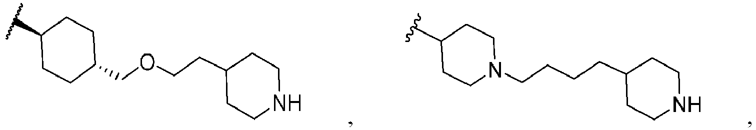 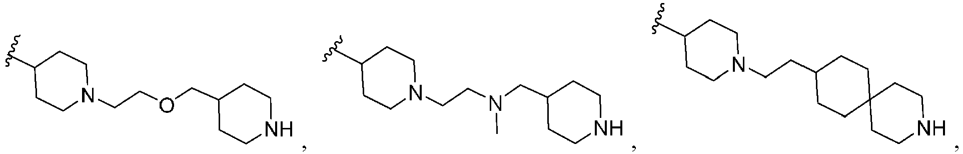 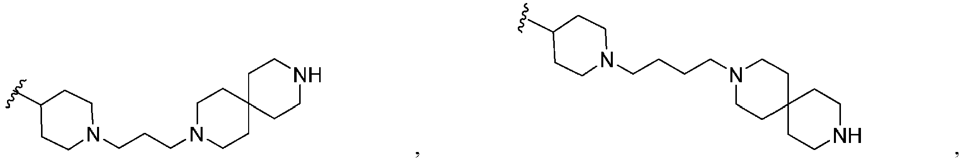 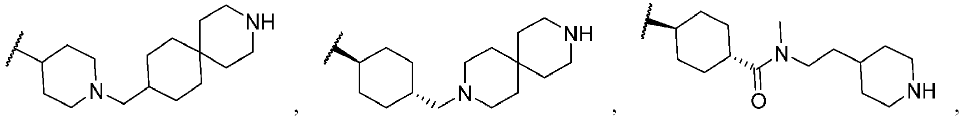 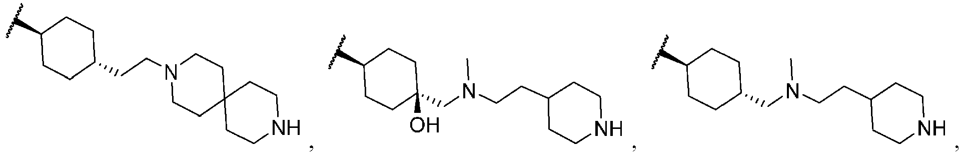 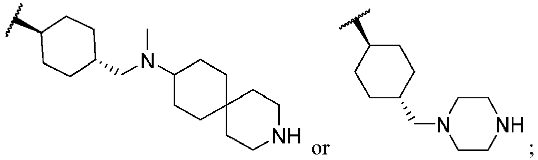
Z' is 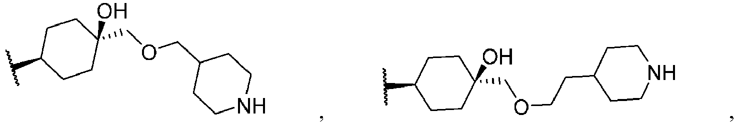 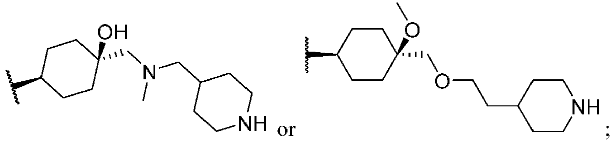
wherein m, n, A, A', R₁, R₂, R₃ and R₄ are as previously described.

In certain embodiments of the present invention, the compound of formula VII is a compound of

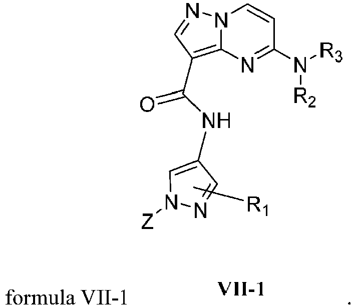

In certain embodiments of the present invention,

The compound of formula VII is any of the following compounds: 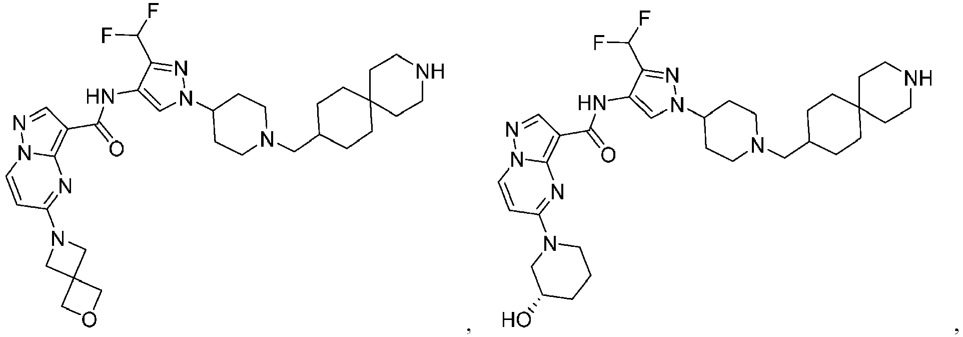 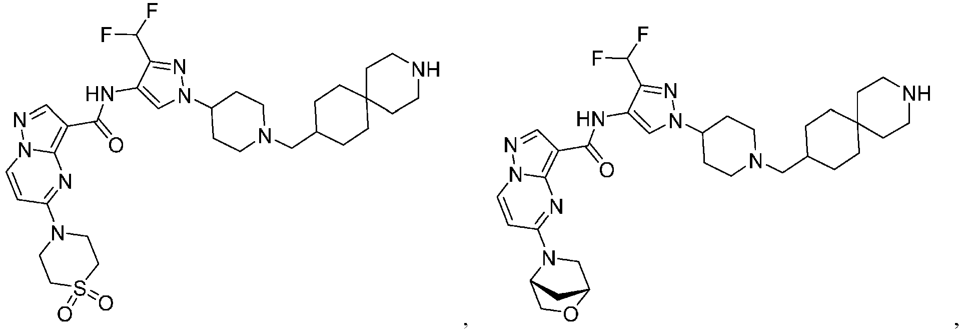 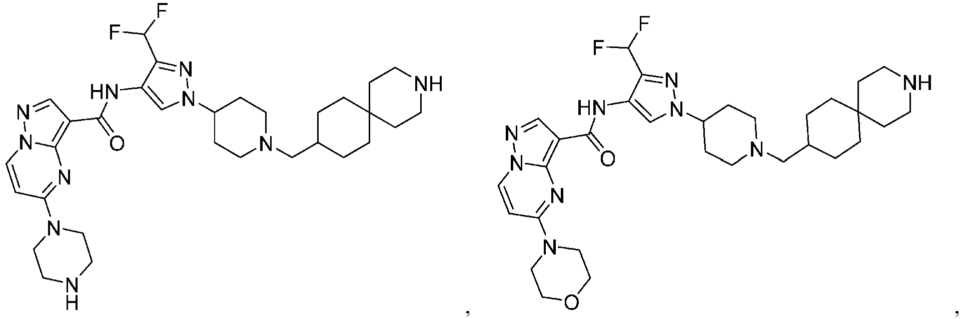 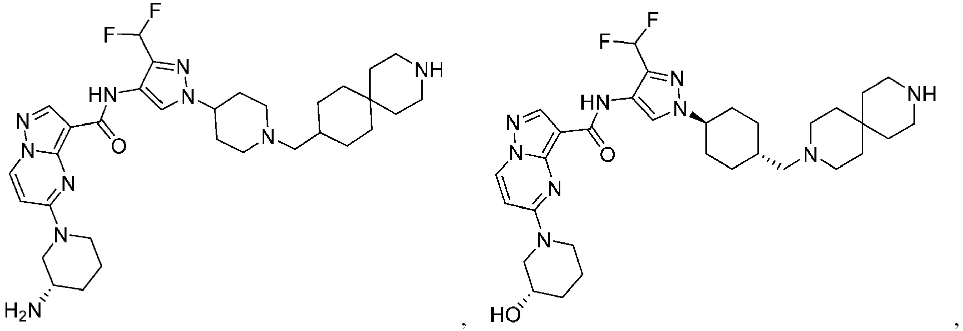 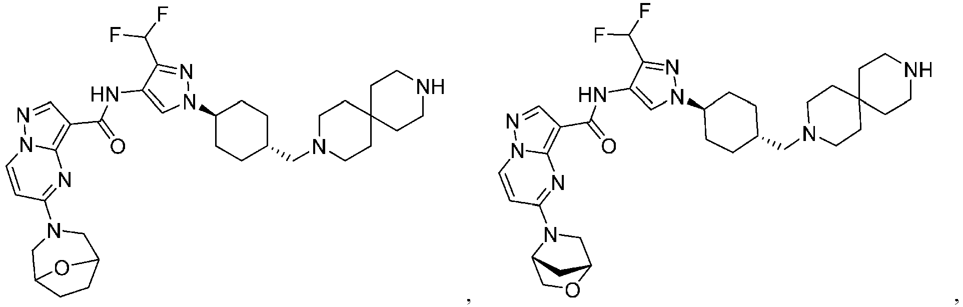 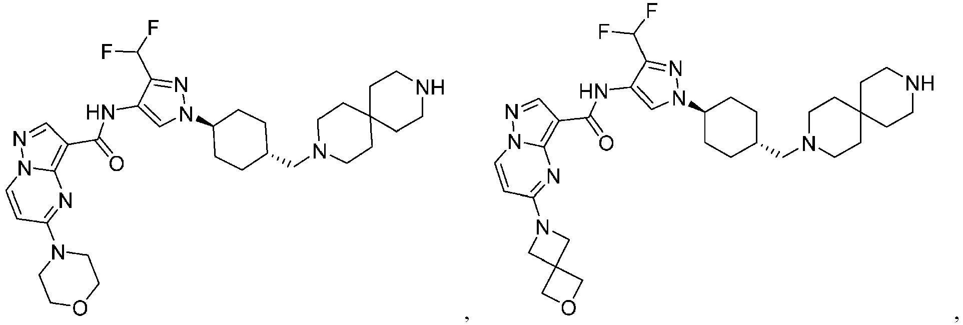 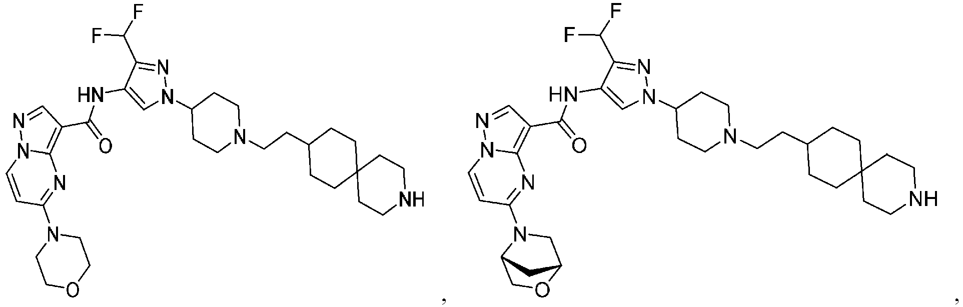 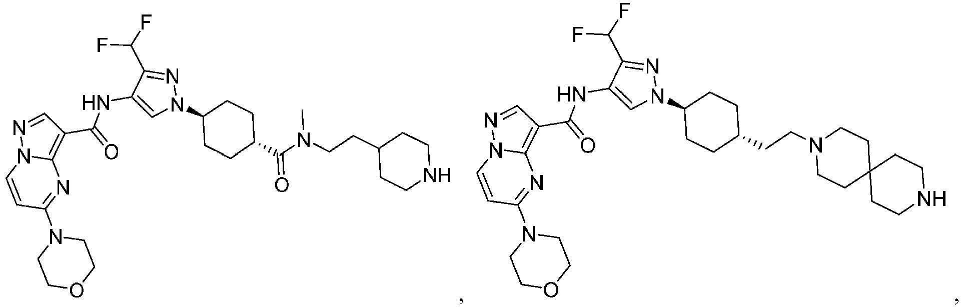 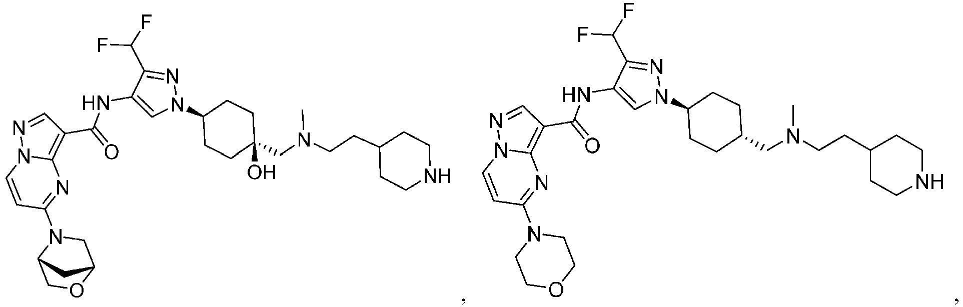 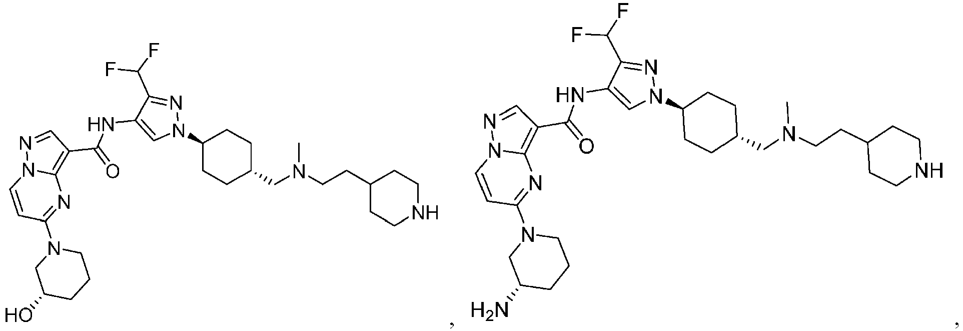 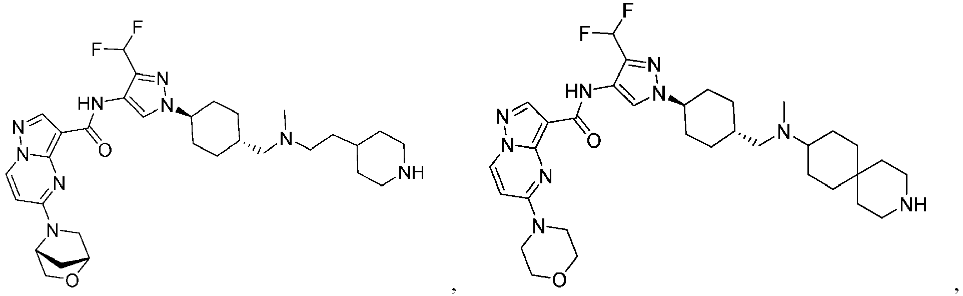 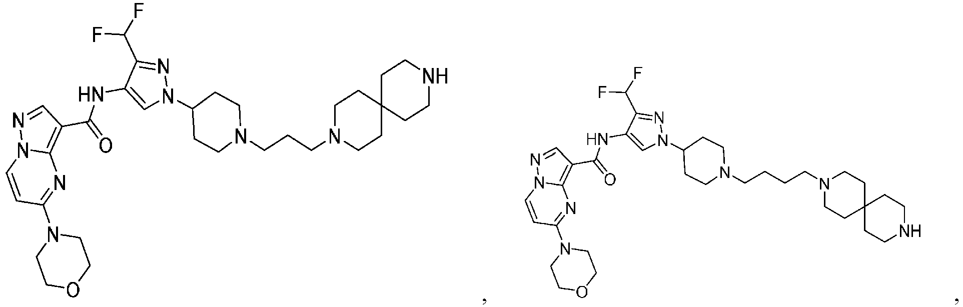 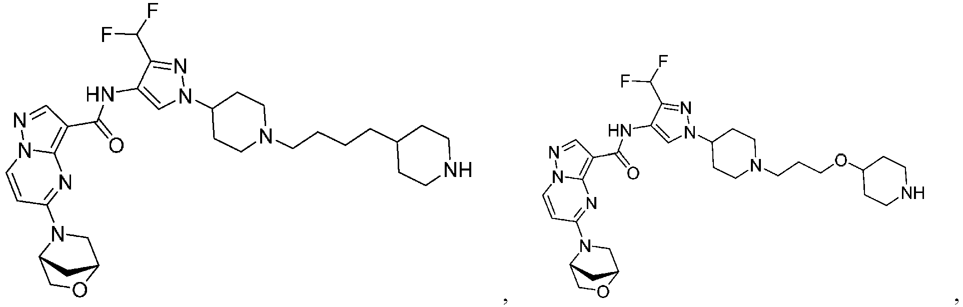 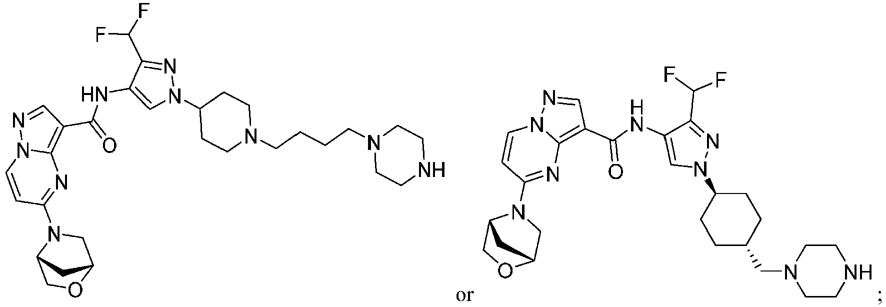 and/or, the compound of formula VIII is any of the following compounds:

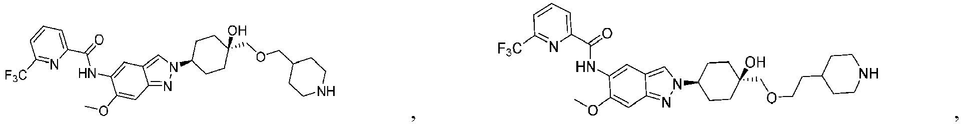

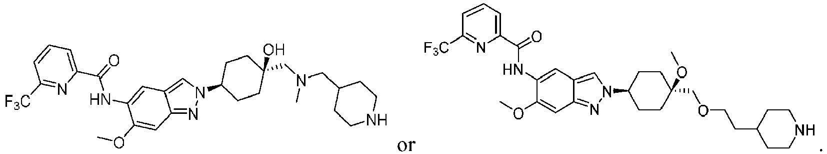

Preferably, in certain embodiments of the present invention, the compound of formula I, and/or the stereoisomer, enantiomer, diastereomer, deuteride, metabolite, hydrate, solvate and/or prodrug thereof and/or the pharmaceutically acceptable salt thereof are/is a compound of formula II, and/or a stereoisomer, enantiomer, diastereomer, deuteride, metabolite, hydrate, solvate and/or prodrug thereof and/or a pharmaceutically acceptable salt thereof, 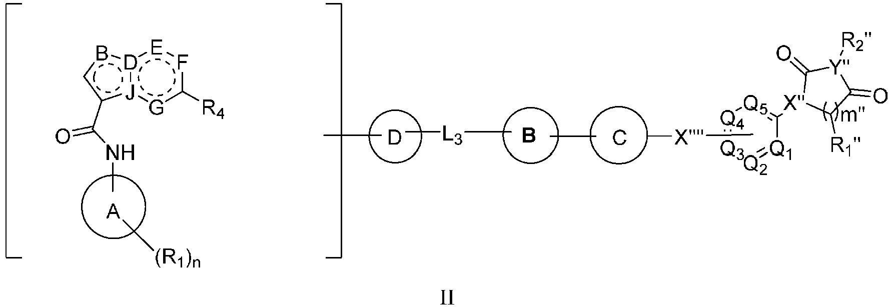 wherein the R₁, n, R₄, B, D, E, F, G, J and ring A are as defined and described in PTM-2'; the Q₁, Q₂, Q₃, Q₄, Q₅, X", Y", m", R₁" and R₂" are as defined and described in the ULM-1 herein; the L₃, ring D, ring B, ring C and X‴′ are as defined and described in the LA herein.

Preferably, in certain embodiments of the present invention, the compound of formula I, and/or the stereoisomer, enantiomer, diastereomer, deuteride, metabolite, hydrate, solvate and/or prodrug thereof and/or the pharmaceutically acceptable salt thereof are/is a compound of formula III, and/or a stereoisomer, enantiomer, diastereomer, deuteride, metabolite, hydrate, solvate and/or prodrug thereof and/or a pharmaceutically acceptable salt thereof, 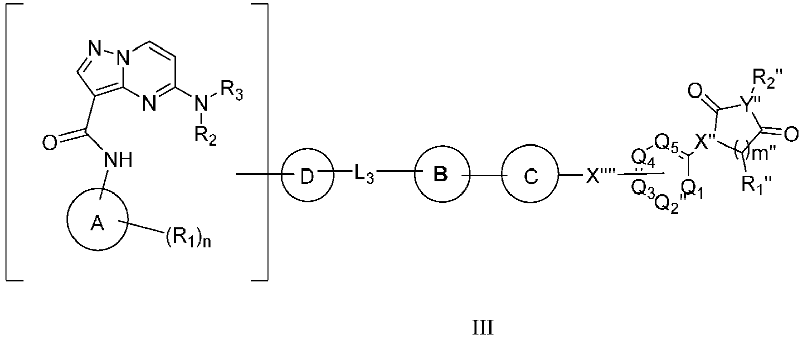 wherein the R₁, n, R₂, R₃ and ring A are as defined and described in PTM-3; the Q₁, Q₂, Q₃, Q₄, Q₅, X", Y", m", R₁" and R₂" are as defined and described in the ULM-1 herein; the L₃, ring D, ring B, ring C and Xʺʺ are as defined and described in the LA herein.

Preferably, in certain embodiments of the present invention, the compound of formula I, and/or the stereoisomer, enantiomer, diastereomer, deuteride, metabolite, hydrate, solvate and/or prodrug thereof and/or the pharmaceutically acceptable salt thereof are/is a compound of formula IV, and/or a stereoisomer, enantiomer, diastereomer, deuteride, metabolite, hydrate, solvate and/or prodrug thereof and/or a pharmaceutically acceptable salt thereof, 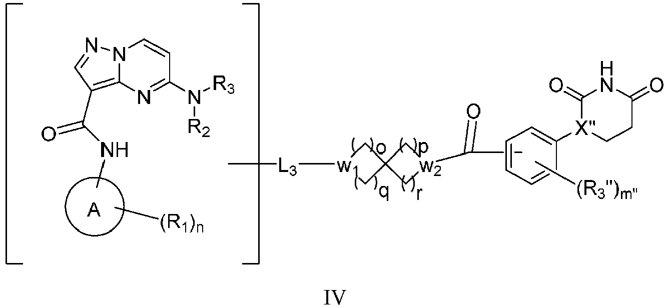 wherein the R₁, n, R₂, R₃, ring A are as defined and described in PTM-3; the X", m" and R₃" are as defined and described in the ULM-1 herein; the L₃ is as defined and described in the LA herein; o, p, q and r are each independently 1 or 2; w₁ and w₂ are each independently CH or N, and at least one of them is N.

Preferably, in certain embodiments of the present invention, the compound of formula I, and/or the stereoisomer, enantiomer, diastereomer, deuteride, metabolite, hydrate, solvate and/or prodrug thereof and/or the pharmaceutically acceptable salt thereof are/is a compound of formula V, and/or a stereoisomer, enantiomer, diastereomer, deuteride, metabolite, hydrate, solvate and/or prodrug thereof and/or a pharmaceutically acceptable salt thereof, 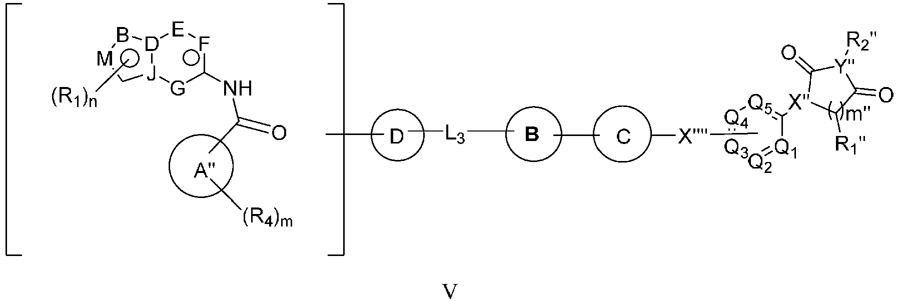 wherein the R₁, n, R₄, B, D, E, F, G, J and ring A" are as defined and described in PTM-2'; the Q₁, Q₂, Q₃, Q₄, Q₅, X", Y", m", R₁" and R₂" are as defined and described in the ULM-1 herein; the L₃, ring D, ring B, ring C and Xʺʺ are as defined and described in the LA herein.

Preferably, in certain embodiments of the present invention, the compound of formula I, and/or the stereoisomer, enantiomer, diastereomer, deuteride, metabolite, hydrate, solvate and/or prodrug thereof and/or the pharmaceutically acceptable salt thereof are/is a compound of formula VI, and/or a stereoisomer, enantiomer, diastereomer, deuteride, metabolite, hydrate, solvate and/or prodrug thereof and/or a pharmaceutically acceptable salt thereof, 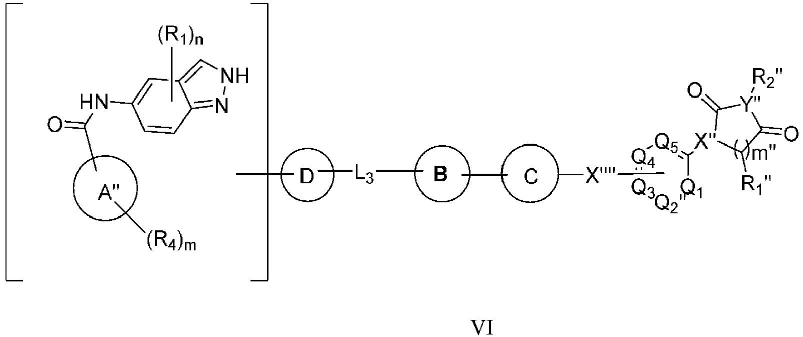 wherein the R₁, n, R₂, R₃ and ring A" are as defined and described in PTM-3; the Q₁, Q₂, Q₃, Q₄, Q₅, X", Y", m", R₁" and R₂" are as defined and described in the ULM-1 herein; the L₃, ring D, ring B, ring C and Xʺʺ are as defined and described in the LA herein.

The present invention provides a method for preparing the compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof.

The present invention provides a pharmaceutical composition, comprising a therapeutically effective amount of the compound of formula 1, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, and a pharmaceutically acceptable carrier, diluent or excipient.

The present invention provides a pharmaceutical composition, comprising a therapeutically effective amount of the compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, and a pharmaceutically acceptable carrier.

The present invention provides a method for degrading the IRAK4 protein, comprising contacting the compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof with the IRAK4 protein.

The present invention provides use of a substance A in the preparation of a degrader for the IRAK4 protein, the substance A comprising the previously described compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the previously described pharmaceutical composition thereof.

The compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof of the present invention is used as a medicament for treating or preventing IRAK4-mediated diseases or disorders.

The compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof of the present invention is used as a medicament for treating or preventing diseases or disorders mediated by TLR (except TLR3R) or the IL-1β receptor family (including IL-1R, IL-18R, IL-33R and IL-36R).

The compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof of the present invention is used as a medicament for treating or preventing IRAK4-mediated diseases or disorders, wherein the IRAK4-mediated diseases or disorders are MyD88-driven diseases or disorders.

The present invention provides use of the compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof in the preparation of a medicament for treating or preventing IRAK4-mediated diseases or disorders.

In the present invention, the IRAK4-mediated diseases or disorders are MyD88-driven diseases or disorders.

The present invention provides use of the compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof in the preparation of a medicament for treating or preventing diseases or disorders mediated by TLR (except TLR3R) or the IL-1β receptor family (including IL-1R, IL-18R, IL-33R and IL-36R).

The present invention provides use of the compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof in the preparation of a medicament for treating or preventing IRAK4-regulated diseases or disorders, wherein the IRAK4-mediated diseases or disorders are MyD88-driven diseases or disorders.

The present invention provides use of the compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof in the preparation of a medicament for treating or preventing disorders or diseases including cancer, neurodegenerative disorders, viral diseases, autoimmune diseases, inflammatory diseases, genetic disorders, hormone-associated diseases, metabolic disorders, diseases associated with organ transplantation, immunodeficiency disorders, destructive bone diseases, proliferative disorders, infectious diseases, conditions associated with cell death, thrombin-induced platelet aggregation, liver diseases, pathological immune conditions involving T cell activation, cardiovascular disorders or CNS.

The present invention provides use of the compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof in the preparation of a medicament for treating or preventing a cancer or proliferative disease, wherein the cancer or proliferative disease is brain cancer, kidney cancer, liver cancer, adrenal cancer, bladder cancer, breast cancer, stomach cancer, ovarian cancer, colon cancer, rectal cancer, prostate cancer, pancreatic cancer, lung cancer, vaginal cancer, cervical cancer, testicular cancer, genitourinary tract cancer, esophageal cancer, laryngeal cancer, skin cancer, bone cancer, thyroid cancer, sarcoma, glioblastoma, neuroblastoma, multiple myeloma, gastrointestinal cancer, neck or head tumor, epidermal hyperplasia, psoriasis, prostate hyperplasia, adenoma, adenocarcinoma, keratoacanthoma, squamous cell carcinoma, large cell carcinoma, non-small cell lung cancer, lymphoma, Hodgkin's and non-Hodgkin's, breast cancer, follicular carcinoma, undifferentiated carcinoma, papillary tumor, seminoma, melanoma, ABC DLBCL, Hodgkin's lymphoma, primary cutaneous T-cell lymphoma, chronic lymphocytic leukemia, smoldering indolent multiple myeloma, leukemia, diffuse large B-cell lymphoma (DLBCL), chronic lymphocytic leukemia (CLL), chronic lymphocytic lymphoma, primary exudative lymphoma, Burkitt's lymphoma/leukemia, acute lymphocytic leukemia, B-cell prolymphocytic leukemia, lymphoplasmacytic lymphoma, Waldenstroms's macroglobulinemia (WM), splenic marginal zone lymphoma, multiple myeloma, or plasmacytoma or intravascular large B-cell lymphoma.

The present invention provides use of the compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof in the preparation of a medicament for treating or preventing a neurodegenerative disease, wherein the neurodegenerative disease is a neurodegenerative disease caused by Alzheimer's disease, Parkinson's disease, amyotrophic lateral sclerosis, Huntington's disease, cerebral ischemia or traumatic injury, glutamate neurotoxicity, hypoxia, epilepsy, diabetes therapy, metabolic syndrome, obesity, organ transplantation or graft-versus-host disease.

The present invention provides use of the compound of formula I, formula II, formula III, formula IV, formula V or formula VI, and/or the stereoisomer, enantiomer, diastereomer, deuteride, hydrate, solvate, metabolite and/or prodrug thereof and/or the pharmaceutically acceptable salt thereof, or the pharmaceutical composition thereof in the preparation of a medicament for treating or preventing an inflammatory disease, wherein the inflammatory disease is an eye disease, such as eye allergy, conjunctivitis, dry eye or spring conjunctivitis, a disease affecting the nose, including allergic rhinitis; an autoimmune hematological disorder, such as hemolytic anemia, aplastic anemia, pure red blood cell anemia and idiopathic thrombocytopenia, systemic lupus erythematosus, rheumatoid arthritis, polychondritis, scleroderma, dermatomyositis, chronic active hepatitis, myasthenia gravis, Stephen-Johnson syndrome, idiopathic stomatitis diarrhea, autoimmune inflammatory bowel disease, irritable bowel syndrome, celiac disease, tooth root periostitis, lung hyaline membrane disease, nephropathy, glomerular disease, alcoholic liver disease, multiple sclerosis, endocrine ophthalmopathy, Grave's disease, sarcomatosis, dry eye, spring conjunctival keratitis, interstitial pulmonary fibrosis, psoriatic arthritis, systemic juvenile idiopathic arthritis, nephritis, vasculitis, interstitial cystitis, diverticulitis, glomerulonephritis, chronic granulomatous disease, adenomyosis, leptospirosis nephropathy, glaucoma, retinal disease, aging, headache, pain, complex regional pain syndrome, cardiac hypertrophy, muscle atrophy, catabolism, obesity, slow fetal growth, hypercholesterolemia, heart disease, chronic heart failure, mesothelioma, anhidrotic ectodermal dysplasia, Behcet's disease, pigment incontinence, Paget's disease, pancreatitis, hereditary periodic fever syndrome, asthma, acute lung injury, acute respiratory distress syndrome, eosinophilia, allergic reaction, systemic allergic reaction, sinusitis, eye allergy, silica-induced disease, COPD, lung disease, cystic fibrosis, liver fibrosis, renal fibrosis, alcoholic fatty liver, non-alcoholic fatty liver, heart fibrosis, psoriasis, Crohn's disease, inflammatory bowel disease, acid-induced lung injury, pulmonary hypertension, polyneuropathy, cataract, muscle inflammation combined with systemic sclerosis, inclusion body myositis, myasthenia gravis, thyroiditis, Addison's disease, lichen planus, type 1 diabetes, type 2 diabetes, appendicitis, atopic dermatitis, asthma, allergy, bronchiolitis, bronchitis, bursitis, cervicitis, cholangitis, cholecystitis, chronic transplant rejection, colitis, conjunctivitis, cystitis, lacrimal gland inflammation, dermatitis, dermatomyositis, encephalitis, endocarditis, endometritis, enteritis, enterocolitis, upper ankle inflammation, epididymitis, fasciitis, fibrous tissue inflammation, gastritis, gastroenteritis, Henoch-Schonlein purpura, hepatitis, suppurative sweat inflammation, immunoglobulin A nephropathy, interstitial lung disease, laryngitis, mastitis, meningitis, myelitis, myocarditis, myositis, nephritis, oophoritis, orchitis, osteitis, otitis, pancreatitis, mumps, pericarditis, peritonitis, pharyngitis, pleurisy, phlebitis, local pneumonia, pneumonia, polymyositis, proctitis, prostatitis, pyelonephritis, rhinitis, salpingitis, sinusitis, stomatitis, articular membrane inflammation, tendinitis, tonsillitis, ulcerative colitis, uveitis, vaginitis, vasculitis, vulvitis, alopecia areata, erythema multiforme, dermatitis herpetiformis, sclerosis, vitiligo, hypersensitivity vasculitis, urticaria, bullous pemphigoid, pemphigus vulgaris, deciduous pemphigus, paraneoplastic pemphigus, acquired bullous epidermal laxity, acute and chronic gout, chronic gouty arthritis, psoriasis, psoriatic arthritis, rheumatoid arthritis, juvenile rheumatoid arthritis, cryopyrin-associated periodic syndrome or osteoarthritis.

The present invention provides a method for treating or preventing IRAK4-mediated diseases or disorders, comprising administering to a patient in need thereof a therapeutically effective amount of the compound of formula I, formula II, formula III or formula IV, and/or the stereoisomer, enantiomer, diastereomer, deuteride, hydrate, solvate, metabolite and/or prodrug thereof and/or the pharmaceutically acceptable salt thereof, or the pharmaceutical composition thereof.

The present invention provides a method for treating or preventing IRAK4-mediated diseases or disorders, comprising administering to a patient in need thereof a therapeutically effective amount of the compound of formula I, formula II, formula III or formula IV, and/or the stereoisomer, enantiomer, diastereomer, deuteride, hydrate, solvate, metabolite and/or prodrug thereof and/or the pharmaceutically acceptable salt thereof, or the pharmaceutical composition thereof, wherein the IRAK4-mediated diseases or disorders are MyD88-driven diseases or disorders.

The present invention provides a method for treating or preventing diseases or disorders mediated by TLRs (except TLR3R) or the IL-1 receptor family (including IL-1R, IL-18R, IL-33R and IL-36R), comprising administering to a patient in need thereof a therapeutically effective amount of the compound of formula I, formula II, formula III or formula IV, and/or the stereoisomer, enantiomer, diastereomer, deuteride, hydrate, solvate, metabolite and/or prodrug thereof and/or the pharmaceutically acceptable salt thereof, or the pharmaceutical composition thereof.

The present invention provides a method for preparing the compound of formula I, and/or the stereoisomer, enantiomer, diastereomer, deuteride, hydrate, solvate, metabolite and/or prodrug thereof and/or the pharmaceutically acceptable salt thereof,

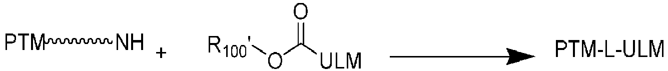

wherein the 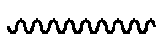 is a portion of linking chain L; the NH may be a primary or secondary amine; the NH group undergoes a substitution reaction with an ester group in 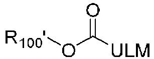 under alkaline conditions to prepare the compound of formula I, wherein the R₁₀₀' is pentafluorophenyl or *p*-nitrophenyl; the PTM is 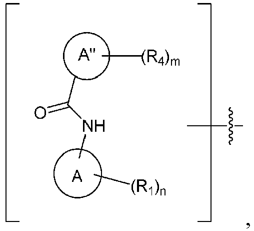 wherein the ring A, ring A", n, R₁, R₄ and m are as defined and described in PTM-1; the ULM is 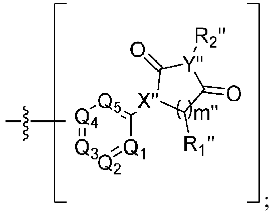 the Q₁, Q₂, Q₃, Q₄, Q₅, X", Y", m", R₁" and R₂" are as defined in the ULM-1 herein; the alkali in the substitution reaction includes, but is not limited to, triethylamine, *N,N*-diisopropylethylamine, potassium carbonate, sodium carbonate and sodium bicarbonate.

The present invention provides a method for preparing the compound of formula II, and/or the stereoisomer, enantiomer, diastereomer, deuteride, hydrate, solvate, metabolite and/or prodrug thereof and/or the pharmaceutically acceptable salt thereof.

The present invention provides a method for preparing the compound of formula IV, and/or the stereoisomer, enantiomer, diastereomer, deuteride, hydrate, solvate, metabolite and/or prodrug thereof and/or the pharmaceutically acceptable salt thereof,

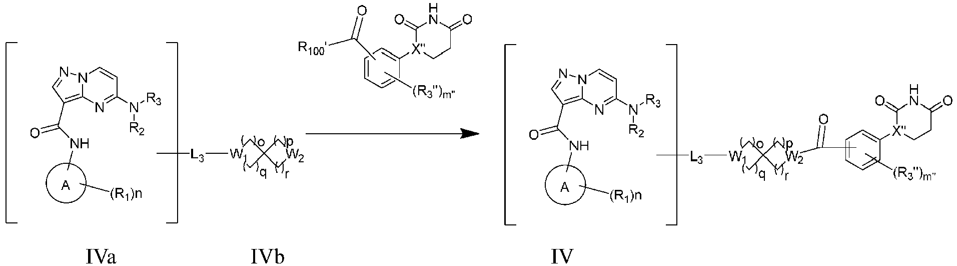

wherein the ring A, n, R₁, R₂ and R₃ are as defined and described in PTM-3; L₃ is as defined and described in LA; W₂ is NH; W₁ is CH or N; o, p, q and r are each independently 1 or 2; X" is CH or N; R3" and m" are as defined and described in ULM-1; R₁₀₀' is pentafluorophenyl or *p*-nitrophenyl; the formula IVa undergoes a substitution reaction with formula IVb to give the compound of formula IV; the alkali in the substitution reaction includes, but is not limited to, triethylamine, *N,N-*diisopropylethylamine, potassium carbonate, sodium carbonate and sodium bicarbonate.

Detailed description: Unless otherwise stated, the following terms used in the specification and claims have the following meanings.

"Alkyl" refers to a saturated aliphatic hydrocarbon group, including linear or branched alkyl; C₁-C₈ alkyl refers to an alkyl group containing 1-8 carbon atoms, e.g., methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, preferably C₁-C₆ alkyl, and further preferably, C₁-C₄ alkyl. The alkyl groups may be substituted or unsubstituted.

"Cycloalkyl" refers to a saturated or partially unsaturated, monocyclic or polycyclic hydrocarbon substituent; "C₃-C₁₂ cycloalkyl" refers to a cycloalkyl group containing 3 to 12 carbon atoms; "C₃-C₈ cycloalkyl" refers to a cycloalkyl group containing 3 to 8 carbon atoms; "C₅-C₁₀ cycloalkyl" refers to a cycloalkyl group containing 5 to 10 carbon atoms.

Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like, preferably cyclopropyl, cyclobutyl, cyclopentyl and cyclohexyl, preferably C₃-C₈ cycloalkyl, and further preferably C₃-C₆ cycloalkyl.

Polycyclic cycloalkyl groups include spiro, fused and bridged cycloalkyl groups. "Spirocycloalkyl" refers to a polycyclic group in which monocyclic rings share a carbon atom (called spiro atom), and they may contain one or more double bonds, but none of the rings has a fully conjugated π-electron system. According to the number of spiro atoms shared among the rings, a spirocycloalkyl group may be a monospirocycloalkyl group, a bispirocycloalkyl group or a polyspirocycloalkyl, preferably a bispirocycloalkyl group. Non-limiting examples of spirocycloalkyl groups include:

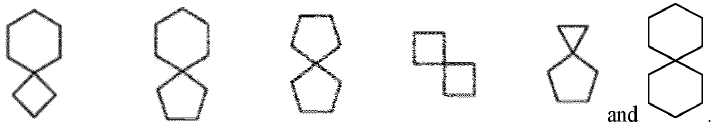

"Fused cycloalkyl" refers to an all-carbon polycyclic group in which each ring shares a pair of adjacent carbon atoms with other rings in the system, wherein one or more of the rings may contain one or more double bonds, but none of the rings has a fully conjugated π-electron system. According to the number of constituent rings, a fused cycloalkyl group may be a bicyclic, tricyclic, tetracyclic or polycyclic fused cycloalkyl group, preferably a bicyclic fused cycloalkyl group. Non-limiting examples of fused cycloalkyl groups include:

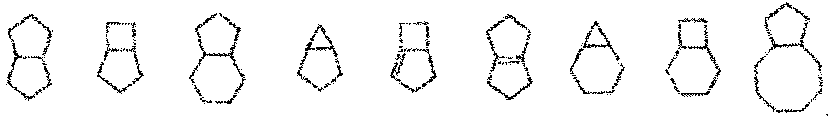

"Bridged cycloalkyl" refers to an all-carbon polycyclic group in which any two rings share two carbon atoms not directly connected, and they may contain one or more double bonds, but none of the rings has a fully conjugated π-electron system. According to the number of constituent rings, a bridged cycloalkyl group may be a bicyclic, tricyclic, tetracyclic or polycyclic bridged cycloalkyl group. Non-limiting examples of bridged cycloalkyl groups include:

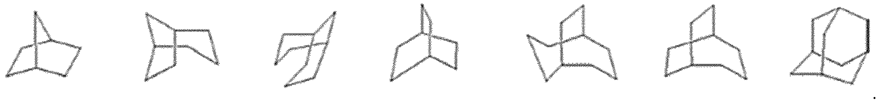

The cycloalkyl ring may be fused to an aryl, heteroaryl or heterocycloalkyl ring, wherein the ring attached to the parent structure is a cycloalkyl group; non-limiting examples include indanyl, tetrahydronaphthyl, benzocycloheptyl, and the like. The cycloalkyl groups may be optionally substituted or unsubstituted.

"Heterocycloalkyl" refers to a saturated or partially unsaturated, monocyclic or polycyclic hydrocarbon substituent, wherein one or more of the ring atoms are selected from nitrogen, oxygen or S(O)ᵣ (wherein r is an integer of 0, 1 or 2), but a ring portion of -O-O-, -O-S- or -S-S- is not included, and the other ring atoms are carbon atoms. "3-12 membered heterocycloalkyl" refers to a cyclic group containing 3 to 12 ring atoms, "5-10 membered heterocycloalkyl" refers to a cyclic group containing 5 to 10 ring atoms, and "3-8 membered heterocycloalkyl" refers to a cyclic group containing 3 to 8 ring atoms. A "3-12 membered heterocycloalkyl" group containing 1-3 heteroatoms selected from N, O or S is preferred. A 3-12 membered heterocycloalkyl group containing 1 or 2 N atoms is further preferred.

Non-limiting examples of monocyclic heterocycloalkyl groups include pyrrolidinyl, piperidinyl, piperazinyl, morpholinyl, thiomorpholinyl, homopiperazinyl and the like, preferably piperidinyl and piperazinyl.

Polycyclic heterocycloalkyl groups include spiro, fused and bridged heterocycloalkyl groups. "Spiroheterocycloalkyl" refers to a polycyclic heterocycloalkyl group in which monocyclic rings share an atom (called spiro atom), wherein one or more of the ring atoms are selected from nitrogen, oxygen or S(O)r (wherein r is an integer of 0, 1 or 2), and the other ring atoms are carbon atoms. They may contain one or more double bonds, but none of the rings has a fully conjugated π-electron system. According to the number of spiro atoms shared among the rings, a spirocycloalkyl group may be a monospiroheterocycloalkyl group, a bispiroheterocycloalkyl group or a polyspiroheterocycloalkyl group, preferably a saturated "3-12 membered bispiroheterocycloalkyl" group containing 1-3 heteroatoms selected from N, O or S, and further preferably a saturated "3-12 membered bispiroheterocycloalkyl" group containing 1 or 2 N atoms. Non-limiting examples of spiroheterocycloalkyl groups include: 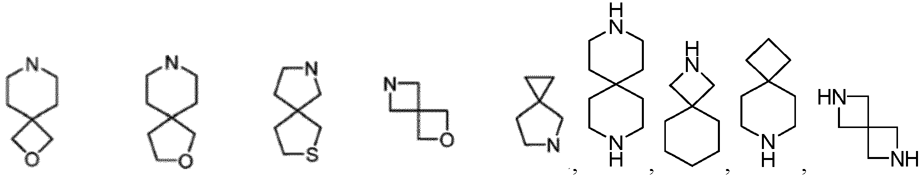 and

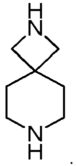

"Fused heterocycloalkyl" refers to a polycyclic heterocycloalkyl group in which each ring shares a pair of adjacent atoms with the other rings in the system, and one or more of the rings may contain one or more double bonds, but none of the rings has a fully conjugated π-electron system, wherein one or more of the ring atoms are selected from nitrogen, oxygen or S(O)r (wherein r is an integer of 0, 1 or 2), and the other ring atoms are carbon atoms. According to the number of constituent rings, a fused heterocycloalkyl group may be a bicyclic, tricyclic, tetracyclic or polycyclic fused heterocycloalkyl group, preferably a "3-12 membered bicyclic fused heterocycloalkyl" group containing 1-3 heteroatoms selected from N, O or S, and further preferably a saturated "3-12 membered bicyclic fused heterocycloalkyl" group containing 1 or 2 N atoms. Non-limiting examples of fused heterocycloalkyl groups include:

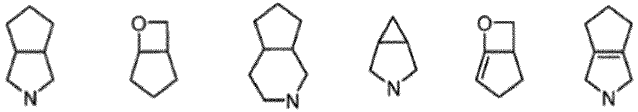

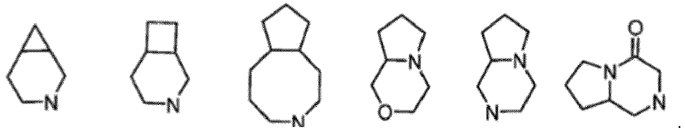

"Bridged heterocycloalkyl" refers to a polycyclic heterocycloalkyl group in which any two rings share two atoms not directly connected, and they may contain one or more double bonds, but none of the rings has a fully conjugated π-electron system, wherein one or more of the ring atoms are selected from nitrogen, oxygen or S(O)ᵣ (wherein r is an integer of 0, 1 or 2), and the other ring atoms are carbon atoms. According to the number of constituent rings, a bridged heterocycloalkyl group may be a bicyclic, tricyclic, tetracyclic or polycyclic bridged cycloalkyl group; non-limiting examples of bridged heterocycloalkyl groups include: 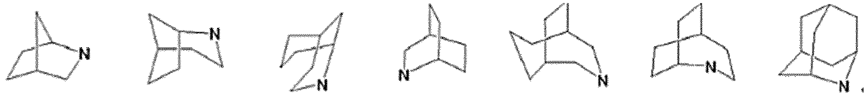 and

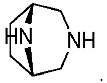

The heterocycloalkyl ring may be fused to an aryl, heteroaryl or cycloalkyl ring, wherein the ring attached to the parent structure is a heterocycloalkyl group; non-limiting examples include: 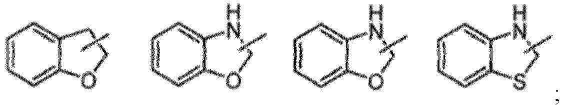 the heterocycloalkyl groups may be optionally substituted or unsubstituted.

"Aryl" refers to an all-carbon monocyclic or fused polycyclic group (i.e., rings that share pairs of adjacent carbon atoms) and a polycyclic group having a conjugated π-electron system; "6-10 membered aryl" refers to an all-carbon aryl group containing 6-10 carbon atoms, such as phenyl and naphthyl, preferably phenyl. The aryl ring may be fused to a heteroaryl, heterocycloalkyl or cycloalkyl ring, wherein the ring attached to the parent structure is an aryl ring; non-limiting examples include: 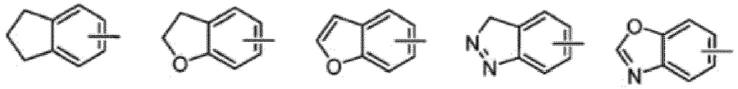 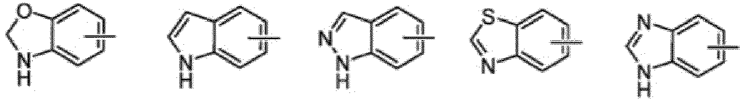 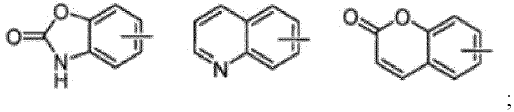 the aryl groups may be optionally substituted or unsubstituted.

"Heteroaryl" refers to a heteroaromatic system containing 1 to 4 heteroatoms, and the heteroatoms include nitrogen, oxygen or S(O)ᵣ (wherein r is an integer of 0, 1 or 2); 5-6 membered heteroaryl refers to a heteroaromatic system containing 5-6 ring atoms; 5-10 membered heteroaryl refers to a heteroaromatic system containing 5-10 ring atoms; a 5-6 membered heteroaryl group is preferred; a 5-6 membered heteroaryl group containing 1 or 2 N atoms is further preferred; non-limiting examples include furanyl, thienyl, pyridyl, pyrrolyl, *N*-alkylpyrrolyl, pyrimidinyl, pyrazinyl, pyrazole, imidazolyl, triazolyl, tetrazolyl and the like, preferably pyridyl. The heteroaryl ring may be fused to an aryl, heterocycloalkyl or cycloalkyl ring, wherein the ring attached to the parent structure is a heteroaryl ring; non-limiting examples include: 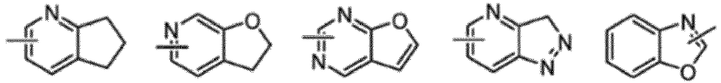 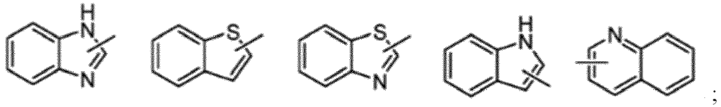 the heteroaryl groups may be optionally substituted or unsubstituted.

"Alkenyl" refers to an alkyl group as defined above, which consists of at least two carbon atoms and at least one carbon-carbon double bond; "C₂₋₈ alkenyl" refers to a linear or branched alkenyl group containing 2-8 carbon atoms, including but not limited to ethenyl, 1-propenyl, 2-propenyl, 1-, 2- or 3-butenyl and the like, preferably a "C₂₋₆ alkenyl" group, and further preferably a "C₂₋₄ alkenyl" group. The alkenyl groups may be substituted or unsubstituted.

"Alkynyl" refers to an alkyl group as defined above, which consists of at least two carbon atoms and at least one carbon-carbon triple bond; "C₂₋₈ alkynyl" refers to a linear or branched alkynyl group containing 2-8 carbon atoms, including but not limited to ethynyl, 1-propynyl, 2-propynyl, 1-, 2- or 3-butynyl, preferably a "C₂₋₆ alkynyl" group, and further preferably a "C₂₋₄ alkynyl" group. The alkynyl groups may be substituted or unsubstituted.

"-ylene" refers to a divalent group. For example, alkylene refers to a divalent alkyl group; alkenylene refers to a divalent alkenyl group; alkynylene refers to a divalent alkynyl group; cycloalkylene refers to a divalent cycloalkyl group; heterocycloalkylene refers to a divalent heterocycloalkyl group; arylene refers to a divalent aryl group; heteroarylene refers to a divalent heteroaryl group; the alkyl, alkenyl, alkynyl, cycloalkyl, heterocycloalkyl, aryl and heteroaryl are as defined above; the "-ylene" groups may be optionally substituted or unsubstituted.

"Alkoxy" refers to -O-(alkyl), wherein the alkyl group is as defined above. "Ci-Cs alkoxy" refers to an alkyloxy group containing 1-8 carbon atoms; non-limiting examples include methoxy, ethoxy, propoxy, butoxy and the like. The alkoxy groups may be substituted or unsubstituted.

"Cycloalkoxy" refers to -O-(unsubstituted cycloalkyl), wherein the cycloalkyl group is as defined above. C₃-C₈ cycloalkoxy refers to a cycloalkyloxy group containing 3-8 carbon atoms; non-limiting examples include cyclopropyloxy, cyclobutyloxy, cyclopentyloxy, cyclohexyloxy and the like. The cycloalkoxy groups may be optionally substituted or unsubstituted.

"Haloalkyl" refers to an alkyl group optionally substituted with one or more fluorine, chlorine, bromine or iodine atoms, wherein the alkyl group is as defined above; non-limiting examples include difluoromethyl, dichloromethyl, dibromomethyl, trifluoromethyl, trichloromethyl, tribromomethyl and the like.

"Haloalkoxy" refers to a group in which the hydrogen on the alkyl group is substituted with one or more fluorine, chlorine, bromine or iodine atoms, wherein the alkoxy group is as defined above. Examples include difluoromethoxy, dichloromethoxy, dibromomethoxy, trifluoromethoxy, trichloromethoxy, tribromomethoxy and the like.

"Hydroxyalkyl" refers to an alkyl group optionally substituted with one or more -OH groups, wherein the alkyl group is as defined above; non-limiting examples include hydroxymethyl, hydroxyethyl, hydroxypropyl and hydroxyisopropyl.

"Aminoalkyl" refers to an alkyl group optionally substituted with one or more -NH₂ groups, wherein the alkyl group is as defined above; non-limiting examples include aminomethyl, aminoethyl, aminopropyl and aminoisopropyl.

"Ammonia" and "amine" are recognized in the art and refer to substituted or unsubstituted ammonia.

"Oxo" refers to the =O group.

"Halogen" refers to fluorine, chlorine, bromine or iodine.

"Cyano" refers to -CN.

"Hydroxy" refers to -OH.

"Raney-Ni" refers to Raney nickel (a hydrogen active catalytic reducing agent).

"HATU" refers to 2-(7-azabenzotriazol-1-yl)-*N*,*N*,*N'*,*N'*-tetramethyluronium hexafluorophosphate.

"DIEA" refers to diisopropylethylamine.

"MeOH" refers to methanol.

"NaBH₃CN" refers to sodium cyanoborohydride.

"LiAlH₄" refers to aluminum lithium hydride.

"THF" refers to tetrahydrofuran.

"DMF" refers to dimethylformamide.

"Py-BOP" refers to benzotriazol-1-yl-oxytripyrrolidinophosphonium hexafluorophosphate.

"HATU" refers to 2-(7-azabenzotriazol-1-yl)-*N*,*N*,*N'*,*N'*-tetramethyluronium hexafluorophosphate.

"TFA" refers to triethylamine.

"EA" refers to ethyl acetate.

"CbzCl" refers to benzyl chloroformate.

"Cs₂CO₃" refers to cesium carbonate.

"NaHCO₃" refers to sodium bicarbonate.

"IBX" refers to 2-iodoxybenzoic acid.

"STAB" refers to sodium triacetoxyborohydride.

"DMSO" refers to dimethyl sulfoxide.

"K₂CO₃" refers to potassium carbonate.

"DCM" refers to dichloromethane.

"KI" refers to potassium iodide.

"NH₄Cl" refers to ammonium chloride.

"NazSOa" refers to sodium sulfate.

"LiBH₄" refers to lithium borohydride.

"NMP" refers to N-methylpyrrolidone.

"*i*-PrOH" refers to isopropanol.

"EtOH" refers to ethanol.

"Pd(OH)₂/C" refers to palladium hydroxide/carbon.

"DCE" refers to 1,2-dichloroethane.

"PE" refers to petroleum ether.

"Sat." refers to a saturated solution.

"PCC" refers to pyridinium chlorochromate.

"CuI" refers to cuprous iodide.

"TsCl" refers to 4-toluenesulfonyl chloride.

"HNO₃" refers to nitric acid.

"NaBH(OAc)₃" refers to sodium triacetoxyborohydride.

"Dess-Martin" refers to (1,1,1-triacetoxy)-1,1-dihydro-1,2-benziodoxol-3(1*H*)-one.

"DMP" refers to dimethyl phthalate.

"TEA" refers to triethylamine.

"DAST" refers to diethylaminosulfur trifluoride.

"NBS" refers to N-bromosuccinimide.

"(Ph)₂NH" refers to diphenylamine.

"Pd₂(dba)₃" refers to tris(dibenzylideneacetone)dipalladium.

"Xantphos" refers to 4,5-bis(diphenylphosphino)-9,9-dimethylxanthene.

"NaOt-Bu" refers to sodium tert-butoxide.

"DPBS" refers to Dulbecco's phosphate-buffered saline.

"PBS" refers to phosphate-buffered saline.

"SDS-PAGE" refers to sodium dodecyl sulfate-polyacrylamide gel electrophoresis.

"PVDF" refers to polyvinylidene fluoride.

"More" independently means 1, 2, 3, 4, 5, 6, 7, 8, 9 or 10.

"Optional" or "optionally" means that the event or circumstance subsequently described may, but does not necessarily, occur, and that the description includes instances where the event or circumstance occurs or does not occur. For example, "a heterocycloalkyl group optionally substituted with alkyl" means that the alkyl may, but does not necessarily, exist, and that the description includes cases where the heterocycloalkyl group is or is not substituted with the alkyl group.

"Substituted" means that one or more, preferably up to 5, further preferably 1-3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when an amino or hydroxy group having free hydrogen is bound to a carbon atom having an unsaturated (such as olefin) bond.

"Pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activity.

The present invention also provides a pharmaceutically acceptable salt of the compound of formula I, formula II, formula III or formula IV The term "pharmaceutically acceptable salt" refers to a relatively nontoxic acid addition salt or base addition salt of the compound of the present invention. The acid addition salt is a salt formed from the compound of formula (I) of the present invention and a suitable inorganic acid or organic acid. These salts may be prepared during the final isolation and purification of the compound or by reacting the purified compound of formula (I) in its free base form with a suitable organic acid or inorganic acid. The representative acid addition salt includes hydrobromide, hydrochloride, sulfate, bisulfate, sulfite, acetate, oxalate, valerate, oleate, palmitate, stearate, laurate, borate, benzoate, lactate, phosphate, biphosphate, carbonate, bicarbonate, toluate, citrate, maleate, fumarate, succinate, tartrate, benzoate, methanesulfonate, p-toluenesulfonate, gluconate, lactobionate, lauryl sulfonate and the like. The base addition salt is a salt formed from the compound of formula (I) and a suitable inorganic base or organic base, including, for example, a salt formed with alkali metal, alkaline earth metal and quaternary ammonium cations, such as sodium salts, lithium salts, potassium salts, calcium salts, magnesium salts, tetramethyl quaternary ammonium salts, tetraethyl quaternary ammonium salts and the like; the amine salt includes salts formed with ammonia (NH₃) and primary, secondary or tertiary amines, such as methylamine salts, dimethylamine salts, trimethylamine salts, triethylamine salts, ethylamine salts and the like.

The compound of the present invention or the pharmaceutically acceptable salt thereof may be administered to mammals including humans, and may be administered orally, rectally, parenterally (intravenously, intramuscularly or subcutaneously), topically (powders, ointments or drops) or intratumorally.

The compound of the present invention may be administered at a dose of about 0.05-300 mg/kg body weight/day, preferably 10-300 mg/kg body weight/day, and preferably 10-150 mg/kg body weight/day.

The compound of the present invention or the pharmaceutically acceptable salt thereof may be formulated into a solid dosage form for oral administration, including but not limited to capsules, tablets, pills, pulvis, granules and the like. In these solid dosage forms, the compound of formula (I) of the present invention, as an active ingredient, is mixed with at least one conventional inert excipient (or carrier), such as sodium citrate or dicalcium phosphate, or with the following ingredients: (1) fillers or extenders, such as starch, lactose, sucrose, glucose, mannitol, silicic acid and the like; (2) binders, such as hydroxymethyl cellulose, alginate, gelatin, polyvinylpyrrolidone, sucrose, acacia and the like; (3) humectants, such as glycerol and the like; (4) disintegrants, such as agar, calcium carbonate, potato or tapioca starch, alginic acid, certain complex silicates, sodium carbonate and the like; (5) solution retarders, such as paraffin and the like; (6) absorption accelerators, such as quaternary ammonium compounds and the like; (7) wetting agents, such as cetyl alcohol, glycerol monostearate and the like; (8) adsorbents, such as kaolin and the like; and (9) lubricants, such as talc, calcium stearate, magnesium stearate, solid polyethylene glycol, sodium lauryl sulfate and the like, or mixtures thereof. Capsules, tablets and pills may further comprise buffers.

The solid dosage forms such as tablets, dragees, capsules, pills and granules may be coated or microencapsulated using coatings and shells such as enteric coatings and other materials well known in the art. They may comprise opacifying agents, and the active ingredient in such a composition may be released in a certain part of the digestive tract in a delayed manner. Examples of embedding components that can be used are polymeric substances and wax-based substances. If necessary, the active ingredient can also be in microcapsule form with one or more of the above-mentioned excipients.

The compound of the present invention or the pharmaceutically acceptable salt thereof may be formulated into a liquid dosage form for oral administration, including but not limited to pharmaceutically acceptable emulsions, solutions, suspensions, syrups, tinctures and the like. In addition to the compound of formula (I) or the pharmaceutically acceptable salt thereof as an active ingredient, the liquid dosage form may comprise inert diluents commonly used in the art, such as water and other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil, sesame oil and the like, or mixtures of these substances. In addition to such inert diluents, the liquid dosage form in the present invention may further comprise conventional adjuvants, such as wetting agents, emulsifiers, suspending agents, sweeteners, flavoring agents, perfuming agents and the like.

The suspending agents include, for example, ethoxylated isostearyl alcohols, polyoxyethylene sorbitol and sorbitan esters, microcrystalline cellulose, aluminum methylate, agar and the like, or mixtures of these substances.

The compound of the present invention or the pharmaceutically acceptable salt thereof may be formulated into a dosage form for parenteral injection, including but not limited to physiologically acceptable sterile aqueous or anhydrous solutions, dispersions, suspensions or emulsions, and sterile powders for redissolving into sterile injectable solutions or dispersions. Suitable carriers, diluents, solvents or excipients include water, ethanol, polyols and suitable mixtures thereof.

The compound of the present invention or the pharmaceutically acceptable salt thereof may also be formulated into a dosage form for topical administration, including, for example, ointments, pulvis, suppositories, drops, sprays, inhalants and the like. The compound of formula (I) or the pharmaceutically acceptable salt thereof in the present invention as an active ingredient is mixed under sterile conditions with a physiologically acceptable carrier and optional preservatives, buffers or propellants that may be required if necessary.

The present invention also provides a pharmaceutical composition comprising the compound of formula (I) or the pharmaceutically acceptable salt thereof as an active ingredient, and a pharmaceutically acceptable carrier, excipient or diluent. In the preparation of the pharmaceutical composition, the compound of formula (I) or the pharmaceutically acceptable salt thereof in the present invention is typically mixed with the pharmaceutically acceptable carrier, excipient or diluent.

The composition in the present invention may be formulated into a conventional pharmaceutical formulation according to a conventional preparation method. For example, tablets, pills, capsules, pulvis, granules, emulsions, suspensions, dispersions, solutions, syrups, elixirs, ointments, drops, suppositories, inhalants, sprays and the like.

The compound or the pharmaceutically acceptable salt thereof described in the present invention may be administered alone or, if desired, in combination with other pharmaceutically acceptable therapeutic agents, such as other anti-tumor drugs, anti-inflammatory drugs or autoimmune drugs. The ingredients to be combined may be administered simultaneously or sequentially, and administered in a single formulation or in different formulations. The combination may include not only a combination of the compound in the present invention and one additional active agent, but also a combination of the compound in the present invention and two or more additional active agents.

The present invention uses an IRAK4 kinase activity test experiment to prove that the compounds of the present invention can effectively bind to the IRAK4 target protein or produce inhibitory effects; uses Western-Blot to prove that the compounds of the present invention can effectively specifically degrade the IRAK4 protein in THP-1 cells; uses an LPS-induced normal human whole blood PBMC-secreted cytokine concentration determination experiment to prove that the compounds of the present invention can effectively inhibit the production of immune cell IL-6; and uses Western-Blot to prove that the compounds of the present invention have significant degradation effects on both the IRAK4 long chain (IRAK4-L) and the IRAK4 short chain (IRAK4-S) in human PBMCs. The compound of formula I, formula II, formula III or formula IV, and/or the stereoisomer, enantiomer, diastereomer, deuteride, hydrate, solvate, metabolite and/or prodrug thereof and/or the pharmaceutically acceptable salt thereof can effectively degrade the IRAK4 protein, thereby achieving a preventing or treating effect on IRAK4-related diseases or disorders.

### DETAILED DESCRIPTION

The present invention will be further explained in detail below with reference to examples, which are not intended to limit the present invention, and the present invention is not merely limited to the contents of the examples. Starting materials in the examples of the present invention are known and commercially available, or may be synthesized by using or according to methods known in the art. Unless otherwise stated, experimental procedures without specific conditions indicated in the examples of the present invention are generally conducted according to conventional conditions, or according to conditions recommended by the manufacturers of the starting materials or commercial products.

### I. Compound preparation examples

### Intermediate 1: 3-(2,4-dioxotetrahydropyrimidin-1(2H)-yl)-4-methoxybenzoic acid

### Step 1: preparation of 3-(2,4-dioxotetrahydropyrimidin-1(2H)-yl)-4-methoxybenzoic acid

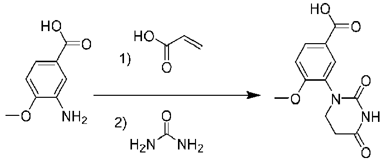

3-Amino-4-methoxybenzoic acid (5.0 g, 2.93 mmol) was suspended in acrylic acid (8.05 mL, 117 mmol), and the suspension was stirred at 100 °C for 3 h. The reaction liquid was then cooled to room temperature with stirring. Acetic acid (33 mL) was added, and the stirred suspension was heated at 100 °C for 10 min. Urea (11.00 g, 183 mmol) was added, and the reaction liquid was stirred overnight at 120 °C. The reaction liquid was added to a mixture of ice water and concentrated hydrochloric acid (37%), and the mixture was stirred. The resulting suspension was stored in a refrigerator at 5 °C overnight and then filtered, and the solid was washed with water and dried to give a solid. The solid was triturated in a hydrochloric acid solution (0.05 M), filtered, washed with methyl *tert*-butyl ether, and dried under reduced pressure at 40 °C to give the target product, 3-(2,4-dioxotetrahydropyrimidin-1(2*H*)-yl)-4-methoxybenzoic acid.

1H NMR (400 MHz, DMSO-*d*₆) δ 12.70 (s, 1H), 10.34 (s, 1H), 7.92 (dd, *J* = 8.6, 2.2 Hz, 1H), 7.83 (d, *J* = 2.2 Hz, 1H), 7.21 (d, *J* = 8.8 Hz, 1H), 3.94-3.82 (m, 3H), 3.60 (t, *J* = 6.7 Hz, 2H), 2.69 (s, 2H).

The following intermediates were prepared by referring to the method for intermediate 1:

| Intermediate | Structure | Name | LC-MS |
|---|---|---|---|
| Intermediate 2 | 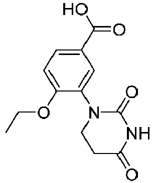 | 3-(2,4-dioxotetrahydropyrimidin-1(2*H*)-yl)-4-ethoxybenzoic acid | (ESI, *m*/*z*): [M+H]⁺ = 279.1 |
| Intermediate 3 | 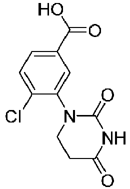 | 4-chloro-3-(2,4-dioxotetrahydropyrimidin-1(2*H*)-yl)benzoic acid | (ESI, *m*/*z*): [M+H]⁺ = 269.0 |
| Intermediate 4 | 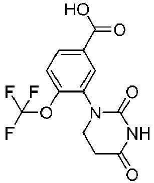 | 3-(2,4-dioxotetrahydropyrimidin-1(2*H*)-yl)-4-(trifluoromethoxy)benzoic acid | (ESI, *m*/*z*): [M+H]⁺ = 319.1 |
| Intermediate 5 | 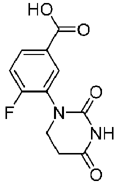 | 3-(2,4-dioxotetrahydropyrimidin-1(2*H*)-yl)-4-fluorobenzoic acid | (ESI, *m*/*z*): [M+H]⁺ = 253.0 |
| Intermediate 6 | 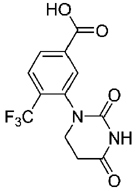 | 3-(2,4-dioxotetrahydropyrimidin-1(2*H*)-yl)-4-(trifluoromethyl)benzoic acid | (ESI, *m*/*z):* [M+H]⁺ = 303.0 |
| Intermediate 7 | 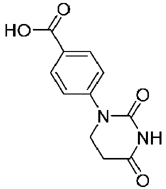 | 4-(2,4-dioxotetrahydropyrimidin-1(2*H*)-yl)benzoic acid | (ESI, *m*/*z*): [M+H]⁺ = 235.1 |
| Intermediate 35 | 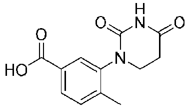 | 3-(2,4-dioxotetrahydropyrimidin-1(2*H*)-yl)-4-methylbenzoic acid | (ESI, *m*/*z*): [M+H]⁺ = 249.1 |

### Intermediate 8: 3-(2,6-dioxopiperidin-3-yl)-4-fluorobenzoic acid

### Step 1: preparation of 2,6-bis(benzyloxy)pyridine

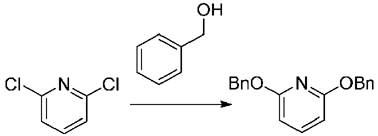

Benzyl alcohol (109.6 g, 1.01 mol) was added to a solution of sodium hydride (60% in mineral oil, 54.0 g, 1.35 mol) in *N,N*-dimethylformamide (600 mL) at 0 °C. After 30 min of stirring, 2,6-dichloropyridine (50.0 g, 0.338 mol) was then added. The reaction mixture was heated to 80 °C and stirred overnight. After the reaction mixture was cooled to room temperature, it was quenched with ice water (1000 mL). The solid was collected by filtration and dried to give the target product, 2,6-bis(benzyloxy)pyridine.

¹H NMR (DMSO-*d*₆, 400 MHz): *δ* 7.68 (t, *J* = 8.4 Hz, 1H), 7.48-7.36 (m, 10H), 6.45 (d, *J* = 8.4 Hz, 2H), 5.38 (s, 4H).

LC-MS: (ESI, *m*/*z):* [M+H]⁺ = 292.2.

### Step 2: preparation of 2,6-bis(benzyloxy)-3-bromopyridine

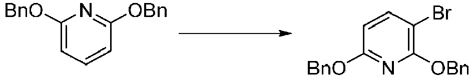

*N*-Bromosuccinimide (41.7 g, 0.23 mol) was added to a solution of 2,6-bis(benzyloxy)pyridine (75.9 g, 0.26 mol) in acetonitrile (600 mL). The reaction mixture was stirred at 80 °C for 2 h. After the reaction was complete, the reaction mixture was concentrated under reduced pressure. The concentrate was diluted with ethyl acetate (500 mL) and washed with water and saturated brine. The organic layer was collected, dried over anhydrous sodium sulfate, filtered, and concentrated. The concentrate was purified by silica gel column chromatography to give the target product, 2,6-bis(benzyloxy)-3-bromopyridine.

¹H NMR (DMSO-*d*₆, 400 MHz): *δ* 7.88 (d, *J* = 8.4 Hz, 1H), 7.45-7.37 (m, 10H), 6.44 (d, *J* = 8.4 Hz, 1H), 5.42 (s, 2H), 5.33 (s, 2H).

LC-MS: (ESI, *m*/*z*): [M+H]⁺ = 372.1.

### Step 3: preparation of 3-(2,6-bis(benzyloxy)pyridin-3-yl)-4-fluorobenzoic acid

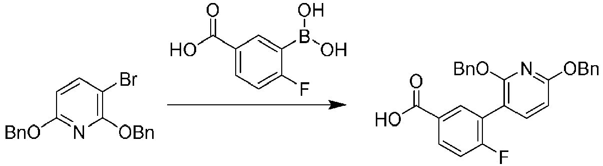

The PdCl₂(dppf)-chloroform complex (612 mg, 0.84 mmol) and sodium carbonate (1.77 g, 16.74 mmol) were added to a mixture of 2,6-bis(benzyloxy)-3-bromopyridine (3.1 g, 8.37 mmol) and 3-borono-4-fluorobenzoic acid (1.85 g, 10.05 mmol) in dioxane (31 mL) and water (7.5 mL). The reaction mixture was heated at 90 °C for 16 h under nitrogen atmosphere. After the reaction was complete, 15% hydrochloric acid solution (20 mL) was added. The mixture was extracted with ethyl acetate (100 mL × 3), washed with saturated brine (50 mL), dried over anhydrous sodium sulfate, and then concentrated under reduced pressure, and the concentrate was purified by silica gel column chromatography to give the target product, 3-(2,6-bis(benzyloxy)pyridin-3-yl)-4-fluorobenzoic acid.

LC-MS: (ESI, *m*/*z*): [M+H]⁺ = 430.2

### Step 4: preparation of 3-(2,6-dioxopiperidin-3-yl)-4-fluorobenzoic acid

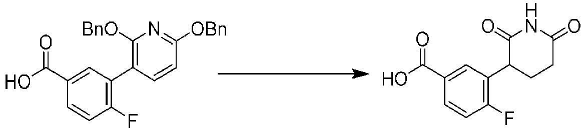

Palladium/carbon (10%, 120 mg) was added to a solution of 3-(2,6-bis(benzyloxy)pyridin-3-yl)-4-fluorobenzoic acid (1.50 g, 3.49 mmol) in methanol (10 mL). The reaction mixture was stirred at room temperature for 48 h under hydrogen atmosphere. After the reaction was complete, the reaction mixture was filtered through celite, and the filtrate was concentrated under reduced pressure. The concentrate was purified by column chromatography to give the target product, 3-(2,6-dioxopiperidine-3-yl)-4-fluorobenzoic acid.

LC-MS: (ESI, *m*/*z*): [M+H]⁺ = 252.1

¹H NMR (400 MHz, DMSO-*d*₆) δ 13.04 (s, 1H), 10.92 (s, 1H), 7.91-7.95 (m, 2H), 7.30-7.34 (m, 1H), 4.15-4.19 (m, 1H), 2.70-2.79 (m, 1H), 2.53-2.57 (m, 1H), 2.23-2.27 (m, 1H), 2.01-2.07 (m, 1H).

### Intermediate 9: pentafluorophenyl 3-(2,4-dioxotetrahydropyrimidin-1(2H)-yl)-4-methoxybenzoate

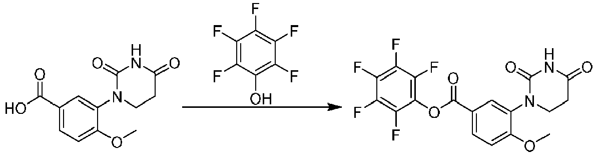

A mixture of 3-(2,4-dioxotetrahydropyrimidin-1(2*H*)-yl)-4-methoxybenzoic acid (2.0 g, 7.58 mmol), 2,3,4,5,6-pentafluorophenol (1.67 g, 9.09 mmol) and *N,N'*-dicyclohexylcarbodiimide (1.87 g, 9.09 mmol) in *N,N*-dimethylformamide (20 mL) was stirred at room temperature for 3 h. The reaction liquid was poured into water (200 mL), and the resulting solution was stirred for 0.5 h and extracted with ethyl acetate (200 mL × 3). The organic phase was collected, washed with water (500 mL × 2) and saturated brine (300 mL), dried over anhydrous sodium sulfate, filtered, and concentrated. The concentrate was purified by silica gel column chromatography to give the target product, pentafluorophenyl 3-(2,4-dioxotetrahydropyrimidin-1(2*H*)-yl)-4-methoxybenzoate.

LC-MS: (ESI, *m*/*z):* [M+H]⁺ = 431.1

The following intermediates were prepared by referring to the method for intermediate 9:

| Intermediate | Structure | Name | LC-MS |
|---|---|---|---|
| Intermediate 10 | 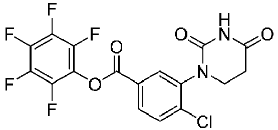 | pentafluorophenyl 4-chloro-3-(2,4-dioxotetrahydropyrimidin-1(2*H*)-yl)benzoate | (ESI, *m*/*z*): [M+H]¹ = 435.0 |
| Intermediate 11 | 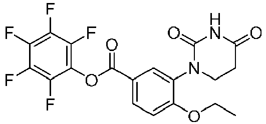 | pentafluorophenyl 3-(2,4-dioxotetrahydropyrimidin-1(2*H*)-yl)-4-ethoxybenzoate | (ESI, *m*/*z*): [M+H]¹ = 445.1 |
| Intermediate 12 | 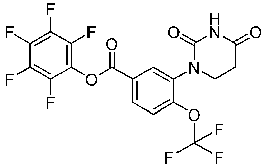 | pentafluorophenyl 3-(2,4-dioxotetrahydropyrimidin-1(2*H*)-yl)-4-(trifluoromethoxy)benzoate | (ESI, *m*/*z*): [M+H]⁺ = 485.0 |
| Intermediate 13 | 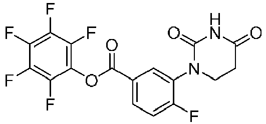 | pentafluorophenyl 3-(2,4-dioxotetrahydropyrimidin-1(2*H*)-yl)-4-fluorobenzoate | (ESI, *m*/*z*): [M+H]⁺ = 419.0 |
| Intermediate 14 | 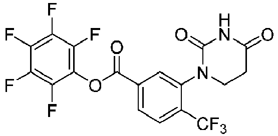 | pentafluorophenyl 3-(2,4-dioxotetrahydropyrimidin-1(2*H*)-yl)-4-(trifluoromethyl)benzoate | (ESI, *m*/*z*): [M+H]⁺ = 469.0 |
| Intermediate 15 | 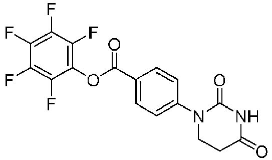 | pentafluorophenyl 4-(2,4-dioxotetrahydropyrimidin-1(2*H*)-yl)benzoate | (ESI, *m*/*z*): [M+H]¹ = 401.0 |
| Intermediate 16 | 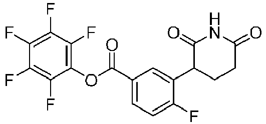 | pentafluorophenyl 3-(2,6-dioxopiperidin-3-yl)-4-fluorobenzoate | (ESI, *m*/*z*): [M+H]⁺ = 418.0 |
| Intermediate 36 | 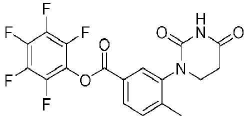 | pentafluorophenyl 3-(2,4-dioxotetrahydropyrimidin-1(2*H*)-yl)-4-methylbenzoate | (ESI, *m*/*z*): [M+H]⁺ = 415.0 |

### Intermediate 17: 5-((1R,4R)-2-oxa-5-azabicyclo[2.2.1]heptan-5-yl)pyrazolo[1,5-a]pyrimidine-3-carboxylic acid

### Step 1: preparation of ethyl 5-((1R,4R)-2-oxa-5-azabicyclo[2.2.1]heptan-5-yl)pyrazolo[1,5-a]pyrimidine-3-carboxylate

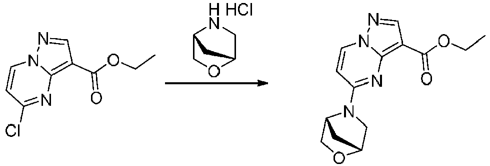

Ethyl 5-chloropyrazolo[1,5-*a*]pyrimidine-3-carboxylate (5.0 g, 22.1 mmol) and (1*R*,4*R*)-2-oxa-5-azabicyclo[2.2.1]heptane hydrochloride (3.0 g, 22.1 mmol) were dissolved in acetonitrile (60 mL), and *N,N-*diisopropylethylamine (11.4 g, 88.5 mmol) was added. The mixture was stirred at 60 °C for 2 h and concentrated by rotary evaporation under reduced pressure to remove the solvent, and water (100 mL) was then added. The mixture was extracted with ethyl acetate (100 mL × 3), and the organic phase was washed with saturated brine (100 mL), dried over anhydrous sodium sulfate, filtered, and concentrated under reduced pressure to give the target product (crude), ethyl 5-((1*R*,4*R*)-2-oxa-5-azabicyclo[2.2.1]heptan-5-yl)pyrazolo[1,5-*a*]pyrimidine-3-carboxylate.

LC-MS: (ESI, *m*/*z):* [M+H]⁺ = 289.0.

### Step 2: preparation of 5-((1R,4R)-2-oxa-5-azabicyclo[2.2.1]heptan-5-yl)pyrazolo[1,5-a]pyrimidine-3-carboxylic acid

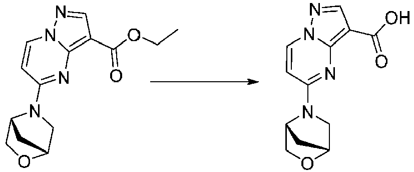

Ethyl 5-((1*R*,4*R*)-2-oxa-5-azabicyclo[2.2.1]heptan-5-yl)pyrazolo[1,5-*a*]pyrimidine-3-carboxylate (5.5 g, 19.0 mmol) was added to ethanol (50 mL) and tetrahydrofuran (25 mL), and a saturated aqueous solution of lithium hydroxide (an aqueous solution prepared from 912 mg of lithium hydroxide and 0.75 mL of water) was added. The reaction mixture was stirred overnight at room temperature and concentrated under reduced pressure, and the concentrate was directly purified by reversed-phase chromatography (0.05% formic acid, 15% acetonitrile) to give 5-((1*R*,4*R*)-2-oxa-5-azabicyclo[2.2.1]heptan-5-yl)pyrazolo[1,5-*a*]pyrimidine-3-carboxylic acid.

LC-MS: (ESI, *m*/*z*): [M+H]⁺ = 261.0.

¹H NMR (400 MHz, DMSO-*d*₆) δ 11.67 (s, 1H), 8.76 (dd, *J* = 7.6, 3.8 Hz, 1H), 8.22 (d, *J* = 3.8 Hz, 1H), 6.90-6.30 (m, 1H), 5.30-5.00 (m, 1H), 4.85-4.74 (m, 1H), 3.93-3.70 (m, 2H), 3.65-3.50 (m, 2H), 1.90-2.05 (m, 2H).

### Intermediate 18: 5-((1R,4R)-2-oxa-5-azabicyclo[2.2.1]heptan-5-yl)-N-(3-(difluoromethyl)-1-(piperidin-4-yl)-1H-pyrazol-4-yl)pyrazolo[1,5-a]pyrimidine-3-carboxamide

### Step 1: preparation of benzyl 4-(tosyloxy)piperidine-1-carboxylate

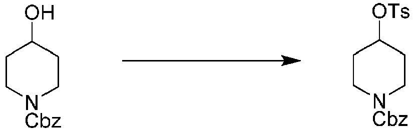

A mixture of benzyl 4-hydroxypiperidine-1-carboxylate (8.0 g, 34 mmol), *p*-toluenesulfonyl chloride (7.8 g, 40.1 mmol), triethylamine (10.3 g, 102 mmol) and 4-dimethylaminopyridine (0.41 g, 3.4 mmol) in dichloromethane (100 mL) was stirred overnight at room temperature. The reaction mixture was diluted with water (100 mL) and extracted with dichloromethane (100 mL × 3). The organic layer was collected, washed with saturated brine (200 mL), dried over anhydrous sodium sulfate, filtered, and concentrated. The concentrate was purified by column chromatography to give the target product, benzyl 4-(tosyloxy)piperidine-1-carboxylate.

LC-MS: (ESI, m/z): [M+Na]⁺ = 412.1.

### Step 2: preparation of benzyl 4-(3-formyl-1H-pyrrol-1-yl)piperidine-1-carboxylate

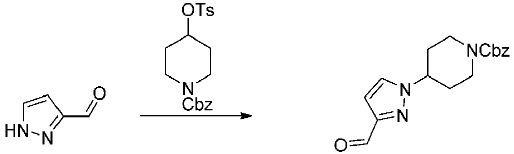

A mixture of 1*H*-pyrazole-3-carbaldehyde (2.5 g, 26 mmol), benzyl 4-(tosyloxy)piperidine-1-carboxylate (12.5 g, 32.1 mmol), cesium carbonate (25.4 g, 78 mmol) and potassium iodide (5.33 g, 32.1 mmol) in *N*,*N*-dimethylformamide (50 mL) was stirred overnight at 50 °C. The reaction mixture was diluted with water (250 mL) and extracted with ethyl acetate (250 mL × 3). The organic layer was collected, washed with saturated brine (500 mL), dried over anhydrous sodium sulfate, filtered, and concentrated. The concentrate was purified by column chromatography to give the target product, benzyl 4-(3-formyl-1*H*-pyrrol-1-yl)piperidine-1-carboxylate.

LC-MS: (ESI, *m*/*z):* [M+Na]^{I} = 336.3.

### Step 3: preparation of benzyl 4-(3-(difluoromethyl)-1H-pyrazol-1-yl)piperidine-1-carboxylate

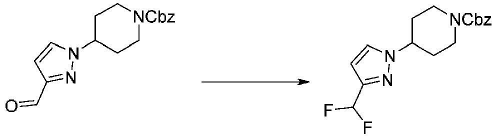

Diethylaminosulfur trifluoride (4.62 g, 28.7 mmol) was added to a mixture of benzyl 4-(3-formyl-1*H*-pyrrol-1-yl)piperidine-1-carboxylate (1.5 g, 4.8 mmol) in dichloromethane (50 mL) at 0 °C. The reaction mixture was stirred overnight at room temperature. The reaction mixture was concentrated under reduced pressure, and the concentrate was purified by column chromatography to give the target product, benzyl 4-(3-(difluoromethyl)-1*H-*pyrazol-1-yl)piperidine-1-carboxylate.

LC-MS: (ESI, *m*/*z):* [M+H]⁺ = 336.1.

### Step 4: preparation of 4-(3-(difluoromethyl)-4-nitro-1H-pyrazol-1-yl)piperidine

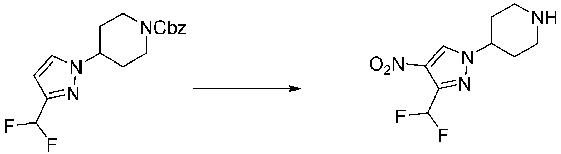

Nitric acid (1 mL) was added to a mixture of benzyl 4-(3-(difluoromethyl)-1*H*-pyrazol-1-yl)piperidine-1-carboxylate (1.0 g, 3.0 mmol) in sulfuric acid (5 mL) at 0 °C. The reaction mixture was stirred overnight at room temperature. The reaction mixture was poured into ice water (30 mL) and extracted with methanol/dichloromethane (10%, 50 mL × 3). The organic layer was collected, washed with saturated brine (100 mL), dried over anhydrous sodium sulfate, filtered, and concentrated under reduced pressure to give the target product, 4-(3-(difluoromethyl)-4-nitro-1*H*-pyrazol-1-yl)piperidine. The resulting crude product was directly used in the next step.

LC-MS: (ESI, *m*/*z):* [M+H]⁺ = 247.1.

### Step 5: preparation of tert-butyl 4-(3-(difluoromethyl)-4-nitro-1H-pyrazol-1-yl)piperidine-1-carboxylate

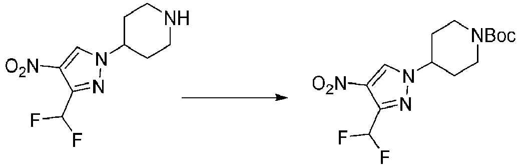

A mixture of 4-(3-(difluoromethyl)-4-nitro-1*H*-pyrazol-1-yl)piperidine (650 mg, 2.6 mmol), *di-tert-*butyl dicarbonate (680 mg, 3.1 mmol), triethylamine (788 mg, 7.8 mmol) and 4-dimethylaminopyridine (32 mg, 0.26 mmol) in dichloromethane (10 mL) was stirred overnight at room temperature. The reaction mixture was diluted with water (20 mL) and extracted with dichloromethane (20 mL × 3). The organic layer was collected, washed with saturated brine (50 mL), dried over anhydrous sodium sulfate, filtered, and concentrated. The concentrate was purified by chromatography to give the target product, *tert*-butyl 4-(3-(difluoromethyl)-4-nitro-1*H*-pyrazol-1-yl)piperidine-1-carboxylate.

LC-MS: (ESI, *m*/*z*): [M-57+2H]⁺ = 291.1.

### Step 6: preparation of tert-butyl 4-(4-amino-3-(difluoromethyl)-1H-pyrazol-1-yl)piperidine-1-carboxylate

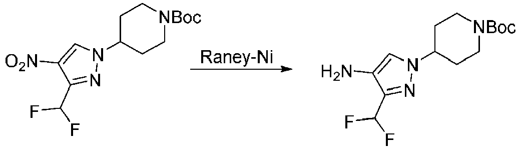

Raney-Ni (70 mg) and hydrazine hydrate (85%, 1.0 mL) were added to a solution of tert-butyl 4-(3-(difluoromethyl)-4-nitro-1*H*-pyrazol-1-yl)piperidine-1-carboxylate (710 mg, 2.052 mmol) in ethanol (20 mL). The reaction mixture was stirred at room temperature for 2 h and filtered, and the filtrate was concentrated under reduced pressure to give the target product, *tert*-butyl 4-(4-amino-3-(difluoromethyl)-1*H*-pyrazol-1-yl)piperidine-1-carboxylate. The resulting crude product was used in the next step without further purification.

LC-MS: (ESI, *m*/*z*): [M-57+2H]⁺ = 261.3.

### Step 7: preparation of tert-butyl 4-(4-(5-((1R,4R)-2-oxa-5-aza[2.2.1]heptan-5-yl)pyrazolo[1,5-α]pyrimidine-3-carboxamido)-3-(difluoromethyl)-1H-pyrazol-1-yl)piperidine-1-carboxylate

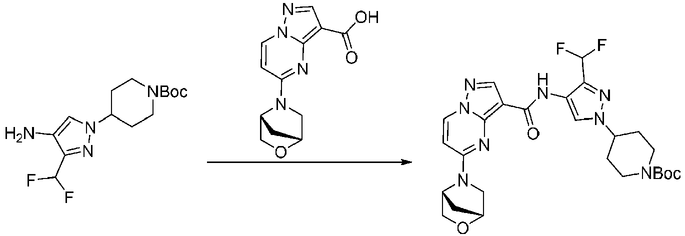

5-((1*R*,4*R*)-2-Oxa-5-azabicyclo[2.2.1]heptan-5-yl)pyrazolo[1,5-*a*]pyrimidine-3-carboxylic acid (461 mg, 1.77 mmol), HATU (874 mg, 2.3 mmol) and *N,N-*diisopropylethylamine (685 mg, 5.1 mmol) were added to a mixture of tert-butyl 4-(4-amino-3-(difluoromethyl)-1*H*-pyrazol-1-yl)piperidine-1-carboxylate (560 mg, 1.77 mmol) in *N,N*-dimethylformamide (5 mL). The reaction mixture was stirred at room temperature for 2 h. The reaction mixture was quenched with water (100 mL). The resulting solution was extracted with ethyl acetate (50 mL × 3). The organic layer was collected, washed with saturated brine (150 mL), dried over anhydrous sodium sulfate, filtered, and concentrated. The concentrate was purified by column chromatography to give the target product, *tert*-butyl 4-(4-(5-((1*R*,4*R*)-2-oxa-5-aza[2.2.1]heptan-5-yl)pyrazolo[1,5-*a*]pyrimidine-3-carboxamido)-3-(diffuoromethyl)-1*H*-pyrazol-1-yl)piperidine-1-carboxylate.

LC-MS: (ESI, *m*/*z*): [M+H]⁺ = 559.2.

### Step 8: preparation of 5-((1R,4R)-2-oxa-5-azabicyclo[2.2.1]heptan-5-yl)-N-(3-(difluoromethyl)-1-(piperidin-4-yl)-1H-pyrazol-4-yl)pyrazolo[1,5-a]pyrimidine-3-carboxamide

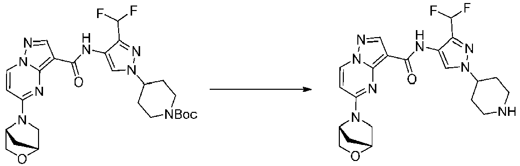

A solution of *tert*-butyl 4-(4-(5-((1*R*,4*R*)-2-oxa-5-aza[2.2.1]heptan-5-yl)pyrazolo[1,5-*a*]pyrimidine-3-carboxamido)-3-(difluoromethyl)-1*H*-pyraaol-1-yl)piperidine-1-carboxylate (540 mg, 0.97 mmol) in trifluoroacetic acid/dichloromethane (8 mL, 1:3) was stirred at room temperature for 1 h. The reaction mixture was concentrated under reduced pressure to give a crude product of 5-((1*R*,4*R*)-2-oxa-5-azabicyclo[2.2.1]heptan-5-yl)-*N*-(3-(difluoromethyl)-1-(piperidin-4-yl)-1*H-*pyrazol-4-yl)pyrazolo[1,5-*a*]pyrimidine-3-carboxamide. The crude product was directly used in the next step.

LC-MS: (ESI, *m*/*z*): [M+H]⁺ = 459.2.

### Intermediate 19: N-(1-(1-((3-azaspiro[5.5]undecan-9-yl)methyl)piperidin-4-yl)-3-(difluoromethyl)-1H-pyrazol-4-yl)-5-((1R,4R)-2-oxa-5-azabicyclo[2.2.1]heptan-5-yl)pyrazolo[1,5-a]pyrimidine-3-carboxamide

### Step 1: preparation of tert-butyl 9-((4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)methyl)-3-azaspiro [5.5]undecane-3-carboxylate

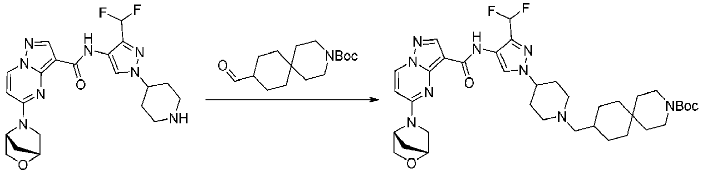

*N,N*-Diisopropylethylamine (0.5 mL) and *tert*-butyl 9-formyl-3-azaspiro[5.5]undecane-3-carboxylate (94 mg, 0.334 mmol) were added to a solution of 5-((1*R*,4*R*)-2-oxa-5-azabicyclo[2.2.1]heptan-5-yl)-*N-*(3-(difluoromethyl)-1-(piperidin-4-yl)-1*H*-pyrazol-4-yl)pyrazolo[1,5-*a*]pyrimidine-3-carboxamide (200 mg, crude, 0.334 mmol) in methanol (10 mL). After the mixture was stirred for 30 min, sodium cyanoborohydride (106 mg, 1.67 mmol) was added, and the reaction mixture was stirred at room temperature for 3 h. The resulting mixture was diluted with water (30 mL) and extracted with ethyl acetate (30 mL × 3). The organic layer was collected, washed with saturated brine (50 mL), dried over anhydrous sodium sulfate, filtered, and concentrated. The concentrate was purified by column chromatography to give the target product, *tert-butyl* 9-((4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate.

LC-MS: (ESI, *m*/*z*): [M+H]⁺ = 724.4.

### Step 2: preparation of N-(1-(1-((3-azaspiro[5.5]undecan-9-yl)methyl)piperidin-4-yl)-3-(difluoromethyl)-1H-pyrazol-4-yl)-5-((1R,4R)-2-oxa-5-azabicyclo[2.2.1]heptan-5-yl)pyrazolo[1,5-a]pyrimidine-3-carboxamide

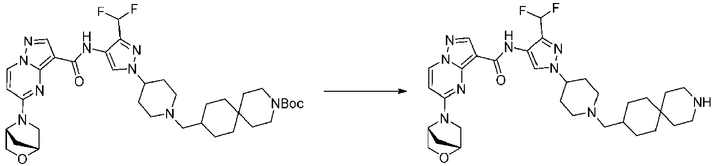

A mixture of *tert*-butyl 9-((4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate (130 mg, 0.18 mmol) in trifluoroacetic acid/dichloromethane (6 mL, 1:2) was stirred at room temperature for 1 h. The reaction mixture was concentrated under reduced pressure to give a crude product of *N*-(1-(1-((3-azaspiro[5.5]undecan-9-yl)methyl)piperidin-4-yl)-3-(difluoromethyl)-1*H-*pyrazol-4-yl)-5-((1*R*,4*R*)-2-oxa-5-azabicyclo[2.2.1]heptan-5-yl)pyrazolo[1,5-*a*]pyrimidine-3-carboxamide. The crude product was directly used in the next step.

LC-MS: (ESI, *m*/*z*): [M+H]⁺ = 624.4.

### Intermediate 20: N-(1-((1R,4R)-4-((3,9-diazaspiro[5.5]undecan-3-yl)methyl)cyclohexyl)-3-(difluoromethyl)-1H-pyrazol-4-yl)-5-((1R,4R)-2-oxa-5-aaabicyclo[2.2.1]heptan-5-yl)pyraaolo[1,5-a]pyrimidine-3-carboxamide

### Step 1: preparation of tert-butyl 9-(((1R,4R)-4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)cyclohexyl)methyl)-3,9-diazaspiro[5.5]undecane-3-carboxylate

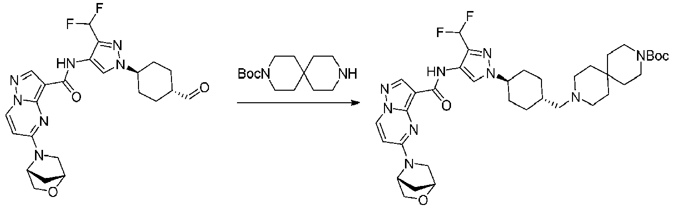

A reaction mixture of 5-((1R,4R)-2-oxa-5-azabicyclo[2.2.1]heptan-5-yl)-*N*-(3-(difluoromethyl)-1-((1*R*,4*R*)-4-formylcyclohexyl)-1*H*-pyrazol-4-yl)pyrazolo[1,5-*a*]pyrimidine-3-carboxamide (150 mg, 0.31 mmol), *tert*-butyl 3,9-diazaspiro[5.5]undecane-3-carboxylate (86 mg, 0.34 mmol) and sodium triacetoxyborohydride (326 mg, 1.55 mmol) in tetrahydrofuran (15 mL) was stirred overnight at room temperature. The reaction mixture was quenched with water (50 mL). The resulting solution was extracted with dichloromethane (20 mL × 3). The organic layer was collected, washed with saturated brine (60 mL), dried over anhydrous sodium sulfate, filtered, and concentrated. The concentrate was purified by column chromatography to give *tert*-butyl 9-(((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)cyclohexyl)methyl)-3,9-diazaspiro[5.5]undecane-3-carboxylate.

LC-MS: (ESI, *m*/*z*): [M+H]⁺ = 724.3.

### Step 2: preparation of N-(1-((1R,4R)-4-((3,9-diazaspiro[5.5]undecan-3-yl)methyl)cyclohexyl)-3-(difluoromethyl)-1H-pyrazol-4-yl)-5-((1R,4R)-2-oxa-5-azabicyclo[2.2.1]heptan-5-yl)pyrazolo[1,5-a]pyrimidine-3-carboxamide

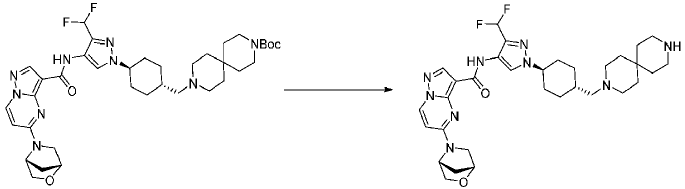

Trifluoroacetic acid (2 mL) was added to a stirred solution of *tert*-butyl 9-(((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H-*pyrazol-1-yl)cyclohexyl)methyl)-3,9-diazaspiro[5.5]undecane-3-carboxylate (200 mg, 0.27 mmol) in dichloromethane (5 mL). The reaction mixture was stirred at room temperature for 2 h. The reaction mixture was concentrated to give a crude product of *N*-(1-((1R,4R)-4-((3,9-diazaspiro[5.5]undecan-3-yl)methyl)cyclohexyl)-3-(difluoromethyl)-1*H*-pyrazol-4-yl)-5-((1*R*,4*R*)-2-oxa-5-azabicyclo[2.2.1]heptan-5-yl)pyrazolo[1,5-*a*]pyrimidine-3-carboxamide. The crude product was directly used in the next step.

LC-MS: (ESI, *m*/*z*): [M+H]⁺ = 624.3.

### Intermediate 21: ((1R,4R)-4-(4-amino-3-(difluoromethyl)-1H-pyrazol-1-yl)cyclohexyl)methanol

### Step 1: preparation of methyl (1S,4S)-4-(p-toluenesulfonyloxy)cyclohexane-1-carboxylate

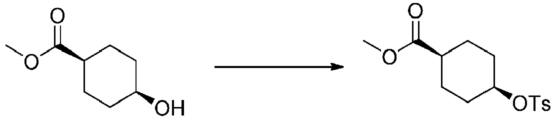

The compound methyl (1*S*,4*S*)-4-hydroxycyclohexane-1-carboxylate (5.0 g, 31.6 mmol) was dissolved in DCM (50 mL), and DMAP (1.9 g, 15.8 mmol), TEA (16.0 g, 158 mmol) and TsCl (9.1 g, 47.5 mmol) were added. The mixture was stirred overnight at room temperature. Water (100 mL) was added to the reaction liquid, and the resulting mixture was extracted with DCM (100 mL × 3). The organic phase was washed with saturated brine (200 mL), dried over anhydrous sodium sulfate, and filtered. The crude product was purified by column chromatography (EA/PE = 1:1) to give methyl (1*S*,4*S*)-4-(*p-*toluenesulfonyloxy)cyclohexane-1 -carboxylate.

LC-MS: (ES, *m*/*z*): [M+NH₄⁺]⁺ = 330.0.

¹H NMR (400 MHz, DMSO-*d*₆) δ 7.83-7.76 (m, 2H), 7.47 (d, *J=* 7.9 Hz, 2H), 4.66 (s, 1H), 3.59 (s, 3H), 2.42 (s, 3H), 1.67-1.53 (m, 8H).

### Step 2: preparation of methyl (1R,4R)-4-(3-formyl-1H-pyrazol-1-yl)cyclohexane-1-carboxylate

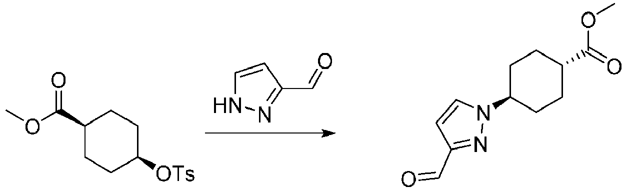

1*H*-Pyrazole-3-carbaldehyde (2.4 g, 25.0 mmol) was dissolved in DMF (50 mL), and methyl (1*S*,4*S*)-4-(*p*-toluenesulfonyloxy)cyclohexane-1-carboxylate (9.4 g, 30.0 mmol), Cs₂CO₃ (24.5 g, 75 mmol) and KI (5.0 g, 30.0 mmol) were added. The mixture was stirred overnight at 50 °C, and water (500 mL) was added. After 5 min of stirring, the mixture was extracted with EA (500 mL × 3). The organic phase was washed with saturated brine (500 mL), dried over anhydrous sodium sulfate, and filtered. The crude product was purified by column chromatography (EA:PE = 7:13) to give methyl (1*R*,4*R*)-4-(3-formyl-1*H*-pyrazol-1-yl)cyclohexane-1 -carboxylate.

LC-MS: (ES, *m*/*z):* [M+H]⁺ = 237.1.

¹H NMR (400 MHz, DMSO-*d*₆) δ 9.86 (s, 1H), 7.97 (d, *J* = 2.4 Hz, 1H), 6.77 (d, *J* = 2.4 Hz, 1H), 4.39-4.29 (m, 1H), 3.62 (s, 3H), 2.47-2.38 (m, 1H), 2.14-2.01 (m, 4H), 1.91-1.77 (m, 2H), 1.62-1.48 (m, 2H).

### Step 3: preparation of methyl (1R,4R)-4-(3-(difluoromethyl)-1H-pyrazol-1-yl)cyclohexane-1-carboxylate

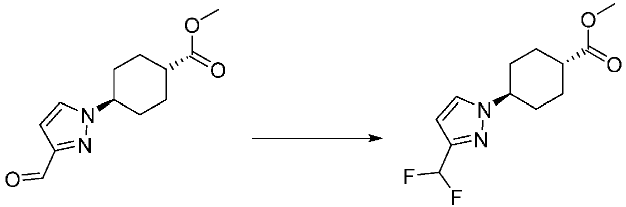

Methyl (1*R*,4*R*)-4-(3-formyl-1*H*-pyrazol-1-yl)cyclohexane-1-carboxylate (1.0 g, 4.2 mmol) was dissolved in anhydrous DCM (20 mL), and DAST (4.1 g, 25.4 mmol) was added at 0 °C. The mixture was slowly warmed to room temperature and stirred overnight. A saturated aqueous solution of sodium bicarbonate was added dropwise to the reaction mixture in an ice bath to quench the reaction. The aqueous phase was separated and extracted twice with dichloromethane. After concentration under reduced pressure, the resulting crude product was purified by column chromatography (EA:PE = 1:4) to give methyl (1*R*,4*R*)-4-(3-(difluoromethyl)-1*H*-pyrazol-1 -yl)cyclohexane-1-carboxylate.

LC-MS: (ES, *m*/*z*): [M+H]^{I} = 259.1.

¹H NMR (400 MHz, DMSO-*d*₆) δ 7.88 (s, 1H), 7.08-6.85 (m, 1H), 6.49 (s, 1H), 4.28-4.17 (m, 1H), 3.62 (s, 3H), 2.47-2.36 (m, 1H), 2.15-1.95 (m, 4H), 1.86-1.73 (m, 2H), 1.61-1.48 (m, 2H).

### Step 4: preparation of methyl (1R,4R)-4-(3-(difluoromethyl)-4-nitro-1H-pyrazol-1-yl)cyclohexane-1 -carboxylate

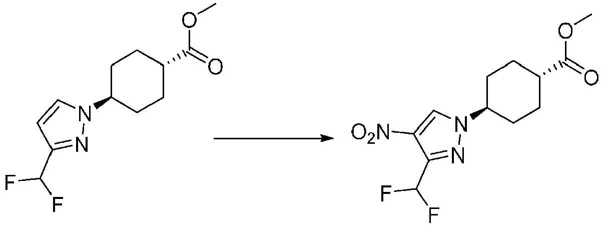

Methyl (1*R*,4*R*)-4-(3-(difluoromethyl)-1*H*-pyrazol-1-yl(cyclohexane-1-carboxylate (1.0 g, 3.9 mmol) was dissolved in concentrated H₂SO₄ (5 mL), and concentrated HNO₃ (1 mL) was added dropwise at 0 °C. The mixture was slowly warmed to room temperature and stirred overnight. The reaction liquid was slowly poured into an ice water mixture (20 mL). The resulting mixture was filtered, and the filter cake was washed with water 3 times and then dried under vacuum to give methyl (1*R*,4*R*)-4-(3-(difluoromethyl)-4-nitro-1*H*-pyrazol-1-yl)cyclohexane-1 -carboxylate. The resulting product was directly used in the next step.

¹H NMR (400 MHz, DMSO-*d*₆) δ 9.05 (s, 1H), 7.39-7.20 (m, 1H), 4.42-4.30 (m, 1H), 3.62 (s, 3H), 2.46-2.37 (m, 1H), 2.16-2.00 (m, 4H), 1.93-1.72 (m, 2H), 1.59-1.40 (m, 2H).

### Step 5: preparation of methyl (1R,4R)-4-(4-amino-3-(difluoromethyl)-1H-pyrazol-1-yl)cyclohexane-1-carboxylate

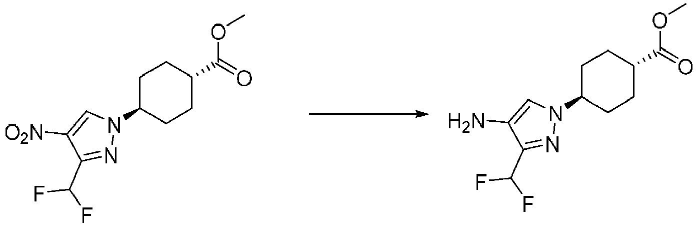

Methyl (1*R*,4*R*)-4-(3-(difluoromethyl)-4-nitro-1*H*-pyrazol-1-yl)cyclohexane-1-carboxylate (880 mg, 2.9 mmol) was dissolved in THF (10 mL), and Pd/C (440 mg, 50%) was added. The mixture was purged with H₂ 3 times and stirred at room temperature for 4 h under H₂ atmosphere. The reaction liquid was filtered through celite, and the filtrate was concentrated under reduced pressure to give methyl (1*R*,4*R*)-4-(4-amino-3-(difluoromethyl)-1*H*-pyrazol-1 -yl)cyclohexane-1-carboxylate. The resulting crude product was directly used in the next step.

LC-MS: (ES, *m*/*z):* [M+H]⁺ = 274.1.

### Step 6: preparation of ((1R,4R)-4-(4-amino-3-(difluoromethyl)-1H-pyrazol-1-yl)cyclohexyl)methanol

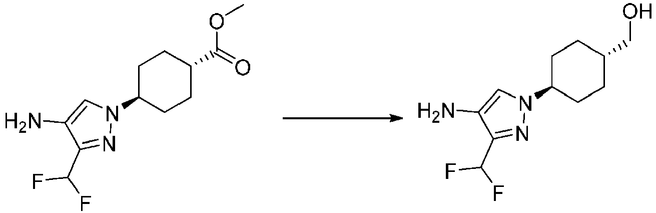

Methyl (1*R*,4*R*)-4-(4-amino-3-(difluoromethyl)-1*H*-pyrazol-1-yl)cyclohexane-1-carboxylate (750 mg, 2.7 mmol) was dissolved in anhydrous THF (5 mL), and BH₃/THF (27.5 mL, 1 M/THF, 27.5 mmol) was added at 0 °C. The mixture was slowly warmed to room temperature and stirred overnight. MeOH was added dropwise to the reaction liquid in an ice bath until no visible bubbles were generated, and the resulting mixture was concentrated under reduced pressure. The crude product was purified by column chromatography (MeOH:DCM = 1:9) to give ((1*R*,4*R*)-4-(4-amino-3-(difluoromethyl)-1*H*-pyrazol-1-yl)cyclohexyl)methanol.

LC-MS: (ES, *m*/*z*): [M+H]⁺ = 246.1.

¹H NMR (400 MHz, DMSO-*d*₆) δ 7.17 (d, *J* = 14.3 Hz, 1H), 7.06-6.68 (m, 1H), 4.46 (t, *J* = 5.3 Hz, 1H), 4.03 (s, 2H), 3.94 (td, *J* = 8.2, 4.0 Hz, 1H), 3.27-3.22 (m, 2H), 1.97 (d, *J* = 9.5 Hz, 2H), 1.83 (d, *J* = 11.4 Hz, 2H), 1.69-1.57 (m, 2H), 1.39 (d, *J* = 2.9 Hz, 1H), 1.12-0.97 (m, 2H).

### Intermediate 22: 5-((1R,4R)-2-oxa-5-azabicyclo[2.2.1]heptan-5-yl)-N-(3-(difluoromethyl)-1-((1R,4R)-4-formylcyclohexyl)-1H-pyrazol-4-yl)pyrazolo[1,5-a]pyrimidine-3-carboxamide

### Step 1: preparation of 5-((1R,4R)-2-oxa-5-azabicyclo[2.2.1]heptan-5-yl)-N-(3-(difluoromethyl)-1-((1R,4R)-4-(hydroxymethyl)cyclohexyl)-1H-pyrazol-4-yl)pyrazolo[1,5-a]pyrimidine-3-carboxamide

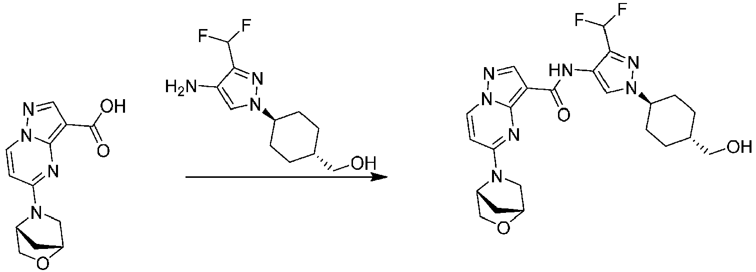

((1*R*,4*R*)-4-(4-Amino-3-(difluoromethyl)-1*H*-pyrazol-1-yl)cyclohexyl)methanol (360 mg, 1.5 mmol) was dissolved in DMF (10 mL), and 5-((1*R*,4*R*)-2-oxa-5-azabicyclo[2.2.1]heptan-5-yl)pyrazolo[1,5-*a*]pyrimidine-3-carboxylic acid (380 mg, 1.5 mmol), DIEA (566 mg, 4.4 mmol) and HATU (726 g, 1.9 mmol) were added. The mixture was stirred overnight at room temperature, and water (50 mL) was added. After 5 min of stirring, the mixture was extracted with EA (50 mL × 3). The organic phase was washed with saturated brine (50 mL), dried over anhydrous sodium sulfate, and filtered. The crude product was purified by column chromatography (MeOH:DCM = 1:9) to give 5-((1*R*,4*R*)-2-oxa-5-azabicyclo[2.2.1]heptan-5-yl)-*N*-(3-(difluoromethyl)-1-((1*R*,4*R*)-4-(hydroxymethyl)cyclohexyl)-1*H*-pyrazol-4-yl)pyrazolo[1,5-*a*]pyrimidine-3-carboxamide.

LC-MS: (ES, *m*/*z*): [M+H]⁺ = 488.2.

¹H NMR (400 MHz, DMSO-*d*₆) δ 9.49 (d, *J* = 5.4 Hz, 1H), 8.78 (d, *J* = 7.7 Hz, 1H), 8.39 (d, *J* = 4.2 Hz, 1H), 8.23 (d, *J* = 5.4 Hz, 1H), 7.30-6.94 (m, 1H), 6.90-6.34 (m, 1H), 5.33-5.03 (m, 1H), 4.77 (d, *J* = 16.7 Hz, 1H), 4.52-4.30 (m, 1H), 4.22-4.05 (m, 1H), 3.89-3.69 (m, 2H), 3.67-3.42 (m, 2H), 3.17-2.97 (m, 2H), 2.12-1.80 (m, 6H), 1.80-1.61 (m, 2H), 1.48-1.36 (m, 1H), 1.19-1.01 (m, 2H).

### Step 2: preparation of 5-((1R,4R)-2-oxa-5-azabicyclo[2.2.1]heptan-5-yl)-N-(3-(difluoromethyl)-1-((1R,4R)-4-formylcyclohexyl)-1H-pyrazol-4-yl)pyrazolo[1,5-a]pyrimidine-3-carboxamide

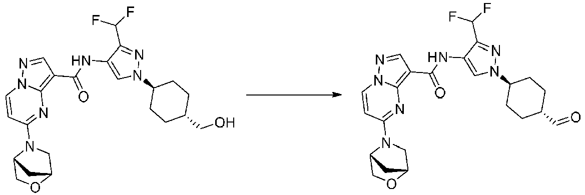

IBX (460 mg, 1.64 mmol) was added to a solution of 5-((1*R*,4*R*)-2-oxa-5-azabicyclo[2.2.1]heptan-5-yl)-*N*-(3-(difluoromethyl)-1-((1*R*,4*R*)-4-(hydroxymethyl)cyclohexyl)-1*H*-pyrazol-4-yl)pyrazolo[1,5-*a*]pyrimidine-3-carboxamide (400 mg, 0.82 mmol) in ACN (20 mL), and the reaction liquid was stirred at 85 °C for 1 h. After the reaction liquid was concentrated under reduced pressure, water (50 mL) was added, and the resulting mixture was extracted with ethyl acetate (50 mL × 3). The organic phase was washed with saturated sodium chloride solution (100 mL), dried over anhydrous sodium sulfate, and then concentrated. The crude product was purified by column chromatography (MeOH:DCM = 1:9) to give 5-((1*R*,4*R*)-2-oxa-5-azabicyclo[2.2.1]heptan-5-yl)-*N*-(3-(difluoromethyl)-1-((1*R*,4*R*)-4-formylcyclohexyl)-1*H*-pyrazol-4-yl)pyrazolo[1,5-*a*]pyrimidine-3-carboxamide.

LC-MS: (ES, *m*/*z*): [M+H]⁺ = 486.2.

¹H NMR (400 MHz, DMSO-*d*₆) δ 9.62 (s, 1H), 9.51 (d, *J* = 5.7 Hz, 1H), 8.78 (d, *J* = 7.7 Hz, 1H), 8.44-8.35 (m, 1H), 8.26 (d, *J* = 5.2 Hz, 1H), 7.27-6.98 (m, 1H), 6.93-6.40 (m 1H), 5.30-5.05 (m, 1H), 4.77 (d, *J* = 16.6 Hz, 1H), 4.26-4.15 (m, 1H), 3.84-3.72 (m, 2H), 3.67-3.59 (m, 2H), 3.45 (d, *J* = 9.9 Hz, 1H), 2.45-2.35 (m, 1H), 2.18-1.74 (m, 8H), 1.45-1.28 (m, 2H).

### Intermediate 23: 5-((1R,4R)-2-oxa-5-azabicyclo[2.2.1]heptan-5-yl)-N-(3-(difluoromethyl)-1-((1R,4R)-4-((methyl(2-(piperidin-4-yl)ethyl)amino)methyl)cyclohexyl)-1H-pyrazol-4-yl)pyrazolo[1,5-α]pyrimidine-3-carboxamide

### Step 1: preparation of tert-butyl 4-(2-(phenyl(methyl)amino)ethyl)piperidine-1-carboxylate

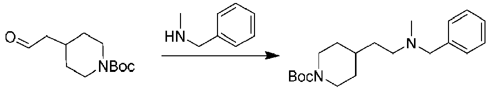

*N-*Methyl-1-phenylmethylamine (1.067 g, 8.82 mmol) andAcOH (0.79 g, 13.23 mmol) were added to a solution of *tert*-butyl 4-(2-oxoethyl)piperidine-1-carboxylate (1 g, 4.41 mmol) in DCE (3 mL) under N₂ atmosphere. After the mixture was stirred at room temperature for 30 min, STAB (2.8 g, 13.23 mmol) was added, and then the reaction liquid was stirred at room temperature for 2 h. The reaction liquid was diluted with water (20 mL), and the mixture was extracted with EA (30 mL × 2). The organic phase was dried over anhydrous Na₂SO₄, filtered, and concentrated under reduced pressure. The resulting crude product was purified by column chromatography (MeOH:DCM = 0-1:20) to give tert-butyl 4-(2-(phenyl(methyl)amino)ethyl)piperidine-1 -carboxylate.

LC-MS: (ESI, m/z): [M+H]⁺ = 333.2.

### Step 2: preparation of tert-butyl 4-(2-(methylamino)ethyl)piperidine-1-carboxylate

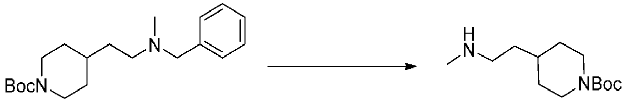

Pd(OH)₂/C (250 mg) was added to a solution of *tert*-butyl 4-(2-(phenyl(methyl)amino)ethyl)piperidine-1-carboxylate (1 g, 3.01 mmol) in THF (3 mL), and the reaction liquid was then stirred at room temperature for 2 h under H₂ atmosphere. The reaction liquid was filtered through celite, and the filtrate was concentrated under reduced pressure to give tert-butyl 4-(2-(methylamino)ethyl)piperidine-1 -carboxylate.

LC-MS: (ESI, m/z): [M+H]⁺ = 243.2.

### Step 3: preparation of tert-butyl 4-(2-((((1R,4R)-4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)cyclohexyl)methyl)(methyl)amino)ethyl)piperidine-1-carboxylate

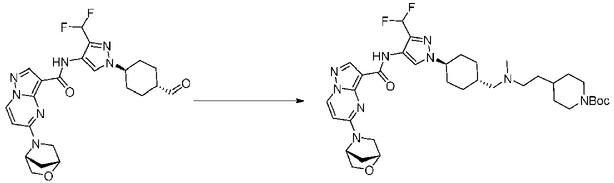

*tert*-Butyl 4-(2-(methylamino)ethyl)piperidine-1-carboxylate (329 mg, 0.928 mmol) and STAB (419 mg, 1.862 mmol) were added to a solution of 5-((1*R*,4*R*)-2-oxa-5-azabicyclo[2.2.1]heptan-5-yl)-N-(3-(difluoromethyl)-1-((1*R*,4*R*)-4-formylcyclohexyl)-1*H*-pyrazol-4-yl)pyrazolo[1,5-*a*]pyrimidine-3-carboxamide (300 mg, 0.618 mmol) in THF (3 mL), and the reaction was stirred at room temperature for 2 h under N2 atmosphere. Water (20 mL) was added to quench the reaction, and the mixture was extracted with DCM (20 mL × 2). The organic phase was dried over anhydrous Na₂SO₄, filtered, and concentrated under reduced pressure. The resulting crude product was purified by column chromatography (MeOH:DCM = 0-1:9) to give *tert*-butyl 4-(2-((((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)cyclohexyl)methyl)(methyl)amino)ethyl)piperidine-1-carboxylate.

LC-MS: (ESI, m/z): [M+H]⁺ = 712.4.

### Step 4: preparation of 5-((1R,4R)-2-oxa-5-azabicyclo[2.2.1]heptan-5-yl)-N-(3-(difluoromethyl)-1-((1R,4R)-4-((methyl(2-(piperidin-4-yl)ethyl)amino)methyl)cyclohexyl)-1H-pyrazol-4-yl)pyrazolo[1,5-α]pyrimidine-3-carboxamide

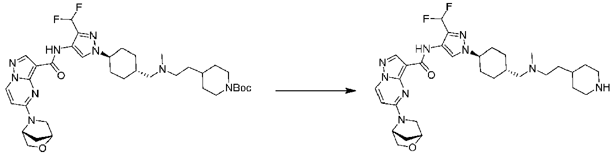

TFA (1 mL) was added to a solution of *tert*-butyl 4-(2-((((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H-*pyrazol-1-yl)cyclohexyl)methyl)(methyl)amino)ethyl)piperidine-1-carboxylate (200 mg, 0.281 mmol) in DCM (3 mL), and the reaction liquid was stirred at room temperature for 2 h under N2 atmosphere. The reaction liquid was directly concentrated under reduced pressure to give 5-((1*R*,4*R*)-2-oxa-5-azabicyclo[2.2.1]heptan-5-yl)-*N*-(3-(difluoromethyl)-1-((1*R*,4*R*)-4-((methyl(2-(piperidin-4-yl)ethyl)amino)methyl)cyclohexyl)-1*H*-pyrazol-4-yl)pyrazolo[1,5-*a*]pyrimidine-3-carboxamide.

LC-MS: (ESI, m/z): [M+H]⁺ = 612.4.

### Intermediate 24: preparation of N-(3-(difluoromethyl)-1-((1R,4R)-4-(formyl)cyclohexyl)-1H-pyrazol-4-yl)-5-morpholinopyrazolo[1,5-a]pyrimidine-3-carboxamide

### Step 1: preparation of ethyl 5-morpholinopyrazolo[1,5-a]pyrimidine-3-carboxylate

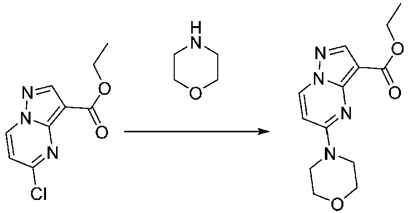

*tert*-Butyl 5-chloropyrazolo[1,5-*a*]pyrimidine-3-carboxylate (6 g, 27 mmol), morpholine (2.3 g, 27 mmol) and *N,N-*diisopropylethylamine (10.4 g, 81 mmol) were dissolved in acetonitrile (60 mL), and the mixture was heated to 60 °C and stirred overnight. The reaction liquid was directly concentrated under reduced pressure, and the crude product was purified by column chromatography (ethyl acetate) to give ethyl 5-morpholinopyrazolo[1,5-*a*]pyrimidine-3 -carboxylate.

LC-MS: (ESI, m/z): [M+H]⁺ = 277.3.

### Step 2: preparation of 5-morpholinopyrazolo[1,5-a]pyrimidine-3-carboxylic acid

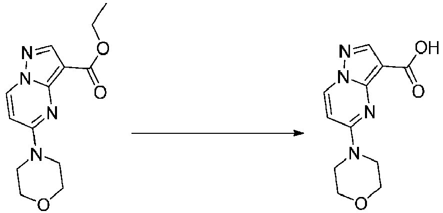

Ethyl 5-morpholinopyrazolo[1,5-*a*]pyrimidine-3-carboxylate (4.3 g, 16 mmol) was dissolved in a mixed solution (methanol/water = 5:1, 40 mL), and lithium hydroxide (2.6 g, 64 mmol) was added. The mixture was heated to 60 °C and stirred for 2 h. The reaction liquid was adjusted to pH = 2 with dilute hydrochloric acid (2 mol/L) and filtered, and the solid was dried under vacuum to give 5-morpholinopyrazolo[1,5-*a*]pyrimidine-3-carboxylic acid.

### Step 3: preparation of N-(3-(difluoromethyl)-1-((1R,4R)-4-(hydroxymethyl)cyclohexyl)-1H-pyrazol-4-yl)-5-morpholinopyrazolo[1,5-a]pyrimidine-3-carboxamide

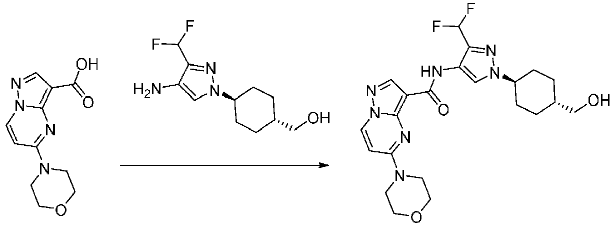

5-Morpholinopyrazolo[1,5-*a*]pyrimidine-3-carboxylic acid (248 mg, 1.0 mmol), ((1R,4R)-4-(4-amino-3-(difluoromethyl)-1*H-*pyrazol-1-yl)cyclohexyl)methanol (245 mg, 1.0 mmol) and HATU (494 mg, 1.3 mmol) were dissolved in DMF (5 mL), and DIEA (387 mg, 3.0 mmol) was added. The reaction liquid was stirred at room temperature for 16 h. After the reaction was complete, the reaction liquid was diluted with water (50 mL), stirred, and filtered. The filter cake was washed with water (5 mL × 3) and dried under vacuum to give *N*-(3-(difluoromethyl)-1-((1*R*,4*R*)-4-(hydroxymethyl)cyclohexyl)-1*H*-pyrazol-4-yl)-5-morpholinopyrazolo[1,5-*a*]pyrimidine-3-carboxamide.

LC-MS: (ESI, m/z): [M+H]⁺ = 476.1.

### Step 4: preparation of N-(3-(difluoromethyl)-1-((1R,4R)-4-(formyl)cyclohexyl)-1H-pyrazol-4-yl)-5-morpholinopyrazolo[1,5-a]pyrimidine-3-carboxamide

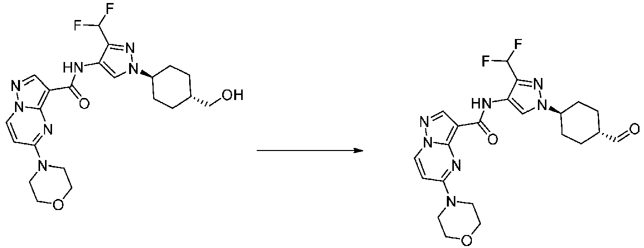

*N-*(3-(Difluoromethyl)-1-((1*R*,4*R*)-4-(hydroxymethyl)cyclohexyl)-1*H*-pyrazol-4-yl)-5-morpholinopyrazolo[1,5-*a*]pyrimidine-3-carboxamide (80 mg, 0.17 mmol) was dissolved in DCM (4 mL), and the Dess-Martin (144 mg, 0.34 mmol) reagent was added. The reaction liquid was stirred overnight at room temperature. After filtration, the solid was washed with DCM (10 mL × 3). The organic phase was concentrated under reduced pressure, and the crude product was purified by normal-phase column chromatography to give *N-*(3-(difluoromethyl)-1-((1*R*,4*R*)-4-(formyl)cyclohexyl)-1*H*-pyrazol-4-yl)-5-morpholinopyrazolo[1,5-*a*]pyrimidine-3-carboxamide.

LC-MS: (ESI, m/z): [M+H]⁺ = 474.1.

### Intermediate 25: N-(1-(1-((3-azaspiro[5.5]undecan-9-yl)methyl)piperidin-4-yl)-3-(difluoromethyl)-1H-pyrazol-4-yl)-5-morpholinopyrazolo[1,5-a]pyrimidine-3-carboxamide

### Step 1: preparation of tert-butyl 4-(3-(difluoromethyl)-4-(5-morpholinopyrazolo[1,5-a]pyrimidine-3-carboxamido)-1H-pyrazol-1-yl)piperidine-1-carboxylate

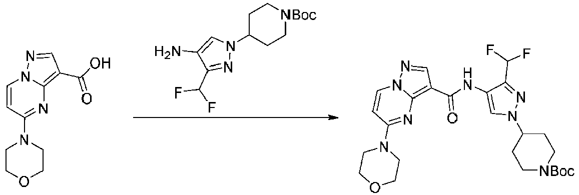

HATU (900 mg, 2.4 mmol) was added to a solution of 5-morpholinopyrazolo[1,5-*a*]pyrimidine-3-carboxylic acid (470 mg, 1.9 mmol) and DIEA (1 mL, 4.7 mmol) in DMF (20 mL) at 25 °C. After the mixture was stirred at 25 °C for 2 h, *tert*-butyl 4-(4-amino-3-(difluoromethyl)-1*H-*pyrazol-1-yl)piperidine-1-carboxylate (0.5 g, 1.5 mmol) was added, and the reaction liquid was then stirred overnight at 25 °C. Water (30 mL) was added to the reaction liquid, and the mixture was then extracted with EA (50 mL × 3). The organic phases were combined, washed with saturated brine, dried over anhydrous Na₂SO₄, filtered, and concentrated under reduced pressure. The resulting crude product was purified by flash column chromatography (EA) to give *tert*-butyl 4-(3-(difluoromethyl)-4-(5-morpholinopyrazolo[1,5-*a*]pyrimidine-3-carboxamido)-1*H-*pyrazol-1-yl)piperidine-1-carboxylate.

LC-MS: (ESI, m/z): [M+H]⁺ = 547.4.

### Step 2: preparation of N-(3-(difluoromethyl)-1-(piperidin-4-yl)-1H-pyrazol-4-yl)-5-morpholinopyrazolo[1,5-a]pyrimidine-3-carboxamide

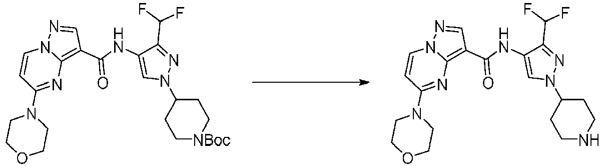

TFA (1 mL) was added to a solution of *tert*-butyl 4-(3-(difluoromethyl)-4-(5-morpholinopyrazolo[1,5-*a*]pyrimidine-3-carboxamido)-1*H*-pyrazol-1-yl)piperidine-1-carboxylate (0.2 g, 0.366 mmol) in DCM (3 mL) at 25 °C. The reaction liquid was stirred at 25 °C for 1 h. The reaction liquid was directly concentrated under reduced pressure to give *N*-(3-(difluoromethyl)-1-(piperidin-4-yl)-1*H-*pyrazol-4-yl)-5-morpholinopyrazolo[1,5-*a*]pyrimidine-3-carboxamide.

LC-MS: (ESI, m/z): [M+H]^{I} = 447.2.

### Step 3: preparation of tert-butyl 9-((4-(3-(difluoromethyl)-4-(5-morpholinopyrazolo[1,5-a]pyrimidine-3-carboxamido)-1H-pyrazol-1-yl)piperidin-1-yl)methyl)-3-azaspiro[5.5]undecane-3-carboxylate

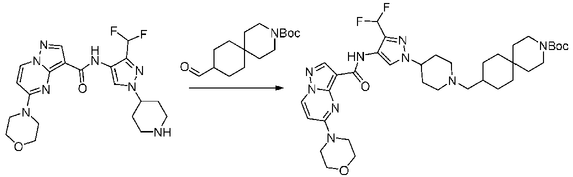

*tert*-Butyl 9-formyl-3-azaspiro[5.5]undecane-3-carboxylate (150 mg, 0.45 mmol) and NaBH₃CN (100 mg, 2.25 mmol) were added to a solution of *N*-(3-(difluoromethyl)-1-(piperidin-4-yl)-1*H*-pyrazol-4-yl)-5-morpholinopyrazolo[1,5-*a*]pyrimidine-3-carboxamide (200 mg, 0.45 mmol) andDIEA(0.5 mL, 1.35 mmol) in MeOH (5 mL) at 25 °C. The reaction liquid was stirred at 25 °C for 16 h. After filtration and concentration under reduced pressure, the resulting crude product was purified by flash column chromatography (MeOH:DCM = 3:97) to give *tert*-butyl 9-((4-(3-(difluoromethyl)-4-(5-morpholinopyrazolo[1,5-*a*]pyrimidine-3-carboxamido)-1*H*-pyrazol-1-yl)piperidin-1-yl)methyl)-3-azaspiro[5.5]undecane-3-carboxylate.

LC-MS: (ESI, m/z): [M+H]⁺ = 712.4.

### Step 4: preparation of N-(1-(1-((3-azaspiro[5.5]undecan-9-yl)methyl)piperidin-4-yl)-3-(difluoromethyl)-1H-pyrazol-4-yl)-5-morpholinopyrazolo[1,5-a]pyrimidine-3-carboxamide

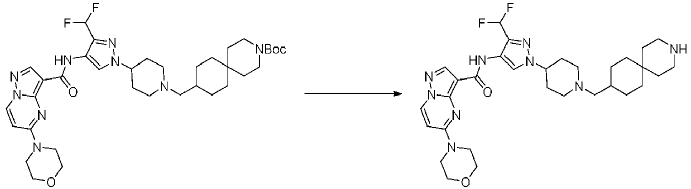

TFA (1 mL) was added to a solution of *tert*-butyl 9-((4-(3-(difluoromethyl)-4-(5-morpholinopyrazolo[1,5-*a*]pyrimidine-3-carboxamido)-1*H*-pyrazol-1-yl)piperidin-1-yl)methyl)-3-azaspiro[5.5]undecane-3-carboxylate (0.14 g, 0.2 mmol) in DCM (3 mL) at 25 °C. The reaction liquid was stirred at 25 °C for 1 h. The reaction liquid was directly concentrated under reduced pressure to give *N-*(1-(1-((3-azaspiro[5.5]undecan-9-yl)methyl)piperidin-4-yl)-3-(diffuoromethyl)-1*H-*pyrazol-4-yl)-5-morpholinopyrazolo[1,5-*a*]pyrimidine-3-carboxamide. The resulting product was directly used in the next step.

LC-MS: (ESI, m/z): [M+H]⁺ = 612.5.

### Intermediate 26: ((1R,4R)-4-(3-(difluoromethyl)-4-nitro-1H-pyrazol-1-yl)cyclohexyl)methanol

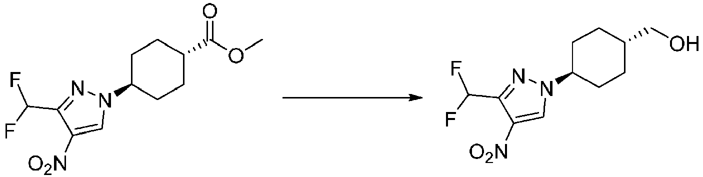

A solution of methyl (1*R*,4*R*)-4-(3-(difluoromethyl)-4-nitro-1*H*-pyrazol-1-yl)cyclohexane-1-carboxylate (4.0 g, 13.2 mmol) in THF (50 mL) was cooled to -20 °C, and LiAlH₄ (1 M in THF, 16 mL) was added dropwise. After the reaction liquid was stirred at room temperature for 0.5 h, a 15% sodium hydroxide solution (8 mL) was slowly added, and water (50 mL) was added. The mixture was extracted with a MeOH/DCM mixed solution (V/V = 1:9, 50 mL × 3), dried over anhydrous sodium sulfate, and concentrated under reduced pressure. The crude product was purified by column chromatography (MeOH:DCM = 1:9) to give ((1*R*,4*R*)-4-(3-(difluoromethyl)-4-nitro-1*H*-pyrazol-1-yl)cyclohexyl)methanol.

LC-MS: (ESI, m/z): [M-56]^{I} = 276.0.

### Intermediate 27: N-(1-(1-(2-(3-azaspiro[5.5]undecan-9-yl)ethyl)piperidin-4-yl)-3-(difluoromethyl)-1H-pyrazol-4-yl)-5-morpholinopyrazolo[1,5-a]pyrimidine-3-carboxamide

### Step 1: preparation of tert-butyl 9-(2-oxoethyl)-3-azaspiro[5.5]undecane-3-carboxylate

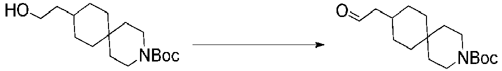

*tert*-Butyl 9-(2-hydroxyethyl)-3-azaspiro[5.5]undecane-3-carboxylate (250 mg, 0.842 mmol) and PCC (543.8 mg, 2.523 mmol) were added to DCM (5 mL). The mixture was stirred at room temperature for 2 h and then filtered. Water (20 mL) was added, and the mixture was extracted with ethyl acetate (25 mL × 4), washed with saturated brine, dried over anhydrous sodium sulfate, and concentrated under reduced pressure to give a crude product of *tert*-butyl 9-(2-oxoethyl)-3-azaspiro[5.5]undecane-3-carboxylate.

LC-MS: (ESI, m/z): [M-56]⁺ = 240.1.

### Step 2: preparation of tert-butyl 9-(2-(4-(3-(difluoromethyl)-4-(5-morpholinopyrazolo[1,5-a]pyrimidine-3-carboxamido)-1H-pyrazol-1-yl)piperidin-1-yl)ethyl)-3-azaspiro[5.5]undecane-3-carboxylate

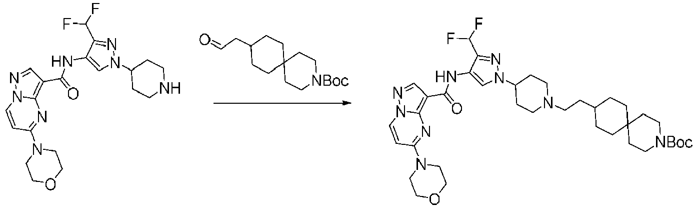

*N*-(3-(Difluoromethyl)-1-(piperidin-4-yl)-1*H-*pyrazol-4-yl)-5-morpholinopyrazolo[1,5-*a*]pyrimidine-3-carboxamide (140 mg, 0.314 mmol) and tert-butyl 9-(2-oxoethyl)-3-azaspiro[5.5]undecane-3-carboxylate (185 mg, 0.628 mmol) were added to THF, and sodium triacetoxyborohydride (133 mg, 0.628 mmol) was then added. The mixture was stirred at room temperature for 2 h, and water (20 mL) was added. The mixture was extracted with ethyl acetate (25 mL × 4), washed with saturated brine, dried over anhydrous sodium sulfate, concentrated under reduced pressure, and purified by normal-phase column chromatography (MeOH:DCM = 1:19) to give *tert*-butyl 9-(2-(4-(3-(difluoromethyl)-4-(5-morpholinopyrazolo[1,5-*a*]pyrimidine-3-carboxamido)-1*H*-pyrazol-1-yl)piperidin-1-yl)ethyl)-3-azaspiro[5.5]undecane-3-carboxylate.

LC-MS: (ESI, m/z): [M+H]⁺ = 726.4.

### Step 3: preparation of N-(1-(1-(2-(3-azaspiro[5.5]undecan-9-yl)ethyl)piperidin-4-yl)-3-(difluoromethyl)-1H-pyrazol-4-yl)-5-morpholinopyrazolo[1,5-a]pyrimidine-3-carboxamide

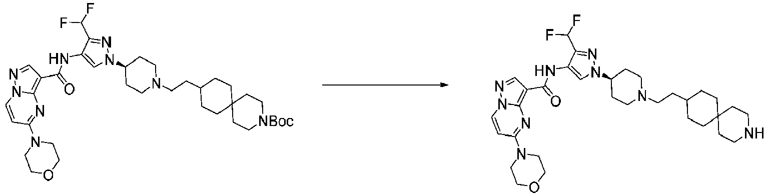

*tert*-Butyl 9-(2-(4-(3-(difluoromethyl)-4-(5-morpholinopyrazolo[1,5-*a*]pyrimidine-3-carboxamido)-1*H*-pyrazol-1-yl)piperidin-1-yl)ethyl)-3-azaspiro[5.5]undecane-3-carboxylate (121 mg, 0.167 mmol) was added to TFA (0.5 mL) and DCM (2.5 mL), and the mixture was stirred at room temperature for 2 h. The reaction liquid was concentrated under reduced pressure to give a crude product of *N*-(1-(1-(2-(3-azaspiro[5.5]undecane-9-yl)ethyl)piperidin-4-yl)-3-(difluoromethyl)-1*H*-pyrazol-4-yl)-5-morpholinopyrazolo[1,5-*a*]pyrimidine-3-carboxamide.

LC-MS: (ESI, m/z): [M+H]⁺ = 626.4.

### Intermediate 28: N-(3-(difluoromethyl)-1-((1R,4S)-4-formylcyclohexyl)-1H-pyrazol-4-yl)-5-((3S)-3-((tetrahydro-2H-pyran-2-yl)oxy)piperidin-1-yl)pyrazolo[1,5-a]pyrimidine-3-carboxamide

### Step 1: preparation of ethyl (S)-5-(3-hydroxypiperidin-1-yl)pyrazolo[1,5-a]pyrimidine-3-carboxylate

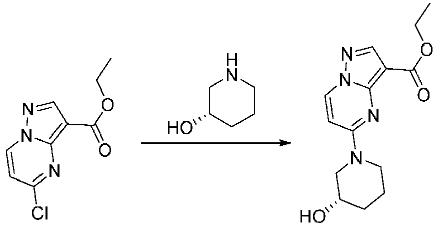

Ethyl 5-chloropyrazolo[1,5-*a*]pyrimidine-3-carboxylate (1.0 g, 4.4 mmol), (*S*)-piperidin-3-ol (445 mg, 4.4 mmol) and *N,N-*diisopropylethylamine (1.7 g, 13.3 mmol) were dissolved in acetonitrile (10 mL), and the reaction liquid was heated to 60 °C and stirred overnight. The reaction liquid was directly concentrated under reduced pressure, and the crude product was purified by column chromatography (ethyl acetate:petroleum ether = 87:13) to give ethyl (*S*)-5-(3-hydroxypiperidin-1-yl)pyrazolo[1,5-*a*]pyrimidine-3-carboxylate.

LC-MS: (ESI, m/z): [M+H]⁺ = 291.1.

### Step 2: preparation of (S)-5-(3-hydroxypiperidin-1-yl)pyrazolo[1,5-a]pyrimidine-3-carboxylic acid

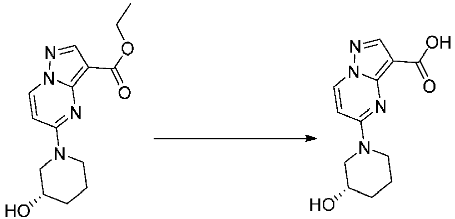

Ethyl (*S*)-5-(3-hydroxypiperidin-1-yl)pyrazolo[1,5-*a*]pyrimidine-3-carboxylate (1.1 g, 3.9 mmol) was dissolved in a mixed solution (methanol/water = 5:1, 10 mL), and lithium hydroxide (643 mg, 16 mmol) was added. The mixture was heated to 60 °C and stirred for 2 h. The reaction liquid was adjusted to pH = 2 with dilute hydrochloric acid (2 mol/L) and filtered, and the filter cake was dried under vacuum to give (5)-5-(3-hydroxypiperidin-1-yl)pyrazolo[1,5-*a*]pyrimidine-3-carboxylic acid.

LC-MS: (ESI, m/z): [M+H]⁺ = 263.1.

### Step 3: preparation of 5-((3S)-3-((tetrahydro-2H-pyran-2-yl)oxy)piperidin-1-yl)pyrazolo[1,5-a]pyrimidine-3-carboxylic acid

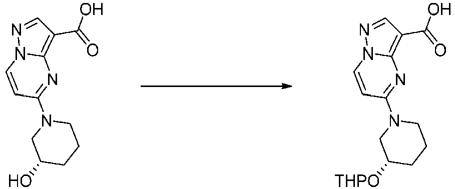

(*S*)-5-(3-Hydroxypiperidin-1-yl)pyrazolo[1,5-*a*]pyrimidine-3-carboxylic acid (300 mg, 1.14 mmol) was dissolved in tetrahydrofuran (15 mL), and 3,4-dihydro-2*H*-pyran (962 mg, 11.4 mmol) and tetrabutylammonium tribromide (111 mg, 0.23 mmol) were added. The mixture was heated to 60 °C and stirred overnight. The reaction liquid was concentrated under reduced pressure, and the crude product was purified by column chromatography (ethyl acetate) to give 5-((3*S*)-3-((tetrahydro-2*H*-pyran-2-yl)oxy)piperidin-1-yl)pyrazolo[1,5-*a*]pyrimidine-3-carboxylic acid.

LC-MS: (ESI, m/z): [M+H]⁺ = 347.2.

### Step 4: preparation of N-(3-(difluoromethyl)-1-((1R,4S)-4-(hydroxymethyl)cyclohexyl)-1H-pyrazol-4-yl)-5-((3S)-3-((tetrahydro-2H-pyran-2-yl)oxy)piperidin-1-yl)pyrazolo[1,5-a]pyrimidine-3-carboxamide

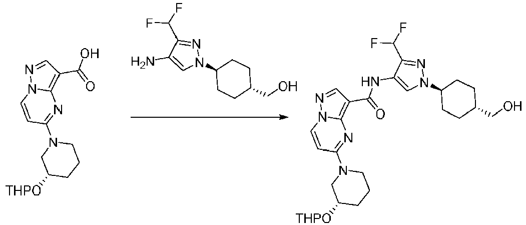

5-((3*S*)-3-((Tetrahydro-2*H*-pyran-2-yl)oxy)piperidin-1-yl)pyrazolo[1,5-*a*]pyrimidine-3-carboxylic acid (180 mg, 0.52 mmol) and ((1*R*,4*R*)-4-(4-amino-3-(difluoromethyl)-1*H-*pyrazol-1-yl)cyclohexyl)methanol (137 mg, 0.52 mmol) were dissolved in DMF (10 mL), and *N,N-*diisopropylethylamine (201 mg, 1.56 mmol) and py-BOP (348 mg, 0.67 mmol) were added with stirring. The mixture was stirred overnight at 60 °C. The reaction liquid was cooled to room temperature, and water (100 mL) was added. The mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (methanol:dichloromethane = 1:9) to give *N*-(3-(difluoromethyl)-1-((1*R*,4*S*)-4-(hydroxymethyl)cyclohexyl)-1*H*-pyrazol-4-yl)-5-((3*S*)-3-((tetrahydro-2*H*-pyran-2-yl)oxy)piperidin-1-yl)pyrazolo[1,5-*a*]pyrimidine-3-carboxamide.

LC-MS: (ESI, m/z): [M+H]⁺ = 574.3.

### Step 5: preparation of N-(3-(difluoromethyl)-1-((1R,4S)-4-formylcyclohexyl)-1H-pyrazol-4-yl)-5-((3S)-3-((tetrahydro-2H-pyran-2-yl)oxy)piperidin-1-yl)pyrazolo[1,5-a]pyrimidine-3-carboxamide

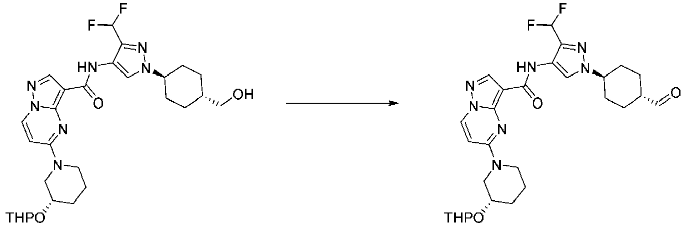

*N*-(3-(Difluoromethyl)-1-((1*R*,4*S*)-4-(hydroxymethyl)cyclohexyl)-1*H*-pyrazol-4-yl)-5-((3*S*)-3-((tetrahydro-2*H-*pyran-2-yl)oxy)piperidin-1-yl)pyrazolo[1,5-*a*]pyrimidine-3-carboxamide (200 mg, 0.35 mmol) was dissolved in acetonitrile (20 mL), and IBX (196 mg, 0.70 mmol) was added. The mixture was stirred at 80 °C for 2 h. The reaction liquid was concentrated under reduced pressure, and the crude product was purified by column chromatography (methanol:dichloromethane = 1:9) to give N (3-(difluoromethyl)-1-((1*R*,4*S*)-4-formylcyclohexyl)-1*H*-pyrazol-4-yl)-5-((3*S*)-3-((tetrahydro-2*H*-pyran-2-yl)oxy)piperidin-1-yl)pyrazolo[1,5-*a*]pyrimidine-3-carboxamide.

LC-MS: (ESI, m/z): [M+H]^{I} = 572.3.

### Intermediate 29: preparation of (S)-N-(1-(1-((3-azaspiro[5.5]undecan-9-yl)methyl)piperidin-4-yl)-3-(difluoromethyl)-1H-pyrazol-4-yl)-5-(3-hydroxypiperidin-1-yl)pyrazolo[1,5-a]pyrimidine-3-carboxamide

### Step 1: preparation of tert-butyl 4-(3-(difluoromethyl)-4-(5-((3S)-3-((tetrahydro-2H-pyran-2-yl)oxy)piperidin-1-yl)pyrazolo[1,5-a]pyrimidine-3-carboxamido)-1H-pyrazol-1-yl)piperidine-1-carboxylate

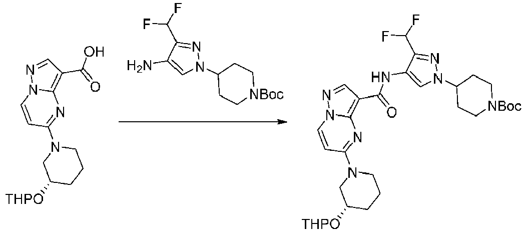

5-((3*S*)-3-((Tetrahydro-2*H*-pyran-2-yl)oxy)piperidin-1-yl)pyrazolo[1,5-*a*]pyrimidine-3-carboxylic acid (210 mg, 0.61 mmol) and tert-butyl 4-(4-amino-3-(difluoromethyl)-1*H-*pyrazol-1-yl)piperidine-1-carboxylate (191 mg, 0.61 mmol) were dissolved in DMF (10 mL), and *N,N*-diisopropylethylamine (236 mg, 1.83 mmol) and Py-BOP (475 mg, 0.91 mmol) were added with stirring. The mixture was heated to 60 °C and stirred overnight. Water (100 mL) was added to the reaction liquid, and the mixture was extracted with ethyl acetate (30 mL × 3). The organic phase was washed with saturated brine (60 mL), dried over anhydrous sodium sulfate, filtered, and concentrated under reduced pressure. The crude product was purified by column chromatography (methanol:dichloromethane = 1:9) to give *tert*-butyl 4-(3-(difluoromethyl)-4-(5-((3S)-3-((tetrahydro-2*H-*pyran-2-yl)oxy)piperidin-1-yl)pyrazolo[1,5-*a*]pyrimidine-3-carboxamido)-1*H*-pyrazol-1-yl)piperidine-1-carboxylate.

LC-MS: (ESI, m/z): [M+H]⁺ = 645.3.

### Step 2: preparation of (S)-N-(3-(-difluoromethyl)-1-(piperidin-4-yl)-1H-pyrazol-4-yl)-5-(3-hydroxypiperidin-1-yl)pyrazolo[1,5-a]pyrimidine-3-carboxamide

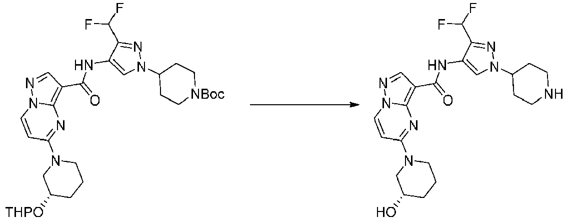

*tert*-Butyl 4-(3-(difluoromethyl)-4-(5-((3*S*)-3-((tetrahydro-2*H*-pyran-2-yl)oxy)piperidin-1-yl)pyrazolo[1,5-*a*]pyrimidine-3-carboxamido)-1*H*-pyrazol-1-yl)piperidine-1-carboxylate (180 mg, 0.28 mmol) was dissolved in dichloromethane (3 mL), and trifluoroacetic acid (1 mL) was added. The mixture was stirred at room temperature for 1 h. The reaction liquid was directly concentrated under reduced pressure to give (*S*)-*N*-(3-(difluoromethyl)-1-(piperidin-4-yl)-1*H*-pyrazol-4-yl)-5-(3-hydroxypiperidin-1-yl)pyrazolo[1,5-*a*]pyrimidine-3-carboxamide, which was directly used in the next step without purification.

LC-MS: (ESI, m/z): [M+H]⁺ = 461.2.

### Step 3: preparation of tert-butyl (S)-9-((4-(3-(difluoromethyl)-4-(5-(3-hydroxypiperidin-1-yl)pyrazolo[1,5-a]pyrimidine-3-carboxamido)-1H-pyrazol-1-yl)piperidin-1-yl)methyl)-3-azaspiro [5.5]undecane-3-carboxylate

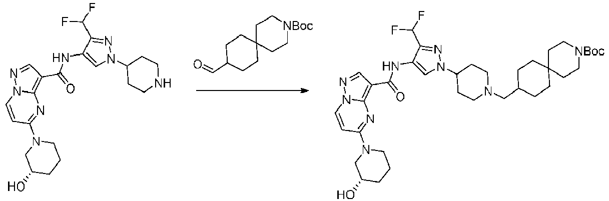

(*S*)-*N*-(3-(Difluoromethyl)-1-(piperidin-4-yl)-1*H*-pyrazol-4-yl)-5-(3-hydroxypiperidin-1-yl)pyrazolo[1,5-*a*]pyrimidine-3-carboxamide (180 mg, crude, 0.28 mmol) and tert-butyl 9-formyl-3-azaspiro[5.5]undecane-3-carboxylate (87 mg, 0.31 mmol) were dissolved in tetrahydrofuran (10 mL), and sodium triacetoxyborohydride (180 mg, 0.84 mmol) was added with stirring. The mixture was stirred overnight at room temperature. Water (50 mL) was added to the reaction liquid, and the mixture was extracted with dichloromethane (20 mL × 3). The organic phase was dried over anhydrous sodium sulfate, filtered, and concentrated under reduced pressure. The crude product was purified by column chromatography (methanol:dichloromethane = 1:9) to give tert-butyl (*S*)-9-((4-(3-(difluoromethyl)-4-(5-(3-hydroxypiperidin-1 -yl)pyrazolo[1,5-*a*]pyrimidine-3-carboxamido)-1*H*-pyrazol-1-yl)piperidin-1-yl)methyl)-3-azaspiro[5.5]undecane-3-carboxylate.

LC-MS: (ESI, m/z): [M+H]⁺ = 726.3.

### Step 4: preparation of (S)-N-(1-(1-((3-azaspiro[5.5]undecan-9-yl)methyl)piperidin-4-yl)-3-(difluoromethyl)-1H-pyrazol-4-yl)-5-(3-hydroxypiperidin-1-yl)pyrazolo[1,5-a]pyrimidine-3-carboxamide

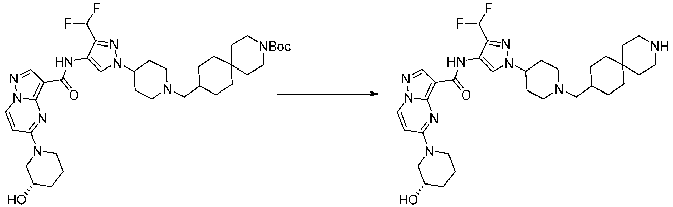

tert-Butyl (*S*)-9-((4-(3-(difluoromethyl)-4-(5-(3-hydroxypiperidin-1-yl)pyrazolo[1,5-*a*]pyrimidine-3-carboxamido)-1*H*-pyrazol-1-yl)piperidin-1-yl)methyl)-3-azaspiro[5.5]undecane-3-carboxylate (160 mg, 0.22 mmol) was dissolved in dichloromethane (3 mL), trifluoroacetic acid (1 mL) was added, and the mixture was stirred at room temperature for 2 h. The reaction liquid was directly concentrated under reduced pressure to give (*S*)-*N*-(1-(1-((3-azaspiro[5.5]undecan-9-yl)methyl)piperidin-4-yl)-3-(difluoromethyl)-1*H*-pyrazol-4-yl)-5-(3-hydroxypiperidin-1-yl)pyrazolo[1,5-*a*]pyrimidine-3-carboxamide, which was directly used in the next step without purification.

LC-MS: (ESI, m/z): [M+H]⁺ = 626.4.

### Intermediate 30: N-(3-(difluoromethyl)-1-((1R,4R)-4-formylcyclohexyl)-1H-pyrazol-4-yl)-5-(2-oxa-6-azaspiro[3.3]heptan-6-yl)pyrazolo[1,5-a]pyrimidine-3-carboxamide

### Step 1: preparation of ethyl 5-(2-oxa-6-azaspiro[3.3]heptan-6-yl)pyrazolo[1,5-a]pyrimidine-3-carboxylate

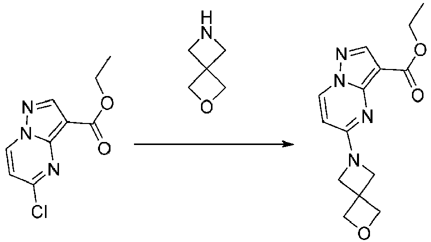

Ethyl 5-chloropyrazolo[1,5-*a*]pyrimidine-3-carboxylate (1.0 g, 4.4 mmol), 2-oxa-6-azaspiro[3.3]heptane (439 mg, 4.4 mmol), and *N,N*-diisopropylethylamine (1.7 g, 13.3 mmol) were dissolved in acetonitrile (10 mL), and the reaction liquid was heated to 60 °C and stirred overnight. The reaction liquid was directly concentrated under reduced pressure, and the crude product was purified by column chromatography (ethyl acetate) to give ethyl 5-(2-oxa-6-azaspiro[3.3]heptan-6-yl)pyrazolo[1,5-*a*]pyrimidine-3-carboxylate.

LC-MS: (ESI, m/z): [M+H]⁺ = 289.2.

### Step 2: preparation of 5-(2-oxa-6-azaspiro[3.3]heptan-6-yl)pyrazolo[1,5-a]pyrimidine-3-carboxylic acid

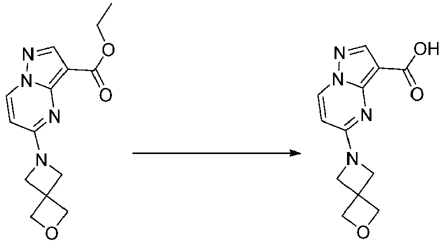

Ethyl 5-(2-oxa-6-azaspiro[3.3]heptan-6-yl)pyrazolo[1,5-*a*]pyrimidine-3-carboxylate (1.1 g, 3.8 mmol) was dissolved in 10 mL of a mixed solution (methanol/water = 5:1), lithium hydroxide (639 mg, 15 mmol) was added, and the mixture was heated to 60 °C and stirred for 2 h. The reaction liquid was adjusted to pH = 2 with 2 mol/L dilute hydrochloric acid and filtered. The solid was dried under vacuum to give 5-(2-oxa-6-azaspiro[3.3]heptan-6-yl)pyrazolo[1,5-*a*]pyrimidine-3-carboxylic acid.

LC-MS: (ESI, m/z): [M+H]^{I} = 261.1.

### Step 3: preparation of N-(3-(difluoromethyl)-1-((1R,4R)-4-(hydroxymethyl)cyclohexyl)-1H-pyrazol-4-yl)-5-(2-oxa-6-azaspiro[3.3]heptan-6-yl)pyrazolo[1,5-a]pyrimidine-3-carboxamide

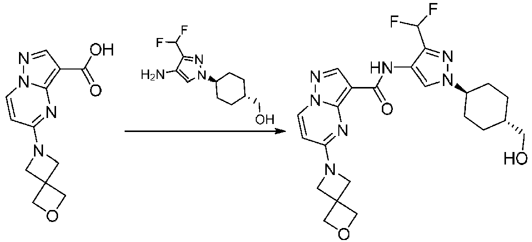

5-(2-Oxa-6-azaspiro[3.3]heptan-6-yl)pyrazolo[1,5-*a*]pyrimidine-3-carboxylic acid (110 mg, 0.41 mmol) and ((1*R*,4*R*)-4-(4-amino-3-(diffuoromethyl)-1*H-*pyrazol-1-yl)cyclohexyl)methanol (100 mg, 0.41 mmol) were dissolved in DMF (3 mL). HATU (202 mg, 0.53 mmol) and DIEA (212 mg, 1.6 mmol) were added. The reaction liquid was heated to 90 °C, stirred overnight, diluted with water (80 mL), extracted with ethyl acetate (100 mL × 2), washed with saturated brine, dried over anhydrous sodium sulfate, and concentrated under reduced pressure. The crude product was purified by column chromatography (PE:EA = 1:9) to give the compound *N-*(3-(difluoromethyl)-1-((1*R*,4*R*)-4-(hydroxymethyl)cyclohexyl)-1*H-*pyrazol-4-yl)-5-(2-oxa-6-azaspiro[3.3]heptan-6-yl)pyrazolo[1,5-a]pyrimidine-3-carboxamide.

LC-MS: (ESI, m/z): [M+H]⁺ = 488.2.

### Step 4: preparation of N-(3-(difluoromethyl)-1-((1R,4R)-4-formylcyclohexyl)-1H-pyrazol-4-yl)-5-(2-oxa-6-azaspiro[3.3]heptan-6-yl)pyrazolo[1,5-a]pyrimidine-3-carboxamide

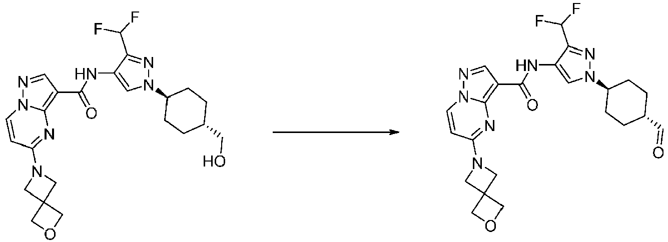

The compound *N*-(3-(difluoromethyl)-1-((1*R*,4*R*)-4-(hydroxymethyl)cyclohexyl)-1H-pyrazol-4-yl)-5-(2-oxa-6-azaspiro[3.3]heptan-6-yl)pyrazolo[1,5-*a*]pyrimidine-3-carboxamide (120 mg, 0.246 mmol) was dissolved in DCM (3 mL), then PCC (107 mg, 0.493 mmol) was added, and the reaction liquid was stirred at room temperature for 2 h. The reaction liquid was diluted with water (20 mL) and extracted with dichloromethane (25 mL × 4). The organic phase was dried over anhydrous sodium sulfate and concentrated under reduced pressure. The crude product was purified by column chromatography (DCM:MEOH = 1:10) to give the compound *N*-(3-(difluoromethyl)-1-((*R*,4*R*)-4-formylcyclohexyl)-1*H*-pyrazol-4-yl)-5-(2-oxa-6-azaspiro[3.3]heptan-6-yl)pyrazolo[1,5-*a*]pyrimidine-3-carboxamide.

LC-MS: (ESI, m/z): [M+H]⁺ = 486.3.

### Intermediate 31: N-(3-(difluoromethyl)-1-(piperidin-4-yl)-1H-pyrazol-4-yl)-5-(1,1-dioxothiomorpholinyl)pyrazolo[1,5-a]pyrimidine-3-carboxamide

### Step 1: preparation of ethyl 5-(1,1-dioxothiomorpholinyl)pyrazolo[1,5-a]pyrimidine-3-carboxylate

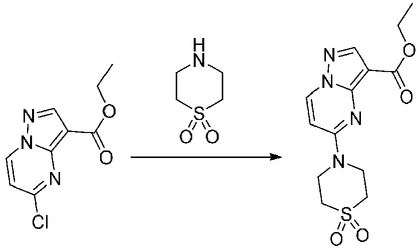

Ethyl 5-chloropyrazolo[1,5-*a*]pyrimidine-3-carboxylate (1.0 g, 4.4 mmol), thiomorpholine 1,1-dioxide (906 mg, 5.3 mmol), and *N,N*-diisopropylethylamine (2.3 g, 18 mmol) were dissolved in acetonitrile (10 mL), and the mixture was heated to 60 °C and stirred overnight. The reaction liquid was directly concentrated under reduced pressure, and the crude product was purified by column chromatography (ethyl acetate:petroleum ether = 9:1) to give ethyl 5-(1,1-dioxothiomorpholinyl)pyrazolo[1,5-*a*]pyrimidine-3-carboxylate.

LC-MS: (ESI, m/z): [M+H]⁺ = 325.0.

### Step 2: preparation of 5-(1,1-dioxothiomorpholinyl)pyrazolo[1,5-a]pyrimidine-3-carboxylic acid

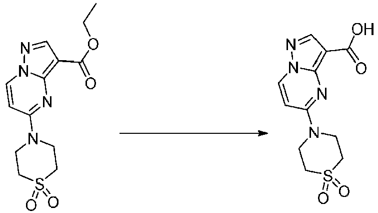

Ethyl 5-(1,1-dioxothiomorpholinyl)pyrazolo[1,5-*a*]pyrimidine-3-carboxylate (900 mg, 2.8 mmol) was dissolved in a mixed solution (methanol/water = 5:1, 10 mL), lithium hydroxide (470 mg, 11.2 mmol) was added, and the mixture was heated to 60 °C and stirred for 2 h. The reaction liquid was adjusted to pH = 3 with dilute hydrochloric acid (2 mol/L) and filtered, and the filter cake was dried under vacuum to give 5-(1,1-dioxothiomorpholinyl)pyrazolo[1,5-*a*]pyrimidine-3-carboxylic acid.

LC-MS: (ESI, m/z): [M+H]⁺ = 297.0.

### Step 3: preparation of tert-butyl 4-(3-(difluoromethyl)-4-(5-(1,1-dioxothiomorpholinyl)pyrazolo[1,5-a]pyrimidine-3-carboxamido)-1H-pyrazol-1-yl)piperidine-1-carboxylate

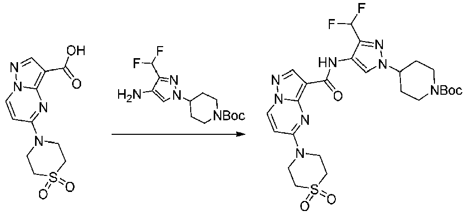

5-(1,1-Dioxothiomorpholinyl)pyrazolo[1,5-*a*]pyrimidine-3-carboxylic acid (150 mg, 0.51 mmol) and tert-butyl 4-(4-amino-3-(difluoromethyl)-1*H*-pyrazol-1-yl)piperidine-1-carboxylate (161 mg, 0.51 mmol) were dissolved in DMF (5 mL), DIEA (271 mg, 2.1 mmol) and HATU (251 mg, 0.66 mmol) were added while stirring, and the reaction liquid was stirred at room temperature for 12 h. The reaction liquid was diluted with water (60 mL) and extracted with ethyl acetate (60 mL × 3). The organic phase was washed with saturated brine (100 mL), dried over anhydrous sodium sulfate, filtered, and concentrated under reduced pressure. The crude product was purified by column chromatography (ethyl acetate:petroleum ether = 7:3) to give tert-butyl 4-(3-(difluoromethyl)-4-(5-(1,1-dioxothiomorpholinyl)pyrazolo[1,5-*a*]pyrimidine-3-carboxamido)-1H-pyrazol-1-yl)piperidine-1-carboxylate.

LC-MS: (ESI, m/z): [M+Na]⁺= 617. 1.

### Step 4: preparation of N-(3-(difluoromethyl)-1-(piperidin-4-yl)-1H-pyrazol-4-yl)-5-(1,1-dioxothiomorpholinyl)pyrazolo[1,5-a]pyrimidine-3-carboxamide

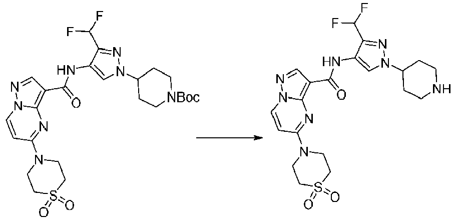

*tert*-Butyl 4-(3-(difluoromethyl)-4-(5-(1,1-dioxothiomorpholinyl)pyrazolo[1,5-*α*]pyrimidine-3-carboxamido)-1*H*-pyrazol-1-yl)piperidine-1-carboxylate (80 mg, 0.13 mmol) was dissolved in dichloromethane (2 mL), trifluoroacetic acid (0.4 mL) was added, and the mixture was stirred at room temperature for 2 h. The reaction liquid was directly concentrated under reduced pressure to give *N*-(3-(difluoromethyl)-1-(piperidin-4-yl)-1*H*-pyrazol-4-yl)-5-(1,1-dioxothiomorpholinyl)pyrazolo[1,5-*a*]pyrimidine-3-carboxamide, which was directly used in the next step without purification.

LC-MS: (ESI, m/z): [M+H]⁺ = 495.2.

### Intermediate 32: 3-(3-(2,4-dioxotetrahydropyrimidin-1(2H)-yl)-4-methylbenzoyl)-3-azaspiro[5.5]undecane-9-carbaldehyde

### Step 1: preparation of 3-azaspiro[5.5]undecane-9-carbaldehyde

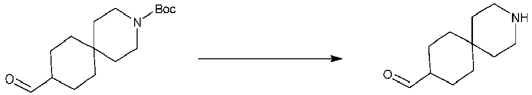

tert-Butyl 9-formyl-3-azaspiro[5.5]undecane-3-carboxylate (200 mg, 0.71 mmol) was dissolved in DCM (2 mL), TFA (0.4 mL) was added, and the reaction liquid was stirred at room temperature for 1 h. The reaction liquid was directly concentrated under reduced pressure to give 3-azaspiro[5.5]undecane-9-carbaldehyde.

LC-MS: (ESI, m/z): [M+H]^{I} = 182.2.

### Step 2: preparation of 3-(3-(2,4-dioxotetrahydropyrimidin-1(2H)-yl)-4-methylbenzoyl)-3-azaspiro[5.5]undecane-9-carbaldehyde

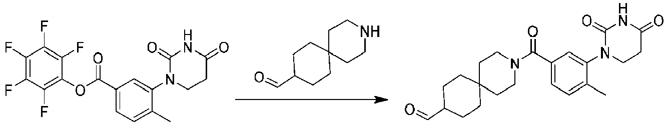

3-Azaspiro[5.5]undecane-9-carbaldehyde (345 mg, 0.71 mmol) and pentafluorophenyl 3-(2,4-dioxotetrahydropyrimidin-1(2*H*)-yl)-4-methylbenzoate (295 mg, 0.71 mmol) were dissolved in DMSO (5 mL), and the mixture was stirred at room temperature for 1 h. Water (50 mL) was added to the reaction liquid. The mixture was extracted with EA (30 mL × 3). The organic phases were combined, washed with saturated brine (50 mL), dried over anhydrous sodium sulfate, filtered, and concentrated under reduced pressure. The crude product was purified by normal phase column chromatography to give 3-(3-(2,4-dioxotetrahydropyrimidin-1(2*H*)-yl)-4-methylbenzoyl)-3-azaspiro[5.5]undecane-9-carbaldehyde.

LC-MS: (ESI, m/z): [M+H]⁺ = 412.2.

### Intermediate 33: 3-(4-chloro-3-(2,4-dioxotetrahydropyrimidin-1(2H)-yl)benzoyl)-3-azaspiro[5.5]undecane-9-carbaldehyde

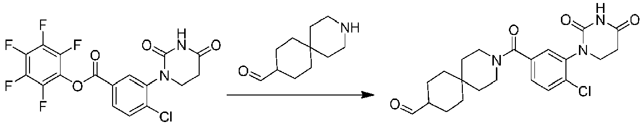

Intermediate 33 was prepared by referring to the method for intermediate 32.

LC-MS: (ESI, m/z): [M+H]^{|} = 432.1.

### Intermediate 34: tert-butyl (S)-(1-(3-((3-(difluoromethyl)-1-(piperidin-4-yl)-1H-pyrazol-4-yl)carbamoyl)pyrazolo[1,5-a]pyrimidin-5-yl)piperidin-3-yl)carbamate

### Step 1: preparation of ethyl (S)-5-(3-((tert-butoxycarbonyl)amino)piperidin-1-yl)pyrazolo[1,5-α]pyrimidine-3-carboxylate

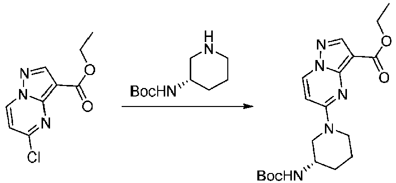

Ethyl 5-chloropyrazolo[1,5-*a*]pyrimidine-3-carboxylate (2.0 g, 8.9 mmol), tert-butyl (*S*)-piperidin-3-ylcarboxylate (1.8 g, 8.9 mmol), and *N,N-*diisopropylethylamine (3.5 g, 27 mmol) were dissolved in acetonitrile (20 mL). The reaction liquid was heated to 60 °C, stirred overnight, and directly concentrated under reduced pressure. The crude product was purified by column chromatography (ethyl acetate:petroleum ether = 9:1) to give ethyl (*S*)-5-(3-((*tert*-butoxycarbonyl)amino)piperidin-1-yl)pyrazolo[1,5-*a*]pyrimidine-3-carboxylate.

LC-MS: (ESI, m/z): [M+H]⁺= 390.0.

### Step 2: preparation of (S)-5-(3-((tert-butoxycarbonyl)amino)piperidin-1-yl)pyrazolo[1,5-a]pyrimidine-3-carboxylic acid

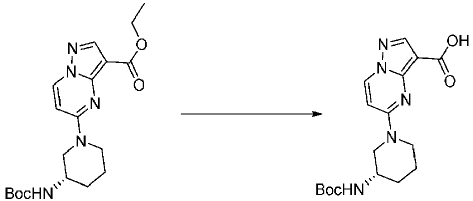

Ethyl (*S*)-5-(3-((*tert*-butoxycarbonyl)amino)piperidin-1-yl)pyrazolo[1,5-*a*]pyrimidine-3-carboxylate (3.2 g, 8.2 mmol) was dissolved in a mixed solution (methanol/water = 5:1, 30 mL), lithium hydroxide (787 mg, 32.8 mmol) was added, and the mixture was heated to 60 °C and stirred for 2 h. The reaction liquid was adjusted to pH = 2 with dilute hydrochloric acid (2 mol/L) and filtered, and the filter cake was dried under vacuum to give (*S*)-5-(3-((*tert*-butoxycarbonyl)amino)piperidin-1-yl)pyrazolo[1,5-*a*]pyrimidine-3-carboxylic acid.

LC-MS: (ESI, m/z): [M+H]^{|} = 362.5.

### Step 3: preparation of 4-(3-(difluoromethyl)-4-nitro- I H-pyrazol- I -yl)piperidine

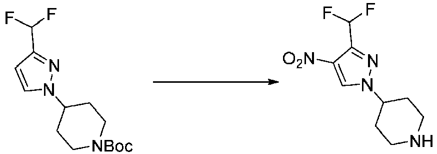

tert-Butyl 4-(3-(difluoromethyl)-1*H*-pyrazol-1-yl)piperidine-1-carboxylate (1 g, 3 mmol) was dissolved in concentrated sulfuric acid (10 mL) and cooled to zero. Concentrated nitric acid (1 mL) was slowly added dropwise, and the reaction liquid was stirred at room temperature overnight. The reaction liquid was slowly poured into ice water (300 mL). The aqueous phase was adjusted to pH = 8 with sodium carbonate, extracted with a mixed solution of methanol/dichloromethane (10/1, 60 mL × 3), dried over anhydrous sodium sulfate, filtered, and concentrated under reduced pressure to give 4-(3-(difluoromethyl)-4-nitro-1 H-pyrazol-1 - yl)piperidine.

LC-MS: (ESI, m/z): [M+H]^{|} = 247.0.

### Step 4: preparation of benzyl 4-(3-(difluoromethyl)-4-nitro-1H-pyrazol-1-yl)piperidine-1-carboxylate

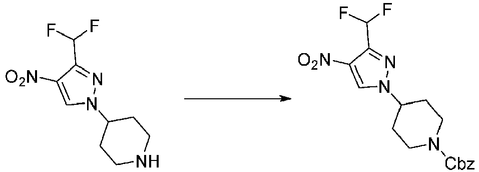

4-(3-(Difluoromethyl)-4-nitro-1*H*-pyrazol-1-yl)piperidine (460 mg, 1.9 mmol) was dissolved in dichloromethane (6 mL), DIEA (735 mg, 5.7 mmol) and CbzCl (391 mg, 2.3 mmol) were added, and the mixture was stirred at room temperature for 2 h. Water (80 mL) was added to the reaction liquid, and the mixture was extracted with dichloromethane (100 mL × 3). The organic phase was dried over anhydrous sodium sulfate, filtered, and concentrated under reduced pressure. The crude product was purified by column chromatography (ethyl acetate:petroleum ether = 1:1) to give benzyl 4-(3-(difluoromethyl)-4-nitro-1*H-*pyrazol-1-yl)piperidine-1-carboxylate.

LC-MS: (ESI, m/z): [M+H]⁺= 381.2.

### Step 5: preparation of benzyl 4-(4-amino-3-(difluoromethyl)-1Hpyrazol-1-yl)piperidine-1-carboxylate

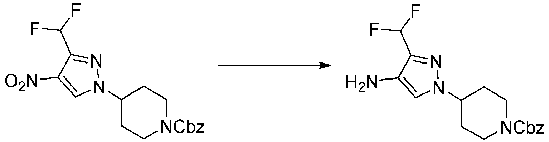

Benzyl 4-(3-(difluoromethyl)-4-nitro-1*H*-pyrazol-1-yl)piperidine-1-carboxylate (500 mg, 1.3 mmol), iron powder (294 mg, 5.2 mmol), and NH₄Cl (418 mg, 7.9 mmol) were added to water (10 mL). The reaction liquid was heated to 90 °C, stirred for 2 h, filtered, diluted with water (80 mL), and extracted with ethyl acetate (100 mL × 3). The organic phase was dried over anhydrous sodium sulfate, filtered, and concentrated under reduced pressure to give benzyl 4-(4-amino-3-(difluoromethyl)-1*H*-pyrazol-1-yl)piperidine-1-carboxylate.

LC-MS: (ESI, m/z): [M+H]⁺ = 351.2.

### Step 6: preparation of benzyl (S)-4-(4-(5-(3-((tert-butoxycarbonyl)amino)piperidin-1-yl)pyrazolo[1,5-a]pyrimidine-3-carboxamido)-3-(difluoromethyl)-1H-pyrazol-1 -yl)piperidine-1 -carboxylate

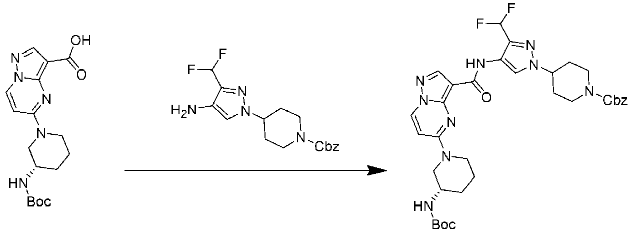

(*S*)-5-(3-((*tert*-Butoxycarbonyl)amino)piperidin-1-yl)pyrazolo[1,5-*a*]pyrimidine-3-carboxylic acid (56 mg, 0.15 mmol), benzyl 4-(4-amino-3-(difluoromethyl)-1*H*-pyrazol-1-yl)piperidine-1-carboacylate (54 mg, 0.15 mmol), and Py-BOP (240 mg, 0.45 mmol) were dissolved in DMF (5 mL), then DIEA (60 mg, 0.45 mmol) was added, and the mixture was stirred at room temperature for 16 h. Water (50 mL) was added to the reaction liquid, and the mixture was extracted with ethyl acetate (30 mL × 3). The organic phases were combined, washed with saturated brine (50 mL), dried over anhydrous sodium sulfate, filtered, and concentrated under reduced pressure. The crude product was purified by normal phase column chromatography to give benzyl (*S*)-4-(4-(5-(3-((*tert*-butoxycarbonyl)amino)piperidin-1-yl)pyrazolo[1,5-*α*]pyrimidine-3-carboxamido)-3-(difluoromethyl)-1*H*-pyrazol-1-yl)piperidine-1-carboxylate.

LC-MS: (ESI, m/z): [M+H]^{|} = 694.4.

### Step 7: preparation of tert-butyl (S)-(1-(3-((3-(difluoromethyl)-1-(piperidin-4-yl)-1H-pyrazol-4-yl)carbamoyl)pyrazolo[1,5-a]pyrimidin-5-yl)piperidin-3-yl)carbamate

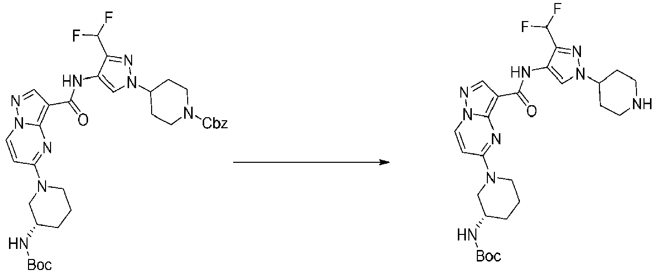

Benzyl (*S*)-4-(4-(5-(3-((*tert*-butoxycarbonyl)amino)piperidin-1-yl)pyrazolo[1,5-*a*]pyrimidine-3-carboxamido)-3-(difluoromethyl)-1*H*-pyrazol-1-yl)piperidine-1-carboacylate (90 mg, 0.13 mmol) was dissolved in THF (3 mL), and Pd/C (10%, 50 mg) was added. The mixture was purged three times with H₂ and stirred at room temperature for 1 h under hydrogen atmosphere. The reaction liquid was filtered through celite, the filter cake was washed with THF (10 mL × 3), and the filtrate was concentrated under reduced pressure to give a crude product of tert-butyl (*S*)-(1-(3-((3-(difluoromethyl)-1-(piperidin-4-yl)-1*H*-pyrazol-4-yl)carbamoyl)pyrazolo[1,5-*a*]pyrimidin-5-yl)piperidin-3-yl)carbamate.

LC-MS: (ESI, m/z): [M+H]⁺= 560.3.

### Example 1: 5-((1R,4R)-2-oxa-5-azabicyclo[2.2.1]heptan-5-yl)-N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)pyrazolo[1,5-a]pyrimidine-3-carboxamide

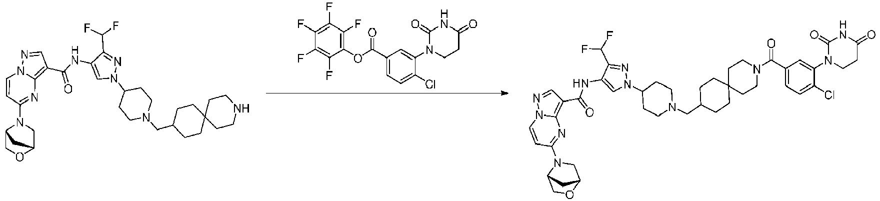

A solution of *N*-(1-(1-((3-azaspiro[5.5]undecan-9-yl)methyl)piperidin-4-yl)-3-(difluoromethyl)-1*H-*pyrazol-4-yl)-5-((1*R*,4*R*)-2-oxa-5-azabicyclo[2.2.1]heptan-5-yl)pyrazolo[1,5-*a*]pyrimidine-3-carboxamide (150 mg, crude, 0.18 mmol), pentafluorophenyl 4-chloro-3-(2,4-dioxotetrahydropyrimidin-1(2*H*)-yl)benzoate (78 mg, 0.18 mmol), and *N,N-*diisopropylethylamine (116 mg, 0.9 mmol) in dimethyl sulfoxide (5 mL) was stirred at room temperature overnight. The resulting mixture was poured into water (50 mL) and stirred for 10 min. The mixture was filtered, and the filter cake was purified by preparative HPLC to give the target product, 5-((1*R*,4*R*)-2-oxa-5-azabicyclo[2.2.1]heptan-5-yl)-*N*(1-(1-((3-(4-chloro-3-(2,4-dioxotetrahydropyrimidin-1 (2H)-yl)benzoyl)-3-azaspiro[5.5]undecan-9-yl)methyl)piperidin-4-yl)-3-(difluoromethyl)-1*H*-pyrazol-4-yl)pyrazolo[1,5-*a*]pyrimidine-3-carboxamide.

LC-MS: (ESI, *m*/*z):* [M+H]⁺= 874.5.

¹H NMR (400 MHz, DMSO-*d*₆) δ 10.51 (s, 1H), 9.50 (d, *J* = 5.9 Hz, 1H), 8.78 (d, *J* = 7.7 Hz, 1H), 8.39 (d, *J=* 4.0 Hz, 1H), 8.26 (d, *J=* 5.7 Hz, 1H), 7.63 (d, *J=* 8.2 Hz, 1H), 7.55 (d, *J=* 1.9 Hz, 1H), 7.39 (dd, *J* = 8.3, 2.0 Hz, 1H), 7.27-6.96 (m, 1H), 6.90-6.41 (m, 1H), 5.30-5.05 (m, 1H), 4.77 (d, *J* = 17.4 Hz, 1H), 4.26-4.14 (m, 1H), 3.86-3.70 (m, 3H), 3.66-3.41 (m, 5H), 3.30-3.25 (m, 2H), 2.96-2.84 (m, 2H), 2.79-2.70 (m, 2H), 2.22-2.08 (m, 2H), 2.06-1.83 (m, 8H), 1.75-1.65 (m, 2H), 1.63-1.24 (m, 7H), 1.19-0.91 (m, 4H).

### Example 2: 5-((1R,4R)-2-oxa-5-azabicyclo[2.2.1]heptan-5-yl)-N-(1-((1r,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1Hpyrazol-4-yl)pyrazolo[1,5-a]pyrimidine-3-carboxamide

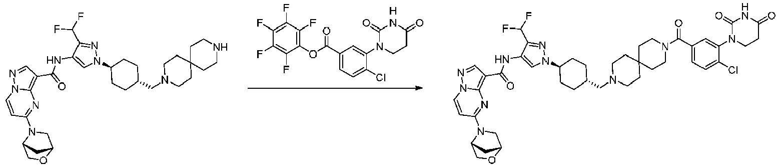

Example 2 was prepared by referring to the method for Example 1.

LC-MS: (ESI, m/z): [M+H]⁺ = 874.4

¹H NMR (400 MHz, DMSO-*d*₆) δ 10.51 (s, 1H), 9.50 (d, *J=* 6.3 Hz, 1H), 8.78 (d, *J* = 7.7 Hz, 1H), 8.38 (d, *J=* 4.4 Hz, 1H), 8.25 (d, *J=* 5.7 Hz, 1H), 7.64 (d, *J=* 8.2 Hz, 1H), 7.55 (d, *J=* 1.9 Hz, 1H), 7.39 (dd, *J* = 8.2, 1.9 Hz, 1H), 7.26-6.95 (m, 1H), 6.91-6.41 (m, 1H), 5.32-5.03 (m, 1H), 4.77 (d, *J* = 17.4 Hz, 1H), 4.17 (t, *J* = 12.9 Hz, 1H), 3.84-3.71 (m, 3H), 3.67-3.57 (m, 4H), 3.45 (d, *J* = 9.8 Hz, 1H), 3.30-3.25 (m, 2H), 2.79-2.71 (m, 2H), 2.38-2.24 (m, 4H), 2.13-1.77 (m, 8H), 1.79-1.66 (m, 2H), 1.60-1.35 (m, 9H), 1.12-0.92 (m, 2H).

### Example 3: 5-((1R,4R)-2-oxa-5-azabicyclo[2.2.1]heptan-5-yl)-N-(1-((1R,4R)-4-((4-(4-chloro-3-(2,4-dioxotetrahydropyrimidin-1(2H)-yl)benzoyl)piperazin-1-yl)methyl)cyclohexyl)-3-(difluoromethyl)-1H-pyrazol-4-yl)pyrazolo[1,5-a]pyrimidine-3-carboxamide

### Step 1: preparation of tert-butyl 4-(((1R,4R)-4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)cyclohexyl)methyl)piperazine-1-carboxylate

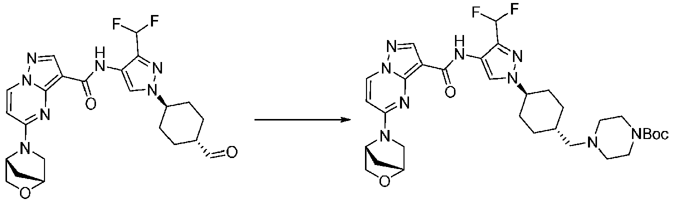

5-((1*R*,4*R*)-2-Oxa-5-aaabicyclo[2.2.1]heptan-5-yl)-*N-*(3-(difluoromethyl)-1-((1R,4R)-4-formylcyclohexyl)-1*H*-pyrazol-4-yl)pyrazolo[1,5-*a*]pyrimidine-3-carboxamide (130 mg, 0.268 mmol) and *tert*-butyl piperazine-1-carboxylate (60 mg, 0.322 mmol) were dissolved in tetrahydrofuran (10 mL), and the mixture was stirred at room temperature. Sodium triacetoxyborohydride (284 mg, 1.34 mmol) was added, and the mixture was stirred at room temperature for 2 h. Water (30 mL) was added, and the mixture was extracted with ethyl acetate (50 mL × 3). The organic phase was washed with saturated brine (50 mL), dried over anhydrous sodium sulfate, filtered, and concentrated under reduced pressure. The crude product was purified by column chromatography (methanol:dichloromethane = 1:9) to give *tert-butyl4-(((1R,4R)-4-(4-(5-((1R,4R)-*2-oxa-5-azabicyclo[2.2.1]heptan-5-yl)pyrazolo[1,5-*a*]pyrimidine-3-carboxamido)-3-(difluoromethyl)-1*H-*pyrazol-1 -yl)cyclohexyl)methyl)piperazine-1 -carboxylate.

LC-MS: (ESI, *m*/*z):* [M+H]⁺ = 656.3.

### Step 2: preparation of 5-((1R,4R)-2-oxa-5-azabicyclo[2.2.1]heptan-5-yl)-N-(3-(difluoromethyl)-1-((1R,4R)-4-(piperazin-1-ylmethyl)cyclohexyl)-1H-pyrazol-4-yl)pyrazolo[1,5-a]pyrimidine-3-carboxamide

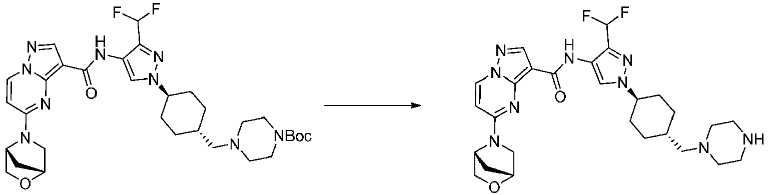

*tert-Butyl* 4-(((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)cyclohexyl)methyl)piperazine-1 - carboxylate (120 mg, 0.183 mmol) was dissolved in dichloromethane (5 mL), trifluoroacetic acid (1 mL) was added, and the mixture was stirred at room temperature for 2 h. The reaction liquid was directly concentrated under reduced pressure to give 5-((1*R*,4*R*)-2-oxa-5-azabicyclo[2.2.1]heptan-5-yl)-*N-*(3-(difluoromethyl)-1-((1*R*,4*R*)-4-(piperazin-1-ylmethyl)cyclohexyl)-1*H*-pyrazol-4-yl)pyrazolo[1,5-*a*]pyrimidine-3-carboxamide, which was directly used in the next step without purification.

LC-MS: (ESI, *m*/*z):* [M+H]⁺ = 556.3

### Step 3: preparation of 5-((1R,4R)-2-oxa-5-azabicyclo[2.2.1]heptan-5-yl)-N-(1-((1R,4R)-4-((4-(4-chloro-3-(2,4-dioxotetrahydropyrimidin-1(2H)-yl)benzoyl)piperazin-1-yl)methyl)cyclohexyl)-3-(difluoromethyl)-1H-pyrazol-4-yl)pyrazolo[1,5-a]pyrimidine-3-carboxamide

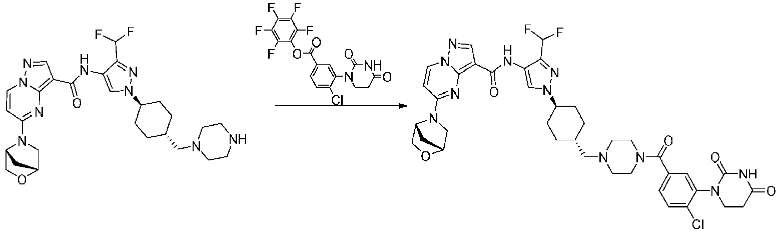

5-((1*R*,4*R*)-2-Oxa-5-azabicyclo[2.2.1]heptan-5-yl)-*N-*(3-(difluoromethyl)-1-((1*R*,4*R*)-4-(piperazin-1-ylmethyl)cyclohexyl)-1H-pyrazol-4-yl)pyrazolo[1,5-*a*]pyrimidine-3-carboxamide (120 mg, 0.185 mmol, trifluoroacetate) and pentafluorophenyl 4-chloro-3-(2,4-dioxotetrahydropyrimidin-1(2*H*)-yl)benzoate (96 mg, 0.22 mmol) were dissolved in dimethyl sulfoxide (10 mL), and the mixture was stirred at room temperature. DIEA (0.5 mL) was added, and the mixture was stirred at room temperature for 2 h. Water (50 mL) was added, and the mixture was extracted with ethyl acetate (50 mL × 3). The organic phase was washed with saturated brine (50 mL), dried over anhydrous sodium sulfate, and concentrated under reduced pressure. The crude product was purified by pre-HPLC to give 5-((1*R*,4*R*)-2-oxa-5-azabicyclo[2.2.1]heptan-5-yl)-*N-*(1-((1*R*,4*R*)-4-((4-(4-chloro-3-(2,4-dioxotetrahydropyrimidin-1(2*H*)-yl)benzoyl)piperazin-1-yl)methyl)cyclohexyl)-3-(difluoromethyl)-1*H-*pyrazol-4-yl)pyrazolo[1,5-*a*]pyrimidine-3-carboxamide.

LC-MS: (ESI, *m*/*z*): [M+H]⁺= 806.2.

¹H NMR (400 MHz, DMSO-*d*₆) δ 10.50 (s, 1H), 9.49 (d, *J=* 6.0 Hz, 1H), 8.78 (d, *J=* 7.8 Hz, 1H), 8.38 (d, *J* = 4.3 Hz, 1H), 8.25 (d, *J* = 5.6 Hz, 1H), 7.65 (d, *J=* 8.2 Hz, 1H), 7.57 (d, *J* = 2.0 Hz, 1H), 7.41 (dd, *J* = 8.2, 2.0 Hz, 1H), 7.27-6.94 (m, 1H), 6.89-6.38 (m, 1H), 5.32-5.02 (m, 1H), 4.77 (d, *J* = 17.6 Hz, 1H), 4.25-4.15 (m, 1H), 3.86-3.71 (m, 3H), 3.68-3.57 (m, 4H), 3.45 (d, *J=* 10.3 Hz, 2H), 2.80-2.70 (m, 2H), 2.45-2.25 (m, 4H), 2.16 (d, *J* = 7.0 Hz, 2H), 2.11-1.83 (m, 7H), 1.1-1.67 (m, 2H), 1.68-1.50 (m, 1H), 1.16-0.98 (m, 2H).

### Example 4: 5-((1R,4R)-2-oxa-5-azabicyclo[2.2.1]heptan-5-yl)-N-(1-((1R,4R)-4-((((1-(4-chloro-3-(2,4-dioxotetrahydropyrimidin-1(2H)-yl)benzoyl)piperidin-4-yl)methyl)(methyl)amino)methyl)cyclohexyl)-3-(difluoromethyl)-1H-pyrazol-4-yl)pyrazolo[1,5-a]pyrimidine-3-carboxamide

### Step 1: preparation of tert-butyl 4-(((((1R,4R)-4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)cyclohexyl)methyl)(methyl)amino)methyl)piperidine-1-carboxylate

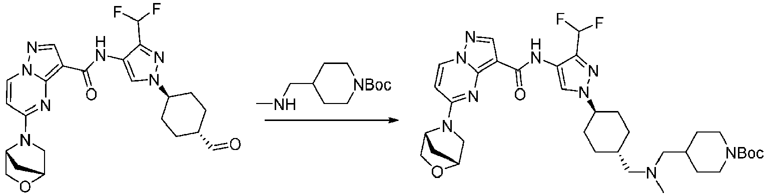

5-((1*R*,4*R*)-2-Oxa-5-azabicyclo[2.2.1]heptan-5-yl)-*N-*(3-(difluoromethyl)-1-((1*R*,4*R*)-4-formylcyclohexyl)-1*H*-pyrazol-4-yl)pyrazolo[1,5-*a*]pyrimidine-3-carboxamide (130 mg, 0.268 mmol) and tert-butyl 4-((methylamino)methyl)piperidine-1-carboxylate (73 mg, 0.320 mmol) were dissolved in tetrahydrofuran (10 mL), and the mixture was stirred at room temperature. Sodium triacetoxyborohydride (284 mg, 1.34 mmol) was added, and the mixture was stirred at room temperature for 2 h. Water (30 mL) was added, and the mixture was extracted with ethyl acetate (50 mL × 3). The organic phase was washed with saturated brine (50 mL), dried over anhydrous sodium sulfate, filtered, and concentrated under reduced pressure. The crude product was purified by column chromatography (methanol:dichloromethane = 1:9) to give tert-butyl 4-(((((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)cyclohexyl)methyl)(methyl)amino)methyl)piperidine-1-carboxylate.

LC-MS: (ESI, m/z): [M+H]⁺= 698.4.

### Step 2: preparation of 5-((1R,4R)-2-oxa-5-azabicyclo[2.2.1]heptan-5-yl)-N-(3-(difluoromethyl)-1-((1R,4R)-4-((methyl(piperidin-4-ylmethyl)amino)methyl)cyclohexyl)-1H-pyrazol-4-yl)pyrazolo[1,5-a]pyrimidine-3-carboxamide

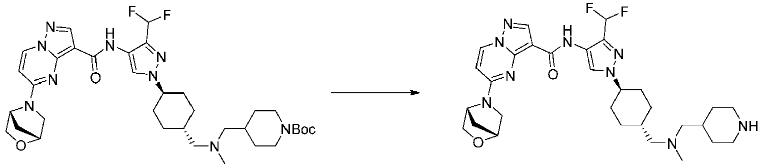

tert-Butyl 4-(((((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)cyclohexyl)methyl)(methyl)amino)methyl)piperidine-1-carboxylate (150 mg, 0.215 mmol) was dissolved in dichloromethane (5 mL), trifluoroacetic acid (1 mL) was added, and the mixture was stirred at room temperature for 2 h and directly concentrated under reduced pressure to give 5-((1*R*,4*R*)-2-oxa-5-azabicyclo[2.2.1]heptan-5-yl)-*N*-(3-(difluoromethyl)-1-((1*R*,4*R*)-4-((methyl(piperidin-4-ylmethyl)amino)methyl)cyclohexyl)-1*H*-pyrazol-4-yl)pyrazolo[1,5-*a*]pyrimidine-3-carboxamide, which was directly used in the next step without purification.

LC-MS: (ESI, m/z): [M+H]⁺ = 598.3.

### Step 3: preparation of 5-((1R,4R)-2-oxa-5-azabicyclo[2.2.1]heptan-5-yl)-N(1-((1R,4R)-4-((((1-(4-chloro-3-(2,4-dioxotetrahydropyrimidin-1(2H)-yl)benzoyl)piperidin-4-yl)methyl)(methyl)amino)methyl)cyclohexyl)-3-(difluoromethyl)-1H-pyrazol-4-yl)pyrazolo[1,5-a]pyrimidine-3-carboxamide

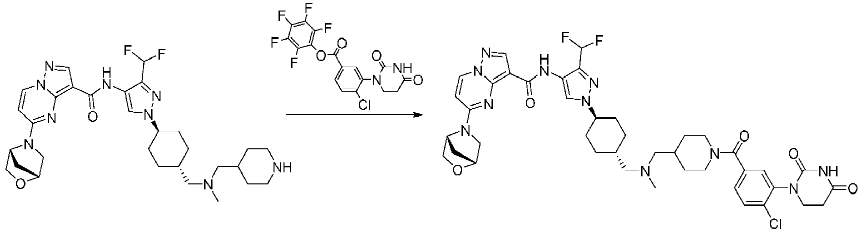

5-((1*R*,4*R*)-2-Oxa-5-azabicyclo[2.2.1]heptan-5-yl)-*N-*(3-(difluoromethyl)-1-((1*R*,4*R*)-4-((methyl(piperidin-4-ylmethyl)amino)methyl)cyclohexyl)-1*H*-pyrazol-4-yl)pyrazolo[1,5*-a*]pyrimidine-3-carboxamide (150 mg, 0.251 mmol, trifluoroacetate) and pentafluorophenyl 4-chloro-3-(2,4-dioxotetrahydropyrimidin-1(2*H*)-yl)benzoate (134 mg, 0.309 mmol) were dissolved in dimethyl sulfoxide (10 mL), and the mixture was stirred at room temperature. DIEA (0.5 mL) was added, and the mixture was stirred at room temperature for 2 h. Water (50 mL) was added, and the mixture was extracted with ethyl acetate (50 mL × 3). The organic phase was washed with saturated brine (50 mL), dried over anhydrous sodium sulfate, and concentrated under reduced pressure. The crude product was purified by pre-HPLC to give 5-((1*R*,4*R*)-2-oxa-5-azabicyclo[2.2.1]heptan-5-yl)-*N*-(1-((1*R*,4*R*)-4-((((1-(4-chloro-3-(2,4-dioxotetrahydropyrimidin-*1*(2*H*)-yl)benzoyl)piperidin-4-yl)methyl)(methyl)amino)methyl)cyclohexyl)-3-(difluoromethyl)-1*H*-pyrazol-4-yl)pyrazolo[1,5-*a*]pyrimidine-3-carboxamide.

LC-MS: (ESI, m/z): [M+H]⁺ = 848.3.

¹H NMR (400 MHz, DMSO-*d*₆) δ 10.50 (s, 1H), 9.50 (d, *J=* 5.8 Hz, 1H), 8.78 (d, *J=* 7.7 Hz, 1H), 8.38 (d, *J=* 4.4 Hz, 1H), 8.25 (d, *J=* 5.5 Hz, 1H), 8.17 (s, 1H), 7.64 (d, *J=* 8.2 Hz, 1H), 7.55 (d, *J=* 1.9 Hz, 1H), 7.38 (dd, J= 8.2, 2.0 Hz, 1H), 7.27-7.94 (m, 1H), 6.90-6.42 (m, 1H), 5.33-5.00 (m, 1H), 4.77 (d, *J=* 17.4 Hz, 1H), 4.52-4.33 (m, 1H), 4.17 (t, *J=* 10.6 Hz, 1H), 3.86-3.70 (m, 3H), 3.68-3.53 (m, 3H), 3.45 (d, *J=* 10.1 Hz, 1H), 3.14-2.96 (m, 1H), 2.90-2.68 (m, 3H), 2.25-1.46 (m, 19H), 1.18-0.88 (m, 4H).

### Example 5: 5-((1R,4R)-2-oxa-5-azabicyclo[2.2.1]heptan-5-yl)-N-(3-chloro-1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-1H-pyrazol-4-yl)pyrazolo[1,5-a]pyrimidine-3-carboxamide

### Step 1: preparation of methyl (1R,4R)-4-(4-nitro-1H-pyrazol-1-yl)cyclohexane-1-carboxylate

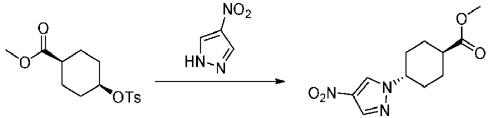

4-Nitro-1*H-*pyrazole (1.8 g, 15.9 mmol) and methyl (1S,4S)-4-(p-toluenesulfonyl)cyclohexane-1-carboxylate (5.2 g, 16.7 mmol) were dissolved in DMF (50 mL). Cs₂CO₃ (15.6 g, 47.7 mmol) and KI (2.78 g, 16.7 mmol) were added, and the mixture was stirred at 50 °C overnight. Water (200 mL) was added, and the mixture was stirred for 10 min and extracted with ethyl acetate (100 mL × 3). The organic phase was washed with saturated brine (150 mL), dried over anhydrous sodium sulfate, filtered, and concentrated under reduced pressure. The crude product was purified by column chromatography (EA:PE = 7:11) to give methyl (1R,4R)-4-(4-nitro-1*H*-pyrazol-1-yl)cyclohexane-1-carboxylate.

LC-MS: (ESI, *m*/*z):* [M+H]⁺= 254.1.

¹H NMR (400 MHz, DMSO-*d*₆) δ 8.90 (s, 1H), 8.26 (s, 1H), 4.33-4.25 (m, 1H), 3.63 (s, 3H), 2.42-2.35 (m, 1H), 2.15-2.00 (m, 4H), 1.89-1.76 (m, 2H), 1.59-1.45 (m, 2H).

### Step 2: preparation of methyl (1R,4R)-4-(4-amino-3-chloro-1H-pyrazol-1-yl)cyclohexane-1-carboxylate

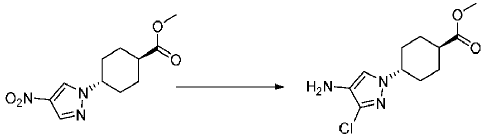

Methyl (1R,4R)-4-(4-nitro-1H-pyrazol-1-yl)cyclohexane-1-carboxylate (1.4 g, 5.5 mmol) was dissolved in ethanol (45 mL). HCl (4.6 mL, 55.3 mmol) and Pd (5%, alumina-supported, 140 mg) were added. Triethylsilane (3.85 g, 33.2 mmol) was added dropwise under N₂ atmosphere, and the mixture was stirred at room temperature overnight. The pH was adjusted to 8 with a saturated NaHCO₃ solution, and the mixture was extracted with ethyl acetate (150 mL × 3). The organic phase was washed with saturated brine (200 mL), dried over anhydrous sodium sulfate, filtered, and concentrated under reduced pressure. The crude product was purified by column chromatography (MeOH:DCM = 1:19) to give methyl (1R,4R)-4-(4-amino-3-chloro-1*H*-pyrazol-1-yl)cyclohexane-1-carboxylate.

LC-MS: (ESI, *m*/*z*): [M+H] ⁺ =258.1 and [M+14] ⁺ =272.1.

### Step 3: preparation of ((1R,4R)-4-(4-amino-3-chloro-1H-pyrazol-1-yl)cyclohexyl)methanol

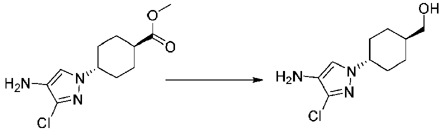

Methyl (1*R*,4*R*)-4-(4-amino-3-chloro-1*H-*pyrazol-1-yl)cyclohexane-1-carboxylate (1.0 g, 3.9 mmol) was dissolved in THF (20 mL), BH₃/THF (1 M, 39 mL, 39 mmol) was added, and the mixture was reacted at room temperature overnight. After the reaction was quenched with MeOH (10 mL), the mixture was concentrated under reduced pressure. The crude product was purified by column chromatography (MeOH:DCM = 1:19) to give ((1*R*,4*R*)-4-(4-amino-3-chloro-1*H*-pyrazol-1-yl)cyclohexyl)methanol.

LC-MS: (ESI, *m*/*z):* [M+H]⁺= 230.2.

### Step 4: preparation of 5-((1R,4R)-2-oxa-5-azabicyclo[2.2.1]heptan-5-yl)-N-(3-chloro-1-((1R,4R)-4-(hydroxymethyl)cyclohexyl)-1H-pyrazol-4-yl)pyrazolo[1,5-a]pyrimidine-3-carboxamide

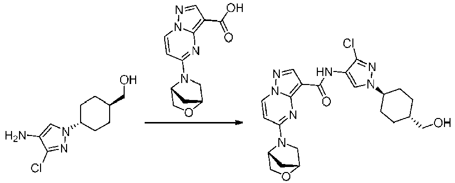

((1*R*,4*R*)-4-(4-Amino-3-chloro-1*H*-pyrazol-1-yl)cyclohexyl)methanol (300 mg, 1.3 mmol), HATU (570 mg, 1.5 mmol), and DIEA (387 mg, 3.0 mmol) were added to a solution of 5-((1*R*,4*R*)-2-oxa-5-azabicyclo[2.2.1]heptan-5-yl)pyrazolo[1,5-*a*]pyrimidine-3-carboxylic acid (260 mg, 1.0 mmol) in DMF (10 mL), and the mixture was stirred at room temperature overnight. Water (100 mL) was added, and the mixture was stirred for 10 min and extracted with ethyl acetate (100 mL × 3). The organic phase was washed with saturated brine (150 mL), dried over anhydrous sodium sulfate, filtered, and concentrated under reduced pressure. The crude product was purified by column chromatography (MeOH:DCM = 1:9) to give 5-((1*R*,4*R*)-2-oxa-5-azabicyclo[2.2.1]heptan-5-y1)-*N*-(3-chloro-1-((1*R*,4*R*)-4-(hydroxymethyl)cyclohexyl)-1*H*-pyrazol-4-yl)pyrazolo[1,5-*a*]pyrimidine-3-carboxamide.

LC-MS: (ESI, *m*/*z):* [M+H]⁺= 472.0.

### Step 5: preparation of 5-((1R,4R)-2-oxo-5-azabicyclo[2.2.1]heptan-5-yl)-N-(3-chloro-1-((1R,4R)-4-formylcyclohexyl)-1H-pyrazol-4-yl)pyrazolo[1,5-a]pyrimidine-3-carboxamide

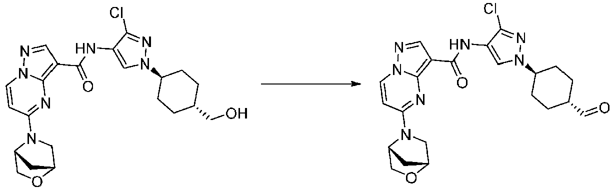

IBX (297 mg, 1.06 mmol) was added to a solution of 5-((1 *R*,4*R*)-2-oxa-5-azabicyclo[2.2.1]heptan-5-yl)-*N*-(3-chloro-1-((1*R*,4*R*)-4-(hydroxymethyl)cyclohexyl)-1*H*-pyrazol-4-yl)pyrazolo[1,5-*a*]pyrimidine-3-carboxamide (250 mg, 0.53 mmol) in ACN (20 mL), and the mixture was stirred at 85 °C for 1.5 h and concentrated under reduced pressure. The crude product was purified by column chromatography (MeOH:DCM = 1:9) to give 5-((1*R*,4*R*)-2-oxo-5-azabicyclo[2.2.1]heptan-5-yl)-*N*-(3-chloro-1-((1*R*,4*R*)-4-formylcyclohexyl)-1*H*-pyrazol-4-yl)pyrazolo[1,5-*a*]pyrimidine-3-carboxamide.

LC-MS: (ESI, m/z): [M+H]⁺= 470.2.

### Step 6: preparation of tert-butyl 9-(((1R,4R)-4-(4-(5-((1R,4R)-2-oxa-5-azabicyclo[2.2.1]heptan-5-yl)pyrazolo[1,5-a]pyrimidine-3-carboxamido)-3-chloro-1H-pyrazol-1-yl)cyclohexyl)methyl)-3,9-diazaspiro[5.5]undecane-3-carboxylate

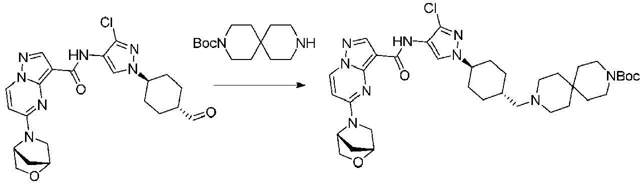

STAB (285 mg, 1.34 mmol) was added to a solution of 5-((1 *R*,4*R*)-2-oxo-5-azabicyclo[2.2.1]heptan-5-yl)-*N*-(3-chloro-1-((1*R*,4*R)*-4-formylcyclohexyl)-1*H*-pyrazol-4-yl)pyrazolo[1,5-*a*]pyrimidine-3-carboxamide (210 mg, 0.45 mmol) and tert-butyl 3,9-diazaspiro[5.5]undecane-3-carboxylate (114 mg, 0.45 mmol) in THF (10 mL), and the mixture was stirred at room temperature overnight and concentrated under reduced pressure. Water (30 mL) was added, and the mixture was extracted with ethyl acetate (30 mL × 3). The organic phase was washed with saturated brine (50 mL), dried over anhydrous sodium sulfate, filtered, and concentrated under reduced pressure. The crude product was purified by column chromatography (MeOH:DCM = 1:9) to give *tert-butyl* 9-(((1R,4R)-4-(4-(5-((1*R*,4*R*)-2-oxa-5-azabicyclo[2.2.1]heptan-5-yl)pyrazolo[1,5-*a*]pyrimidine-3-carboxamido)-3-chloro-1*H*-pyrazol-1-yl)cyclohexyl)methyl)-3,9-diazaspiro[5.5]undecane-3-carboxylate.

LC-MS: (ESI, *m*/*z*): [M+Na]⁺ = 708.2.

### Step 7: preparation of N-(1-((1R,4R)-4-((3,9-diazaspiro[5.5]undecan-3-yl)methyl)cyclohexyl)-3-chloro-1H-pyrazol-4-yl)-5-((1R,4R)-2-oxa-5-azabicyclo[2.2.1]heptan-5-yl)pyrazolo[1,5-a]pyrimidine-3-carboxamide

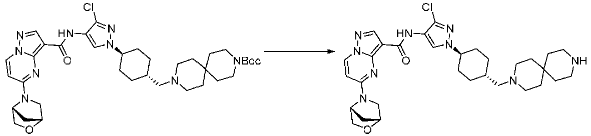

TFA (1 mL) was added to a solution of *tert*-butyl 9-(((1*R*,4*R*)-4-(4-(5-((1R,4R)-2-oxa-5-azabicyclo[2.2.1]heptan-5-yl)pyrazolo[1,5-*a*]pyrimidine-3-carboxamido)-3-chloro-1*H*-pyrazol-1-yl)cyclohexyl)methyl)-3,9-diazaspiro[5.5]undecane-3-carboxylate (190 mg, 0.27 mmol) in DCM (5 mL), and the mixture was stirred at room temperature for 2 h and concentrated under reduced pressure to give *N*-(1-((1*R*,4*R*)-4-((3,9-diazaspiro[5.5]undecan-3-yl)methyl)cyclohexyl)-3-chloro-1*H*-pyrazol-4-yl)-5-((1*R*,4*R*)-2-oxa-5-azabicyclo[2.2.1]heptan-5-yl)pyrazolo[1,5-*a*]pyrimidine-3-carboxamide.

LC-MS: (ESI, *m*/*z):* [M+H]' = 608.3.

### Step 8: preparation of 5-((1R,4R)-2-oxa-5-azabicyclo[2.2.1]heptan-5-yl)-N-(3-chloro-1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-1H-pyrazol-4-yl)pyrazolo[1,5-a]pyrimidine-3-carboxamide

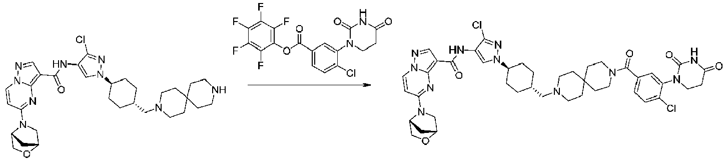

*N-*(1-((1*R*,4*R*)-4-((3,9-Diazaspiro[5.5]undecan-3-yl)methyl)cyclohexyl)-3-chloro-1*H*-pyrazol-4-yl)-5-((1*R*,4*R*)-2-oxa-5-azabicyclo[2.2.1]heptan-5-yl)pyrazolo[1,5*-a*]pyrimidine-3-carboxamide (190mg, 0.31 mmol) and pentafluorophenyl 4-chloro-3-(2,4-dioxotetrahydropyrimidin-1(2*H*)-yl)benzoate (136 mg, 0.31 mmol) were dissolved in DMSO (5 mL), then DIEA (202 mg, 1.6 mmol) was added, and the reaction liquid was stirred at room temperature for 2 h. Water (50 mL) was added to the reaction liquid, and the mixture was stirred for 10 min and filtered. The solid was purified by preparative reversed-phase chromatography to give 5-((1*R*,4*R*)-2-oxa-5-azabicyclo[2.2.1]heptan-5-yl)-N-(3-chloro-1-((1*R*,4*R*)-4-((9-(4-chloro-3-(2,4-dioxotetrahydropyrimidin-1(2*H*)-yl)benzoyl)-3,9-diazaspiro[5.5]undecan-3-yl)methyl)cyclohexyl)-1*H-*pyrazol-4-yl)pyrazolo[1,5-*a*]pyrimidine-3-carboxamide.

LC-MS: (ESI, *m*/*z*): [M+H]⁺= 858.2.

¹H NMR (400 MHz, DMSO-*d*₆) δ 10.50 (s, 1H), 9.44 (d, *J=* 8.7 Hz, 1H), 8.80 (d, *J=* 7.7 Hz, 1H), 8.24 (d, *J* = 3.5 Hz, 1H), 8.06 (s, 1H), 7.63 (d, *J* = 8.2 Hz, 1H), 7.55 (s, 1H), 7.39 (d, *J* = 8.3 Hz, 1H), 6.95-6.39 (m, 1H), 5.30-5.00 (m, 1H), 4.79 (s, 1H), 4.25-4.15 (m, 1H), 3.92-3.38 (m, 9H), 2.80-2.68 (m, 2H), 2.40-2.20 (m, 4H), 2.11 (d, *J* = 6.5 Hz, 2H), 2.06-1.95 (m, 2H), 1.94-1.76 (m, 6H), 1.70-1.30 (m, 9H), 1.10-0.95 (m, 2H).

### Example 6: 5-((1R,4R)-2-oxa-5-azabicyclo[2.2.1]heptan-5-yl)-N-(2-chloro-4-(4-((9-(4-chloro-3-(2,4-dioxotetrahydropyrimidin-1(2H)-yl)benzoyl)-3,9-diazaspiro[5.5]undecan-3-yl)methyl)piperidin-1-yl)phenyl)pyrazolo[1,5-a]pyrimidine-3-carboxamide

### Step 1: preparation of (1-(3-chloro-4-nitrophenyl)piperidin-4-yl)methanol

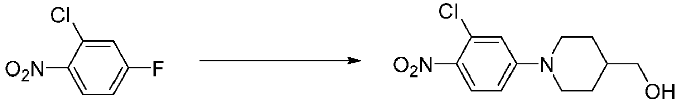

K₂CO₃ (1.57 g, 11.4 mmol) and 2-(piperidin-4-yl)ethan-1-ol (0.78 g, 6.8 mmol) were added to a solution of 2-chloro-4-fluoro-1-nitrobenzene (1.0 g, 5.7 mmol) in DMF (20 mL), and the mixture was stirred at 100 °C for 1 h. Water (200 mL) was added, and the mixture was stirred for 10 min and extracted with ethyl acetate (100 mL × 3). The organic phase was washed with saturated brine (150 mL), dried over anhydrous sodium sulfate, filtered, and concentrated under reduced pressure. The crude product was purified by column chromatography (EA:PE = 1:1) to give (1-(3-chloro-4-nitrophenyl)piperidin-4-yl)methanol.

LC-MS: (ESI, *m*/*z):* [M+H]⁺= 271.0.

### Step 2: preparation of (1-(4-amino-3-chlorophenyl)piperidin-4-yl)methanol

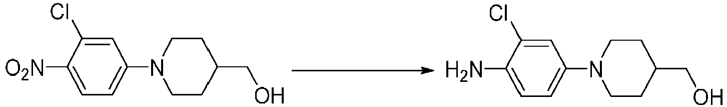

(1-(3-Chloro-4-nitrophenyl)piperidin-4-yl)methanol (1.3 g, 4.8 mmol) was dissolved in ethanol (30 mL), Raney-Ni (1 mL) and N₂H₄-H₂O (1 mL) were added, and the mixture was stirred at room temperature for 1 h, filtered, concentrated, and dried to give (1-(4-amino-3-chlorophenyl)piperidin-4-yl)methanol.

LC-MS: (ESI, *m*/*z*): [M+H]⁺= 241.1.

### Step 3: preparation of 5-((1R,4R)-2-oxa-5-azabicyclo[2.2.1]heptan-5-yl)-N-(2-chloro-4-(4-(hydroxymethyl)piperidin-1-yl)phenyl)pyrazolo[1,5-a]pyrimidine-3-carboxamide

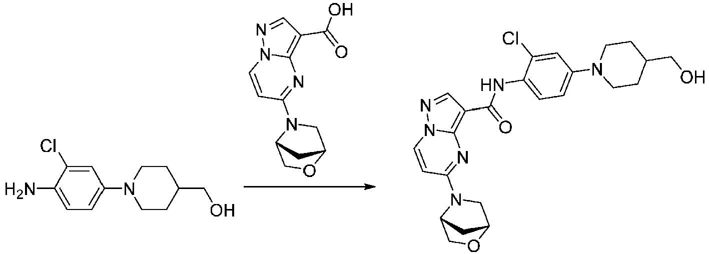

5-((1*R*,4*R*)-2-Oxa-5-azabicyclo[2.2.1]heptan-5-yl)pyrazolo[1,5-*a*]pyrimidine-3-carboxylic acid (0.6 g, 2.3 mmol), HATU (1.33 g, 3.5 mmol), and DIEA (0.89 g, 6.9 mmol) were added to a solution of (1-(4-amino-3-chlorophenyl)piperidin-4-yl)methanol (1.0 g, 4.2 mmol) in DMF (20 mL), and the mixture was stirred at room temperature overnight. Water (200 mL) was added, and the mixture was stirred for 10 min and extracted with ethyl acetate (100 mL × 3). The organic phase was washed with saturated brine (150 mL), dried over anhydrous sodium sulfate, filtered, and concentrated under reduced pressure. The crude product was purified by column chromatography (MeOH:DCM = 1:9) to give 5-((1R,4R)-2-oxa-5-azabicyclo[2.2.1]heptan-5-yl)-N-(2-chloro-4-(4-(hydroacymethyl)piperidin-1 -yl)phenyl)pyrazolo[1,5-*a*]pyrimidine-3-carboxamide.

LC-MS: (ESI, *m*/*z):* [M+H]^{|} = 483.1.

### Step 4: preparation of 5-((1R,4R)-2-oxa-5-azabicyclo[2.2.1]heptan-5-yl)-N-(2-chloro-4-(4-formylpiperidin-1-yl)phenyl)pyrazolo[1,5-a]pyrimidine-3-carboxamide

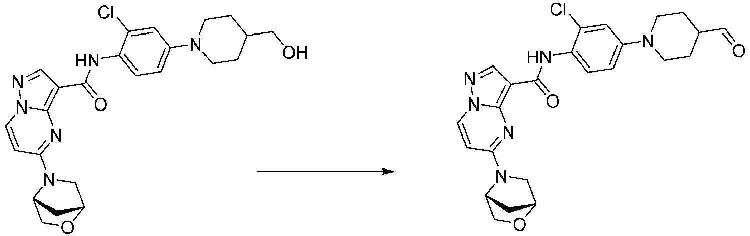

IBX (560 mg, 2.0 mmol) was added to a solution of 5-((1*R*,4*R*)-2-oxa-5-azabicyclo[2.2.1]heptan-5-yl)-*N-*(2-chloro-4-(4-(hydroxymethyl)piperidin-1-yl)phenyl)pyrazolo[1,5-*a*]pyrimidine-3-carboxamide(500 mg, 1.0 mmol) in CAN (20 mL), and the mixture was stirred at 100 °C for 1.5 h. The reaction liquid was directly concentrated under reduced pressure. The crude product was purified by column chromatography (MeOH:DCM = 1:9) to give 5-((1R,4R)-2-oxa-5-azabicyclo[2.2.1]heptan-5-yl)-N-(2-chloro-4-(4-formylpiperidin-1-yl)phenyl)pyrazolo[1,5-*a*]pyrimidine-3-carboxamide.

LC-MS: (ESI, *m*/*z):* [M+H]⁺= 481.1.

### Step 5: preparation of tert-butyl 9-((1-(4-(5-((1R,4R)-2-oxa-5-azabicyclo[2.2.1]heptan-5-yl)pyrazolo[1,5-a]pyrimidine-3-carboxamido)-3-chlorophenyl)piperidin-4-yl)methyl)-3,9-diazaspiro[5.5]undecane-3-carboxylate

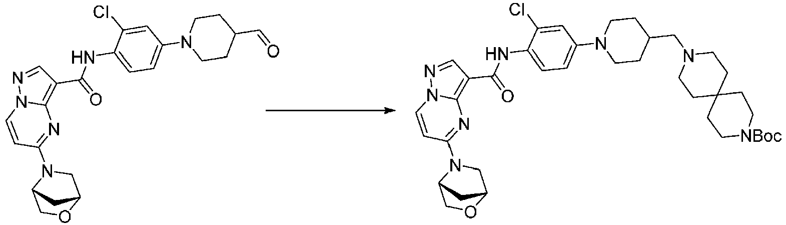

STAB (528 mg, 2.49 mmol) was added to a solution of 5-((1*R*,4*R*)-2-oxa-5-azabicyclo[2.2.1]heptan-5-yl)-*N*-(2-chloro-4-(4-formylpiperidin-1-yl)phenyl)pyrazolo[1,5-*a*]pyrimidine-3-carboxamide (400 mg, 0.83 mmol) and *tert*-butyl 3,9-diazaspiro[5.5]undecane-3-carboxylate (232 mg, 0.91 mmol) in THF (10 mL), and the mixture was stirred at room temperature overnight and concentrated under reduced pressure. Water (20 mL) was added, and the mixture was extracted with ethyl acetate (20 mL × 3). The organic phase was washed with saturated brine (50 mL), dried over anhydrous sodium sulfate, filtered, and concentrated under reduced pressure. The crude product was purified by column chromatography (MeOH:DCM = 1:9) to give *tert-butyl* 9-((1-(4-(5-((1*R*,4*R*)-2-oxa-5-azabicyclo[2.2.1]heptan-5-yl)pyrazolo[1,5-*a*]pyrimidine-3-carboxamido)-3-chlorophenyl)piperidin-4-yl)methyl)-3,9-diazaspiro[5.5]undecane-3-carboxylate.

LC-MS: (ESI, *m*/*z):* [M+Na]⁺ = 719.3.

### Step 6: preparation of N-(4-(4-((3,9-diazaspiro[5.5]undecan-3-yl)methyl)piperidin-1-yl)-2-chlorophenyl)-5-((1R,4R)-2-oxa-5-azabicyclo[2.2.1]undecan-5-yl)pyrazolo[1,5-a]pyrimidine-3-carboxamide

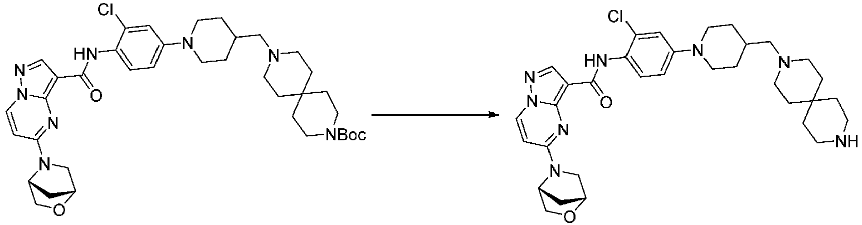

TFA (1 mL) was added to a solution of *tert*-butyl 9-((1-(4-(5-((1R,4R)-2-oxa-5-azabicyclo[2.2.1]heptan-5-yl)pyrazolo[1,5-*a*]pyrimidine-3-carboxamido)-3-chlorophenyl)piperidin-4-yl)methyl)-3,9-diazaspiro[5.5]undecane-3-carboxylate (150 mg, 0.21 mmol) in DCM (5 mL), and the mixture was stirred at room temperature for 2 h and concentrated under reduced pressure to give N-(4-(4-((3,9-diazaspiro[5.5]undecan-3-yl)methyl)piperidin-1-yl)-2-chlorophenyl)-5-((1*R*,4*R*)-2-oxa-5-azabicyclo[2.2.1]undecan-5-yl)pyrazolo[1,5-*a*]pyrimidine-3-carboxamide.

LC-MS: (ESI, *m*/*z):* [M+H]⁺= 619.2.

### Step 7: preparation of 5-((1R,4R)-2-oxa-5-azabicyclo[2.2.1]heptan-5-yl)-N-(2-chloro-4-(4-((9-(4-chloro-3-(2,4-dioxotetrahydropyrimidin-1(2H)-yl)benzoyl)-3,9-diazaspiro[5.5]undecan-3-yl)methyl)piperidin-1-yl)phenyl)pyrazolo[1,5-a]pyrimidine-3-carboxamide

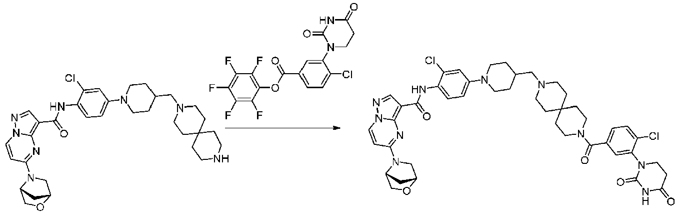

*N*-(4-(4-((3,9-Diazaspiro[5.5]undecan-3-yl)methyl)piperidin-1-yl)-2-chlorophenyl)-5-((1*R,*4*R*)-2-oxa-5-azabicyclo[2.2.1]undecan-5-yl)pyrazolo[1,5-*a*]pyrimidine-3-carboxamide (150 mg, 0.24 mmol) and pentafluorophenyl 4-chloro-3-(2,4-dioxotetrahydropyrimidin-1(2*H*)-yl)benzoate (105 mg, 0.24 mmol) were dissolved in DMSO (5 mL), DIEA (155 mg, 1.2 mmol) was added, and then the reaction liquid was stirred at room temperature for 2 h. Water (50 mL) was added to the reaction liquid, and the mixture was stirred for 2 min and filtered. The solid was purified by preparative reversed-phase chromatography to give 5-((1R,4R)-2-oxa-5-azabicyclo[2.2.1]heptan-5-yl)-*N*-(2-chloro-4-(4-((9-(4-chloro-3-(2,4-dioxotetrahydropyrimidin-1(2*H*)-yl)benzoyl)-3,9-diazaspiro[5.5]undecan-3-yl)methyl)piperidin-1-yl)phenyl)pyrazolo[1,5-*a*]pyrimidine-3-carboxamide.

LC-MS: (ESI, *m*/*z):* [M+H]⁺= 869.3.

¹H NMR (400 MHz, DMSO-*d*₆) δ 10.51 (s, 1H), 9.60 (d, *J=* 11.9 Hz, 1H), 8.80 (d, *J=* 7.7 Hz, 1H), 8.26 (d, *J=* 3.3 Hz, 1H), 8.08 (dd, *J=* 15.0, 9.1 Hz, 1H), 7.64 (d, *J=* 8.2 Hz, 1H), 7.55 (d, *J=* 2.0 Hz, 1H), 7.39 (dd, *J=* 8.2, 2.0 Hz, 1H), 7.00 (d, *J=* 2.4 Hz, 1H), 6.99-6.92 (m, 1H), 6.90-6.44 (m, 1H), 5.34-5.04 (m, 1H), 4.77 (d, *J=* 12.1 Hz, 1H), 3.84-3.70 (m, 3H), 3.68-3.43 (m, 7H), 3.30-3.20 (m, 2H), 2.78-2.70 (m, 2H), 2.64 (t, *J=* 11.6 Hz, 2H), 2.39-2.21 (m, 4H), 2.14 (d, *J=* 6.9 Hz, 2H), 2.07-1.92 (m, 2H), 1.82-1.30 (m, 11H), 1.23-1.08 (m, 2H).

### Example 7: 5-((1R,4R)-2-oxa-5-azabicyclo[2.2.1]heptan-5-yl)-N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-methyl-1H-pyrazol-4-yl)pyrazolo[1,5-a]pyrimidine-3-carboxamide

### Step 1: preparation of methyl (1R,4R)-4-(3-methyl-4-nitro-1H-pyrazol-1-yl)cyclohexane-1-carboxylate

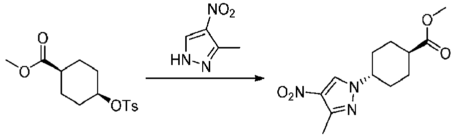

A solution of methyl (1*S*,4*S*)-4-(*p*-toluenesulfonyl)cyclohexane-1-carboxylate (2.0 g, 15.7 mmol), 3-methyl-4-nitro-1*H*-pyrazole (5.9 g, 18.8 mmol), Cs₂CO₃ (15.4 g, 47.1 mmol), and KI (3.1 g, 18.8 mmol) in DMF (30.0 mL) was stirred at 120 °C for 2 h. Water (200 mL) was added to the reaction liquid, and then the mixture was extracted with EA (200 mL × 2). The organic phases were combined, dried over anhydrous Na₂SO₄, filtered, and concentrated under reduced pressure. The resulting crude product was purified by column chromatography (EA:PE = 0-1:5) to give methyl (1*R*,4*R*)-4-(3-methyl-4-nitro-1*H*-pyrazol-1-yl)cyclohexane-1-carboxylate.

LC-MS: (ESI, *m*/*z*): [M+H]⁺= 268.0.

¹H NMR (400 MHz, DMSO-*d*₆) δ 8.79 (s, 1H), 4.24-4.14 (m, 1H), 3.61 (s, 3H), 2.42 (s, 3H), 2.44-2.34 (m, 1H), 2.14-1.96 (m, 4H), 1.87-1.71 (m, 2H), 1.62-1.41 (m, 2H).

### Step 2: preparation of methyl (1R,4R)-4-(4-amino-3-methyl-1H-pyrazol-1-yl)cyclohexane-1-carboxylate

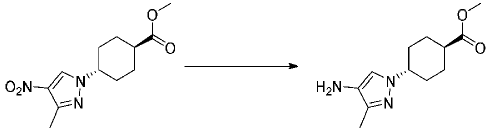

A solution of methyl (1R,4R)-4-(3-methyl-4-nitro-1H-pyrazol-1-yl)cyclohexane-1-carboxylate (2.0 g, 7.4 mmol), Fe (2.5 g, 37.0 mmol), and NH₄Cl (4.8 g, 74.0 mmol) in MeOH (50.0 mL) was stirred at 80 °C for 3 h. The reaction liquid was filtered through celite, and the filtrate was concentrated under reduced pressure. The resulting crude product was purified by Prep-HPLC (acetonitrile/0.05% aqueous NH₄OH solution, 5%-95%) to give methyl (1*R*,4*R*)-4-(4-amino-3-methyl-1*H*-pyrazol-1-yl)cyclohexane-1-carboxylate.

LC-MS: (ESI, m/z): [M+H]^{|} = 238.3.

### Step 3: preparation of ((1R,4R)-4-(4-amino-3-methyl-1H-pyrazol-1-yl)cyclohexyl)methanol

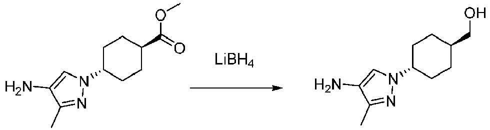

A solution of methyl (1*R*,4*R*)-4-(4-amino-3-methyl-1*H*-pyrazol-1-yl)cyclohexane-1-carboxylate (300.0 mg, 1.3 mmol) and LiBH₄ (111.0 mg, 4.0 mmol) in THF/MeOH (10.0 mL/2.0 mL) was stirred at 60 °C for 1 h. Water (20.0 mL) was added to quench the reaction, and then the mixture was extracted with DCM (50 mL × 2). The organic phase was dried over anhydrous Na₂SO₄, filtered, and concentrated under reduced pressure. The resulting crude product was purified by column chromatography (MeOH:DCM = 0-1:9) to give ((1*R*,4*R*)-4-(4-amino-3-methyl-1*H-*pyrazol-1-yl)cyclohexyl)methanol.

LC-MS: (ESI, *m*/*z*): [M+H]⁺= 210.1.

### Step 4: preparation of 5-((1R,4R)-2-oxa-5-azabicyclo[2.2.1]heptan-5-yl)-N (1-((1R,4R)-4-(hydroxymethyl)cyclohexyl)-3-methyl-1H-pyrazol-4-yl)pyrazolo[1,5-a]pyrimidine-3-carboxamide

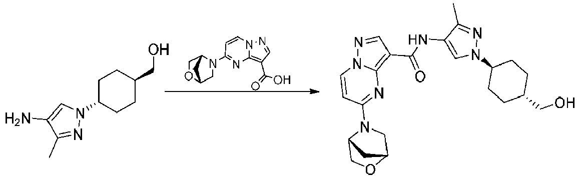

((1*R*,4*R*)-4-(4-Amino-3-methyl-1*H*-pyrazol-1-yl)cyclohexyl)methanol (230.0 mg, 1.1 mmol) was added to a solution of 5-((1*R*,4*R*)-2-oxa-5-azabicyclo[2.2.1]heptan-5-yl)pyrazolo[1,5-*a*]pyrimidine-3-carboxylic acid (260.0 mg, 1.0 mmol),HATU(418.0 mg, 1.1 mmol), andDIEA(387.0 mg, 3.0 mmol) in DMF (500.0 mL). The reaction liquid was stirred at room temperature for 1.0 h. Cold water (250.0 mL) was added to the reaction liquid. The resulting suspension was filtered, and the filter cake was washed with water and dried under vacuum to give 5-((1*R*,4*R*)-2-oxa-5-azabicyclo[2.2.1]heptan-5-yl)-*N-*(1-((1*R*,4*R*)-4-(hydroxymethyl)cyclohexyl)-3-methyl-1*H*-pyrazol-4-yl)pyrazolo[1,5-*a*]pyrimidine-3-carboxamide. The resulting crude product was directly used in the next step without purification.

LC-MS: (ESI, m/z): [M+H]⁺= 452.1.

### Step 5: preparation of 5-((1R,4R)-2-oxa-5-azabicyclo[2.2.1]heptan-5-yl)-N-(1-((1R,4R)-4-formylcyclohexyl)-3-methyl-1H-pyrazol-4-yl)pyrazolo[1,5-a]pyrimidine-3-carboxamide

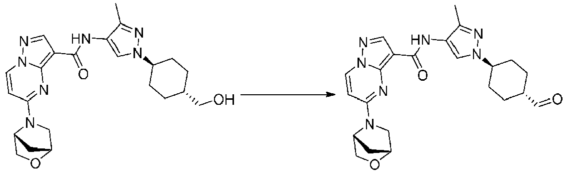

A mixture of 5-((1*R*,4*R*)-2-oxa-5-azabicyclo[2.2.1]heptan-5-yl)-*N*-(1-((1*R*,4*R*)-4-(hydroxymethyl)cyclohexyl)-3-methyl-1*H*-pyrazol-4-yl)pyrazolo[1,5-*a*]pyrimidine-3-carboxamide (320.0 mg, 0.7 mmol) and IBX (392 mg, 1.4 mmol) in ACN (10 mL) was stirred at 60 °C for 1 h. The reaction liquid was filtered through celite, and the filtrate was concentrated under reduced pressure. The resulting crude product was purified by column chromatography (MeOH:DCM = 0-1:9) to give 5-((1*R*,4*R*)-2-oxa-5-azabicyclo[2.2.1]heptan-5-yl)-*N*-(1-((1R,4R)-4-formylcyclohexyl)-3-methyl-1 *H*-pyrazol-4-yl)pyrazolo[1,5-a]pyrimidine-3-carboxamide.

LC-MS: (ESI, *m*/*z):* [M+H]⁺= 450.1.

### Step 6: preparation of tert-butyl 9-(((1R,4R)-4-(4-(5-((1R,4R)-2-oxa-5-azabicyclo[2.2.1]heptan-5-yl)pyrazolo[1,5-a]pyrimidine-3-carboxamido)-3-methyl-1H-pyrazol-1-yl)cyclohexyl)methyl)-3,9-diazaspiro[5.5]undecane-3-carboxylate

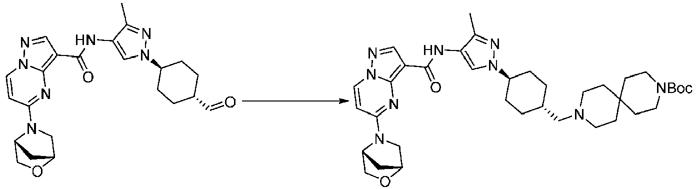

STAB (381.6 mg, 1.8 mmol) was added to a solution of 5-((1*R*,4*R*)-2-oxa-5-azabicyclo[2.2.1]heptan-5-yl)-*N*-(1-((1*R*,4*R*)-4-formylcyclohexyl)-3-methyl-1*H*-pyrazol-4-yl)pyrazolo[1,5-a]pyrimidine-3-carboxamide (250.0 mg, 0.6 mmol) and tert-butyl 3,9-diazaspiro[5.5]undecane-3-carboxylate (203.0 mg, 0.8 mmol) in THF (10 mL). The reaction liquid was then stirred at 25 °C for 1.0 h. The reaction liquid was filtered through celite, and the filtrate was concentrated under reduced pressure. The resulting crude product was purified by column chromatography (MeOH:DCM = 0-1:9) to give tert-butyl 9-(((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamido)-3-methyl-1*H-*pyrazol-1-yl)cyclohexyl)methyl)-3,9-diazaspiro[5.5] undecane-3-carboxylate.

LC-MS: (ESI, *m*/*z*): [M+H]⁺ = 688.3.

### Step 7: preparation of N (1-((1R,4R)-4-((3,9-diazaspiro[5.5]undecan-3-yl)methyl)cyclohexyl)-3-methyl-1H-pyrazol-4-yl)-5-((1R,4R)-2-oxa-5-azabicyclo[2.2.1]heptan-5-yl)pyrazolo[1,5-a]pyrimidine-3-carboxamide

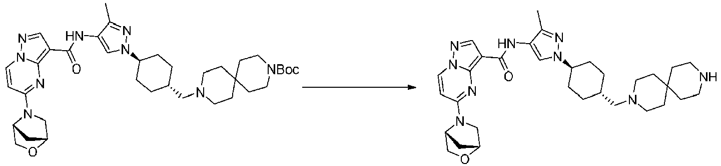

A solution of tert-butyl 9-(((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamido)-3-methyl-1*H*-pyrazol-1-yl)cyclohexyl)methyl)-3,9-diazaspiro[5.5]undecane-3-carboxylate (300.0 mg, 0.4 mmol) in TFA/DCM (1.0 mL/3.0 mL) was stirred at room temperature for 1.0 h. The reaction liquid was directly concentrated under vacuum under reduced pressure to give *N-*(1-((1*R*,4*R*)-4-((3,9-diazaspiro[5.5]undecan-3-yl)methyl)cyclohexyl)-3-methyl-1*H-*pyrazol-4-yl)-5-((1*R*,4*R*)-2-oxa-5-azabicyclo[2.2.1]heptan-5-yl)pyrazolo[1,5-*a*]pyrimidine-3-carboxamide. The resulting crude product was directly used in the next step.

LC-MS: (ESI, *m*/*z*): [M+H]⁺= 588.6.

### Step 8: preparation of 5-((1R,4R)-2-oxa-5-azabicyclo[2.2.1]heptan-5-yl)-N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-methyl-1H-pyrazol-4-yl)pyrazolo[1,5-a]pyrimidine-3-carboxamide

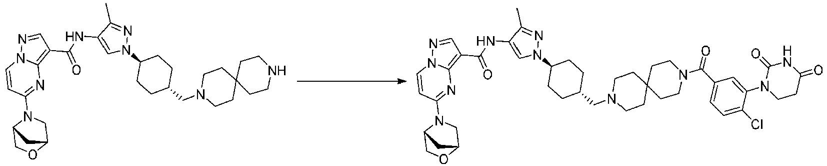

DIEA (54.20 mg, 0.42 mmol) and pentafluorophenyl 4-chloro-3-(2,4-dioxotetrahydropyrimidin-1(2H)-yl)benzoate (143.2 mg, 0.33 mmol) were added to a solution of N-(1-((1R,4R)-4-((3,9-diazaspiro[5.5]undecan-3-yl)methyl)cyclohexyl)-3-methyl-1*H-*pyrazol-4-yl)-5-((1*R*,4*R*)-2-oxa-5-azabicyclo[2.2.1]heptan-5-yl)pyrazolo[1,5-*a*]pyrimidine-3-carboxamide (200.0 mg, 0.3 mmol) in DMSO (5.0 mL), and the reaction mixture was stirred at room temperature for 2 h. Water (15.0 mL) was added to the reaction liquid, and then the mixture was extracted with DCM (30.0 mL × 2). The organic phases were combined, washed with brine, dried over anhydrous Na₂SO₄, filtered, and concentrated under reduced pressure. The resulting crude product was purified by Prep-HPLC (acetonitrile/0.05% aqueous NH₄HCO₃ solution, 5%-95%) to give 5-((1R,4R)-2-oxa-5-azabicyclo[2.2.1]heptan-5-yl)-N-(1-((1R,4R)-4-((9-(4-chloro-3-(2,4-dioxotetrahydropyrimidin-1(2*H*)-yl)benzoyl)-3,9-diazaspiro[5.5]undecan-3-yl)methyl)cyclohexyl)-3-methyl-1*H*-pyrazol-4-yl)pyrazolo[1,5-a]pyrimidine-3-carboxamide.

LC-MS: (ESI, m/z): [M+H]⁺ = 838.4.

¹H NMR (400 MHz, DMSO-*d*₆) δ 10.51 (s, 1H), 9.21 (d, J= 7.6 Hz, 1H), 8.80 (d, *J* = 7.8 Hz, 1H), 8.22 (s, 1H), 8.04 (s, 1H), 7.64 (d, *J=* 8.2 Hz, 1H), 7.55 (d, *J=* 1.9 Hz, 1H), 7.39 (dd, *J=* 8.2, 1.9 Hz, 1H), 6.92-6.43 (m, 1H), 5.25-5.05 (m, 1H), 4.78 (s, 1H), 4.05-3.88 (m, 1H), 3.88-3.57 (m, 7H), 3.45 (d, *J=* 10.1 Hz, 1H), 3.30-3.26 (m, 2H), 2.78-2.68 (m, 2H), 2.41-1.93 (m, 12H), 1.86 (d, *J* = 11.5 Hz, 2H), 1.77-1.62 (m, 2H), 1.60-1.31 (m, 10H), 1.11-0.95 (m, 2H).

### Example 8: N-(2-((1S,4S)-4-(((1-(4-chloro-3-(2,4-dioxotetrahydropyrimidin-1(2H)-yl)benzoyl)piperidin-4-yl)methoxy)methyl)-4-hydroxycyclohexyl)-6-methoxy-2H-indol-5-yl)-6-(trifluoromethyl)pyridinecarboxamide

### Step 1: preparation of tert-butyl (1-oxaspiro[2.5]octan-6-yl)carbamate

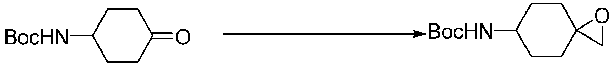

NaH (1.17 g, 29.3 mmol) was added to a solution of Me₃OS^{|}I⁻ (6.2 g, 28.2 mmol) in DMSO (50 mL) at 0 °C under stirring, and the reaction mixture was stirred at room temperature for 1 h. Then a solution of tert-butyl (4-oxocyclohexyl)carbamate (5.0 g, 23.5 mmol) in DMSO (20 mL) was added dropwise. After the addition, the reaction liquid was stirred at room temperature for another 1 h. Water (700 mL) was added to the reaction liquid, and the mixture was extracted with EtOAc (100 mL × 3). The organic phase was washed with saturated brine (300 mL), dried over anhydrous Na₂SO₄, filtered, and concentrated under reduced pressure. The crude product was purified by column chromatography (EA:PE = 0-1:1) to give tert-butyl (1-oxaspiro [2.5] octan-6-yl)carbamate.

¹H NMR (400 MHz, DMSO-*d*₆) δ 6.79 (d, *J=* 7.2 Hz, 1H), 3.37 (m, 1H), 2.57 (s, 2H), 1.83 (td, *J=* 13.3, 4.1 Hz, 2H), 1.77-1.64 (m, 2H), 1.48-1.40 (m, 2H), 1.38 (s, 9H), 1.20 (d, *J=* 13.1 Hz, 2H).

### Step 2: preparation of tert-butyl (4-hydroxy-4-(hydroxymethyl)cyclohexyl)carbamate

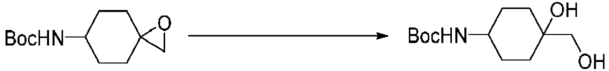

2 N NaOH (47 mL) was added to a solution of tert-butyl (1-oxaspiro[2.5]octan-6-yl)carbamate (4.7 g, 20.7 mmol) in NMP (50 mL) while stirring, and then the reaction liquid was stirred at 100 °C for 2 h. Water (500 mL) was added to the reaction liquid, and the mixture was extracted with EA (100 mL × 3). The organic phase was washed with saturated brine (300 mL), dried over anhydrous Na₂SO₄, filtered, and concentrated under reduced pressure. The crude product was purified by flash column chromatography (MeOH:DCM = 0-1:9) to give *tert*-butyl (4-hydroxy-4-(hydroxymethyl)cyclohexyl)carbamate.

¹H NMR (400 MHz, DMSO-*d*₆) δ 6.67 (d, *J=* 7.9 Hz, 1H), 4.46 (t, *J=* 5.8 Hz, 1H), 3.81 (s, 1H), 3.11 (t, *J* = 6.8 Hz, 3H), 1.56-1.45 (m, 4H), 1.44-1.34 (m, 13H).

### Step 3: preparation of tert-butyl (4-hydroxy-4-((pyridin-4-ylmethoxy)methyl)cyclohexyl)carbamate

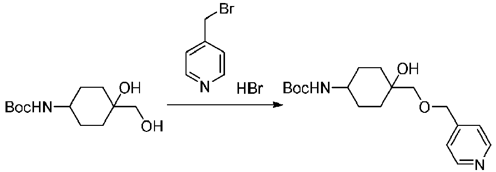

NaH (292 mg, 7.3 mmol) was added to a solution of *tert-butyl* (4-hydroxy-4-(hydroxymethyl)cyclohexyl)carbamate (600 mg, 2.4 mmol) in THF (20 mL) at 0 °C, the reaction mixture was stirred at 0 °C for 20 min and then stirred at room temperature for another 30 min, and 4-(bromomethyl)pyridine hydrogen bromide salt (617 mg, 2.4 mmol) was added. The reaction liquid was stirred at room temperature overnight. Water (30 mL) was added to the reaction liquid, and the mixture was extracted with EA (20 mL × 3). The organic phase was washed with saturated brine (60 mL), dried over anhydrous Na₂SO₄, filtered, and concentrated under reduced pressure. The resulting crude product was purified by flash column chromatography (EA:PE = 0-4:1) to give *tert-butyl* (4-hydroxy-4-((pyridin-4-ylmethoxy)methyl)cyclohexyl)carbamate.

LC-MS: (ESI, *m*/*z):* [M+H]^{|} = 337.1.

¹H NMR (400 MHz, DMSO-*d*₆) δ 8.53 (d, *J=* 5.8 Hz, 2H), 7.34 (d, *J=* 5.6 Hz, 2H), 6.69 (d, *J=* 7.6 Hz, 1H), 4.55 (s, 2H), 4.19 (s, 1H), 3.23 (s, 2H), 3.18-3.07 (m, 1H), 1.60-1.48 (m, 6H), 1.42-1.35 (m, *J=* 9.2 Hz, 11H).

### Step 4: preparation of tert-butyl (4-hydroxy-4-((piperidin-4-ylmethoxy)methyl)cyclohexyl)carbamate

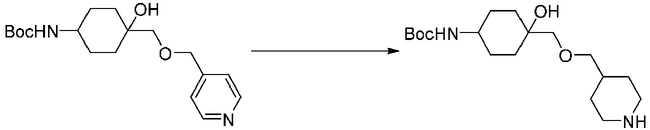

A mixture of tert-butyl (4-hydroxy-4-((pyridin-4-ylmethoxy)methyl)cyclohexyl)carbamate (700 mg, 2.07 mmol) and Pd/C (400 mg) in *i*-PrOH/H₂O (18 mL/21 mL) was stirred at 75 °C overnight under H₂ atmosphere. The reaction liquid was filtered through celite, and the filtrate was concentrated under reduced pressure to give tert-butyl (4-hydroxy-4-((piperidin-4-ylmethoxy)methyl)cyclohexyl)carbamate. The resulting crude product was directly used in the next step.

LC-MS: (ESI, *m*/*z*): [M+H]⁺= 343.2.

### Step 5: preparation of benzyl 4-(((4-((tert-butoxycarbonyl)amino)-1-hydroxycyclohexyl)methoxy)methyl)piperidine-1-carboxylate

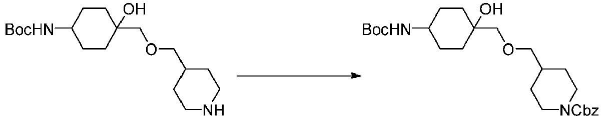

A solution of tert-butyl (4-hydroxy-4-((piperidin-4-ylmethoxy)methyl)cyclohexyl)carbamate (630 mg, 1.83 mmol) and CbzCl (376 mg, 2.20 mmol) inACN (10 mL) and a saturated aqueous NaHCO₃ solution (10 mL) were stirred at room temperature overnight. The reaction liquid was concentrated under reduced pressure and extracted with DCM (50 mL × 3). The organic phase was washed with saturated brine (150 mL), dried over anhydrous Na₂SO₄, filtered, and concentrated under reduced pressure. The crude product was purified by flash column chromatography (EA:PE = 0-7:3) to give benzyl *4-(((4-((tert-*butoacycarbonyl)amino)-1-hydroxycyclohexyl)methoxy)methyl)piperidine-1-carboxylate.

LC-MS: (ESI, *m*/*z*): [M+Na] '= 499.3.

### Step 6: preparation of benzyl 4-(((4-amino-1-hydroxycyclohexyl)methoxy)methyl)piperidine-1-carboxylate

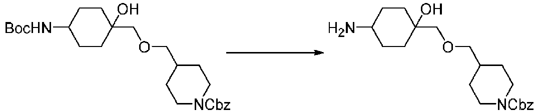

TFA (3 mL) was added to a solution of benzyl 4-(((4-((tert-butoxycarbonyl)amino)-1-hydroxycyclohexyl)methoxy)methyl)piperidine-1-carboxylate (300 mg, 0.63 mmol) in DCM (12 mL) at -10 to 0 °C while stirring, and then the reaction liquid was stirred at room temperature for 2 h. The pH of the reaction mixture was adjusted to 9.0 with DIEA, and then the mixture was directly concentrated under reduced pressure to give benzyl 4-(((4-amino-1-hydroxycyclohexyl)methoxy)methyl)piperidine-1-carboxylate. The resulting crude product was directly used in the next step without purification.

LC-MS: (ESI, m/z): [M+H]⁺= 377.2.

### Step 7: preparation of benzyl 4-((((1S,4S)-1-hydroxy-4-(6-methoxy-5-nitro-2H-indol-2-yl)cyclohexyl)methoxy)methyl)piperidine-1 -carboxylate

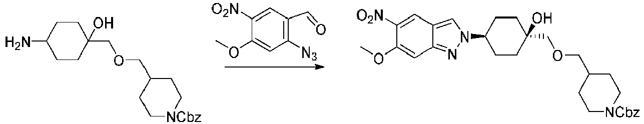

2-Azido-4-methoxy-5-nitrobenzaldehyde (170 mg, 0.76 mmol) was added to a solution of benzyl 4-(((4-amino-1-hydroxycyclohexyl)methoxy)methyl)piperidine-1-carboxylate (237 mg, 0.63 mmol) in toluene (10 mL) while stirring. The reaction liquid was stirred at 100 °C for 3 h. The reaction liquid was directly concentrated under reduced pressure. The crude product was purified by flash column chromatography (EA:PE = 0-4:1) and then purified by Prep-HPLC (CH₃CN/0.08% aqueous NH₄HCO₃ solution, 5%-95%) to give benzyl 4-((((1*S*,4*S*)-1-hydroxy-4-(6-methoxy-5-nitro-2*H*-indol-2-yl)cyclohexyl)methoxy)methyl)piperidine-1-carboxylate.

LC-MS: (ESI, m/z): [M+H]^{|} = 553.2.

¹H NMR (400 MHz, DMSO-*d*₆) δ 8.58 (s, 1H), 8.37 (s, 1H), 7.40-7.29 (m, 5H), 7.26 (s, 1H), 5.07 (s, 2H), 4.45 (t, *J=* 12.0 Hz, 1H), 4.36 (s, 1H), 4.06-3.99 (m, 2H), 3.90 (s, 3H), 3.29 (d, *J=* 6.2 Hz, 2H), 3.20 (s, 2H), 2.85-2.75 (m, 2H), 2.35-2.20 (m, 2H), 1.89 (d, *J =* 11.8 Hz, 2H), 1.76-1.61 (m, 7H), 1.15-1.02 (m, 2H).

### Step 8: preparation of tert-butyl 4-((((1S,4S)-4-(5-amino-6-methoxy-2H-indol-2-yl)-1-hydroxycyclohexyl)methoxy)methyl)piperidine-1-carboxylate

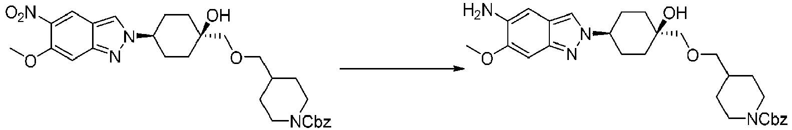

Raney-Ni (0.3 mL) and N₂H₄ H₂O (0.3 mL) were added to a solution of benzyl 4-((((1*S*,4*S*)-1-hydroxy-4-(6-methoxy-5-nitro-2*H*-indol-2-yl)cyclohexyl)methoxy)methyl)piperidine-1-carboxylate (200 mg, 0.36 mmol) in EtOH (20 mL). The reaction liquid was stirred at room temperature for 2 h. The reaction liquid was filtered through celite, and the filtrate was directly concentrated under reduced pressure to give *tert-butyl* 4-((((1*S*,4*S*)-4-(5-amino-6-methoxy-2*H*-indol-2-yl)-1-hydroxycyclohexyl)methoxy)methyl)piperidine-1-carboxylate. The resulting crude product was directly used in the next step.

LC-MS: (ESI, m/z): [M+H]⁺ = 523.2.

### Step 9: preparation of benzyl 4-((((1S,4S)-1-hydroxy-4-(6-methoxy-5-(6-(trifluoromethyl)pyridinecarboxamido)-2H-indol-2-yl)cyclohexyl)methoxy)methyl)piperidine-1-carboxylate

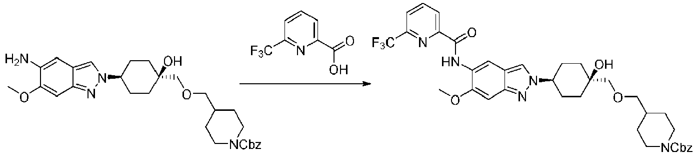

6-(Trifluoromethyl)picolinic acid (90 mg, 0.47 mmol), HATU (178 mg, 0.47 mmol), and DIEA (140 mg, 1.08 mmol) were added to a solution of tert-butyl 4-((((1S,4S)-4-(5-amino-6-methoxy-2H-indol-2-yl)-1-hydroxycyclohexyl)methoxy)methyl)piperidine-1-carboxylate (190 mg, 0.36 mmol) in DMF (5 mL) while stirring. Then the reaction liquid was stirred at room temperature for 2 h. Water (30 mL) was added to the reaction liquid, and the mixture was extracted with EA (20 mL × 3). The organic phase was washed with saturated brine (60 mL), dried over anhydrous Na₂SO₄, filtered, and concentrated under reduced pressure. The resulting crude product was purified by flash column chromatography (EA:PE = 0-4:1) to give benzyl 4-((((1*S*,4*S*)-1-hydroxy-4-(6-methoxy-5-(6-(trifluoromethyl)pyridinecarboxamido)-2*H*-indol-2-yl)cyclohexyl)methoxy)methyl)piperidine-1 -carboxylate.

LC-MS: (ESI, m/z): [M+H]⁺= 696.3.

¹H NMR (400 MHz, DMSO-d6) δ 10.51 (s, 1H), 8.69 (s, 1H), 8.47 (d, *J=* 7.7 Hz, 1H), 8.41 (t, *J=* 7.8 Hz, 1H), 8.32 (s, 1H), 8.22 (d, *J=* 7.7 Hz, 1H), 7.41-7.29 (m, 5H), 7.16 (s, 1H), 5.08 (s, 2H), 4.40-4.29 (m, 2H), 4.15-3.95 (m 5H), 3.29 (d, *J=* 6.2 Hz, 2H), 3.21 (s, 2H), 2.91-2.70 (m, 2H), 2.35-2.15 (m, 2H), 1.90 (d, *J=* 9.5 Hz, 2H), 1.85-1.60 (m, 7H), 1.12-.1.02 (m, 2H).

### Step 10: preparation of N-(2-((1S,4S)-4-hydroxy-4-((piperidin-4-ylmethoxy)methyl)cyclohexyl)-6-methoxy-2H-indol-5-yl)-6-(trifluoromethyl)pyridinecarboxamide

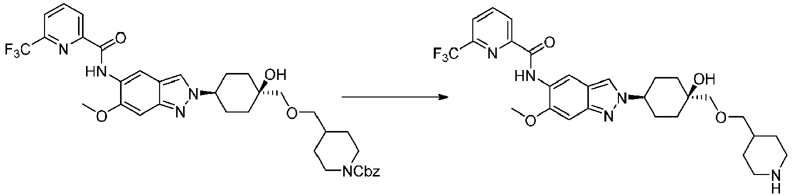

Pd(OH)₂/C (80 mg) was added to a solution of benzyl 4-((((1*S*,4*S*)-1-hydroxy-4-(6-methoxy-5-(6-(trifluoromethyl)pyridinecarboxamido)-2*H*-indol-2-yl)cyclohexyl)methoxy)methyl)piperidine-1-carboxylate (170 mg, 0.24 mmol) in EA (20 mL). Then the reaction liquid was stirred at 70 °C overnight under H₂ atmosphere. The reaction liquid was filtered through celite, and the filtrate was directly concentrated under reduced pressure to give *N*-(2-((1*S*,4*S*)-4-hydroxy-4-((piperidin-4-ylmethoxy)methyl)cyclohexyl)-6-methoxy-277-indol-5-yl)-6-(trifluoromethyl)pyridinecarboxamide.

LC-MS: (ESI, m/z): [M+H]⁺= 562.3.

### Step 11: preparation of N-(2-((1S,4S)-4-(((1-(4-chloro-3-(2,4-dioxotetrahydropyrimidin-1(2H)-yl)benzoyl)piperidin-4-yl)methoxy)methyl)-4-hydroxycyclohexyl)-6-methoxy-2H-indol-5-yl)-6-(trifluoromethyl)pyridinecarboxamide

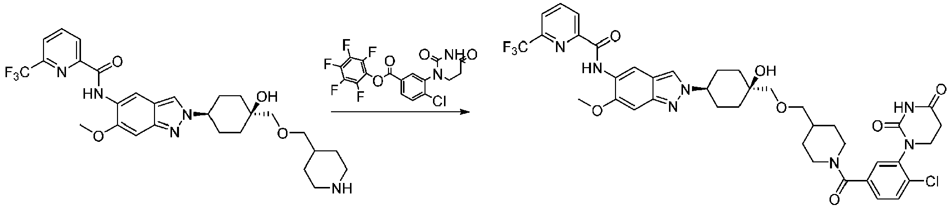

A mixture of *N*-(2-((1*S*,4*S*)-4-hydroxy-4-((piperidin-4-ylmethoxy)methyl)cyclohexyl)-6-methoxy-2*H*-indol-5-yl)-6-(trifluoromethyl)pyridinecarboxamide (130 mg, 0.23 mmol), pentafluorophenyl 4-chloro-3-(2,4-dioxotetrahydropyrimidin-1(2H)-yl)benzoate (121 mg, 0.28 mmol), and DIEA (89 mg, 0.69 mmol) in DMSO (6 mL) was stirred at room temperature overnight. The reaction liquid was diluted with water (50 mL) and extracted with DCM (20 mL × 3). The organic phase was washed with saturated brine (60 mL), dried over anhydrous Na₂SO₄, filtered, and concentrated under reduced pressure. The resulting crude product was purified by Prep-HPLC (CH₃CN/0.08% aqueous NH₄HCO₃ solution, 5%-95%) to give *N*-(2-((1*S*,4*S*)-4-(((1-(4-chloro-3-(2,4-dioxotetrahydropyrimidin-1(2H)-yl)benzoyl)piperidin-4-yl)methoxy)methyl)-4-hydroxycyclohexyl)-6-methoxy-277-indol-5-yl)-6-(trifluoromethyl)pyridinecarboxamide.

LC-MS: (ESI, *m*/*z*): [M+H]⁺= 812.1.

¹H NMR (400 MHz, DMSO-*d*₆) δ 10.51 (d, *J* = 7.5 Hz, 2H), 8.69 (s, 1H), 8.46 (d, *J* = 7.7 Hz, 1H), 8.40 (t, *J=* 7.8 Hz, 1H), 8.32 (s, 1H), 8.21 (d, *J=* 7.6 Hz, 1H), 7.65 (d, *J=* 8.2 Hz, 1H), 7.57 (d, *J=* 1.4 Hz, 1H), 7.40 (dd, *J=* 8.2, 1.5 Hz, 1H), 7.16 (s, 1H), 4.58-4.29 (m, 3H), 3.99 (s, 3H), 3.84-3.55 (m, 3H), 3.33-3.28 (m, 2H), 3.22 (s, 2H), 3.13-3.03 (m, 1H), 2.88-2.70 (m, 3H), 2.35-2.20 (m, 2H), 1.94-1.59 (m, 9H), 1.21-1.15 (m, 2H).

### Example 9: 5-((1R,4R)-2-oxa-5-azabicyclo[2.2.1]heptan-5-yl)-N-(1-((1R,4R)-4-((2-(1-(4-chloro-3-(2,4-dioxotetrahydropyrimidin-1(2H)-yl)benzoyl)piperidin-4-yl)ethoxy)methyl)cyclohexyl)-3-(difluoromethyl)-1H-pyrazol-4-yl)pyrazolo[1,5-a]pyrimidine-3-carboxamide

### Step 1: preparation of tert-butyl 4-(2-(((1R,4R)-4-(3-(diffuoromethyl)-4-nitro-1H-pyrazol-1-yl)cyclohexyl)methoxy)ethyl)piperidine-1-carboxylate

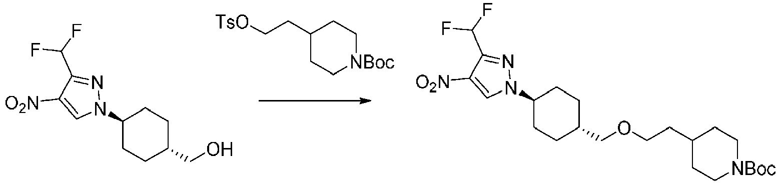

NaH (60%, 580 mg, 14.5 mmol) was added to a solution of ((1*R*,4*R*)-4-(3-(difluoromethyl)-4-nitro-1*H*-pyrazol-1-yl)cyclohexyl)methanol (1.0 g, 3.64 mmol) in anhydrous THF (30 mL) at 0 °C, and then the mixture was stirred at room temperature for 1 h. *tert-Butyl* 4-(2-(p-toluenesulfonyl)ethyl)piperidine-1-carboxylate (2.79 g, 7.28 mmol) and KI (1.21 g, 7.28 mmol) were added, and the reaction liquid was stirred at 70 °C overnight. Water (50 mL) was slowly added to the reaction liquid, and the mixture was extracted with EtOAc (100 mL × 3). The combined organic phases were washed with saturated brine (50 mL) and concentrated under reduced pressure. The resulting crude product was purified by flash column chromatography (EA:PE = 0-7:13) to give tert-butyl 4-(2-(((1*R*,4*R*)-4-(3-(difluoromethyl)-4-nitro-1*H-*pyrazol-1-yl)cyclohexyl)methoxy)ethyl)piperidine-1-carboxylate.

LC-MS: (ESI, *m*/*z):* [M+Na]⁺ = 509.2.

### Step 2: preparation of tert-butyl 4-(2-(((1R,4R)-4-(4-amino-3-(difluoromethyl)-1H-pyrazol-1-yl)cyclohexyl)methoxy)ethyl)piperidine-1-carboxylate

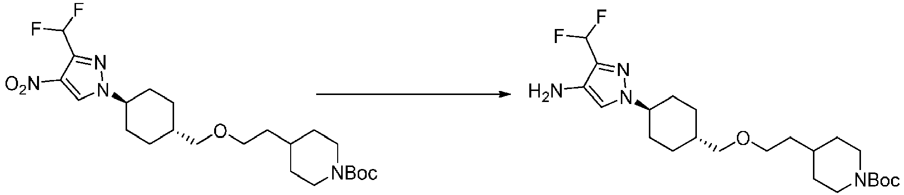

Raney-Ni (0.4 mL) and hydrazine hydrate (85%, 0.4 mL) were added to a solution of tert-butyl 4-(2-(((1*R*,4*R*)-4-(3-(difluoromethyl)-4-nitro-1*H*-pyrazol-1-yl)cyclohexyl)methoxy)ethyl)piperidine-1-carboxylate (185 mg, 0.38 mmol) in ethanol (10 mL), and then the reaction liquid was stirred at room temperature for 1 h. The mixture was filtered, and the filtrate was directly concentrated under reduced pressure to give *tert-butyl* 4-(2-(((1*R*,4*R*)-4-(4-amino-3-(difluoromethyl)-1*H-*pyrazol-1-yl)cyclohexyl)methoxy)ethyl)piperidine-1 -carboxylate.

LC-MS: (ESI, m/z): [M+H]⁺= 457.2.

### Step 3: preparation of tert-butyl 4-(2-(((1R,4R)-4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)cyclohexyl)methoxy)ethyl)piperidine-1-carboxylate

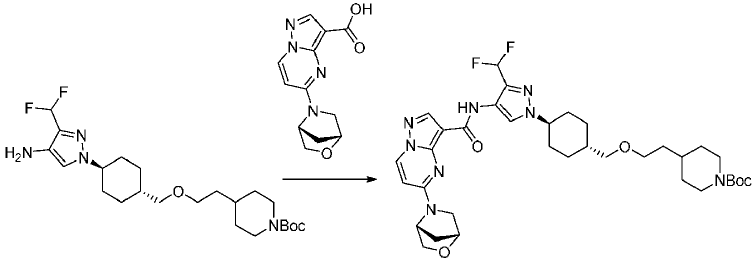

5-((1*R*,*4*R)-2-Oxa-5-azabicyclo[2.2.1]heptan-5-yl)pyrazolo[1,5-*a*]pyrimidine-3-carboxylic acid (86 mg, 0.33 mmol), HATU (188 mg, 0.50 mmol), and DIEA (128 mg, 0.99 mmol) were added to a solution of *tert-butyl* 4-(2-(((1*R*,4*R*)-4-(4-amino-3-(difluoromethyl)-1*H*-pyrazol-1-yl)cyclohexyl)methoxy)ethyl)piperidine-1-carboxylate (150 mg, 0.33 mmol) in DMF (5 mL). The reaction liquid was stirred at room temperature for 2 h. The reaction liquid was diluted with water (50 mL) and then extracted with EA (50 mL × 3). The organic phase was washed with saturated brine (150 mL), dried over anhydrous Na₂SO₄, filtered, and concentrated under reduced pressure. The resulting crude product was purified by reversed-phase chromatography (CH₃CN/H₂O (0.5% FA), 5%-95%) to give tert-butyl 4-(2-(((1R,4R)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1 -yl)cyclohexyl)methoxy)ethyl)piperidine-1 -carboxylate.

LC-MS: (ESI, *m*/*z):* [M+Na]⁺ = 721.2.

### Step 4: preparation of 5-((1R,4R)-2-oxa-5-azabicyclo[2.2.1]heptan-5-yl)-N-(3-(difluoromethyl)-1-((1R,4R)-4-((2-(piperidin-4-yl)ethoxy)methyl)cyclohexyl)-1H-pyrazol-4-yl)pyrazolo[1,5-a]pyrimidine-3-carboxamide

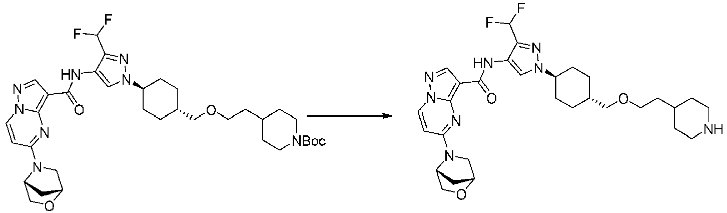

A reaction liquid of tert-butyl 4-(2-(((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)cyclohexyl)methoxy)ethyl)piperidine-1-carboxylate (80 mg, 0.11 mmol) in TFA/DCM (3 mL, 1/5) was stirred at room temperature for 1 h. The reaction liquid was directly concentrated under reduced pressure to give 5-((1*R*,4*R*)-2-oxa-5-azabicyclo[2.2.1]heptan-5-yl)-*N-*(3-(difluoromethyl)-1-((1*R*,4*R*)-4-((2-(piperidin-4-yl)ethoxy)methyl)cyclohexyl)-1*H*-pyrazol-4-yl)pyrazolo[1,5-*a*]pyrimidine-3-carboxamide. The resulting product was directly used in the next step.

LC-MS: (ESI, *m*/*z*): [M+H]⁺= 599.3

### Step 5: preparation of 5-((1R,4R)-2-oxa-5-azabicyclo[2.2.1]heptan-5-yl)-N-(1-((1R,4R)-4-((2-(1-(4-chloro-3-(2,4-dioxotetrahydropyrimidin-1(2H)-yl)benzoyl)piperidin-4-yl)ethoxy)methyl)cyclohexyl)-3-(difluoromethyl)-1H-pyrazol-4-yl)pyrazolo[1,5-a]pyrimidine-3-carboxamide

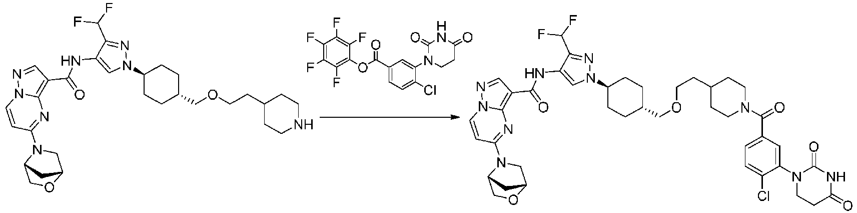

A solution of 5-((1*R*,4*R*)-2-oxa-5-azabicyclo[2.2.1]heptan-5-yl)-*N*-(3-(difluoromethyl)-1-((1*R*,4*R*)-4-((2-(piperidin-4-yl)ethoxy)methyl)cycloheaηl)-1*H*-pyrazol-4-yl)pyrazolo[1,5*-a*]pyrimidine-3-carboxamide (80 mg, 0.13 mmol), pentafluorophenyl 3-(2,4-dioxotetrahydropyrimidin-1(2*H*)-yl)-4-methoxybenzoate (58 mg, 0.13 mmol), and DIEA (86 mg, 0.66 mmol) in DMSO (5 mL) was stirred at room temperature overnight. The reaction liquid was poured into water (50 mL) and stirred for 10 min. The mixture was filtered, and the filter cake was purified by flash column chromatography (MeOH:DCM = 0-1:9) to give 5-((1R,4R)-2-oxa-5-azabicyclo[2.2.1]heptan-5-yl)-N-(1-(1*R*,4*R*)-4-((2-(1-(4-chloro-3-(2,4-dioxotetrahydropyrimidin-1(2*H*)-yl)benzoyl)piperidin-4-yl)ethoxy)methyl)cyclohexyl)-3-(difluoromethyl)-1*H*-pyrazol-4-yl)pyrazolo[1,5-*a*]pyrimidine-3-carboxamide.

LC-MS: (ESI, *m*/*z):* [M+H]⁺= 849.1.

¹H NMR (400 MHz, DMSO-*d*₆) δ 10.51 (s, 1H), 9.50 (d, *J* = 5.7 Hz, 1H), 8.78 (d, *J* = 7.7 Hz, 1H), 8.39 (d, *J=* 4.3 Hz, 1H), 8.25 (d, *J=* 5.6 Hz, 1H), 7.64 (d, *J=* 8.2 Hz, 1H), 7.55 (d, *J=* 1.9 Hz, 1H), 7.38 (dd, *J* = 8.2, 1.9 Hz, 1H), 7.29-6.93 (m, 1H), 6.90-6.40 (m, 1H), 5.30-5.03 (m, 1H), 4.77 (d, *J=* 17.2 Hz, 1H), 4.50-4.35 (m, 1H), 4.24-4.11 (m, 1H), 3.86-3.70 (m, 3H), 3.66-3.52 (m, 3H), 3.50-3.40 (m, 3H), 3.22 (d, *J=* 6.4 Hz, 2H), 3.12-2.95 (m, 1H), 2.83-2.70 (m, 3H), 2.08-1.91 (m, 4H), 1.88-1.54 (m, 8H), 1.55-1.44 (m, 2H), 1.19-1.04 (m, 4H).

### Example 10: N-(2-((1S,4S)-4-((2-(1-(4-chloro-3-(2,4-dioxotetrahydropyrimidin-1(2H)-yl)benzoyl)piperidin-4-yl)ethoxy)methyl)-4-hydroxycyclohexyl)-6-methoxy-2H-indol-5-yl)-6-(trifluoromethyl)pyridinecarboxamide

### Step 1: preparation of benzyl 4-(2-((4-((tert-butoxycarbonyl)amino)-1-hydroxycyclohexyl)methoxy)ethyl)piperidine-1-carboxylate

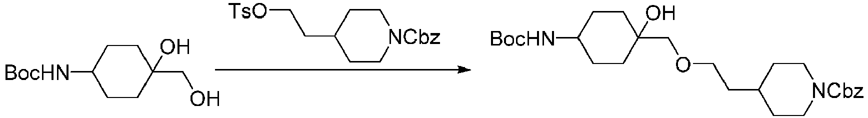

NaH (376 mg, 9.4 mmol) was added to a solution of tert-butyl (4-hydroxy-4-(hydroxymethyl)cyclohexyl)carbamate (770 mg, 3.13 mmol) in THF (20 mL) at 0 °C, and the mixture was stirred at 0 °C for 2 h. Then benzyl 4-(2-(p-toluenesulfonyl)ethyl)piperidine-1 -carboxylate (2.62 g, 6.26 mmol) and KI (1.04 g, 6.26 mmol) were added. The reaction liquid was stirred at 70 °C overnight. The reaction liquid was diluted with water (50 mL) and extracted with EA (30 mL × 3). The organic phase was washed with saturated brine (100 mL), dried over anhydrous Na₂SO₄, filtered, and concentrated under reduced pressure. The resulting crude product was purified by flash column chromatography (EA:PE = 0-3:2) to give benzyl 4-(2-((4-((*tert*-butoxycarbonyl)amino)-1-hydroxycyclohexyl)methoxy)ethyl)piperidine-1-carboxylate.

LC-MS: (ESI, *m*/*z*): [M+Na]⁺ = 513.1.

¹H NMR (400 MHz, DMSO-*d*₆) δ 7.41-7.29 (m, 5H), 6.67 (d, *J=* 7.5 Hz, 1H), 5.06 (s, 2H), 4.05-3.90 (m, 3H), 3.42 (t, *J* = 6.4 Hz, 2H), 3.10 (s, 3H), 2.88-2.68 (m, 2H), 1.65 (d, *J* = 12.6 Hz, 2H), 1.55-1.32 (m, 20H), 1.13-0.92 (m, 2H).

### Step 2: preparation of benzyl 4-(2-((4-amino-1-hydroxycyclohexyl)methoxy)ethyl)piperidine-1-carboxylate

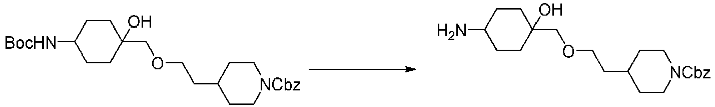

TFA (1.2 mL) was added to a solution of benzyl 4-(2-((4-((tert-butoxycarbonyl)amino)-1-hydroxycyclohexyl)methoxy)ethyl)piperidine-1-carboxylate (270 mg, 0.55 mmol) in DCM (3 mL) at -10 to 0 °C while stirring, and then the reaction liquid was stirred at room temperature for 2 h. DIEA was added to the reaction liquid to adjust the pH to 9.0, and the mixture was concentrated under reduced pressure to give benzyl 4-(2-((4-amino-1-hydroxycyclohexyl)methoxy)ethyl)piperidine-1-carboxylate.

LC-MS: (ESI, *m*/*z*): [M+H]⁺= 391.2.

### Step 3: preparation of benzyl 4-(2-(((1S,4S)-1-hydroxy-4-(6-methoxy-5-nitro-2H-indol-2-yl)cyclohexyl)methoxy)ethyl)piperidine-1-carboxylate

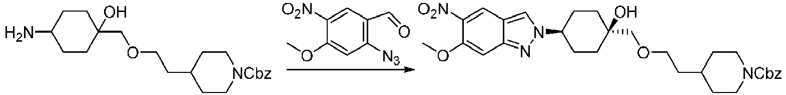

2-Azido-4-methoxy-5-nitrobenzaldehyde (150 mg, 0.66 mmol) was added to a solution of benzyl 4-(2-((4-amino-1-hydroxycyclohexyl)methoxy)ethyl)piperidine-1-carboxylate (400 mg, 0.55 mmol) in toluene (15 mL) while stirring. The reaction liquid was stirred at 100 °C for 3 h. The reaction liquid was concentrated under reduced pressure, and the resulting crude product was purified by flash column chromatography (EA:PE = 0-9:1) to give benzyl 4-(2-(((1S,4S)-1-hydroxy-4-(6-methoxy-5-nitro-2H-indol-2-yl)cyclohexyl)methoxy)ethyl)piperidine-1-carboxylate.

LC-MS: (ESI, *m*/*z*): [M+H]⁺= 567.3.

¹H NMR (400 MHz, DMSO-*d*₆) δ 8.59 (s, 1H), 8.38 (s, 1H), 7.41-7.29 (m, 5H), 7.27 (s, 1H), 5.07 (s, 2H), 4.50-4.40 (m, 1H), 4.34 (s, 1H), 4.05-3.95 (m, 2H), 3.91 (s, 3H), 3.47 (t, *J=* 6.4 Hz, 2H), 3.21 (s, 2H), 2.90-2.70 (m, 2H), 2.35-2.19 (m, 2H), 1.90 (d, *J=* 9.7 Hz, 2H), 1.75-1.50 (m, 7H), 1.50-1.40 (m, 2H), 1.11-0.98 (m, 2H).

### Step 4: preparation of benzyl 4-(2-(((1S,4S-4-(5-amino-6-methoxy-2H-indol-2-yl)-1-hydroxycyclohexyl)methoxy)ethyl)piperidine-1-carboxylate

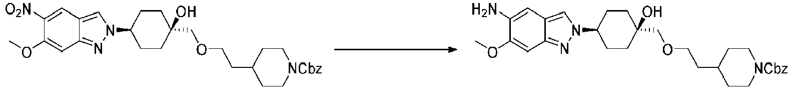

Raney-Ni (0.5 mL) and N₂H₄ H₂O (0.5 mL) were added to a solution of benzyl 4-(2-(((1S,4S)-1-hydroxy-4-(6-methoxy-5-nitro-2H-indol-2-yl)cyclohexyl)methoxy)ethyl)piperidine-1-carboxylate (250 mg, 0.44 mmol) in EtOH (20 mL). The reaction liquid was stirred at room temperature for 2 h. The reaction liquid was filtered through celite, and the filtrate was concentrated under reduced pressure to give benzyl 4-(2-(((1*S*,4*S*)-4-(5-amino-6-methoxy-2*H*-indol-2-yl)-1-hydroxycyclohexyl)methoxy)ethyl)piperidine-1-carboxylate. The resulting product was directly used in the next step.

LC-MS: (ESI, *m*/*z*): [M+H]⁺= 537.3

### Step 5: preparation of benzyl 4-(2-(((1S,4S)-1-hydroxy-4-(6-methoxy-5-(6-(trifluoromethyl)pyridinecarboxamido)-2H-indol-2-yl)cyclohexyl)methoxy)ethyl)piperidine-1-carboxylate

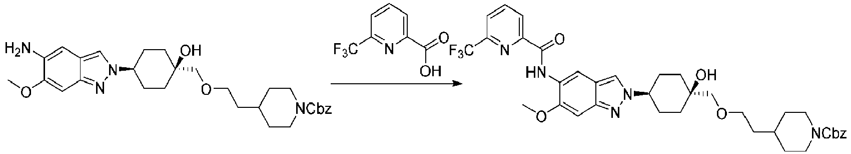

6-(Trifluoromethyl)picolinic acid (101 mg, 0.53 mmol), HATU (217 mg, 0.57 mmol), and DIEA (170 mg, 1.32 mmol) were added to a solution of benzyl 4-(2-(((1*S*,4*S*)-4-(5-amino-6-methoxy-2*H*-indol-2-yl)-1-hydroxycyclohexyl)methoxy)ethyl)piperidine-1-carboxylate (236 mg, 0.44 mmol) in DMF (5 mL) while stirring. Then the reaction liquid was stirred at room temperature for 2 h. The reaction liquid was diluted with water (50 mL) and extracted with EA (20 mL × 3). The organic phase was washed with saturated brine (60 mL), dried over anhydrous Na₂SO₄, filtered, and concentrated under reduced pressure. The resulting crude product was purified by flash column chromatography (EA:PE = 0-4:1) to give benzyl 4-(2-(((1*S*,4*S*)-1-hydroxy-4-(6-methoxy-5-(6-(trifluoromethyl)pyridinecarboxamido)-2H-indol-2-yl)cyclohexyl)methoxy)ethyl)piperidine-1-carboxylate.

LC-MS: (ESI, *m*/*z*): [M+H]⁺= 710.2.

¹H NMR (400 MHz, DMSO-d6) δ 10.50 (s, 1H), 8.69 (s, 1H), 8.46 (d, *J=* 7.7 Hz, 1H), 8.40 (t, *J=* 7.8 Hz, 1H), 8.32 (s, 1H), 8.21 (d, *J=* 7.7 Hz, 1H), 7.41-7.28 (m, 5H), 7.16 (s, 1H), 5.06 (s, 2H), 4.40-4.20 (m, 2H), 4.10-3.90 (m, 5H), 3.47 (t, *J=* 6.4 Hz, 2H), 3.21 (s, 2H), 2.90-2.70 (m, 2H), 2.32-2.18 (m, 2H), 1.90 (d, *J=* 10.4 Hz, 2H), 1.72-1.53 (m, 7H), 1.52-1.40 (m, 2H), 1.15-1.00 (m, 2H).

### Step 6: preparation of N-(2-((1S,4S)-4-hydroxy-4-((2-(piperidin-4-yl)ethoxy)methyl)cyclohexyl)-6-methoxy-2H-indol-5-yl)-6-(trifluoromethyl)pyridinecarboxamide

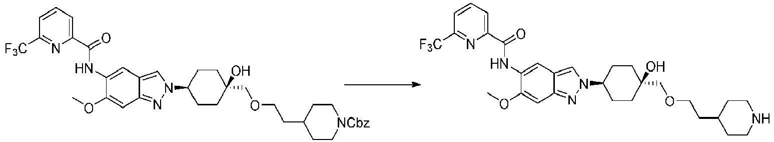

Pd(OH)₂/C (100 mg) was added to a solution of benzyl 4-(2-(((1*S*,4*S*)-1 -hydroxy-4-(6-methoxy-5-(6-(trifluoromethyl)pyridinecarboxamido)-2*H*-indol-2-yl)cyclohexyl)methoxy)ethyl)piperidine-1-carboxylate (200 mg, 0.28 mmol) in EA (20 mL). Then the reaction liquid was stirred at 70 °C overnight under H₂ atmosphere. The reaction liquid was filtered through celite, and the filtrate was concentrated to give *N-*(2-((1*S*,4*S*)-4-hydroxy-4-((2-(piperidin-4-yl)ethoxy)methyl)cyclohexyl)-6-methoxy-2*H*-indol-5-yl)-6-(trifluoromethyl)pyridinecarboxamide. The resulting product was directly used in the next step.

LC-MS: (ESI, *m*/*z):* [M+H]⁺= 576.2.

### Step 7: preparation of N-(2-((1S,4S)-4-((2-(1-(4-chloro-3-(2,4-dioxotetrahydropyrimidin-1(2H)-yl)benzoyl)piperidin-4-yl)ethoxy)methyl)-4-hydroxycyclohexyl)-6-methoxy-2H-indol-5-yl)-6-(trifluoromethyl)pyridinecarboxamide

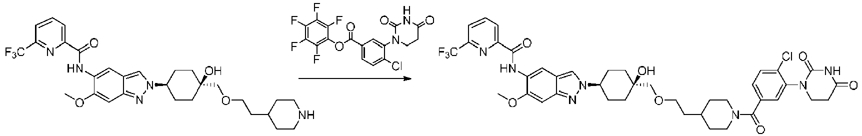

A solution of *N*-(2-((1*S*,4*S*)-4-hydroxy-4-((2-(piperidin-4-yl)ethoxy)methyl)cyclohexyl)-6-methoxy-2*H*-indol-5-yl)-6-(trifluoromethyl)pyridinecarboxamide (130 mg, 0.22 mmol), pentafluorophenyl 4-chloro-3-(2,4-dioxotetrahydropyrimidin-1(2*H*)-yl)benzoate (118 mg, 0.27 mmol), and DIEA (87 mg, 0.68 mmol) in DMSO (3 mL) was stirred at room temperature overnight. The reaction liquid was diluted with water (30 mL) and extracted with DCM (10 mL × 3). The organic phase was washed with saturated brine (30 mL), dried over anhydrous Na₂SO₄, filtered, and concentrated under reduced pressure. The resulting crude product was purified by flash column chromatography (MeOH:DCM = 0-1:9) to give *N*-(2-((1*S*,4*S*)-4-((2-(1-(4-chloro-3-(2,4-dioxotetrahydropyrimidin-1(2*H*)-yl)benzoyl)piperidin-4-yl)ethoxy)methyl)-4-hydroxycyclohexyl)-6-methoxy-2*H*-indol-5-yl)-6-(trifluoromethyl)pyridinecarboxamide.

LC-MS: (ESI, *m*/*z):* [M+H]⁺= 826.1.

¹H NMR (400 MHz, DMSO-*d*₆) δ 10.51 (d, *J* = 5.7 Hz, 2H), 8.69 (s, 1H), 8.46 (d, *J* = 7.8 Hz, 1H), 8.41 (t, *J=* 7.8 Hz, 1H), 8.33 (s, 1H), 8.22 (d, *J=* 7.7 Hz, 1H), 7.64 (d, *J=* 8.2 Hz, 1H), 7.55 (d, *J=* 1.8 Hz, 1H), 7.39 (dd, *J* = 8.2, 1.8 Hz, 1H), 7.16 (s, 1H), 4.50-4.26 (m, 3H), 3.98 (s, 3H), 3.83-3.53 (m, 3H), 3.48 (t, *J* = 6.3 Hz, 2H), 3.21 (s, 2H), 3.13-2.97 (m, 1H), 2.86-2.71 (m, 3H), 2.32-2.18 (m, 2H), 1.90 (d, *J* = 9.8 Hz, 2H), 1.85-1.45 (m, 9H), 1.15-1.05 (m, 2H).

### Example 11: 5-((1R,4R)-2-oxa-5-azabicyclo[2.2.1]heptan-5-yl)-N-(1-((1R,4R)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)pyrazolo[1,5-a]pyrimidine-3-carboxamide

### Step 1: preparation of tert-butyl 4-(2-(phenyl(methyl)amino)ethyl)piperidine-1-carboxylate

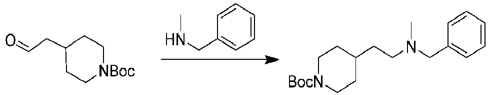

*N-*Methyl-1-phenylmethylamine (1.067 g, 8.82 mmol) andAcOH (0.79 g, 13.23 mmol) were added to a solution of tert-butyl 4-(2-oxoethyl)piperidine-1-carboxylate (1 g, 4.41 mmol) in DCE (3 mL) under N₂ atmosphere. After the mixture was stirred at room temperature for 30 min, STAB (2.8 g, 13.23 mmol) was added, and then the reaction liquid was stirred at room temperature for 2 h. The reaction liquid was diluted with water (20 mL), and the mixture was extracted with EA (30 mL × 2). The organic phase was dried over anhydrous Na₂SO₄, filtered, and concentrated under reduced pressure. The resulting crude product was purified by column chromatography (MeOH:DCM = 0-1:20) to give *tert-butyl* 4-(2-(phenyl(methyl)amino)ethyl)piperidine-1 -carboxylate.

LC-MS: (ESI, *m*/*z*): [M+H]⁺= 333.2.

### Step 2: preparation of tert-butyl 4-(2-(methylamino)ethyl)piperidine-1-carboxylate

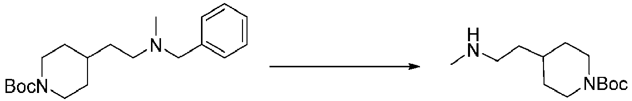

Pd(OH)₂/C (250 mg) was added to a solution of *tert-butyl* 4-(2-(phenyl(methyl)amino)ethyl)piperidine-1-carboxylate (1 g, 3.01 mmol) in THF (3 mL), and the reaction liquid was then stirred at room temperature for 2 h under H₂ atmosphere. The reaction liquid was filtered through celite, and the filtrate was concentrated under reduced pressure to give tert-butyl 4-(2-(methylamino)ethyl)piperidine-1 -carboxylate.

LC-MS: (ESI, m/z): [M+H]⁺= 243.2.

### Step 3: preparation of tert-butyl 4-(2-((((1R,4R)-4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)cyclohexyl)methyl)(methyl)amino)ethyl)piperidine-1-carboxylate

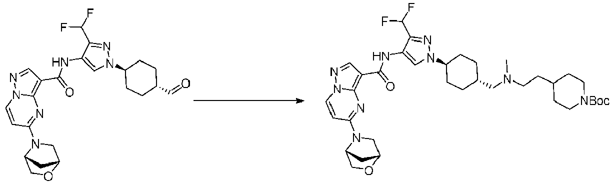

tert-Butyl 4-(2-(methylamino)ethyl)piperidine-1-carboxylate (329 mg, 0.928 mmol) and STAB (419 mg, 1.862 mmol) were added to a solution of 5-((1R,4R)-2-oxa-5-azabicyclo[2.2.1]heptan-5-yl)-N-(3-(difluoromethyl)-1-((1*R*,4*R*)-4-formylcyclohexyl)-1*H*-pyrazol-4-yl)pyrazolo[1,5-*a*]pyrimidine-3-carboxamide (300 mg, 0.618 mmol) in THF (3 mL), and the reaction was stirred at room temperature for 2 h under N2 atmosphere. Water (20 mL) was added to quench the reaction, and the mixture was extracted with DCM (20 mL × 2). The organic phase was dried over anhydrous Na₂SO₄, filtered, and concentrated under reduced pressure. The resulting crude product was purified by column chromatography (MeOH:DCM = 0-1:9) to give tert-butyl 4-(2-((((1*R*,*4*R)-4-(4-(5-((*1*R,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)cyclohexyl)methyl)(methyl)amino)ethyl)piperidine-1-carboxylate.

LC-MS: (ESI, m/z): [M+H]^{|} = 712.4.

### Step 4: preparation of 5-((1R,4R)-2-oxa-5-azabicyclo[2.2.1]heptan-5-yl)-N-(3-(difluoromethyl)-1-((1R,4R)-4-((methyl(2-(piperidin-4-yl)ethyl)amino)methyl)cyclohexyl)-1H-pyrazol-4-yl)pyrazolo[1,5-a]pyrimidine-3-carboxamide

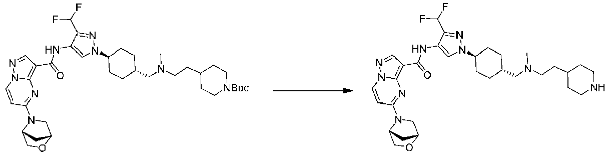

TFA (1 mL) was added to a solution of tert-butyl 4-(2-((((1R,4R)-4-(4-(5-((1R,4R)-2-oxa-5-azabicyclo[2.2.1]heptan-5-yl)pyrazolo[1,5-*a*]pyrimidine-3-carboxamido)-3-(difluoromethyl)-1*H-*pyrazol-1-yl)cyclohexyl)methyl)(methyl)amino)ethyl)piperidine-1-carboxylate (200 mg, 0.281 mmol) in DCM (3 mL), and the reaction liquid was stirred at room temperature for 2 h under N2 atmosphere. The reaction liquid was directly concentrated under reduced pressure to give 5-((1*R*,4*R*)-2-oxa-5-azabicyclo[2.2.1]heptan-5-yl)-*N*-(3-(difluoromethyl)-1-((1*R*,4*R*)-4-((methyl(2-(piperidin-4-yl)ethyl)amino)methyl)cyclohexyl)-1H-pyrazol-4-yl)pyrazolo[1,5-*a*]pyrimidine-3-carboxamide.

LC-MS: (ESI, m/z): [M+H]⁺= 612.4.

### Step 5: preparation of 5-((1R,4R)-2-oxa-5-azabicyclo[2.2.1]heptan-5-yl)-N(1-((1R,4R)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)pyrazolo[1,5-a]pyrimidine-3-carboxamide

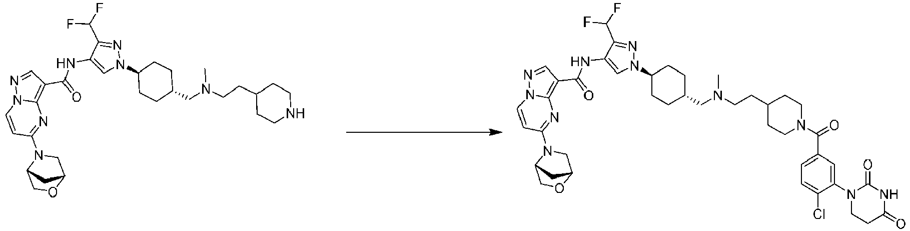

DIEA (109 mg, 1.564 mmol) and pentafluorophenyl 4-chloro-3-(2,4-dioxotetrahydropyrimidin-1(2*H*)-yl)benzoate (134 mg, 1.043 mmol) were added to a solution of 5-((1R,4R)-2-oxa-5-azabicyclo[2.2.1]heptan-5-yl)-N-(3-(difluoromethyl)-1-((1R,4R)-4-((methyl(2-(piperidin-4-yl)ethyl)amino)methyl)cyclohexyl)-1*H*-pyrazol-4-yl)pyrazolo[1,5-*a*]pyrimidine-3-carboxamide (175mg, 0.521 mmol) in DMSO (3 mL), and then the reaction liquid was stirred at room temperature for 3 h. Water (15 mL) was added to the reaction liquid, and the mixture was extracted with DCM (30 mL × 2) and dried over anhydrous Na₂SO₄. The organic phase was concentrated under reduced pressure, and the resulting crude product was purified by Prep-HPLC (acetonitrile/0.05% aqueous NH₄HCO₃ solution, 5%-95%) to give 5-((1*R*,4*R*)-2-oxa-5-azabicyclo[2.2.1]heptan-5-yl)-*N*-(1-((1*R*,4*R*)-4-(((2-(1-(4-chloro-3-(2,4-dioxotetrahydropyrimidin-1(2*H*)-yl)benzoyl)piperidin-4-yl)ethyl)(methyl)amino)methyl)cyclohexyl)-3-(difluoromethyl)-1*H-*pyrazol-4-yl)pyrazolo[1,5*-a*]pyrimidine-3-carboxamide.

LC-MS: (ESI, *m*/*z*): [M+H]⁺= 862.1.

¹H NMR (400 MHz, DMSO-*d*₆) δ 10.50 (s, 1H), 9.50 (d, *J=* 6.0 Hz, 1H), 8.78 (d, J= 7.7 Hz, 1H), 8.38 (d, *J=* 4.4 Hz, 1H), 8.25 (d, *J=* 5.6 Hz, 1H), 7.62 (t, *J=* 7.7 Hz, 1H), 7.54 (d, *J=* 1.9 Hz, 1H), 7.38 (dd, *J=* 8.2, 2.0 Hz, 1H), 7.26-6.95 (m, 1H), 6.90-6.42 (m, 1H), 5.31-5.05 (m, 1H), 4.77 (d, J= 17.3 Hz, 1H), 4.52-4.34 (m, 1H), 4.24-4.12 (m, 1H), 3.86-3.70 (m, 3H), 3.65-3.53 (m, 3H), 3.45 (d, *J=* 10.0 Hz, 1H), 3.11-2.94 (m, 1H), 2.77-2.65 (m, 3H), 2.29 (t, *J=* 7.0 Hz, 2H), 2.15-2.07 (m, 5H), 2.05-1.82 (m, 6H), 1.80-1.68 (m, 3H), 1.66-1.46 (m, 3H), 1.41-1.32 (m, 2H), 1.18-0.95 (m, 4H).

### Example 12: 5-((1R,4R)-2-oxa-5-azabicyclo[2.2.1]heptan-5-yl)-N (1-(1-(3-(1-(4-chloro-3-(2,4-dioxotetrahydropyrimidin-1(2H)-yl)benzoyl)piperidin-4-yl)prop-2-yn-1-yl)piperidin-4-yl)-3-(difluoromethyl)-1H-pyrazol-4-yl)pyrazolo[1,5-a]pyrimidine-3-carboxamide

### Step 1: preparation of tert-butyl 4-(3-(4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)prop-1-yn-1-yl)piperidine-1-carboxylate

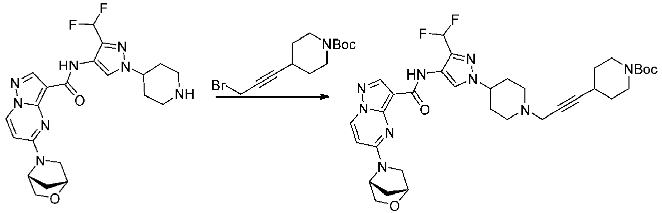

A mixture of 5-((1*R*,4*R*)-2-oxa-5-azabicyclo[2.2.1]heptan-5-yl)-*N*-(3-(difluoromethyl)-1-(piperidin-4-yl)-1*H*-pyrazol-4-yl)pyrazolo[1,5-*a*]pyrimidine-3-carboxamide (150 mg, TFA salt, 0.33 mmol), tert-butyl 4-(3-bromoprop-1-yn-1-yl)piperidine-1-carboxylate (99 mg, 0.33 mmol), and K₂CO₃ (227 mg, 1.65 mmol) in THF (20 mL) was stirred at 70 °C overnight. The reaction liquid was diluted with water (20 mL), extracted with DCM (20 mL × 3), dried over anhydrous Na₂SO₄, and concentrated under reduced pressure. The resulting crude product was purified by flash column chromatography (EA:PE = 0-1) to give tert-butyl 4-(3-(4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)- 177-pyrazol-1 -yl)piperidin-1 -yl)prop-1 -yn-1 -yl)piperidine-1 -carboxylate.

LC-MS: (ESI, *m*/*z):* [M+H]⁺= 680.3.

### Step 2: preparation of 5-((1R,4R)-2-oxa-5-azabicyclo[2.2.1]heptan-5-yl)-N-(3-(difluoromethyl)-1-(1-(3-(piperidin-4-yl)prop-2-yn-1-yl)piperidin-4-yl)-1Hpyrazol-4-yl)pyrazolo[1,5-a]pyrimidine-3-carboxamide

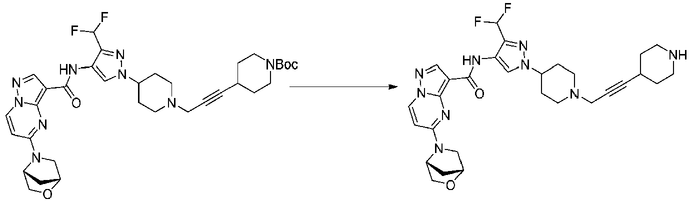

A mixture of tert-butyl 4-(3-(4-(4-(5-((1R,4R)-2-oxa-5-azabicyclo[2.2.1]heptan-5-yl)pyrazolo[1,5-a]pyrimidine-3-carboxamido)-3-(difluoromethyl)-1*H*-pyrazol-1-yl)piperidin-1-yl)prop-1-yn-1-yl)piperidine-1-carboxylate (70 mg, 0.10 mmol) in TFA/DCM (3 mL, 1/5) was stirred at room temperature for 1 h. The reaction liquid was directly concentrated under reduced pressure to give 5-((1R,4R)-2-oxa-5-azabicyclo[2.2.1]heptan-5-yl)-*N*-(3-(difluoromethyl)-1-(1-(3-(piperidin-4-yl)prop-2-yn-1-yl)piperidin-4-yl)-1*H*-pyrazol-4-yl)pyrazolo[1,5-*a*]pyrimidine-3-carboxamide. The resulting product was directly used in the next step.

LC-MS: (ESI, *m*/*z):* [M+H]⁺= 580.2.

### Step 3: preparation of 5-((1R,4R)-2-oxa-5-azabicyclo[2.2.1]heptan-5-yl)-N-(1-(1-(3-(1-(4-chloro-3-(2,4-dioxotetrahydropyrimidin-1(2H)-yl)benzoyl)piperidin-4-yl)prop-2-yn-1-yl)piperidin-4-yl)-3-(difluoromethyl)-1H-pyrazol-4-yl)pyrazolo[1,5-a]pyrimidine-3-carboxamide

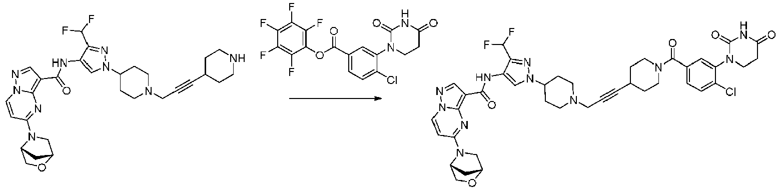

A mixture of 5-((1R,4R)-2-oxa-5-azabicyclo[2.2.1]heptan-5-yl)-N (3-(difluoromethyl)-1-(1-(3-(piperidin-4-yl)prop-2-yn-1-yl)piperidin-4-yl)-1*H-*pyrazol-4-yl)pyrazolo[1,5-*a*]pyrimidine-3-carboxamide (70 mg, crude, 0.12 mmol), pentafluorophenyl 4-chloro-3-(2,4-dioxotetrahydropyrimidin-1(2*H*)-yl)benzoate (52 mg, 0.12 mmol), and DIEA (774 mg, 0.60 mmol) in DMSO (5 mL) was stirred at room temperature overnight. The reaction liquid was poured into water (50 mL) and stirred for 10 min. The mixture was filtered, and the filter cake was purified by flash column chromatography (MeOH:DCM = 0-1:9) to give 5-((1*R*,4*R*)-2-oxa-5-azabicyclo[2.2.1]heptan-5-yl)-*N-*(1-(1-(3-(1-(4-chloro-3-(2,4-dioxotetrahydropyrimidin-1(2*H*)-yl)benzoyl)piperidin-4-yl)prop-2-yn-1-yl)piperidin-4-yl)-3-(difluoromethyl)-1*H-*pyrazol-4-yl)pyrazolo[1,5-*a*]pyrimidine-3-carboxamide.

LC-MS: (ESI, *m*/*z):* [M+H]^{|} = 830.3.

¹H NMR (400 MHz, DMSO-*d*₆) δ 10.51 (s, 1H), 9.51 (d, J= 5.8 Hz, 1H), 8.79 (d, J= 7.7 Hz, 1H), 8.42 (s, 1H), 8.26 (d, J= 5.6 Hz, 1H),7.64 (d, J= 8.2 Hz, 1H), 7.58 (d, *J=* 1.9 Hz, 1H), 7.41 (dd, J= 8.2, 2.0 Hz, 1H), 7.28-6.97 (m, 1H), 6.90-6.43 (m, 1H), 5.30-5.02 (m, 1H), 4.77 (d, *J=* 18.9 Hz, 1H), 4.30-4.20 (m, 1H), 4.18-3.90 (m, 1H), 3.88-3.70 (m, 3H), 3.68-3.56 (m, 3H), 3.54-3.36 (m, 3H), 3.30-3.21 (m, 2H), 3.00-2.64 (m, 5H), 2.40-2.20 (m, 2H), 2.12-1.73 (m, 8H), 1.62-1.42 (m, 2H).

### Example 13: 5-((1R,4R)-2-oxa-5-azabicyclo[2.2.1]heptan-5-yl)-N-(3-(difluoromethyl)-1-(1-((3-(3-(2,4-dioxotetrahydropyrimidin-1(2H)-yl)-4-methoxybenzoyl)-3-azaspiro[5.5]undecan-9-yl)methyl)piperidin-4-yl)-1H-pyrazol-4-yl)pyrazolo[1,5-a]pyrimidine-3-carboxamide

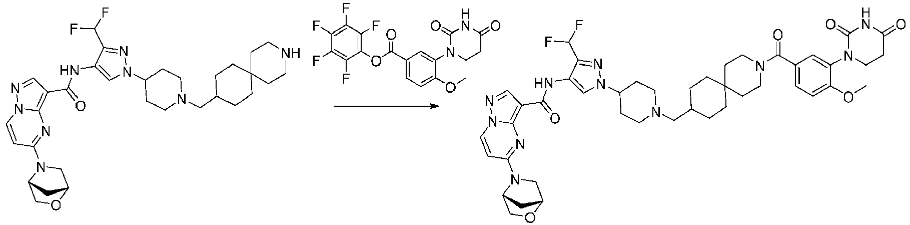

A mixture of *N*-(1-(1-((3-azaspiro[5.5]undecan-9-yl)methyl)piperidin-4-yl)-3-(difluoromethyl)-1*H-*pyrazol-4-yl)-5-((1*R*,4*R*)-2-oxa-5-azabicyclo[2.2.1]heptan-5-yl)pyrazolo[1,5*-a*]pyrimidine-3-carboxamide (160 mg, 0.26 mmol), pentafluorophenyl 3-(2,4-dioxotetrahydropyrimidin-1(2*H*)-yl)-4-methoxybenzoate (110 mg, 0.26 mmol), and DIEA (168 mg, 1.3 mmol) in DMSO (5 mL) was stirred at room temperature overnight. The reaction liquid was poured into water (50 mL) and stirred for 10 min. The mixture was filtered, and the filter cake was purified by Prep-HPLC to give 5-((1*R*,4*R*)-2-oxa-5-azabicyclo[2.2.1]heptan-5-yl)-*N-*(3-(difluoromethyl)-1-(1-((3-(3-(2,4-dioxotetrahydropyrimidin-1(2*H*)-yl)-4-methoxybenzoyl)-3-azaspiro[5.5]undecan-9-yl)methyl)piperidin-4-yl)-1*H*-pyrazol-4-yl)pyrazolo[1,5-*a*]pyrimidine-3-carboxamide.

LC-MS: (ESI, *m*/*z*): [M+H]⁺= 870.3.

¹H NMR (400 MHz, DMSO-*d*₆) δ 10.33 (s, 1H), 9.50 (d, *J* = 5.7 Hz, 1H), 8.79 (d, *J* = 7.7 Hz, 1H), 8.39 (d, *J=* 4.0 Hz, 1H), 8.26 (d, *J* = 5.6 Hz, 1H), 7.41-7.29 (m, 2H), 7.27-6.95 (m, 2H), 6.91-6.40 (m, 1H), 5.31-5.00 (m, 1H), 4.77 (d, *J=* 17.5 Hz, 1H), 4.27-4.15 (m, 1H), 3.86-3.71 (m, 5H), 3.65-3.39 (m, 8H), 2.89 (d, *J=* 10.4 Hz, 2H), 2.68 (t, *J=* 6.5 Hz, 2H), 2.15 (d, *J* = 6.1 Hz, 2H), 2.08-1.87 (m, 8H), 1.76-1.65 (m, 2H), 1.63-1.38 (m, 5H), 1.36-1.21 (m, 2H), 1.19-0.95 (m, 4H).

### Example 14: 5-((1R,4R)-2-oxa-5-azabicyclo[2.2.1]heptan-5-yl)-N-(1-(1-(3-(1-(4-chloro-3-(2,4-dioxotetrahydropyrimidin-1(2H)-yl)benzoyl)piperidin-4-yl)propyl)piperidin-4-yl)-3-(difluoromethyl)-1H-pyrazol-4-yl)pyrazolo[1,5-a]pyrimidine-3-carboxamide

### Step 1: preparation of tert-butyl 4-(3-oxopropyl)piperidine-1-carboxylate

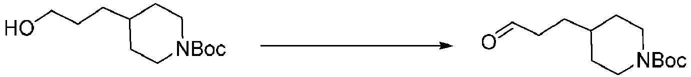

IBX (2.88 g, 10.285 mmol) was added to a solution of tert-butyl 4-(3-hydroxypropyl)piperidine-1-carboxylate (1.0 g, 4.115 mmol) in ACN (30 mL), and the mixture was stirred at 80 °C for 2 h. After the reaction was complete, the mixture was extracted with ethyl acetate (50 mL × 3), and the organic phase was washed with saturated brine (150 mL), dried over anhydrous sodium sulfate, filtered, and concentrated under reduced pressure. The crude product was purified by column chromatography (PE:EA = 1:1) to give tert-butyl 4-(3-oxopropyl)piperidine-1-carboxylate.

¹H NMR (400 MHz, DMSO-*d*₆) δ 9.68 (t, *J=* 1.5 Hz, 1H), 3.96-3.86 (m, 2H), 2.70-2.60 (m, 2H), 2.46 (td, *J* = 7.5, 1.4 Hz, 2H), 1.66-1.56 (m, 3H), 1.56-1.36 (m, 4H), 1.39 (s, 9H).

### Step 2: preparation of tert-butyl 4-(3-(4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)propyl)piperidine-1-carboxylate

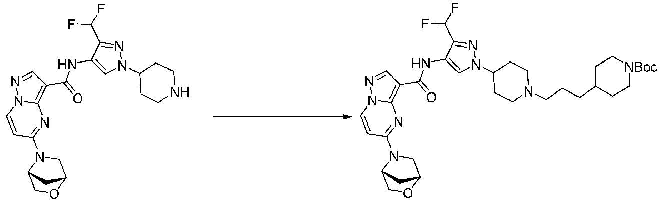

tert-Butyl 4-(3-oxopropyl)piperidine-1-carboxylate (95 mg, 0.394 mmol) and STAB (208 mg, 0.981 mmol) were added to a solution of 5-((1*R*,4*R*)-2-oxa-5-azabicyclo[2.2.1]heptan-5-yl)-*N-*(3-(difluoromethyl)-1-(piperidin-4-yl)-1*H*-pyrazol-4-yl)pyrazolo[1,5-*a*]pyrimidine-3-carboxamide (150 mg, 0.327 mmol) in THF (30 mL), and the mixture was stirred at room temperature for 2 h and extracted with ethyl acetate (40 mL × 3). The organic phase was washed with saturated brine (150 mL), dried over anhydrous sodium sulfate, filtered, and concentrated under reduced pressure. The crude product was purified by column chromatography (PE:EA = 3:7) to give tert-butyl 4-(3-(4-(4-(5-((1R,4R)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)propyl)piperidine-1-carboxylate.

LC-MS: (ESI, *m*/*z*): [M+H]⁺= 684.2.

### Step 3: preparation of 5-((1R,4R)-2-oxa-5-azabicyclo[2.2.1]heptan-5-yl)-N-(3-(difluoromethyl)-1-(1-(3-(piperidin-4-yl)propyl)piperidin-4-yl)-1H-pyrazol-4-yl)pyrazolo[1,5-a]pyrimidine-3-carboxamide

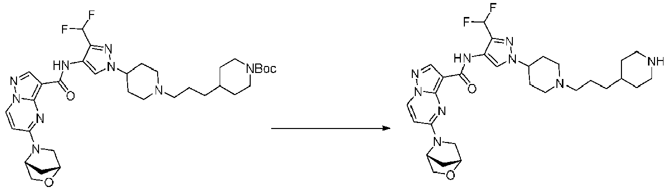

TFA (5 mL) was added to a solution of tert-butyl 4-(3-(4-(4-(5-((1R,4R)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)propyl)piperidine-1-carboxylate (200 mg, 0.293 mmol) in DCM (20 mL), and the mixture was stirred at room temperature for 2 h, made basic with a saturated NaHCO₃ solution, and extracted with ethyl acetate (60 mL × 3). The organic phase was washed with saturated brine (150 mL) and concentrated under reduced pressure to give 5-((1*R*,4*R*)-2-oxa-5-azabicyclo[2.2.1]heptan-5-yl)-*N-*(3-(difluoromethyl)-1-(1-(3-(piperidin-4-yl)propyl)piperidin-4-yl)-1*H*-pyrazol-4-yl)pyrazolo[1,5-*a*]pyrimidine-3-carboxamide.

LC-MS: (ESI, *m*/*z*): [M+H]⁺= 584.2.

### Step 4: preparation of 5-((1R,4R)-2-oxa-5-azabicyclo[2.2.1]heptan-5-yl)-N*(1-(1-(3-(1-(4-chloro-3-(2,4-dioxotetrahydropyrimidin-1(2H)-yl)benzoyl)piperidin-4-yl)propyl)piperidin-4-yl)-3-(difluoromethyl)-1 H-pyrazol-4-yl)pyrazolo[1,5-a]pyrimidine-3-carboxamide

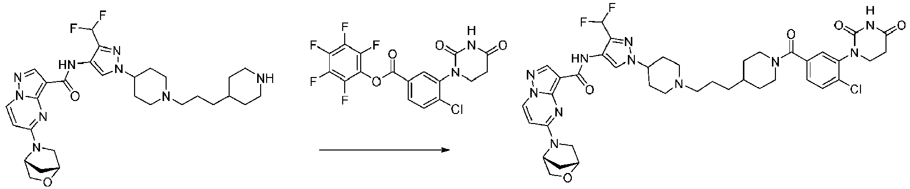

5-((1 *R*,4*R*)-2-Oxa-5-azabicyclo[2.2.1]heptan-5-yl)-*N-*(3-(difluoromethyl)-1-(1-(3-(piperidin-4-yl)propyl)piperidin-4-yl)-1*H*-pyrazol-4-yl)pyrazolo[1,5-*a*]pyrimidine-3-carboxamide (150 mg, 0.257 mmol) and pentafluorophenyl 4-chloro-3-(2,4-dioxotetrahydropyrimidin-1(2H)-yl)benzoate (134 mg, 0.308 mmol) were dissolved in DMSO (5 mL). DIEA (100 mg, 0.775 mmol) was added, and then the mixture was stirred at room temperature for 2 h. Water (50 mL) was added to the reaction liquid, and the mixture was stirred for 2 min and filtered. The filter cake was purified by preparative reversed-phase chromatography to give 5-((1*R*,4*R*)-2-oxa-5-azabicyclo[2.2.1]heptan-5-yl)*-N*-(1-(1-(3-(1-(4-chloro-3-(2,4-dioxotetrahydropyrimidin-1(2*H*)-yl)benzoyl)piperidin-4-yl)propyl)piperidin-4-yl)-3-(difluoromethyl)-1*H*-pyrazol-4-yl)pyrazolo[1,5-*a*]pyrimidine-3-carboxamide.

LC-MS: (ESI, *m*/*z):* [M+H]⁺ = 834.0.

¹H NMR (400 MHz, DMSO-*d*₆) δ 10.51 (s, 1H), 9.50 (d, *J* = 5.8 Hz, 1H), 8.79 (d, *J* = 7.7 Hz, 1H), 8.40 (d, *J=* 3.7 Hz, 1H), 8.26 (d, *J* = 5.6 Hz, 1H), 7.64 (d, *J=* 8.2 Hz, 1H), 7.55 (d, *J=* 1.8 Hz, 1H), 7.39 (dd, *J* = 8.2, 1.9 Hz, 1H), 7.27-6.96 (m, 1H), 6.90-6.40 (m, 1H), 5.30-5.05 (m, 1H), 4.77 (d,J= 17.5 Hz, 1H), 4.52-4.33 (m, 1H), 4.26-4.14 (m, 1H), 3.85-3.72 (m, 3H), 3.66-3.57 (m, 3H), 3.45 (d, *J=* 10.2 Hz, 1H), 3.11-2.89 (m, 3H), 2.79-2.66 (m, 3H), 2.30 (t, *J=* 6.9 Hz, 2H), 2.09-1.84 (m, 9H), 1.82-1.69 (m, 1H), 1.68-1.58 (m, 1H), 1.56-1.40 (m, 3H), 1.30-1.20 (m, 2H), 1.16-1.02 (m, 2H).

### Example 15: N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)-5-morpholinopyrazolo[1,5 -a]pyrimidine-3-carboxamide

### Step 1: preparation of tert-butyl 4-(3-(difluoromethyl)-4-(5-morpholinopyrazolo[1,5-a]pyrimidine-3-carboxamido)-1H-pyrazol-1-yl)piperidine-1-carboxylate

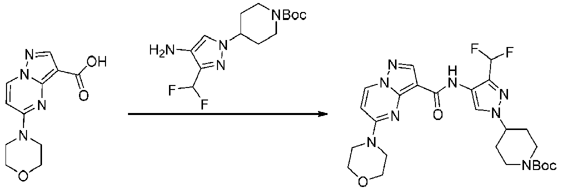

HATU (900 mg, 2.4 mmol) was added to a solution of 5-morpholinopyrazolo[1,5-*a*]pyrimidine-3-carboxylic acid (470 mg, 1.9 mmol) and DIEA (1 mL, 4.7 mmol) in DMF (20 mL) at 25 °C. After the mixture was stirred at 25 °C for 2 h, tert-butyl 4-(4-amino-3-(difluoromethyl)-1*H-*pyrazol-1-yl)piperidine-1-carboxylate (0.5 g, 1.5 mmol) was added, and the reaction liquid was then stirred overnight at 25 °C. Water (30 mL) was added to the reaction liquid, and the mixture was then extracted with EA (50 mL × 3). The organic phases were combined, washed with saturated brine, dried over anhydrous Na₂SO₄, filtered, and concentrated under reduced pressure. The resulting crude product was purified by flash column chromatography (EA) to give *tert-butyl* 4-(3-(difluoromethyl)-4-(5-morpholinopyrazolo[1,5-*a*]pyrimidine-3-carboxamido)-1*H-*pyrazol-1-yl)piperidine-1-carboxylate.

LC-MS: (ESI, m/z): [M+H]^{|} = 547.4.

### Step 2: preparation of N-(3-(difluoromethyl)-1-(piperidin-4-yl)-1H-pyrazol-4-yl)-5-morpholinopyrazolo[1,5 -a]pyrimidine-3-carboxamide

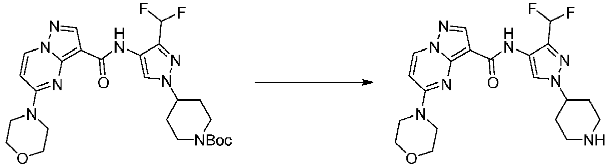

TFA (1 mL) was added to a solution of tert-butyl 4-(3-(difluoromethyl)-4-(5-morpholinopyrazolo[1,5-a]pyrimidine-3-carboxamido)-1H-pyrazol-1 -yl)piperidine-1-carboxylate (0.2 g, 0.366 mmol) in DCM (3 mL) at 25 °C. The reaction liquid was stirred at 25 °C for 1 h. The reaction liquid was directly concentrated under reduced pressure to give *N*-(3-(difluoromethyl)-1-(piperidin-4-yl)-1*H-*pyrazol-4-yl)-5-morpholinopyrazolo[1,5-*a*]pyrimidine-3-carboxamide.

LC-MS: (ESI, m/z): [M+H]^{|} = 447.2.

### Step 3: preparation of tert-butyl 9-((4-(3-(difluoromethyl)-4-(5-morpholinopyrazolo[1,5-α]pyrimidine-3-carboxamido)-1 H-pyrazol-1-yl)piperidin-1-yl)methyl)-3-azaspiro[5.5]undecane-3-carboxylate

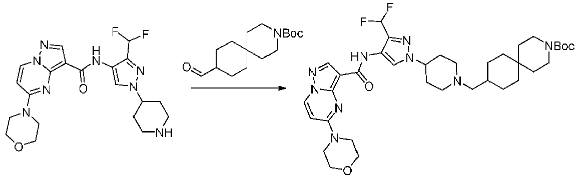

tert-Butyl 9-formyl-3-azaspiro[5.5]undecane-3-carboxylate (150 mg, 0.45 mmol) and NaBH₃CN (100 mg, 2.25 mmol) were added to a solution of N-(3-(difluoromethyl)-1-(piperidin-4-yl)-1H-pyrazol-4-yl)-5-morpholinopyrazolo[1,5-*a*]pyrimidine-3-carboxamide (200 mg, 0.45 mmol) andDIEA(0.5 mL, 1.35 mmol) in MeOH (5 mL) at 25 °C. The reaction liquid was stirred at 25 °C for 16 h. After filtration and concentration under reduced pressure, the resulting crude product was purified by flash column chromatography (MeOH:DCM = 3:97) to give tert-butyl 9-((4-(3-(difluoromethyl)-4-(5-morpholinopyrazolo[1,5-*a*]pyrimidine-3-carboxamido)-1*H*-pyrazol-1-yl)piperidin-1-yl)methyl)-3-azaspiro[5.5]undecane-3-carboxylate.

LC-MS: (ESI, m/z): [M+H]⁺= 712.4.

### Step 4: preparation of N-(1-(1-((3-azaspiro[5.5]undecan-9-yl)methyl)piperidin-4-yl)-3-(difluoromethyl)-1 H-pyrazol-4-yl)-5-morpholinopyrazolo[1,5-a]pyrimidine-3-carboxamide

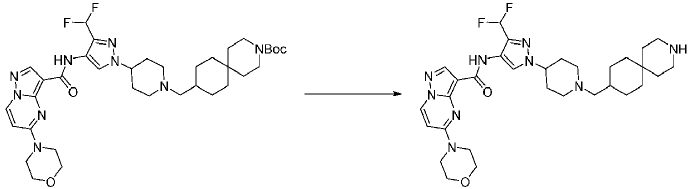

TFA (1 mL) was added to a solution of tert-butyl 9-((4-(3-(difluoromethyl)-4-(5-morpholinopyrazolo[1,5-*a*]pyrimidine-3-carboxamido)-1*H*-pyrazol-1-yl)piperidin-1-yl)methyl)-3-azaspiro[5.5]undecane-3-carboxylate (0.14 g, 0.2 mmol) in DCM (3 mL) at 25 °C. The reaction liquid was stirred at 25 °C for 1 h. The reaction liquid was directly concentrated under reduced pressure to give N (1-(1-((3-azaspiro[5.5]undecan-9-yl)methyl)piperidin-4-yl)-3-(difluoromethyl)-1*H-*pyrazol-4-yl)-5-morpholinopyrazolo[1,5-*a*]pyrimidine-3-carboxamide. The resulting product was directly used in the next step.

LC-MS: (ESI, m/z): [M+H]^{|} = 612.5.

### Step 5: preparation of N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)-5-morpholinopyrazolo[1,5-a]pyrimidine-3-carboxamide

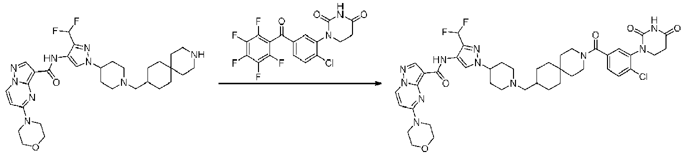

DIEA (0.1 mL, 0.54 mmol) was added to a solution of *N-*(1-(1-((3-azaspiro[5.5]undecan-9-yl)methyl)piperidin-4-yl)-3-(difluoromethyl)-1*H*-pyrazol-4-yl)-5-morpholinopyrazolo[1,5-*a*]pyrimidine-3-carboxamide (110 mg, 0.18 mmol) and 1-(2-chloro-5-pentafluorobenzoyl)phenyl)dihydropyrimidine-2,4(1*H*,3*H*)-dione (90 mg, 0.19 mmol) in DMSO (2 mL) at 25 °C. The reaction liquid was stirred at 25 °C for 16 h. The reaction liquid was poured into water (10 mL) and filtered, and the filter cake was purified by Prep-HPLC to give *N*-(1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H-*pyrazol-4-yl)-5-morpholinopyrazolo[1,5 -*a*]pyrimidine-3 -carboxamide.

LC-MS: (ESI, *m*/*z):* [M+H]⁺= 862.4.

¹H NMR (400 MHz, DMSO-*d*₆) δ 10.51 (s, 1H), 9.40 (s, 1H), 8.83 (d, *J=* 7.9 Hz, 1H), 8.39 (s, 1H), 8.29 (s, 1H), 7.63 (d, *J=* 8.2 Hz, 1H), 7.55 (d, *J=* 1.9 Hz, 1H), 7.39 (dd, *J* = 8.2, 1.8 Hz, 1H), 7.25-6.95 (m, 1H), 6.91 (d, *J=* 8.0 Hz, 1H), 4.26-4.14 (m, 1H), 3.87-3.68 (m, 9H), 3.66-3.50 (m, 3H), 3.31-3.20 (m, 2H), 2.95-2.83 (m, 2H), 2.80-2.68 (m, 2H), 2.21-2.08 (m, 2H), 2.07-1.87 (m, 6H), 1.70 (d, *J=* 8.7 Hz, 2H), 1.63-1.41 (m, 5H), 1.38-1.25 (m, 2H), 1.16-1.02 (m, 4H).

### Example 16: 5-(8-oxa-3-azabicyclo[3.2.1]octan-3-yl)-N-(3-(difluoromethyl)-1-(1-((3-(3-(2,4-dioxotetrahydropyrimidin-1(2H)-yl)-4-methoxybenzoyl)-3-azaspiro[5.5]undecan-9-yl)methyl)piperidin-4-yl)-1H-pyrazol-4-yl)pyrazolo[1,5-a]pyrimidine-3-carboxamide

### Step 1: preparation of ethyl 5-(8-oxa-3-azabicyclo[3.2.1]octan-3-yl)pyrazolo[1,5-a]pyrimidine-3-carboxylate

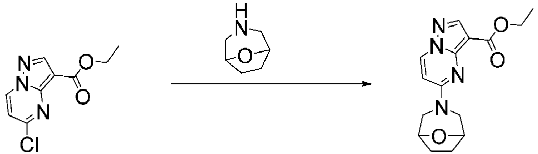

8-Oxa-3-azabicyclo[3.2.1]octane (1.3 g, 8.9 mmol) and DIEA (3.4 g, 26 mmol) were added to a solution of ethyl 5-chloropyrazolo[1,5-*a*]pyrimidine-3-carboxylate (2.0 g, 8.9 mmol) inACN (20 mL), and the reaction liquid was heated to 60 °C and reacted for 2 h. Water (200 mL) was added to quench the reaction, and the mixture was extracted with EA (200 mL × 2). The organic phase was dried over anhydrous Na₂SO₄, filtered, and concentrated under reduced pressure. The resulting crude product was purified by column chromatography (PE:EA = 1-5:1) to give ethyl 5-(8-oxa-3-azabicyclo[3.2.1]octan-3-yl)pyrazolo[1,5-α]pyrimidine-3-carboxylate.

LC-MS: (ESI, *m*/*z):* [M+H]^{|} = 303.2.

### Step 2: preparation of 5-(8-oxa-3-azabicyclo[3.2.1]octan-3-yl)pyrazolo[1,5-α]pyrimidine-3-carboxylic acid

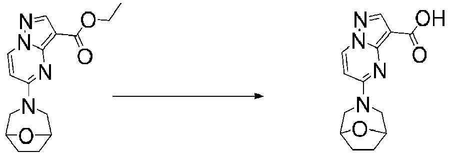

LiOH (1.4 g, 34 mmol) was added to a mixture of ethyl 5-(8-oxa-3-azabicyclo[3.2.1]octan-3-yl)pyrazolo[1,5-a]pyrimidine-3-carboxylate (2.6 g, 8.6 mmol) in H₂O/MeOH (3 mL/26 mL), and then the reaction liquid was heated to 60 °C and reacted for 12 h. The reaction liquid was filtered through celite, and the filtrate was concentrated under reduced pressure. The resulting crude product was purified by Prep-HPLC (acetonitrile/0.05% aqueous NH₄OH solution, 5%-95%) to give 5-(8-oxa-3-azabicyclo[3.2.1]octan-3-yl)pyrazolo[1,5-*a*]pyrimidine-3-carboxylic acid.

LC-MS: (ESI, *m*/*z):* [M+H]⁺= 275.3.

### Step 3: preparation of tert-butyl 4-(4-(5-(8-oxa-3-azabicyclo[3.2.1]octan-3-yl)pyrazolo[1,5-a]pyrimidine-3-carboxamido)-3-(difluoromethyl)-1H-pyrazol-1-yl)piperidine-1-carboxylate

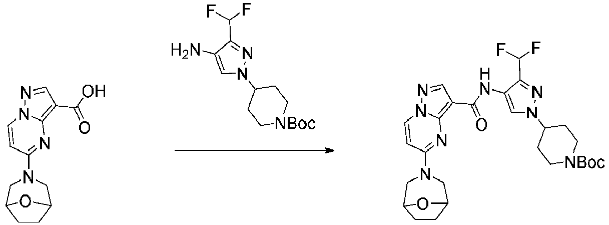

tert-Butyl 4-(4-amino-3-(difluoromethyl)-1*H*-pyrazol-1-yl)piperidine-1-carboxylate (316 mg, 1 mmol), HATU (456 mg, 1.2 mmol), and DIEA (387 mg, 3 mmol) were added to a solution of 5-(8-oxa-3-azabicyclo[3.2.1]octan-3-yl)pyrazolo[1,5-*a*]pyrimidine-3-carboxylic acid (274 mg, 1 mmol) in DMF (5 mL), and then the reaction liquid was reacted at 25 °C for 12 h. The reaction liquid was poured into water and then extracted with EA (50 mL × 2), and the organic phase was dried over anhydrous Na₂SO₄, filtered and concentrated under reduced pressure. The resulting crude product was purified by column chromatography (MeOH:DCM = 0-1:9) to give tert-butyl 4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)piperidine-1-carboxylate.

LC-MS: (ESI, *m*/*z):* [M+H]⁺= 573.4.

### Step 4: preparation of 5-(8-oxa-3-azabicyclo[3.2.1]octan-3-yl)-N-(3-(difluoromethyl)-1-(piperidin-4-yl)-1H-pyrazol-4-yl)pyrazolo[1,5-a]pyrimidine-3-carboxamide

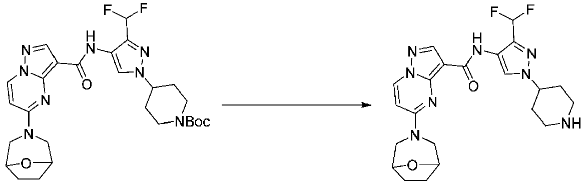

A solution of *tert*-butyl 4-(4-(5-(8-oxa-3-azabicyclo[3.2.1]octan-3-yl)pyrazolo[1,5-a]pyrimidine-3-carboxamido)-3-(difluoromethyl)-1*H*-pyrazol-1-yl)piperidine-1-carboxylate (240 mg, 0.42 mmol) in HCl/1,4-dioxane (5 mL) was stirred at room temperature for 1 h. The reaction liquid was directly concentrated under reduced pressure to give 5-(8-oxa-3-azabicyclo[3.2.1]octan-3-yl)-*N-*(3-(difluoromethyl)-1-(piperidin-4-yl)-1*H*-pyrazol-4-yl)pyrazolo[1,5-*a*]pyrimidine-3-carboxamide.

LC-MS: (ESI, *m*/*z):* [M+H]⁺= 473.4.

### Step 5: preparation of tert-butyl 9-((4-(4-(5-(8-oxa-3-azabicyclo[3.2.1]octan-3-yl)pyrazolo[1,5-a]pyrimidine-3-carboxamido)-3-(-difluoromethyl)-1H-pyrazol-1-yl)piperidin-1-yl)methyl)-3 azaspiro [5.5]undecane-3-carboxylate

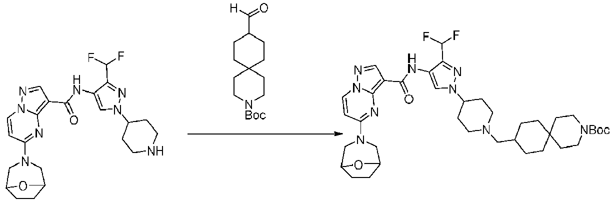

tert-Butyl 9-formyl-3-azaspiro[5.5]undecane-3-carboxylate (77 mg, 0.27 mmol) and STAB (175 mg, 0.8 mmol) were added to a solution of 5-(8-oxa-3-azabicyclo[3.2.1]octan-3-yl)-N (3-(difluoromethyl)-1-(piperidin-4-yl)-1*H*-pyrazol-4-yl)pyrazolo[1,5-a]pyrimidine-3-carboxamide (130 mg, 0.27 mmol) in THF (10 mL). The reaction liquid was then stirred at 25 °C for 12 h. The reaction liquid was filtered through celite, and the filtrate was concentrated under reduced pressure. The resulting crude product was purified by column chromatography (MeOH:DCM = 0-1:9) to give tert-butyl 9-((4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)piperidin-1-yl)methyl)-3-azaspiro[5.5] undecane-3-carboxylate.

LC-MS: (ESI, m/z): [M+H]⁺ = 738.3.

### Step 6: preparation of N-(1-(1-((3-azaspiro[5.5]undecan-9-yl)methyl)piperidin-4-yl)-3-(difluoromethyl)-1H-pyrazol-4-yl)-5-(8-oxa-3-azabicyclo[3.2.1]octan-3-yl)pyrazolo[1,5-a]pyrimidine-3-carboxamide

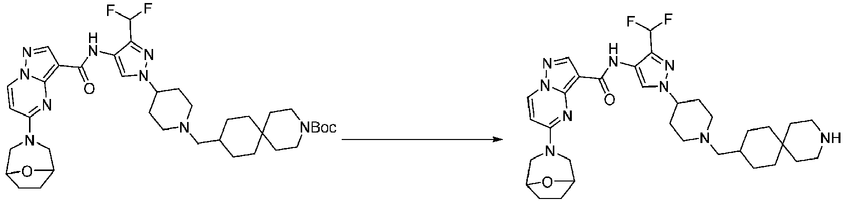

A solution of *tert-butyl* 9-((4-(4-(5-(8-oxa-3-azabicyclo[3.2.1]octan-3-yl)pyrazolo[1,5-α]pyrimidine-3-carboxamido)-3-(difluoromethyl)-1 H-pyrazol-1-yl)piperidin-1-yl)methyl)-3-azaspiro[5.5]undecane-3-carboxylate (100 mg, 0.14 mmol) in HCl/1,4-dioxane (4 mL) was stirred at 25 °C for 1 h. Then the reaction liquid was directly concentrated under reduced pressure to give *N*-(1-(1-((3-azaspiro[5.5]undecan-9-yl)methyl)piperidin-4-yl)-3-(difluoromethyl)-1*H*-pyrazol-4-yl)-5-(8-oxa-3-azabicyclo[3.2.1]octan-3-yl)pyrazolo[1,5-*a*]pyrimidine-3-carboxamide.

LC-MS: (ESI, m/z): [M+H]⁺= 638.3.

### Step 7: preparation of 5-(8-oxa-3-azabicyclo[3.2.1]octan-3-yl)-N-(3-(difluoromethyl)-1-(1-((3-(3-(2,4-dioxotetrahydropyrimidin-1(2H)-yl)-4-methoxybenzoyl)-3-azaspiro[5.5]undecan-9-yl)methyl)piperidin-4-yl)-1H-pyrazol-4-yl)pyrazolo[1,5-a]pyrimidine-3-carboxamide

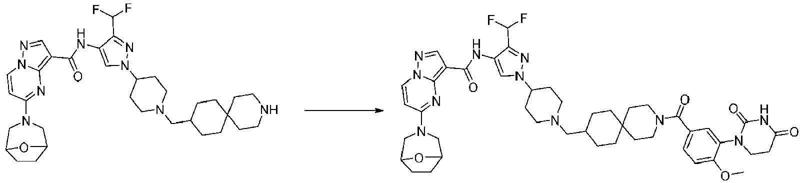

Pentafluorophenyl 3-(2,4-dioxotetrahydropyrimidin-1(2*H*)-yl)-4-methoxybenzoate (123 mg, 0.28 mmol) and DIEA (93 mg, 0.72 mmol) were added to a solution of N-(1-(1-((3-azaspiro[5.5]undecan-9-yl)methyl)piperidin-4-yl)-3-(difluoromethyl)-177-pyrazol-4-yl)-5-(8-oxa-3-azabicyclo[3.2.1]octan-3-yl)pyrazolo[1,5-*a*]pyrimidine-3-carboxamide (150.0 mg, 0.24 mmol) in DMSO (2 mL), and then the reaction liquid was stirred at room temperature for 12 h. The reaction liquid was poured into water (20 mL) and extracted with EA (30 mL × 2), and the organic phase was dried over anhydrous Na₂SO₄ and concentrated under reduced pressure. The resulting crude product was purified by Prep-HPLC (acetonitrile/0.05% aqueous NH₄HCO₃ solution, 5%-95%) to give 5-(8-oxa-3-azabicyclo[3.2.1]octan-3-yl)-N (3-(difluoromethyl)-1-(1-((3-(3-(2,4-dioxotetrahydropyrimidin-1(2*H*)-yl)-4-methoxybenzoyl)-3-azaspiro[5.5]undecan-9-yl)methyl)piperidin-4-yl)-1*H*pyrazol-4-yl)pyrazolo[1,5-*a*]pyrimidine-3-carboxamide.

LC-MS: (ESI, *m*/*z):* [M+H]^{|} = 884.5.

¹H NMR (400 MHz, DMSO-*d*₆) δ 10.33 (s, 1H), 9.41 (s, 1H), 8.81 (d, *J* = 7.8 Hz, 1H), 8.39 (s, 1H), 8.28 (d, J= 1.3 Hz, 1H), 7.37 (d, J= 8.4 Hz, 1H), 7.32 (s, 1H), 7.27-6.95 (m, 2H), 6.82 (d, *J=* 7.9 Hz, 1H), 4.50-4.37 (m, 2H), 4.30-4.00 (m, 2H), 3.84 (s, 3H), 3.68-3.34 (m, 6H), 3.28-3.11 (m, 2H), 2.98-2.78 (m, 2H), 2.73-2.60 (m, 2H), 2.22-1.63 (m, 14H), 1.61-1.17 (m, 8H), 1.18-0.93 (m, 4H).

### Example 17: N-(2-((1S,4S)-4-((((3-(4-chloro-3-(2,4-dioxotetrahydropyrimidin-1(2H)-yl)benzoyl)-3-azaspiro[5.5]undecan-9-yl)methyl)(methyl)amino)methyl)-4-hydroxycyclohexyl)-6-methoxy-2H-indol-5-yl)-6-(trifluoromethyl)pyridinecarboxamide

### Step 1: preparation of tert-butyl (4-hydroxy-4-(methylamino)methyl)cyclohexyl)carbamate

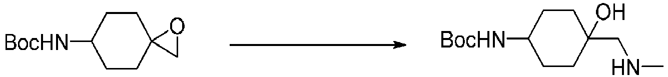

Methylamine (40 mL, 25%-30% aqueous solution) was added to a mixture of tert-butyl (1-oxaspiro[2.5]octan-6-yl)carbamate (4.2 g, 18.42 mmol) in EtOH/H₂O (90 mL/15 mL). Then the reaction liquid was stirred at 25 °C overnight. The reaction liquid was directly concentrated under reduced pressure, and the resulting crude product was purified by flash column chromatography (MeOH:DCM = 0-1:1) to give *tert-*butyl (4-hydroxy-4-(methylamino)methyl)cyclohexyl)carbamate.

LC-MS: (ESI, *m*/*z):* [M+H]^{|} = 259.2.

### Step 2: preparation of benzyl ((4-((tert-butoxycarbonyl)amino)-1-hydroxycyclohexyl)methyl)(methyl)carbamate

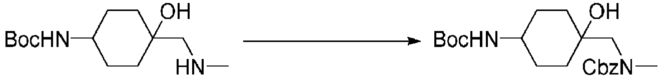

A mixture of tert-butyl (4-hydroxy-4-(methylamino)methyl)cyclohexyl)carbamate (1.2 g, 4.61 mmol) and benzyl chloroformate (941 mg, 5.53 mmol) in ACN/sat. NaHCO₃ (20 mL/20 mL) was stirred at room temperature overnight. The reaction liquid was diluted with water (50 mL) and then extracted with EA (50 mL × 3). The organic phase was washed with saturated brine (150 mL), dried over anhydrous Na₂SO₄, filtered, and concentrated under reduced pressure. The resulting crude product was purified by flash column chromatography (EA:PE = 0-7:3) to give benzyl ((4-((tert-butoxycarbonyl)amino)-1-hydroxycyclohexyl)methyl)(methyl)carbamate.

LC-MS: (ESI, *m*/*z*): [M+Na]⁺ = 415.2.

### Step 3: preparation of benzyl ((4-amino-1-hydroxycyclohexyl)methyl)(methyl)carbamate

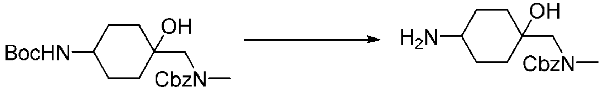

TFA (5 mL) was added to a solution of benzyl ((4-((tert-butoxycarbonyl)amino)-1-hydroxycyclohexyl)methyl)(methyl)carbamate (1.5 g, 3.81 mmol) in DCM (15 mL) at 0 °C while stirring, and then the reaction liquid was stirred at 0 °C for 2 h. The pH of the reaction liquid was adjusted to 9.0 with DIEA, and then the mixture was directly concentrated under reduced pressure to give benzyl ((4-amino-1-hydroxycyclohexyl)methyl)(methyl)carbamate. The resulting crude product was directly used in the next step.

LC-MS: (ESI, m/z): [M+H]⁺= 293.1.

### Step 4: preparation of benzyl (((1S,4S)-1-hydroxy-4-(6-methoxy-5-nitro-2H-indol-2-yl)cyclohexyl)methyl)(methyl)carbamate

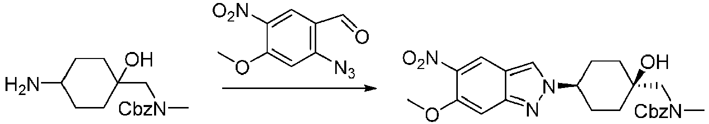

2-Azido-4-methoxy-5-nitrobenzaldehyde (1.2 g, 5.7 mmol) was added to a solution of benzyl ((4-amino-1-hydroxycyclohexyl)methyl)(methyl)carbamate (1.5 g, 3.8 mmol) in toluene (40 mL) while stirring. The reaction liquid was stirred at 100 °C for 3 h. The reaction liquid was directly concentrated under reduced pressure. The resulting crude product was purified by flash column chromatography (EA:PE = 0-1) to give benzyl (((1*S*,4*S*)-1-hydroxy-4-(6-methoxy-5-nitro-2*H*-indol-2-yl)cyclohexyl)methyl)(methyl)carbamate.

LC-MS: (ESI, m/z): [M+H]⁺= 469.2

¹H NMR (400 MHz, DMSO-*d*₆) δ 8.58 (s, 1H), 8.38 (s, 1H), 7.45-7.23 (m, 6H), 5.09 (s, 2H), 4.66-4.36 (m, 2H), 3.91 (s, 3H), 3.28 (s, 2H), 3.10-2.95 (m, 3H), 2.30-2.20 (m, 2H), 1.95-1.60 (m, 2H), 1.78-1.63 (m, 2H), 1.59-1.41 (m, 2H).

### Step 5: preparation of benzyl (((1S,4S)-4-(5-amino-6-methoxy-2H-indol-2-yl)-1-hydroxycyclohexylmethyl)(methyl)carbamate

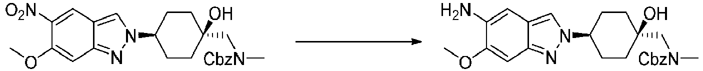

N₂H₄ H₂O (1.2 mL) and Raney-Ni (0.5 mL) were added to a solution of benzyl (((1*S*,4*S*)-1-hydroxy-4-(6-methoxy-5-nitro-2H-indol-2-yl)cyclohexyl)methyl)(methyl)carbamate (1.1 g, 2.34 mmol) in EtOH (20 mL). The reaction liquid was stirred at room temperature for 2 h. The reaction liquid was filtered through celite, and the filtrate was concentrated under reduced pressure to give benzyl (((1*S*,4*S*)-4-(5-amino-6-methoxy-2*H*-indol-2-yl)-1-hydroxycyclohexylmethyl)(methyl)carbamate. The resulting crude product was directly used in the next step.

LC-MS: (ESI, m/z): [M+H]⁺= 439.2

### Step 6: preparation of benzyl (((1S,4S)-1-hydroxy-4-(6-methoxy-5-(6-(trifluoromethyl)pyridineformyl)-2H-indol-2-yl)cyclohexyl)methyl)(methyl)carbamate

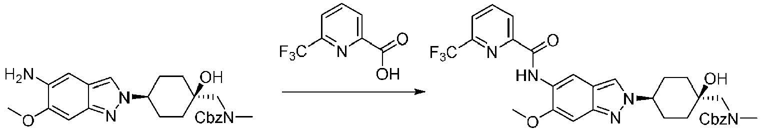

6-(Trifluoromethyl)picolinic acid (538 mg, 2.78 mmol), HATU (1.06 g, 2.78 mmol), and DIEA (828 mg, 6.42 mmol) were added to a solution of benzyl (((1*S*,4*S*)-4-(5-amino-6-methoxy-2*H*-indol-2-yl)-1-hydroxycyclohexylmethyl)(methyl)carbamate (940 mg, 2.14 mmol) in DMF (10 mL) while stirring. Then the reaction liquid was stirred at room temperature overnight. The reaction liquid was diluted with water (100 mL) and extracted with EA (30 mL × 3). The organic phase was washed with saturated brine (100 mL), dried over anhydrous Na₂SO₄, filtered, and concentrated under reduced pressure. The resulting crude product was purified by flash column chromatography (EA:PE = 0-9:1) to give benzyl (((1*S*,4*S*)-1-hydroxy-4-(6-methoxy-5-(6-(trifluoromethyl)pyridineformyl)-2*H*-indol-2-yl)cyclohexyl)methyl)(methyl)carbamate.

LC-MS: (ESI, *m*/*z*): [M+H]⁺= 612.2.

¹H NMR (400 MHz, DMSO-*d*₆) δ 10.50 (s, 1H), 8.69 (s, 1H), 8.46 (d, *J=* 7.5 Hz, 1H), 8.40 (t, *J=* 7.8 Hz, 1H), 8.31 (s, 1H), 8.21 (dd, *J=* 7.7, 1.0 Hz, 1H), 7.42-7.28 (m, 5H), 7.16 (s, 1H), 5.09 (s, 2H), 4.60-4.31 (m, 2H), 3.98 (s, 3H), 3.28 (s, 2H), 3.10-2.95 (m, 3H), 2.30-2.15 (m, 2H), 1.95-1.60 (m, 2H), 1.67 (t, *J=* 14.9 Hz, 2H), 1.57-1.40 (m, 2H).

### Step 7: preparation of N-(2-((1S,4S)-4-hydroxy-4-((methylamino)methyl)cyclohexyl)-6-methoxy-2H-indol-5-yl)-6-(trifluoromethyl)pyridinecarboxamide

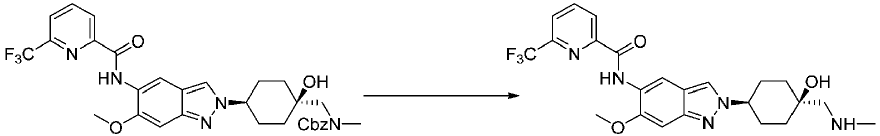

Pd(OH)₂/C (200 mg) was added to a solution of benzyl (((1S,4S)-1-hydroxy-4-(6-methoxy-5-(6-(trifluoromethyl)pyridineformyl)-2/7-indol-2-yl)cyclohexyl)methyl)(methyl)carbamate (500 mg, 0.81 mmol) in EA (20 mL). Then the reaction liquid was stirred at 70 °C overnight under H₂ atmosphere. The reaction liquid was filtered through celite, and the filtrate was concentrated under reduced pressure to give N-(2-((1*S*,4*S*)-4-hydroxy-4-((methylamino)methyl)cyclohexyl)-6-methoxy-2*H*-indol-5-yl)-6-(trifluoromethyl)pyridinecarboxamide. The resulting crude product was directly used in the next step.

LC-MS: (ESI, *m*/*z):* [M+H]^{|} = 478.1.

### Step 8: preparation of tert-butyl 9-(((((1S,4S)-1-hydroxy-4-(6-methoxy-5-(6-(trifluoromethyl)pyridinecarboxamido)-2H-indol-2-yl)cyclohexyl)methyl)(methyl)amino)methyl)-3-azaspiro[5.5]undecane-3-carboxylate

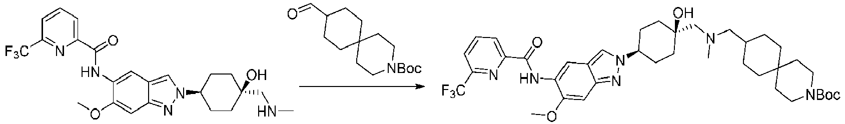

A mixture of N-(2-((1*S*,4*S*)-4-hydroxy-4-((methylamino)methyl)cyclohexyl)-6-methoxy-2*H*-indol-5-yl)-6-(trifluoromethyl)pyridinecarboxamide (200 mg, 0.41 mmol), tert-butyl 9-formyl-3-azaspiro[5.5]undecane-3-carboxylate (141 mg, 0.50 mmol), and STAB (434 mg, 2.05 mmol) in THF (10 mL) was stirred at room temperature overnight. The reaction liquid was diluted with water (30 mL), and then the mixture was extracted with DCM (10 mL × 3). The organic phase was washed with saturated brine (30 mL), dried over anhydrous Na₂SO₄, filtered, and concentrated under reduced pressure. The resulting crude product was purified by flash column chromatography (MeOH:DCM = 0-1:9) to give tert-butyl 9-(((((1*S*,4*S*)-1-hydroxy-4-(6-methoxy-5-(6-(trifluoromethyl)pyridinecarboxamido)-2*H*-indol-2-yl)cyclohexyl)methyl)(methyl)amino)methyl)-3-azaspiro[5.5]undecane-3-carboxylate.

LC-MS: (ESI, *m*/*z):* [M+H]⁺ = 743.3.

### Step 9: preparation of N-(2-((1S,4S)-4-((((3-azaspiro[5.5]undecan-9-yl)methyl)(methyl)amino)methyl)-4-hydroxycyclohexyl)-6-methoxy-2H-indol-5-yl)-6-(trifluoromethyl)pyridinecarboxamide

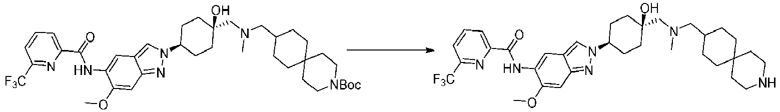

TFA (2 mL) was added to a solution of tert-butyl 9-(((((1S,4S)-1-hydroxy-4-(6-methoxy-5-(6-(trifluoromethyl)pyridinecarboxamido)-2*H*-indol-2-yl)cyclohexyl)methyl)(methyl)amino)methyl)-3-azaspiro[5.5]undecane-3-carboxylate (200 mg, 0.27 mmol) in DCM (5 mL) at 0 °C. Then the reaction liquid was stirred at room temperature for 2 h. The pH of the reaction liquid was adjusted to 9.0 with DIE. The organic phase was washed with water (100 mL), dried over anhydrous Na₂SO₄, filtered, and concentrated under reduced pressure to give *N*-(2-((1*S*,4*S*)-4-((((3-azaspiro[5.5]undecan-9-yl)methyl)(methyl)amino)methyl)-4-hydroxycyclohexyl)-6-methoxy-2*H*-indol-5-yl)-6-(trifluoromethyl)pyridinecarboxamide. The resulting crude product was directly used in the next step.

LC-MS: (ESI, *m*/*z*): [M+H]⁺= 643.3.

### Step 10: preparation of N-(2-((1S,4S)-4-((((3-(4-chloro-3-(2,4-dioxotetrahydropyrimidin-1(2H)-yl)benzoyl)-3-azaspiro[5.5]undecan-9-yl)methyl)(methyl)amino)methyl)-4-hydroxycyclohexyl)-6-methoxy-2/7-indol-5-yl)-6-(trifluoromethyl)pyridinecarboxamide

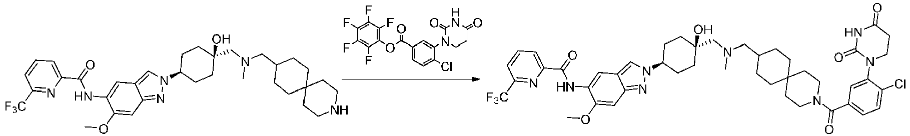

A mixture of *N*-(2-((1*S*,4*S*)-4-((((3-azaspiro[5.5]undecan-9-yl)methyl)(methyl)amino)methyl)-4-hydroxycyclohexyl)-6-methoxy-2*H*-indol-5-yl)-6-(trifluoromethyl)pyridinecarboxamide (130 mg, 0.20 mmol), pentafluorophenyl 4-chloro-3-(2,4-dioxotetrahydropyrimidin-1(2*H*)-yl)benzoate (105 mg, 0.24 mmol), and DIEA (77 mg, 0.60 mmol) in DMSO (5 mL) was stirred at room temperature overnight. The reaction liquid was diluted with water (50 mL), and then the mixture was extracted with DCM (20 mL × 3). The organic phase was washed with saturated brine (60 mL), dried over anhydrous Na₂SO₄, filtered, and concentrated under reduced pressure. The resulting crude product was purified by Prep-HPLC (CH₃CN/0.08% aqueous NH₄HCO₃ solution, 5%-95%) to give A-(2-((15,45)-4-((((3-(4-chloro-3-(2,4-dioxotetrahydropyrimidin-1(2*H*)-yl)benzoyl)-3-azaspiro[5.5]undecan-9-yl)methyl)(methyl)amino)methyl)-4-hydroxycyclohexyl)-6-methoxy-2*H*-indol-5-yl)-6-(trifluoromethyl)pyridinecarboxamide.

LC-MS: (ESI, *m*/*z):* [M+H]⁺= 893.1.

¹H NMR (400 MHz, DMSO-*d*₆) δ 10.51 (s, 2H), 8.69 (s, 1H), 8.47 (d, *J=* 7.8 Hz, 1H), 8.41 (t, *J=* 7.8 Hz, 1H), 8.32 (s, 1H), 8.22 (d, *J=* 7.8 Hz, 1H), 7.63 (d, *J=* 8.2 Hz, 1H), 7.55 (d, *J=* 1.7 Hz, 1H), 7.39 (d, *J=* 8.3 Hz, 1H), 7.15 (s, 1H), 4.37-4.27 (m, 1H), 4.10-3.90 (m, 4H), 3.82-3.49 (m, 4H), 3.35-3.25 (m, 1H), 2.80-2.70 (m, 2H), 2.36-2.15 (m, 9H), 1.95-1.85 (m, 2H), 1.78-1.22 (m, 14H), 1.18-0.98 (m, 4H).

### Example 18: 5-((1R,4R)-2-oxa-5-azabicyclo[2.2.1]heptan-5-yl)-N-(1-(1-(2-((1-(4-chloro-3-(2,4-dioxotetrahydropyrimidin-1(2H)-yl)benzoyl)piperidin-4-yl)methoxy)ethyl)piperidin-4-yl)-3-(difluoromethyl)-1H-pyrazol-4-yl)pyrazolo[1,5-a]pyrimidine-3-carboxamide

### Step 1: preparation of tert-butyl 4-((2-hydroxyethoxy)methyl)piperidine-1-carboxylate

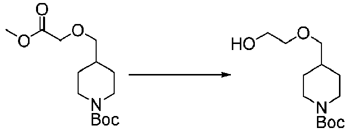

LiAlH₄ (1.0 mol/L, 5.8 mL) was added dropwise to a solution of methyl 4-((2-methoxy-2-oxoethoxy)methyl)piperidine-1-carboxylate (1.1 g, 3.83 mmol) in anhydrous THF (30 mL) at 0 °C under N₂ atmosphere, and then the reaction liquid was stirred at room temperature for 3 h. A 15% NaOH solution was slowly added dropwise to the reaction liquid while stirring. The mixture was filtered, and the filtrate was extracted with EA (30 mL × 2). The organic phase was washed with saturated brine, dried over anhydrous Na₂SO₄, filtered, and concentrated under reduced pressure. The resulting crude product was purified by column chromatography (MeOH:DCM = 0-3:97) to give tert-butyl 4-((2-hydroxyethoxy)methyl)piperidine-1-carboxylate.

LC-MS: (ESI, *m*/*z):* [M+H]^{|} = 260.1.

### Step 2: preparation of tert-butyl 4-((2-oxoethoxy)methyl)piperidine-1-carboxylate

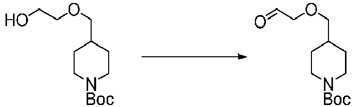

A mixture of tert-butyl 4-((2-hydroxyethoxy)methyl)piperidine-1-carboxylate (700 mg, 2.7 mmol) and IBX (1.5 g, 5.4 mmol) in acetonitrile (15 mL) was stirred at 80 °C for 2 h. The mixture was filtered, diluted with water (30 mL), and extracted with EA (30 mL × 2). The organic phase was washed with saturated brine, dried over anhydrous Na₂SO₄, filtered, and concentrated under reduced pressure. The resulting crude product was purified by column chromatography (EA:PE = 0-2:3) to give *tert-butyl* 4-((2-oxoethoxy)methyl)piperidine-1-carboxylate.

LC-MS: (ESI, *m*/*z*): [M-t-Bu]⁺ = 202.1.

### Step 3: preparation of tert-butyl 4-((2-(4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)ethoxy)methyl)piperidine-1 -carboxylate

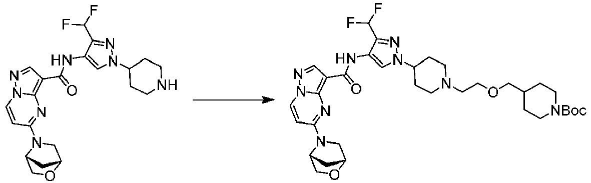

STAB (277.8 mg, 1.44 mmol) was added to a solution of 5-((1*R*,4*R*)-2-oxa-5-azabicyclo[2.2.1]heptan-5-yl)-*N*-(3-(-(piperidin-4-yl)-1*H*-pyrazol-4-yl)pyrazolo[1,5-*a*]pyrimidine-3-carboxamide (220.0 mg, 0.48 mmol) and tert-butyl 4-((2-oxoethoxy)methyl)piperidine-1-carboxylate (185.2 mg, 0.72 mmol) in THF (5 mL). Then the reaction liquid was stirred at room temperature for 4 h. Water (20 mL) was added to quench the reaction, and then the mixture was extracted with DCM (20 mL × 2). The organic phase was washed with saturated brine, dried over anhydrous Na₂SO₄, filtered, and concentrated under reduced pressure. The resulting crude product was purified by column chromatography (MeOH:DCM = 0-1:9) to give *tert-butyl* 4-((2-(4-(4-(5-((1R,4R)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)ethoxy)methyl)piperidine-1 -carboxylate.

LC-MS: (ESI, *m*/*z):* [M+H]⁺= 700.2.

### Step 4: preparation of 5-((1R,4R)-2-oxa-5-azabicyclo[2.2.1]heptan-5-yl)-N-(3-(difluoromethyl)-1-(1-(2-(piperidin-4-ylmethoxy)ethyl)piperidin-4-yl)-1H-pyrazol-4-yl)pyrazolo[1,5-a]pyrimidine-3-carboxamide

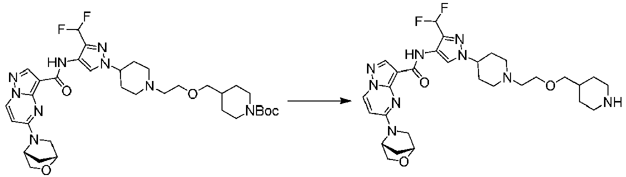

TFA (1.0 mL) was added to a solution of tert-butyl 4-((2-(4-(4-(5-((1R,4R)-2-oxa-5-azabicyclo[2.2.1]heptan-5-yl)pyrazolo[1,5-*a*]pyrimidine-3-carboxamido)-3-(difluoromethyl)-1*H-*pyrazol-1-yl)piperidin-1-yl)ethoxy)methyl)piperidine-1-carboxylate (150.0 mg, 0.214 mmol) in DCM (3.0 mL), and the reaction liquid was stirred at room temperature for 2 h. The reaction liquid was directly concentrated under reduced pressure to give 5-((1*R*,4*R*)-2-oxa-5-azabicyclo[2.2.1]heptan-5-yl)-*N*-(3-(difluoromethyl)-1-(1-(2-(piperidin-4-ylmethoxy)ethyl)piperidin-4-yl)-1*H*-pyrazol-4-yl)pyrazolo[1,5-*a*]pyrimidine-3-carboxamide.

LC-MS: (ESI, m/z): [M+H]⁺= 600.4.

### Step 5: preparation of 5-((1R,4R)-2-oxa-5-azabicyclo[2.2.1]heptan-5-yl)-N-(1-(1-(2-((1-(4-chloro-3-(2,4-dioxotetrahydropyrimidin-1(2H)-yl)benzoyl)piperidin-4-yl)methoxy)ethyl)piperidin-4-yl)-3-(difluoromethyl)-1H-pyrazol-4-yl)pyrazolo[1,5-a]pyrimidine-3-carboxamide

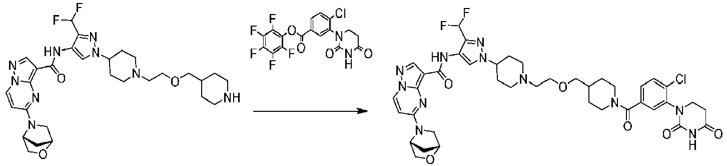

DIEA (77.5 mg, 0.6 mmol) and pentafluorophenyl 4-chloro-3-(2,4-dioxotetrahydropyrimidin-1(2*H*)-yl)benzoate (95.6 mg, 0.22 mmol) were added to a solution of 5-((1*R*,4*R*)-2-oxa-5-azabicyclo[2.2.1]heptan-5-yl)-*N*-(3-(difluoromethyl)-1-(1-(2-(piperidin-4-ylmethoxy)ethyl)piperidin-4-yl)-1*H*-pyrazol-4-yl)pyrazolo[1,5-*a*]pyrimidine-3-carboxamide (120.0 mg, 0.2 mmol) in DMSO (5.0 mL), and then the reaction liquid was stirred at room temperature for 2 h. Water (15 mL) was added to quench the reaction, and then the mixture was extracted with DCM (30 mL × 2). The organic phase was washed with saturated brine, dried over anhydrous Na₂SO₄, filtered, and concentrated under reduced pressure. The resulting crude product was purified by Prep-HPLC (acetonitrile/0.05% aqueous NH₄HCO₃ solution, 5%-95%) to give 5-((1*R*,4*R*)-2-oxa-5-azabicyclo[2.2.1]heptan-5-yl)-*N-*(1-(1-(2-((1-(4-chloro-3-(2,4-dioxotetrahydropyrimidin-1(2*H*)-yl)benzoyl)piperidin-4-yl)methoxy)ethyl)piperidin-4-yl)-3-(difluoromethyl)-1*H*-pyrazol-4-yl)pyrazolo[1,5-*a*]pyrimidine-3-carboxamide.

LC-MS: (ESI, *m*/*z*): [M+H]⁺= 850.3.

¹H NMR (400 MHz, DMSO-*d*₆) δ 10.50 (s, 1H), 9.50 (d, *J* = 5.6 Hz, 1H), 8.79 (d, *J* = 7.7 Hz, 1H), 8.39 (d, *J* = 3.8 Hz, 1H), 8.26 (d, *J* = 5.6 Hz, 1H), 7.63 (d, *J* = 8.2 Hz, 1H), 7.55 (d, *J* = 2.0 Hz, 1H), 7.38 (dd, *J* = 8.2, 2.0 Hz, 1H), 7.25-6.95 (m, 1H), 6.91-6.40 (m, 1H), 5.30-5.05 (m, 1H), 4.77 (d, J = 17.6 Hz, 1H), 4.55-4.35 (m, 1H), 4.28-4.10 (m, 1H), 3.83-3.75 (m, 3H), 3.66-3.57 (m, 3H), 3.53-3.42 (m, 3H), 3.30-3.26 (m, 2H), 3.06-2.96 (m, 3H), 2.77-2.66 (m, 3H), 2.34-2.20 (m, 3H), 2.06-1.85 (m, 10H), 1.14-1.08(m, 2H).

### Example 19: N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)-5-(piperazin-1-yl)pyrazolo[1,5-a]pyrimidine-3-carboxamide

### Step 1: preparation of ethyl 5-(4-((benzyloxy)carbonyl)piperazin-1-yl)pyrazolo[1,5-a]pyrimidine-3-carboxylate

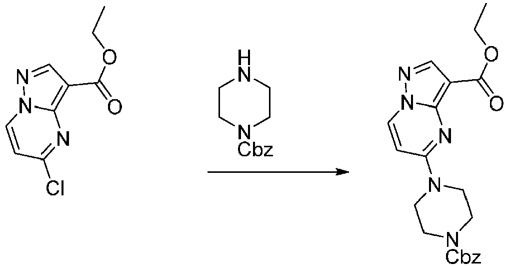

Ethyl 5-chloropyrazolo[1,5-a]pyrimidine-3-carboxylate (3.0 g, 13.3 mmol) and benzyl piperazine-1-carboxylate (2.9 g, 13.3 mmol) were dissolved in a solution of ACN (30 mL). DIEA (5.2 g, 40 mmol) was added, and the reaction liquid was heated to 60 °C, stirred for 2 h, and concentrated under reduced pressure. The crude product was dissolved in ethyl acetate (100 mL), washed with saturated brine, and concentrated under reduced pressure. The crude product was purified by column chromatography (PE:EA = 2:1) to give ethyl 5-(4-((benzyloxy)carbonyl)piperazin-1-yl)pyrazolo[1,5-*a*]pyrimidine-3-carboxylate.

LC-MS: (ESI, *m*/*z):* [M+H]⁺ = 410.2.

¹H NMR (400 MHz, DMSO-*d*₆) δ 8.76 (d, J = 7.9 Hz, 1H), 8.22 (s, 1H), 7.41-7.33 (m, 5H), 6.86 (d, J = 7.9 Hz, 1H), 5.13 (s, 2H), 4.19 (q, J = 7.1 Hz, 2H), 3.88-3.74 (m, 4H), 3.62-3.49 (m, 4H), 1.28 (t, J = 7.1 Hz, 3H).

### Step 2: preparation of 5-(4-((benzyloxy)carbonyl)piperazin-1-yl)pyrazolo[1,5-a]pyrimidine-3-carboxylic acid

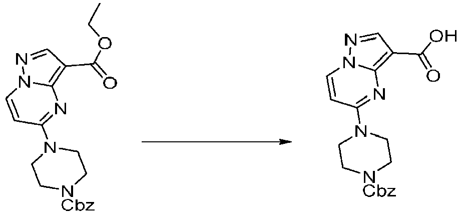

Ethyl 5-(4-((benzyloxy)carbonyl)piperazin-1-yl)pyrazolo[1,5-a]pyrimidine-3-carboxylate (2.6 g, 6.3 mmol) was dissolved in methanol (26 mL), lithium hydroxide (1.0 g, 25.2 mmol) was added, and the reaction liquid was stirred at 60 °C for 2 h. After the reaction was complete, dilute hydrochloric acid (2 mol/L) was added to the reaction liquid to adjust the pH of the reaction liquid to 2. Then the mixture was filtered, and the resulting filter cake was washed with water and dried under vacuum to give 5-(4-((benzyloxy)carbonyl)piperazin-1-yl)pyrazolo[1,5-*a*]pyrimidine-3-carboxylic acid.

LC-MS: (ESI, *m*/*z*): [M+H]⁺ = 382.2.

### Step 3: preparation of benzyl 4-(3-((1-(1-(tert-butoxycarbonyl)piperidin-4-yl)-3-(difluoromethyl)-1H-pyrazol-4-yl)carbamoyl)pyrazolo[1,5-a]pyrimidin-5-yl)piperazine-1-carboxylate

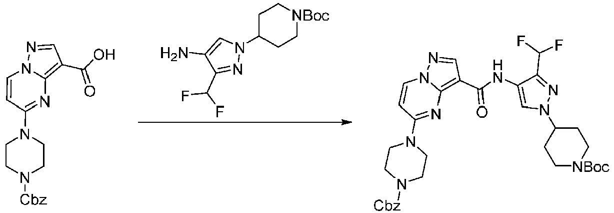

HATU (380 mg, 0.50 mmol) was added to a solution of 5-(4-((benzyloxy)carbonyl)piperazin-1-yl)pyrazolo[1,5-*a*]pyrimidine-3-carboxylic acid (270 mg, 0.4 mmol) and DIEA (0.5 mL, 1.0 mmol) in DMF (5 mL) at 25 °C. After the mixture was stirred at room temperature for 2 h, tert-butyl 4-(4-amino-3-(difluoromethyl)-1*H*-pyrazol-1-yl)piperidine-1-carboxylate (0.21 g, 0.3 mmol) was added, and the reaction liquid was stirred at 25 °C overnight. Water (30 mL) was added to quench the reaction, and the mixture was extracted with EA (50 mL × 3). The organic phase was washed with saturated brine, dried over anhydrous Na₂SO₄, filtered, and concentrated under reduced pressure. The resulting crude product was purified by column chromatography (MeOH:DCM = 0-3:97) to give benzyl 4-(3-((1-(1-(tert-butoxycarbonyl)piperidin-4-yl)-3-(difluoromethyl)-1*H*-pyrazol-4-yl)carbamoyl)pyrazolo[1,5-*a*]pyrimidin-5-yl)piperazine-1-carboxylate.

LC-MS: (ESI, m/z): [M+H]⁺ = 680.4.

### Step 4: preparation of benzyl 4-(3-((3-(difluoromethyl)-1-(piperidin-4-yl)-1H-pyrazol-4-yl)carbonyl)pyrazolo[1,5-a]pyrimidin-5-yl)piperazine-1-carboxylate

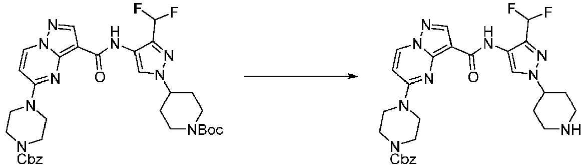

TFA (1 mL) was added to a solution of benzyl 4-(3-((1-(1-(tert-butoxycarbonyl)piperidin-4-yl)-3-(difluoromethyl)-1Hpyrazol-4-yl)carbamoyl)pyrazolo[1,5-a]pyrimidin-5-yl)piperazine-1-carboaylate (0.29 g, 0.42 mmol) in DCM (5 mL), and then the reaction liquid was stirred at room temperature for 2 h. The reaction liquid was directly concentrated under reduced pressure to give benzyl 4-(3-((3-(difluoromethyl)-1-(piperidin-4-yl)-1*H*-pyrazol-4-yl)carbonyl)pyrazolo[1,5-*a*]pyrimidin-5-yl)piperazine-1-carboxylate.

LC-MS: (ESI, *m*/*z*): [M+H]⁺ = 580.5.

### Step 5: preparation of tert-butyl 9-((4-(4-(5-(4-((benzyloxy)carbonyl)piperazin-1-yl)pyrazolo[1,5-a]pyrimidine-3-carboxamido)-3-(difluoromethyl)-1H-pyrazol-1-yl)piperidin-1-yl)methyl)-3-azaspiro [5.5]undecane-3-carboxylate

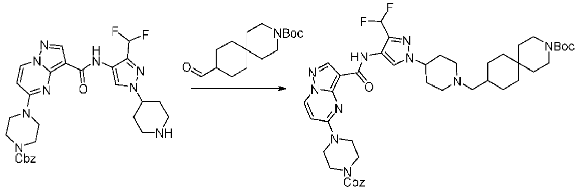

tert-Butyl 9-formyl-3-azaspiro[5.5]undecane-3-carboxylate (144 mg, 0.45 mmol) and NaBH₃CN (138 mg, 1.2 mmol) were added to a solution of benzyl 4-(3-((3-(difluoromethyl)-1-(piperidin-4-yl)-1H-pyrazol-4-yl)carbonyl)pyrazolo[1,5-*a*]pyrimidin-5-yl)piperazine-1-carboxylate (230 mg, 0.4 mmol) and DIEA (0.35 mL, 2.0 mmol) in MeOH (10 mL). The reaction liquid was stirred at 25 °C overnight. Water (30 mL) was added to quench the reaction, and then the mixture was extracted with EA (50 mL × 3). The organic phase was washed with saturated brine, dried over anhydrous Na₂SO₄, filtered, and concentrated under reduced pressure. The resulting crude product was purified by flash column chromatography (MeOH:DCM = 0-3:97) to give *tert*-butyl 9-((4-(4-(5-(4-((benzyloxy)carbonyl)piperazin-1-yl)pyrazolo[1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate.

LC-MS: (ESI, *m*/*z):* [M+H]⁺ = 845.5.

### Step 6: preparation of benzyl 4-(3-((1-(1-((3-azaspiro[5.5]undecan-9-yl)methyl)piperidin-4-yl)-3-(difluoromethyl)-1H-pyrazol-4-yl)carbonyl)pyrazolo[1,5-a]pyrimidin-5-yl)piperazine-1-carboxylate

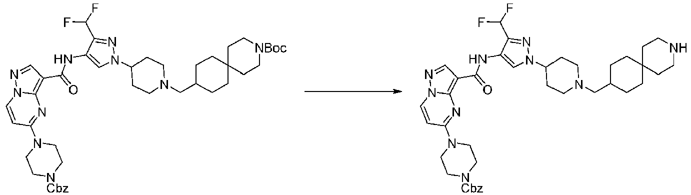

TFA (1 mL) was added to a solution of tert-butyl 9-((4-(4-(5-(4-((benzyloxy)carbonyl)piperazin-1-yl)pyrazolo[1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate (0.29 g, 0.34 mmol) in DCM (3 mL), and the mixture was stirred at 25 °C for 1 h. The reaction liquid was directly concentrated under reduced pressure to give benzyl 4-(3-((1-(1-((3-azaspiro[5.5]undecan-9-yl)methyl)piperidin-4-yl)-3-(difluoromethyl)-1*H*-pyrazol-4-yl)carbonyl)pyrazolo[1,5-*a*]pyrimidin-5-yl)piperazine-1-carboxylate.

LC-MS: (ESI, *m*/*z*): [M+H]⁺ = 745.4.

### Step 7: preparation of benzyl 4-(3-((1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)carbonyl)pyrazolo[1,5-a]pyrimidin-5-yl)piperazine-1-carboxylate

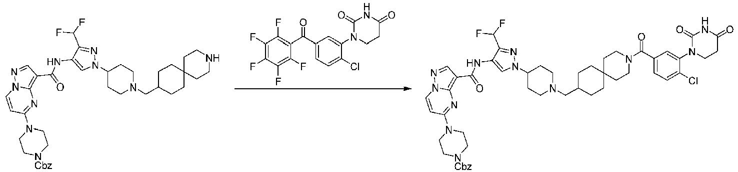

DIEA (0.1 mL, 0.6 mmol) was added to a solution of benzyl 4-(3-((1-(1-((3-azaspiro[5.5]undecan-9-yl)methyl)piperidin-4-yl)-3-(difluoromethyl)-1*H*-pyrazol-4-yl)carbonyl)pyrazolo[1,5-*a*]pyrimidin-5-yl)piperazine-1-carboxylate (240 mg, 0.32 mmol) and pentafluorophenyl 4-chloro-3-(2,4-dioxotetrahydropyrimidin-1(2*H*)-yl)benzoate (144 mg, 0.33 mmol) in DMSO (5 mL). The reaction liquid was then stirred at 25 °C for 16 h. Water (30 mL) was added to the reaction liquid, and the mixture was extracted with EA (50 mL × 3). The organic phase was washed with saturated brine, dried over anhydrous sodium sulfate, filtered, and concentrated under reduced pressure. The resulting crude product was purified by flash column chromatography (MeOH:DCM = 0-3:97) to give benzyl 4-(3-((1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)carbonyl)pyrazolo[1,5-*a*]pyrimidin-5-yl)piperazine-1-carboxylate.

LC-MS: (ESI, m/z): [M+H]⁺ = 995.0.

### Step 8: preparation of N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H pyrazol-4-yl)-5-(piperazin-1-yl)pyrazolo[1,5-a]pyrimidine-3-carboxamide

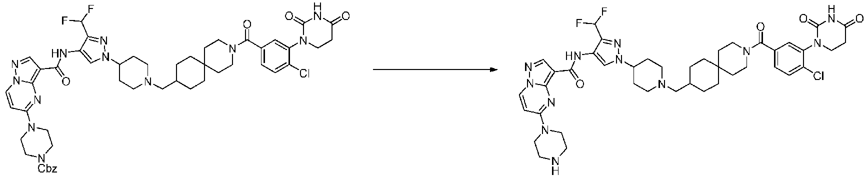

Iodotrimethylsilane (0.85 mL, 0.6 mmol) was added to a solution of benzyl 4-(3-((1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)carbonyl)pyrazolo[1,5-*a*]pyrimidin-5-yl)piperazine-1-carboxylate (290 mg, 0.29 mmol) in DCM (15 mL) at 0 °C, and then the reaction liquid was stirred at 25 °C for 16 h. The reaction liquid was poured into water, extracted with DCM (20 mL × 3), dried over anhydrous Na₂SO₄, filtered, and concentrated under reduced pressure. The resulting crude product was purified by Prep-HPLC to give *N-*(1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)-5-(piperazin-1-yl)pyrazolo[1,5-*a*]pyrimidine-3-carboxamide.

LC-MS: (ESI, *m*/*z*): [M+H]⁺ = 861.1.

¹H NMR (400 MHz, MeOD) δ 8.62 (d, J = 7.9 Hz, 1H), 8.43 (brs, 2.67H, FA), 8.35 (s, 1H), 7.64 (d, J = 8.3 Hz, 1H), 7.54 (d, J = 1.6 Hz, 1H), 7.43 (d, J = 8.2 Hz, 1H), 7.08-6.77 (m, 2H), 4.57-4.43 (m, 1H), 4.15-4.03 (m, 4H), 3.79 (t, J = 6.4 Hz, 2H), 3.75-3.65 (m, 2H), 3.63-3.48 (m, 2H), 3.47-3.37 (m, 2H), 3.37-3.30 (m, 2H), 3.05-2.95 (m, 2H), 2.95-2.77 (m, 4H), 2.43-2.27 (m, 4H), 1.92-1.75 (m, 3H), 1.74-1.65 (m, 3H), 1.65-1.45 (m, 2H), 1.39-1.12 (m, 5H).

### Example 20: 5-((1R,4R)-2-oxa-5-azabicyclo[2.2.1]heptan-5-yl)-N-(1-(1-(2-(((1-(4-chloro-3-(2,4-dioxotetrahydropyrimidin-1(2H)-yl)benzoyl)piperidin-4-yl)methyl)(methyl)amino)ethyl)piperidin-4-yl)-3-(difluoromethyl)-1H-pyrazol-4-yl)pyrazolo[1,5-a]pyrimidine-3-carboxamide

### Step 1: preparation of (9H-fluoren-9-yl)methyl (2-(4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)ethyl)(methyl)carbamate

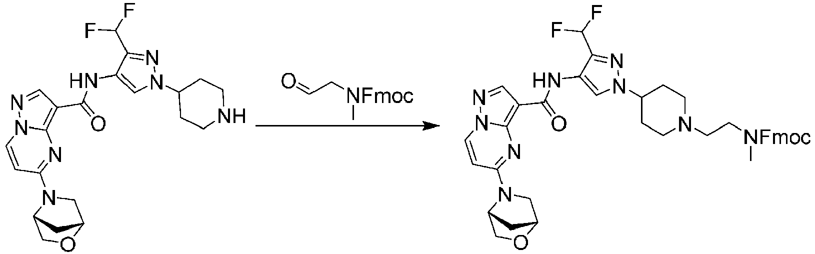

(9*H*-Fluoren-9-yl)methyl methyl(2-oxoethyl)carboxylate (91 mg, 0.31 mmol) and STAB (165 mg, 0.78 mmol) were added to a solution of 5-((1*R*,4*R*)-2-oxa-5-azabicyclo[2.2.1]heptan-5-yl)-*N*-(3-(difluoromethyl)-1-(piperidin-4-yl)-1*H*-pyrazol-4-yl)pyrazolo[1,5-a]pyrimidine-3-carboxamide (120 mg, 0.26 mmol) in THF (10 mL). The reaction liquid was stirred at room temperature for 16 h. Water (50 mL) was added to quench the reaction, and the mixture was extracted with DCM (20 mL × 3). The organic phase was dried over anhydrous Na₂SO₄, filtered, and concentrated under reduced pressure. The resulting crude product was purified by flash column chromatography (MeOH:DCM = 0-1:9) to give (9H-fluoren-9-yl)methyl (2-(4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)ethyl)(methyl)carbamate.

LC-MS: (ESI, *m*/*z*): [M+H]¹ = 738.5.

### Step 2: preparation of 5-((1R,4R)-2-oxa-5-azabicyclo[2.2.1]heptan-5-yl)-N-(3-(difluoromethyl)-1-(1-(2-(methylamino)ethyl)piperidin-4-yl)-1H-pyrazol-4-yl)pyrazolo[1,5-a]pyrimidine-3-carboxamide

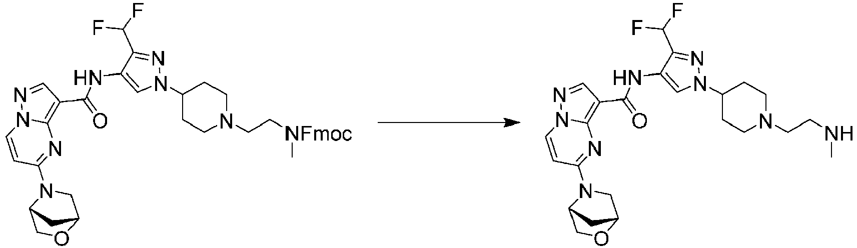

Pyridine (0.1 mL) was added to a solution of (9*H*-fluoren-9-yl)methyl (2-(4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)ethyl)(methyl)carbamate (120 mg, 0.16 mmol) in DCM (5 mL). The reaction liquid was stirred at room temperature for 16 h. The reaction liquid was directly concentrated under reduced pressure. The resulting crude product was purified by flash column chromatography (MeOH:DCM = 0-1:9) to give 5-((1*R*,4*R*)-2-oxa-5-azabicyclo[2.2.1]heptan-5-yl)-*N*-(3-(difluoromethyl)-1-(1-(2-(methylamino)ethyl)piperidin-4-yl)-1*H*-pyrazol-4-yl)pyrazolo[1,5-*a*]pyrimidine-3-carboxamide.

LC-MS: (ESI, *m*/*z*): [M+H]⁺ = 516.6.

### Step 3: preparation of tert-butyl 4-(((2-(4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)ethyl)(methyl)amino)methyl)piperidine-1-carboxylate

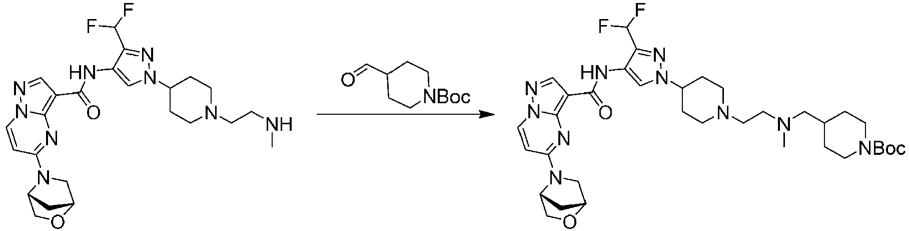

*tert*-Butyl 4-formylpiperidine-1-carboxylate (54 mg, 0.26 mmol) and STAB (108 mg, 0.51 mmol) were added to a solution of 5-((1*R*,4*R*)-2-oxa-5-azabicyclo[2.2.1]heptan-5-yl)-*N*-(3-(difluoromethyl)-1-(1-(2-(methylamino)ethyl)piperidin-4-yl)-1*H*-pyrazol-4-yl)pyrazolo[1,5-*a*]pyrimidine-3-carboxamide (90 mg, 0.17 mmol) in THF (10 mL). The reaction liquid was stirred at room temperature for 16 h. Water (50 mL) was added to quench the reaction, and the mixture was extracted with DCM (20 mL × 3). The organic phase was dried over anhydrous Na₂SO₄, filtered, and concentrated under reduced pressure. The resulting crude product was purified by flash column chromatography (MeOH:DCM = 0-1:4) to give tert-butyl 4-(((2-(4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)ethyl)(methyl)amino)methyl)piperidine-1-carboxylate.

LC-MS: (ESI, *m*/*z):* [M+H]⁺ = 713.4.

### Step 4: preparation of 5-((1R,4R)-2-oxa-5-azabicyclo[2.2.1]heptan-5-yl)-N-(3-(difluoromethyl)-1-(1-(2-(methyl(piperidin-4-ylmethyl)amino)ethyl)piperidin-4-yl)-1H-pyrazol-4-yl)pyrazolo[1,5-a]pyrimidine-3-carboxamide

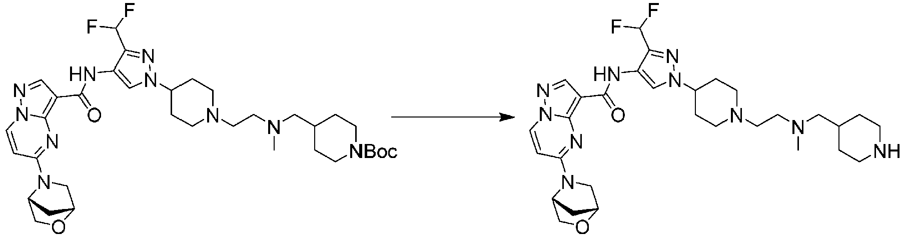

TFA (1 mL) was added to a solution of tert-butyl 4-(((2-(4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)ethyl)(methyl)amino)methyl)piperidine-1-carboxylate (100 mg, 0.14 mmol) in DCM (5 mL). The reaction liquid was stirred at room temperature for 2 h. The reaction liquid was directly concentrated under reduced pressure to give 5-((1*R*,4*R*)-2-oxa-5-azabicyclo[2.2.1]heptan-5-yl)-*N*-(3-(difluoromethyl)-1-(1-(2-(methyl(piperidin-4-ylmethyl)amino)ethyl)piperidin-4-yl)-1*H*-pyrazol-4-yl)pyrazolo[1,5-*a*]pyrimidine-3-carboxamide. The resulting product was directly used in the next step.

LC-MS: (ESI, *m*/*z*): [M+H]⁺ = 613.3.

### Step 5: preparation of 5-((1R,4R)-2-oxa-5-azabicyclo[2.2.1]heptan-5-yl)-N-(1-(1-(2-(((1-(4-chloro-3-(2,4-dioxotetrahydropyrimidin-1(2H)-yl)benzoyl)piperidin-4-yl)methyl)(methyl)amino)ethyl)piperidin-4-yl)-3-(difluoromethyl)-1H-pyrazol-4-yl)pyrazolo[1,5-a]pyrimidine-3-carboxamide

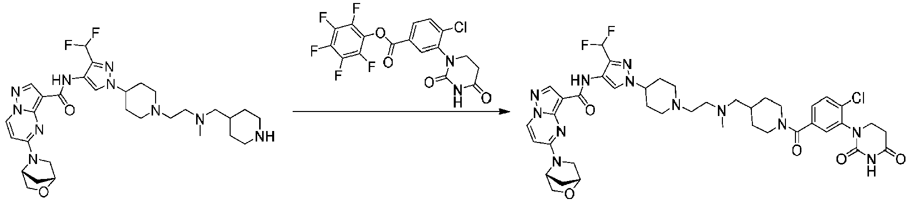

DIEA (52 mg, 0.4 mmol) was added to a solution of 5-((1*R*,4*R*)-2-oxa-5-azabicyclo[2.2.1]heptan-5-yl)-*N*-(3-(difluoromethyl)-1-(1-(2-(methyl(piperidin-4-ylmethyl)amino)ethyl)piperidin-4-yl)-1*H*-pyrazol-4-yl)pyrazolo[1,5-a]pyrimidine-3-carboxamide (80 mg, 0.13 mmol) and pentafluorophenyl 4-chloro-3-(2,4-dioxotetrahydropyrimidin-1(2*H*)-yl)benzoate (67 mg, 0.156 mmol) in DMSO (2 mL). Then the reaction liquid was stirred at room temperature for 2 h. The reaction liquid was poured into water and filtered, and the filter cake was purified by Prep-HPLC (CH₃CN/0.08% aqueous NH₄HCO₃ solution, 5%-95%) to give 5-((1*R*,4*R*)-2-oxa-5-azabicyclo[2.2.1]heptan-5-yl)-*N*-(1-(1-(2-(((1-(4-chloro-3-(2,4-dioxotetrahydropyrimidin-1(2*H*)-yl)benzoyl)piperidin-4-yl)methyl)(methyl)amino)ethyl)piperidin-4-yl)-3-(difluoromethyl)-1H-pyrazol-4-yl)pyrazolo[1,5-a]pyrimidine-3-carboxamide.

LC-MS: (ESI, m/z): [M+H]⁺ = 863.3.

¹H NMR (400 MHz, DMSO-*d*₆) δ 10.49 (s, 1H), 9.50 (d, *J* = 5.8 Hz, 1H), 8.78 (d, *J* = 7.7 Hz, 1H), 8.39 (d, *J* = 3.6 Hz, 1H), 8.25 (d, *J* = 5.6 Hz, 1H), 7.63 (d, *J* = 8.2 Hz, 1H), 7.54 (d, *J* = 1.9 Hz, 1H), 7.38 (dd, *J* = 8.2, 1.9 Hz, 1H), 7.25-6.95 (m, 1H), 6.92-6.43 (m, 1H), 5.40-5.06 (m, 1H), 4.77 (d, *J* = 17.3 Hz, 1H), 4.60-4.40 (m, 1H), 4.30-4.10 (m, 1H), 3.92-3.75 (m 3H), 3.68-3.56 (m, 3H), 3.55-3.45 (m, 1H), 3.10-2.90 (m, 3H), 2.84-2.68 (m, 3H), 2.48-2.40 (m, 4H), 2.24-1.55 (m, 16H),

### Example 21: 5-((1R,4R)-2-oxa-5-azabicyclo[2.2.1]heptan-5-yl)-N-(1-(1-(4-(1-(4-chloro-3-(2,4-dioxotetrahydropyrimidin-1(2H)-yl)benzoyl)piperidin-4-yl)butyl)piperidin-4-yl)-3-(difluoromethyl)-1H-pyrazol-4-yl)pyrazolo[1,5-a]pyrimidine-3-carboxamide

### Step 1: preparation of tert-butyl 4-(4-(4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)butyl)piperidine-1-carboxylate

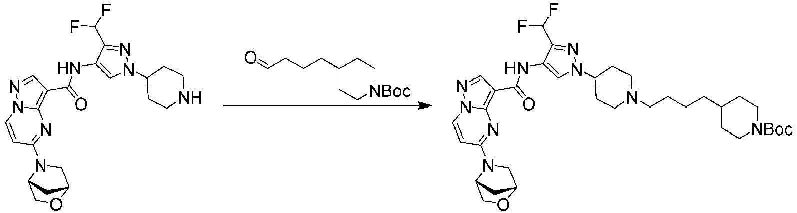

*tert*-Butyl 4-(4-oxobutyl)piperidine-1-carboxylate (100 mg, 0.36 mmol) and STAB (170 mg, 0.71 mmol) were added to a solution of 5-((1*R*,4*R*)-2-oxa-5-azabicyclo[2.2.1]heptan-5-yl)-*N*-(3-(difluoromethyl)-1-(piperidin-4-yl)-1*H*-pyrazol-4-yl)pyrazolo[1,5-*a*]pyrimidine-3-carboxamide (110 mg, 0.24 mmol) in THF (5 mL). The reaction liquid was stirred at 25 °C for 2 h. Water (30 mL) was added to quench the reaction, and the mixture was extracted with DCM (30 mL × 3). The organic phase was dried over anhydrous Na₂SO₄, filtered, and concentrated under reduced pressure. The resulting crude product was purified by flash column chromatography (MeOH:DCM = 0-3:97) to give tert-butyl 4-(4-(4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)butyl)piperidine-1-carboxylate.

LC-MS: (ESI, *m*/*z*): [M+H]⁺ = 698.0.

### Step 2: preparation of 5-((1R,4R)-2-oxa-5-azabicyclo[2.2.1]heptan-5-yl)-N-(3-(difluoromethyl)-1-(1-(4-(piperidin-4-yl)butyl)piperidin-4-yl)-1H-pyrazol-4-yl)pyrazolo[1,5-a]pyrimidine-3-carboxamide

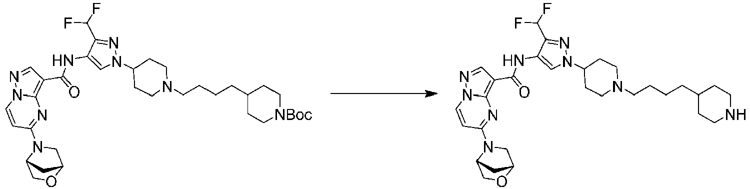

TFA (1 mL) was added to a solution of tert-butyl 4-(4-(4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-y)butyl)piperidine-1-carboxylate (0.2 g, 0.26 mmol) in DCM (5 mL). The reaction liquid was stirred at 25 °C for 2 h. The reaction liquid was directly concentrated under reduced pressure to give 5-((1*R*,4*R*)-2-oxa-5-azabicyclo[2.2.1]heptan-5-yl)-*N*-(3-(difluoromethyl)-1-(1-(4-(piperidin-4-yl)butyl)piperidin-4-yl)-1*H*-pyrazol-4-yl)pyrazolo[1,5-*a*]pyrimidine-3-carboxamide.

LC-MS: (ESI, *m*/*z*): [M+H]⁺ = 598.1.

### Step 3: preparation of 5-((1R,4R)-2-oxa-5-azabicyclo[2.2.1]heptan-5-yl)-N-(1-(1-(4-(1-(4-chloro-3-(2,4-dioxotetrahydropyrimidin-1(2H)-yl)benzoyl)piperidin-4-yl)butyl)piperidin-4-yl)-3-(difluoromethyl)-1H-pyrazol-4-yl)pyrazolo[1,5-a]pyrimidine-3-carboxamide

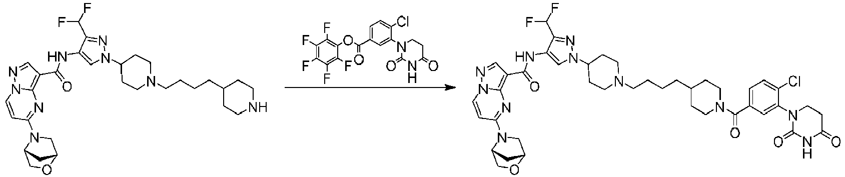

DIEA (0.14 mL, 0.78 mmol) was added to a solution of 5-((1R,4R)-2-oxa-5-azabicyclo[2.2.1]heptan-5-yl)-*N*-(3-(difluoromethyl)-1-(1-(4-(piperidin-4-yl)butyl)piperidin-4-yl)-1*H-*pyrazol-4-yl)pyrazolo[1,5-a]pyrimidine-3-carboxamide (160 mg, 0.26 mmol) and pentafluorophenyl 4-chloro-3-(2,4-dioxotetrahydropyrimidin-1(2H)-yl)benzoate (130 mg, 0.26 mmol) in DMSO (4 mL). The reaction liquid was stirred at 25 °C for 2 h. Water (10 mL) was added to quench the reaction, and the mixture was extracted with DCM (20 mL × 3), dried over anhydrous Na₂SO₄, filtered, and concentrated under reduced pressure. The resulting crude product was purified by Prep-HPLC to give 5-((1R,4R)-2-oxa-5-azabicyclo[2.2.1]heptan-5-yl)-*N*-(1-(1-(4-(1-(4-chloro-3-(2,4-dioxotetrahydropyrimidin-1(2*H*)-yl)benzoyl)piperidin-4-yl)butyl)piperidin-4-yl)-3-(difluoromethyl)-1*H*-pyrazol-4-yl)pyrazolo[1,5-*a*]pyrimidine-3-carboxamide.

LC-MS: (ESI, m/z): [M+H]⁺ = 848.3.

¹H NMR (400 MHz, DMSO-*d*₆) δ 10.49 (s, 1H), 9.50 (d, J = 5.3 Hz, 1H), 8.78 (d, J = 7.7 Hz, 1H), 8.40 (s, 1H), 8.25 (d, J = 5.0 Hz, 1H), 7.63 (d, J = 8.3 Hz, 1H), 7.54 (s, 1H), 7.38 (d, J = 8.1 Hz, 1H), 7.20-7.00 (m, 1H), 6.98-6.45 (m, 1H), 5.40-5.05 (m, 1H), 4.77 (d, J = 17.3 Hz, 1H), 4.54-4.34 (m, 1H), 4.28-4.12 (m, 1H), 3.83-3.72 (m, 3H), 3.67-3.57 (m, 3H), 3.45 (d, J = 9.6 Hz, 1H), 3.02-2.85 (m, 3H), 2.80-2.70 (m, 2H), 2.36-2.26 (m, 2H), 2.08-1.88 (m, 8H), 1.76-1.47 (m, 4H), 1.47-1.22 (m, 6H), 1.18-1.02 (m, 2H).

### Example 22: N-(2-((1S,4S)-4-((2-(1-(4-chloro-3-(2,4-dioxotetrahydropyrimidin-l(2H)-yl)benzoyl)piperidin-4-yl)ethoxy)methyl)-4-methoxycyclohexyl)-6-methoxy-2H-indol-5-yl)-6-(trifluoromethyl)pyridineamide

### Step 1: preparation of benzyl 4-(2-(((1S,4S)-4-(6-methoxy-5-nitro-2H-indazol-2-yl)-1-((methylthio)methoxy)cyclohexyl)methoxy)ethyl)piperidine-1-carboxylate

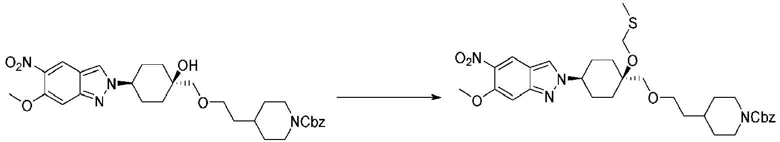

The compound benzyl 4-(2-(((1*S*,4*S*)-1-hydroxy-4-(6-methoxy-5-nitro-2H-indazol-2-yl)cyclohexyl)methoxy)ethyl)piperidine-1-carboxylate (700 mg, 1.24 mmol) and AczO (151 mg, 1.48 mmol) were dissolved in anhydrous DMSO (20 mL), and the mixture was stirred at room temperature for 16 h under nitrogen atmosphere. Water (50 mL) was added to the reaction liquid, and the mixture was extracted with ethyl acetate (50 mL × 3). The organic phase was washed with saturated brine (150 mL), dried over anhydrous sodium sulfate, filtered, and concentrated under reduced pressure. The crude product was purified by column chromatography (ethyl acetate:petroleum ether = 1:9) to give benzyl 4-(2-(((1*S*,4*S*)-4-(6-methoxy-5-nitro-2*H-*indazol-2-yl)-1-((methylthio)methoxy)cyclohexyl)methoxy)ethyl)piperidine-1-carboxylate.

LC-MS: (ESI, m/z): [M+H]⁺ = 627.2.

### Step 2: preparation of benzyl 4-(2-(((1S,4S)-4-(5-amino-6-methoxy-2H-indazol-2-yl)-1-methoxycyclohexyl)methoxy)ethyl)piperidine-1 -carboxylate

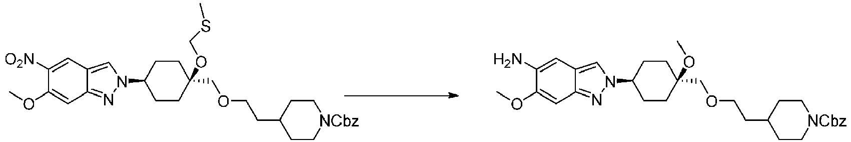

The compound benzyl 4-(2-(((1*S*,4*S*)-4-(6-methoxy-5-nitro-2*H*-indazol-2-yl)-1-((methylthio)methoxy)cyclohexyl)methoxy)ethyl)piperidine-1-carboxylate (600 mg, 0.96 mmol) and pretreated Raney-Ni (200 mg) (pretreatment method: washing with water 3 times, then washing with acetone 3 times, and finally washing with ethanol 3 times) were dissolved in anhydrous ethanol (50 mL), and the mixture was stirred at room temperature for 3 h under hydrogen atmosphere and filtered. The filtrate was directly concentrated under reduced pressure, and the crude product was purified by reversed-phase silica gel column chromatography (ACN:H₂O = 1:1, flow rate 40 mL/min) to give benzyl 4-(2-(((1*S*,4*S*)-4-(5-amino-6-methoxy-2*H*-indazol-2-yl)-1-methoxycyclohexyl)methoxy)ethyl)piperidine-1-carboxylate.

LC-MS: (ESI, m/z): [M+H]^{I} = 551.3.

### Step 3: preparation of benzyl 4-(2-(((1S,4S)-1-methoxy-4-(6-methoxy-5-(6-(trifluoromethyl)pyridinecarboxamido)-2H-indazol-2-yl)cycloheacyl)methoxy)ethyl)piperidine-1-carboxylate

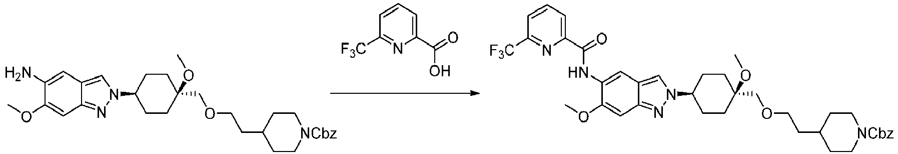

The compound 6-(trifluoromethyl)picolinic acid (60 mg, 0.3 mmol), HATU (114 mg, 0.3 mmol), and DIEA (100 mg, 0.78 mmol) were dissolved in DMF (10 mL), and the mixture was stirred at room temperature for 20 min. Benzyl 4-(2-(((1*S*,4*S*)-4-(5-amino-6-methoxy-2*H*-indazol-2-yl)-1-methoxycyclohexyl)methoxy)ethyl)piperidine-1-carboxylate (150 mg, 0.26 mmol) was added, and the mixture was reacted at room temperature for 1 h. Water (100 mL) was added to the reaction liquid, and the mixture was extracted with ethyl acetate (30 mL × 3). The organic phase was washed with saturated brine (100 mL), dried over anhydrous sodium sulfate, filtered, and concentrated under reduced pressure. The crude product was purified by column chromatography (ethyl acetate:petroleum ether = 1:1) to give benzyl 4-(2-(((1*S*,4*S*)-1-methoxy-4-(6-methoxy-5-(6-(trifluoromethyl)pyridinecarboxamido)-2*H*-indazol-2-yl)cyclohexyl)methoxy)ethyl)piperidine-1-carboxylate.

LC-MS: (ESI, m/z): [M+H]⁺ = 724.4.

### Step 4: preparation of N-(6-methoxy-2-((1S,4S)-4-methoxy-4-((2-(piperidin-4-yl)ethoxy)methyl)cyclohexyl)-2H-indazol-5-yl)-6-(trifluoromethyl)pyridinecarboxamide

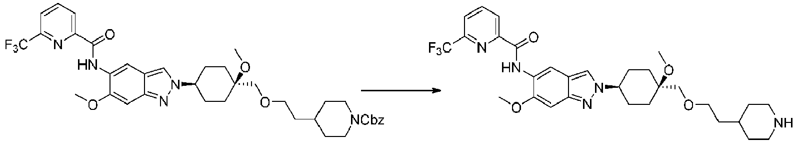

Benzyl 4-(2-(((1*S*,4*S*)-1-methoxy-4-(6-methoxy-5-(6-(trifluoromethyl)pyridinecarboxamido)-2*H-*indazol-2-yl)cyclohexyl)methoxy)ethyl)piperidine-1-carboxylate (70 mg, 0.097 mmol) was dissolved in trifluoroacetic acid (3 mL), and the mixture was heated to 90 °C and stirred for 1 h. The reaction liquid was directly concentrated under reduced pressure to give *N*-(6-methoxy-2-((1*S*,4*S*)-4-methoxy-4-((2-(piperidin-4-yl)ethoxy)methyl)cyclohexyl)-2*H*-indazol-5-yl)-6-(trifluoromethyl)pyridinecarboxamide, which was directly used in the next step without purification.

LC-MS: (ESI, m/z): [M+H]^{|} = 590.4.

### Step 5: preparation of N-(2-((1S,4S)-4-((2-(1-(4-chloro-3-(2,4-dioxotetrahydropyrimidin-1(2H)-yl)benzoyl)piperidin-4-yl)ethoxy)methyl)-4-methoxycyclohexyl)-6-methoxy-2H-indazol-5-yl)-6-(trifluoromethyl)pyridinecarboxamide

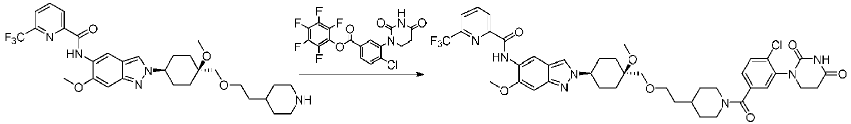

*N*-(6-Methoxy-2-((1*S*,4*S*)-4-methoxy-4-((2-(piperidin-4-yl)ethoxy)methyl)cyclohexyl)-2*H-*indazol-5-yl)-6-(trifluoromethyl)pyridinecarboxamide (70 mg, 0.097 mmol) was dissolved in dimethyl sulfoxide (3 mL). Pentafluorophenyl 4-chloro-3-(2,4-dioxotetrahydropyrimidin-1(2*H*)-yl)benzoate (48 mg, 0.11 mmol) and *N*,*N*-diisopropylethylamine (37 mg, 0.29 mmol) were added while stirring. After the mixture was stirred at room temperature for 2 h, water (50 mL) was added, and the mixture was extracted with dichloromethane (20 mL × 3). The organic phase was washed with saturated brine (3 × 60 mL), dried over anhydrous sodium sulfate, filtered, and concentrated under reduced pressure to give a crude product. The crude product was purified by pre-HPLC (CH₃CN/0.08% aqueous NH₄HCO₃ solution, 5%-95%) to give *N*-(2-((1*S*,4*S*)-4-((2-(1-(4-chloro-3-(2,4-dioxotetrahydropyrimidin-1(2*H*)-yl)benzoyl)piperidin-4-yl)ethoxy)methyl)-4-methoxycyclohexyl)-6-methoxy-2*H*-indazol-5-yl)-6-(trifluoromethyl)pyridinecarboxamide.

LC-MS: (ESI, m/z): [M+H]⁺ = 840.3.

¹H NMR (400 MHz, DMSO-*d*₆) δ 10.51 (d, *J* = 4.5 Hz, 2H), 8.68 (s, 1H), 8.49-8.37 (m, 2H), 8.32 (s, 1H), 8.22 (d, *J* = 7.6 Hz, 1H), 7.64 (d, *J* = 8.2 Hz, 1H), 7.55 (d, *J* = 1.9 Hz, 1H), 7.39 (dd, *J* = 8.2, 2.0 Hz, 1H), 7.18 (s, 1H), 4.52-4.30 (m, 2H), 3.98 (s, 3H), 3.80-3.52 (m, 3H), 3.47 (t, *J=* 6.2 Hz, 2H), 3.34 (s, 2H), 3.19 (s, 3H), 3.15-2.95 (m, 1H), 2.85-2.70 (m, 3H), 2.19-1.97 (m, 2H),-1.96-1.85 (m, 4H), 1.85-1.55 (m, 3H), 1.55-1.45 (m, 4H), 1.25-1.05 (m, 2H).

### Example 23: 5-((1R,4R)-2-oxa-5-azabicyclo[2.2.1]heptan-5-yl)-N-(1-(1-(3-((1-(4-chloro-3-(2,4-dioxotetrahydropyrimidin-1(2H)-yl)benzoyl)piperidin-4-yl)oxy)propyl)piperidin-4-yl)-3-(difluoromethyl)-1H-pyrazol-4-yl)pyrazolo[1,5-a]pyrimidine-3-carboxamide

### Step 1: preparation of benzyl 4-(3-hydroxypropoxy)piperidine-1-carboxylate

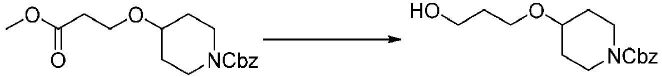

Benzyl 4-(3-methoxy-3-oxopropyl)piperidine-1-carboxylate (500 mg, 1.49 mmol) was dissolved in anhydrous THF (10 mL). A BH₃/THF solution (15 mL, 14.9 mmol) was added at 0 °C, and the mixture was stirred at room temperature for 16 h. Methanol was added to quench the reaction liquid, and the mixture was concentrated under reduced pressure to give a crude product. The crude product was purified by column chromatography (ethyl acetate:petroleum ether = 9:1) to give benzyl 4-(3-hydroxypropoxy)piperidine-1-carboxylate.

LC-MS: (ESI, m/z): [M+H]⁺ = 294.2.

### Step 2: preparation of 3-(piperidin-4-yloxy)propan-1-ol

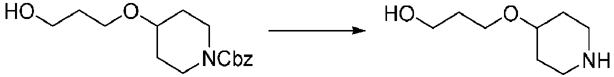

Benzyl 4-(3-hydroxypropoxy)piperidine-1-carboxylate (200 mg, 0.68 mmol) was dissolved in ethyl acetate (10 mL). Pd(OH)₂/C (80 mg) was added, and the mixture was heated to 70 °C under hydrogen atmosphere and stirred overnight. The reaction liquid was cooled to room temperature and filtered, and the filtrate was concentrated under reduced pressure to give 3-(piperidin-4-yloxy)propan-1-ol. The crude product was directly used in the next step without purification.

LC-MS: (ESI, m/z): [M+H]⁺ = 160.2.

### Step 3: preparation of 1-(2-chloro-5-(4-(3-hydroxypropoxy)piperidine-1-carbonyl)phenyl)dihydropyrimidine-2,4(1H,3H)-dione

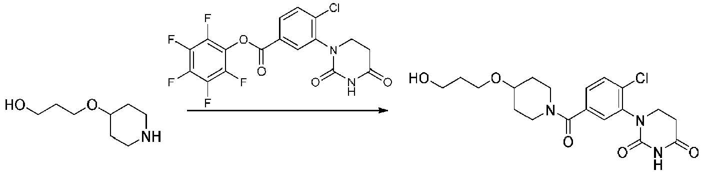

3-(Piperidin-4-yloxy)propan-1-ol (100 mg, 0.62 mmol) was dissolved in DMSO (10 mL). Pentafluorophenyl 4-chloro-3-(2,4-dioxotetrahydropyrimidin-1(2*H*)-yl)benzoate (300 mg, 0.69 mmol) and DIEA (240 mg, 1.86 mmol) were added, and the mixture was stirred at room temperature for 2 h. Water (100 mL) was added to the reaction liquid, and the mixture was extracted with dichloromethane (30 mL × 3). The organic phase was dried over anhydrous sodium sulfate, filtered, and concentrated under reduced pressure to give a crude product. The crude product was purified by column chromatography (methanol:dichloromethane = 1:9) to give 1-(2-chloro-5-(4-(3-hydroxypropoxy)piperidine-1-carbonyl)phenyl)dihydropyrimidine-2,4(1*H*,3*H*)-dione.

LC-MS: (ESI, m/z): [M+H]⁺ = 410.1.

### Step 4: preparation of 3-((1-(4-chloro-3-(2,4-dioxotetrahydropyrimidin-1(2H)-yl)benzoyl)piperidin-4-yl)oxy)propionaldehyde

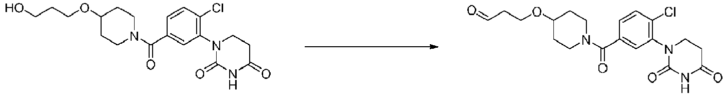

1-(2-Chloro-5-(4-(3-hydroxypropoxy)piperidine-1-carbonyl)phenyl)dihydropyrimidine-2,4(1*H*,3*H*)-dione (60 mg, 0.147 mmol) was dissolved in DCM (5 mL). PCC (63 mg, 0.294 mmol) was added, and the mixture was stirred at room temperature for 16 h. The reaction liquid was concentrated under reduced pressure, and the crude product was purified by column chromatography (methanol:dichloromethane = 1:9) to give 3-((1-(4-chloro-3-(2,4-dioxotetrahydropyrimidin-1(2*H*)-yl)benzoyl)piperidin-4-yl)oxy)propionaldehyde.

LC-MS: (ESI, m/z): [M+H]⁺ = 408.1.

### Step 5: preparation of 5-((1R,4R)-2-oxa-5-azabicyclo[2.2.1]heptan-5-yl)-N-(1-(1-(3-((1-(4-chloro-3-(2,4-dioxotetrahydropyrimidin-1(2H)-yl)benzoyl)piperidin-4-yl)oxy)propyl)piperidin-4-yl)-3-(difluoromethyl)-1H-pyrazol-4-yl)pyrazolo[1,5-a]pyrimidine-3-carboxamide

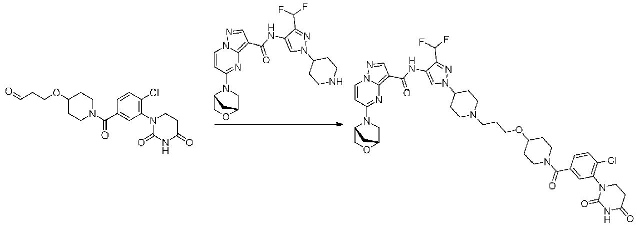

3-((1-(4-Chloro-3-(2,4-dioxotetrahydropyrimidin-1(2*H*)-yl)benzoyl)piperidin-4-yl)oxy)propionaldehyde (55 mg, 0.135 mmol) was dissolved in THF (5 mL). 5-((1R,4R)-2-Oxa-5-azabicyclo[2.2.1]heptan-5-yl)-*N*-(3-(difluoromethyl)-1-(piperidin-4-yl)-1*H*-pyrazol-4-yl)pyrazolo[1,5-a]pyrimidine-3-carboxamide (crude: 70 mg, 0.135 mmol) and STAB (86 mg, 0.405 mmol) were added, and the mixture was stirred at room temperature for 2 h. Water (100 mL) was added to the reaction liquid, and the mixture was extracted with ethyl acetate (30 mL × 3). The organic phase was washed with a saturated aqueous sodium chloride solution (100 mL), dried over anhydrous sodium sulfate, filtered, and concentrated under reduced pressure to give a crude product. The crude product was purified by pre-HPLC (CH₃CN/0.08% aqueous NH₄HCO₃ solution, 5%-95%) to give 5-((1*R*,4*R*)-2-oxa-5-azabicyclo[2.2.1]heptan-5-yl)-*N*-(1-(1-(3-((1-(4-chloro-3-(2,4-dioxotetrahydropyrimidin-1(2*H*)-yl)benzoyl)piperidin-4-yl)oxy)propyl)piperidin-4-yl)-3-(difluoromethyl)-1*H*-pyrazol-4-yl)pyrazolo[1,5-*a*]pyrimidine-3-carboxamide.

LC-MS: (ESI, m/z): [M+H]⁺ = 850.4.

¹H NMR (400 MHz, DMSO-*d*₆) δ 10.51 (s, 1H), 9.50 (d, *J* = 5.7 Hz, 1H), 8.79 (d, *J* = 7.8 Hz, 1H), 8.40 (d, *J* = 3.2 Hz, 1H), 8.26 (d, *J* = 5.5 Hz, 1H), 7.67-7.55 (m, 2H), 7.40 (d, *J* = 8.7 Hz, 1H), 7.30-6.92 (m, 1H), 6.88-6.45 (m, 1H), 5.28-5.08 (m, 1H), 4.77 (d, *J* = 17.7 Hz, 1H), 4.30-4.10 (m, 1H), 4.02-3.68 (m, 4H), 3.62-3.47 (m, 7H), 3.30-3.10 (m, 2H), 3.00-2.85 (m, 2H), 2.78-2.69 (m, 2H), 2.45-2.31 (m, 2H), 2.10-1.55 (m, 12H), 1.52-1.37 (m, 2H).

### Example 24: N-(3-(difluoromethyl)-1-(1-((3-(3-(2,4-dioxotetrahydropyrimidin-1(2H)-yl)-4-fluorobenzoyl)-3-azaspiro[5.5]undecan-9-yl)methyl)piperidin-4-yl)-1H-pyrazol-4-yl)-5-morpholinopyrazolo[1,5-a]pyrimidine-3-carboxamide

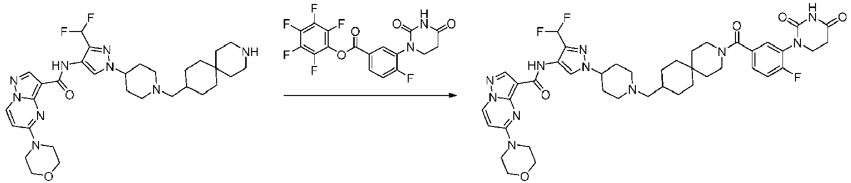

*N*-(1-(1-((3-Azaspiro[5.5]undecan-9-yl)methyl)piperidin-4-yl)-3-(difluoromethyl)-1*H*-pyrazol-4-yl)-5-morpholinopyrazolo[1,5-*a*]pyrimidine-3-carboxamide (100 mg, 0.14 mmol) was dissolved in dimethyl sulfoxide (3 mL). Pentafluorophenyl 3-(2,4-dioxotetrahydropyrimidin-1(2H)-yl)-4-fluorobenzoate (58 mg, 0.14 mmol) and *N*,*N*-diisopropylethylamine (54 mg, 0.42 mmol) were added while stirring. After the mixture was stirred at room temperature for 2 h, water (50 mL) was added. The mixture was filtered, and the filter cake was purified by pre-HPLC (CH₃CN/0.08% aqueous NH₄HCO₃ solution, 5%-95%) to give N-(3-(difluoromethyl)-1-(1-((3-(3-(2,4-dioxotetrahydropyrimidin-1(2*H*)-yl)-4-fluorobenzoyl)-3-azaspiro[5.5]undecan-9-yl)methyl)piperidin-4-yl)-1*H*-pyrazol-4-yl)-5-morpholinopyrazolo[1,5-a]pyrimidine-3-carboxamide.

LC-MS: (ESI, m/z): [M+H]⁺ = 846.4.

¹H NMR (400 MHz, DMSO-*d*₆) δ 10.53 (s, 1H), 9.40 (s, 1H), 8.83 (d, *J* = 7.9 Hz, 1H), 8.39 (s, 1H), 8.29 (s, 1H), 7.50 (d, *J* = 7.7 Hz, 1H), 7.36 (d, *J* = 4.0 Hz, 2H), 7.23-6.97 (m, 1H), 6.91 (d, *J* = 8.0 Hz, 1H), 4.25-4.15 (m, 1H), 3.84-3.69 (m, 10H), 3.65-3.49 (m, 2H), 3.31-3.24 (m, 2H), 2.89 (d, *J* = 10.3 Hz, 2H), 2.73 (t, *J* = 6.6 Hz, 2H), 2.20-2.04 (m, 2H), 2.04-1.80 (m, 6H), 1.70-1.43 (m, 7H), 1.43-1.20 (m, 2H), 1.20-0.96 (m, 4H).

### Example 25: N-(3-(difluoromethyl)-1-(1-((3-(3-(2,4-dioxotetrahydropyrimidin-1(2H)-yl)-4-(trifluoromethyl)benzoyl)-3-azaspiro[5.5]undecan-9-yl)methyl)piperidin-4-yl)-1H-pyrazol-4-yl)-5-morpholinopyrazolo[1,5-a]pyrimidine-3-carboxamide

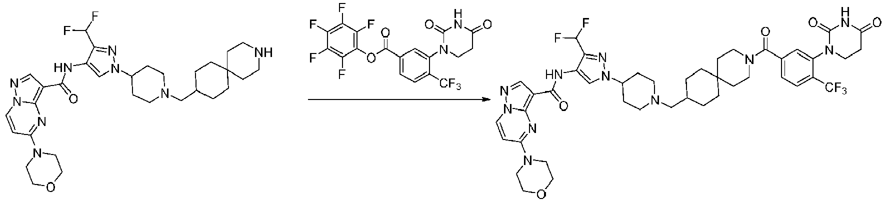

*N*-(1-(1-((3-Azaspiro[5.5]undecan-9-yl)methyl)piperidin-4-yl)-3-(difluoromethyl)-1*H*-pyrazol-4-yl)-5-morpholinopyrazolo[1,5-a]pyrimidine-3-carboxamide (100 mg, 0.14 mmol) was dissolved in dimethyl sulfoxide (3 mL). Pentafluorophenyl 3-(2,4-dioxotetrahydropyrimidin-1(2*H*)-yl)-4-(trifluoromethyl)benzoate (65 mg, 0.14 mmol) and *N*,*N*-diisopropylethylamine (54 mg, 0.42 mmol) were added while stirring. After the mixture was stirred at room temperature for 2 h, water (50 mL) was added. The mixture was filtered, and the filter cake was purified by pre-HPLC (CH₃CN/0.08% aqueous NH₄HCO₃ solution, 5%-95%) to give *N*-(3-(difluoromethyl)-1-(1-((3-(3-(2,4-dioxotetrahydropyrimidin-1(2*H*)-yl)-4-(trifluoromethyl)benzoyl)-3-azaspiro[5.5]undecan-9-yl)methyl)piperidin-4-yl)-1H-pyrazol-4-yl)-5-morpholinopyrazolo[1,5-*a*]pyrimidine-3-carboxamide.

LC-MS: (ESI, m/z): [M+H]⁺ = 896.4.

¹H NMR (400 MHz, DMSO-*d*₆) δ 10.53 (s, 1H), 9.40 (s, 1H), 8.83 (d, J= 7.9 Hz, 1H), 8.39 (s, 1H), 8.29 (s, 1H), 7.87 (d, *J* = 8.2 Hz, 1H), 7.71 (s, 1H), 7.59 (d, *J* = 7.9 Hz, 1H), 7.23-6.97 (m, 1H), 6.91 (d, *J* = 8.0 Hz, 1H), 4.28-4.12 (m, 1H), 3.94-3.80 (m, 1H), 3.80-3.70 (m, 8H), 3.65-3.55 (m, 2H), 3.51-3.41 (m, 1H), 3.30-3.20 (m, 2H), 2.94-2.80 (m, 2H), 2.75-2.60 (m, 2H), 2.24-2.06 (m, 2H), 2.06-1.90 (m, 6H), 1.80-1.60 (m, 2H), 1.60-1.30 (m, 6H), 1.30-1.20 (m, 1H), 1.17-0.92 (m, 4H).

### Example 26: N-(3-(difluoromethyl)-1-(1-((3-(4-(2,6-oxopiperidin-3-yl)benzoyl)-3-azaspiro[5.5]undecan-9-yl)methyl)piperidin-4-yl)-1H-pyrazol-4-yl)-5-morpholinopyrazolo[1,5-a]pyrimidine-3-carboxamide

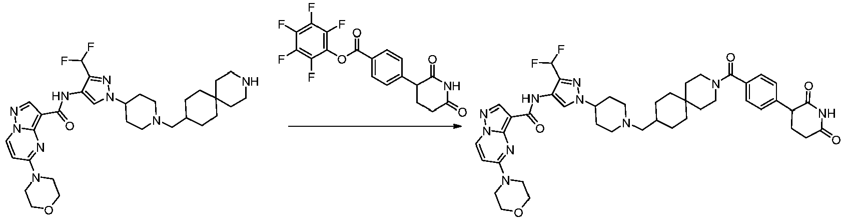

*N*-(1-(1-((3-Azaspiro[5.5]undecan-9-yl)methyl)piperidin-4-yl)-3-(difluoromethyl)-1*H*-pyrazol-4-yl)-5-morpholinopyrazolo[1,5-*a*]pyrimidine-3-carboxamide (100 mg, 0.14 mmol) was dissolved in dimethyl sulfoxide (3 mL). Pentafluorophenyl 4-(2,6-dioxopiperidin-3-yl)benzoate (56 mg, 0.14 mmol) and *N*,*N-*diisopropylethylamine (54 mg, 0.42 mmol) were added while stirring. After the mixture was stirred at room temperature for 2 h, water (50 mL) was added, and the mixture was extracted with dichloromethane (20 mL × 3). The organic phase was washed with saturated brine (60 mL × 3), dried over anhydrous sodium sulfate, filtered, and concentrated under reduced pressure to give a crude product. The filter cake was purified by pre-HPLC (CH₃CN/0.08% aqueous NH₄HCO₃ solution, 5%-95%) to give N-(3-(difluoromethyl)-1-(1-((3-(4-(2,6-oxopiperidin-3-yl)benzoyl)-3-azaspiro[5.5]undecan-9-yl)methyl)piperidin-4-yl)-1H-pyrazol-4-yl)-5-morpholinopyrazolo[1,5-*a*]pyrimidine-3-carboxamide.

LC-MS: (ESI, m/z): [M+H]⁺ = 827.4.

¹H NMR (400 MHz, DMSO-*d*₆) δ 10.87 (s, 1H), 9.40 (s, 1H), 8.83 (d, J= 7.9 Hz, 1H), 8.39 (s, 1H), 8.29 (s, 1H), 7.40-7.25 (m, 4H), 7.23-6.95 (m, 1H), 6.91 (d, *J* = 8.0 Hz, 1H), 4.35-4.06 (m, 1H), 3.97-3.88 (m, 1H), 3.84-3.73 (m, 8H), 3.64-3.50 (m, 2H), 3.31-3.22 (m, 2H), 2.92-2.80 (m, 2H), 2.75-2.62 (m, 1H), 2.56-2.51 (m, 1H), 2.30-1.90 (m, 10H), 1.76-1.25 (m, 9H), 1.20-0.93 (m, 4H).

### Example 27: N-(3-(difluoromethyl)-1-(1-((3-(4-(2,4-dioxotetrahydropyrimidin-1(2H)-yl)benzoyl)-3-azaspiro[5.5]undecan-9-yl)methyl)piperidin-4-yl)-1H-pyrazol-4-yl)-5-morpholinopyrazolo[1,5-a]pyrimidine-3-carboxamide

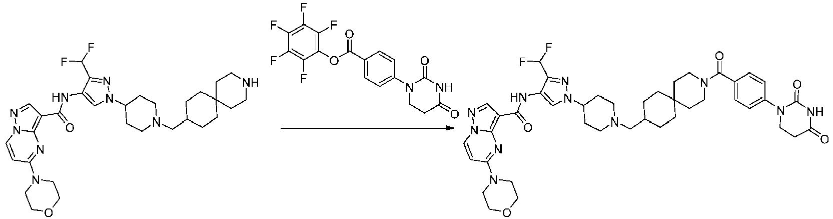

*N*-(1-(1-((3-Azaspiro[5.5]undecan-9-yl)methyl)piperidin-4-yl)-3-(difluoromethyl)-1*H*-pyrazol-4-yl)-5-morpholinopyrazolo[1,5-*a*]pyrimidine-3-carboxamide (100 mg, 0.14 mmol) was dissolved in dimethyl sulfoxide (3 mL). Pentafluorophenyl 4-(2,4-dioxotetrahydropyrimidin-1(2*H*)-yl)benzoate (56 mg, 0.14 mmol) and *N*,*N*-diisopropylethylamine (54 mg, 0.42 mmol) were added while stirring. After the mixture was stirred at room temperature for 2 h, water (50 mL) was added. The mixture was filtered, and the filter cake was purified by pre-HPLC (CH₃CN/0.08% aqueous NH₄HCO₃ solution, 5%-95%) to give *N*-(3-(difluoromethyl)-1-(1-((3-(4-(2,4-dioxotetrahydropyrimidin-l(2*H*)-yl)benzoyl)-3-azaspiro[5.5]undecan-9-yl)methyl)piperidin-4-yl)-1*H*-pyrazol-4-yl)-5-morpholinopyrazolo[1,5-*a*]pyrimidine-3-carboxamide.

LC-MS: (ESI, m/z): [M+H]¹ = 828.4.

¹H NMR (400 MHz, DMSO-*d*₆) δ 10.43 (s, 1H), 9.40 (s, 1H), 8.83 (d, *J=* 7.9 Hz, 1H), 8.39 (s, 1H), 8.29 (s, 1H), 7.39 (s, 4H), 7.23-6.97 (m, 1H), 6.91 (d, *J* = 8.0 Hz, 1H), 4.26-4.15 (m, 1H), 3.92-3.70 (m, 10H), 3.68-3.50 (m, 2H), 3.31-3.22 (m, 2H), 2.89 (d, *J=* 10.1 Hz, 2H), 2.72 (t, *J=* 6.6 Hz, 2H), 2.24-2.09 (m, 2H), 2.07-1.84 (m, 6H), 1.75-1.29 (m, 9H), 1.18-0.92 (m, 4H).

### Example 28: N-(3-(difluoromethyl)-1-(1-(2-(3-(3-(2,4-dioxotetrahydropyrimidin-1(2H)-yl)-4-methoxybenzoyl)-3-azaspiro[5.5]undecan-9-yl)ethyl)piperidin-4-yl)-1H pyrazol-4-yl)-5-morpholinopyrazolo[1,5-a]pyrimidine-3-carboxamide

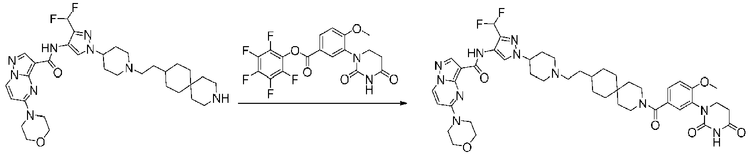

*N*-(1-(1-(2-(3-Azaspiro[5.5]undecan-9-yl)ethyl)piperidin-4-yl)-3-(difluoromethyl)-1*H*-pyrazol-4-yl)-5-morpholinopyrazolo[1,5-*a*]pyrimidine-3-carboxamide (103 mg, 0.165 mmol) and pentafluorophenyl 3-(2,4-dioxotetrahydropyrimidin-1(2*H*)-yl)-4-methoxybenzoate (85 mg, 0.198 mmol) were added to DMSO (3 mL). Then DIEA (63.8 mg, 0.495 mmol) was added. The mixture was stirred at room temperature for 16 h, and then water (20 mL) was added. The mixture was extracted with ethyl acetate (25 mL × 4), washed with saturated brine, dried over anhydrous sodium sulfate, and concentrated under reduced pressure. The crude product was purified by Prep-HPLC (acetonitrile/0.5% aqueous formic acid solution, 5%-95%) to give the product of formate of *N*-(3-(difluoromethyl)-1-(1-(2-(3-(3-(2,4-dioxotetrahydropyrimidin-1(2*H*)-yl)-4-methoxybenzoyl)-3-azaspiro[5.5]undecan-9-yl)ethyl)piperidin-4-yl)-1*H*-pyrazol-4-yl)-5-morpholinopyrazolo[1,5-*a*]pyrimidine-3-carboxamide.

LC-MS: (ESI, m/z): [M+H]⁺ = 872.5.

¹H NMR (400 MHz, DMSO-*d*₆) δ 10.34 (s, 1H), 9.40 (s, 1H), 8.83 (d, *J* = 7.9 Hz, 1H), 8.39 (s, 1H), 8.29 (s, 1H), 8.27 (s, 0.62H, HCOOH), 7.42-7.28 (m, 2H), 7.26-6.94 (m, 2H), 6.91 (d, *J* = 7.9 Hz, 1H), 4.30-4.10 (m, 1H), 3.84 (s, 3H), 3.79-3.61(m, 8H), 3.62-3.54 (m, 6H), 2.97-2.91 (m, 2H), 2.71-2.65 (m, 2H), 2.37-2.17 (m, 2H), 2.05-1.85 (m, 6H), 1.75-1.62 (m, 2H), 1.55-1.17 (m, 9H), 1.17-0.93 (m, 4H).

### Example 29: N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)-5-morpholinopyrazolo[1,5-a]pyrimidine-3-carboxamide

### Step 1: preparation of tert-butyl 9-(((1R,4R)-4-(3-(difluoromethyl)-4-(5-morpholinopyrazolo[1,5-a]pyrimidine-3-carboxamido)-1H-pyrazol-1-yl)cyclohexyl)methyl)-3,9-diazaspiro[5.5]undecane-3-carboxylate

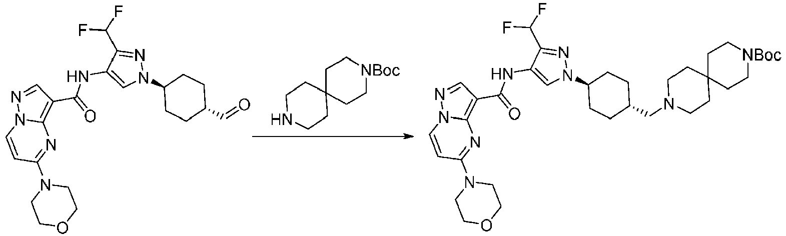

*N*-(3-(Difluoromethyl)-1-((1*R*,4*R*)-4-(formyl)cyclohexyl)-1*H*-pyrazol-4-yl)-5-morpholinopyrazolo[1,5-*a*]pyrimidine-3-carboxamide (50 mg, 0.10 mmol) and tert-butyl 3,9-diazaspiro[5.5]undecane-3-carboxylate (24 mg, 0.10 mmol) were dissolved in THF (3 mL). Sodium triacetoxyborohydride (67 mg, 0.32 mmol) was added to the reaction liquid while stirring, and the reaction liquid was stirred at room temperature for 2 h. Water (50 mL) was added to the reaction liquid, and the mixture was extracted with ethyl acetate (30 mL × 3). The organic phases were combined, dried over anhydrous sodium sulfate, and concentrated under reduced pressure. The crude product was purified by normal phase column chromatography to give *tert*-butyl 9-(((1*R*,4*R*)-4-(3-(difluoromethyl)-4-(5-morpholinopyrazolo[1,5-*a*]pyrimidine-3-carboxamido)-1*H*-pyrazol-1-yl)cyclohexyl)methyl)-3,9-diazaspiro[5.5]undecane-3-carboxylate.

LC-MS: (ESI, m/z): [M+H]⁺ = 712.3.

### Step 2: preparation of N (1-((1R,4R)-4-((3,9-diazaspiro[5.5]undecan-3-yl)methyl)cyclohexyl)-3-(difluoromethyl)-1H-pyrazol-4-yl)-5-morpholinopyrazolo[1,5-a]pyrimidine-3-carboxamide

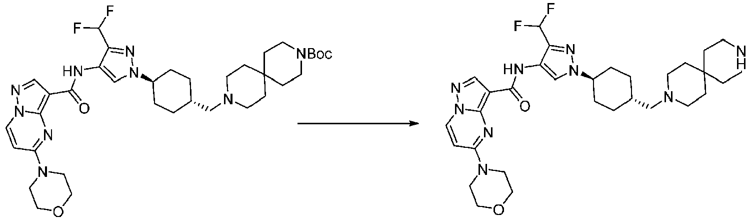

*tert*-Butyl 9-(((1*R*,4*R*)-4-(3-(difluoromethyl)-4-(5-morpholinopyrazolo[1,5-*a*]pyrimidine-3-carboxamido)-1H-pyrazol-1-yl)cyclohexyl)methyl)-3,9-diazaspiro[5.5]undecane-3-carboacylate (95 mg, 0.13 mmol) was dissolved in DCM (3 mL). TFA (1 mL) was added, and the reaction liquid was stirred at room temperature for 1 h. The reaction liquid was directly concentrated under reduced pressure to give N (1-((1*R*,4*R*)-4-((3,9-diazaspiro[5.5]undecan-3-yl)methyl)cycloheacyl)-3-(difluoromethyl)-1*H*-pyrazol-4-yl)-5-morpholinopyrazolo[1,5-a]pyrimidine-3-carboxamide (crude), which was directly used in the next step.

LC-MS: (ESI, m/z): [M+H]⁺ = 612.3.

### Step 3: preparation of N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)-5-morpholinopyrazolo[1,5-a]pyrimidine-3-carboxamide

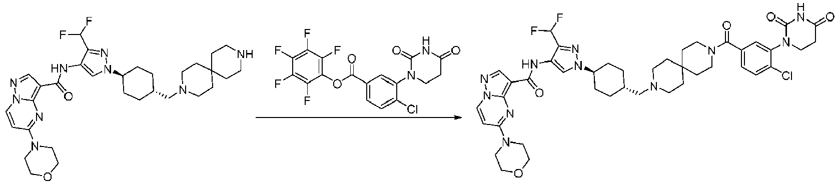

*N*-(1-((1*R*,4*R*)-4-((3,9-Diazaspiro[5.5]undecan-3-yl)methyl)cyclohexyl)-3-(difluoromethyl)-1*H-*pyrazol-4-yl)-5-morpholinopyrazolo[1,5-a]pyrimidine-3-carboxamide (130 mg, 0.13 mmol) and pentafluorophenyl 4-chloro-3-(2,4-dioxotetrahydropyrimidin-1(2*H*)-yl)benzoate (57 mg, 0.13 mmol) were dissolved in DMSO (5 mL). DIEA (84 mg, 0.65 mmol) was added to the reaction liquid while stirring, and the reaction liquid was stirred at room temperature for 1 h. After the reaction was complete, the reaction liquid was poured into water (50 mL), and the mixture was extracted with EA (30 mL × 3). The organic phases were combined, washed with saturated brine (80 mL), dried over anhydrous sodium sulfate, and filtered. The filtrate was concentrated under reduced pressure and separated and purified by prep-HPLC to give *N*-(1-((1*R*,4*R*)-4-((9-(4-chloro-3-(2,4-dioxotetrahydropyrimidin-1(2*H*)-yl)benzoyl)-3,9-diazaspiro[5.5]undecan-3-yl)methyl)cyclohexyl)-3-(difluoromethyl)-1*H*-pyrazol-4-yl)-5-morpholinopyrazolo[1,5-*a*]pyrimidine-3-carboxamide.

LC-MS: (ESI, m/z): [M+H]⁺ = 862.3.

¹H NMR (400 MHz, DMSO-*d*₆) δ 10.50 (s, 1H), 9.39 (s, 1H), 8.82 (d, *J* = 6.3 Hz, 1H), 8.37 (s, 1H), 8.28 (s, 1H), 7.63 (d, *J* = 7.9 Hz, 1H), 7.55 (s, 1H), 7.39 (d, *J* = 7.9 Hz, 1H), 7.25-6.95 (m, 1H), 6.90 (d, *J* = 7.6 Hz, 1H), 4.27-4.07 (m, 1H), 3.90-3.50 (m, 12H), 3.40-3.30 (m, 2H), 2.79-2.70 (m, 2H), 2.43-2.23 (m, 4H), 2.20-1.95 (m, 4H), 1.93-1.83 (m, 2H), 1.82-1.30 (m, 11H), 1.09-0.94 (m, 2H).

### Example 30: N-(1-((1R,4R)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)-5-morpholinopyrazolo[1,5-a]pyrimidine-3-carboxamide

### Step 1: preparation of tert-butyl 4-(2-((((1R,4R)-4-(3-(difluoromethyl)-4-(5-morpholinopyrazolo[1,5-a]pyrimidine-3-carboxamido)-1H-pyrazol-1-yl)cyclohexyl)methyl)(methyl)amino)ethyl)piperidine-1-carboxylate

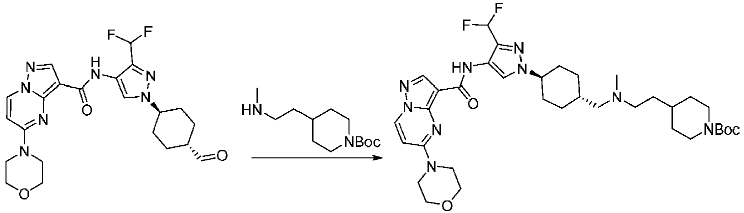

*N*-(3-(Difluoromethyl)-1-((1*R*,4*R*)-4-(formyl)cyclohexyl)-1*H*-pyrazol-4-yl)-5-morpholinopyrazolo[1,5-*a*]pyrimidine-3-carboxamide (110 mg, 0.23 mmol) was dissolved in THF (10 mL). *tert*-Butyl 4-(2-(methylamino)ethyl)piperidine-1-carboxylate (56 mg, 0.23 mmol) and STAB (146 mg, 0.69 mmol) were added, and the mixture was reacted at room temperature for 2 h. The mixture was diluted with water (50 mL) and extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 1:11) to give *tert*-butyl 4-(2-((((1*R*,4*R*)-4-(3-(difluoromethyl)-4-(5-morpholinopyrazolo[1,5-*a*]pyrimidine-3-carboxamido)-1*H*-pyrazol-1-yl)cyclohexyl)methyl)(methyl)amino)ethyl)piperidine-1-carboxylate.

LC-MS: (ESI, m/z): [M+H]⁺ = 700.4.

### Step 2: preparation of N-(3-(difluoromethyl)-1-((1R,4R)-4-((methyl(2-(piperidin-4-yl)ethyl)amino)methyl)cyclohexyl)-1H-pyrazol-4-yl)-5-morpholinopyrazolo[1,5-a]pyrimidine-3-carboxamide

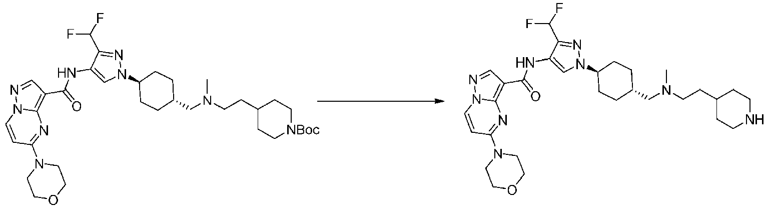

*tert*-Butyl 4-(2-((((1*R*,4*R*)-4-(3-(difluoromethyl)-4-(5-morpholinopyrazolo[1,5-*a*]pyrimidine-3-carboxamido)-1*H*-pyrazol-1-yl)cyclohexyl)methyl)(methyl)amino)ethyl)piperidine-1-carboxylate (95 mg, 0.14 mmol) was dissolved in DCM (3 mL). TFA (1 mL) was added, and the mixture was reacted at room temperature for 1 h. The reaction liquid was directly concentrated under reduced pressure to give N-(3-(difluoromethyl)-1-((1*R*,4*R*)-4-((methyl(2-(piperidin-4-yl)ethyl)amino)methyl)cyclohexyl)-1*H*-pyrazol-4-yl)-5-morpholinopyrazolo[1,5-*a*]pyrimidine-3-carboxamide, which was directly used in the next step.

LC-MS: (ESI, m/z): [M+H]⁺ = 600.3.

### Step 3: preparation of N-(1-((1R,4R)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)-5-morpholinopyrazolo[1,5-a]pyrimidine-3-carboxamide

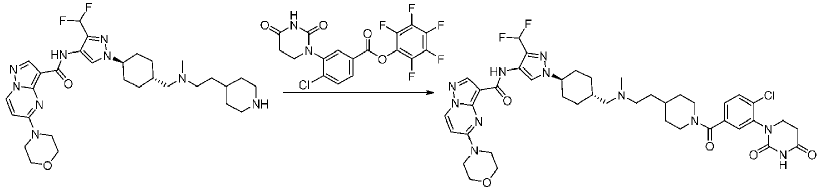

*N*-(3-(Difluoromethyl)-1-((1*R*,4*R*)-4-((methyl(2-(piperidin-4-yl)ethyl)amino)methyl)cyclohexyl)-1*H*-pyrazol-4-yl)-5-morpholinopyrazolo[1,5-*a*]pyrimidine-3-carboxamide (95 mg, 0.14 mmol) was dissolved in DMSO (5 mL). DIEA (77 mg, 0.60 mmol) and pentafluorophenyl 4-chloro-3-(2,4-dioxotetrahydropyrimidin-1(2*H*)-yl)benzoate (61 mg, 0.14 mmol) were added, and the mixture was reacted at room temperature for 2 h. Water (50 mL) was added, and the mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by preparative reversed-phase chromatography to give *N*-(1-((1R,4R)-4-(((2-(1-(4-chloro-3-(2,4-dioxotetrahydropyrimidin-1(2*H*)-yl)benzoyl)piperidin-4-yl)ethyl)(methyl)amino)methyl)cyclohexyl)-3-(difluoromethyl)-1*H*-pyrazol-4-yl)-5-morpholinopyrazolo[1,5-*a*]pyrimidine-3-carboxamide.

LC-MS: (ESI, m/z): [M+H]⁺ = 850.4.

¹H NMR (400 MHz, DMSO-*d*₆) δ 10.50 (s, 1H), 9.39 (s, 1H), 8.83 (d, *J* = 7.9 Hz, 1H), 8.38 (s, 1H), 8.29 (s, 1H), 7.63 (d, *J* = 8.2 Hz, 1H), 7.54 (d, *J* = 1.8 Hz, 1H), 7.38 (dd, *J* = 8.2, 1.8 Hz, 1H), 7.24-6.97 (m, 1H), 6.91 (d, *J* = 8.0 Hz, 1H), 4.49-4.38 (m, 1H), 4.23-4.13 (m, 1H), 3.83-3.76 (m, 4H), 3.75-3.70 (m, 4H), 3.66-3.52 (m, 2H), 3.10-2.95 (m, 1H), 2.84-2.67 (m, 3H), 2.29 (t, *J* = 7.0 Hz, 2H), 2.14-2.01 (m, 7H), 1.93-1.84 (m, 2H), 1.82-1.46 (m, 7H), 1.41-1.32 (m, 2H), 1.17-0.95 (m, 4H).

### Example 31: 5-(8-oxa-3-azabicyclo[3.2.1]octan-3-yl)-N-(1-((1R,4R)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)pyrazolo[1,5-a]pyrimidine-3-carboxamide

### Step 1: preparation of 5-(8-oxa-3-azabicyclo[3.2.1]octan-3-yl)-N-(3-(difluoromethyl)-1-((1R,4R)-4-(hydroxymethyl)cyclohexyl)-1H-pyrazol-4-yl)pyrazolo[1,5-a]pyrimidine-3-carboxamide

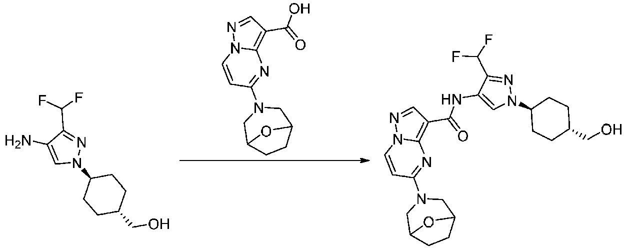

((1*R*,4*R*)-4-(4-Amino-3-(difluoromethyl)-1H-pyrazol-1-yl)cyclohexyl)methanol (410 mg, 1.67 mmol) was dissolved in DMF (10 mL). 5-(8-Oxa-3-azaspirobicyclo[3.2.1]octan-3-yl)pyrazolo[1,5-a]pyrimidine-3-carboxylic acid (459 mg, 1.67 mmol), HATU (825 mg, 2.17 mmol), and DIEA (646 mg, 5.01 mmol) were added, and the mixture was reacted at room temperature for 2 h. Water (50 mL) was added, and the mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by column chromatography to give 5-(8-oxa-3-azabicyclo[3.2.1]octan-3-yl)-*N*-(3-(difluoromethyl)-1-((1*R*,4*R*)-4-(hydroxymethyl)cyclohexyl)-1*H*-pyrazol-4-yl)pyrazolo[1,5-*a*]pyrimidine-3-carboxamide.

LC-MS: (ESI, m/z): [M+H]^{|} = 502.3.

### Step 2: preparation of 5-(8-oxa-3-azabicyclo[3.2.1]octan-3-yl)-N-(3-(difluoromethyl)-1-((1R,4R)-4-formylcyclohexyl)-1H-pyrazol-4-yl)pyrazolo[1,5-a]pyrimidine-3-carboxamide

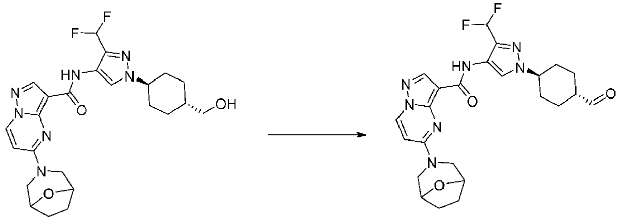

5-(8-Oxa-3-azabicyclo[3.2.1]octan-3-yl)-A-(3-(difluoromethyl)-1-((1R,4R)-4-(hydroxymethyl)cyclohexyl)-1*H*-pyrazol-4-yl)pyrazolo[1,5-*a*]pyrimidine-3-carboxamide (290 mg, 0.58 mmol) was dissolved in ACN (10 mL). IBX (324 mg, 1.16 mmol) was added, and the mixture was reacted at 85 °C for 1.5 h and concentrated under reduced pressure to remove ACN. Water (30 mL) was added, and the mixture was extracted with ethyl acetate (30 mL × 3). The organic phase was washed with saturated brine (50 mL), dried over anhydrous sodium sulfate, filtered, and concentrated under reduced pressure. The crude product was purified by column chromatography to give 5-(8-oxa-3-azabicyclo[3.2.1]octan-3-yl)-N-(3-(difluoromethyl)-1-((1*R*,4*R*)-4-formylcyclohexyl)-1*H*-pyrazol-4-yl)pyrazolo[1,5-*a*]pyrimidine-3-carboxamide.

LC-MS: (ESI, m/z): [M+H]⁺ = 500.3.

### Step 3: preparation of tert-butyl 4-(2-((((1R,4R)-4-(4-(5-(8-oxa-3-azabicyclo[3.2.1]octan-3-yl)pyrazolo[1,5-a]pyrimidine-3-carboxamido)-3-(difluoromethyl)-1H-pyrazol-1-yl)cyclohexyl)methyl)(methyl)amino)ethyl)piperidine-1-carboxylate

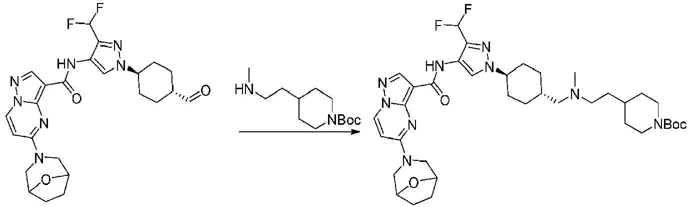

5-(8-Oxa-3-azabicyclo[3.2.1]octan-3-yl)-*N*-(3-(difluoromethyl)-1-((1R,4R)-4-formylcyclohexyl)-1*H*-pyrazol-4-yl)pyrazolo[1,5-*a*]pyrimidine-3-carboxamide (100 mg, 0.20 mmol) was dissolved in THF (10 mL). *tert*-Butyl 4-(2-(methylamino)ethyl)piperidine-1-carboxylate (48 mg, 0.20 mmol) and STAB (127 mg, 0.6 mmol) were added, and the mixture was reacted at room temperature for 2 h. The mixture was diluted with water (50 mL) and extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 1:11) to give *tert*-butyl 4-(2-((((1*R*,4*R*)-4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)cyclohexyl)methyl)(methyl)amino)ethyl)piperidine-1-carboxylate.

LC-MS: (ESI, m/z): [M+H]⁺ = 726.4.

### Step 4: preparation of 5-(8-oxa-3-azabicyclo[3.2.1]octan-3-yl)-N-(3-(difluoromethyl)-1-((1R,4R)-4-((methyl(2-(piperidin-4-yl)ethyl)amino)methyl)cyclohexyl)-1H-pyrazol-4-yl)pyrazolo[1,5-a]pyrimidine-3-carboxamide

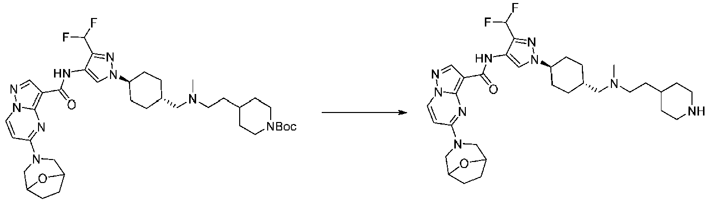

*tert*-Butyl 4-(2-((((1*R*,4*R*)-4-(4-(5-(8-oxa-3-azabicyclo[3.2.1]octan-3-yl)pyrazolo[1,5-a]pyrimidine-3-carboxamido)-3-(difluoromethyl)-1H-pyrazol-1-yl)cyclohexyl)methyl)(methyl)amino)ethyl)piperidine-1-carboxylate (110 mg, 0.15 mmol) was dissolved in DCM (3 mL). TFA (1 mL) was added, and the reaction liquid was reacted at room temperature for 1 h and concentrated under reduced pressure to give 5-(8-oxa-3-azabicyclo[3.2.1]octan-3-yl)-*N*-(3-(difluoromethyl)-1-((1*R*,4*R*)-4-((methyl(2-(piperidin-4-yl)ethyl)amino)methyl)cyclohexyl)-1*H*-pyrazol-4-yl)pyrazolo[1,5-a]pyrimidine-3-carboxamide, which was directly used in the next step.

LC-MS: (ESI, m/z): [M+H]⁺ = 626.3.

### Step 5: preparation of 5-(8-oxa-3-azabicyclo[3.2.1]octan-3-yl)-N-(1-((1R,4R)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)pyrazolo[1,5-a]pyrimidine-3-carboxamide

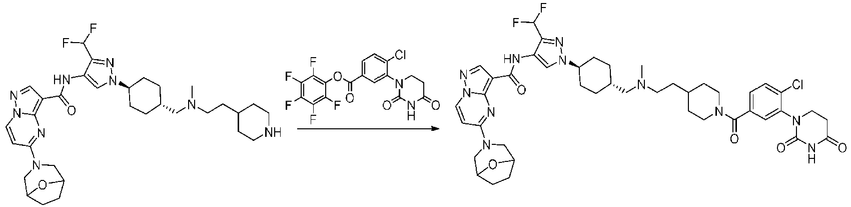

5-(8-Oxa-3-azabicyclo[3.2.1]octan-3-yl)-*N*-(3-(difluoromethyl)-1-((1R,4R)-4-((methyl(2-(piperidin-4-yl)ethyl)amino)methyl)cyclohexyl)-1*H*-pyrazol-4-yl)pyrazolo[1,5-*a*]pyrimidine-3-carboxamide (110 mg, 0.15 mmol) was dissolved in DMSO (5 mL). DIEA (97 mg, 0.75 mmol) and pentafluorophenyl 4-chloro-3-(2,4-dioxotetrahydropyrimidin-1(2*H*)-yl)benzoate (65 mg, 0.15 mmol) were added, and the mixture was reacted at room temperature for 2 h. Water (50 mL) was added, and the mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by preparative reversed-phase chromatography to give 5-(8-oxa-3-azabicyclo[3.2.1]octan-3-yl)-*N*-(1-((1*R*,4*R*)-4-(((2-(1 -(4-chloro-3 -(2,4-dioxotetrahydropyrimidin-1(2*H*)-yl)benzoyl)piperidin-4-yl)ethyl)(methyl)amino)methyl)cyclohexyl)-3-(difluoromethyl)-1*H*-pyrazol-4-yl)pyrazolo[1,5-*a*]pyrimidine-3-carboxamide.

LC-MS: (ESI, m/z): [M+H]⁺ = 876.2.

¹H NMR(400 MHz, DMSO-*d*₆) δ 10.50 (s, 1H), 9.41 (s, 1H), 8.82 (d, *J* = 7.9 Hz, 1H), 8.38 (s, 1H), 8.28 (s, 1H), 7.63 (d, *J* = 8.2 Hz, 1H), 7.54 (d, *J* = 1.9 Hz, 1H), 7.38 (dd, *J* = 8.2, 2.0 Hz, 1H), 7.30-6.95 (m, 1H), 6.82 (d, *J* = 8.0 Hz, 1H), 4.48-4.42 (m, 2H), 4.26-4.09 (m, 2H), 3.79-3.50 (m, 4H), 3.27-3.17 (m, 2H), 3.10-2.90 (m, 1H), 2.85-2.70 (m, 3H), 2.29 (t, *J* = 6.8 Hz, 2H), 2.15-2.07 (m, 5H), 2.07-2.01 (m, 2H), 1.95-1.83 (m, 4H), 1.82-1.47 (m, 9H), 1.41-1.31 (m, 2H), 1.17-0.96 (m, 4H).

### Example 32: 5-(8-oxa-3-azabicyclo[3.2.1]octan-3-yl)-N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)pyrazolo[1,5-a]pyrimidine-3-carboxamide

### Step 1: preparation of tert-butyl 9-(((1R,4R)-4-(4-(5-(8-oxa-3-azabicyclo[3.2.1]octan-3-yl)pyrazolo[1,5-a]pyrimidine-3-carboxamido)-3-(difluoromethyl)-1H-pyrazol-1-yl)cyclohexyl)methyl)-3,9-diazaspiro[5.5]undecane-3-carboxylate

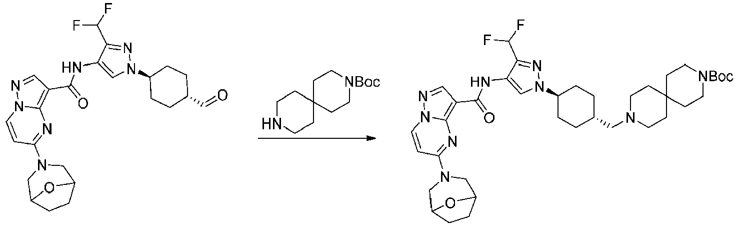

5-(8-Oxa-3-azabicyclo[3.2.1]octan-3-yl)-*N*-(3-(difluoromethyl)-1-((1R,4R)-4-formylcyclohexyl)-1*H*-pyrazol-4-yl)pyrazolo[1,5-*a*]pyrimidine-3-carboxamide (100 mg, 0.20 mmol) was dissolved in THF (10 mL). *tert*-Butyl 3,9-diazaspiro[5.5]undecane-3-carboxylate (48 mg, 0.20 mmol) and STAB (127 mg, 0.6 mmol) were added, and the mixture was reacted at room temperature for 2 h. The mixture was diluted with water (50 mL) and extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 1:11) to give *tert*-butyl 9-(((1*R*,4*R*)-4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)cyclohexyl)methyl)-3,9-diazaspiro[5.5]undecane-3-carboxylate.

LC-MS: (ESI, m/z): [M+H]⁺ = 738.5.

### Step 2: preparation of N-(1-((1R,4R)-4-((3,9-diazaspiro[5.5]undecan-3-yl)methyl)cyclohexyl)-3-(difluoromethyl)-1H-pyrazol-4-yl)-5-(8-oxa-3-azabicyclo[3.2.1]octan-3-yl)pyrazolo[1,5-a]pyrimidine-3-carboxamide

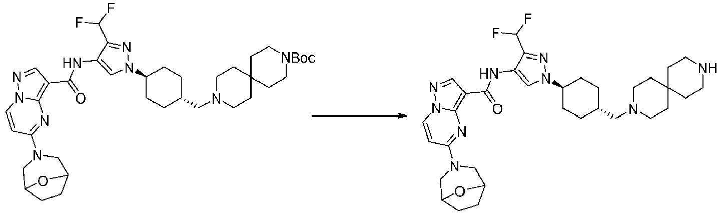

*tert*-Butyl 9-(((1*R*,4*R*)-4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)cyclohexyl)methyl)-3,9-diazaspiro[5.5]undecane-3-carboxylate (105 mg, 0.14 mmol) was dissolved in DCM (3 mL). TFA (1 mL) was added, and the mixture was reacted at room temperature for 1 h. The mixture was concentrated under reduced pressure to give *N*-(1-((1*R*,4*R*)-4-((3,9-diazaspiro[5.5]undecan-3-yl)methyl)cyclohexyl)-3-(difluoromethyl)-1*H*-pyrazol-4-yl)-5-(8-oxa-3-azabicyclo[3.2.1]octan-3-yl)pyrazolo[1,5-*a*]pyrimidine-3-carboxamide, which was directly used in the next step.

### Step 3: preparation of 5-(8-oxa-3-azabicyclo[3.2.1]octan-3-yl)-N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)pyrazolo[1,5-a]pyrimidine-3-carboxamide

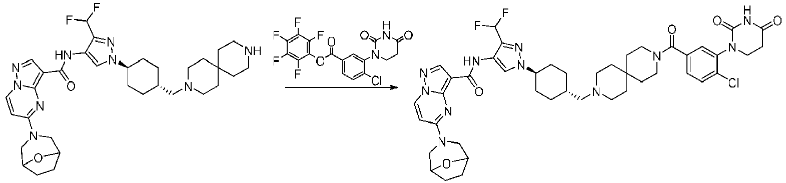

*N*-(1-((1*R*,4*R*)-4-((3,9-Diazaspiro[5.5]undecan-3-yl)methyl)cyclohexyl)-3-(diffuoromethyl)-1*H-*pyrazol-4-yl)-5-(8-oxa-3-azabicyclo[3.2.1]octan-3-yl)pyrazolo[1,5-*a*]pyrimidine-3-carboxamide (110 mg, 0.14 mmol) was dissolved in DMSO (5 mL). DIEA (90 mg, 0.70 mmol) and pentafluorophenyl 4-chloro-3-(2,4-dioxotetrahydropyrimidin-1(2H)-yl)benzoate (61 mg, 0.14 mmol) were added, and the mixture was reacted at room temperature for 2 h. Water (50 mL) was added, and the mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by preparative reversed-phase chromatography to give 5-(8-oxa-3-azabicyclo[3.2.1]octan-3-yl)-*N*-(1-((1*R*,4*R*)-4-((9-(4-chloro-3-(2,4-dioxotetrahydropyrimidin-1(2*H*)-yl)benzoyl)-3,9-diazaspiro[5.5]undecan-3-yl)methyl)cyclohexyl)-3-(diffuoromethyl)-1*H*-pyrazol-4-yl)pyrazolo[1,5-*a*]pyrimidine-3-carboxamide.

LC-MS: (ESI, m/z): [M+H]⁺ = 888.2.

¹H NMR (400 MHz, DMSO-*d*₆) δ 10.51 (s, 1H), 9.41 (s, 1H), 8.81 (d, *J* = 7.9 Hz, 1H), 8.37 (s, 1H), 8.28 (s, 1H), 7.64 (d, *J* = 8.2 Hz, 1H), 7.55 (d, *J* = 1.8 Hz, 1H), 7.39 (dd, *J* = 8.2, 1.9 Hz, 1H), 7.24-6.97 (m, 1H), 6.82 (d, *J* = 8.0 Hz, 1H), 4.47-4.41 (m, 2H), 4.31-3.93 (m, 3H), 3.81-3.72 (m, 1H), 3.67-3.51 (m, 3H), 3.25-3.15 (m, 2H), 2.77-2.72 (m, 2H), 2.35-2.25 (m, 4H), 2.15-2.07 (m, 2H), 2.16-2.98 (m, 2H), 1.96-1.29 (m, 19H), 1.10-0.92 (m, 2H).

### Example 33: 5-((1R,4R)-2-oxa-5-azabicyclo[2.2.1]heptan-5-yl)-N-(3-(difluoromethyl)-1-((1R,4R)-4-(((2-(1-(3-(2,4-dioxotetrahydropyrimidin-1(2H)-yl)-4-(trifluoromethoxy)benzoyl)piperidin-4-yl)ethyl)(methyl)amino)methyl)cyclohexyl)-1H-pyrazol-4-yl)pyrazolo[1,5-a]pyrimidine-3-carboxamide

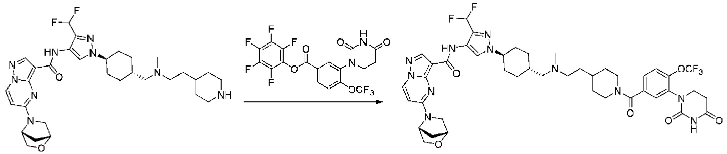

5-((1*R*,4*R*)-2-Oxa-5-azabicyclo[2.2.1]heptan-5-yl)-*N*-(3-(difluoromethyl)-1-((1*R*,4*R*)-4-((methyl(2-(piperidin-4-yl)ethyl)amino)methyl)cyclohexyl)-1*H*-pyrazol-4-yl)pyrazolo[1,5-*a*]pyrimidine-3-carboxamide (90 mg, 0.12 mmol) was dissolved in dimethyl sulfoxide (3 mL). Pentafluorophenyl 3-(2,4-dioxotetrahydropyrimidin-1(2H)-yl)-4-(trifluoromethoxy)benzoate (68 mg, 0.14 mmol) and *N,N-*diisopropylethylamine (46 mg, 0.36 mmol) were added while stirring. After the mixture was stirred at room temperature for 2 h, water (50 mL) was added. The mixture was filtered, and the filter cake was purified by pre-HPLC (CH₃CN/0.08% aqueous NH₄HCO₃ solution, 5%-95%) to give 5-((IR,4R)-2-oxa-5-azabicyclo[2.2.1]heptan-5-yl)-*N*-(3-(difluoromethyl)-1-((1*R*,4*R*)-4-(((2-(1-(3-(2,4-dioxotetrahydropyrimidin-1(2*H*)-yl)-4-(trifluoromethoxy)benzoyl)piperidin-4-yl)ethyl)(methyl)amino)methyl)cyclohexyl)-1*H*-pyrazol-4-yl)pyrazolo[1,5-*a*]pyrimidine-3-carboxamide.

LC-MS: (ESI, m/z): [M+H]⁺ = 912.4.

¹H NMR (400 MHz, DMSO-*d*₆) δ 10.54 (s, 1H), 9.50 (d, *J* = 6.1 Hz, 1H), 8.78 (d, *J* = 7.7 Hz, 1H), 8.39 (d, *J* = 4.2 Hz, 1H), 8.25 (t, *J* = 4.6 Hz, 1H), 7.60 (d, *J* = 1.9 Hz, 1H), 7.54-7.43 (m, 2H), 7.28-6.95 (m, 1H), 6.88-6.44 (m, 1H), 5.28-5.08 (m, 1H), 4.77 (d, *J* = 18.1 Hz, 1H), 4.52-4.40 (m, 1H), 4.24-4.15 (m, 1H), 3.86-3.42 (m, 7H), 3.10-2.95 (m, 1H), 2.80-2.60 (m, 3H), 2.30 (t, *J* = 7.0 Hz, 2H), 2.14-1.48 (m, 17H), 1.43-1.30 (m, 2H), 1.20-0.93 (m, 4H).

### Example 34: N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)-5-morpholinopyrazolo[1,5-a]pyrimidine-3-carboxamide

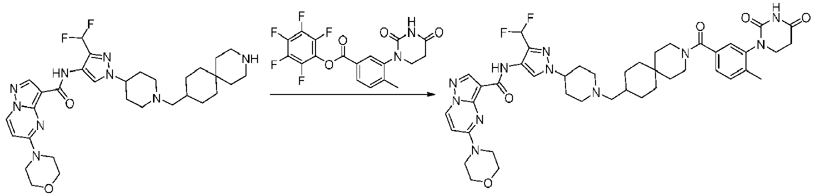

*N*-(1-(1-((3-Azaspiro[5.5]undecan-9-yl)methyl)piperidin-4-yl)-3-(difluoromethyl)-1*H*-pyrazol-4-yl)-5-morpholinopyrazolo[1,5-a]pyrimidine-3-carboxamide (100 mg, 0.12 mmol) was dissolved in dimethyl sulfoxide (3 mL). Pentafluorophenyl 3-(2,4-dioxotetrahydropyrimidin-1(2H)-yl)-4-methylbenzoate (58 mg, 0.14 mmol) and *N*,*N*-diisopropylethylamine (46 mg, 0.36 mmol) were added while stirring. After the mixture was stirred at room temperature for 2 h, water (50 mL) was added. The mixture was filtered, and the filter cake was purified by pre-HPLC (CH₃CN/0.08% aqueous NH₄HCO₃ solution, 5%-95%) to give N-(3-(difluoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H*-pyrazol-4-yl)-5-morpholinopyrazolo[1,5-*a*]pyrimidine-3-carboxamide.

LC-MS: (ESI, m/z): [M+H]⁺ = 842.4.

¹H NMR (400 MHz, DMSO-*d*₆) δ 10.37 (s, 1H), 9.40 (s, 1H), 8.83 (d, *J* = 7.9 Hz, 1H), 8.39 (s, 1H), 8.29 (s, 1H), 7.37-7.29 (m, 2H), 7.28-6.96 (m, 2H), 6.91 (d, *J* = 7.9 Hz, 1H), 4.25-4.15 (m, 1H), 3.83-3.63 (m, 9H), 3.61-3.47 (m, 3H), 3.32-3.24 (m, 2H), 2.95-2.65 (m, 4H), 2.25-1.85 (m, 11H), 1.70 (d, J = 9.3 Hz, 2H), 1.58-1.15 (m, 7H), 1.12-0.91 (m, 4H).

### Example 35: N-(3-(difluoromethyl)-1-((1R,4S)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)-5-((S)-3-hydroxypiperidin-1-yl)pyrazolo[1,5-a]pyrimidine-3-carboxamide

### Step 1: preparation of tert-butyl 4-(2-((((1S,4R)-4-(3-(difluoromethyl)-4-(5-((3S)-3-((tetrahydro-2H-pyran-2-yl)oxy)piperidin-1-yl)pyrazolo[1,5-a]pyrimidine-3-carboxamido)-1H-pyrazol-1-yl)cyclohexyl)methyl)(methyl)amino)ethyl)piperidine-1-carboxylate

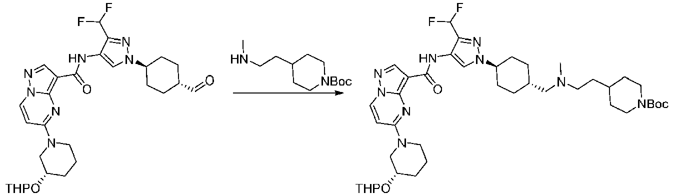

*N*-(3-(Difluoromethyl)-1-((1*R*,4*S*)-4-formylcyclohexyl)-1*H*-pyrazol-4-yl)-5-((3*S*)-3-((tetrahydro-2*H*-pyran-2-yl)oxy)piperidin-1-yl)pyrazolo[1,5-*a*]pyrimidine-3-carboxamide (120 mg, 0.21 mmol) and *tert-*butyl 4-(2-(methylamino)ethyl)piperidine-1-carboaylate (51 mg, 0.21 mmol) were dissolved in tetrahydrofuran (10 mL). Sodium triacetoxyborohydride (222 mg, 1.05 mmol) was added while stirring. The mixture was stirred at room temperature overnight. Water (50 mL) was added, and the mixture was extracted with dichloromethane (20 mL × 3). The organic phase was washed with saturated brine (60 mL), dried over anhydrous sodium sulfate, filtered, and concentrated under reduced pressure. The crude product was purified by column chromatography (methanol:dichloromethane = 1:9) to give tert-butyl 4-(2-((((1S,4R)-4-(3-(difluoromethyl)-4-(5-((3*S*)-3-((tetrahydro-2*H*-pyran-2-yl)oxy)piperidin-1-yl)pyrazolo[1,5-*a*]pyrimidine-3-carboxamido)-1*H*-pyrazol-1-yl)cyclohexyl)methyl)(methyl)amino)ethyl)piperidine-1-carboxylate.

LC-MS: (ESI, m/z): [M+H]⁺ = 798.5.

### Step 2: preparation of N-(3-(difluoromethyl)-1-((1R,4S)-4-((methyl(2-(piperidin-4-yl)ethyl)amino)methyl)cyclohexyl)-1H-pyrazol-4-yl)-5-((S)-3-hydroxypiperidin-1-yl)pyrazolo[1,5-a]pyrimidine-3-carboxamide

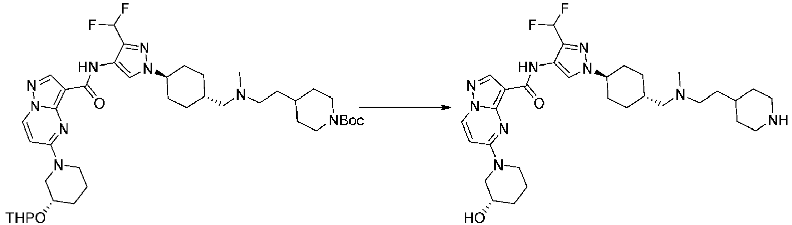

*tert*-Butyl 4-(2-((((1*S*,4*R*)-4-(3-(difluoromethyl)-4-(5-((3*S*)-3-((tetrahydro-2*H*-pyran-2-yl)oxy)piperidin-1-yl)pyrazolo[1,5-*a*]pyrimidine-3-carboxamido)-1*H*-pyrazol-1-yl)cyclohexyl)methyl)(methyl)amino)ethyl)piperidine-1-carboxylate (80 mg, 0.10 mmol) was dissolved in dichloromethane (3 mL). Trifluoroacetic acid (1 mL) was added, and the mixture was stirred at room temperature for 2 h. The reaction liquid was directly concentrated under reduced pressure to give *N*-(3-(difluoromethyl)-1-((1*R*,4*S*)-4-((methyl(2-(piperidin-4-yl)ethyl)amino)methyl)cyclohexyl)-1*H*-pyrazol-4-yl)-5-((*S*)-3-hydroxypiperidin-1-yl)pyrazolo[1,5-*a*]pyrimidine-3-carboxamide, which was directly used in the next step without purification.

LC-MS: (ESI, m/z): [M+H]⁺ = 614.3.

### Step 3: preparation of N-(3-(difluoromethyl)-1-((1R,4S)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)-5-((S)-3-hydroxypiperidin-1-yl)pyrazolo[1,5-a]pyrimidine-3-carboxamide

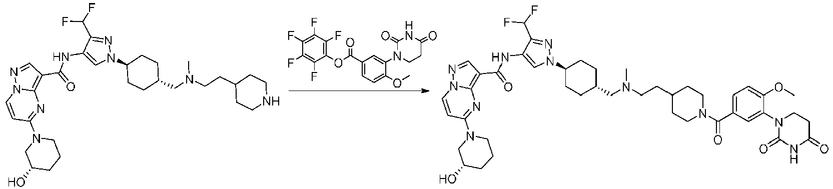

*N*-(3-(Difluoromethyl)-1-((1*R*,4*S*)-4-((methyl(2-(piperidin-4-yl)ethyl)amino)methyl)cyclohexyl)-1*H*-pyrazol-4-yl)-5-((*S*)-3-hydroxypiperidin-1-yl)pyrazolo[1,5-*a*]pyrimidine-3-carboxamide (80 mg, 0.10 mmol) was dissolved in dimethyl sulfoxide (3 mL). Pentafluorophenyl 3-(2,4-dioxotetrahydropyrimidin-1(2*H*)-yl)-4-methoxybenzoate (47 mg, 0.11 mmol) and *N*,*N*-diisopropylethylamine (38 mg, 0.30 mmol) were added while stirring. The mixture was stirred at room temperature overnight. Water (50 mL) was added. The mixture was filtered, and the filter cake was purified by pre-HPLC (CH₃CN/0.08% aqueous NH₄HCO₃ solution, 5%-95%) to give *N*-(3-(difluoromethyl)-1-((1*R*,4*S*)-4-(((2-(1-(3-(2,4-dioxotetrahydropyrimidin-1(2*H*)-yl)-4-methoxybenzoyl)piperidin-4-yl)ethyl)(methyl)amino)methyl)cyclohexyl)-1*H*-pyrazol-4-yl)-5-((*S*)-3-hydroxypiperidin-1-yl)pyrazolo[1,5-a]pyrimidine-3-carboxamide.

LC-MS: (ESI, m/z): [M+H]⁺ = 860.4.

¹H NMR (400 MHz, DMSO-*d*₆) δ 10.34 (s, 1H), 9.37 (s, 1H), 8.72 (d, *J* = 8.0 Hz, 1H), 8.37 (s, 1H), 8.24 (d, *J* = 5.1 Hz, 1H), 7.36 (dd, *J* = 8.5, 2.1 Hz, 1H), 7.32 (d, *J* = 2.1 Hz, 1H), 7.24-6.94 (m, 2H), 6.86 (d, *J* = 8.1 Hz, 1H), 4.89 (d, *J* = 4.1 Hz, 1H), 4.50-4.15 (m, 2H), 4.03-3.80 (m, 5H), 3.72-3.53 (m, 4H), 3.51-3.40 (m, 1H), 3.10-2.65 (m, 4H), 2.30 (t, *J* = 7.0 Hz, 2H), 2.15-1.95 (m, 7H), 1.92-1.25 (m, 15H), 1.17-0.95 (m, 4H).

### Example 36: 5-((S)-3-aminopiperidin-1-yl)-N-(3-(difluoromethyl)-1-((1R,4S)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)pyrazolo[1,5-a]pyrimidine-3-carboxamide

### Step 1: preparation of tert-butyl ((S)-1-(3-((3-(difluoromethyl)-1-((1R,4S)-4-(hydroxymethyl)cyclohexyl)-1H-pyrazol-4-yl)carbamoyl)pyrazolo[1,5-a]pyrimidin-5-yl)piperidin-3-yl)carboxylate

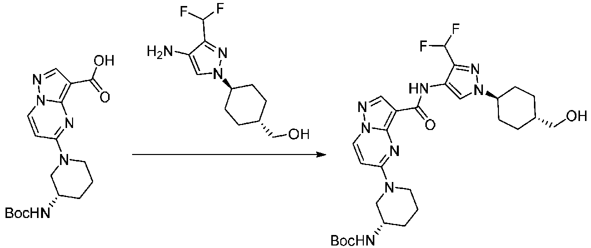

(S)-5-(3-((*tert*-Butoxycarbonyl)amino)piperidin-1-yl)pyrazolo[1,5-*a*]pyrimidine-3-carboxylic acid (150 mg, 0.42 mmol) was dissolved in DMF (10 mL). ((1*R*,4*R*)-4-(4-Amino-3-(difluoromethyl)-1*H*-pyrazol-1-yl)cyclohexyl)methanol (112 mg, 0.46 mmol), DIEA (163 mg, 1.26 mmol), and py-BOP (284 mg, 0.55 mmol) were added, and the mixture was reacted at room temperature for 2 h. Water (50 mL) was added, and the mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by column chromatography (EA:PE = 0-1) to give *tert-butyl* ((S)-1-(3-((3-(difluoromethyl)-1-((1*R*,4*S*)-4-(hydroxymethyl)cyclohexyl)-1H-pyrazol-4-yl)carbamoyl)pyrazolo[1,5-a]pyrimidin-5-yl)piperidin-3-yl)carboxylate.

LC-MS: (ESI, m/z): [M+H]⁺ = 589.3.

### Step 2: preparation of tert-butyl ((S)-1-(3-((3-(difluoromethyl)-1-((1R,4S)-4-formylcyclohexyl)-1H-pyrazol-4-yl)carbamoyl)pyrazolo[1,5-a]pyrimidin-5-yl)piperidin-3-yl)carboxylate

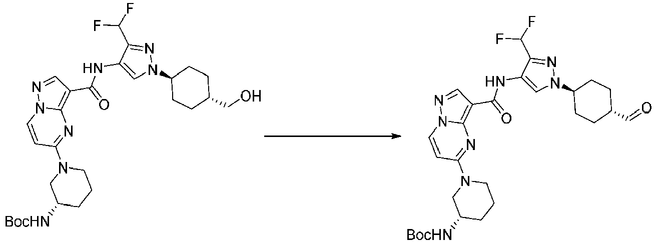

*tert*-Butyl ((*S*)-1-(3-((3-(difluoromethyl)-1-((1*R*,4*S*)-4-(hydroxymethyl)cyclohexyl)-1*H*-pyrazol-4-yl)carbamoyl)pyrazolo[1,5-*a*]pyrimidin-5-yl)piperidin-3-yl)carboxylate (190 mg, 0.32 mmol) was dissolved inACN (10 mL). IBX (181 mg, 0.65 mmol) was added, and the mixture was reacted at 85 °C for 1.5 h. Water (30 mL) was added to the reaction liquid, and the mixture was extracted with ethyl acetate (30 mL × 3). The organic phase was washed with saturated brine (50 mL), dried over anhydrous sodium sulfate, filtered, and concentrated under reduced pressure. The crude product was purified by column chromatography (EA:PE = 0-1) to give *tert*-butyl ((*S*)-1-(3-((3-(difluoromethyl)-1-((1*R*,4*S*)-4-formylcyclohexyl)-1*H*-pyrazol-4-yl)carbamoyl)pyrazolo[1,5-*a*]pyrimidin-5-yl)piperidin-3-yl)carboxylate.

LC-MS: (ESI, m/z): [M+H]⁺ = 586.6.

### Step 3: preparation of N-benzyl-N-methyl-2-(piperidin-4-yl)ethan-l-amine

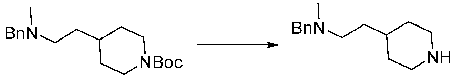

*tert*-Butyl 4-(2-(benzyl(methyl)amino)ethyl)piperidine-1-carboxylate (345 mg, 1.04 mmol) was dissolved in DCM (5 mL). TFA (1 mL) was added, and the mixture was reacted at room temperature for 1 h. The reaction liquid was directly concentrated under reduced pressure to give *N*-benzyl-*N*-methyl-2-(piperidin-4-yl)ethan-1-amine, which was directly used in the next step.

LC-MS: (ESI, m/z): [M+H]⁺ = 233.2.

### Step 5: preparation of 1-(5-(4-(2-(benzyl(methyl)amino)ethyl)piperidine-1-carbonyl)-2-methoxyphenyl)dihydropyrimidine-2,4(1H,3H)-dione

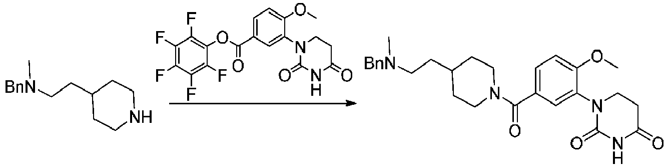

*N*-Benzyl-*N*-methyl-2-(piperidin-4-yl)ethan-1-amine (250 mg, 1.04 mmol) was dissolved in DMSO (10 mL). DIEA (671 mg, 5.2 mmol) and pentafluorophenyl 3-(2,4-dioxotetrahydropyrimidin-1(2*H*)-yl)-4-methoxybenzoate (451 mg, 1.04 mmol) were added, and the mixture was reacted at room temperature for 2 h. Water (100 mL) was added to the reaction liquid, and the mixture was stirred for 5 min and extracted with ethyl acetate (100 mL × 3). The organic phase was washed with saturated brine (150 mL), dried over anhydrous sodium sulfate, filtered, and concentrated under reduced pressure. The crude product was purified by column chromatography (MeOH:DCM = 0-1:9) to give 1-(5-(4-(2-(benzyl(methyl)amino)ethyl)piperidine-1-carbonyl)-2-methoxyphenyl)dihydropyrimidine-2,4(1*H*,3*H*)-dione.

LC-MS: (ESI, m/z): [M+H]¹ = 479.2.

### Step 6: preparation of 1-(2-methoxy-5-(4-(2-(methylamino)ethyl)piperidine-1-carbonyl)phenyl)dihydropyrimidine-2,4(1H,3H)-dione

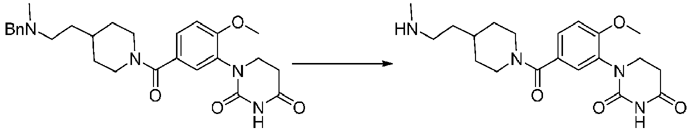

1-(5-(4-(2-(Benzyl(methyl)amino)ethyl)piperidine-1-carbonyl)-2-methoxyphenyl)dihydropyrimidine-2,4(1*H*,3*H*)-dione (240 mg, 0.5 mmol) was dissolved in MeOH (10 mL). Pd/C (72 mg, 30%) was added, and the mixture was reacted at room temperature for 2 h. The mixture was filtered and concentrated under reduced pressure to give 1-(2-methoxy-5-(4-(2-(methylamino)ethyl)piperidine-1-carbonyl)phenyl)dihydropyrimidine-2,4(1*H*,3*H*)-dione, which was directly used in the next step.

LC-MS: (ESI, m/z): [M+Na]⁺ = 411.1.

### Step 7: preparation of tert-butyl ((S)-1-(3-((3-(difluoromethyl)-1-((1R,4S)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)carbonyl)pyrazolo[1,5-a]pyrimidin-5-yl)piperidin-3-yl)carboxylate

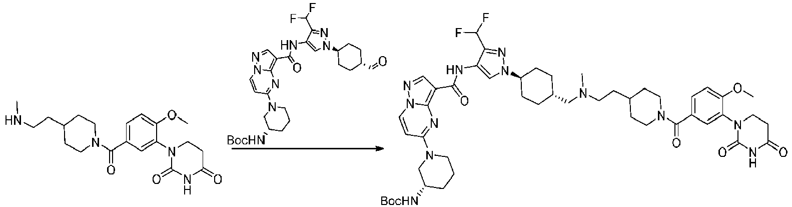

1-(2-Methoxy-5-(4-(2-(methylamino)ethyl)piperidine-1-carbonyl)phenyl)dihydropyrimidine-2,4(1*H*,3*H*)-dione (95 mg, 0.24 mmol) was dissolved in THF (10 mL). *tert*-Butyl ((*S*)-1-(3-((3-(difluoromethyl)-1-((1*R*,4*S*)-4-formylcyclohexyl)-1*H*-pyrazol-4-yl)carbamoyl)pyrazolo[1,5-*a*]pyrimidin-5-yl)piperidin-3-yl)carboxylate (120 mg, 0.20 mmol) and STAB (127 mg, 0.60 mmol) were added, and the mixture was reacted at room temperature for 2 h. The reaction liquid was diluted with water (50 mL), and the mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 0-1:9) to give tert-butyl ((*S*)-1-(3-((3-(difluoromethyl)-1-((1*R*,4*S*)-4-(((2-(1-(3-(2,4-dioxotetrahydropyrimidin-1(2*H*)-yl)-4-methoxybenzoyl)piperidin-4-yl)ethyl)(methyl)amino)methyl)cyclohexyl)-1H-pyrazol-4-yl)carbonyl)pyrazolo[1,5-*a*]pyrimidin-5-yl)piperidin-3-yl)carboxylate.

LC-MS: (ESI, m/z): [M+H]⁺ = 959.4.

### Step 8: preparation of 5-((S)-3-aminopiperidin-1-yl)-N-(3-(difluoromethyl)-1-((1R,4S)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)pyrazolo[1,5-a]pyrimidine-3-carboxamide

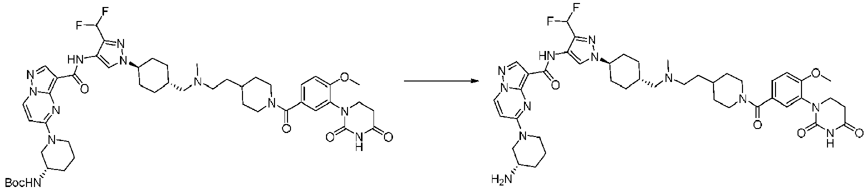

*tert*-Butyl ((*S*)-1-(3-((3-(difluoromethyl)-1-((1*R*,4*S*)-4-(((2-(1-(3-(2,4-dioxotetrahydropyrimidin-1(2*H*)-yl)-4-methoxybenzoyl)piperidin-4-yl)ethyl)(methyl)amino)methyl)cyclohexyl)-1*H*-pyrazol-4-yl)carbonyl)pyrazolo[1,5-a]pyrimidin-5-yl)piperidin-3-yl)carboxylate (100 mg, 0.10 mmol) was dissolved in DCM (5 mL). TFA (1 mL) was added, and the mixture was reacted at room temperature for 1 h. The reaction liquid was directly concentrated under reduced pressure, and the crude product was purified by preparative reversed-phase chromatography to give 5-((*S*)-3-aminopiperidin-1-yl)-*N*-(3-(difluoromethyl)-1-((1*R*,4*S*)-4-(((2-(1-(3-(2,4-dioxotetrahydropyrimidin-1(2*H*)-yl)-4-methoxybenzoyl)piperidin-4-yl)ethyl)(methyl)amino)methyl)cyclohexyl)-1*H*-pyrazol-4-yl)pyrazolo[1,5-*a*]pyrimidine-3-carboxamide.

LC-MS: (ESI, m/z): [M+H]⁺ = 859.4.

¹H NMR(400 MHz, DMSO-*d*₆) δ 10.33 (s, 1H), 9.36 (s, 1H), 8.74 (d, *J* = 8.0 Hz, 1H), 8.38 (s, 1H), 8.24 (s, 1H), 7.36 (dd, *J* = 8.4, 2.1 Hz, 1H), 7.32 (d, *J* = 2.1 Hz, 1H), 7.27-6.97 (m, 2H), 6.88 (d, *J* = 8.1 Hz, 1H), 4.52-4.15 (m, 4H), 3.84 (s, 3H), 3.59 (t, *J* = 6.6 Hz, 2H), 3.24-2.93 (m, 3H), 2.80-2.60 (m, 4H), 2.40-2.25 (m, 2H), 2.16-1.99 (m, 8H), 1.95-1.80 (m, 3H), 1.80-1.29 (m, 13H), 1.17-0.96 (m, 4H).

### Example 37: N-(3-(difluoromethyl)-1-(1-(2-(1-(3-(2,4-dioxotetrahydropyrimidin-1(2H)-yl)-4-fluorobenzoyl)piperidin-4-yl)ethyl)piperidin-4-yl)-1H-pyrazol-4-yl)-5-morpholinopyrazolo[1,5-a]pyrimidine-3-carboxamide

### Step 1: preparation of tert-butyl 4-(2-(4-(3-(difluoromethyl)-4-(5-morpholinopyrazolo[1,5-a]pyrimidine-3-carboxamido)-1H-pyrazol-1-yl)piperidin-1-yl)ethyl)piperidine-1-carboxylate

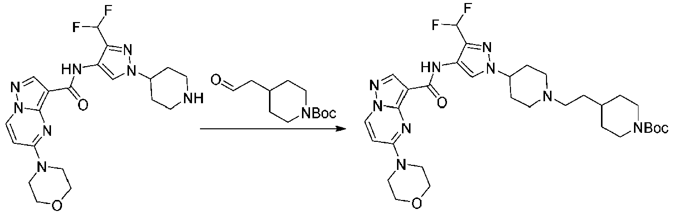

*N*-(3-(Difluoromethyl)-1-(piperidin-4-yl)-1*H*-pyrazol-4-yl)-5-morpholinopyrazolo[1,5-*a*]pyrimidine-3-carboxamide (125 mg, 0.280 mmol) was added to a solution of *tert-butyl* 4-(2-oxoethyl)piperidine-1-carboxylate (95.466 mg, 0.421 mmol) in THF (3 mL). STAB (178.5 mg, 0.842 mmol) was added, and the mixture was stirred at room temperature for 2 h. The reaction liquid was diluted with water (20 mL), and the mixture was extracted with ethyl acetate (25 mL × 4), washed with saturated brine, dried over anhydrous sodium sulfate, concentrated under reduced pressure, and purified by normal phase column chromatography (EA:PE = 3:7) to give *tert-butyl* 4-(2-(4-(3-(difluoromethyl)-4-(5-morpholinopyrazolo[1,5-*a*]pyrimidine-3-carboxamido)-1*H*-pyrazol-1-yl)piperidin-1-yl)ethyl)piperidine-1-carboxylate.

LC-MS: (ESI, m/z): [M+H]⁺ = 658.5.

### Step 2: preparation of N-(3-(difluoromethyl)-1-(1-(2-(piperidin-4-yl)ethyl)piperidin-4-yl)-1H-pyrazol-4-yl)-5-morpholinopyrazolo[1,5-a]pyrimidine-3-carboxamide

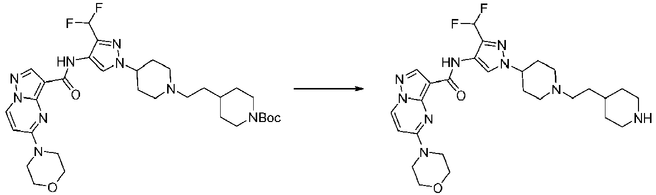

*tert*-Butyl 4-(2-(4-(3-(difluoromethyl)-4-(5-morpholinopyrazolo[1,5-*a*]pyrimidine-3-carboxamido)-1*H*-pyrazol-1-yl)piperidin-1-yl)ethyl)piperidine-1-carboxylate (160 mg, 0.347 mmol) was added to TFA (1 mL) and DCM (3 mL), and the mixture was stirred at room temperature for 2 h. The reaction liquid was directly concentrated under reduced pressure to give a crude product of *N*-(3-(difluoromethyl)-1-(1-(2-(piperidin-4-yl)ethyl)piperidin-4-yl)-1*H*-pyrazol-4-yl)-5-morpholinopyrazolo[1,5-*a*]pyrimidine-3-carboxamide.

LC-MS: (ESI, m/z): [M+H]⁺ = 558.4.

### Step 3: preparation of N-(3-(difluoromethyl)-1-(1-(2-(1-(3-(2,4-dioxotetrahydropyrimidin-1(2H)-yl)-4-fluorobenzoyl)piperidin-4-yl)ethyl)piperidin-4-yl)-1H-pyrazol-4-yl)-5-morpholinopyrazolo[1,5-a]pyrimidine-3-carboxamide

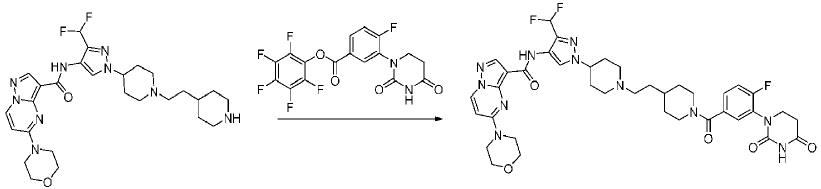

*N*-(3-(Difluoromethyl)-1-(1-(2-(piperidin-4-yl)ethyl)piperidin-4-yl)-1*H*-pyrazol-4-yl)-5-morpholinopyrazolo[1,5-*a*]pyrimidine-3-carboxamide (121 mg, 0.217 mmol) was added to a solution of pentafluorophenyl 3-(2,4-dioxotetrahydropyrimidin-1(2*H*)-yl)-4-fluorobenzoate (90.8 mg, 0.217 mmol) in DMSO (5 mL). DIEA (84.1 mg, 0.652 mmol) was added, and the mixture was stirred at room temperature for 16 h. The reaction liquid was diluted with water (40 mL), and the mixture was extracted with ethyl acetate (40 mL × 4), washed with saturated brine, dried over anhydrous sodium sulfate, and concentrated under reduced pressure. The crude product was purified by preparative reversed-phase chromatography (acetonitrile-0.05% aqueous NH₄HCO₃ solution, 5%-95%) to give the product *N*-(3-(difluoromethyl)-1-(1-(2-(1-(3-(2,4-dioxotetrahydropyrimidin-1(2*H*)-yl)-4-fluorobenzoyl)piperidin-4-yl)ethyl)piperidin-4-yl)-1*H*-pyrazol-4-yl)-5-morpholinopyrazolo[1,5-*a*]pyrimidine-3-carboxamide.

LC-MS: (ESI, m/z): [M+H]⁺ = 792.3.

¹H NMR (400 MHz, DMSO-*d*₆) δ 10.53 (s, 1H), 9.40 (s, 1H), 8.82 (s, 1H), 8.39 (s, 1H), 8.29 (s, 1H), 7.59-7.27 (m, 3H), 7.24-6.83 (m, 2H), 4.51-4.15 (m, 2H), 3.83-3.55 (m, 11H), 3.12-2.85 (m, 3H), 2.81-2.64 (m, 3H), 2.38-2.30 (m, 2H), 2.08-1.55 (m, 9H), 1.46-1.31 (m, 2H), 1.20-0.99 (m, 2H).

### Example 38: N(1-((1R,4R)-4-((4-((3-(4-chloro-3-(2,4-dioxotetrahydropyrimidin-1(2H)-yl)phenyl)prop-2-yn-1-yl)oxy)piperidin-1-yl)methyl)cyclohexyl)-3-(difluoromethyl)-1H-pyrazol-4-yl)-5-morpholinopyrazolo[1,5-a]pyrimidine-3-carboxamide

### Step 1: preparation of tert-butyl 4-(prop-2-yn-1-yloxy)piperidine-1-carboxylate

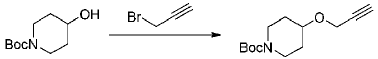

NaH (2.98 g, 124.2 mmol) was slowly added to tert-butyl 4-hydroxypiperidine-1-carboxylate (5 g, 24.84 mmol) in anhydrous THF (60 mL) at 0 °C, and the mixture was stirred for 30 min. Then 3-bromoprop-1-yne (5.91 g, 49.68 mmol) was added. The reaction liquid was slowly heated to room temperature, stirred for 5 h, and then filtered. Water (100 mL) was added, and the mixture was extracted with ethyl acetate (60 mL × 4), washed with saturated brine, dried over anhydrous sodium sulfate, concentrated under reduced pressure, and purified by normal phase column chromatography (EA:PE = 1:9) to give tert-butyl 4-(prop-2-yn-1-yloxy)piperidine-1-carboxylate.

LC-MS: (ESI, m/z): [M-56]⁺ = 184. 1.

### Step 2: preparation of tert-butyl 4-((3-(4-chloro-3-(2,4-dioxotetrahydropyrimidin-1(2H)-yl)phenyl)prop-2-yn-1-yl)oxy)piperidine-1-carboxylate

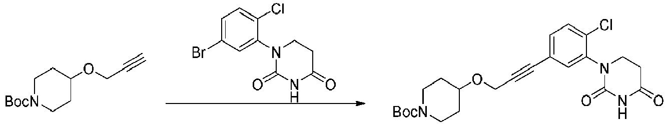

tert-Butyl 4-(prop-2-yn-1-yloacy)piperidine-1-carboxylate (232 mg, 0.971 mmol) was added to 1-(5-bromo-2-chlorophenyl)dihydropyrimidine-2,4(1*H*,3*H*)-dione (350 mg, 1.164 mmol) in anhydrous DMF (10 mL). CuI (47.5 mg, 0.146 mmol), CsCO₃ (948 mg, 2.913 mmol), and Pd(PPh₃)₂Cl₂ (102.1 mg, 0.146 mmol) were added, and the reaction liquid was stirred at 80 °C for 4 h under N₂ atmosphere. Water (40 mL) was added to the reaction liquid, and the mixture was extracted with ethyl acetate (40 mL × 4), washed with saturated brine, dried over anhydrous sodium sulfate, concentrated under reduced pressure, and purified by normal phase column chromatography (EA:PE = 2:3) to give tert-butyl 4-((3-(4-chloro-3-(2,4-dioxotetrahydropyrimidin-1(2*H*)-yl)phenyl)prop-2-yn-1-yl)oxy)piperidine-1 -carboxylate.

LC-MS: (ESI, m/z): [M+H]⁺ = 462.2.

### Step 3: preparation of 1-(2-chloro-5-(3-(piperidin-4-yloxy)prop-1-yn-1-yl)phenyl)dihydropyrimidine-2,4(1H,3H)-dione

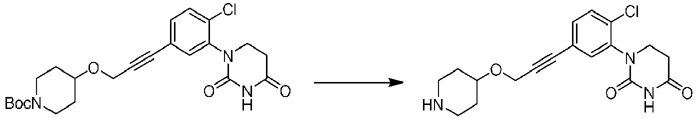

*tert*-Butyl 4-((3-(4-chloro-3-(2,4-dioxotetrahydropyrimidin-1(2*H*)-yl)phenyl)prop-2-yn-1-y))oxy)piperidine-1-carboxylate (160 mg, 0.347 mmol) was added to TFA (1 mL) and DCM (3 mL), and the mixture was stirred at room temperature for 2 h. The reaction liquid was directly concentrated under reduced pressure to give a crude product of 1-(2-chloro-5-(3-(piperidin-4-yloxy)prop-1-yn-1-yl)phenyl)dihydropyrimidine-2,4(1*H*,3*H*)-dione.

LC-MS: (ESI, m/z): [M+H]⁺ = 362.1.

### Step 4: preparation of N(1-((1R,4R)-4-((4-((3-(4-chloro-3-(2,4-dioxotetrahydropyrimidin-1(2H)-yl)phenyl)prop-2-yn-1-yl)oxy)piperidin-1-yl)methyl)cyclohexyl)-3-(difluoromethyl)-1H-pyrazol-4-yl)-5-morpholinopyrazolo[1,5-a]pyrimidine-3-carboxamide

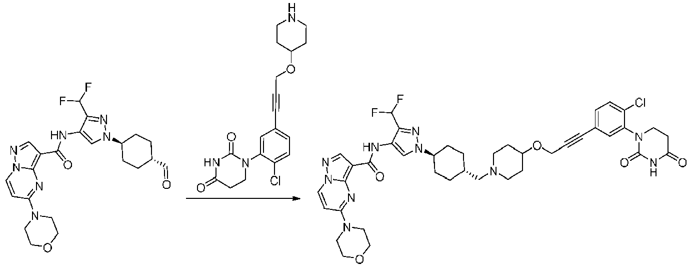

1-(2-Chloro-5-(3-(piperidin-4-yloxy)prop-1-yn-1-yl)phenyl)dihydropyrimidine-2,4(1*H*,3*H*)-dione (137.4 mg, 0.381 mmol) and DIEA (73.6 mg, 0.570 mmol) were added to THF (5 mL), and the mixture was stirred at room temperature for 10 min. Then *N*-(3-(difluoromethyl)-1-((1*R*,4*R*)-4-(formyl)cyclohexyl)-1*H-*pyrazol-4-yl)-5-morpholinopyrazolo[1,5-*a*]pyrimidine-3-carboxamide (90 mg, 0.190 mmol) was added, and the mixture was stirred for 10 min. Then STAB (120.9 mg, 0.914 mmol) was added, and the reaction liquid was stirred at room temperature for 2 h. Water (20 mL) was added to the reaction liquid, and the mixture was extracted with ethyl acetate (25 mL × 4), washed with saturated brine, dried over anhydrous sodium sulfate, and concentrated under reduced pressure. The crude product was purified by preparative reversed-phase chromatography (acetonitrile-0.05% aqueous FA solution, 5%-95%) to give *N*-(1-((1R,4R)-4-((4-((3-(4-chloro-3-(2,4-dioxotetrahydropyrimidin-1(2*H*)-yl)phenyl)prop-2-yn-1-yl)oxy)piperidin-1-yl)methyl)cyclohexyl)-3-(difluoromethyl)-1*H*-pyrazol-4-yl)-5-morpholinopyrazolo[1,5-*a*]pyrimidine-3-carboxamide.

LC-MS: (ESI, m/z): [M+H]⁺ = 819.3.

¹H NMR (400 MHz, DMSO-*d*₆) δ 10.51 (s, 1H), 9.40 (s, 1H), 8.83 (d, *J* = 7.9 Hz, 1H), 8.38 (s, 1H), 8.29 (s, 1H), 7.67-7.54 (m, 2H), 7.48-7.41 (m, 1H), 7.27-6.94 (m, 1H), 6.92-6.86 (m, 1H), 4.43 (s, 2H), 4.23-4.13 (m, 1H), 3.86-3.65 (m, 10H), 3.64-3.54 (m, 2H), 2.77-2.65 (m, 4H), 2.10-1.66 (m, 11H), 1.63-1.40 (m, 3H), 1.13-0.93 (m, 2H).

### Example 39: N-(3-(difluoromethyl)-1-((1R,4R)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)-5-(2-oxa-6-azaspiro[3.3]heptan-6-yl)pyrazolo[1,5-a]pyrimidine-3-carboxamide

### Step 1: preparation of tert-butyl 4-(2-(((benzyloxy)carbonyl)(methyl)amino)ethyl)piperidine-1-carboxylate

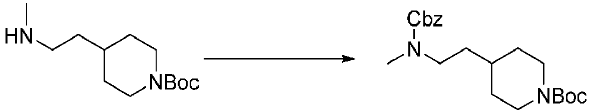

tert-Butyl 4-(2-(methylamino)ethyl)piperidine-1-carboxylate (200 mg, 0.82 mmol) was dissolved in dichloromethane (4 mL). Triethylamine (249, 2.46 mmol) and CbzCl (139 mg, 0.82 mmol) were added, and the reaction liquid was stirred at room temperature for 1 h. The reaction liquid was diluted with water (50 mL), and the mixture was extracted with dichloromethane (80 mL × 3), dried over anhydrous sodium sulfate, and concentrated under reduced pressure. The crude product was purified by column chromatography (PE:EA = 3:7) to give the compound *tert*-butyl 4-(2-(((benzyloxy)carbonyl)(methyl)amino)ethyl)piperidine-1-carboxylate.

LC-MS: (ESI, m/z): [M+H-100]⁺ = 277.2.

### Step 2: preparation of benzyl methyl(2-(piperidin-4-yl)ethyl)carbamate

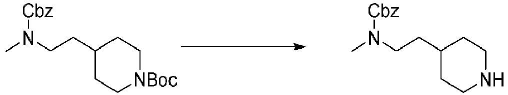

The compound *tert-*butyl 4-(2-(((benzyloxy)carbonyl)(methyl)amino)ethyl)piperidine-1-carboxylate (200 mg, 0.53 mmol) was dissolved in a hydrogen chloride/1,4-dioxane (4 mL) solution, and the reaction liquid was stirred at room temperature for 1 h. The reaction liquid was directly concentrated under reduced pressure to give benzyl methyl(2-(piperidin-4-yl)ethyl)carbamate.

LC-MS: (ESI, m/z): [M+H]⁺ = 277.2.

### Step 3: preparation of benzyl (2-(1-(3-(2,4-dioxotetrahydropyrimidin-1(2H)-yl)-4-methoxybenzoyl)piperidin-4-yl)ethyl)(methyl)carbamate

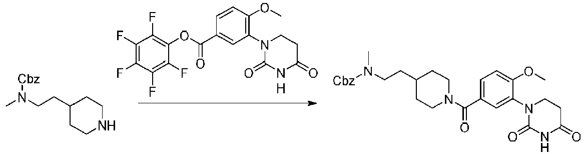

The compounds benzyl methyl(2-(piperidin-4-yl)ethyl)carbamate (200 mg, 0.72 mmol) and pentafluorophenyl 3-(2,4-dioxotetrahydropyrimidin-1(2*H*)-yl)-4-methoxybenzoate (309 mg, 0.72 mmol) were added to a DMSO (4 mL) solution. Then DIEA (278 mg, 2.1 mmol) was added, and the reaction liquid was stirred at room temperature for 3 h. The reaction liquid was diluted with water (50 mL), and the mixture was extracted with ethyl acetate (70 mL × 4), washed with saturated brine, dried over anhydrous sodium sulfate, and concentrated under reduced pressure. The crude product was purified by column chromatography (PE:EA = 1:3) to give the compound benzyl (2-(1-(3-(2,4-dioxotetrahydropyrimidin-1(2*H*)-yl)-4-methoxybenzoyl)piperidin-4-yl)ethyl)(methyl)carbamate.

LC-MS: (ESI, m/z): [M+H]⁺ = 523.2.

### Step 4: preparation of 1-(2-methoxy-5-(4-(2-(methylamino)ethyl)piperidine-1-carbonyl)phenyl)dihydropyrimidine-2,4(1H,3H)-dione

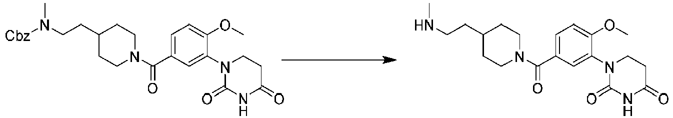

The compound benzyl (2-(1-(3-(2,4-dioxotetrahydropyrimidin-1(2*H*)-yl)-4-methoxybenzoyl)piperidin-4-yl)ethyl)(methyl)carbamate (120 mg, 0.23 mmol) was dissolved in a methanol (3 mL) solution. Palladium on carbon (10%, 24 mg) was added, and the reaction liquid was stirred at room temperature for 1 h. The reaction liquid was filtered under vacuum through celite, and the filtrate was concentrated under reduced pressure to give the compound 1-(2-methoxy-5-(4-(2-(methylamino)ethyl)piperidine-1-carbonyl)phenyl)dihydropyrimidine-2,4(1H,3H)-dione.

LC-MS: (ESI, m/z): [M+H]⁺ = 389.2.

### Step 5: preparation of N-(3-(difluoromethyl)-1-((1R,4R)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)-5-(2-oxa-6-azaspiro[3.3]heptan-6-yl)pyrazolo[1,5-a]pyrimidine-3-carboxamide

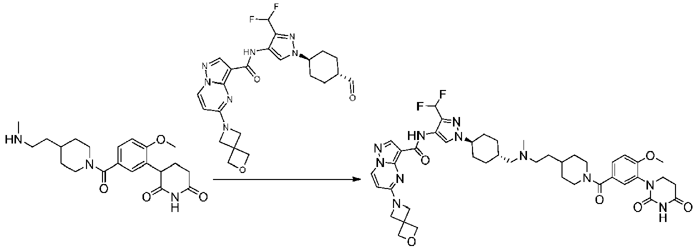

The compounds *N*-(3-(difluoromethyl)-1-((1*R*,4*R*)-4-formylcyclohexyl)-1*H*-pyrazol-4-yl)-5-(2-oxa-6-azaspiro[3.3]heptan-6-yl)pyrazolo[1,5-a]pyrimidine-3-carboxamide (80 mg, 0.165 mmol) and 1-(2-methoxy-5-(4-(2-(methylamino)ethyl)piperidine-1-carbonyl)phenyl)dihydropyrimidine-2,4(1*H*,3*H*)-dione (64 mg, 0.165 mmol) were dissolved in DCE (3 mL). STAB (70 mg, 0.330 mmol) was added, and the reaction liquid was stirred at room temperature for 2 h. The reaction liquid was diluted with water (20 mL), and the mixture was extracted with ethyl acetate (25 mL × 4), washed with saturated brine, dried over anhydrous sodium sulfate, and concentrated under reduced pressure. The crude product was purified by preparative reversed-phase chromatography to give the compound *N*-(3-(difluoromethyl)-1-((1*R*,4*R*)-4-(((2-(1-(3-(2,4-dioxotetrahydropyrimidin-1(2*H*)-yl)-4-methoxybenzoyl)piperidin-4-yl)ethyl)(methyl)amino)methyl)cyclohexyl)-1H-pyrazol-4-yl)-5-(2-oxa-6-azaspiro[3.3]heptan-6-yl)pyrazolo[1,5-*a*]pyrimidine-3-carboxamide.

LC-MS: (ESI, m/z): [M+H]⁺ = 858.4.

¹H NMR (400 MHz, DMSO-*d*₆) δ 10.34 (s, 1H), 9.69 (s, 1H), 8.76 (d, *J* = 7.6 Hz, 1H), 8.37 (s, 1H), 8.25 (s, 1H), 7.35 (s, 1H), 7.42-7.00 (m, 3H), 6.38 (d, *J* = 7.6 Hz, 1H), 4.78-4.70 (m, 4H), 4.48-4.35 (m, 4H), 4.25-4.10 (m, 1H), 3.84 (s, 3H), 3.59 (t, *J* = 5.0 Hz, 2H), 3.30-3.10 (m, 3H), 2.75-2.63 (m, 2H), 2.36-2.25 (m, 2H), 2.20-1.99 (m, 7H), 1.93-1.83 (m, 2H), 1.77-1.50 (m, 7H), 1.44-1.30 (m, 2H), 1.20-0.96 (m, 4H).

### Example 40: 5-((1R,4R)-2-oxa-5-azabicyclo[2.2.1]heptan-5-yl)-N-(1-(1-(4-(4-(4-chloro-3-(2,4-dioxotetrahydropyrimidin-1(2H)-yl)benzoyl)piperazin-1-yl)ₙ-butyl)piperidin-4-yl)-3-(difluoromethyl)-1H-pyrazol-4-yl)pyrazolo[1,5-a]pyrimidine-3-carboxamide

### Step 1: preparation of tert-butyl 4-(4-chloro-3-(2,4-dioxotetrahydropyrimidin-1(2H)-yl)benzoyl)piperazine-1-carboxylate

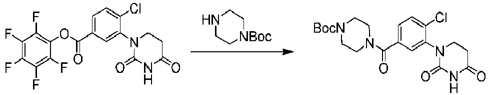

Pentafluorophenyl 4-chloro-3-(2,4-dioxotetrahydropyrimidin-1(2H)-yl)benzoate (450 mg, 1.037 mmol) and tert-butyl piperazine-1-carboxylate (386.28 mg, 2.074 mmol) were added to DMSO (5 mL). Then DIEA (401.31 mg, 3.111 mmol) was added, and the mixture was stirred at room temperature for 2 h. Water (20 mL) was added to the reaction liquid, and the mixture was extracted with ethyl acetate (25 mL × 4), washed with saturated brine, dried over anhydrous sodium sulfate, concentrated under reduced pressure, and purified by normal phase column chromatography (MeOH:DCM = 1:19) to give *tert*-butyl 4-(4-chloro-3-(2,4-dioxotetrahydropyrimidin-1(2*H*)-yl)benzoyl)piperazine-1-carboxylate.

LC-MS: (ESI, m/z): [M+Na]⁺ = 459.1

### Step 2: preparation of 1-(2-chloro-5-(piperazine-1-carbonyl)phenyl)dihydropyrimidine-2,4(1H,3H)-dione

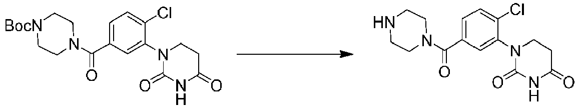

*tert*-Butyl 4-(4-chloro-3-(2,4-dioxotetrahydropyrimidin-1(2*H*)-yl)benzoyl)piperazine-1-carboxylate (402 mg, 0.922 mmol) was added to TFA (0.5 mL) and DCM (2.5 mL), and the mixture was stirred at room temperature for 2 h. The reaction liquid was directly concentrated under reduced pressure to give a crude product of 1-(2-chloro-5-(piperazine-1-carbonyl)phenyl)dihydropyrimidine-2,4(1*H*,3*H*)-dione.

LC-MS: (ESI, m/z): [M+H]⁺ = 337.0.

### Step 3: preparation of 1-(5-(4-(3-(1,3-dioxolan-2-yl)propyl)piperazine-1-carbonyl)-2-chlorophenyl)dihydropyrimidine-2,4(1H,3H)-dione

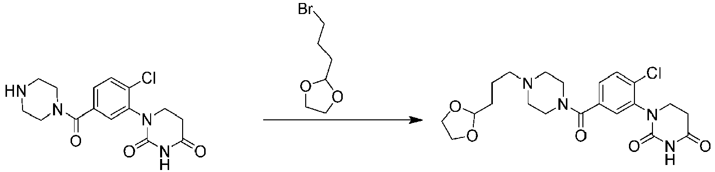

1-(2-Chloro-5-(piperazine-1-carbonyl)phenyl)dihydropyrimidine-2,4(1H,3H)-dione (295 mg, 0.878 mmol) and 2-(3-bromopropyl)-1,3-dioxolane (256.9 mg, 1.316 mmol) were added to DMF (5 mL). Then CsCO₃ (858.2 mg, 2.634 mmol) was added, and the reaction liquid was stirred at 100 °C for 16 h. Water (40 mL) was added to the reaction liquid, and the mixture was extracted with ethyl acetate (30 mL × 4), washed with saturated brine, dried over anhydrous sodium sulfate, concentrated under reduced pressure, and purified by normal phase column chromatography (MeOH:DCM = 1:11) to give 1-(5-(4-(3-(1,3-dioxolan-2-yl)propyl)piperazine-1-carbonyl)-2-chlorophenyl)dihydropyrimidine-2,4(1*H*,3*H*)-dione.

LC-MS: (ESI, m/z): [M+H]⁺ = 451.1.

### Step 4: preparation of 4-(4-(4-chloro-3-(2,4-dioxotetrahydropyrimidin-1(2H)-yl)benzoyl)piperazin-1-yl)ₙ-butyraldehyde

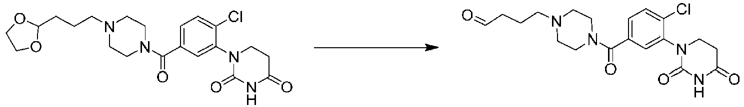

1-(5-(4-(3-(1,3-Dioxolan-2-yl)propyl)piperazine-1-carbonyl)-2-chlorophenyl)dihydropyrimidine-2,4(1H,3H)-dione (100 mg, 0.22 mmol) was dissolved in DCM (0.5 mL). TFA (0.5 mL) was added, and the mixture was reacted at room temperature for 48 h. DCE (10 mL) and DIEA (1 mL) were added to the reaction liquid, and the mixture was concentrated under reduced pressure at room temperature to give 4-(4-(4-chloro-3-(2,4-dioxotetrahydropyrimidin-1(2*H*)-yl)benzoyl)piperazin-1-yl)*ₙ*-butyraldehyde, which was directly used in the next step.

LC-MS: (ESI, m/z): [M+H]⁺ = 407.1.

### Step 5: preparation of 5-((1R,4R)-2-oxa-5-azabicyclo[2.2.1]heptan-5-yl)-N-(1-(1-(4-(4-(4-chloro-3-(2,4-dioxotetrahydropyrimidin-1(2H)-yl)benzoyl)piperazin-1-yl)n-butyl)piperidin-4-yl)-3-(difluoromethyl)-1H-pyrazol-4-yl)pyrazolo[1,5-a]pyrimidine-3-carboxamide

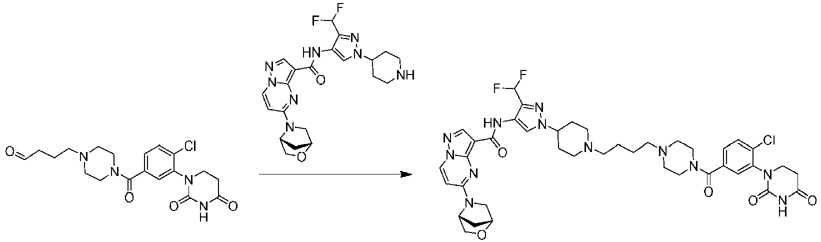

5-((1*R*,4*R*)-2-Oxa-5-azabicyclo[2.2.1]heptan-5-yl)-*N*-(3-(difluoromethyl)-1-(piperidin-4-yl)-1*H-*pyrazol-4-yl)pyrazolo[1,5-*a*]pyrimidine-3-carboxamide (40 mg, 0.087 mmol) was added to a solution of 4-(4-(4-chloro-3-(2,4-dioxotetrahydropyrimidin-1(2*H*)-yl)benzoyl)piperazin-1-yl)*n*-butyraldehyde (crude) in DCE, and the mixture was stirred at room temperature for 0.5 h. STAB (55 mg, 0.26 mmol) was added, and the mixture was reacted at room temperature for 2 h, diluted with water (30 mL), and extracted with ethyl acetate (30 mL × 3). The organic phase was washed with saturated brine (50 mL), dried over anhydrous sodium sulfate, filtered, and concentrated under reduced pressure. The crude product was purified by preparative reversed-phase chromatography to give 5-((1*R*,4*R*)-2-oxa-5-azabicyclo[2.2.1]heptan-5-yl)-*N*-(1-(1-(4-(4-(4-chloro-3-(2,4-dioxotetrahydropyrimidin-1(2*H*)-yl)benzoyl)piperazin-1-yl)*n*-butyl)piperidin-4-yl)-3-(difluoromethyl)-1*H*-pyrazol-4-yl)pyrazolo[1,5-*a*]pyrimidine-3-carboxamide.

LC-MS: (ESI, m/z): [M+H]⁺ = 849.2.

¹H NMR (400 MHz, DMSO-*d*₆) δ 10.50 (s, 1H), 9.50 (d, *J* = 5.7 Hz, 1H), 8.78 (d, *J* = 7.7 Hz, 1H), 8.39 (d, *J* = 3.9 Hz, 1H), 8.26 (d, *J* = 5.6 Hz, 1H), 7.64 (d, *J* = 8.2 Hz, 1H), 7.57 (d, *J* = 1.9 Hz, 1H), 7.40 (dd, *J* = 8.2, 2.0 Hz, 1H), 7.26-6.95 (m, 1H), 6.87-6.44 (m, 1H), 5.28-5.07 (m, 1H), 4.77 (d, *J* = 16.2 Hz, 1H), 4.25-4.12 (m, 1H), 3.84-3.70 (m, 3H), 3.67-3.55 (m, 4H), 3.45 (d, *J* = 10.2 Hz, 1H), 2.93 (d, *J* = 10.9 Hz, 2H), 2.82-2.70 (m, 2H), 2.45-2.25 (m, 9H), 2.12-1.78 (m, 9H), 1.51-1.38 (m, 4H).

### Example 41: N-(3-(difluoromethyl)-1-((1R,4R)-4-(((2-(1-(3-(2,4-dioxotetrahydropyrimidin-1(2H)-yl)-4-fluorobenzoyl)piperidin-4-yl)ethyl)(methyl)amino)methyl)cyclohexyl)-1H-pyrazol-4-yl)-5-(4-methylpiperazin-1-yl)pyrazolo[1,5-a]pyrimidine-3-carboxamide

### Step 1: preparation of ethyl 5-(4-methylpiperazin-1-yl)pyrazolo[1,5-a]pyrimidine-3-carboxylate

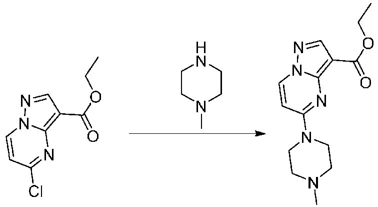

Ethyl 5-chloropyrazolo[1,5-*a*]pyrimidine-3-carboxylate (1 g, 4.4 mmol), 1-methylpiperazine (445 mg, 4.4 mmol), and *N*,*N*-diisopropylethylamine (1.7 g, 13.3 mmol) were dissolved in acetonitrile (10 mL), and the mixture was heated to 60 °C and stirred overnight. The reaction liquid was directly concentrated under reduced pressure, and the crude product was purified by column chromatography (methanol:dichloromethane = 1:9) to give ethyl 5-(4-methylpiperazin-1-yl)pyrazolo[1,5-*a*]pyrimidine-3-carboxylate.

LC-MS: (ESI, m/z): [M+H]' = 290.3.

### Step 2: preparation of 5-(4-methylpiperazin-1-yl)pyrazolo[1,5-a]pyrimidine-3-carboxylic acid

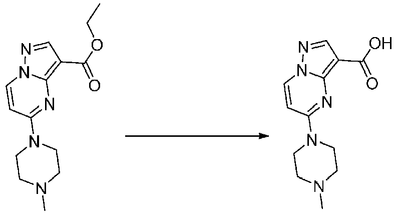

Ethyl 5-(4-methylpiperazin-1-yl)pyrazolo[1,5-a]pyrimidine-3-carboxylate (900 mg, 3.1 mmol) was dissolved in a mixed solution (methanol/water = 5:1, 10 mL), lithium hydroxide (520 mg, 12.4 mmol) was added, and the mixture was heated to 60 °C and stirred for 2 h. The reaction liquid was adjusted to pH = 3 with hydrochloric acid (2 mol/L) and filtered, and the filter cake was dried under vacuum to give 5-(4-methylpiperazin-1-yl)pyrazolo[1,5-a]pyrimidine-3-carboxylic acid.

### Step 3: preparation of N-(3-(difluoromethyl)-1-((1R,4R)-4-(hydroxymethyl)cyclohexyl)-1H-pyrazol-4-yl)-5-(4-methylpiperazin-1-yl)pyrazolo[1,5-a]pyrimidine-3-carboxamide

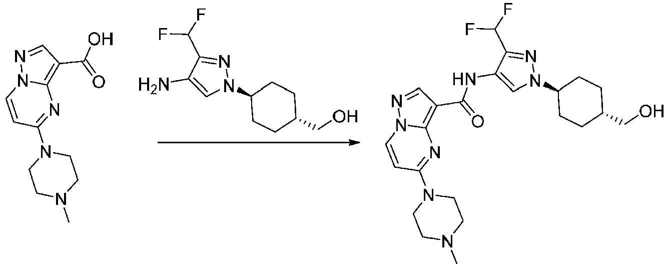

5-(4-Methylpiperazin-1-yl)pyrazolo[1,5-a]pyrimidine-3-carboxylic acid (265 mg, 1.02 mmol) was dissolved in DMF (10 mL). ((1R,4R)-4-(4-Amino-3-(difluoromethyl)-1H-pyrazol-1-yl)cyclohexyl)methanol (250 mg, 1.02 mmol), DIEA (395 mg, 3.06 mmol), and pyBOP (690 mg, 1.33 mmol) were added, and the mixture was reacted at 50 °C overnight. Water (50 mL) was added to the reaction liquid, and the mixture was stirred for 5 min, extracted with ethyl acetate (50 mL × 3), washed with saturated brine (100 mL), dried over anhydrous sodium sulfate, filtered, and concentrated under reduced pressure. The crude product was purified by column chromatography (MeOH:DCM = 0-1:9) to give *N*-(3-(difluoromethyl)-1-((1*R*,4*R*)-4-(hydroxymethyl)cyclohexyl)-1*H*-pyrazol-4-yl)-5-(4-methylpiperazin-1-yl)pyrazolo[1,5-*a*]pyrimidine-3-carboxamide.

LC-MS: (ESI, m/z): [M+H]⁺ = 489.2.

### Step 4: preparation of N (3-(difluoromethyl)-1-((1R,4R)-4-formylcyclohexyl)-1H pyrazol-4-yl)-5-(4-methylpiperazin-1-yl)pyrazolo[1,5-a]pyrimidine-3-carboxamide

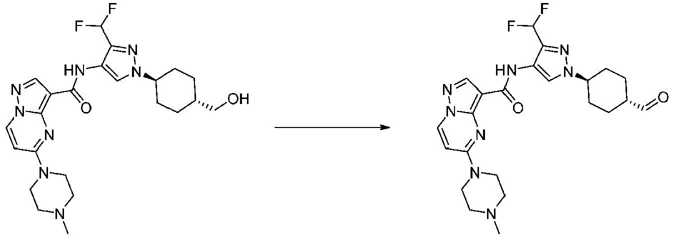

*N*-(3-(Difluoromethyl)-1-((1*R*,4*R*)-4-(hydroxymethyl)cyclohexyl)-1*H*-pyrazol-4-yl)-5-(4-methylpiperazin-1-yl)pyrazolo[1,5-a]pyrimidine-3-carboxamide (350 mg, 0.72 mmol) was dissolved inACN (10 mL). IBX (402 mg, 1.43 mmol) was added, and the mixture was reacted at 85 °C for 1.5 h. The reaction liquid was concentrated under reduced pressure to remove ACN, then water (30 mL) was added, and the mixture was extracted with ethyl acetate (30 mL × 3). The organic phase was washed with saturated brine (50 mL), dried over anhydrous sodium sulfate, filtered, and concentrated under reduced pressure. The crude product was purified by column chromatography (MeOH:DCM = 0-1:9) to give *N*-(3-(difluoromethyl)-1-((1*R*,4*R*)-4-formylcyclohexyl)-1*H*-pyrazol-4-yl)-5-(4-methylpiperazin-1-yl)pyrazolo[1,5-*a*]pyrimidine-3-carboxamide.

LC-MS: (ESI, m/z): [M+H]⁺ = 487.3.

### Step 5: preparation of tert-butyl 4-(2-((((1R,4R)-4-(3-(difluoromethyl)-4-(5-(4-methylpiperazin-1-yl)pyrazolo[1,5-a]pyrimidine-3-carboxamido)-1H-pyrazol-1-yl)cyclohexyl)methyl)(methyl)amino)ethyl)piperidine-1-carboxylate

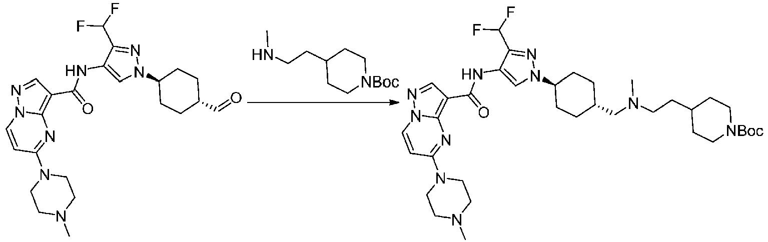

*N*-(3-(Difluoromethyl)-1-((1*R*,4*R*)-4-formylcyclohexyl)-1*H*-pyrazol-4-yl)-5-(4-methylpiperazin-1-yl)pyrazolo[1,5-*a*]pyrimidine-3-carboxamide (180 mg, 0.37 mmol) and tert-butyl 4-(2-(methylamino)ethyl)piperidine-1-carboxylate (107 mg, 0.44 mmol) were dissolved in tetrahydrofuran (10 mL). STAB (235 mg, 1.11 mmol) was added, and the mixture was reacted at room temperature for 2 h. The reaction liquid was diluted with water (50 mL) and extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 0-1:9) to give *tert*-butyl 4-(2-((((1*R*,4*R*)-4-(3-(difluoromethyl)-4-(5-(4-methylpiperazin-1-yl)pyrazolo[1,5-*a*]pyrimidine-3-carboxamido)-1*H*-pyrazol-1-yl)cyclohexyl)methyl)(methyl)amino)ethyl)piperidine-1-carboxylate.

LC-MS: (ESI, m/z): [M+H]⁺ = 713.4.

### Step 6: preparation of N-(3-(difluoromethyl)-1-((1R,4R)-4-((methyl(2-(piperidin-4-yl)ethyl)amino)methyl)cyclohexyl)-1H-pyrazol-4-yl)-5-(4-methylpiperazin-1-yl)pyrazolo[1,5-a]pyrimidine-3-carboxamide

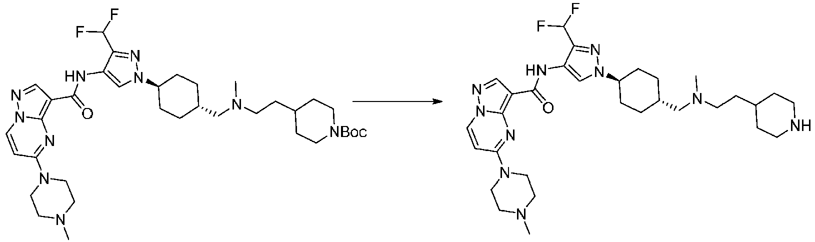

*tert*-Butyl 4-(2-((((1*R*,4*R*)-4-(3-(difluoromethyl)-4-(5-(4-methylpiperazin-1-yl)pyrazolo[1,5-*a*]pyrimidine-3-carboxamido)-1*H*-pyrazol-1-yl)cyclohexyl)methyl)(methyl)amino)ethyl)piperidine-1-carboxylate (220 mg, 0.31 mmol) was dissolved in DCM (5 mL). TFA (1 mL) was added, and the mixture was reacted at room temperature for 1 h. The reaction liquid was directly concentrated under reduced pressure to give *N*-(3-(difluoromethyl)-1-((1*R*,4*R*)-4-((methyl(2-(piperidin-4-yl)ethyl)amino)methyl)cyclohexyl)-1*H-*pyrazol-4-yl)-5-(4-methylpiperazin-1-yl)pyrazolo[1,5-a]pyrimidine-3-carboxamide. The resulting product was directly used in the next step.

LC-MS: (ESI, m/z): [M+H]⁺ = 613.3.

### Step 7: preparation of N-(3-(difluoromethyl)-1-((1R,4R)-4-(((2-(1-(3-(2,4-dioxotetrahydropyrimidin-1(2H)-yl)-4-fluorobenzoyl)piperidin-4-yl)ethyl)(methyl)amino)methyl)cyclohexyl)-1H-pyrazol-4-yl)-5-(4-methylpiperazin-1-yl)pyrazolo[1,5-a]pyrimidine-3-carboxamide

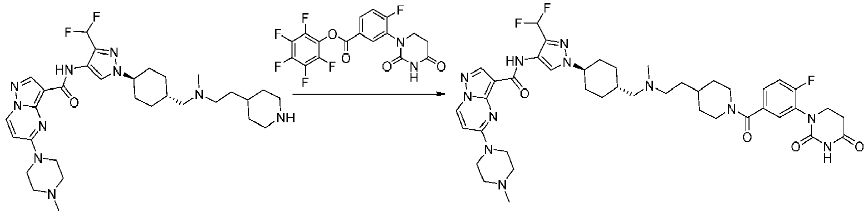

*N*-(3-(Difluoromethyl)-1-((1*R*,4*R*)-4-((methyl(2-(piperidin-4-yl)ethyl)amino)methyl)cyclohexyl)-1*H*-pyrazol-4-yl)-5-(4-methylpiperazin-1-yl)pyrazolo[1,5-*a*]pyrimidine-3-carboxamide (220 mg, 0.31 mmol) was dissolved in DMSO (10 mL). DIEA (200 mg, 1.55 mmol) and pentafluorophenyl 3-(2,4-dioxotetrahydropyrimidin-1(2*H*)-yl)-4-fluorobenzoate (130 mg, 0.31 mmol) were added, and the mixture was reacted at room temperature for 2 h. Water (100 mL) was added to the reaction liquid, and the mixture was stirred for 5 min and extracted with ethyl acetate (100 mL × 3). The organic phase was washed with saturated brine (150 mL), dried over anhydrous sodium sulfate, filtered, and concentrated under reduced pressure. The crude product was purified by preparative reversed-phase chromatography to give N (3-(difluoromethyl)-1-((1*R*,4*R*)-4-(((2-(1-(3-(2,4-dioxotetrahydropyrimidin-1(2*H*)-yl)-4-fluorobenzoyl)piperidin-4-yl)ethyl)(methyl)amino)methyl)cyclohexyl)-1*H*-pyrazol-4-yl)-5-(4-methylpiperazin-1-yl)pyrazolo[1,5-*a*]pyrimidine-3-carboxamide.

LC-MS: (ESI, m/z): [M+H]⁺ = 847.8.

¹H NMR (400 MHz, DMSO-*d*₆) δ 10.54 (s, 1H), 9.41 (s, 1H), 8.79 (d, *J* = 7.9 Hz, 1H), 8.39 (s, 1H), 8.27 (s, 1H), 7.50 (d, *J* = 6.9 Hz, 1H), 7.37 (d, *J* = 7.8 Hz, 2H), 7.26-6.97 (m, 1H), 6.92 (d, *J* = 8.0 Hz, 1H), 4.50-4.37 (m, 1H), 4.22-4.10 (m, 1H), 3.85-3.75 (m, 6H), 3.70-3.50 (m, 2H), 3.10-2.95 (m, 1H), 2.72 (t, *J* = 6.6 Hz, 2H), 2.45-2.38 (m, 4H), 2.32-2.25 (m, 2H), 2.24 (s, 3H), 2.11-1.97 (m, 7H), 1.93-1.85 (m, 2H), 1.81-1.45 (m, 6H), 1.41-1.31 (m, 2H), 1.18-0.95 (m, 4H).

### Example 42: N-(1-(1-(2-(3-(4-chloro-3-(2,4-dioxotetrahydropyrimidin-1(2H)-yl)benzoyl)-3-azaspiro[5.5]undecan-9-yl)ethyl)piperidin-4-yl)-3-(difluoromethyl)-1H-pyrazol-4-yl)-5-morpholinopyrazolo[1,5-a]pyrimidine-3-carboxamide

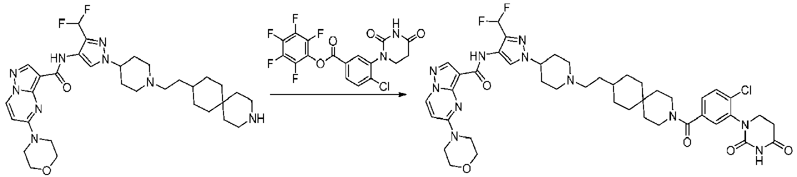

*N*-(1-(1-(2-(3-Azaspiro[5.5]undecan-9-yl)ethyl)piperidin-4-yl)-3-(difluoromethyl)-1*H*-pyrazol-4-yl)-5-morpholinopyrazolo[1,5-*a*]pyrimidine-3-carboxamide (23 mg, 0.03 mmol) was dissolved in dimethyl sulfoxide (3 mL). Pentafluorophenyl 4-chloro-3-(2,4-dioxotetrahydropyrimidin-1(2H)-yl)benzoate (14 mg, 0.03 mmol) and *N*,*N*-diisopropylethylamine (38 mg, 0.30 mmol) were added while stirring, and the mixture was stirred at room temperature for 2 h. Water (20 mL) was added to the reaction liquid. The mixture was filtered, and the filter cake was purified by p-TLC (methanol:dichloromethane = 1:9) to give *N*-(1-(1-(2-(3-(4-chloro-3-(2,4-dioxotetrahydropyrimidin-1(2*H*-yl)benzoyl)-3-azaspiro[5.5]undecan-9-yl)ethyl)piperidin-4-yl)-3-(difluoromethyl)-1*H*-pyrazol-4-yl)-5-morpholinopyrazolo[1,5-*a*]pyrimidine-3-carboxamide.

LC-MS: (ESI, m/z): [M+H]⁺ = 876.3.

¹H NMR (400 MHz, DMSO-*d*₆) δ 10.51 (s, 1H), 9.40 (s, 1H), 8.83 (d, *J* = 7.9 Hz, 1H), 8.39 (s, 1H), 8.29 (s, 1H), 7.63 (d, *J* = 8.2 Hz, 1H), 7.55 (d, *J* = 1.9 Hz, 1H), 7.42-7.35 (m, 1H), 7.26-6.97 (m, 1H), 6.91 (d, *J* = 8.0 Hz, 1H), 4.31-4.11 (m, 1H), 3.86-3.66 (m, 9H), 3.61-3.50 (m, 3H), 3.29-3.24 (m, 1H), 2.99-2.90 (m, 2H), 2.80-2.71 (m, 2H), 2.43-2.30 (m, 2H), 2.15-1.84 (m, 6H), 1.73-1.60 (m, 2H), 1.55-1.23 (m, 10H), 1.16-1.01 (m, 4H).

### Example 43: 5-((1R,4R)-2-oxa-5-azabicyclo[2.2.1]heptan-5-yl)-N-(1-(1-(2-(3-(4-chloro-3-(2,4-dioxotetrahydropyrimidin-1(2H)-yl)benzoyl)-3-azaspiro[5.5]undecan-9-yl)ethyl)piperidin-4-yl)-3-(difluoromethyl)-1H-pyrazol-4-yl)pyrazolo[1,5-a]pyrimidine-3-carboxamide

### Step 1: preparation of tert-butyl 9-(2-(4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)ethyl)-3-azaspiro[5.5]undecane-3-carboxylate

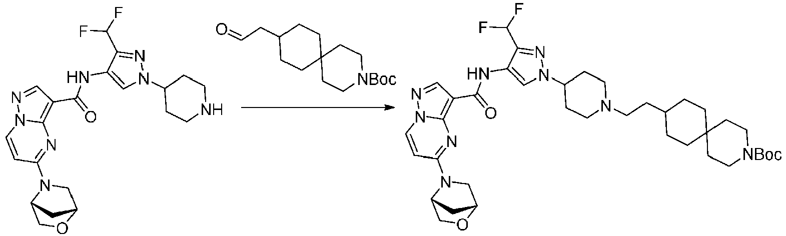

5-((1*R*,4*R*)-2-Oxa-5-azabicyclo[2.2.1]heptan-5-yl)-*N*-(3-(difluoromethyl)-1-(piperidin-4-yl)-1*H-*pyrazol-4-yl)pyrazolo[1,5-*a*]pyrimidine-3-carboxamide (50 mg, 0.11 mmol) and *tert*-butyl 9-(2-oxoethyl)-3-azaspiro[5.5]undecane-3-carboxylate (40 mg, 0.13 mmol) were dissolved in tetrahydrofuran (10 mL). Sodium triacetoxyborohydride (70 mg, 0.33 mmol) was added, and the mixture was stirred at room temperature for 2 h. Water (50 mL) was added to the reaction liquid, and the mixture was extracted with dichloromethane (20 mL × 3). The organic phase was washed with saturated brine (60 mL), dried over anhydrous sodium sulfate, filtered, and concentrated under reduced pressure. The crude product was purified by p-TLC (methanol:dichloromethane = 1:9) to give *tert*-butyl 9-(2-(4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)ethyl)-3-azaspiro[5.5]undecane-3-carboxylate.

LC-MS: (ESI, m/z): [M+H]⁺ = 738.4.

### Step 2: preparation of N-(1-(1-(2-(3-azaspiro[5.5]undecan-9-yl)ethyl)piperidin-4-yl)-3-(difluoromethyl)-1H-pyrazol-4-yl)-5-((1R,4R)-2-oxa-5-azabicyclo[2.2.1]heptan-5-yl)pyrazolo[1,5-a]pyrimidine-3-carboxamide

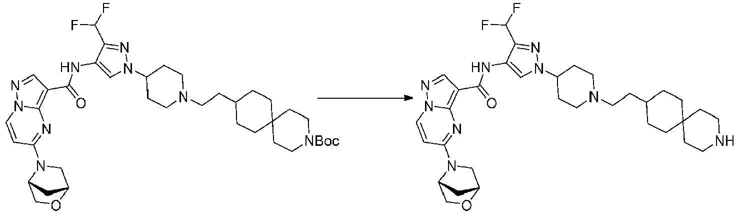

*tert*-Butyl 9-(2-(4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)ethyl)-3-azaspiro[5.5]undecane-3-carboxylate (40 mg, 0.05 mmol) was dissolved in dichloromethane (3 mL). Trifluoroacetic acid (1 mL) was added, and the mixture was stirred at room temperature for 2 h. The reaction liquid was directly concentrated under reduced pressure to give *N*-(1-(1-(2-(3-azaspiro[5.5]undecan-9-yl)ethyl)piperidin-4-yl)-3-(difluoromethyl)-1*H*-pyrazol-4-yl)-5-((1*R*,4*R*)-2-oxa-5-azabicyclo[2.2.1]heptan-5-yl)pyrazolo[1,5-a]pyrimidine-3-carboxamide. The resulting product was directly used in the next step without purification.

LC-MS: (ESI, m/z): [M+H]⁺ = 638.3.

### Step 3: preparation of 5-((1R,4R)-2-oxa-5-azabicyclo[2.2.1]heptan-5-yl)-N-(1-(1-(2-(3-(4-chloro-3-(2,4-dioxotetrahydropyrimidin-1(2H)-yl)benzoyl)-3-azaspiro[5.5]undecan-9-yl)ethyl)piperidin-4-yl)-3-(difluoromethyl)-1H-pyrazol-4-yl)pyrazolo[1,5-a]pyrimidine-3-carboxamide

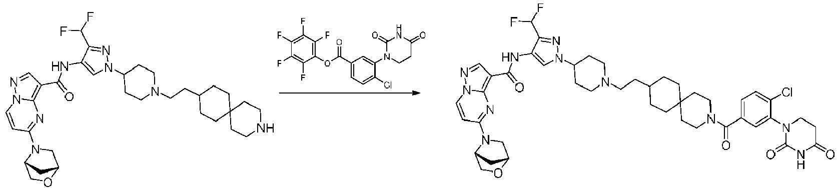

*N*-(1-(1-(2-(3-Azaspiro[5.5]undecan-9-yl)ethyl)piperidin-4-yl)-3-(difluoromethyl)-1*H*-pyrazol-4-yl)-5-((1*R*,4*R*)-2-oxa-5-azabicyclo[2.2.1]heptan-5-yl)pyrazolo[1,5-*a*]pyrimidine-3-carboxamide (40 mg, 0.05 mmol) was dissolved in dimethyl sulfoxide (3 mL). Pentafluorophenyl 4-chloro-3-(2,4-dioxotetrahydropyrimidin-1(2*H*)-yl)benzoate (24 mg, 0.05 mmol) and *N*,*N*-diisopropylethylamine (64 mg, 0.50 mmol) were added while stirring, and the mixture was stirred at room temperature overnight. Water (30 mL) was added to the reaction liquid. The mixture was filtered, and the filter cake was purified by pre-HPLC (CH₃CN/0.08% aqueous NH₄HCO₃ solution, 5%-95%) to give 5-((1R,4R)-2-oxa-5-azabicyclo[2.2.1]heptan-5-yl)-*N*-(1-(1-(2-(3-(4-chloro-3-(2,4-dioxotetrahydropyrimidin-1(2*H*)-yl)benzoyl)-3-azaspiro[5.5]undecan-9-yl)ethyl)piperidin-4-yl)-3-(difluoromethyl)-1*H*-pyrazol-4-yl)pyrazolo[1,5-*a*]pyrimidine-3-carboxamide.

LC-MS: (ESI, m/z): [M+H]⁺ = 888.3.

¹H NMR (400 MHz, DMSO-*d*₆) δ 10.50 (s, 1H), 9.50 (d, *J* = 5.7 Hz, 1H), 8.78 (d, *J* = 7.8 Hz, 1H), 8.39 (d, *J* = 3.9 Hz, 1H), 8.25 (d, *J* = 5.6 Hz, 1H), 7.63 (d, *J* = 8.2 Hz, 1H), 7.55 (d, *J* = 1.8 Hz, 1H), 7.39 (dd, *J* = 8.2, 1.9 Hz, 1H), 7.26-6.97 (m, 1H), 6.88-6.47 (m, 1H), 5.28-5,07 (m, 1H), 4.77 (d, *J* = 17.8 Hz, 1H), 4.30-4.10 (m, 1H), 3.85-3.72 (m, 3H), 3.67-3.41 (m, 5H), 3.30-3.24 (m, 2H), 2.97-2.88 (m, 2H), 2.80-2.70 (m, 2H), 2.35-2.27 (m, 2H), 2.03-1.80 (m, 8H), 1.72-1.65 (m, 2H), 1.55-1.26 (m, 9H), 1.24-0.97 (m, 4H).

### Example 44: 5-((1R,4R)-2-oxa-5-azabicyclo[2.2.1]heptan-5-yl)-N-(3-(diffuoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)pyrazolo[1,5-a]pyrimidine-3-carboxamide

### Step 1: preparation of tert-butyl 9-((4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)methyl)-3-azaspiro[5.5]undecane-3-carboxylate

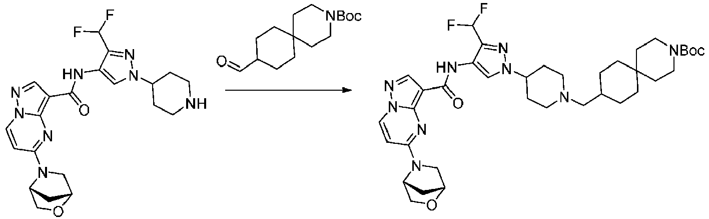

5-((1*R*,4*R*)-2-Oxa-5-azabicyclo[2.2.1]heptan-5-yl)-*N-*(3-(difluoromethyl)-1-(piperidin-4-yl)-1*H-*pyrazol-4-yl)pyrazolo[1,5-*a*]pyrimidine-3-carboxamide (100 mg, crude) was dissolved in THF (10 mL). *tert-*Butyl 9-formyl-3-azaspiro[5.5]undecane-3-carboxylate (50 mg, 0.18 mmol) and STAB (115 mg, 0.54 mmol) were added, and the mixture was reacted at room temperature for 2 h. The reaction liquid was diluted with water (50 mL) and extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 0-1:9) to give tert-butyl 9-((4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate.

LC-MS: (ESI, m/z): [M+H]⁺ = 724.3.

### Step 2: preparation of N-(1-(1-((3-azaspiro[5.5]undecan-9-yl)methyl)piperidin-4-yl)-3-(difluoromethyl)-1H-pyrazol-4-yl)-5-((1R,4R)-2-oxa-5-aaabicyclo[2.2.1]heptan-5-yl)pyrazolo[1,5-a]pyrimidine-3-carboxamide

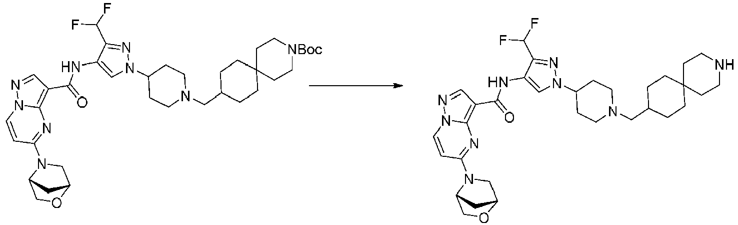

*tert*-Butyl 9-((4-(4-(5-((1R,4R)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate (100 mg, 0.14 mmol) was dissolved in DCM (4 mL). TFA (1 mL) was added, and the mixture was reacted at room temperature for 1 h. The reaction liquid was directly concentrated under reduced pressure to give *N*-(1-(1-((3-azaspiro[5.5]undecan-9-yl)methyl)piperidin-4-yl)-3-(difluoromethyl)-1*H*-pyrazol-4-yl)-5-((1*R*,4*R*)-2-oxa-5-azabicyclo[2.2.1]heptan-5-yl)pyrazolo[1,5-*a*]pyrimidine-3-carboxamide, which was directly used in the next step.

LC-MS: (ESI, m/z): [M+H]⁺ = 624.4.

### Step 3: preparation of 5-((1R,4R)-2-oxa-5-azabicyclo[2.2.1]heptan-5-yl)-N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1Hpyrazol-4-yl)pyrazolo[1,5-a]pyrimidine-3-carboxamide

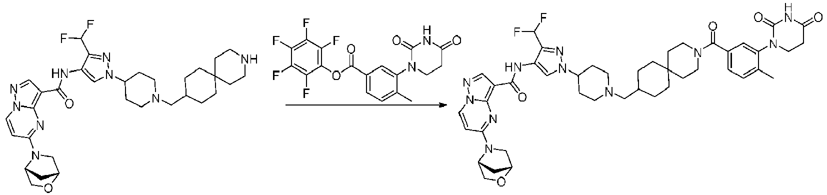

*N*-(1-(1-((3-Azaspiro[5.5]undecan-9-yl)methyl)piperidin-4-yl)-3-(difluoromethyl)-1*H*-pyrazol-4-yl)-5-((1*R*,4*R*)-2-oxa-5-azabicyclo[2.2.1]heptan-5-yl)pyrazolo[1,5-*a*]pyrimidine-3-carboxamide (100 mg, 0.14 mmol) was dissolved in DMSO (10 mL). DIEA (90 mg, 0.70 mmol) and pentafluorophenyl 3-(2,4-dioxotetrahydropyrimidin-1(2*H*)-yl)-4-methylbenzoate (58 mg, 0.14 mmol) were added, and the mixture was reacted at room temperature for 2 h. Water (100 mL) was added to the reaction liquid, and the mixture was stirred for 5 min and extracted with ethyl acetate (100 mL × 3). The organic phase was washed with saturated brine (150 mL), dried over anhydrous sodium sulfate, filtered, and concentrated under reduced pressure. The crude product was purified by preparative reversed-phase chromatography to give 5-((1R,4R)-2-oxa-5-azabicyclo[2.2.1]heptan-5-yl)-*N*-(3-(difluoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H*-pyrazol-4-yl)pyrazolo[1,5-*a*]pyrimidine-3-carboxamide.

LC-MS: (ESI, m/z): [M+H]⁺ = 854.4.

¹H NMR (400 MHz, DMSO-*d*₆) δ 10.37 (s, 1H), 9.50 (d, *J* = 5.7 Hz, 1H), 8.78 (d, *J* = 7.6 Hz, 1H), 8.39 (d, *J* = 3.6 Hz, 1H), 8.26 (d, *J* = 5.6 Hz, 1H), 7.36-7.29 (m, 2H), 7.26-6.96 (m, 2H), 6.88-6.44 (m, 1H), 5.28-5.07 (m, 1H), 4.77 (d, *J* = 17.6 Hz, 1H), 4.31-4.11 (m, 1H), 3.88-3.71 (m, 3H), 3.63-3.45 (m, 5H), 2.95-2.85 (m, 2H), 2.81-2.65 (m, 2H), 2.25-2.10 (m, 5H), 2.07-1.87 (m, 8H),

### Example 45: N-(3-(difluoromethyl)-1-((1R,4S)-4-((9-(3-(2,4-dioxotetrahydropyrimidin-1(2H)-yl)-4-methylbenzoyl)-3,9-diazaspiro[5.5]undecan-3-yl)methyl)cyclohexyl)-1H-pyrazol-4-yl)-5-((S)-3-hydroxypiperidin-1-yl)pyrazolo[1,5-a]pyrimidine-3-carboxamide

### Step 1: preparation of tert-butyl 9-(((1S,4R)-4-(3-(difluoromethyl)-4-(5-((3S)-3-((tetrahydro-2H-pyran-2-yl)oxy)piperidin-1-yl)pyrazolo[1,5-a]pyrimidine-3-carboxamido)-1H-pyrazol-1-yl)cyclohexyl)methyl)-3,9-diazaspiro[5.5]undecane-3-carboxylate

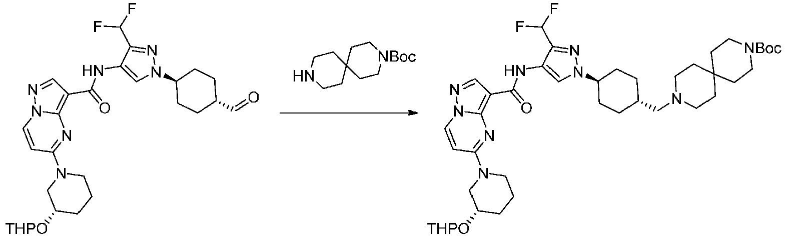

*N*-(3-(Difluoromethyl)-1-((1*R*,4*S*)-4-formylcyclohexyl)-1*H*-pyrazol-4-yl)-5-((3*S*)-3-((tetrahydro-2*H*-pyran-2-yl)oxy)piperidin-1-yl)pyrazolo[1,5-*a*]pyrimidine-3-carboxamide (90 mg, 0.15 mmol) and *tert-*butyl 3,9-diazaspiro[5.5]undecane-3-carboxylate (40 mg, 0.15 mmol) were dissolved in tetrahydrofuran (10 mL). Sodium triacetoxyborohydride (95 mg, 0.45 mmol) was added, and the mixture was stirred at room temperature for 2 h. Water (50 mL) was added, and the mixture was extracted with dichloromethane (20 mL × 3). The organic phase was washed with saturated brine (60 mL), dried over anhydrous sodium sulfate, filtered, and concentrated under reduced pressure. The crude product was purified by column chromatography (methanol:dichloromethane = 1:9) to give tert-butyl 9-(((1*S*,4*R*)-4-(3-(difluoromethyl)-4-(5-((3*S*)-3-((tetrahydro-2*H*-pyran-2-yl)oxy)piperidin-1-yl)pyrazolo[1,5-*a*]pyrimidine-3-carboxamido)-1*H*-pyrazol-1-yl)cyclohexyl)methyl)-3,9-diazaspiro[5.5]undecane-3-carboxylate.

LC-MS: (ESI, m/z): [M+H]⁺ = 810.4.

### Step 2: preparation of N-(1-((1R,4S)-4-((3,9-diazaspiro[5.5]undecan-3-yl)methyl)cyclohexyl)-3-(difluoromethyl)-1H-pyrazol-4-yl)-5-((S)-3-hydroxypiperidin-1-yl)pyrazolo[1,5-a]pyrimidine-3-carboxamide

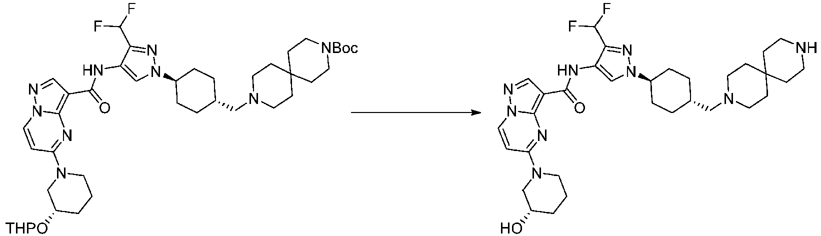

tert-Butyl 9-(((1*S*,4*R*)-4-(3-(difluoromethyl)-4-(5-((3*S*)-3-((tetrahydro-2*H*-pyran-2-yl)oxy)piperidin-1-yl)pyrazolo[1.5-*a*]pyrimidine-3-carboxamido)-1*H*-pyrazol-1-yl)cyclohexyl)methyl)-3,9-diazaspiro[5.5]undecane-3-carboxylate (100 mg, 0.12 mmol) was dissolved in dichloromethane (3 mL). Trifluoroacetic acid (1 mL) was added, and the mixture was stirred at room temperature for 1 h. The reaction liquid was directly concentrated under reduced pressure to give *N*-(1-((1*R*,4*S*)-4-((3,9-diazaspiro[5.5]undecan-3-yl)methyl)cyclohexyl)-3-(difluoromethyl)-1*H*-pyrazol-4-yl)-5-((*S*)-3-hydroxypiperidin-1-yl)pyrazolo[1,5-*a*]pyrimidine-3-carboxamide, which was directly used in the next step without purification.

LC-MS: (ESI, m/z): [M+H]⁺ = 626.3.

### Step 3: preparation of N-(3-(difluoromethyl)-1-((1R,4S)-4-((9-(3-(2,4-dioxotetrahydropyrimidin-1(2H)-yl)-4-methylbenzoyl)-3,9-diazaspiro[5.5]undecan-3-yl)methyl)cyclohexyl)-1H-pyrazol-4-yl)-5-((S)-3-hydroxypiperidin-1-yl)pyrazolo[1,5-a]pyrimidine-3-carboxamide

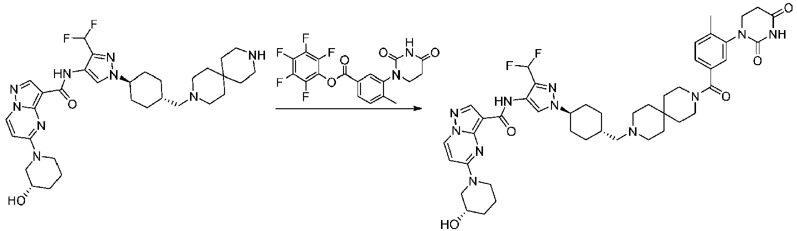

*N*-(1-((1*R*,4*S*)-4-((3,9-Diazaspiro[5.5]undecan-3-yl)methyl)cyclohexyl)-3-(difluoromethyl)-1*H-*pyrazol-4-yl)-5-((*S*)-3-hydroxypiperidin-1-yl)pyrazolo[1,5-*a*]pyrimidine-3-carboxamide (100 mg, 0.12 mmol) was dissolved in dimethyl sulfoxide (3 mL). Pentafluorophenyl 3-(2,4-dioxotetrahydropyrimidin-1(2H)-yl)-4-methylbenzoate (55 mg, 0.13 mmol) and *N*,*N*-diisopropylethylamine (155 mg, 1.2 mmol) were added, and the mixture was stirred at room temperature overnight. Water (30 mL) was added to the reaction liquid. The mixture was filtered, and the filter cake was purified by pre-HPLC (CH₃CN/0.08% aqueous NH₄HCO₃ solution, 5%-95%) to give *N-*(3-(diffuoromethyl)-1-((1*R*,4*S*)-4-((9-(3-(2,4-dioxotetrahydropyrimidin-1(2*H*-yl)-4-methylbenzoyl)-3,9-diazaspiro[5.5]undecan-3-yl)methyl)cyclohexyl)-1*H*-pyrazol-4-yl)-5-((*S*)-3-hydroxypiperidin-1-yl)pyrazolo[1,5-*a*]pyrimidine-3-carboxamide.

LC-MS: (ESI, m/z): [M+H]⁺ = 856.4.

¹H NMR (400 MHz, DMSO-*d*₆) δ 10.39 (s, 1H), 9.38 (s, 1H), 8.72 (d, *J* = 8.0 Hz, lH), 8.36 (s, 1H), 8.24 (s, 1H), 7.36-7.29 (m, 2H), 7.27-6.94 (m, 2H), 6.86 (d, *J* = 8.0 Hz, 1H), 4.93-4.88 (m, 1H), 4.20-4.10 (m, 1H), 4.01-3.74 (m, 3H), 3.70-3.44 (m, 6H), 3.33-3.25 (m, 2H), 2.84-2.64 (m, 2H), 2.40-2.21 (m, 4H), 2.21 (s, 3H), 2.15-1.96 (m, 4H), 1.87-1.70 (m, 6H), 1.65-1.26 (m, 11H), 1.10-0.95 (m, 2H).

### Example 46: 5-((1R,4R)-2-oxa-5-aza[2.2.1]heptan-5-yl)-N-(1-((1R,4R)-4-(2-(((1-(4-chloro-3-(2,4-dioxotetrahydropyrimidin-1(2H)-yl)benzoyl)piperidin-4-yl)methyl)(methyl)amino)ethyl)cyclohexyl)-3-(difluoromethyl)-1H-pyrazol-4-yl)pyrazolo[1,5-a]pyrimidine-3-carboxamide

### Step 1: preparation of ethyl 2-(4-hydroxycyclohexyl)acetate

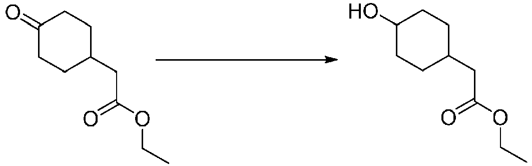

NaBH₄ (3.28 g, 86.96 mmol) was slowly added to a solution of ethyl 2-(4-oxocyclohexyl)acetate (8 g, 43.48 mmol) in anhydrous methanol (100 mL) at 0 °C, and the mixture was stirred for 2 h. Then a 10% aqueous NH₄Cl solution was added to quench the reaction. The mixture was diluted with water (100 mL) and extracted with ethyl acetate (150 mL × 4). The organic phase was washed with saturated brine, dried over anhydrous sodium sulfate, and concentrated under reduced pressure to give a crude product of ethyl 2-(4-hydroxycyclohexyl)acetate.

¹H NMR (400 MHz, CDCl₃) δ 4.13 (q, *J* = 7.1 Hz, 2H), 3.60-3.47 (m, 1H), 2.25-2.14 (m, 2H), 2.05-1.93 (m, 2H), 1.81-1.69 (m, 3H), 1.35-1.20 (m, 5H), 1.11-0.97 (m, 2H).

### Step 2: preparation of ethyl 2-(4-(p-toluenesulfonyloxy)cyclohexyl)acetate

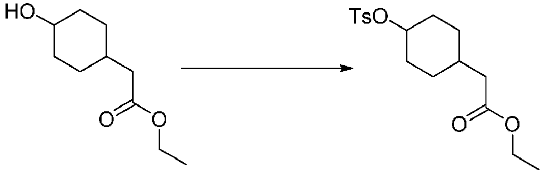

TsCl (11.55 g, 60.48 mmol) was added to a solution of ethyl 2-(4-hydroxycyclohexyl)acetate (7.5 g, 40.32 mmol) in DCM (120 mL). DMAP (1.49 g, 12.10 mmol) and TEA (12.3 g, 120.96 mmol) were added, and the mixture was stirred at room temperature for 16 h under N₂ atmosphere, diluted with water (100 mL), and extracted with ethyl acetate (80 mL × 4). The organic phase was washed with saturated brine, dried over anhydrous sodium sulfate, concentrated under reduced pressure, and purified by normal phase column chromatography (EA:PE = 3:7) to give ethyl 2-(4-(p-toluenesulfonyloxy)cyclohexyl)acetate.

LC-MS: (ESI, m/z): [M+18]⁺ = 358.1.

¹H NMR (400 MHz, DMSO-*d*₆) δ 7.79 (d, *J* = 8.3 Hz, 2H), 7.47 (d, *J* = 8.0 Hz, 2H), 4.41-4.30 (m, 1H), 4.04-4.01 (m, 2H), 2.42 (s, 3H), 2.16 (dd, *J* = 23.3, 6.9 Hz, 2H), 1.81-1.73 (m, 2H), 1.70-1.55 (m, 3H), 1.48-1.36 (m, 2H), 1.17-1.13 (m, 3H), 1.09-0.94 (m, 2H).

### Step 3: preparation of ethyl 2-(4-(3-formyl-1H-pyrazol-1-yl)cyclohexyl)acetate

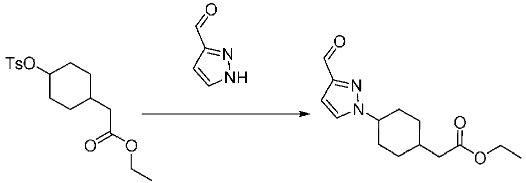

Ethyl 2-(4-(*p*-toluenesulfonyloxy)cyclohexyl)acetate (9.1 g, 26.76 mmol) was added to a solution of 1*H*-pyrazole-3-carbaldehyde (2.98 g, 26.76 mmol) in DMF (120 mL). Then CsCO₃ (14.4 g, 36.49 mmol) and KI (5.85 g, 29.19 mmol) were added, and the mixture was stirred at 50 °C for 16 h under N₂ atmosphere. The mixture was diluted with water (100 mL) and extracted with ethyl acetate (80 mL × 4). The organic phase was washed with saturated brine, dried over anhydrous sodium sulfate, concentrated under reduced pressure, and purified by normal phase column chromatography (EA:PE = 1:4) to give ethyl 2-(4-(3-formyl-1*H*-pyrazol-1-yl)cyclohexyl)acetate.

LC-MS: (ESI, m/z): [M+H]⁺ = 265.3.

¹H NMR (400 MHz, CDCl₃) δ 9.97 (s, 1H), 7.54 (s, 1H), 6.81 (s, 1H), 4.35-4.28 (m, 1H), 4.18-4.08 (m, 2H), 2.64-2.34 (m, 2H), 2.28-2.15 (m, 4H), 2.06-1.81 (m, 3H), 1.79-1.65 (m, 2H), 1.30-1.21 (m, 3H).

### Step 4: preparation of ethyl 2-(4-(3-(difluoromethyl)-1H-pyrazol-1-yl)cyclohexyl)acetate

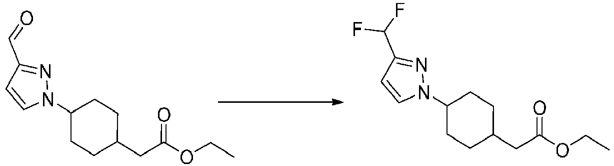

Ethyl 2-(4-(3-formyl-1*H*-pyrazol-1-yl)cyclohexyl)acetate (7.2 g, 27.27 mmol) was dissolved in anhydrous dichloromethane (100 mL). DAST (17.58 g, 109.08 mmol) was slowly added at 0 °C, and the mixture was stirred at room temperature for 16 h under N₂ atmosphere. Methanol (50 mL) was slowly added to the reaction liquid, and the mixture was diluted with water (100 mL) and extracted with ethyl acetate (80 mL × 4). The organic phase was washed with saturated brine, dried over anhydrous sodium sulfate, concentrated under reduced pressure, and purified by normal phase column chromatography (EA:PE = 3:17) to give ethyl 2-(4-(3-(difluoromethyl)-1*H*-pyrazol-1-yl)cyclohexyl)acetate.

LC-MS: (ESI, m/z): [M+H]⁺ = 287.0.

### Step 5: preparation of ethyl 2-(4-(3-(difluoromethyl)-4-nitro-1H-pyrazol-1-yl)cyclohexyl)acetate

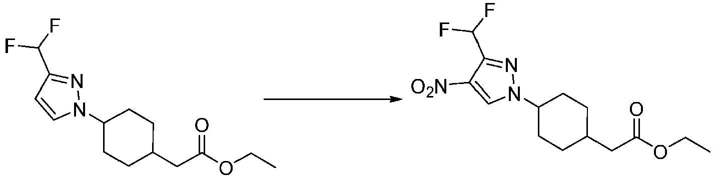

Ethyl 2-(4-(3-(difluoromethyl)-1*H*-pyrazol-1-yl)cyclohexyl)acetate (2.7 g, 9.44 mmol) was added to a solution of HNO₃ (2.9 g, 47.2 mmol) in acetic acid (30 mL). Acetic anhydride (1.9 g, 18.9 mmol) was added, and the mixture was stirred at 50 °C for 16 h under N₂ atmosphere. Water (100 mL) was added to the reaction liquid, and the mixture was extracted with ethyl acetate (80 mL × 4). The organic phase was washed with saturated brine, dried over anhydrous sodium sulfate, concentrated under reduced pressure, and purified by normal phase column chromatography (EA:PE = 1:9) to give ethyl 2-(4-(3-(difluoromethyl)-4-nitro-1H-pyrazol-1 -yl)cyclohexyl)acetate.

LC-MS: (ESI, m/z): [M+H]⁺ = 332.2.

### Step 6: preparation of ethyl 2-((1R,4R)-4-(3-(difluoromethyl)-4-nitro-1H-pyrazol-1-yl)cyclohexyl)acetate

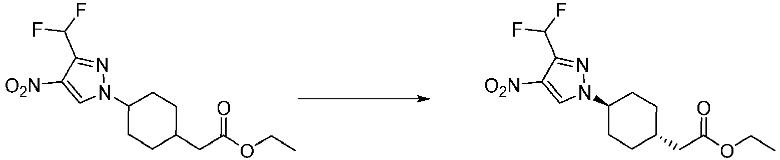

Ethyl 2-(4-(3-(difluoromethyl)-4-nitro-1*H*-pyrazol-pyrazol-1-yl)cyclohexyl)acetate (1.45 g, 4.38 mmol) was purified by preparative SFC (acetonitrile-0.1% aqueous FA solution, 5%-95%) to give the product of ethyl 2-((1*R*,4*R*)-4-(3-(difluoromethyl)-4-nitro-1*H*-pyrazol-1-yl)cyclohexyl)acetate.

LC-MS: (ESI, m/z): [M+H]⁺ = 332.2.

¹H NMR (400 MHz, CDCl₃) δ 8.23 (s, 1H), 7.24-6.98 (m, 1H), 4.32-4.28 (m, 1H), 4.14 (q, *J* = 7.1 Hz, 2H), 2.35 (d, *J* = 7.5 Hz, 2H), 2.28-2.13 (m, 3H), 2.02-1.98 (m, 2H), 1.76-1.74 (m, 2H), 1.57-1.49 (m, 2H), 1.26 (t, *J* = 5.2 Hz, 3H).

### Step 7: preparation of ethyl 2-((1R,4R)-4-(4-amino-3-(difluoromethyl)-1H-pyrazol-1-yl)cyclohexyl)acetate

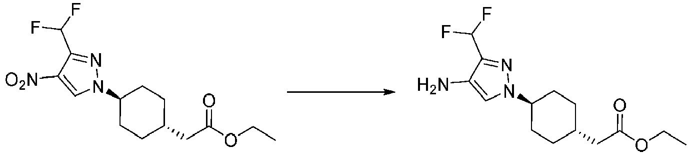

Ethyl 2-((1*R*,4*R*)-4-(3-(difluoromethyl)-4-nitro-1*H*-pyrazol-1-yl)cyclohexyl)acetate (850 mg, 2.56 mmol) was added to a solution of anhydrous ethanol (4 mL). A Raney-Ni solid-water mixture (1 mL) and hydrazine hydrate (0.8 mL) were added, and the mixture was stirred at room temperature for 2 h. The reaction liquid was filtered, the filter cake was washed with ethanol, and the filtrate was directly concentrated under reduced pressure to give ethyl 2-((1*R*,4*R*)-4-(4-amino-3-(difluoromethyl)-1*H*-pyrazol-1-yl)cyclohexyl)acetate.

LC-MS: (ESI, m/z): [M+H]⁺ = 302.5.

### Step 8: preparation of 2-((1R,4R)-4-(4-amino-3-(difluoromethyl)-1H-pyrazol-1-yl)cyclohexyl)ethyl-1 -ol

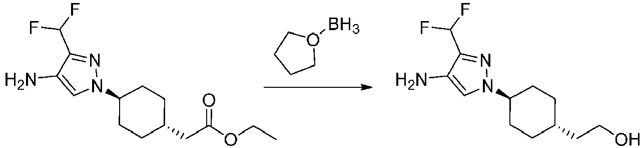

Ethyl 2-((1*R*,4*R*)-4-(4-amino-3-(difluoromethyl)-1*H*-pyrazol-1-yl)cyclohexyl)acetate (100 mg, 0.33 mmol) was dissolved in anhydrous tetrahydrofuran (5 mL). A solution of borane in tetrahydrofuran (4.98 mL, 4.98 mmol) was slowly added to the solution under nitrogen atmosphere, and the reaction liquid was heated to 50 °C and stirred for 3 h. The reaction liquid was cooled to room temperature. Methanol was slowly added dropwise until no bubbles were generated, and the resulting solution was concentrated under reduced pressure. The crude product was purified by column chromatography (methanol:dichloromethane = 1:9) to give 2-((1*R*,4*R*)-4-(4-amino-3-(difluoromethyl)-1*H*-pyrazol-1-yl)cyclohexyl)ethyl-1-ol.

LC-MS: (ESI, m/z): [M+H]⁺ = 260.2.

### Step 9: preparation of 5-((1R,4R)-2-oxa-5-azabicyclo[2.2.1]heptan-5-yl)-N-(3-(difluoromethyl)-1-((1R,4R)-4-(2-hydroxyethyl)cyclohexyl)-1H-pyrazol-4-yl)pyrazolo[1,5-a]pyrimidine-3-carboxamide

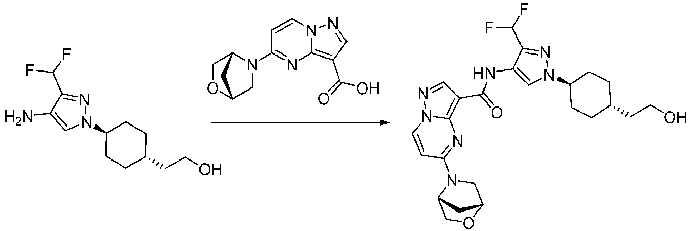

2-((1*R*,4*R*)-4-(4-Amino-3-(difluoromethyl)-1*H*-pyrazol-1-yl)cyclohexyl)ethyl-1-ol (80 mg, 0.30 mmol) and 5-((1*R*,4*R*)-2-oxa-5-azabicyclo[2.2.1]heptan-5-yl)pyrazolo[1,5-*a*]pyrimidine-3-carboxylic acid (156 mg, 0.60 mmol) were dissolved in DMF (5 mL). *N*,*N-*Diisopropylethylamine (116 mg, 0.90 mmol) and Py-BOP (203 mg, 0.39 mmol) were added while stirring, and the mixture was stirred at 60 °C overnight. The reaction liquid was cooled to room temperature. Water (50 mL) was added, and the mixture was extracted with ethyl acetate (30 mL × 3). The organic phase was washed with saturated brine (50 mL), dried over anhydrous sodium sulfate, filtered, and concentrated under reduced pressure. The crude product was purified by p-TLC (methanol:dichloromethane = 1:9) to give 5-((1*R*,4*R*)-2-oxa-5-azabicyclo[2.2.1]heptan-5-yl)-*N*-(3-(difluoromethyl)-1-((1*R*,4*R*)-4-(2-hydroxyethyl)cyclohexyl)-1*H*-pyrazol-4-yl)pyrazolo[1,5-*a*]pyrimidine-3-carboxamide.

LC-MS: (ESI, m/z): [M+H]⁺ = 502.2.

### Step 10: preparation of 5-((1R,4R)-2-oxa-5-azabicyclo[2.2.1]heptan-5-yl)-N-(3-(difluoromethyl)-1-((1R,4R)-4-(2-oxoethyl)cyclohexyl)-1H-pyrazol-4-yl)pyrazolo[1,5-a]pyrimidine-3-carboxamide

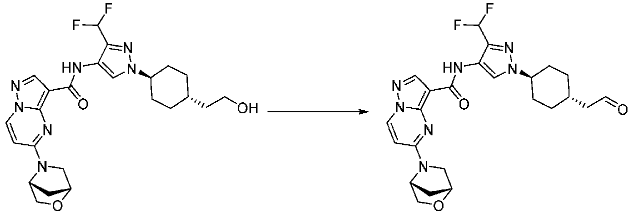

5-((1*R*,4*R*)-2-Oxa-5-azabicyclo[2.2.1]heptan-5-yl)-*N-*(3-(difluoromethyl)-1-((1*R*,4*R*)-4-(2-hydroxyethyl)cyclohexyl)-1*H*-pyrazol-4-yl)pyrazolo[1,5-*a*]pyrimidine-3-carboxamide (100 mg, 0.20 mmol) was dissolved in acetonitrile (10 mL). IBX (112 mg, 0.40 mmol) was added, and the mixture was stirred at 80 °C for 2 h. The reaction liquid was concentrated under reduced pressure, and the crude product was purified by column chromatography (methanol:dichloromethane = 1:9) to give 5-((1*R*,4*R*)-2-oxa-5-azabicyclo[2.2.1]heptan-5-yl)-*N*-(3-(difluoromethyl)-1-((1*R*,4*R*)-4-(2-oxoethyl)cyclohexyl)-1*H*-pyrazol-4-yl)pyrazolo[1,5-*a*]pyrimidine-3-carboxamide.

LC-MS: (ESI, m/z): [M+H]⁺ = 500.2.

### Step 11: preparation of tert-butyl 4-(((2,4-dimethoxyphenyl)(methyl)amino)methyl)piperidine-1-carboxylate

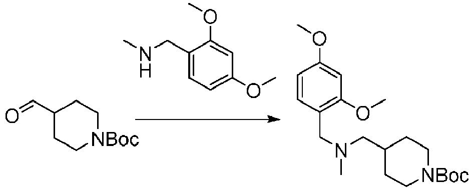

tert-Butyl 4-formylpiperidine-1-carboxylate (200 mg, 1.0 mmol) and 1-(2,4-dimethoxyphenyl)-N-methylmethanamine (190 mg, 1.1 mmol) were dissolved in tetrahydrofuran (10 mL). Then sodium triacetoxyborohydride (636 mg, 3.0 mmol) was added, and the mixture was stirred at room temperature for 2 h. Water (50 mL) was added to the reaction liquid, and the mixture was extracted with dichloromethane (20 mL × 3), dried over anhydrous sodium sulfate, filtered, and concentrated under reduced pressure. The crude product was purified by column chromatography (ethyl acetate:petroleum ether = 4:1) to give tert-butyl 4-(((2,4-dimethoxyphenyl)(methyl)amino)methyl)piperidine-1-carboxylate.

LC-MS: (ESI, m/z): [M+H]⁺ = 379.2.

### Step 12: preparation of tert-butyl 4-((methylamino)methyl)piperidine-1-carboxylate

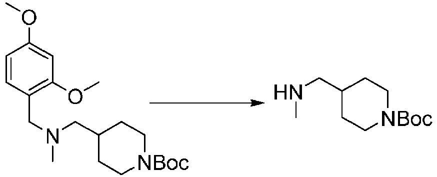

tert-Butyl 4-(((2,4-dimethoxyphenyl)(methyl)amino)methyl)piperidine-1-carboxylate (100 mg, 0.26 mmol) was dissolved in ethyl acetate (10 mL), 10% palladium on carbon (32 mg, 0.03 mmol) was added, and the resulting mixture was heated to 70 °C under hydrogen atmosphere and stirred overnight. After the reaction liquid was cooled to room temperature, the reaction liquid was filtered through celite, and the filtrate was directly concentrated under reduced pressure to give tert-butyl 4-((methylamino)methyl)piperidine-1-carboxylate.

LC-MS: (ESI, m/z): [M+H]⁺ = 229.2.

### Step 13: preparation of tert-butyl 4-(((2-((1R,4R)-4-(4-(5-((1R,4R)-2-oxa-5-azabicyclo[2.2.1]heptan-5-yl)pyrazolo[1,5-a]pyrimidine-3-carboxamino)-3-(difluoromethyl)-1H-pyrazol-1-yl)cyclohexyl)ethyl)(methyl)amino)methyl)piperidine-1-carboxylate

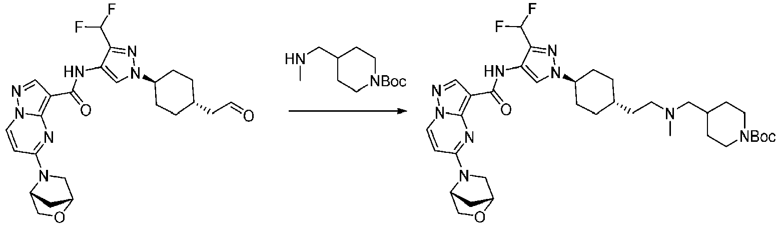

5-((1*R*,4*R*)-2-Oxa-5-azabicyclo[2.2.1]heptan-5-yl)-*N*-(3-(difluoromethyl)-1-((1*R*,4*R*)-4-(2-oxoethyl)cyclohexyl)-1*H*-pyrazol-4-yl)pyrazolo[1,5-*a*]pyrimidine-3-carboxamide (60 mg, 0.12 mmol) and tert-butyl 4-((methylamino)methyl)piperidine-1-carboxylate (60 mg, 0.26 mmol) were dissolved in tetrahydrofuran (10 mL). Sodium triacetoxyborohydride (127 mg, 0.6 mmol) was added, and the mixture was stirred at room temperature for 2 h. Water (50 mL) was added to the reaction liquid, and the mixture was extracted with dichloromethane (20 mL × 3). The organic phase was dried over anhydrous sodium sulfate, filtered, and concentrated under reduced pressure. The crude product was purified by column chromatography (methanol:dichloromethane = 1:9) to give tert-butyl 4-(((2-((1R,4R)-4-(4-(5-((1R,4R)-2-oxa-5-azabicyclo[2.2.1]heptan-5-yl)pyrazolo[1,5-*a*]pyrimidine-3-carboxamino)-3-(difluoromethyl)-1*H-*pyrazol-1-yl)cyclohexyl)ethyl)(methyl)amino)methyl)piperidine-1-carboxylate.

LC-MS: (ESI, m/z): [M+H]⁺ = 712.4.

### Step 14: preparation of 5-((1R,4R)-2-oxa-5-azabicyclo[2.2.1]heptan-5-yl)-N-(3-(difluoromethyl)-1-((1R,4R)-4-(2-(methyl(piperidin-4-ylmethyl)amino)ethyl)cyclohexyl)-1H-pyrazol-4-yl)pyrazolo[1,5-a]pyrimidine-3-carboxamide

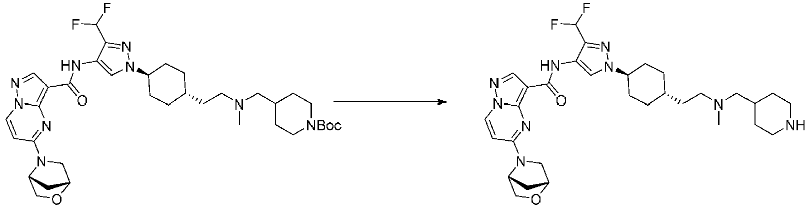

*tert*-Butyl 4-(((2-((1*R*,4*R*)-4-(4-(5-((1*R*,4*R*)-2-oxa-5-azabicyclo[2.2.1]heptan-5-yl)pyrazolo[1,5-*a*]pyrimidine-3-carboxamino)-3-(difluoromethyl)-1*H*-pyrazol-1-yl)cyclohexyl)ethyl)(methyl)amino)methyl)piperidine-1-carboxylate (70 mg, 0.10 mmol) was dissolved in dichloromethane (3 mL). Then trifluoroacetic acid (1 mL) was added, and the mixture was stirred at room temperature for 1 h. The reaction liquid was directly concentrated under reduced pressure to give 5-((1*R*,4*R*)-2-oxa-5-azabicyclo[2.2.1]heptan-5-yl)-*N*-(3-(difluoromethyl)-1-((1*R*,4*R*)-4-(2-(methyl(piperidin-4-ylmethyl)amino)ethyl)cyclohexyl)-1*H*-pyrazol-4-yl)pyrazolo[1,5-*a*]pyrimidine-3-carboxamide, which was directly used in the next step without purification.

LC-MS: (ESI, m/z): [M+H]⁺ = 612.3.

### Step 15: preparation of 5-((1R,4R)-2-oxa-5-aza[2.2.1]heptan-5-yl)-N-(1-((1R,4R)-4-(2-(((1-(4-chloro-3-(2,4-dioxotetrahydropyrimidin-1(2H)-yl)benzoyl)piperidin-4-yl)methyl)(methyl)amino)ethyl)cyclohexyl)-3-(difluoromethyl)-1H-pyrazol-4-yl)pyrazolo[1,5-a]pyrimidine-3-carboxamide

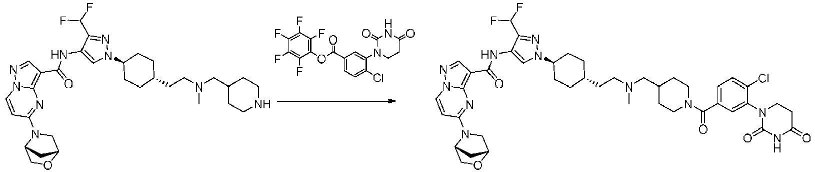

5-((1*R*,4*R*)-2-Oxa-5-azabicyclo[2.2.1]heptan-5-yl)-*N-*(3-(difluoromethyl)-1-((1*R*,4*R*)-4-(2-(methyl(piperidin-4-ylmethyl)amino)ethyl)cyclohexyl)-1*H*-pyrazol-4-yl)pyrazolo[1,5-*a*]pyrimidine-3-carboxamide (70 mg, 0.10 mmol) was dissolved in dimethyl sulfoxide (3 mL). Pentafluorophenyl 4-chloro-3-(2,4-dioxotetrahydropyrimidin-1(2*H*)-yl)benzoate (43 mg, 0.10 mmol) and *N*,*N-*diisopropylethylamine (130 mg, 1.0 mmol) were added while stirring, and the mixture was stirred at room temperature for 2 h. Water (50 mL) was added to the reaction liquid. The mixture was filtered, and the filter cake was purified by pre-HPLC (CH₃CN/0.10% aqueous FA solution, 5%-95%) to give 5-((1*R*,4*R*)-2-oxa-5-aza[2.2.1]heptan-5-yl)-*N*-(1-((1*R*,4*R*)-4-(2-(((1-(4-chloro-3-(2,4-dioxotetrahydropyrimidin-1(2*H*)-yl)benzoyl)piperidin-4-yl)methyl)(methyl)amino)ethyl)cyclohexyl)-3-(difluoromethyl)-1*H*-pyrazol-4-yl)pyrazolo[1,5-*a*]pyrimidine-3-carboxamide.

LC-MS: (ESI, m/z): [M+H]⁺ = 862.3.

¹H NMR (400 MHz, DMSO-*d*₆) δ 10.50 (s, 1H), 9.50 (d, *J* = 6.3 Hz, 1H), 8.79 (d, *J* = 7.7 Hz, 1H), 8.42 (d, *J* = 4.0 Hz, 1H), 8.25 (d, *J* = 5.5 Hz, 1H), 7.63 (d, *J* = 8.2 Hz, 1H), 7.54 (d, *J* = 1.8 Hz, 1H), 7.38 (d, *J* = 8.3,1.9 Hz, 1H), 7.26-6.97 (m, 1H), 6.88-6.45 (m, 1H), 5.28-5.08 (m, 1H), 4.77 (d, *J* = 16.7 Hz, 1H), 4.50-4.38 (m, 1H), 4.30-4.22 (m, 1H), 3.84-3.71 (m, 3H), 3.66-3.58 (m, 3H), 3.48-3.43 (m, 1H), 3.10-3.02 (m, 1H), 2.84-2.69 (m, 3H), 2.35-2.25 (m, 2H), 2.18-2.08 (m, 5H), 2.08-1.91 (m, 4H), 1.85-1.70 (m, 4H), 1.70-1.55 (m, 4H), 1.49-1.40 (m, 4H), 1.20-1.04 (m, 2H).

### Example 47: 5-((S)-3-aminopiperidin-1-yl)-N-(1-((1R,4S)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)pyrazolo[1,5-a]pyrimidine-3-carboxamide

### Step 1: preparation of tert-butyl 4-(2-((((9H-fluoren-9-yl)methoxy)carbonyl)(methyl)amino)ethyl)piperidine-1-carboxylate

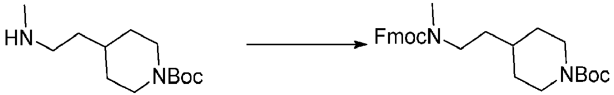

tert-Butyl 4-(2-(methylamino)ethyl)piperidine-1-carboxylate (240 mg, 1.0 mmol) was dissolved in DCM (20 mL). FmocCl (311 mg, 1.2 mmol) and pyridine (237 mg, 3.0 mmol) were added, and the mixture was reacted at room temperature overnight. The reaction liquid was diluted with water (50 mL) and extracted with DCM (30 mL × 3). The organic phase was dried over anhydrous sodium sulfate, filtered, and concentrated under reduced pressure. The crude product was purified by column chromatography (EA:PE = 0-4:1) to give tert-butyl 4-(2-((((9*H*-fluoren-9-yl)methoxy)carbonyl)(methyl)amino)ethyl)piperidine-1-carboxylate.

LC-MS: (ESI, m/z): [M+Na]⁺ = 487.3.

### Step 2: preparation of (9H-fluoren-9-yl)methyl methyl(2-(piperidin-4-yl)ethyl)carbamate

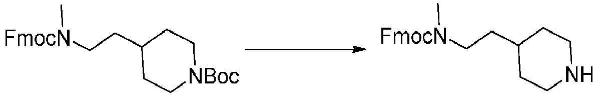

*tert*-Butyl 4-(2-((((9*H*-fluoren-9-yl)methoxy)carbonyl)(methyl)amino)ethyl)piperidine-1-carboxylate (210 mg, 0.45 mmol) was dissolved in DCM (4 mL). TFA (1 mL) was added, and the mixture was reacted at room temperature for 1 h. The reaction liquid was directly concentrated under reduced pressure to give (9H-fluoren-9-yl)methyl methyl(2-(piperidin-4-yl)ethyl)carbamate, which was directly used in the next step.

LC-MS: (ESI, m/z): [M+H]⁺ = 365.2.

### Step 3: preparation of 1-(2-chloro-5-(4-(2-(methylamino)ethyl)piperidine-1-carbonyl)phenyl)dihydropyrimidine-2,4(1H,3H)-dione

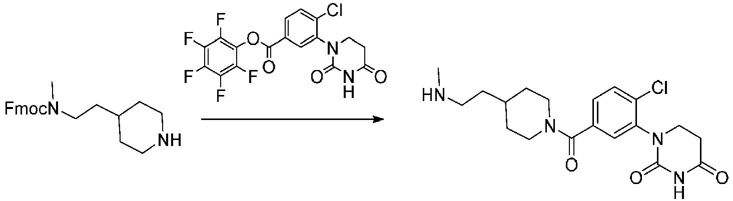

(9*H*-Fluoren-9-yl)methyl methyl(2-(piperidin-4-yl)ethyl)carbamate (210 mg, 0.45 mmol) was dissolved in DMSO (5 mL). DIEA (264 mg, 2.05 mmol) and pentafluorophenyl 4-chloro-3-(2,4-dioxotetrahydropyrimidin-1(2*H*)-yl)benzoate (178 mg, 0.41 mmol) were added, and the mixture was reacted at room temperature overnight. The reaction solution was purified by reversed-phase chromatography (45% ACN/0.1% aqueous NH₄HCO₃ solution) to give 1-(2-chloro-5-(4-(2-(methylamino)ethyl)piperidine-1-carbonyl)phenyl)dihydropyrimidine-2,4(1*H*,3*H*)-dione.

LC-MS: (ESI, m/z): [M+H]⁺ = 393.1.

### Step 4: preparation of tert-butyl ((S)-1-(3-((1-((1R,4S)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)carbamoyl)pyrazolo[1,5-a]pyrimidin-5-yl)piperidin-3-yl)carbamate

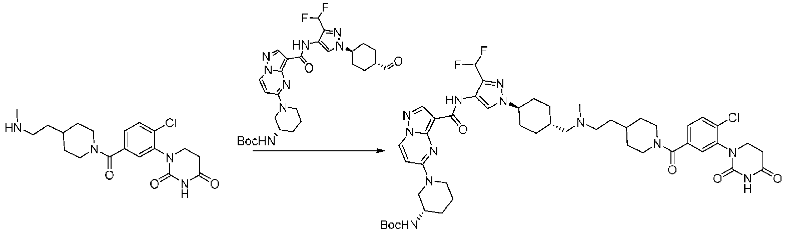

1-(2-Chloro-5-(4-(2-(methylamino)ethyl)piperidine-1-carbonyl)phenyl)dihydropyrimidine-2,4(1*H*,3*H*)-dione (50 mg, 0.13 mmol) was dissolved in THF (10 mL). *tert*-Butyl ((*S*)-1-(3-((3-(difluoromethyl)-1-((1*R*,4*S*)-4-formylcyclohexyl)-1*H*-pyrazol-4-yl)carbamoyl)pyrazolo[1,5-*a*]pyrimidin-5-yl)piperidin-3-yl)carbamate (30 mg, 0.05 mmol) and STAB (83 mg, 0.39 mmol) were added. After the mixture was reacted for 0.5 h, tert-butyl ((*S*)-1-(3-((3-(difluoromethyl)-1-((1*R*,4*S*)-4-formylcyclohexyl)-1*H*-pyrazol-4-yl)carbamoyl)pyrazolo[1,5-*a*]pyrimidin-5-yl)piperidin-3-yl)carbamate (60 mg, 0.10 mmol) was added in two portions within 1 h, and the mixture was reacted at room temperature for 2 h. The reaction liquid was diluted with water (50 mL), and the mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 0-1:9) to give *tert*-butyl ((*S*)-1-(3-((1-((1*R*,4*S*)-4-(((2-(1-(4-chloro-3-(2,4-dioxotetrahydropyrimidin-1(2*H*)-yl)benzoyl)piperidin-4-yl)ethyl)(methyl)amino)methyl)cyclohexyl)-3-(difluoromethyl)-1*H*-pyrazol-4-yl)carbamoyl)pyrazolo[1,5-*a*]pyrimidin-5-yl)piperidin-3-yl)carbamate.

LC-MS: (ESI, m/z): [M+H]⁺ = 963.3.

### Step 5: preparation of 5-((S)-3-aminopiperidin-1-yl)-N-(1-((1R,4S)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)pyrazolo[1,5-a]pyrimidine-3-carboxamide

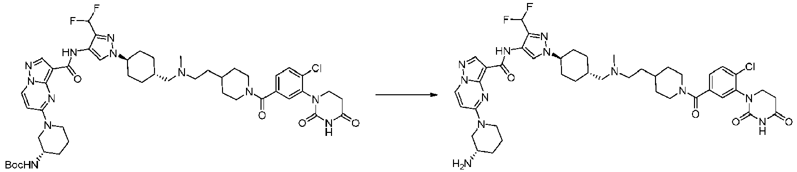

*tert*-Butyl ((*S*)-1-(3-((1-((1*R*,4*S*)-4-(((2-(1-(4-chloro-3-(2,4-dioxotetrahydropyrimidin-1(2*H*)-yl)benzoyl)piperidin-4-yl)ethyl)(methyl)amino)methyl)cyclohexyl)-3-(difluoromethyl)-1*H*-pyrazol-4-yl)carbamoyl)pyrazolo[1,5-*a*]pyrimidin-5-yl)piperidin-3-yl)carbamate (100 mg, 0.10 mmol) was dissolved in DCM (5 mL). TFA (1 mL) was added, and the mixture was reacted at room temperature for 1 h. The reaction liquid was directly concentrated under reduced pressure. Then a saturated NaHCO₃ solution was added to adjust the pH to 8-9, and the mixture was extracted with MeOH/DCM (10%, 20 mL × 3). The organic phase was dried over anhydrous sodium sulfate, filtered, and concentrated. The crude product was purified by preparative reversed-phase chromatography to give 5-((*S*)-3-aminopiperidin-1-yl)-*N*-(1-((1*R*,4*S*)-4-(((2-(1-(4-chloro-3-(2,4-dioxotetrahydropyrimidin-1(2*H*)-yl)benzoyl)piperidin-4-yl)ethyl)(methyl)amino)methyl)cyclohexyl)-3-(difluoromethyl)-1*H*-pyrazol-4-yl)pyrazolo[1,5-*a*]pyrimidine-3-carboxamide.

LC-MS: (ESI, m/z): [M+H]⁺ = 863.1.

¹H NMR (400 MHz, DMSO-*d*₆) δ 11.18-9.87 (s, 1H), 9.36 (s, 1H), 8.74 (d, *J* = 8.0 Hz, 1H), 8.37 (s, 1H), 8.24 (s, 1H), 7.63 (d, *J* = 8.2 Hz, 1H), 7.54 (d, *J* = 2.0 Hz, 1H), 7.38 (dd, *J* = 8.2, 2.0 Hz, 1H), 7.26-6.97 (m, 1H), 6.87 (d, *J* = 8.0 Hz, 1H), 4.50-4.25 (m, 2H), 4.18 (t, *J* = 11.9 Hz, 2H), 3.80-3.70 (m, 1H), 3.70-3.51 (m, 2H), 3.26-3.18 (m, 1H), 3.08-2.97 (m, 2H), 2.85-2.64 (m, 5H), 2.30 (t, *J* = 7.2 Hz, 2H), 2.14-2.07 (m, 5H), 2.07-1.97 (m, 2H), 1.95-1.83 (m, 3H), 1.80-1.30 (m, 12H), 1.18-0.95 (m, 4H).

### Example 48: N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)-5-(2-oxa-6-azaspiro[3.3]heptan-6-yl)pyrazolo[1,5-a]pyrimidine-3-carboxamide

### Step 1: preparation of tert-butyl 9-(4-chloro-3-(2,4-dioxotetrahydropyrimidin-1(2H)-yl)benzoyl)-3,9-diazaspiro[5.5]undecane-3-carboxylate

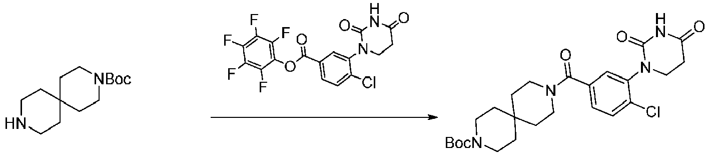

*tert*-Butyl 3,9-diazaspiro[5.5]undecane-3-carboxylate (100 mg, 0.39 mmol) was dissolved in dimethyl sulfoxide (3 mL). Pentafluorophenyl 4-chloro-3-(2,4-dioxotetrahydropyrimidin-1(2*H*-yl)benzoate (205 mg, 0.47 mmol) and *N*,*N-*diisopropylethylamine (153 mg, 1.18 mmol) were added while stirring, and the mixture was stirred at room temperature overnight. Water (50 mL) was added to the reaction liquid, and the mixture was extracted with ethyl acetate (30 mL × 3). The organic phase was washed with saturated brine (100 mL), dried over anhydrous sodium sulfate, filtered, and concentrated under reduced pressure. The crude product was purified by column chromatography (ethyl acetate) to give tert-butyl 9-(4-chloro-3-(2,4-dioxotetrahydropyrimidin-1(2*H*)-yl)benzoyl)-3,9-diazaspiro[5.5]undecane-3-carboxylate.

LC-MS: (ESI, m/z): [M-Boc]⁺ = 405.1.

### Step 2: preparation of 1-(2-chloro-5-(3,9-diazaspiro[5.5]undecane-3-carbonyl)phenyl)dihydropyrimidine-2,4(1H,3H)-dione

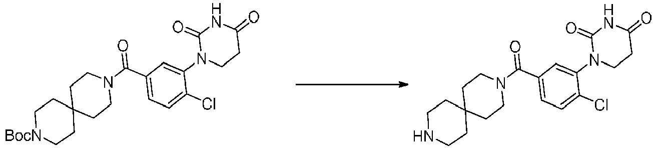

*tert*-Butyl 9-(4-chloro-3-(2,4-dioxotetrahydropyrimidin-1(2*H*)-yl)benzoyl)-3,9-diazaspiro[5.5]undecane-3-carboxylate (170 mg, 0.34 mmol) was dissolved in dichloromethane (2.5 mL). Trifluoroacetic acid (0.5 mL) was added, and the mixture was stirred at room temperature for 2 h. The reaction liquid was directly concentrated under reduced pressure to give 1-(2-chloro-5-(3,9-diazaspiro[5.5]undecane-3-carbonyl)phenyl)dihydropyrimidine-2,4(1*H*,3*H*)-dione, which was directly used in the next step without purification.

LC-MS: (ESI, m/z): [M+H]⁺ = 405.1.

### Step 3: preparation of N-(1-((1R,4R)-4-((9-(4-chloro-3-(2,4-dioxotetrahydropyrimidin-1(2H-yl)benzoyl)-3,9-diazaspiro[5.5]undecan-3-yl)methyl)cyclohexyl)-3-(difluoromethyl)-1H-pyrazol-4-yl)-5-(2-oxa-6-azaspiro[3.3]heptan-6-yl)pyrazolo[1,5-a]pyrimidine-3-carboxamide

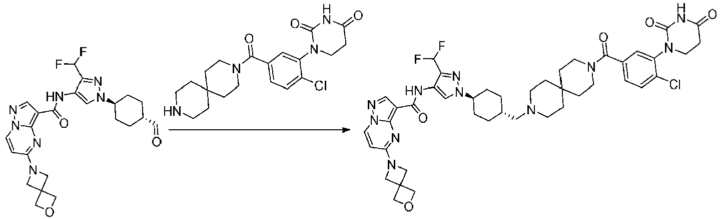

*N-*(3-(Difluoromethyl)-1-((1*R*,4*R*)-4-formylcyclohexyl)-1*H*-pyrazol-4-yl)-5-(2-oxa-6-azaspiro[3.3]heptan-6-yl)pyrazolo[1,5-*a*]pyrimidine-3-carboxamide (50 mg, 0.10 mmol) and 1-(2-chloro-5-(3,9-diazaspiro[5.5]undecane-3-carbonyl)phenyl)dihydropyrimidine-2,4(1*H*,3*H*)-dione (42 mg, 0.10 mmol) were dissolved in dichloroethane (10 mL). Sodium triacetoxyborohydride (44 mg, 0.20 mmol) was added, and the mixture was heated to 70 °C and stirred overnight. Water (50 mL) was added to the reaction liquid, and the mixture was extracted with dichloromethane (20 mL × 3), dried over anhydrous sodium sulfate, filtered, and concentrated under reduced pressure. The crude product was purified by pre-HPLC (CH₃CN/0.08% aqueous NH₄HCO₃ solution, 5%-95%) to give *N*-(1-((1*R*,4*R*)-4-((9-(4-chloro-3-(2,4-dioxotetrahydropyrimidin-1(2*H*)-yl)benzoyl)-3,9-diazaspiro[5.5]undecan-3-yl)methyl)cyclohexyl)-3-(difluoromethyl)-1*H*-pyrazol-4-yl)-5-(2-oxa-6-azaspiro[3.3]heptan-6-yl)pyrazolo[1,5-*a*]pyrimidine-3-carboxamide.

LC-MS: (ESI, m/z): [M+H]⁺ = 874.2.

¹H NMR (400 MHz, CDCl₃) δ 9.76 (s, 1H), 8.44 (s, 1H), 8.39 (d, *J* = 5.3 Hz, 2H), 8.30 (d, *J* = 7.6 Hz, 1H), 7.58-7.48 (m, 2H), 7.43 (d, *J* = 1.8 Hz, 1H), 7.38-7.34 (m, 1H), 6.97-6.65 (m, 1H), 6.02 (d, *J* = 7.5 Hz, 1H), 4.98-4.85 (m, 4H), 4.47-4.37 (m, 4H), 4.13-4.06 (m, 1H), 3.86-3.74 (m, 4H), 3.46-3.38 (m, 2H), 3.00-2.74 (m, 3H), 2.39-2.29 (m, 3H), 2.29-2.10 (m, 3H), 2.10-2.00 (m, 2H), 1.92-1.76 (m, 3H), 1.40-1.10 (m, 10H), 0.95-0.85 (m, 1H).

### Example 49: (S)-5-(3-aminopiperidin-1-yl)-N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)pyrazolo[1,5-a]pyrimidine-3-carboxamide

### Step 1: preparation of tert-butyl (S)-(1-(3-((3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)carbamoyl)pyrazolo[1,5-a]pyrimidin-5-yl)piperidin-3-yl)carboxylate

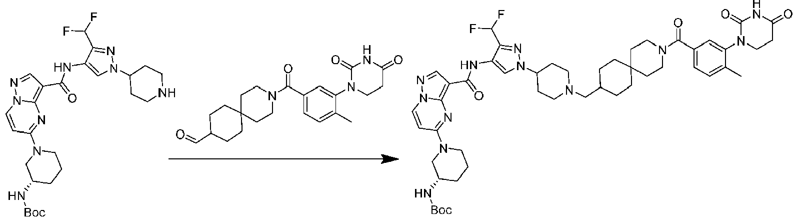

*tert*-Butyl (*S*)-(1-(3-((3-(difluoromethyl)-1-(piperidin-4-yl)-1*H*-pyrazol-4-yl)carbamoyl)pyrazolo[1,5-a]pyrimidin-5-yl)piperidin-3-yl)carbamate (45 mg, 0.08 mmol) and 3-(3-(2,4-dioxotetrahydropyrimidin-1(2*H*)-yl)-4-methylbenzoyl)-3-azaspiro[5.5]undecane-9-carbaldehyde (33 mg, 0.08 mmol) were dissolved in DMF (2 mL). NaBH(OAc)₃ (51 mg, 0.24 mmol) was added, and the mixture was stirred at room temperature for 2 h. Water (50 mL) was added to the reaction liquid, and the mixture was extracted with ethyl acetate (30 mL × 3). The upper organic phases were combined and washed with saturated brine (50 mL), dried over anhydrous sodium sulfate, filtered, and concentrated under reduced pressure. The crude product was purified by normal phase column chromatography to give tert-butyl (*S*)-(1-(3-((3-(difluoromethyl)-1-(1-((3-(3-(2,4-dioxotetrahydropyrimidin-1(*2H*)-yl)-4-methylbenzoyl)-3-azaspiro[5.5]undecan-9-yl)methyl)piperidin-4-yl)-1*H*-pyrazol-4-yl)carbamoyl)pyrazolo[1,5-*a*]pyrimidin-5-yl)piperidin-3-yl)carboxylate.

LC-MS: (ESI, m/z): [M+H]⁺ = 955.5.

### Step 2: preparation of (S)-5-(3-aminopiperidin-1-yl)-N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)pyrazolo[1,5-a]pyrimidine-3-carboxamide

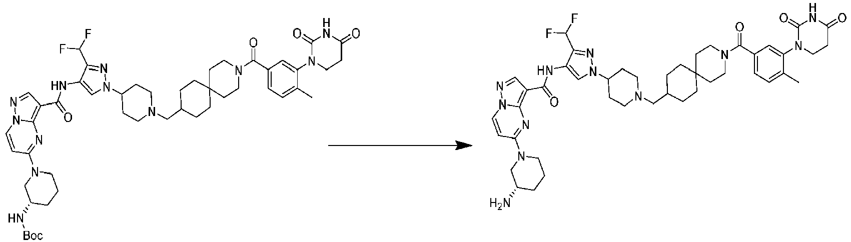

*tert*-Butyl (*S*)-(1-(3-((3-(difluoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H-*pyrazol-4-yl)carbamoyl)pyrazolo[1,5-*a*]pyrimidin-5-yl)piperidin-3-yl)carboxylate (30 mg, 0.03 mmol) was dissolved in DCM (0.5 mL). Then TFA (0.1 mL) was added, and the mixture was stirred at room temperature for 1 h. A saturated aqueous NaHCO₃ solution was added to the reaction liquid until the pH of the solution became slightly basic, and the mixture was extracted with a mixed organic solvent (MeOH:DCM = 1:10, 30 mL × 3), dried over anhydrous sodium sulfate, filtered, and concentrated under reduced pressure. The crude product was purified by p-HPLC to give (*S*)-5-(3-aminopiperidin-1-yl)-*N*-(3-(difluoromethyl)-1-(1-((3-(3-(2,4-dioxotetrahydropyrimidin-1(2*H*-yl)-4-methylbenzoyl)-3-azaspiro[5.5]undecan-9-yl)methyl)piperidin-4-yl)-1*H*-pyrazol-4-yl)pyrazolo[1,5-*a*]pyrimidine-3-carboxamide.

LC-MS: (ESI, m/z): [M+H]⁺ = 855.7.

¹H NMR (400 MHz, MeOD) δ 8.49 (d, *J* = 8.0 Hz, 1H), 8.35 (s, 1H), 8.29 (s, 1H), 7.40 (d, *J* = 7.9 Hz, 1H), 7.35-7.29 (m, 2H), 7.04-6.74 (m, 2H), 4.60-4.50 (m, 1H), 4.50-4.25 (m, 2H), 4.25-4.15 (m, 1H), 3.97-3.79 (m, 1H), 3.80-3.63 (m, 3H), 3.50-3.38 (m, 3H), 3.15-3.08 (m, 1H), 3.06-3.01 (m, 2H), 2.94-2.79 (m, 3H), 2.31 (s, 3H), 2.27-2.21 (m, 2H), 2.21-2.04 (m, 7H), 1.93-1.83 (m, 1H), 1.83-1.76 (m, 2H), 1.70-1.44 (m, 8H), 1.22-1.02 (m, 4H).

### Example 50: N-(3-(difluoromethyl)-1-(1-((3-(3-(2,4-dioxotetrahydropyrimidin-1(2H-yl)-4-methylbenzoyl)-3-azaspiro[5.5]undecan-9-yl)methyl)piperidin-4-yl)-1H-pyrazol-4-yl)-5-(2-oxa-6-azaspiro[3.3]heptan-6-yl)pyrazolo[1,5-a]pyrimidine-3-carboxamide

### Step 1: preparation of tert-butyl 4-(4-(5-(2-oxa-6-azaspiro[33]heptan-6-yl)pyrazolo[1,5-α]pyrimidine-3-carboxamido)-3-(difluoromethyl)-1H-pyrazol-1-yl)piperidine-1-carboxylate

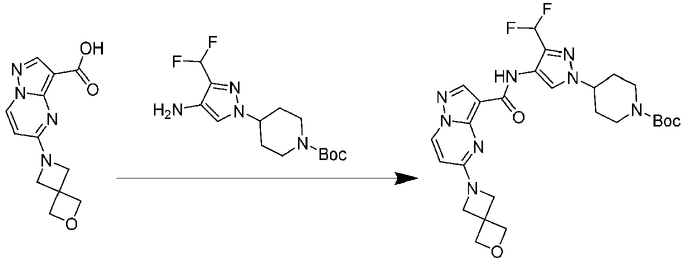

5-(2-Oxa-6-azaspiro[3.3]heptan-6-yl)pyrazolo[1,5-*a*]pyrimidine-3-carboxylic acid (130 mg, 0.5 mmol), benzyl 4-(4-amino-3-(difluoromethyl)-1*H*-pyrazol-pyrazol-1-yl)piperidine-1-carboxylate (158 mg, 0.5 mmol), and Py-BOP (780 mg, 1.5 mmol) were dissolved in DMF (6 mL), then DIEA (780 mg, 1.5 mmol) was added, and the mixture was stirred at room temperature for 16 h. Water (50 mL) was added to the reaction liquid, and the mixture was extracted with ethyl acetate (30 mL × 3). The upper organic phases were combined, washed with saturated brine (50 mL), dried over anhydrous sodium sulfate, filtered, and concentrated under reduced pressure. The crude product was purified by normal phase column chromatography to give tert-butyl 4-(4-(5-(2-oxa-6-azaspiro[3.3]heptan-6-yl)pyrazolo[1,5-*a*]pyrimidine-3-carboxamido)-3-(difluoromethyl)-1*H-*pyrazol-1-yl)piperidine-1-carboxylate.

LC-MS: (ESI, m/z): [M+H]⁺ = 559.2.

### Step 2: preparation of N-(3-(difluoromethyl)-1-(piperidin-4-yl)-1H-pyrazol-4-yl)-5-(2-oxa-6-azaspiro[3.3]heptan-6-yl)pyrazolo[1,5-a]pyrimidine-3-carboxamide

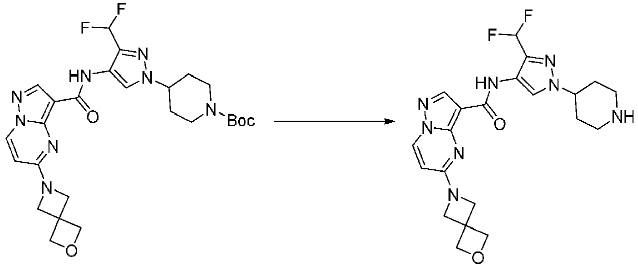

*tert*-Butyl 4-(4-(5-(2-oxa-6-azaspiro[3.3]heptan-6-yl)pyrazolo[1,5-*a*]pyrimidine-3-carboxamido)-3-(difluoromethyl)-1*H*-pyrazol-1-yl)piperidine-1-carboxylate (250 mg, 0.45 mmol) was dissolved in DCM (4 mL). Then TFA (1 mL) was added, and the mixture was stirred at room temperature for 1 h. The reaction liquid was directly concentrated under reduced pressure to give a crude product of *N*-(3-(difluoromethyl)-1-(piperidin-4-yl)-1*H*-pyrazol-4-yl)-5-(2-oxa-6-azaspiro[3.3]heptan-6-yl)pyrazolo[1,5-*a*]pyrimidine-3-carboxamide.

LC-MS: (ESI, m/z): [M+H]⁺ = 459.3.

### Step 3: preparation of N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)-5-(2-oxa-6-azaspiro[3.3]heptan-6-yl)pyrazolo[1,5-a]pyrimidine-3-carboxamide

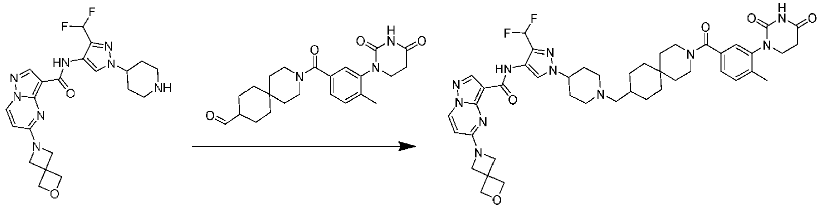

*N*-(3-(Difluoromethyl)-1-(piperidin-4-yl)-1*H*-pyrazol-4-yl)-5-(2-oxa-6-azaspiro[3.3]heptan-6-yl)pyrazolo[1,5-*a*]pyrimidine-3-carboxamide (114 mg, 0.25 mmol) and 3-(3-(2,4-dioxotetrahydropyrimidin-1(2*H*)-yl)-4-methylbenzoyl)-3-azaspiro[5.5]undecane-9-carbaldehyde (102 mg, 0.25 mmol) were dissolved in THF (5 mL). Sodium triacetoxyborohydride (160 mg, 0.75 mmol) was added, and the mixture was stirred at room temperature for 16 h. Water (50 mL) was added to the reaction liquid, and the mixture was extracted with ethyl acetate (30 mL × 3). The upper organic phases were combined and washed with saturated brine (50 mL), dried over anhydrous sodium sulfate, filtered, and concentrated under reduced pressure. The crude product was purified by prep-HPLC to give *N*-(3-(-difluoromethyl)-1-(1-((3-(3-(2,4-dioxotetrahydropyrimidin 1(2*H*-yl)-4-methylbenzoyl)-3-azaspiro[5.5]undecan-9-yl)methyl)piperidin-4-yl)-1*H*-pyrazol-4-yl)-5-(2-oxa-6-azaspiro[3.3]heptan-6-yl)pyrazolo[1,5-*a*]pyrimidine-3-carboxamide as a white solid.

LC-MS: (ESI, m/z): [M+H]⁺ = 854.6.

¹H NMR (400 MHz, MeOD) δ 8.47 (d, *J* = 7.8 Hz, 1H), 8.35 (s, 1H), 8.27 (s, 1H), 7.40 (d, *J* = 7.4 Hz, 1H), 7.36-7.27 (m, 2H), 7.05-6.95 (m, 1H), 6.31 (d, *J* = 8.0 Hz, 1H), 4.62-4.50 (m, 2H), 4.50-4.39 (m, 4H), 4.26-4.11 (m, 1H), 3.95-3.82 (m, 1H), 3.77-3.60 (m, 3H), 3.49-3.40 (m, 2H), 3.05-2.97 (m, 2H), 2.89-2.79 (m, 2H), 2.37-2.20 (m, 5H), 2.20-2.09 (m, 6H), 1.83-1.75 (m, 2H), 1.71-1.41 (m, 7H), 1.37-1.27 (m, 2H), 1.24-1.08 (m, 4H).

### Example 51: 5-((1R,4R)-2-oxa-5-azabicyclo[2.2.1]heptan-5-yl)-N-(1-(1-(4-(4-(4-chloro-3-(2,4-dioxotetrahydropyrimidin-1(2H)-yl)phenyl)piperazin-1-yl)butyl)piperidin-4-yl)-3-(difluoromethyl)-1H-pyrazol-4-yl)pyrazolo[1,5-a]pyrimidine-3-carboxamide

### Step 1: preparation of tert-butyl 4-(4-chloro-3-nitrophenyl)piperazine-1-carboxylate

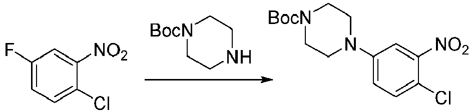

1- Chloro-4-fluoro-2-nitrobenzene (10.0 g, 56.81 mmol) was dissolved in DMSO (100 mL). Then *tert*-butyl piperazine-1-carboxylate (21 g, 85.27 mmol) and DIEA (52 mL) were added, and the mixture was heated to 110 °C and reacted for 48 h. Water (1000 mL) was added to the reaction liquid, and the mixture was stirred for 10 min and extracted with ethyl acetate (600 mL × 3). The organic phase was washed with saturated brine (1000 mL), dried over anhydrous sodium sulfate, filtered, and concentrated under reduced pressure. The crude product was purified by column chromatography (PE:EA = 5:1) to give tert-butyl 4-(4-chloro-3-nitrophenyl)piperazine-1-carboxylate.

LC-MS: (ESI, m/z): [M-t-Bu]⁺ = 286.0.

### Step 2: preparation of 1-(4-chloro-3-nitrophenyl)piperazine

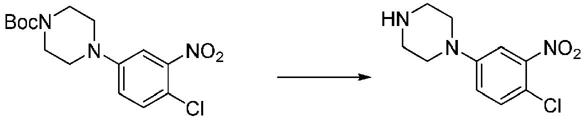

tert-Butyl 4-(4-chloro-3-nitrophenyl)piperazine-1-carboxylate (3.73 g, 10.9 mmol) was dissolved in DCM (30 mL). Then TFA (10 mL) was added, and the mixture was stirred at room temperature for 1 h. The reaction liquid was directly concentrated under reduced pressure to give 1-(4-chlorine-3-nitrophenyl)piperazine.

LC-MS: (ESI, m/z): [M+H]⁺ = 242.0.

### Step 3: preparation of 4-(4-(4-chloro-3-nitrophenyl)piperazin-1-yl)butyl-1-ol

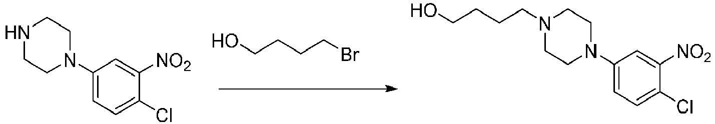

1-(4-Chloro-3-nitrophenyl)piperazine (4.0 g, 10.9 mmol) was dissolved in DMF (25 mL). Then 4-bromobutyl-1-ol (1.75 g, 11.4 mmol), potassium carbonate (4.51 g, 32.7 mmol), and KI (1.89 g, 11.4 mmol) were added, and the reaction liquid was heated to 60 °C and stirred overnight. The reaction liquid was diluted with water (200 mL), and the mixture was stirred for 10 min and extracted with ethyl acetate (100 mL × 3). The organic phase was washed with saturated brine (150 mL), dried over anhydrous sodium sulfate, filtered, and concentrated under reduced pressure. The crude product was purified by column chromatography (DCM:MeOH = 1:19) to give 4-(4-(4-chloro-3-nitrophenyl)piperazin-1-yl)butyl-1-ol.

LC-MS: (ESI, m/z): [M+H]⁺ = 314.1.

### Step 4: preparation of 4-(4-(4-chloro-3-nitrophenyl)piperazin-1-yl)butylbenzoate

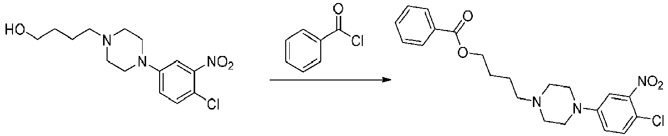

4-(4-(4-Chloro-3-nitrophenyl)piperazin-1-yl)butyl-1-ol (1.0 g, 3.19 mmol) was dissolved in THF (15 mL). Then benzoyl chloride (0.58 g, 4.15 mmol) and TEA (0.97 g, 9.57 mmol) were added, and the reaction liquid was heated to 70 °C and stirred overnight. Water (50 mL) was added to the reaction liquid, and the mixture was extracted with ethyl acetate (50 mL × 3). The organic phase was washed with saturated brine (50 mL), dried over anhydrous sodium sulfate, filtered, and concentrated under reduced pressure. The crude product was purified by column chromatography (DCM:MeOH = 1:13) to give 4-(4-(4-chloro-3-nitrophenyl)piperazin-1-yl)butylbenzoate.

LC-MS: (ESI, m/z): [M+H]⁺ = 418.1.

### Step 5: preparation of 4-(4-(3-amino-4-chlorophenyl)piperazin-1-yl)butylbenzoate

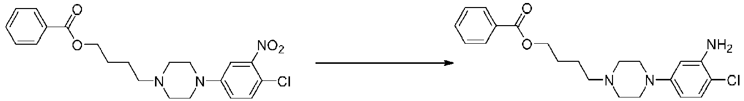

4-(4-(4-Chloro-3-nitrophenyl)piperazin-1-yl)butylbenzoate (1.2 g, 2.87 mmol) was dissolved in ethanol (15 mL). Hydrazine hydrate (1.5 mL) and Raney-Ni (solid-liquid mixture, 1.5 mL) were added, and the mixture was reacted at room temperature for 1 h. The mixture was filtered, and the filtrate was concentrated under reduced pressure to give 4-(4-(3-amino-4-chlorphenyl)piperazin-1-yl)butylbenzoate.

LC-MS: (ESI, m/z): [M+H]⁺ = 388.1.

### Step 6: preparation of 3-((5-(4-(4-(benzoyloxy)butyl)piperazin-1-yl)-2-chlorophenyl)amino)propionic acid

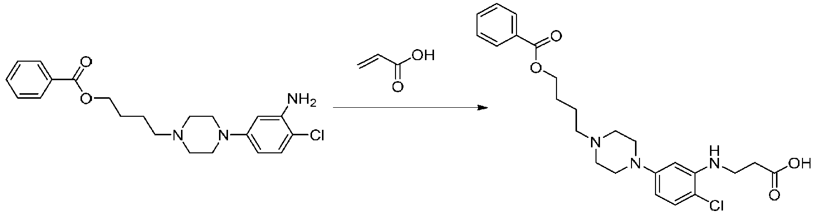

4-(4-(3-Amino-4-chlorophenyl)piperazin-1-yl)butylbenzoate (1.0 g, 2.6 mmol) was dissolved in an acetic acid/water mixture (1/5, 12 mL). Then acrylic acid (0.558 g, 7.75 mmol) was added, and the reaction liquid was heated to 110 °C and stirred overnight. Water (50 mL) was added to the reaction liquid, and the mixture was extracted with ethyl acetate (50 mL × 3). The organic phase was washed with saturated brine (50 mL), dried over anhydrous sodium sulfate, filtered, and concentrated under reduced pressure. The crude product was purified by column chromatography (DCM/MeOH = 12%) to give 3-((5-(4-(4-(benzoyloxy)butyl)piperazin-1-yl)-2-chlorophenyl)amino)propionic acid.

LC-MS: (ESI, m/z): [M+H]⁺ = 460.1.

### Step 7: preparation of 4-(4-(4-chloro-3-(2,4-dioxotetrahydropyrimidin-1(2H)-yl)phenyl)piperazin-1-yl)butylbenzoate

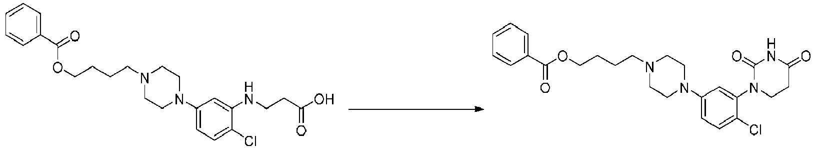

3-((5-(4-(4-(Benzoyloxy)butyl)piperazin-1-yl)-2-chlorophenyl)amino)propionic acid (1.12 g, 2.44 mmol) was dissolved in acetic acid (15 mL). Then urea (2.92 g, 48.8 mmol) was added, and the reaction liquid was heated to 110 °C and stirred for 48 h. Water (50 mL) was added to the reaction liquid, and the mixture was extracted with a MeOH/DCM mixed organic solvent (10%, 50 mL × 3). The organic phase was dried over anhydrous sodium sulfate, filtered, and concentrated under reduced pressure. The crude product was purified by column chromatography (DCM:MeOH = 1:9) to give 4-(4-(4-chloro-3-(2,4-dioxotetrahydropyrimidin-1(2*H*)-yl)phenyl)piperazin-1-yl)butylbenzoate.

LC-MS: (ESI, m/z): [M+H]⁺ = 485.1.

### Step 8: preparation of 1-(2-chloro-5-(4-(4-hydroxybutyl)piperazin-1-yl)phenyl)dihydropyrimidine-2,4(1H,3H)-dione

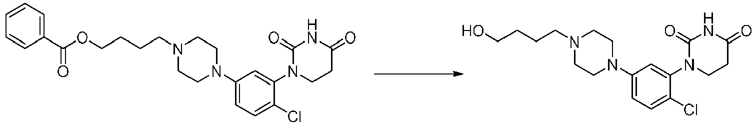

4-(4-(4-Chloro-3-(2,4-dioxotetrahydropyrimidin-1(2*H*)-yl)phenyl)piperazin-1-yl)butylbenzoate (0.74 g, 1.5 mmol) was added to concentrated hydrochloric acid (15 mL), and the mixture was heated to 70 °C and stirred overnight. The reaction liquid was directly concentrated under reduced pressure, and the crude product was purified by reversed-phase column chromatography (ACN/H₂O = 16%, 0.1% aqueous FA solution) to give 1-(2-chloro-5-(4-(4-hydroxybutyl)piperazin-1-yl)phenyl)dihydropyrimidine-2,4(1*H*,3*H*)-dione.

LC-MS: (ESI, m/z): [M+H]⁺ = 381.1.

### Step 9: preparation of 4-(4-(4-chloro-3-(2,4-dioxotetrahydropyrimidin-1(2H)-yl)phenyl)piperazin-1-yl)n-butyraldehyde

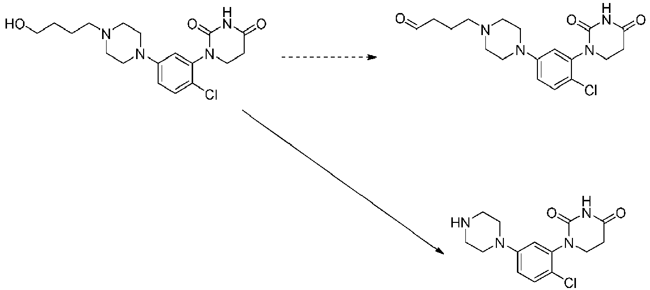

1-(2-Chloro-5-(4-(4-hydroxybutyl)piperazin-1-yl)phenyl)dihydropyrimidine-2,4(1*H*,3*H*)-dione (280 mg, 0.74 mmol) was dissolved in DCM (10 mL). Then Dess-Martin (628 mg, 1.48 mmol) was added, and the reaction liquid was stirred at room temperature overnight. The reaction liquid was directly concentrated under reduced pressure, and the crude product was purified by reversed-phase column chromatography (ACN/H₂O = 16%, 0.1% aqueous FA solution). After analysis, the resulting product was not 4-(4-(4-chloro-3-(2,4-dioxotetrahydropyrimidin-1(2*H*)-yl)phenyl)piperazin-1-yl)*n*-butyraldehyde, but actually 1-(2-chloro-5-(piperazin-1-yl)phenyl)dihydropyrimidine-2,4(1*H*,3*H*)-dione was obtained.

LC-MS: (ESI, m/z): [M+H]⁺ = 309.2.

### Step 10: preparation of 1-(5-(4-(3-(1,3-dioxolan-2-yl)propyl)piperazin-1-yl)-2-chlorophenyl)dihydropyrimidine-2,4(1H,3H)-dione

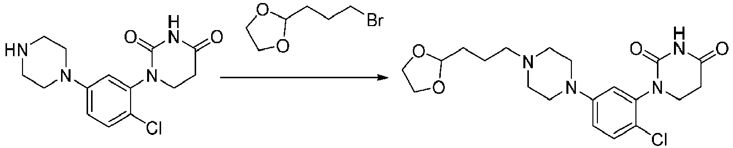

1-(2-Chloro-5-(piperazin-1-yl)phenyl)dihydropyrimidine-2,4(1*H*,3*H*)-dione (65 mg, 0.21 mmol) was dissolved in ACN (10 mL). Then 2-(3-bromopropyl)-1,3-dioxolane (41 mg, 0.21 mmol), K₂CO₃ (87 mg, 0.63 mmol), and KI (18 mg, 0.21 mmol) were added, and the reaction liquid was heated to 80 °C and stirred overnight. The reaction liquid was concentrated under reduced pressure to remove ACN. Then water (50 mL) was added, and the mixture was extracted with ethyl acetate (50 mL × 3). The organic phase was washed with saturated brine (50 mL), dried over anhydrous sodium sulfate, filtered, and concentrated under reduced pressure. The crude product was purified by column chromatography (DCM:MeOH = 1:9) to give 1-(5-(4-(3-(1,3-dioxolan-2-yl)propyl)piperazin-1-yl)-2-chlorophenyl)dihydropyrimidine-2,4(1*H*,3*H*)-dione.

LC-MS: (ESI, m/z): [M+H]⁺ = 423.1.

### Step 11: preparation of 4-(4-(4-chloro-3-(2,4-dioxotetrahydropyrimidin-1(2H)-yl)phenyl)piperazin-1-yl)n-butyraldehyde

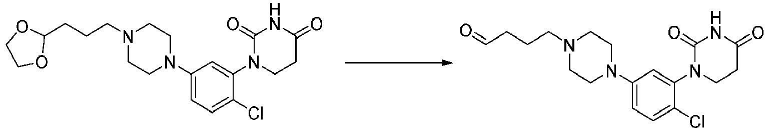

1-(5-(4-(3-(1,3-Dioxolan-2-yl)propyl)piperazin-1-yl)-2-chlorophenyl)dihydropyrimidine-2,4(1*H*,3*H*)-dione (20 mg, 0.047 mmol) was dissolved in DCM (1 mL). Then TFA (1 mL) was added. After the mixture was reacted at room temperature for 48 h, DCE (10 mL) and DIEA (1 mL) were added, and the mixture was concentrated under reduced pressure at room temperature to give 4-(4-(4-chloro-3-(2,4-dioxotetrahydropyrimidin-1(2*H*)-yl)phenyl)piperazin-1-yl)*n*-butyraldehyde (crude, in dichloroethane), which was directly used in the next step.

LC-MS: (ESI, m/z): [M+H]⁺ = 379.1.

### Step 12: preparation of 5-((1R,4R)-2-oxa-5-azabicyclo[2.2.1]heptan-5-yl)-N-(1-(1-(4-(4-(4-chloro-3-(2,4-dioxotetrahydropyrimidin-1(2H)-yl)phenyl)piperazin-1-yl)butyl)piperidin-4-yl)-3-(difluoromethyl)-1H-pyrazol-4-yl)pyrazolo[1,5-a]pyrimidine-3-carboxamide

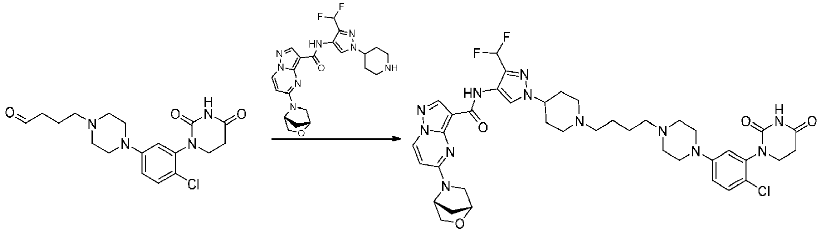

5-((1*R*,4*R*)-2-Oxa-5-azabicyclo[2.2.1]heptan-5-yl)-*N*(3-(difluoromethyl)-1-(piperidin-4-yl)-1*H-*pyrazol-4-yl)pyrazolo[1,5-*a*]pyrimidine-3-carboxamide (20 mg, 0.044 mmol) was added to a solution of 4-(4-(4-chloro-3-(2,4-dioxotetrahydropyrimidin-1(2*H*)-yl)phenyl)piperazin-1-yl)*n*-butyraldehyde (crude) in DCE, and the mixture was stirred at room temperature for 0.5 h. STAB (28 mg, 0.13 mmol) was added, and the mixture was reacted at room temperature for 2 h. The reaction liquid was diluted with water (30 mL), and the mixture was extracted with ethyl acetate (30 mL × 3). The organic phase was washed with saturated brine (50 mL), dried over anhydrous sodium sulfate, filtered, and concentrated under reduced pressure. The crude product was purified by preparative reversed-phase chromatography to give 5-((1R,4R)-2-oxa-5-azabicyclo[2.2.1]heptan-5-yl)-*N*-(1-(1-(4-(4-(4-chloro-3-(2,4-dioxotetrahydropyrimidin-1(2*H*)-yl)phenyl)piperazin-1-yl)butyl)piperidin-4-yl)-3-(diffuoromethyl)-1*H*-pyrazol-4-yl)pyrazolo[1,5-*a*]pyrimidine-3-carboxamide.

LC-MS: (ESI, m/z): [M+H]⁺ = 821.3.

¹H NMR (400 MHz, DMSO-*d*₆) δ 10.40 (s, 1H), 9.50 (d, *J* = 5.8 Hz, 1H), 8.78 (d, *J* = 7.7 Hz, 1H), 8.40 (d, *J* = 3.9 Hz, 1H), 8.26 (d, J = 5.6 Hz, 1H), 7.32 (d, J = 8.9 Hz, 1H), 7.27-6.96 (m, 2H), 6.92 (dd, J = 9.0, 2.9 Hz, 1H), 6.88-6.44 (m, 1H), 5.28-5.07 (m, 1H), 4.77 (d, J = 17.3 Hz, 1H), 4.26-4.15 (m, 1H), 3.85-3.42 (m, 6H), 3.23-3.10 (m, 4H), 2.94 (d, J = 11.9 Hz, 2H), 2.80-2.62 (m, 2H), 2.49-2.46 (m, 4H), 2.40-2.25 (m, 4H), 2.05-1.80 (m, 8H), 1.53-1.41 (m, 4H).

### Example 52: N-(3-(difluoromethyl)-1-(1-((3-(3-(2,4-dioxotetrahydropyrimidin-1(2H)-yl)-4-(trifluoromethoxy)benzoyl)-3-azaspiro[5.5]undecan-9-yl)methyl)piperidin-4-yl)-1H-pyrazol-4-yl)-5-morpholinopyrazolo[1,5-a]pyrimidine-3 -carboxamide

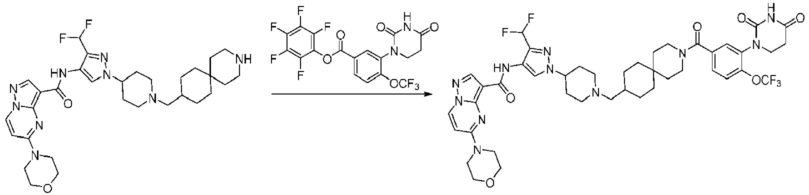

*N*-(1-(1-((3-Azaspiro[5.5]undecan-9-yl)methyl)piperidin-4-yl)-3-(difluoromethyl)-1*H*-pyrazol-4-yl)-5-morpholinopyrazolo[1,5-*a*]pyrimidine-3-carboxamide (100 mg, 0.164 mmol) and pentafluorophenyl 3-(2,4-dioxotetrahydropyrimidin-1(2*H*)-yl)-4-(trifluoromethoxy)benzoate (87 mg, 0.180 mmol) were dissolved in DMSO (5 mL). DIEA (65 mg, 0.492 mmol) was added, and the reaction liquid was stirred at room temperature overnight. The reaction liquid was diluted with water (20 mL), and the mixture was extracted with ethyl acetate (25 mL × 4). The organic phase was washed with saturated brine, dried over anhydrous sodium sulfate, and concentrated under reduced pressure. The crude product was purified by preparative reversed-phase chromatography to give the compound N-(3-(difluoromethyl)-1-(1-((3-(3-(2,4-dioxotetrahydropyrimidin-1(2*H*)-yl)-4-(trifluoromethoxy)benzoyl)-3-azaspiro[5.5]undecan-9-yl)methyl)piperidin-4-yl)-1*H*-pyrazol-4-yl)-5-morpholinopyrazolo[1,5-*a*]pyrimidine-3-carboxamide.

LC-MS: (ESI, m/z): [M+H]⁺ = 912.4.

¹H NMR (400 MHz, DMSO-*d*₆) δ 10.54 (s, 1H), 9.40 (s, 1H), 8.83 (d, *J* = 7.9 Hz, lH), 8.39 (s, 1H), 8.29 (s, 1H), 7.60 (d, *J* = 1.9 Hz, 1H), 7.52 (d, *J* = 7.5 Hz, 1H), 7.47 (dd, *J* = 8.5, 1.8 Hz, 1H), 7.24-6.87 (m, 2H), 4.24-4.17 (m, 1H), 3.83-3.50 (m, 13H), 3.30-3.26 (m, 1H), 2.93-2.85 (m, 2H), 2.75-2.65 (m, 2H), 2.18-2.10 (m, 2H), 2.05-1.88 (m, 6H), 1.70 (d, *J* = 9.0 Hz, 2H), 1.61-1.42 (m, 5H), 1.35-1.20 (m, 2H), 1.12-0.95 (m, 4H).

### Example 53: N-(3-(diffuoromethyl)-1-(1-(2-((1-(3-(2,4-dioxotetrahydropyrimidin-1(2H)-yl)-4-methoxybenzoyl)piperidin-4-yl)oxy)ethyl)piperidin-4-yl)-1H-pyrazol-4-yl)-5-morpholinopyrazolo[1,5-a]pyrimidine-3-carboxamide

### Step 1: preparation of benzyl 4-(2-ethoxy-2-oxoethoxy)piperidine-1-carboxylate

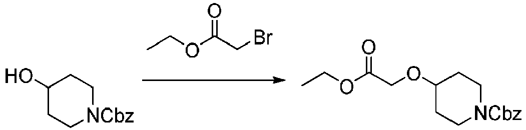

Benzyl 4-hydroxypiperidine-1-carboxylate (3 g, 12.8 mmol) was dissolved in anhydrous tetrahydrofuran (30 mL). Sodium hydride (1.1 g, 25.6 mmol) was added at zero, and the mixture was stirred for 60 min. Ethyl 2-bromoacetate (2.6 g, 15.4 mmol) was added, and the reaction liquid was stirred at room temperature overnight. Water (100 mL) was added to the reaction liquid, and the mixture was extracted with ethyl acetate (150 mL × 3). The organic phase was dried over anhydrous sodium sulfate, filtered, and concentrated under reduced pressure. The crude product was purified by column chromatography (ethyl acetate:petroleum ether = 1:1) to give benzyl 4-(2-ethoxy-2-oxoethoxy)piperidine-1-carboxylate.

LC-MS: (ESI, m/z): [M+Na]⁺ = 344.1.

### Step 2: preparation of benzyl 4-(2-hydroxyethoxy)piperidine-1-carboxylate

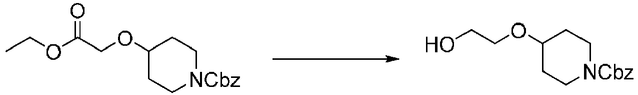

Benzyl 4-(2-ethoxy-2-oxoethoxy)piperidine-1-carboxylate (1.1 g, 3.4 mmol) was dissolved in anhydrous tetrahydrofuran (10 mL). A solution of borane in tetrahydrofuran (2.9 g, 34 mmol) was added at zero, and the reaction liquid was stirred at room temperature overnight. Water (100 mL) was slowly added at zero, and the mixture was extracted with ethyl acetate (100 mL × 3), dried over anhydrous sodium sulfate, filtered, and concentrated under reduced pressure. The crude product was purified by column chromatography (ethyl acetate:petroleum ether = 7:3) to give benzyl 4-(2-hydroxyethoxy)piperidine-1-carboxylate.

LC-MS: (ESI, m/z): [M+Na]⁺ = 302.1.

### Step 3: preparation of benzyl 4-(2-oxoethoxy)piperidine-1-carboxylate

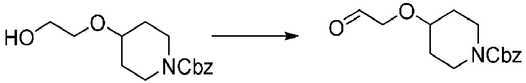

Benzyl 4-(2-hydroxyethoxy)piperidine-1-carboxylate (400 mg, 1.4 mmol) was dissolved in DCM (4 mL). PCC (602 mg, 2.8 mmol) was added, and the reaction liquid was stirred at room temperature for 3 h. The mixture was filtered and directly concentrated under reduced pressure to give benzyl 4-(2-oxoethoxy)piperidine-1-carboxylate, which was directly used in the next step without purification.

LC-MS: (ESI, m/z): [M+H]⁺ = 278.1.

### Step 4: preparation of benzyl 4-(2-(4-(3-(difluoromethyl)-4-(5-morpholinopyrazolo[1,5-a]pyrimidine-3-carboxamido)-1H-pyrazol-1-yl)piperidin-1-yl)ethoxy)piperidine-1-carboxylate

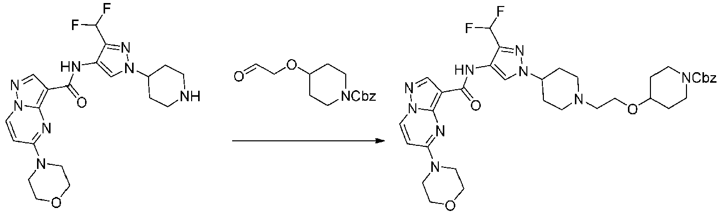

*N*-(3-(Difluoromethyl)-1-(piperidin-4-yl)-1*H-*pyrazol-4-yl)-5-morpholinopyrazolo[1,5-*a*]pyrimidine-3-carboxamide (120 mg, 0.27 mmol) and benzyl 4-(2-oxoethoxy)piperidine-1-carboxylate (75 mg, 0.27 mmol) were dissolved in tetrahydrofuran (5 mL). Sodium triacetoxyborohydride (172 mg, 0.81 mmol) was added while stirring, and the mixture was stirred at room temperature for 3 h. Water (50 mL) was added to the reaction liquid, and the mixture was extracted with ethyl acetate (60 mL × 3). The organic phase was dried over anhydrous sodium sulfate, filtered, and concentrated under reduced pressure. The crude product was purified by column chromatography (methanol:dichloroethane = 1:7) to give benzyl 4-(2-(4-(3-(difluoromethyl)-4-(5-morpholinopyrazolo[1,5-*a*]pyrimidine-3-carboxamido)-1*H*-pyrazol-1-yl)piperidin-1-yl)ethoxy)piperidine-1 -carboxylate.

LC-MS: (ESI, m/z): [M+H]⁺ = 708.3.

### Step 5: preparation of N-(3-(difluoromethyl)-1-(1-(2-(piperidin-4-yloxy)ethyl)piperidin-4-yl)-1H-pyrazol-4-yl)-5-morpholinopyrazolo[1,5-a]pyrimidine-3-carboxamide

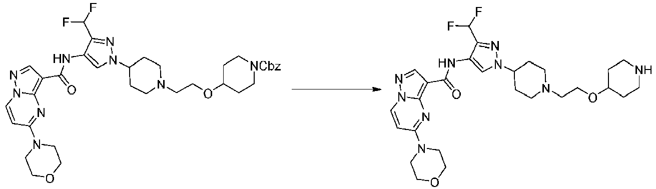

Benzyl 4-(2-(4-(3-(difluoromethyl)-4-(5-morpholinopyrazolo[1,5-*a*]pyrimidine-3-carboxamido)-1*H*-pyrazol-1-yl)piperidin-1-yl)ethoxy)piperidine-1-carboxylate (50 mg, 0.07 mmol) was dissolved in tetrahydrofuran (3 mL). Then palladium on carbon (10%, 10 mg) was added, and the mixture was stirred at room temperature for 1 h. The reaction liquid was filtered through celite, and the filtrate was directly concentrated under reduced pressure to give *N*-(3-(difluoromethyl)-1-(1-(2-(piperidin-4-yloxy)ethyl)piperidin-4-yl)-1*H*-pyrazol-4-yl)-5-morpholinopyrazolo[1,5-*a*]pyrimidine-3-carboxamide,which was directly used in the next step without purification.

LC-MS: (ESI, m/z): [M+H]⁺ = 574.4.

### Step 6: preparation of N-(3-(difluoromethyl)-1-(1-(2-((1-(3-(2,4-dioxotetrahydropyrimidin-1(2H)-yl)-4-methoxybenzoyl)piperidin-4-yl)oxy)ethyl)piperidin-4-yl)-1H-pyrazol-4-yl)-5-morphofinopyrazolo[1,5-a]pyrimidine-3-carboxamide

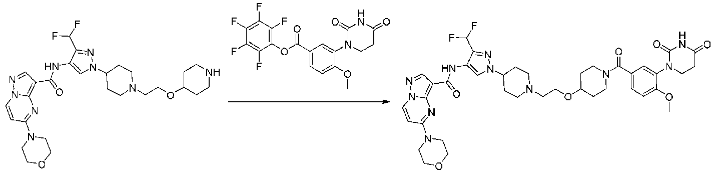

*N*-(3-(Difluoromethyl)-1-(1-(2-(piperidin-4-yloxy)ethyl)piperidin-4-yl)-1*H*-pyrazol-4-yl)-5-morpholinopyrazolo[1,5-*a*]pyrimidine-3-carboxamide (26 mg, 0.045 mmol) and pentafluorophenyl 3-(2,4-dioxotetrahydropyrimidin-1(2*H*)-yl)-4-methoxybenzoate (20 mg, 0.045 mmol) were dissolved in DMSO (2 mL). *N*,*N*-diisopropylethylamine (18 mg, 0.14 mmol) was added while stirring, and the mixture was stirred at room temperature for 3 h. Water (20 mL) was added to the reaction liquid, and the mixture was extracted with ethyl acetate (20 mL × 3). The organic phase was washed with saturated brine (30 mL), dried over anhydrous sodium sulfate, filtered, and concentrated under reduced pressure. The crude product was purified by pre-HPLC (CH₃CN/0.08% aqueous NH₄HCO₃ solution, 5%-95%) to give N-(3-(difluoromethyl)-1-(1-(2-((1-(3-(2,4-dioxotetrahydropyrimidin-1(2*H*)-yl)-4-methoxybenzoyl)piperidin-4-yl)oxy)ethyl)piperidin-4-yl)-1*H-*pyrazol-4-yl)-5-morpholinopyrazolo[1,5-*a*]pyrimidine-3-carboxamide.

LC-MS: (ESI, m/z): [M+H]⁺ = 820.6.

¹H NMR (400 MHz, CD₃OD) δ 8.54 (d, J = 8.0 Hz, 1H), 8.37 (s, 1H), 8.32 (s, 1H), 7.47-7.38 (m, 2H), 7.19 (d, J = 8.6 Hz, 1H), 7.00-6.72 (m, 2H), 3.92 (s, 3H), 3.89-3.77 (m, 8H), 3.70-3.67 (m, 3H), 3.15-3.10 (m, 1H), 2.80 (t, J = 6.8 Hz, 2H), 2.68 (t, J = 5.5 Hz, 2H), 2.40-2.28 (m, 2H), 2.19 (t, J = 7.6 Hz, 1H), 2.14-2.08 (m, 2H), 2.04-1.90 (m, 3H), 1.64-1.55 (m, 2H), 1.34-1.26 (m, 8H).

### Example 54: N-(3-(difluoromethyl)-1-((1R,4R)-4-((2-(1-(3-(2,6-dioxopyridin-3-yl)-4-fluorobenzoyl)piperidin-4-yl)ethyl)(methyl)carbamoyl)cyclohexyl)-1H-pyrazol-4-yl)-5-morpholinopyrazolo[1,5-a]pyrimidine-3-carboxamide

### Step 1: preparation of methyl (1R,4R)-4-(3-(difluoromethyl)-4-(5-morpholinopyrazolo[1,5-a]pyrimidine-3-carboxamido)-1H-pyrazol-1-yl)cyclohexane-1-carboxylate

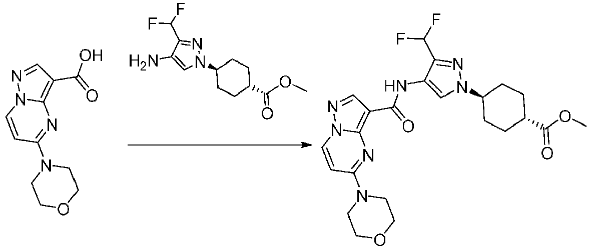

5-Morpholinopyrazolo[1,5-*a*]pyrimidine-3-carboxylic acid (200 mg, 0.73 mmol) was dissolved in DMF (4 mL). Methyl (1*R*,4*R*)-4-(4-amino-3-(difluoromethyl)-1*H*-pyrazol-1-yl)cyclohexane-1-carboxylate (182 mg, 0.73 mmol), HATU (139 mg, 0.95 mmol), and DIEA (377 mg, 2.92 mmol) were added, and the reaction liquid was stirred at room temperature overnight. The reaction liquid was diluted with water (50 mL) and extracted with ethyl acetate (40 mL × 3). The organic phase was washed with saturated brine, dried over anhydrous sodium sulfate, filtered, and concentrated under reduced pressure. The crude product was purified by column chromatography (EA:PE = 9:1) to give methyl (1R,4R)-4-(3-(difluoromethyl)-4-(5-morpholinopyrazolo[1,5-*a*]pyrimidine-3-carboxamido)-1*H*-pyrazol-1-yl)cyclohexane-1-carboxylate.

LC-MS: (ESI, m/z): [M+H]⁺ = 504.1.

### Step 2: preparation of (1R,4R)-4-(3-(difluoromethyl)-4-(5-morpholinopyrazolo[1,5-a]pyrimidine-3-carboxamido)-1H-pyrazol-1-yl)cyclohexane-1 -carboxylic acid

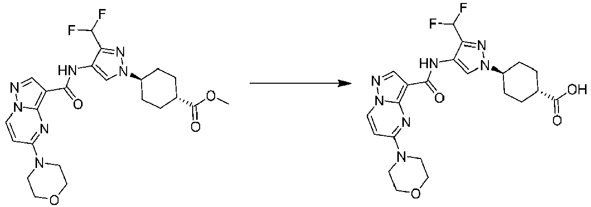

Methyl (1*R*,4*R*)-4-(3-(difluoromethyl)-4-(5-morpholinopyrazolo[1,5-*a*]pyrimidine-3-carboxamido)-1*H*-pyrazol-1-yl)cyclohexane-1-carboxylate (230 mg, 0.46 mmol) was dissolved in a mixed solution of methanol/water (5 mL, V/V = 5/1). Lithium hydroxide (44 mg, 1.84 mmol) was added, and the reaction liquid was heated to 60 °C and stirred for 2 h. The reaction liquid was adjusted to pH = 4 with dilute hydrochloric acid (2 mol/L), and the mixture was extracted with ethyl acetate (40 mL × 3). The organic phase was washed with saturated brine, dried over anhydrous sodium sulfate, filtered, and concentrated under reduced pressure to give (1*R*,4*R*)-4-(3-(difluoromethyl)-4-(5-morpholinopyrazolo[1,5-*a*]pyrimidine-3-carboxamido)-1*H*-pyrazol-1-yl)cyclohexane-1-carboxylic acid, which was directly used in the next step.

LC-MS: (ESI, m/z): [M+H]⁺ = 490.6.

### Step 3: preparation of tert-butyl 4-(2-((1R,4R)-4-(3-(difluoromethyl)-4-(5-morpholinopyrazolo[1,5-a]pyrimidine-3-carboxamido)-1H-pyrazol-1-yl)-N-methylcyclohexane-1-carboxamido)ethyl)piperidine-1-carboxylate

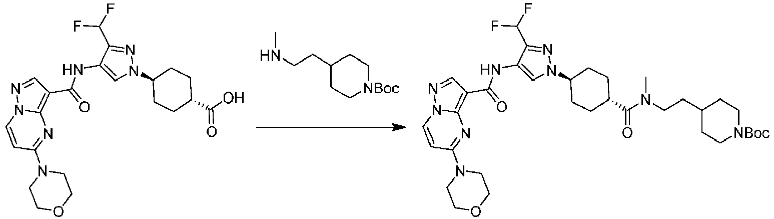

(1*R*,4*R*)-4-(3-(Difluoromethyl)-4-(5-morpholinopyrazolo[1,5-*a*]pyrimidine-3-carboxamido)-1*H-*pyrazol-1-yl)cyclohexane-1-carboxylic acid (140 mg, 0.29 mmol) and *tert*-butyl 4-(2-(methylamino)ethyl)piperidine-1-carboxylate (70 mg, 0.29 mmol) were dissolved in DMF (4 mL). HATU (144 mg, 0.38 mmol) and DIEA (155 mg, 1.2 mmol) were added, and the reaction liquid was stirred at room temperature overnight. The reaction liquid was diluted with water (40 mL) and extracted with ethyl acetate (40 mL × 4). The organic phase was washed with saturated brine, dried over anhydrous sodium sulfate, filtered, and concentrated under reduced pressure. The crude product was purified by column chromatography (EA) to give *tert*-butyl 4-(2-((1*R*,4*R*)-4-(3-(difluoromethyl)-4-(5-morpholinopyrazolo[1,5-*a*]pyrimidine-3-carboxamido)-1*H*-pyrazol-1-yl)-*N-*methylcyclohexane-1 -carboxamido)ethyl)piperidine-1 -carboxylate.

LC-MS: (ESI, m/z): [M+H]⁺ = 714.8.

### Step 4: preparation of N-(3-(difluoromethyl)-1-((1R,4R)-4-(methyl(2-(piperidin-4-yl)ethyl)carbamoyl)cyclohexyl)-1H-pyrazol-4-yl)-5-morpholinopyrazolo[1,5-a]pyrimidine-3-carboxamide

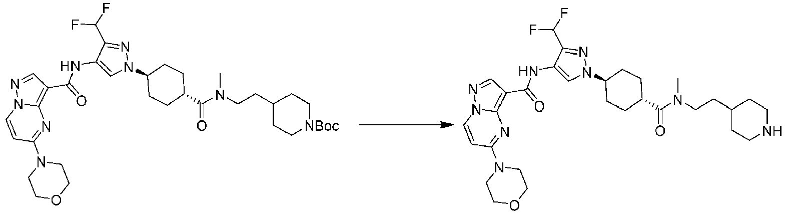

*tert*-Butyl 4-(2-((1*R*,4*R*)-4-(3-(difluoromethyl)-4-(5-morpholinopyrazolo[1,5-a]pyrimidine-3-carboxamido-1*H*-pyrazol-1-yl)-*N*-methylcyclohexane-1-carboxamido)ethyl)piperidine-1-carboxylate (130 mg, 0.18 mmol) was dissolved in dichloromethane (3 mL). Trifluoroacetic acid (0.3 mL) was added, and the mixture was stirred at room temperature for 1 h. The reaction liquid was directly concentrated under reduced pressure to give *N*-(3-(difluoromethyl)-1-((1*R*,4*R*)-4-(methyl(2-(piperidin-4-yl)ethyl)carbamoyl)cyclohexyl)-1*H*-pyrazol-4-yl)-5-morpholinopyrazolo[1,5-*a*]pyrimidine-3-carboxamide, which was directly used in the next step without purification.

LC-MS: (ESI, m/z): [M+H]⁺ = 614.2.

### Step 5: preparation of N-(3-(difluoromethyl)-1-((1R,4R)-4-((2-(1-(3-(2,6-dioxopyridin-3-yl)-4-fluorobenzoyl)piperidin-4-yl)ethyl)(methyl)carbamoyl)cyclohexyl)-1H-pyrazol-4-yl)-5-morpholinopyrazolo[1,5-a]pyrimidine-3-carboxamide

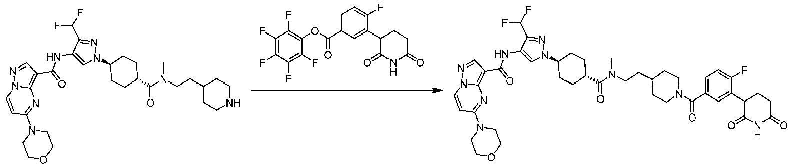

*N*-(3-(Difluoromethyl)-1-((1*R*,4*R*)-4-(methyl(2-(piperidin-4-yl)ethyl)carbamoyl)cyclohexyl)-1*H-*pyrazol-4-yl)-5-morpholinopyrazolo[1,5-*a*]pyrimidine-3-carboxamide (130 mg, 0.18 mmol) and pentafluorophenyl 3-(2,6-dioxopiperidin-3-yl)-4-fluorobenzoate (75.3 mg, 0.18 mmol) were dissolved in DMSO (2 mL). DIEA (93 mg, 0.72 mmol) was added, and the reaction liquid was stirred at room temperature for 3 h. The reaction liquid was diluted with water (20 mL), and the mixture was extracted with ethyl acetate (20 mL × 3). The organic phase was washed with saturated brine (30 mL), dried over anhydrous sodium sulfate, filtered, and concentrated under reduced pressure. The crude product was purified by pre-HPLC (CH₃CN/0.08% aqueous NH₄HCO₃ solution, 5%-95%) to give *N*-(3-(difluoromethyl)-1-((1*R*,4*R*)-4-((2-(1-(3-(2,6-dioxopyridin-3-yl)-4-fluorobenaoyl)piperidin-4-yl)ethyl)(methyl)carbamoyl)cyclohexyl)-1*H*-pyrazol-4-yl)-5-morpholinopyrazolo[1,5-*a*]pyrimidine-3-carboxamide.

LC-MS: (ESI, m/z): [M+H]⁺ = 847.4.

¹H NMR (400 MHz, DMSO-*d*₆) δ 10.90 (s, 1H), 9.40 (s, 1H), 8.83 (d, *J* = 7.9 Hz, 1H), 8.40 (d, *J* = 5.5 Hz, 1H), 8.29 (s, 1H), 7.40-7.33 (m, 2H), 7.31-7.23 (m, 1H), 7.23-6.96 (m, 1H), 6.91 (d, *J* = 8.0 Hz, 1H), 4.52-4.20 (m, 2H), 4.12 (dd, *J* = 12.7, 4.9 Hz, 1H), 3.88-3.66 (m, 8H), 3.62-3.48 (m, 1H), 3.433.34 (m, 1H), 3.01 (s, 3H), 2.82-2.62 (m, 4H), 2.60-2.54 (m, 1H), 2.29-2.17 (m, 1H), 2.08-1.98 (m, 3H), 1.95-1.67 (m, 6H), 1.66-1.35 (m, 6H), 1.21-1.02 (m, 2H).

### Example 55: preparation of N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)-5-(1,1-dioxothiomorpholinyl)pyrazolo[1,5-a]pyrimidine-3-carboxamide

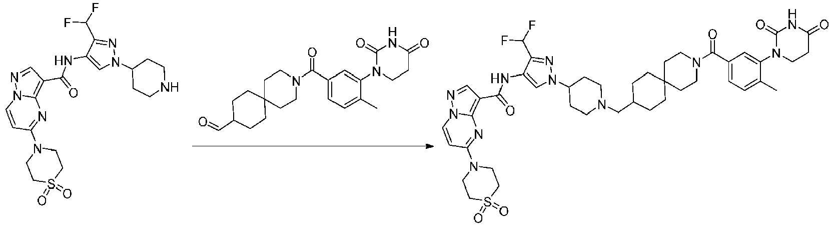

*N*-(3-(Difluoromethyl)-1-(piperidin-4-yl)-1*H*-pyrazol-4-yl)-5-(1,1-dioxothiomorpholinyl)pyrazolo[1,5-*a*]pyrimidine-3-carboxamide (60 mg, 0.1 mmol) and 3-(3-(2,4-dioxotetrahydropyrimidin-1(2*H*)-yl)-4-methylbenzoyl)-3-azaspiro[5.5]undecane-9-carbaldehyde (41 mg, 0.1 mmol) were dissolved in tetrahydrofuran (2 mL). Sodium triacetoxyborohydride (84 mg, 0.4 mmol) was added while stirring, and the mixture was stirred at room temperature for 3 h. The reaction liquid was diluted with water (50 mL), and the mixture was extracted with ethyl acetate (50 mL × 3). The organic phase was washed with saturated brine (100 mL), dried over anhydrous sodium sulfate, filtered, and concentrated under reduced pressure. The crude product was purified by pre-HPLC (CH₃CN/0.08% aqueous NH₄HCO₃ solution, 5%-95%) to give *N*-(3-(difluoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H*-pyrazol-4-yl)-5-(1,1-dioxothiomorpholinyl)pyrazolo[1,5-*a*]pyrimidine-3-carboxamide.

LC-MS: (ESI, m/z): [M+H]⁺ = 890.1

¹H NMR (400 MHz, CD₃OD-*d*₄) δ 8.64 (d, J = 7.9 Hz, 1H), 8.37 (d, *J* = 5.8 Hz, 2H), 7.40 (d, *J* = 7.8 Hz, 1H), 7.36-7.29 (m, 2H), 7.06-6.77 (m, 2H), 4.60-4.52 (m, 2H), 4.42-4.34 (m, 4H), 4.25-4.14 (m, 1H), 3.92-3.82 (m, 1H), 3.77-3.62 (m, 3H), 3.49-3.40 (m, 2H), 3.30-3.22 (m, 4H), 3.06-3.00 (m, 2H), 2.92-2.82 (m, 2H), 2.31 (s, 3H), 2.28-2.22 (m, 2H), 2.20-2.06 (m, 6H), 1.82-1.72 (m, 2H), 1.70-1.63 (m, 2H), 1.55-1.39 (m, 2H), 1.33-1.25 (m, 2H), 1.24-1.12 (m, 3H).

### Example 56: (S)-N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)-5-(3-hydroxypiperidin-1-yl)pyrazolo[1,5-a]pyrimidine-3-carboxamide

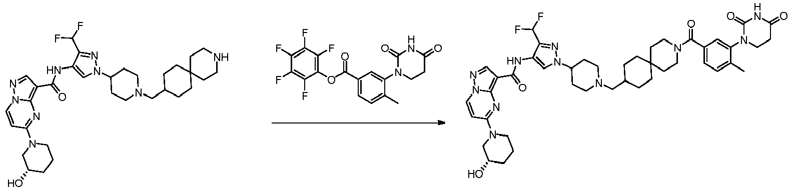

(*S*)-*N*-(1-(1-((3-Azaspiro[5.5]undecan-9-yl)methyl)piperidin-4-yl)-3-(difluoromethyl)-1*H*-pyrazol-4-yl)-5-(3-hydroxypiperidin-1-yl)pyrazolo[1,5-*a*]pyrimidine-3-carboxamide (80 mg, crude, 0.11 mmol) was dissolved in dimethyl sulfoxide (3 mL). Pentafluorophenyl 3-(2,4-dioxotetrahydropyrimidin-1(2*H*)-yl)-4-methylbenzoate (46 mg, 0.11 mmol) and *N*,*N*-diisopropylethylamine (142 mg, 1.1 mmol) were added while stirring, and the mixture was stirred at room temperature overnight. Water (50 mL) was added to the reaction liquid. The mixture was filtered, and the filter cake was purified by Pre-HPLC (CH₃CN/0.08% aqueous NH₄HCO₃ solution, 5%-95%) to give (*S*)-*N*-(3-(difluoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H*-pyrazol-4-yl)-5-(3-hydroxypiperidin-1-yl)pyrazolo [1,5-*a*]pyrimidine-3-carboxamide.

LC-MS: (ESI, m/z): [M+H]⁺ = 856.3.

¹H NMR (400 MHz, DMSO-*d*₆) δ 10.38 (s, 1H), 9.38 (s, 1H), 8.72 (d, *J* = 8.0 Hz, 1H), 8.38 (s, 1H), 8.24 (s, 1H), 7.37-7.29 (m, 2H), 7.26-6.96 (m, 2H), 6.86 (d, *J* = 8.0 Hz, 1H), 4.89 (d, *J* = 2.0 Hz, 1H), 4.26-4.14 (m, 1H), 4.00-3.75 (m, 3H), 3.70-3.39 (m, 6H), 3.30-3.24 (m, 2H), 2.93-2.64 (m, 4H), 2.21 (s, 3H), 2.18-2.10 (m, 2H), 2.06-1.65 (m, 10H), 1.61-1.22 (m, 9H), 1.16-0.95 (m, 4H).

### Example 57: (S)-N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)-5-(3-hydroxypiperidin-1-yl)pyrazolo [1,5-a]pyrimidine-3-carboxamide

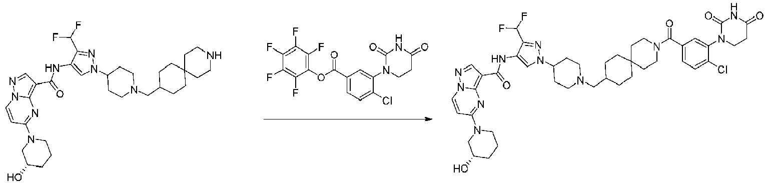

(*S*)-*N-*(1-(1-((3-Azaspiro[5.5]undecan-9-yl)methyl)piperidin-4-yl)-3-(difluoromethyl)-1*H*-pyrazol-4-yl)-5-(3-hydroxypiperidin-1-yl)pyrazolo[1,5-*a*]pyrimidine-3-carboxamide (80 mg, crude, 0.11 mmol) was dissolved in dimethyl sulfoxide (3 mL). Pentafluorophenyl 4-chloro-3-(2,4-dioxotetrahydropyrimidin-1(2*H*)-yl)benzoate (48 mg, 0.11 mmol) and *N*,*N*-diisopropylethylamine (142 mg, 1.1 mmol) were added while stirring, and the mixture was stirred at room temperature overnight. Water (30 mL) was added to the reaction liquid. The mixture was filtered, and the filter cake was purified by Pre-HPLC (CH₃CN/0.08% aqueous NH₄HCO₃ solution, 5%-95%) to give (*S*)-*N*-(1-(1-((3-(4-chloro-3-(2,4-dioxotetrahydropyrimidin-1(2*H*)-yl)benzoyl)-3-azaspiro[5.5] undecan-9-yl)methyl)piperidin-4-yl)-3-(difluoromethyl)-1*H*-pyrazol-4-yl)-5-(3-hydroxypiperidin-1-yl)pyrazolo[1,5-*a*]pyrimidine-3-carboxamide.

LC-MS: (ESI, m/z): [M+H]⁺ = 876.3.

¹H NMR (400 MHz, DMSO-*d*₆) δ 10.51 (s, 1H), 9.38 (s, 1H), 8.72 (d, *J* = 7.9 Hz, lH), 8.38 (s, 1H), 8.24 (s, 1H), 7.63 (d, *J* = 8.2 Hz, 1H), 7.55 (s, 1H), 7.39 (d, *J* = 8.5 Hz, 1H), 7.26-6.97 (m, 1H), 6.86 (d, *J* = 8.0 Hz, 1H), 4.88 (d, *J* = 2.0 Hz, 1H), 4.25-4.15 (m, 1H), 3.97-3.40 (m, 9H), 3.30-3.18 (m, 2H), 2.93-2.85 (m, 2H), 2.78-2.72 (m, 2H), 2.20-2.10 (m, 2H), 2.05-1.65 (m, 10H), 1.60-1.31 (m, 9H), 1.20-0.94 (m, 4H).

### Example 58: N-(3-(difluoromethyl)-1-((1R,4R)-4-(2-(9-(3-(2,4-dioxotetrahydropyrimidin-1(2H)-yl)-4-methoxybenzoyl)-3,9-diazaspiro[5.5]undecan-3-yl)ethyl)cyclohexyl)-1H-pyrazol-4-yl)-5-morpholinopyrazolo[1,5-a]pyrimidine-3-carboxamide

### Step 1: preparation of N-(3-(difluoromethyl)-1-((1R,4R)-4-(2-hydroxyethyl)cyclohexyl)-1H-pyrazol-4-yl)-5-morpholinopyrazolo[1,5-a]pyrimidine-3-carboxamide

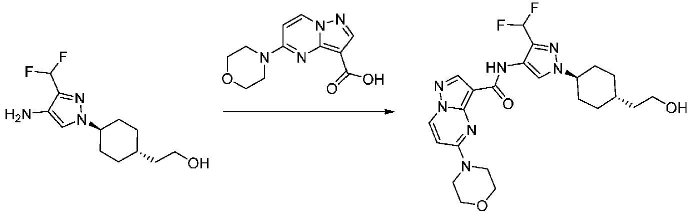

2-((1*R*,4*R*)-4-(4-Amino-3-(difluoromethyl)-1*H*-pyrazol-1-yl)cyclohexyl)ethyl-1-ol (70 mg, 0.27 mmol) and 5-morpholinopyrazolo[1,5-*a*]pyrimidine-3-carboxylic acid (74 mg, 0.30 mmol) were dissolved in DMF (10 mL). *N*,*N*-Diisopropylethylamine (104 mg, 0.81 mmol) and Py-BOP (210 mg, 0.40 mmol) were added while stirring, and the reaction liquid was heated to 60 °C and stirred overnight. Water (100 mL) was added to the reaction liquid, and the mixture was extracted with ethyl acetate (30 mL × 3). The organic phase was washed with saturated brine (100 mL), dried over anhydrous sodium sulfate, filtered, and concentrated under reduced pressure. The crude product was purified by p-TLC (methanol:dichloromethane = 1:9) to give *N*-(3-(difluoromethyl)-1-((1*R*,4*R*)-4-(2-hydroxyethyl)cyclohexyl)-1*H*-pyrazol-4-yl)-5-morpholinopyrazolo[1,5-*a*]pyrimidine-3-carboxamide.

LC-MS: (ESI, m/z): [M+H]⁺ = 490.2.

### Step 2: preparation of N-(3-(difluoromethyl)-1-((1R,4R)-4-(2-oxoethyl)cyclohexyl)-1H-pyrazol-4-yl)-5-morpholinopyrazolo[1,5-a]pyrimidine-3-carboxamide

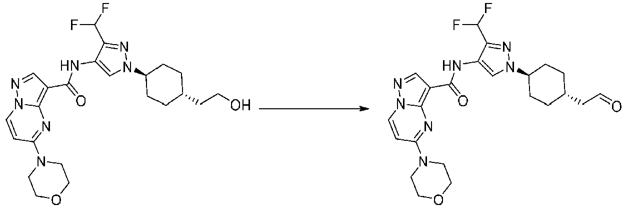

*N*-(3-(Difluoromethyl)-1-((1*R*,4*R*)-4-(2-hydroxyethyl)cyclohexyl)-1*H*-pyrazol-4-yl)-5-morpholinopyrazolo[1,5-*a*]pyrimidine-3-carboxamide (70 mg, 0.14 mmol) was dissolved in dichloromethane (5 mL). The Dess-Martin reagent (118 mg, 0.28 mmol) was added, and the mixture was stirred at room temperature for 1 h. Water (20 mL) was added to the reaction liquid, and the mixture was extracted with dichloromethane (20 mL × 3). The organic phase was dried over anhydrous sodium sulfate, filtered, and concentrated under reduced pressure. The crude product was purified by p-TLC (methanol:dichloromethane = 1:9) to give *N*-(3-(difluoromethyl)-1-((1*R*,4*R*)-4-(2-oxoethyl)cyclohexyl)-1*H-*pyrazol-4-yl)-5-morpholinopyrazolo[1,5-*a*]pyrimidine-3-carboxamide.

LC-MS: (ESI, m/z): [M+H]⁺ = 488.1.

### Step 3: preparation of tert-butyl 9-(2-((1R,4R)-4-(3-(difluoromethyl)-4-(5-morpholinopyrazolo[1,5-a]pyrimidine-3-carboxamido)-1H-pyrazol-1-yl)cycloheacyl)ethyl)-3,9-diazaspiro[5.5]undecane-3-carboxylate

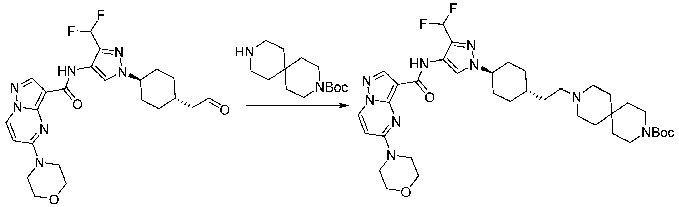

*N*-(3-(Difluoromethyl)-1-((1*R*,4*R*)-4-(2-oxoethyl)cyclohexyl)-1*H*-pyrazol-4-yl)-5-morpholinopyrazolo[1,5-*a*]pyrimidine-3-carboxamide (50 mg, 0.10 mmol) and *tert*-butyl 3,9-diazaspiro[5.5]undecane-3-carboxylate (30 mg, 0.12 mmol) were dissolved in tetrahydrofuran (10 mL). Sodium triacetoxyborohydride (64 mg, 0.30 mmol) was added while stirring, and the mixture was stirred at room temperature for 2 h. Water (20 mL) was added to the reaction liquid, and the mixture was extracted with dichloromethane (20 mL × 3). The organic phase was dried over anhydrous sodium sulfate, filtered, and concentrated under reduced pressure. The crude product was purified by p-TLC (methanol:dichloromethane = 1:9) to give tert-butyl 9-(2-((1*R*,4*R*)-4-(3-(difluoromethyl)-4-(5-morpholinopyrazolo[1,5-*a*]pyrimidine-3-carboxamido)-1*H*-pyrazol-1-yl)cyclohexyl)ethyl)-3,9-diazaspiro[5.5]undecane-3-carboxylate.

LC-MS: (ESI, m/z): [M+H]⁺ = 726.6.

### Step 4: preparation of N-(1-((1R,4R)-4-(2-(3,9-diazaspiro[5.5]undecan-3-yl)ethyl)cyclohexyl)-3-(difluoromethyl)-1H-pyrazol-4-yl)-5-morpholinopyrazolo[1,5-a]pyrimidine-3-carboxamide

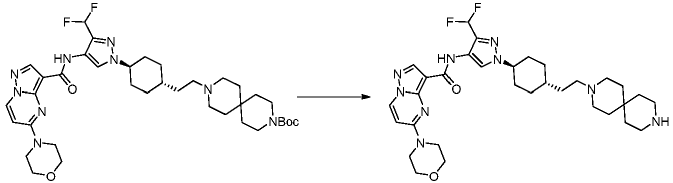

*tert*-Butyl 9-(2-((1*R*,4*R*)-4-(3-(difluoromethyl)-4-(5-morphofinopyrazolo[1,5-*a*]pyrimidine-3-carboxamido)-1*H*-pyrazol-1-yl)cyclohexyl)ethyl)-3,9-diazaspiro[5.5]undecane-3-carboxylate (60 mg, 0.08 mmol) was dissolved in dichloromethane (3 mL), and trifluoroacetic acid (1 mL) was added. The mixture was stirred at room temperature for 1 h. The reaction liquid was directly concentrated under reduced pressure to give *N*-(1-((1*R*,4*R*)-4-(2-(3,9-diazaspiro[5.5]undecan-3-yl)ethyl)cyclohexyl)-3-(difluoromethyl)-1*H*-pyrazol-4-yl)-5-morpholinopyrazolo[1,5-*a*]pyrimidine-3-carboxamide, which was directly used in the next step without purification.

LC-MS: (ESI, m/z): [M+H]⁺ = 626.4.

### Step 5: preparation of N-(3-(difluoromethyl)-1-((1R,4R)-4-(2-(9-(3-(2,4-dioxotetrahydropyrimidin-1(2H)-yl)-4-methoxybenzoyl)-3,9-diazaspiro[5.5]undecan-3-yl)ethyl)cyclohexyl)-1H-pyrazol-4-yl)-5-morpholinopyrazolo[1,5-a]pyrimidine-3-carboxamide

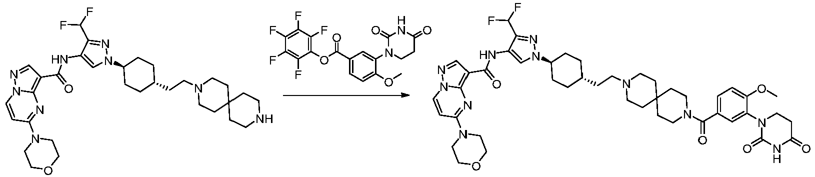

*N*-(1-((1*R*,4*R*)-4-(2-(3,9-Diazaspiro[5.5]undecan-3-yl)ethyl)cyclohexyl)-3-(difluoromethyl)-1*H-*pyrazol-4-yl)-5-morpholinopyrazolo[1,5-*a*]pyrimidine-3-carboxamide (60 mg, crude, 0.08 mmol) was dissolved in dimethyl sulfoxide (3 mL), and pentafluorophenyl 3-(2,4-dioxotetrahydropyrimidin-1(2*H*)-yl)-4-methoxybenzoate (39 mg, 0.09 mmol) and *N*,*N*-diisopropylethylamine (103 mg, 0.8 mmol) were added with stirring. The mixture was stirred at room temperature for 2 h. Water (30 mL) was added to the reaction liquid, and the mixture was filtered. The filter cake was purified by pre-HPLC (CH₃CN/0.08% aqueous NH₄HCO₃, 5%-95%) to give *N*-(3-(difluoromethyl)-1-((1*R*,4*R*)-4-(2-(9-(3-(2,4-dioxotetrahydropyrimidin-1(2*H*)-yl)-4-methoxybenzoyl)-3,9-diazaspiro[5.5]undecan-3-yl)ethyl)cyclohexyl)-1*H*-pyrazol-4-yl)-5-morpholinopyrazolo[1,5-*a*]pyrimidine-3-carboxamide.

LC-MS: (ESI, m/z): [M+H]⁺ = 872.3.

¹H NMR (400 MHz, DMSO-*d*₆) δ 10.32 (s, 1H), 9.40 (s, 1H), 8.82 (d, *J* = 7.9 Hz, 1H), 8.41 (s, 1H), 8.28 (s, 1H), 7.36 (dd, *J* = 8.4, 2.0 Hz, 1H), 7.32 (d, *J* = 2.0 Hz, 1H), 7.25-6.95 (m, 2H), 6.91 (d, *J* = 8.0 Hz, 1H), 4.30-4.20 (m, 1H), 3.84 (s, 3H), 3.82-3.70 (m, 8H), 3.62-3.30 (m, 6H), 2.68 (t, *J* = 6.6 Hz, 2H), 2.36-2.22 (m, 6H), 2.10-1.98 (m, 2H), 1.87-1.73 (m, 2H), 1.70-1.30 (m, 15H).

Example 59: *N*-(3-(difluoromethyl)-1-((1*R*,4*R*)-4-(((3-(3-(2,4-dioxotetrahydropyrimidin-1(2*H*)-yl)-4-methoxybenzoyl)-3-azaspiro[5.5]undecan-9-yl)(methyl)amino)methyl)cyclohexyl)-1*H*-pyrazol-4-yl)-5-morpholinopyrazolo[1,5-*a*]pyrimidine-3-carboxamide

### Step 1: preparation of tert-butyl 9-(benzylamino)-3-azaspiro[5.5]undecane-3-carboxylate

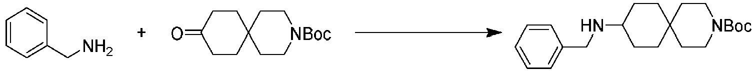

Benzylamine (0.267 g, 2.5 mmol) and tert-butyl 9-oxo-3-azaspiro[5.5]undecane-3-carboxylate (0.66 g, 2.5 mmol) were dissolved in THF (10 mL), and NaBH(OAc)₃ (1.58 g, 7.48 mmol) was added. The reaction liquid was stirred at room temperature for 2 h. The reaction liquid was diluted with water (20 mL) and extracted with ethyl acetate (20 mL × 3). The organic phases were combined, washed with saturated brine, dried over anhydrous Na₂SO₄, filtered, and concentrated under reduced pressure. The crude product was purified by column chromatography (methanol:dichloromethane = 1:24) to give *tert*-butyl 9-(benzylamino)-3-azaspiro[5.5]undecane-3-carboxylate.

LC-MS: (ESI, m/z): [M+H]⁺ = 359.3.

### Step 2: preparation of tert-butyl 9-amino-3-azaspiro[5.5]undecane-3-carboxylate

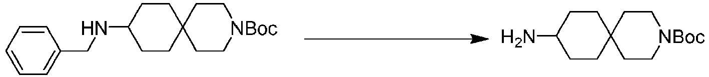

tert-Butyl 9-(benzylamino)-3-azaspiro[5.5]undecane-3-carboxylate (330 mg, 0.92 mmol), Pd/C (100 mg) and Pd(OH)₂ (100 mg) were added to methanol (10 mL), and the mixture was purged with hydrogen 3 times and stirred at room temperature for 2 h. The reaction mixture was filtered, and the filtrate was concentrated under reduced pressure to give *tert*-butyl 9-amino-3-azaspiro[5.5]undecane-3-carboxylate.

LC-MS: (ESI, m/z): [M+H]⁺ = 269.2.

### Step 3: preparation of tert-butyl 9-((((1R,4R)-4-(3-(difluoromethyl)-4-(5-morpholinopyrazolo[1,5-a]pyrimidine-3-carboxamido)-1H-pyrazol-1-yl)cyclohexyl)methyl)amino)-3-azaspiro[5.5]undecane-3-carboxylate

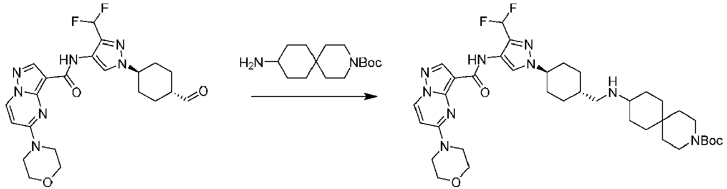

*N*-(3-(Difluoromethyl)-1-((1*R*,4*R*)-4-(formyl)cyclohexyl)-1*H*-pyrazol-4-yl)-5-morpholinopyrazolo[1,5-*a*]pyrimidine-3-carboxamide (100 mg, 0.21 mmol) and *tert*-butyl 9-amino-3-azaspiro[5.5]undecane-3-carboxylate (70 mg, 0.23 mmol) were dissolved in THF (4 mL), and NaBH(OAc)₃ (135 mg, 0.63 mmol) was added. The mixture was stirred at room temperature for 2 h. The reaction liquid was diluted with water (20 mL) and extracted with ethyl acetate (20 mL × 3). The organic phases were combined, washed with saturated brine, dried over anhydrous Na₂SO₄, filtered, and concentrated under reduced pressure. The crude product was purified by column chromatography (methanol:dichloromethane = 1:24) to give *tert-*butyl 9-((((1*R*,4*R*)-4-(3-(difluoromethyl)-4-(5-morpholinopyrazolo[1,5-*a*]pyrimidine-3-carboxamido)-1*H-*pyrazol-1-yl)cyclohexyl)methyl)amino)-3-azaspiro[5.5]undecane-3-carboxylate.

LC-MS: (ESI, m/z): [M+H]⁺ = 726.5.

### Step 4: preparation of tert-butyl 9-((((1R,4R)-4-(3-(difluoromethyl)-4-(5-morpholinopyrazolo[1,5-a]pyrimidine-3-carboxamido)-1H-pyrazol-1-yl)cyclohexyl)methyl)(methyl)amino)-3-azaspiro[5.5]undecane-3-carboxylate

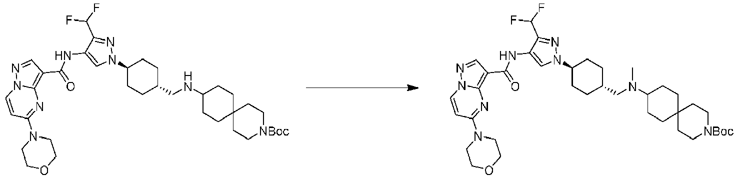

NaBH(OAc)₃ (100 mg, 0.47 mmol) was added to *tert*-butyl 9-((((1*R*,4*R*)-4-(3-(difluoromethyl)-4-(5-morpholinopyrazolo[1,5-*a*]pyrimidine-3-carboxamido)-1*H*-pyrazol-1-yl)cyclohexyl)methyl)amino)-3-azaspiro[5.5]undecane-3-carboxylate (100 mg, 0.13 mmol) and aqueous formaldehyde (37%, 1 mL) in THF (10 mL), and the mixture was heated to 60 °C and stirred for 2 h. The reaction liquid was diluted with water (20 mL) and extracted with ethyl acetate (20 mL × 3). The organic phases were combined, washed with saturated brine, dried over anhydrous Na₂SO₄, filtered, and concentrated under reduced pressure. The crude product was purified by column chromatography (methanol:dichloromethane = 1:19) to give tert-butyl 9-((((1*R*,4*R*)-4-(3-(difluoromethyl)-4-(5-morpholinopyrazolo[1,5-*a*]pyrimidine-3-carboxamido)-1*H*-pyrazol-1-yl)cyclohexyl)methyl)(methyl)amino)-3-azaspiro[5.5]undecane-3-carboxylate.

LC-MS: (ESI, m/z): [M+H]⁺ = 740.4.

### Step 5: preparation of N-(3-(difluoromethyl)-1-((1R,4R)-4-((methyl(3-azaspiro[5.5]undecan-9-yl)amino)methyl)cyclohexyl)-1H-pyrazol-4-yl)-5-morpholinopyrazolo[1,5-a]pyrimidine-3-carboxamide

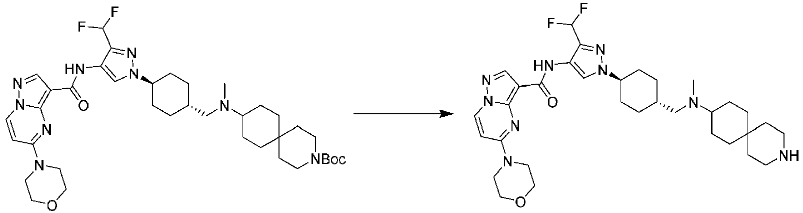

*tert*-Butyl 9-((((1*R*,4*R*)-4-(3-(difluoromethyl)-4-(5-morpholinopyrazolo[1,5-*a*]pyrimidine-3-carboxamido)-1*H*-pyrazol-1-yl)cyclohexyl)methyl)(methyl)amino)-3-azaspiro[5.5]undecane-3-carboxylate (0.10 g, 0.13 mmol) was dissolved in DCM (5 mL), and TFA (1 mL) was added. The mixture was stirred at room temperature for 2 h. The reaction liquid was directly concentrated under reduced pressure to give *N-*(3-(difluoromethyl)-1-((1*R*,4*R*)-4-((methyl(3-azaspiro[5.5]undecan-9-yl)amino)methyl)cyclohexyl)-1*H-*pyrazol-4-yl)-5-morpholinopyrazolo[1,5-*a*]pyrimidine-3-carboxamide, which was directly used in the next step without purification.

LC-MS: (ESI, m/z): [M+H]⁺ = 640.4.

### Step 6: preparation of N-(3-(difluoromethyl)-1-((1R,4R)-4-(((3-(3-(2,4-dioxotetrahydropyrimidin-1(2H)-yl)-4-methoxybenzoyl)-3-azaspiro[5.5]undecan-9-yl)(methyl)amino)methyl)cyclohexyl)-1H-pyrazol-4-yl)-5-morpholinopyrazolo[1,5-a]pyrimidine-3-carboxamide

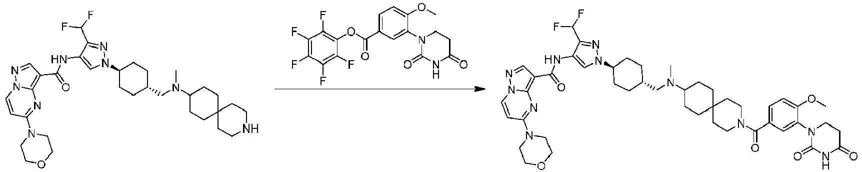

*N*-(3-(Difluoromethyl)-1-((1*R*,4*R*)-4-((methyl(3-azaspiro[5.5]undecan-9-yl)amino)methyl)cyclohexyl)-1*H*-pyrazol-4-yl)-5-morpholinopyrazolo[1,5-*a*]pyrimidine-3-carboxamide (70 mg, 0.1 mmol) and pentafluorophenyl 3-(2,4-dioxotetrahydropyrimidin-1(2*H*)-yl)-4-methoxybenzoate (51 mg, 0.12 mmol) were dissolved in DMSO (1 mL), and DIEA (130 mg, 1 mmol) was added. The mixture was stirred at room temperature for 2 h. The reaction liquid was diluted with water (10 mL) and filtered, and the filter cake was purified by Prep-HPLC to give *N*-(3-(difluoromethyl)-1-((1*R*,4*R*)-4-(((3-(3-(2,4-dioxotetrahydropyrimidin-1(2*H*)-yl)-4-methoxybenzoyl)-3-azaspiro[5.5]undecan-9-yl)(methyl)amino)methyl)cyclohexyl)-1*H*-pyrazol-4-yl)-5-morpholinopyrazolo[1,5-*a*]pyrimidine-3-carboxamide.

LC-MS: (ESI, m/z): [M+H]⁺ = 886.4.

¹H NMR (400 MHz, DMSO-*d*₆) δ 10.33 (s, 1H), 9.39 (s, 1H), 8.82 (d, *J* = 7.9 Hz, 1H), 8.38 (s, 1H), 8.28 (s, 1H), 8.19 (s, 1H), 7.37 (d, *J* = 8.5 Hz, 1H), 7.32 (s, 1H), 7.22-6.95 (m, 2H), 6.91 (d, *J* = 8.0 Hz, 1H), 4.22-4.12 (m, 1H), 3.84 (s, 3H), 3.81-3.75 (m, 4H), 3.74-3.70 (m, 4H), 3.62- 3.55 (m, 5H), 2.68 (t, *J* = 6.4 Hz, 2H), 2.37-2.26 (m, 2H), 2.25-2.23 (m, 2H), 2.21 (s, 3H), 2.06-2.00 (m, 2H), 1.95-1.85 (m, 2H), 1.78-1.70 (m, 4H), 1.56-1.24 (m, 9H), 1.14-0.97 (m, 4H).

### Example 60: N-(3-(difluoromethyl)-1-(1-(3-(9-(3-(2,4-dioxotetrahydropyrimidin-1(2H-yl)-4-methoxybenzoyl)-3,9-diazaspiro[5.5]undecan-3-yl)propyl)piperidin-4-yl)-1H-pyrazol-4-yl)-5-morpholinopyrazolo[1,5-a]pyrimidine-3-carboxamide

### Step 1: preparation of tert-butyl 9-(3-(2,4-dioxotetrahydropyrimidin-1(2H)-yl)-4-methoxybenzoyl)-3,9-diazaspiro[5.5]undecane-3-carboxylate

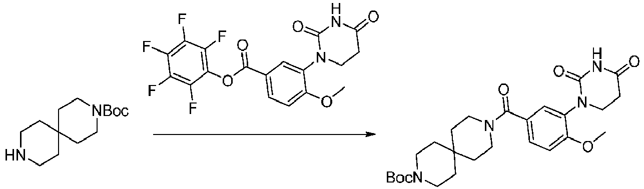

*tert*-Butyl 3,9-diazaspiro[5.5]undecane-3-carboxylate (200 mg, 0.79 mmol) was dissolved in dimethyl sulfoxide (4 mL), and pentafluorophenyl 3-(2,4-dioxotetrahydropyrimidin-1(2H)-yl)-4-methoxybenzoate (340 mg, 0.79 mmol) and *N*,*N*-diisopropylethylamine (413 mg, 3.24 mmol) were added with stirring. The reaction liquid was stirred at room temperature for 3 h. The reaction liquid was diluted with water (40 mL) and extracted with ethyl acetate (50 mL × 3). The organic phase was washed with saturated brine (100 mL), dried over anhydrous sodium sulfate, filtered, and concentrated under reduced pressure. The crude product was purified by column chromatography (ethyl acetate:petroleum ether = 9:1) to give *tert*-butyl 9-(3-(2,4-dioxotetrahydropyrimidin-1(2*H*-yl)-4-methoxybenzoyl)-3,9-diazasprio[5.5]undecane-3-carboxylate.

LC-MS: (ESI, m/z): [M+H]⁺ = 501.2.

### Step 2: preparation of 1-(2-methoxy-5-(3,9-azaspiro[5.5]undecane-3-carbonyl)phenyl)dihydropyrimidine-2,4(1H,3H)-dione

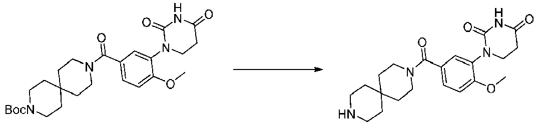

*tert*-Butyl 9-(3-(2,4-dioxotetrahydropyrimidin-1(2*H*)-yl)-4-methoxybenzoyl)-3,9-diazaspiro[5.5]undecane-3-carboxylate (230 mg, 0.46 mmol) was dissolved in dichloromethane (3 mL), and trifluoroacetic acid (0.3 mL) was added. The mixture was stirred at room temperature for 1 h. The reaction liquid was directly concentrated under reduced pressure to give 1-(2-methoxy-5-(3,9-azaspiro[5.5]undecane-3-carbonyl)phenyl)dihydropyrimidine-2,4(1*H*,3*H*)-dione, which was directly used in the next step without purification.

LC-MS: (ESI, m/z): [M+H]⁺ = 401.1.

### Step 3: preparation of 1-(5-(9-(2-(1,3-dioxolan-2-yl)ethyl)-3,9-diazaspiro[5.5]undecane-3-carbonyl)-2-methoxyphenyl)dihydropyrimidine-2,4(1H,3H)-dione

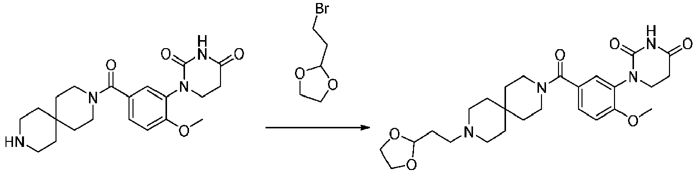

1-(2-Methoxy-5-(3,9-azaspiro[5.5]undecane-3-carbonyl)phenyl)dihydropyrimidine-2,4(1*H*,3*H*)-dione (180 mg, 0.45 mmol) and 2-(2-bromoethyl)-1,3-dioxolane (121 mg, 0.67 mmol) were dissolved in DMF (5 mL), and K₂CO₃ (186 mg,1.3 mmol) was added with stirring. The reaction liquid was heated to 80 °C and stirred for 12 h. The reaction liquid was diluted with water (80 mL) and extracted with ethyl acetate (50 mL × 3). The organic phase was washed with saturated brine (80 mL), dried over anhydrous sodium sulfate, filtered, and concentrated under reduced pressure. The crude product was purified by column chromatography (methanol:dichloromethane = 1:7) to give 1-(5-(9-(2-(1,3-dioxolan-2-yl)ethyl)-3,9-diazaspiro[5.5]undecane-3-carbonyl)-2-methoxyphenyl)dihydropyrimidine-2,4(1*H*,3*H*)-dione.

LC-MS: (ESI, m/z): [M+H]⁺ = 501.7.

### Step 4: preparation of 3-(9-(3-(2,4-dioxotetrahydropyrimidin-1(2H)-yl)-4-methoxybenzoyl)-3,9-diazaspiro[5.5]undecan-3-yl)propanal

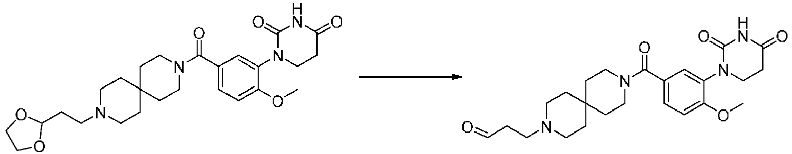

1-(5-(9-(2-(1,3-Dioxolan-2-yl)ethyl)-3,9-diazaspiro[5.5]undecane-3-carbonyl)-2-methoxyphenyl)dihydropyrimidine-2,4(1*H*,3*H*)-dione (200 mg, 0.5 mmol) was dissolved in acetone (5 mL), and hydrochloric acid (2 mol/L, 10 mL) was added. The mixture was stirred at room temperature for 6 h. The reaction liquid was adjusted to pH = 8 with sodium bicarbonate and extracted with ethyl acetate (50 mL × 3). The organic phase was dried over anhydrous sodium sulfate, filtered, and concentrated under reduced pressure to give 3-(9-(3-(2,4-dioxotetrahydropyrimidin-1(2*H*)-yl)-4-methoxybenzoyl)-3,9-diazaspiro[5.5]undecan-3-yl)propanal, which was used in the next step without purification.

LC-MS: (ESI, m/z): [M+H]⁺ = 457.7.

### Step 5: preparation of N-(3-(difluoromethyl)-1-(1-(3-(9-(3-(2,4-dioxotetrahydropyrimidin-1(2H)-yl)-4-methoxybenzoyl)-3,9-diazaspiro[5.5]undecan-3-yl)propyl)piperidin-4-yl)-1H-pyrazol-4-yl)-5-morpholinopyrazolo[1,5-a]pyrimidine-3-carboxamide

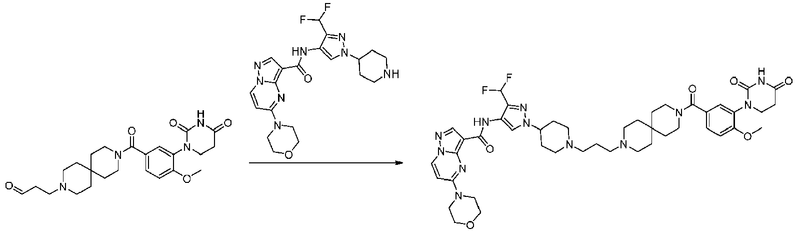

3-(9-(3-(2,4-Dioxotetrahydropyrimidin-1(2*H*)-yl)-4-methoxybenzoyl)-3,9-diazaspiro[5.5]undecan-3-yl)propanal (70 mg, 0.15 mmol) and *N*-(3-(difluoromethyl)-l-(piperidin-4-yl)-1*H*-pyrazol-4-yl)-5-morpholinopyrazolo[1,5-*a*]pyrimidine-3-carboxamide (67 mg, 0.15 mmol) were dissolved in dichloroethane (5 mL), and sodium triacetoxyborohydride (126 mg, 0.6 mmol) was added with stirring. The reaction liquid was stirred at room temperature for 3 h. The reaction liquid was diluted with water (50 mL) and extracted with dichloromethane (60 mL × 3). The organic phase was dried over anhydrous sodium sulfate, filtered, and concentrated under reduced pressure. The crude product was purified by pre-HPLC (CH₃CN/0.08% aqueous NH₄HCO₃, 5%-95%) to give *N*-(3-(difluoromethyl)-1-(1-(3-(9-(3-(2,4-dioxotetrahydropyrimidin-1(2*H*)-yl)-4-methoxybenzoyl)-3,9-diazaspiro[5.5]undecan-3-yl)propyl)piperidin-4-yl)-1*H*-pyrazol-4-yl)-5-morpholinopyrazolo[1,5-*a*]pyrimidine-3-carboxamide.

LC-MS: (ESI, m/z): [M+H]⁺ = 887.2.

¹H NMR (400 MHz, DMSO-*d*₆) δ 10.32 (s, 1H), 9.40 (s, 1H), 8.83 (d, *J* = 7.9 Hz, 1H), 8.39 (s, 1H), 8.29 (s, 1H), 7.37 (d, *J* = 8.4 Hz, 1H), 7.32 (d, *J* = 2.1 Hz, 1H), 7.26-6.97 (m, 2H), 6.91 (d, *J* = 7.9 Hz, 1H), 4.25-4.15 (m, 1H), 3.84 (s, 3H), 3.82-3.70 (m, 8H), 3.62-3.33 (m, 6H), 2.95-2.89 (m, 2H), 2.72-2.62 (m, 2H), 2.36-2.23 (m, 8H), 2.05-1.80 (m, 6H), 1.60-1.24 (m, 10H).

### Example 61: N-(3-(difluoromethyl)-1-(1-(4-(9-(3-(2,4-dioxotetrahydropyrimidin-1(2H-yl)-4-methoxybenzoyl)-3,9-diazaspiro[5.5]undecan-3-yl)butyl)piperidin-4-yl)-177-pyrazol-4-yl)-5-morpholinopyrazolo[1,5-a]pyrimidine-3-carboxamide

### Step 1: preparation of 1-(5-(9-(3-(1,3-dioxolan-2-yl)propyl)-3,9-diazaspiro[5.5]undecane-3-carbonyl)-2-methoxyphenyl)dihydropyrimidine-2,4(1H,3H)-dione

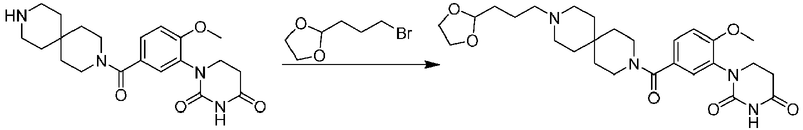

1-(2-Methoxy-5-(3,9-azaspiro[5.5]undecane-3-carbonyl)phenyl)dihydropyrimidine-2,4(1*H*,3*H*)-dione (151 mg, 0.377 mmol) and 2-(3-bromopropyl)-1,3-dioxolane (110.5 mg, 0.566 mmol) were added to acetonitrile (5 mL), and K₂CO₃ (518.3 mg,1.132 mmol) was added. The reaction liquid was heated to 80 °C and stirred for 16 h. The reaction liquid was diluted with water (40 mL), extracted with ethyl acetate (30 mL × 4), washed with saturated brine, dried over anhydrous sodium sulfate, concentrated under reduced pressure, and purified by normal-phase column chromatography (MeOH:DCM = 1:7) to give 1-(5-(9-(3-(1,3-dioxolan-2-yl)propyl)-3,9-diazaspiro[5.5]undecane-3-carbonyl)-2-methoxyphenyl)dihydropyrimidine-2,4(1*H*,3*H*)-dione.

LC-MS: (ESI, m/z): [M+H]⁺ = 515.3.

### Step 2: preparation of 4-(9-(3-(2,4-dioxotetrahydropyrimidin-1(2H)-yl)-4-methoxybenzoyl)-3,9-diazaspiro [5.5]undecan-3-yl)butanal

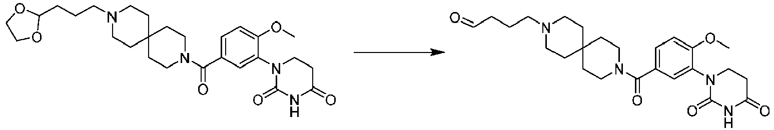

1-(5-(9-(3-(1,3-Dioxolan-2-yl)propyl)-3,9-diazaspiro[5.5]undecane-3-carbonyl)-2-methoxyphenyl)dihydropyrimidine-2,4(1*H*,3*H*)-dione (80 mg, 0.16 mmol) was dissolved in DCM (2 mL), and TFA (0.5 mL) was added. The mixture was stirred overnight at room temperature. DCE (10 mL) and DIEA (1 mL) were added to the reaction liquid, and the mixture was directly concentrated under reduced pressure at room temperature for 10 min to give 4-(9-(3-(2,4-dioxotetrahydropyrimidin-1(2*H*)-yl)-4-methoxybenzoyl)-3,9-diazaspiro[5.5]undecan-3-yl)butanal, which was directly used in the next step.

LC-MS: (ESI, m/z): [M+H]⁺ = 471.5.

### Step 3: preparation of N-(3-(difluoromethyl)-1-(1-(4-(9-(3-(2,4-dioxotetrahydropyrimidin-1(2H)-yl)-4-methoxybenzoyl)-3,9-diazaspiro[5.5]undecan-3-yl)butyl)piperidin-4-yl)-177-pyrazol-4-yl)-5-morpholinopyrazolo[1,5-a]pyrimidine-3-carboxamide

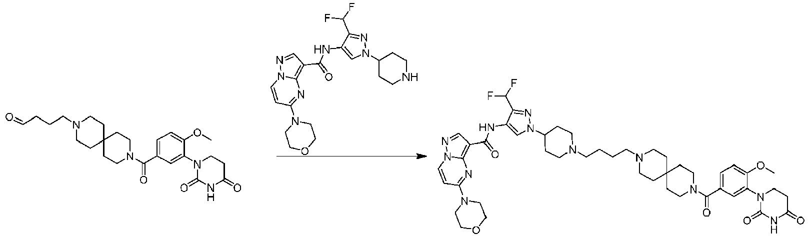

*N*-(3-(Difluoromethyl)-1-(piperidin-4-yl)-1*H*-pyrazol-4-yl)-5-morpholinopyrazolo[1,5-*a*]pyrimidine-3-carboxamide (70 mg, 0.15 mmol) was added to 4-(9-(3-(2,4-dioxotetrahydropyrimidin-1(2*H*)-yl)-4-methoxybenzoyl)-3,9-diazaspiro[5.5]undecan-3-yl)butanal (crude, in DCE). The mixture was stirred at room temperature for 0.5 h, and STAB (97 mg, 0.46 mmol) was then added. The mixture was stirred at room temperature for 2 h. The reaction liquid was diluted with water (30 mL) and extracted with ethyl acetate (30 mL × 3). The organic phase was washed with saturated brine (50 mL), dried over anhydrous sodium sulfate, filtered, and concentrated under reduced pressure. The crude product was purified by preparative reversed-phase chromatography to give *N*-(3-(difluoromethyl)-1-(1-(4-(9-(3-(2,4-dioxotetrahydropyrimidin-1(2*H*)-yl)-4-methoxybenzoyl)-3,9-diazaspiro[5.5]undecan-3-yl)butyl)piperidin-4-yl)-1*H*-pyrazol-4-yl)-5-morpholinopyrazolo[1,5-*a*]pyrimidine-3-carboxamide.

LC-MS: (ESI, m/z): [M+H]⁺ = 901.4.

¹H NMR (400 MHz, DMSO-*d*₆) δ 10.33 (s, 1H), 9.41 (s, 1H), 8.83 (d, *J* = 7.9 Hz, 1H), 8.28 (s, 1H), 8.15 (s, 1H), 7.38 (dd, *J* = 8.5, 1.9 Hz, 1H), 7.33 (d, *J* = 1.9 Hz, 1H), 7.24-6.95 (m, 2H), 6.91 (d, *J* = 8.0 Hz, 1H), 4.33-4.23 (m, 1H), 3.85 (s, 3H), 3.83-3.75 (m, 4H), 3.74-3.68 (m, 4H), 3.64-3.52 (m, 6H), 3.07-2.95 (m, 6H), 2.95-2.86 (m, 2H), 2.68 (t, *J* = 6.6 Hz, 2H), 2.43 (t, *J* = 6.4 Hz, 2H), 2.18 (t, *J* = 11.2 Hz, 2H), 2.05-1.93 (m, 4H), 1.73-1.57 (m, 6H), 1.56-1.42 (m, 6H).

### Example 62: N-(3-(difluoromethyl)-1-(1-(4-(4-(3-(2,6-dioxopiperidin-3-yl)-4-fluorobenzoyl)piperazin-1-yl)butanoyl)piperidin-4-yl)-1H-pyrazol-4-yl)-5-morpholinopyrazolo[1,5-a]pyrimidine-3-carboxamide

### Step 1: preparation of N-(3-(difluoromethyl)-1-(1-(4-hydroxybutanoyl)piperidin-4-yl)-1H-pyrazol-4-yl)-5-morpholinopyrazolo[1,5-a]pyrimidine-3-carboxamide

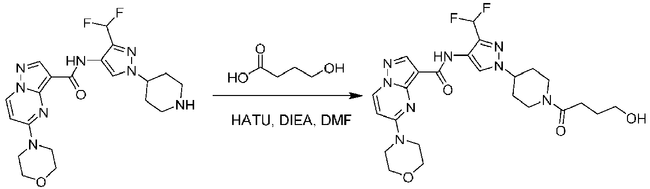

*N-*(3-(Difluoromethyl)-1-(piperidin-4-yl)-1*H*-pyrazol-4-yl)-5-morpholinopyrazolo[1,5-*a*]pyrimidine-3-carboxamide (160 mg, 0.35 mmol), 4-hydroxybutyric acid (36 mg, 0.35 mmol) and HATU (130 mg, 0.42 mmol) were dissolved in DMF (5 mL), and DIEA (135 mg, 1.35 mmol) was then added. The reaction liquid was heated to 90 °C and stirred for 16 h. Water (50 mL) was added to the reaction liquid, and the mixture was extracted with ethyl acetate (30 mL × 3). The organic phases were combined, washed with saturated brine (50 mL), dried over anhydrous sodium sulfate, filtered, and concentrated under reduced pressure. The crude product was purified by normal-phase column chromatography to give N-(3-(difluoromethyl)-1-(1-(4-hydroxybutanoyl)piperidin-4-yl)-1*H*-pyrazol-4-yl)-5-morpholinopyrazolo[1,5-*a*]pyrimidine-3-carboxamide.

LC-MS: (ESI, m/z): [M+H]⁺ = 533.4.

### Step 2: preparation of N-(3-(difluoromethyl)-1-(1-(4-oxobutanoyl)piperidin-4-yl)-1H-pyrazol-4-yl)-5-morpholinopyrazolo[1,5-a]pyrimidine-3-carboxamide

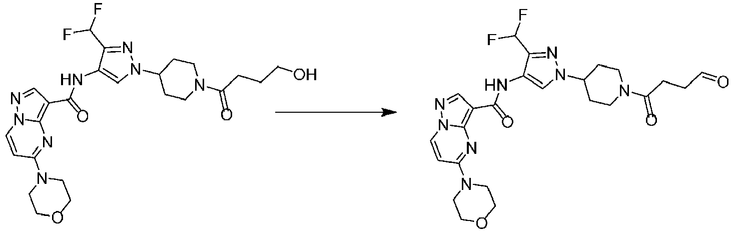

*N-*(3-(Difluoromethyl)-1-(1-(4-hydroxybutanoyl)piperidin-4-yl)-1*H*-pyrazol-4-yl)-5-morpholinopyrazolo[1,5-*a*]pyrimidine-3-carboxamide (26 mg, 0.05 mmol) was dissolved in DCM (2 mL), and DMP (42 mg, 0.10 mmol) was added. The mixture was stirred at room temperature for 2 h. The reaction mixture was filtered. The filter cake was washed with THF (10 mL × 3), and the filtrate was concentrated under reduced pressure to give *N*-(3-(difluoromethyl)-1-(1-(4-oxobutanoyl)piperidin-4-yl)-1*H*-pyrazol-4-yl)-5-morpholinopyrazolo[1,5-*a*]pyrimidine-3-carboxamide.

LC-MS: (ESI, m/z): [M+H]⁺ = 531.5.

### Step 3: preparation of tert-butyl 4-(3-(2,6-oxopiperidin-3-yl)-4-fluorobenzoyl)piperazine-1-carboxylate

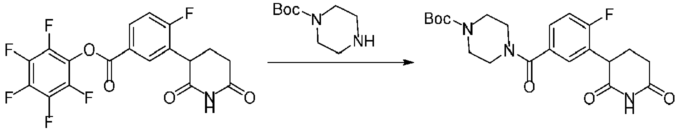

Pentafluorophenyl 3-(2,6-dioxopiperidin-3-yl)-4-fluorobenzoate (56 mg, 0.30 mmol) and tert-butyl piperazine-1-carboxylate (125 mg, 0.30 mmol) were dissolved in DMSO (2 mL), and DIEA (112 mg, 0.90 mmol) was added. The mixture was stirred at room temperature for 2 h. Water (50 mL) was added to the reaction liquid, and the mixture was extracted with ethyl acetate (30 mL × 3). The organic phases were combined, washed with saturated brine (50 mL), dried over anhydrous sodium sulfate, filtered, and concentrated under reduced pressure. The crude product was purified by normal-phase column chromatography to give tert-butyl 4-(3-(2,6-oxopiperidin-3-yl)-4-fluorobenzoyl)piperazine-1-carboxylate.

LC-MS: (ESI, m/z): [M-Boc]⁺ = 320.2.

### Step 4: preparation of 3-(2-fluoro-5-(piperazine-1 -carbonyl)phenyl)piperidine-2,6-dione

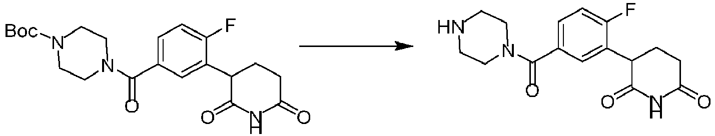

*tert*-Butyl 4-(3-(2,6-oxopiperidin-3-yl)-4-fluorobenzoyl)piperazine-1-carboxylate (80 mg, 0.19 mmol) was dissolved in DCM (0.5 mL), and TFA (0.1 mL) was added. The mixture was stirred at room temperature for 1 h. The reaction liquid was concentrated under reduced pressure to give 3-(2-fluoro-5-(piperazine-1 -carbonyl)phenyl)piperidine-2,6-dione.

LC-MS: (ESI, m/z): [M+H]⁺ = 320.2.

### Step 5: preparation of N-(3-(difluoromethyl)-1-(1-(4-(4-(3-(2,6-dioxopiperidin-3-yl)-4-fluorobenzoyl)piperazin-1-yl)butanoyl)piperidin-4-yl)-1H-pyrazol-4-yl)-5-morpholinopyrazolo[1,5-a]pyrimidine-3-carboxamide

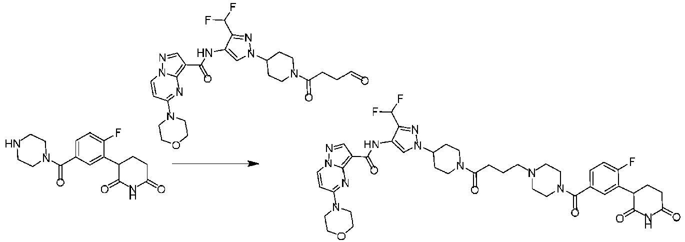

*N*-(3-(Difluoromethyl)-1-(1-(4-oxobutanoyl)piperidin-4-yl)-1*H*-pyrazol-4-yl)-5-morpholinopyrazolo[1,5-*a*]pyrimidine-3-carboxamide (55 mg, 0.10 mmol) and 3-(2-fluoro-5-(piperazine-1-carbonyl)phenyl)piperidine-2,6-dione (32 mg, 0.10 mmol) were dissolved in DCE (2 mL), and NaBH(OAc)₃ (63 mg, 0.30 mmol) was added. The mixture was stirred at room temperature for 2 h. Water (20 mL) was added to the reaction liquid, and the mixture was extracted with ethyl acetate (20 mL × 3). The organic phases were combined, washed with saturated brine (30 mL), dried over anhydrous sodium sulfate, filtered, and concentrated under reduced pressure. The crude product was purified by Prep-HPLC to give N-(3-(difluoromethyl)-1-(1-(4-(4-(3-(2,6-dioxopiperidin-3-yl)-4-ffuorobenzoyl)piperazin-1-yl)butanoyl)piperidin-4-yl)-1*H*-pyrazol-4-yl)-5-morpholinopyrazolo[1,5-*a*]pyrimidine-3-carboxamide.

LC-MS: (ESI, m/z): [M+H]⁺ = 834.1.

¹H NMR (400 MHz, DMSO-*d*₆) δ 10.91 (s, 1H), 9.40 (s, 1H), 8.82 (d, J = 4.3 Hz, 1H), 8.42 (s, 1H), 8.29 (s, 1H), 7.37 (s, 2H), 7.29-6.87 (m, 3H), 4.55-4.49 (m, 2H), 4.14-4.07 (m, 1H), 4.02-3.95 (m, 1H), 3.89-3.65 (m, 10H), 3.20-3.13 (m, 2H), 2.88-2.53 (m, 4H), 2.46-2.16 (m, 9H), 2.10-1.95 (m, 3H), 1.90-1.83 (m, 1H), 1.83-1.56 (m, 3H).

### Example 63: N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H pyrazol-4-yl)-5-(1,1-dioxothiomorpholinyl)pyrazolo[1,5-a]pyrimidine-3-carboxamide

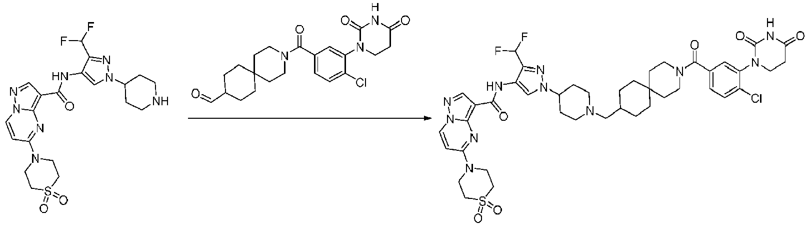

*N*-(3-(Difluoromethyl)-1-(piperidin-4-yl)-1*H*-pyrazol-4-yl)-5-(1,1-dioxothiomorpholinyl)pyrazolo[1,5-*a*]pyrimidine-3-carboxamide (80 mg, 0.16 mmol) and 3-(4-chloro-3-(2,4-dioxotetrahydropyrimidin-1(2*H*)-yl)benzoyl)-3-azaspiro[5.5]undecane-9-carbaldehyde (70 mg, 0.16 mmol) were dissolved in THF (3 mL), and sodium triacetoxyborohydride (102 mg, 0.48 mmol) was added. The mixture was stirred at room temperature for 16 h. Water (50 mL) was added to the reaction liquid, and the mixture was extracted with EA (30 mL × 3). The organic phase was washed with saturated brine (30 mL), dried over anhydrous sodium sulfate, filtered, and concentrated under reduced pressure. The crude product was isolated and purified by Prep-HPLC to give *N*-(1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)-5-(1,1-dioxothiomorpholinyl)pyrazolo[1,5-*a*]pyrimidine-3-carboxamide.

LC-MS: (ESI, m/z): [M+H]⁺ = 910.4.

¹H NMR (400 MHz, DMSO-*d*₆) δ 10.50 (s, 1H), 9.25 (s, 1H), 8.92 (d, *J* = 7.9 Hz, 1H), 8.39 (s, 1H), 8.34 (s, 1H), 7.63 (d, *J* = 8.2 Hz, 1H), 7.55 (d, *J* = 1.8 Hz, 1H), 7.39 (d, *J* = 8.2 Hz, 1H), 7.25-6.97 (m, 2H), 4.31-4.20 (m, 5H), 3.77-3.70 (m, 1H), 3.67-3.57 (m, 3H), 3.33-3.23 (m, 5H), 2.90 (d, *J=* 9.3 Hz, 2H), 2.75-2.67 (m, 3H), 2.18-2.11 (m, 2H), 2.07-1.87 (m, 6H), 1.73-1.65 (m, 2H), 1.62-1.23 (m, 7H), 1.15-0.92 (m, 4H).

### Example 64: (S)-5-(3-aminopiperidin-1-yl)-N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)pyrazolo[1,5-a]pyrimidine-3-carboxamide

### Step 1: preparation of tert-butyl (S)-(1-(3-((1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)carbamoyl)pyrazolo[1,5-a]pyrimidin-5-yl)piperidin-3-yl)carboxylate

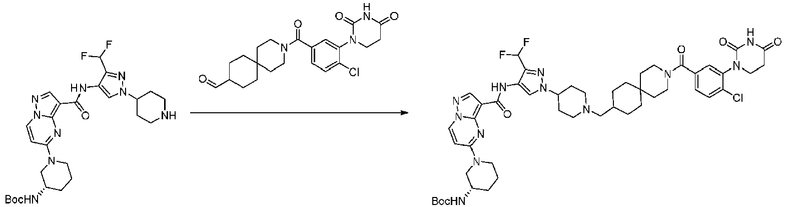

*tert*-Butyl (*S*)-(1-(3-((3-(difluoromethyl)-1-(piperidin-4-yl)-1*H*-pyrazol-4-yl)carbamoyl)pyrazolo[1,5-*a*]pyrimidin-5-yl)piperidin-3-yl)carbamate (70 mg, 0.12 mmol) and 3-(4-chloro-3-(2,4-dioxotetrahydropyrimidin-1(2*H*)-yl)benzoyl)-3-azaspiro[5,5]undecane-9-carbaldehyde (70 mg, 0.15 mmol) were dissolved in THF (2 mL), and NaBH(OAc)₃ (56 mg, 0.37 mmol) was added. The reaction liquid was stirred for 2 h. The reaction liquid was diluted with water (20 mL) and extracted with ethyl acetate (20 mL × 3). The organic phases were combined, washed with saturated brine, dried over anhydrous sodium sulfate, filtered, and concentrated under reduced pressure. The crude product was purified by column chromatography (methanol:dichloromethane = 1:24) to give tert-butyl (*S*)-(1-(3-((1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H-*pyrazol-4-yl)carbamoyl)pyrazolo[1,5-*a*]pyrimidin-5-yl)piperidin-3-yl)carboxylate.

LC-MS: (ESI, m/z): [M+H]⁺ = 975.4.

### Step 2: preparation of (S)-5-(3-aminopiperidin-1-yl)-N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)pyrazolo[1,5-a]pyrimidine-3-carboxamide

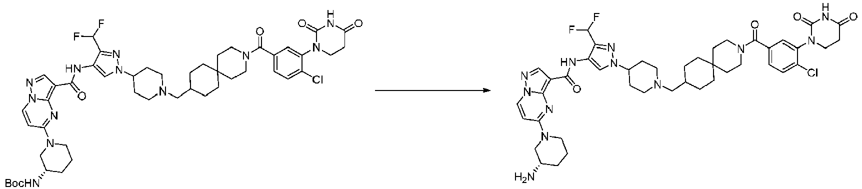

*tert*-Butyl (*S*)-(1-(3-((1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)carbamoyl)pyrazolo[1,5-*a*]pyrimidin-5-yl)piperidin-3-yl)carboxylate (30 mg, 0.03 mmol) was dissolved in DCM (5 mL), and TFA (1 mL) was added. The reaction liquid was stirred at room temperature for 1 h. The reaction liquid was directly concentrated under reduced pressure, and the crude product was purified by Prep-HPLC to give (*S*)-5-(3-aminopiperidin-1-yl)-*N*-(1-(1-((3 -(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)pyrazolo[1,5-*a*]pyrimidine-3-carboxamide.

LC-MS: (ESI, m/z): [M+H]⁺ = 875.3.

¹H NMR (400 MHz, DMSO-*d*₆) δ 8.77 (d, J = 7.9 Hz, 1H), 8.37 (s, 1H), 8.34-8.30 (m, 2H), 7.65 (d, *J* = 8.2 Hz, 1H), 7.53 (s, 1H), 7.40 (d, *J* = 8.4 Hz, 1H), 7.26-6.95 (m, 1H), 6.85 (d, *J* = 7.9 Hz, 1H), 4.23-4.13 (m, 2H), 3.82-3.74 (m, 1H), 3.70-3.40 (m, 7H), 3.30-3.10 (m, 3H), 2.97-2.92 (m, 2H), 2.77-2.73 (m, 2H), 2.25-1.75 (m, 11H), 1.75- 1.15 (m, 12H), 1.15-0.96 (m, 4H).

### Example 65: 5-((1R,4R)-2-oxa-5-azabicyclo[2.2.1]heptan-5-yl)-N-(1-((1S,4S)-4-(((2-(1-(4-chloro-3-(2,4-dioxotetrahydropyrimidin-1(2H)-yl)benzoyl)piperidin-4-yl)ethyl)(methyl)amino)methyl)-4-hydroxycyclohexyl)-3-(difluoromethyl)-1H-pyrazol-4-yl)pyrazolo[1,5-a]pyrimidine-3-carboxamide

### Step 1: preparation of 1,4-dioxaspiro[4.5]decan-8-yl 4-methylbenzenesulfonate

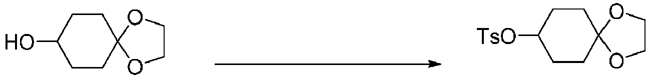

1,4-Dioxaspiro[4.5]decan-8-ol (25.7 g, 163 mmol) was dissolved in dichloromethane (100 mL), and TsCl (46.6 g, 244 mmol), TEA (49.3 g, 488 mmol) and DMAP (4.0 g, 33.0 mmol) were added with stirring. The reaction liquid was stirred overnight at room temperature. The reaction mixture was concentrated under reduced pressure, and water (200 mL) was added. The mixture was extracted with ethyl acetate (100 mL × 3). The organic phase was washed with saturated brine (200 mL), dried over anhydrous sodium sulfate, filtered, and concentrated under reduced pressure. The crude product was purified by column chromatography (ethyl acetate:petroleum ether = 3:7) to give 1,4-dioxaspiro[4.5]decan-8-yl 4-methylbenzenesulfonate.

LC-MS: (ESI, m/z): [M+Na]⁺ = 335.1.

### Step 2: preparation of 1-(1,4-dioxaspiro[4.5]decan-8-yl)-1H-pyrazole-3-carbaldehyde

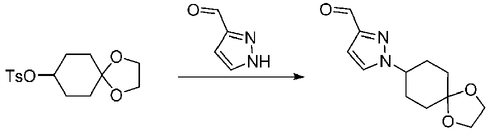

1,4-Dioxaspiro[4.5]decan-8-yl 4-methylbenzenesulfonate (48.5 g, 156.25 mmol) and 1*H*-pyrazole-3-carbaldehyde (12.5 g, 130.21 mmol) were dissolved in DMF (200 mL), and cesium carbonate (127.0 g, 390.63 mmol) and KI (25.9 g, 156.25 mmol) were added with stirring. The reaction liquid was heated to 50 °C and stirred overnight. The reaction liquid was diluted with water (2000 mL) and extracted with ethyl acetate (200 mL × 4). The organic phase was washed with saturated brine (300 mL), dried over anhydrous sodium sulfate, filtered, and concentrated under reduced pressure. The crude product was purified by column chromatography (ethyl acetate:petroleum ether = 1:4) to give 1-(1,4-dioxaspiro[4.5]decan-8-yl)-1*H*-pyrazole-3-carbaldehyde.

LC-MS: (ESI, m/z): [M+H]⁺ = 237.2.

¹H NMR (400 MHz, DMSO-*d*₆) δ 9.87 (s, 1H), 7.99 (d, *J* = 2.3 Hz, 1H), 6.77 (d, *J* = 2.4 Hz, 1H), 4.49-4.39 (m, 1H), 3.96-3.86 (m, 4H), 2.08-1.99 (m, 4H), 1.84-1.75 (m, 2H), 1.75-1.64 (m, 2H).

### Step 3: preparation of 3-(difluoromethyl)-1-(1,4-dioxaspiro[4.5]decan-8-yl)-1H-pyrazole

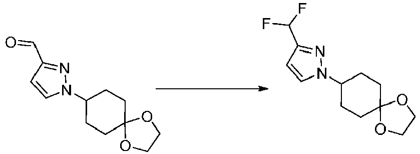

1-(1,4-Oxaspiro[4.5]decan-8-yl)-1*H*-pyrazole-3-carbaldehyde (13.3 g, 56.36 mmol) was dissolved in anhydrous dichloromethane (50 mL), and DAST (54.4 g, 338.14 mmol) was added slowly at 0 °C. The reaction liquid was stirred overnight at room temperature. The reaction liquid was quenched by slow addition of methanol and concentrated under reduced pressure. The crude product was purified by column chromatography (ethyl acetate:petroleum ether = 3:7) to give 3-(difluoromethyl)-1 -(1,4-dioxaspiro[4.5]decan-8-yl)-1*H*-pyrazole.

LC-MS: (ESI, m/z): [M+H]⁺ = 259.1.

### Step 4: preparation of 4-bromo-3-(difluoromethyl)-1-(1,4-dioxaspiro[4.5]decan-8-yl)-1H-pyrazole

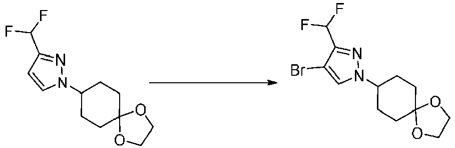

3-(Difluoromethyl)-1-(1,4-dioxaspiro[4.5]decan-8-yl)-1*H*-pyrazole (8.6 g, 33.33 mmol) was dissolved in DMF (50 mL), and NBS (7.12 g, 40.00 mmol) was added with stirring. The reaction liquid was heated to 60 °C and stirred for 2 h. The reaction liquid was diluted with water (500 mL) and extracted with ethyl acetate (100 mL × 4). The organic phase was washed with saturated brine (200 mL), dried over anhydrous sodium sulfate, filtered, and concentrated under reduced pressure. The crude product was purified by column chromatography (ethyl acetate:petroleum ether = 3:7) to give 4-bromo-3-(difluoromethyl)-1-(1,4-dioxaspiro[4.5]decan-8-yl)-1*H*-pyrazole.

LC-MS: (ESI, m/z): [M+H]⁺ = 337.0.

### Step 5: preparation of 4-(4-bromo-3-(difluoromethyl)-1H-pyrazol-1 -yl)cyclohexan-1 -one

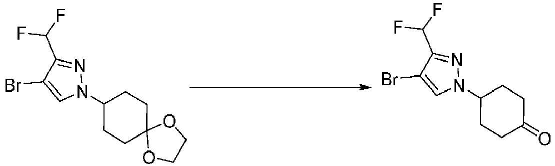

4-Bromo-3-(difluoromethyl)-1-(1,4-dioxaspiro[4.5]decan-8-yl)-1*H*-pyrazole (10.4 g, 30.95 mmol) was dissolved in acetone (40 mL), and HCl (2 mol/L, 60 mL) was added with stirring. The reaction liquid was stirred at 0 °C for 1 h. The reaction liquid was made weakly basic with saturated sodium bicarbonate solution and extracted with ethyl acetate (100 mL × 4). The organic phase was washed with saturated brine (200 mL), dried over anhydrous sodium sulfate, filtered, and concentrated under reduced pressure. The crude product was purified by column chromatography (ethyl acetate:petroleum ether = 1:4) to give 4-(4-bromo-3-(difluoromethyl)-1*H-*pyrpyrazol-1-yl)cyclohexan-1-one.

LC-MS: (ESI, m/z): [M+H]⁺ = 293.0.

### Step 6: preparation of 4-bromo-3-(difluoromethyl)-1-(1-oxaspiro[2.5]octan-6-yl)-1H-pyrazole

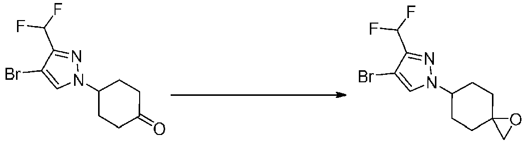

Trimethylsulfoxonium iodide (1.36 g, 6.16 mmol) was dissolved in dimethyl sulfoxide (20 mL), and NaH (246 mg, 10.27 mmol) was added at 0 °C. After the reaction liquid was stirred at room temperature for 1 h, 4-(4-bromo-3-(difluoromethyl)-1*H*-pyrazol-1-yl)cyclohexan-1-one (1.2 g, 5.14 mmol) was added, and the mixture was then stirred overnight at room temperature. The reaction liquid was diluted with water (100 mL) and extracted with ethyl acetate (50 mL × 3). The organic phase was washed with saturated brine (100 mL), dried over anhydrous sodium sulfate, filtered, and concentrated under reduced pressure. The crude product was purified by column chromatography (ethyl acetate:petroleum ether = 1:4) to give 4-bromo-3-(difluoromethyl)-1-(1-oxaspiro[2.5]octan-6-yl)-1*H*-pyrazole.

LC-MS: (ESI, m/z): [M+H]⁺ = 307.0.

### Step 7: preparation of N-(3-(difluoromethyl)-1-(1-oxaspiro[2.5]octan-6-yl)-1H-pyrazol-4-yl)-1,1-diphenylmethanimine

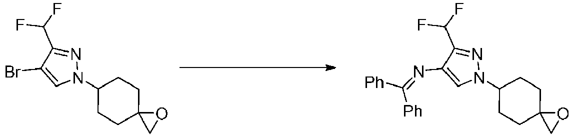

4-Bromo-3-(difluoromethyl)-1-(1-oxaspiro[2.5]octan-6-yl)-1*H*-pyrazole (800 mg, 3.104 mmol) and (Ph)₂NH (618 mg, 3.415 mmol) were dissolved in 1,4-dioxane (40 mL), and Pd₂(dba)₃ (568 mg, 0.62 mmol), Xantphos (717 mg, 1.24 mmol) and NaOt-Bu (894 mg, 9.31 mmol) were added with stirring. The reaction liquid was heated to 90 °C and stirred overnight under nitrogen atmosphere. The reaction liquid was directly concentrated under reduced pressure, diluted with water (100 mL), and extracted with ethyl acetate (50 mL × 3). The organic phase was washed with saturated brine (300 mL), dried over anhydrous sodium sulfate, filtered, and concentrated under reduced pressure. The crude product was purified by column chromatography (ethyl acetate:petroleum ether = 1:3) to give *N-*(3-(difluoromethyl)-1-(1-oxaspiro[2.5]octan-6-yl)-1*H*-pyrazol-4-yl)-1,1-diphenylmethanimine.

LC-MS: (ESI, m/z): [M+H]⁺ = 408.1.

### Step 8: preparation of tert-butyl 4-(2-((((1S,4S)-4-(3-(difluoromethyl)-4-((diphenylmethylene)amino)-1H-pyrazol-1-yl)-1-hydroxycyclohexyl)methyl)(methyl)amino-1-carboxylate

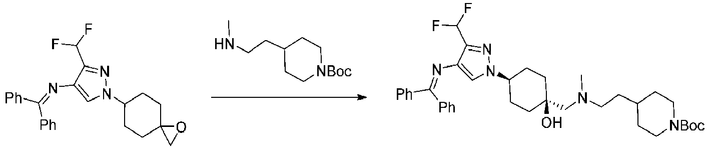

*N-*(3-(Difluoromethyl)-1-(1-oxaspiro[2.5]octan-6-yl)-1*H*pyrazol-4-yl)-1,1-diphenylmethanimine (180 mg, 0.44 mmol) and *tert*-butyl 4-(2-(methylamino)ethyl)piperidine-1-carboxylate (128 mg, 0.53 mmol) were dissolved in ethanol (4 mL). Water (2 mL) was then added, and K₂CO₃ (122 mg, 0.88 mmol) was added. After the reaction liquid was microwaved at 110 °C for half an hour, water (10 mL) was added, and the mixture was extracted with a methanol/dichloromethane solution (V/V = 1/9, 25 mL × 4). The organic phase was washed with saturated brine (30 mL), dried over anhydrous sodium sulfate, filtered, and concentrated under reduced pressure. The crude product was purified by column chromatography (dichloromethane:methanol = 1:9) to give tert-butyl 4-(2-((((1*S*,4*S*)-4-(3-(difluoromethyl)-4-((diphenylmethylene)amino)-1*H*-pyrazol-1 -yl)-1-hydroxycyclohexyl)methyl)(methyl)amino)ethyl)piperidine-1-carboxylate.

LC-MS: (ESI, m/z): [M+H]⁺ = 650.5.

### Step 9: preparation of tert-butyl 4-(2-((((1S,4S)-4-(4-amino-3-(difluoromethyl)-1H-pyrazol-1-yl)-1-hydroxycyclohexyl)methyl)(methyl)amino)ethyl)piperidine-1-carboxylate

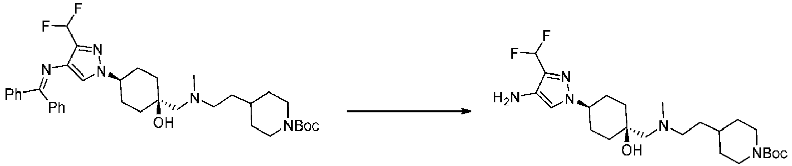

*tert*-Butyl 4-(2-((((1*S*,4*S*)-4-(3-(difluoromethyl)-4-((diphenylmethylene)amino)-1*H*-pyrazol-1-yl)-1-hydroxycyclohexyl)methyl)(methyl)amino)ethyl)piperidine-1-carboxylate (150 mg, 0.23 mmol) was dissolved in tetrahydrofuran (2 mL), and HCl (2 mol/L, 1 mL) was added. The reaction liquid was stirred at room temperature for 30 min. The reaction liquid was diluted with water (10 mL) and extracted with ethyl acetate (25 mL x 2). The aqueous phase was made weakly basic with saturated sodium bicarbonate solution and extracted with a mixed solution of methanol and dichloromethane (V/V = 1/9, 25 mL x 3). The organic phase was washed with saturated brine (30 mL), dried over anhydrous sodium sulfate, filtered, and concentrated under reduced pressure to give *tert*-butyl 4-(2-((((1*S*,4*S*)-4-(4-amino-3-(difluoromethyl)-1*H-*pyrazol-1 -yl)-1 -hydroxy cyclohexyl)methyl)(methyl)amino)ethyl)piperidine-1 -carboxylate.

LC-MS: (ESI, m/z): [M+H]⁺=486.2.

### Step 10: preparation of tert-butyl 4-(2-((((1S,4S)-4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)-1 -hydroxycyclohexyl)methyl)(methyl)amino)ethyl)piperidine-1-carboxylate

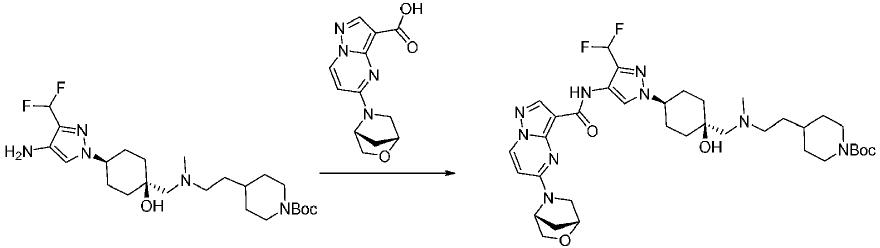

*tert-Butyl* 4-(2-((((1*S*,4*S*)-4-(4-amino-3-(difluoromethyl)-1*H*-pyrazol-1-yl)-1-hydroxycyclohexyl)methyl)(methyl)amino)ethyl)piperidine-1-carboxylate (85 mg, 0.17 mmol) and 5-((1*R*,4*R*)-2-oxa-5-azabicyclo[2.2.1]heptan-5-yl)pyrazolo[1,5-*a*]pyrimidine-3-carboxylic acid (91 mg, 0.34 mmol) were dissolved in DMF (5 mL), and *N*,*N-*diisopropylethylamine (68 mg, 0.51 mmol) and HATU (158 mg, 0.26 mmol) were added with stirring. The reaction liquid was stirred overnight at room temperature. The reaction liquid was diluted with water (50 mL) and extracted with a mixed solution of methanol and dichloromethane (V/V = 1/9, 25 mL × 3). The organic phase was dried over anhydrous sodium sulfate, filtered, and concentrated under reduced pressure. The crude product was purified by column chromatography (dichloromethane: methanol = 1:9) to give *tert*-butyl 4-(2-((((1*S,*4*S*)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)-1 -hydroxycyclohexyl)methyl)(methyl)amino)ethyl)piperidine-1 -carboxylate.

LC-MS: (ESI, m/z): [M+H]⁺=728.3.

### Step 11: preparation of 5-((1R,4R)-2-oxa-5-azabicyclo[2.2.1]heptan-5-yl)-N-(3-(difluoromethyl)-1-((1S,4S)-4-hydroxy-4-((methyl(2-(piperidin-4-yl)ethyl)amino)methyl)cyclohexyl)-1H-pyrazol-4-yl)pyrazolo[1,5-a]pyrimidine-3-carboxamide

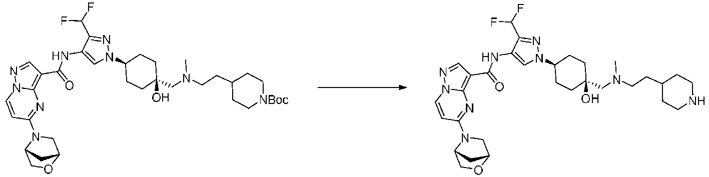

*tert-Butyl* 4-(2-((((1*S*,4*S*)-4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)-1-hydroxycyclohexyl)methyl)(methyl)amino)ethyl)piperidine-1-carboxylate (120 mg, 0.16 mmol) was dissolved in dichloromethane (2.5 mL), and trifluoroacetic acid (0.5 mL) was added. The mixture was stirred at room temperature for 2 h. The reaction liquid was directly concentrated under reduced pressure to give 5-((1*R*,4*R*)-2-oxa-5-azabicyclo[2.2.1]heptan-5-yl)-*N*-(3-(difluoromethyl)-1-((1*S*,4*S*)-4-hydroxy-4-((methyl(2-(piperidin-4-yl)ethyl)amino)methyl)cyclohexyl)-1*H*-pyrazol-4-yl)pyrazolo[1,5-*a*]pyrimidine-3-carboxamide, which was directly used in the next step without purification.

LC-MS: (ESI, m/z): [M+H]⁺=628.3.

### Step 12: preparation of 5-((1R,4R)-2-oxa-5-azabicyclo[2.2.1]heptan-5-yl)-N-(1-((1S,4S)-4-(((2-(1-(4-chloro-3-(2,4-dioxotetrahydropyrimidin-1(2H)-yl)benzoyl)piperidin-4-yl)ethyl)(methyl)amino)methyl)-4-hydroxycyclohexyl)-3-(difluoromethyl)-1H-pyrazol-4-yl)pyrazolo[1,5-a]pyrimidine-3-carboxamide

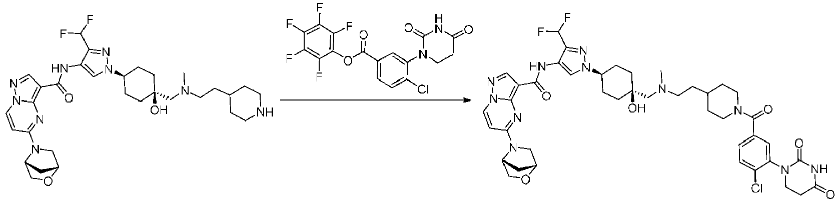

5-((1*R*,4*R*)-2-Oxa-5-azabicyclo[2.2.1]heptan-5-yl)-*N*-(3-(difluoromethyl)-1-((1*S*,4*S*)-4-hydroxy-4-((methyl(2-(piperidin-4-yl)ethyl)amino)methyl)cyclohexyl)-1*H*-pyrazol-4-yl)pyrazolo[1,5-*a*]pyrimidine-3-carboxamide (120 mg, 0.19 mmol) was dissolved in dimethyl sulfoxide (3 mL), and pentafluorophenyl 4-chloro-3-(2,4-dioxotetrahydropyrimidin-1(2*H*)-yl)benzoate (100 mg, 0.23 mmol) and *N,N-*diisopropylethylamine (148 mg, 1.14 mmol) were added with stirring. The reaction liquid was stirred at room temperature for 2 h. The reaction liquid was diluted with water (5 mL) and filtered, and the solid was purified by p-TLC (dichloromethane:methanol = 10:1) to give 5-((1*R*,4*R*)-2-oxa-5-azabicyclo[2.2.1]heptan-5-yl)-*N-*(1-((1*S*,4*S*)-4-(((2-(1-(4-chloro-3 -(2,4-dioxotetrahydropyrimidin-1(2*H*)-yl)benzoyl)piperidin-4-yl)ethyl)(methyl)amino)methyl)-4-hydroxycyclohexyl)-3 -(difluoromethyl)- 1*H*-pyrazol-4-yl)pyrazolo [1,5-*a*]pyrimidine-3-carboxamide.

LC-MS: (ESI, m/z): [M+H]⁺=878.2.

¹H NMR (400 MHz, DMSO-*d*₆) δ 10.50 (s, 1H), 9.51 (s, 1H), 8.78 (d, *J*= 7.7 Hz, 1H), 8.38 (s, 1H), 8.26 (s, 1H), 7.63 (d, *J*= 8.3 Hz, 1H), 7.55 (s, 1H), 7.38 (d, *J*= 7.9 Hz, 1H), 7.26-6.96 (m, 1H), 6.90-6.42 (m, 1H), 5.33-5.05 (m, 1H), 4.77 (d, *J=* 17.1 Hz, 1H), 4.50-4.39 (m, 1H), 4.20-3.97 (m, 2H), 3.87-3.70 (m, 3H), 3.64-3.40 (m, 4H), 3.12-2.92 (m, 1H), 2.82-2.67 (m, 3H), 2.46-2.38 (m, 2H), 2.32-2.21 (m, 5H), 2.12- 1.92 (m, 4H), 1.86-1.55 (m, 7H), 1.52-1.32 (m, 4H), 1.18-1.04 (m, 2H).

### II. Biological Activity Test Examples

### Test Example 1: IRAK4 Kinase Activity Test

The KinEASE-STK S1 serine/threonine kinase kit (Cisbio) was used to test the inhibitory effect of the compounds on the IRAK4 kinase activity, and the method is specifically as follows: A compound was dissolved in dimethyl sulfoxide, and the solution was diluted in the buffer of the kit with an equal gradient, such that the final concentration range of the test compound in the reaction systems was 10,000 nM-0.038nM. Then 2.5 nM IRAK4 kinase (Carna), 1 µM biotinylated polypeptide substrate (Cisbio) and 7 µM adenosine triphosphate (Sigma-Aldrich) were sequentially added, and the mixtures were incubated at 37 °C for 120 min. Subsequently, an anti-phosphoserine/threonine antibody (Cisbio) conjugated with a europium element compound and conjugated modified XL665 streptavidin (Cisbio) were added to the reaction systems to stop the reactions. After 1 h of incubation at room temperature, the fluorescence intensity of each well was measured at the excitation wavelength of 337 nm and the emission wavelengths of 620 nm and 665 nm with a microplate reader EnVision (PerkinElmer) in the HTRF mode, and Ratio values were calculated using the formula Ratio = (665 nm/620 nm) × 10⁴. The inhibition rate of the compound at each concentration was calculated by comparing the fluorescence intensity ratio with that of the control group. Then a non-linear curve fitting of logarithmic concentration-inhibition rate was performed using GraphPad Prism 7, and the IC₅₀ value of the compound was obtained.

**Table 1:**

| Example | IC₅₀ (nM) |
|---|---|
| Example 1 | 30.3 |
| Example 2 | 15.5 |
| Example 3 | 67.2 |
| Example 4 | 37.0 |
| Example 5 | 359.7 |
| Example 6 | 524.4 |
| Example 7 | 313.4 |
| Example 8 | 10.6 |
| Example 9 | 131 |
| Example 10 | 12.92 |
| Example 11 | 18.5 |
| Example 12 | 270.4 |
| Example 13 | 19.3 |
| Example 14 | 124.3 |
| Example 15 | 14.2 |
| Example 16 | 8.8 |
| Example 17 | 15.1 |
| Example 18 | 66.9 |
| Example 19 | 3.8 |
| Example 20 | 89.2 |
| Example 21 | 9.9 |
| Example 22 | 9.29 |
| Example 23 | 22.96 |
| Example 24 | 6.08 |
| Example 25 | 14.13 |
| Example 26 | 14.30 |
| Example 27 | 13.50 |
| Example 28 | 12.87 |
| Example 29 | 17.04 |
| Example 30 | 4.64 |
| Example 31 | 8.05 |
| Example 32 | 7.74 |
| Example 33 | 23.90 |
| Example 34 | 11.03 |
| Example 35 | 3.36 |
| Example 36 | 3.86 |
| Example 37 | 13.08 |
| Example 38 | 19.97 |
| Example 39 | 17.04 |
| Example 40 | 34.50 |
| Example 41 | 25.52 |
| Example 42 | 18.64 |
| Example 43 | 45.01 |
| Example 44 | 13.45 |
| Example 45 | 2.98 |
| Example 46 | 277.70 |
| Example 47 | 7.86 |
| Example 48 | 36.07 |
| Example 49 | 7.05 |
| Example 50 | 36.74 |
| Example 51 | 27.10 |
| Example 52 | 23.83 |
| Example 54 | 9.82 |
| Example 55 | 21.31 |
| Example 56 | 3.87 |
| Example 57 | 4.57 |
| Example 59 | 8.71 |
| Example 60 | 21.94 |
| Example 61 | 9.06 |
| Example 62 | 28.86 |
| Example 63 | 36.24 |
| Example 64 | 5.93 |
| Example 65 | 52.15 |

| | |
|---|---|
| "-" represents not detected. | |

Experimental results: The compounds of the present invention can bind efficiently to the target protein and inhibit the IRAK4 kinase activity.

### Test Example 2: Degradation of IRAK4 in THP-1 Cells by Compounds

THP-1 cells (Stem Cell Bank, Chinese Academy of Sciences) were inoculated onto a 24-well cell culture plate at 0.95 mL/well, with a cell density of 5 × 10⁵ cells/well. The cell plate was incubated overnight in a 37 °C, 5% carbon dioxide incubator, and 50 µL of a solution of a compound in dimethyl sulfoxide was added, such that the final concentration of the compound fell within the range of 0.03-3000 nM. After another 24 h of incubation, cells were collected into 1.5 mL centrifuge tubes and centrifuged at 1000 rpm at 4 °C for 5 min. The cell pellets were washed twice with 1× DPBS. The resuspended cells were lysed with 200 µL of lysis buffer (Western and IP cell lysis buffer (Beyotime), supplemented with 1 mM phenylmethylsulfonyl fluoride and protease inhibitor cocktail (Beyotime)), left to stand on ice for 30 min, and then centrifuged at 14,000 g at 4 °C for 10 min. The supernatant samples were assayed by Western Blot for IRAK4 protein levels.

### Human IRAK4 AlphaLISA Assay

The above cell lysates were also tested for IRAK4 degradation using a human IRAK4 AlphaLISA assay kit (PerkinElmer, AL3117C). The method is specifically as follows: 2-µL test samples of cell lysate supernatant were transferred to a white 384-well plate (PerkinElmer, 6007299), and 4 µL of 5x anti-IRAK4 receptor beads (PerkinElmer, final concentration of 10 µg/mL) was then added. The plate was incubated at 23 °C for 30 min. Then 4 µL of 5x biotin-labeled anti-IRAK4 antibody (PerkinElmer, final concentration of 1 nM) was added, and the plate was incubated at 23 °C for 60 min. Finally, 10 µL of 2x streptavidin-labeled donor beads (final concentration of 40 µg/mL) was added to each well, and the plate was incubated in the dark at 23 °C for 30 min. After the incubation was complete, AlphaLISA signal values were measured on a microplate reader EnVision (PerkinElmer, 2105) in the AlphaScreen standard mode, and IRAK4 concentrations were calculated from these signal values. Then a non-linear curve fitting of logarithmic concentration-inhibition rate was performed using GraphPad Prism 7, and the DC₅₀ and Dₘₐₓ values of the compound were obtained.

**Table 2:**

| Example | DC₅₀ (nM) | Dₘₐₓ (%) |
|---|---|---|
| Example 1 | 3.7 | 90.0 |
| Example 2 | 1.8 | 95.8 |
| Example 3 | - | - |
| Example 4 | - | - |
| Example 5 | - | - |
| Example 6 | - | - |
| Example 7 | 148.5 | 76.4 |
| Example 8 | 8.8 | 86.8 |
| Example 9 | 128.6 | 81.0 |
| Example 10 | 2.8 | 99.3 |
| Example 11 | 12.7 | 90.7 |
| Example 12 | - | - |
| Example 13 | 5.5 | 84.4 |
| Example 14 | - | - |
| Example 15 | 2.5 | 92.1 |
| Example 16 | 6.4 | 87.9 |
| Example 17 | 10.37 | 99.4 |
| Example 18 | 54.3 | 62.5 |
| Example 19 | 5.1 | 97.7 |
| Example 20 | 78.6 | 86.96 |
| Example 21 | 9.6 | 95.5 |
| Example 22 | 3.42 | 96.53 |
| Example 23 | 10.10 | 93.65 |
| Example 24 | 6.16 | 96.32 |
| Example 25 | 62.13 | 97.82 |
| Example 28 | 3.18 | 89.42 |
| Example 29 | 2.75 | 98.48 |
| Example 30 | 5.23 | 90.09 |
| Example 31 | 6.10 | 91.10 |
| Example 32 | 3.46 | 90.53 |
| Example 34 | 3.55 | 87.11 |
| Example 35 | 0.78 | 100 |
| Example 36 | 3.45 | 96.02 |
| Example 37 | 41.96 | 81.07 |
| Example 39 | 10.86 | 93.89 |
| Example 40 | 32.75 | 79.18 |
| Example 42 | 3.66 | 91.70 |
| Example 43 | 15.08 | 79.51 |
| Example 44 | 6.30 | 80.45 |
| Example 45 | 0.82 | 96.16 |
| Example 47 | 4.84 | 99.01 |
| Example 48 | 5.55 | 94.41 |
| Example 49 | 3.22 | 97.07 |
| Example 50 | 5.94 | 82.09 |
| Example 52 | 18.45 | 95.43 |
| Example 54 | 8.13 | 88.18 |
| Example 55 | 7.52 | 79.18 |
| Example 56 | 0.41 | 96.44 |
| Example 57 | 0.58 | 96.57 |
| Example 59 | 7.92 | 70.81 |
| Example 60 | 9.36 | 96.59 |
| Example 61 | 28.96 | 87.91 |
| Example 63 | 9.18 | 79.3 |
| Example 64 | 7.47 | 96.06 |
| Example 65 | 14.26 | 87.24 |

| | | |
|---|---|---|
| "-" represents not detected. | | |

Experimental results: The compounds of the present invention can effectively degrade the IRAK4 kinase protein in cells, and have excellent degradation activity.

Test Example 3: Determination of Concentration of Cytokines Secreted by LPS-Induced Normal Human Whole Blood PBMCs
(1) Determination of concentration of cytokines secreted by LPS-induced normal human whole blood PBMCs

Fresh normal human whole blood (healthy people from 22-50 years of age, freshly collected blood) anticoagulated with heparin sodium was plated onto a 96-well clear cell plate (Labserv, 310109008) at 190 µL/well, and 10 µL of diluted compound solution was then added to corresponding wells, such that the final concentration of the compound fell within the range of 10-20,000 nM. The final concentration of dimethyl sulfoxide was 0.1%. The compound-treated cell plate was sealed with a plate-sealing membrane. After the plate was incubated in a 37 °C, 5% carbon dioxide incubator for 20 h, 10 µL of LPS (0111:B4) (Sigma, L4391) was added, making a final concentration of 100 ng/mL. The cell plate was sealed with a plate-sealing membrane. After the plate was incubated in a 37 °C, 5% carbon dioxide incubator for another 5 h, the plate was centrifuged at 2000 rpm for 10 min, and 30 µL of supernatant plasma was transferred from each well to a new 96-well clear cell plate for cytokine concentration testing and stored at -80 °C before testing.

### Interleukin-6 (IL-6) AlphaLISA assay

Supernatant plasma samples of LPS-induced human whole blood (healthy people from 22-50 years of age, freshly collected blood) were assayed for IL-6 concentrations using an interleukin-6 (IL-6) AlphaLISA assay kit (PerkinElmer, AL223 C).

IL-6 standard solutions with different concentrations, ranging from 0 to 100,000 pg/mL, were prepared by following the instructions for use of the product. 2 µL of each of the IL-6 standard solutions and test samples of cell supernatant were added to a white 384-well plate (PerkinElmer, 6007299), and 8 µL of a mixed solution of 5x anti-IL-6 receptor beads (PerminElmer, final concentration of 10 µg/mL) and biotin-labeled anti-IL-6 antibody (PerminElmer, final concentration of 1 nM) was then added to each well. The plate was incubated at 23 °C for 60 min. Finally, 10 µL of 2x streptavidin-labeled donor beads (final concentration of 40 µg/mL) was added to each well, and the plate was incubated in the dark at 23 °C for another 30 min. After the incubation was complete, AlphaLISA signal values were measured on a microplate reader EnVision (PerkinElmer, 2105) in the AlphaScreen standard mode. A standard curve was plotted from the AlphaLISA signal values of the IL-6 standard solutions, and the IL-6 concentration in each cell lysate supernatant was determined from the standard curve according to the AlphaLISA signal value of the test sample. The inhibition rate of the compound at each concentration was calculated by comparing the IL-6 concentration with that of the control group. Then a non-linear curve fitting of logarithmic concentration-inhibition rate was performed using GraphPad Prism 7, and the IC₅₀ value of the compound was calculated.

### (2) Determination of concentration of cytokines secreted by LPS-induced human PBMCs

Frozen human PBMCs (Milestone, PB010C) were thawed and resuspended in RPMI 1640 medium (Gibco, A1049101) supplemented with 10% fetal bovine serum (Gibco, 10099141), 100 U/mL penicillin and 100 µg/mL streptomycin (Gibco, 15140122). On the same day, PBMCs were plated onto a 96-well clear cell plate (Labserv, 310109008) at 150 µL/well, with a cell density of 2 x 105 cells/well. Then 50 µL of diluted compound solution was added to corresponding wells with cells, such that the final concentration of the compound fell within the range of 0.026-10,000 nM. The final concentration of DMSO was 0.25%. After the compound-treated cell plate was incubated in a 37 °C, 5% CO2 incubator for 1 h or 20 h, 10 µL of LPS (0111:B4) (Sigma, L4391) was added, making a final concentration of 100 ng/mL. After the cell plate was incubated in a 37 °C, 5% CO2 incubator for another 5 h, the plate was centrifuged at 2000 rpm for 10 min, and 100 µL of cell supernatant was transferred from each well to a new 96-well clear cell plate for cytokine concentration testing and stored at -80 °C before testing.

### Interleukin-6 (IL-6) AlphaLISA assay

The LPS-induced human PBMC cell supernatants were assayed for IL-6 concentrations using an interleukin-6 (IL-6) AlphaLISA assay kit (PerkinElmer, AL223 C).

IL-6 standard solutions with different concentrations, ranging from 0 to 100,000 pg/mL, were prepared by following the instructions for use of the product. 2 µL of each of the IL-6 standard solutions and test samples of cell supernatant were added to a white 384-well plate (PerkinElmer, 6007299), and 8 µL of a mixed solution of 5x anti-IL-6 receptor beads (final concentration of 10 µg/mL) and biotin-labeled anti-IL-6 antibody (final concentration of 1 nM) was then added to each well. The plate was incubated at 23 °C for 60 min. Finally, 10 µL of 2x streptavidin-labeled donor beads (final concentration of 40 µg/mL) was added to each well, and the plate was incubated in the dark at 23 °C for another 30 min. After the incubation was complete, AlphaLISA signal values were measured on a microplate reader EnVision (PerkinElmer, 2105) in the AlphaScreen standard mode. A standard curve was plotted from the AlphaLISA signal values of the IL-6 standard solutions, and the IL-6 concentration in each cell lysate supernatant was determined from the standard curve according to the AlphaLISA signal value of the test sample. The inhibition rate of the compound at each concentration was calculated by comparing the IL-6 concentration with that of the control group. Then a non-linear curve fitting of logarithmic concentration-inhibition rate was performed using GraphPad Prism 7, and the IC₅₀ value of the compound was calculated.

**Table 3:**

| Example | Human whole blood IC₅₀ (nM) | hPBMC IC₅₀ (nM) |
|---|---|---|
| Example 1 | 287 | 3.1 |
| Example 2 | 246 | 2.2 |

Experimental results: The compounds of the present invention have good inhibitory effects on IL-6 production by immune cells.

## Claims

1. A compound of formula I, a stereoisomer, enantiomer, diastereomer, deuteride, hydrate, solvate or prodrug thereof or a pharmaceutically acceptable salt thereof:
PTM-L-ULM I
wherein:
the PTM has a structure of the following formula: 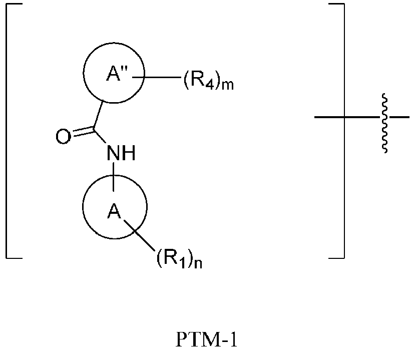
wherein in the PTM-1:
m is 0, 1, 2, 3 or 4;
n is 0, 1, 2, 3 or 4;
ring A" is 6-10 membered aryl or is 5-11 membered monocyclic or bicyclic heteroaryl containing 1, 2 or/and 3 heteroatoms selected from N, O or S;
ring A is 6-10 membered aryl or is 5-11 membered monocyclic or bicyclic heteroaryl containing 1, 2 or/and 3 heteroatoms selected from N, O or S;
each R₁ is independently selected from: C₁-C₄ alkyl, -O(C₁-C₄ alkyl), -N(C₁-C₄ alkyl)₁₋₂, C₃-C₈ cycloalkyl, - O(C₃-C₈ cycloalkyl), -NH(C₃-C₈ cycloalkyl), 3-8 membered heterocycloalkyl, -O(3-8 membered heterocycloalkyl), -NH(3-8 membered heterocycloalkyl), 6-10 membered aryl, -0(6-10 membered aryl), - NH(6-10 membered aryl), 5-6 membered heteroaryl, -O(5-6 membered heteroaryl), -NH(5-6 membered heteroaryl), CN, halogen, -OH, -NH₂, -NHC(O)(C₁-C₄ alkyl), -NHS(O)(C₁-C₄ alkyl), -NHS(O)₂(C₁-C₄ alkyl), -COOH, -C(O)NH₂, -S(O)₂H, -S(O)₂NH₂, -C(O)(C₁-C₄ alkyl), -C(O)NH(C₁-C₄ alkyl), -C(O)N(C₁-C₄ alkyl)₂, -C(O)O(C₁-C₄ alkyl), -S(O)(C₁-C₄ alkyl), -S(O)₂(C₁-C₄ alkyl), -S(O)₂NH(C₁-C₄ alkyl), -S(O)₂N(C₁-C₄ alkyl)₂ or -S(O)NH(C₁-C₄ alkyl), wherein the alkyl, cycloalkyl, heterocycloalkyl, aryl and heteroaryl are optionally substituted with groups selected from halogen, cyano, -OH, C₁-C₄ alkyl, -O(C₁-C₄ alkyl), methylpiperidine, - S(O)₂H, -S(O)₂(C₁-C₄ alkyl), -C(O)N(C₁-C₄ alkyl)₁₋₂ and -C(O)O(C₁-C₄ alkyl);
or the R₁, together with the atom to which it is attached, forms cycloalkyl, heterocycloalkyl, aryl or heteroaryl;
each R₄ is independently hydrogen, C₁-C₄ alkyl, -O(C₁-C₄ alkyl), C₃-C₈ cycloalkyl, 3-8 membered heterocycloalkyl, 6-10 membered aryl, 5-6 membered heteroaryl, CN, halogen, -OH, -NHC(O)(C₁-C₄ alkyl), -NHS(O)(C₁-C₄ alkyl), -NHS(O)₂(C₁-C₄ alkyl), -COOH, -C(O)NH₂, -S(O)₂H, -S(O)₂NH₂, -C(O)(C₁-C₄ alkyl), -C(O)NH(C₁-C₄ alkyl), -C(O)N(C₁-C₄ alkyl)₂, -C(O)O(C₁-C₄ alkyl), -S(O)(C₁-C₄ alkyl), -S(O)₂(C₁-C₄ alkyl), -S(O)₂NH(C₁-C₄ alkyl), -S(O)₂N(C₁-C₄ alkyl)₂, -S(O)NH(C₁-C₄ alkyl) or X(R₂)(R₃), wherein the alkyl, cycloalkyl, heterocycloalkyl, aryl and heteroaryl are optionally substituted with groups selected from halogen, cyano, -OH, C₁-C₄ alkyl, -O(C₁-C₄ alkyl), methylpiperidine, -S(O)₂H, -S(O)₂(C₁-C₄ alkyl), -C(O)N(C₁-C₄ alkyl)₁₋₂, and -C(O)O(C₁-C₄ alkyl); X is O, S, CH₂ or N;
R₂ and R₃ are independently selected from: hydrogen, C₁-C₆ alkyl, C₃-C₈ cycloalkyl, 3-8 membered heterocycloalkyl, 6-10 membered aryl or 5-6 membered heteroaryl, wherein the alkyl, cycloalkyl, heterocycloalkyl, aryl and heteroaryl are each independently optionally substituted with one or more substituents selected from halogen, OH, oxo, N(R_{b})₂, oxopyrrolidinyl and morpholinyl, or R₂ and R₃, together with the atom to which they are attached, form C₃-C₈ cycloalkyl or 3-8 membered heterocycloalkyl, wherein the cycloalkyl or heterocycloalkyl is optionally substituted with one or more substituents selected from Rₐ;
Rₐ is independently selected from oxo, C₁-C₄ alkyl, C₃-C₆ cycloalkyl, CF₃, CHF₂, OH, halogen or NH₂,
wherein the alkyl is optionally substituted with C₃-C₆ cycloalkyl or CF₃; and
R_{b} is independently selected from H or C₁-C₄ alkyl;
L is a linking chain, which links PTM and ULM by covalent bonds;
the ULM has a structure of the following formula: 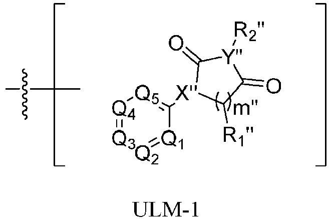
wherein in the ULM-1:
X" is C or N;
Y" is C, N, O or S;
Q₁, Q₂, Q₃, Q₄ and Q₅ are each independently CR₃" or N;
each R₃" is independently hydrogen, deuterium, hydroxy, amino, cyano, halogen, C₁-C₆ alkyl, C₃-C₈ cycloalkyl, 3-8 membered heterocycloalkyl, 6-10 membered aryl, 5-10 membered heteroaryl, -O(C₁-C₆ alkyl), -O-(C₃-C₈ cycloalkyl), -O-(3-8 membered heterocycloalkyl), N(C₁-C₆ alkyl)₁₋₂, NH(C₃-C₈ cycloalkyl), NH(3-8 membered heterocycloalkyl), -0-(6-10 aryl) or -0-(5-10 membered heteroaryl), wherein the alkyl, cycloalkyl, heterocycloalkyl, aryl and heteroaryl are optionally substituted with 1-3 groups independently selected from hydroxy, halogen, cyano and amino; or R₃", together with the atom to which it is attached, forms cycloalkyl, heterocycloalkyl, heteroaryl or aryl;
m" is 1, 2 or 3;
each R₁" is independently hydrogen, deuterium, hydroxy, amino, cyano, halogen, C₁-C₆ alkyl, C₃-C₈ cycloalkyl, 3-8 membered heterocycloalkyl, 6-10 membered aryl, 5-10 membered heteroaryl or -O(C₁-C₆ alkyl), wherein the alkyl, cycloalkyl, heterocycloalkyl, aryl and heteroaryl are optionally substituted with 1-3 groups independently selected from hydroxy, halogen, cyano and amino;
R₂" is hydrogen, deuterium, C₁-C₆ alkyl or C₃-C₆ cycloalkyl, wherein the C₁-C₆ alkyl and C₃-C₆ cycloalkyl are optionally substituted with 1-3 groups independently selected from hydroxy, halogen, cyano, amino and - OC(O)(C₁-C₆ alkyl).

2. The compound of formula I, the stereoisomer, enantiomer, diastereomer, deuteride, hydrate, solvate or prodrug thereof or the pharmaceutically acceptable salt thereof according to claim 1, wherein the PTM is 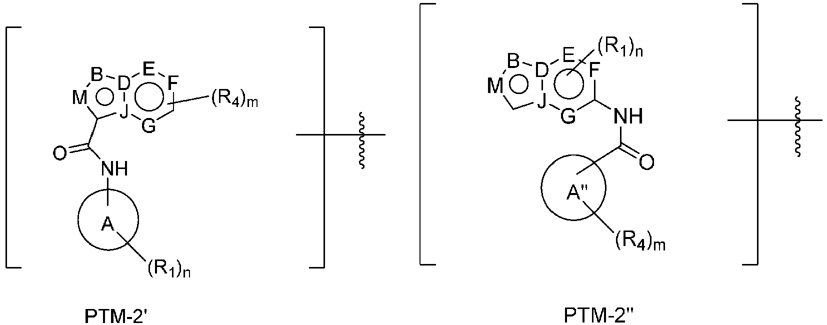
wherein in the PTM-2' and PTM-2":
B is CH, N, O or S;
M is CH, N, O or S;
D is CH or N;
E is CH or N;
F is CH or N;
G is CH or N;
J is CH or N;
when B or M is S or O, D is CH, E is N, F is CH, G is N, and J is CH; and B and M are not simultaneously S and/or O;
the ring A, ring A", R₁, n, R₄ and m are as defined and described in PTM-1 in claim 1.

3. The compound of formula I, the stereoisomer, enantiomer, diastereomer, deuteride, hydrate, solvate or prodrug thereof or the pharmaceutically acceptable salt thereof according to claim 2, wherein 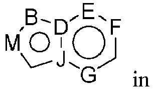
the PTM-2' is 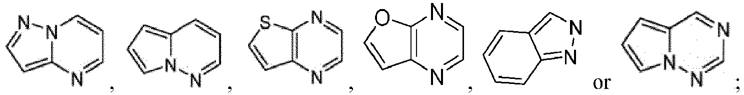
and/or, the ULM has a structure of the following formula: 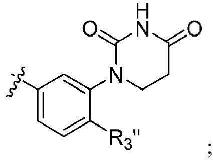 R₃" is halogen, C₁-C₆ alkyl or -O(C₁-C₆ alkyl);
and/or, the pharmaceutically acceptable salt is a formate.

4. The compound of formula I, the stereoisomer, enantiomer, diastereomer, deuteride, hydrate, solvate or prodrug thereof or the pharmaceutically acceptable salt thereof according to claim 2, wherein the PTM is 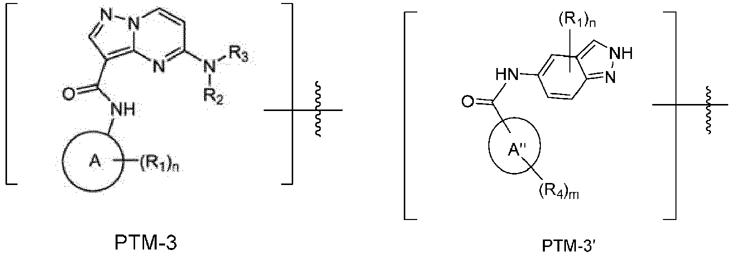 wherein the ring A, ring A", R₁, R₂, R₃, R₄, m and n are as defined and described in PTM-1 in claim 1.

5. The compound of formula I, the stereoisomer, enantiomer, diastereomer, deuteride, hydrate, solvate or prodrug thereof or the pharmaceutically acceptable salt thereof according to any of claims 1, 2 and 4, wherein ring A is phenyl or is 5-6 membered heteroaryl containing 1, 2 or 3 heteroatoms selected from N or O; preferably, ring A is phenyl, pyridyl, pyrrolyl, pyrazolyl, imidazolyl, pyrimidinyl, triazolyl, tetrazolyl, thienyl, thiazolyl, furanyl, oxazolyl, isoxazolyl or isothiazolyl; further preferably, ring A is pyrazolyl or thienyl; and/or, each R₃" is independently hydrogen, deuterium, hydroxy, amino, cyano, halogen, C₁-C₆ alkyl, C₃-C₈ cycloalkyl, 3-8 membered heterocycloalkyl, 6-10 membered aryl, 5-10 membered heteroaryl, -O(C₁-C₆ alkyl), -O-(C₃-C₈ cycloalkyl), -O-(3-8 membered heterocycloalkyl), N(C₁-C₆ alkyl)₁₋₂, NH(C₃-C₈ cycloalkyl), NH(3-8 membered heterocycloalkyl), -O-(6-10 aryl) or -0-(5-10 membered heteroaryl), wherein the alkyl, cycloalkyl, heterocycloalkyl, aryl and heteroaryl are optionally substituted with 1-3 groups independently selected from hydroxy, halogen, cyano and amino; or R₃", together with the atom to which it is attached, forms cycloalkyl or heterocycloalkyl;
and/or, the R₁ is independently selected from: C₁-C₄ alkyl, C₃-C₆ cycloalkyl, 3-6 membered heterocycloalkyl, CN or halogen, wherein the alkyl, cycloalkyl and heterocycloalkyl are optionally substituted with halogen, OH, CH₃ or OCH₃; preferably, R₁ is independently selected from: C₁-C₄ alkyl, C₃-C₆ cycloalkyl, 3-6 membered heterocycloalkyl containing 1 or 2 heteroatoms selected from N, CN or halogen, wherein the alkyl, cycloalkyl and heterocycloalkyl are optionally substituted with halogen, OH, CH₃ or OCH₃; further preferably, the R₁ is CHF₂, CF₃, cyclohexyl, piperidinyl, methyl, cyano or piperazinyl;
and/or, the R₂ and R₃ are each independently selected from: hydrogen, C₁-C₆ alkyl, C₃-C₈ cycloalkyl or 3-8 membered heterocycloalkyl, and are each optionally substituted with one or more halogens, OH, N(R_{b})₂ or morpholinyl groups, or R₂ and R₃, together with the nitrogen atom to which they are attached, form 3-8 membered heterocycloalkyl, wherein the heterocycloalkyl is optionally substituted with one or more substituents selected from Rₐ; Rₐ is independently selected from C₁-C₄ alkyl, C₃-C₆ cycloalkyl, CF₃, CHF₂, OH, halogen or NH₂, wherein the alkyl is optionally substituted with C₃-C₆ cycloalkyl or CF₃; and R_{b} is independently selected from H or C₁-C₄ alkyl; preferably, R₂ and R₃, together with the nitrogen atom to which they are attached, form 3-8 membered heterocycloalkyl containing, in addition to the N atom to which they are attached, 1 additional heteroatom selected from N or O, wherein the heterocycloalkyl is optionally substituted with one or more substituents selected from Rₐ; the Rₐ is independently selected from C₁-C₄ alkyl, CF₃, CHF₂, OH, halogen or NH₂; further preferably, the R₂ and R₃, together with the nitrogen atom to which
they are attached, form 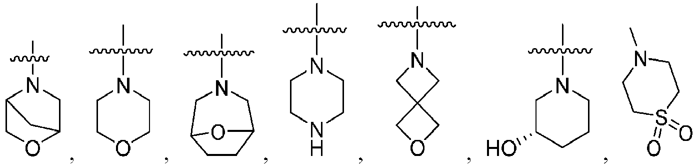 or

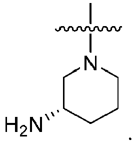

6. The compound of formula I, the stereoisomer, enantiomer, diastereomer, deuteride, hydrate, solvate or prodrug thereof or the pharmaceutically acceptable salt thereof according to any of claims 1, 2 and 4, wherein the PTM has a structure of the following formula: 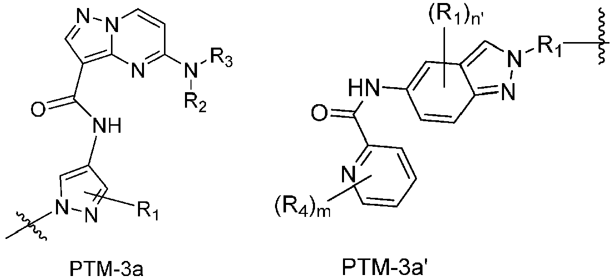 wherein,
n' is 0, 1, 2 or 3;
the R₁, R₂, R₃, R₄, m and n are as defined and described in PTM-1, PTM-2' and PTM-3 in claim 1.

7. The compound of formula I, the stereoisomer, enantiomer, diastereomer, deuteride, hydrate, solvate or prodrug thereof or the pharmaceutically acceptable salt thereof according to any of claims 1, 2 and 4, wherein the PTM has a structure of the following formula: 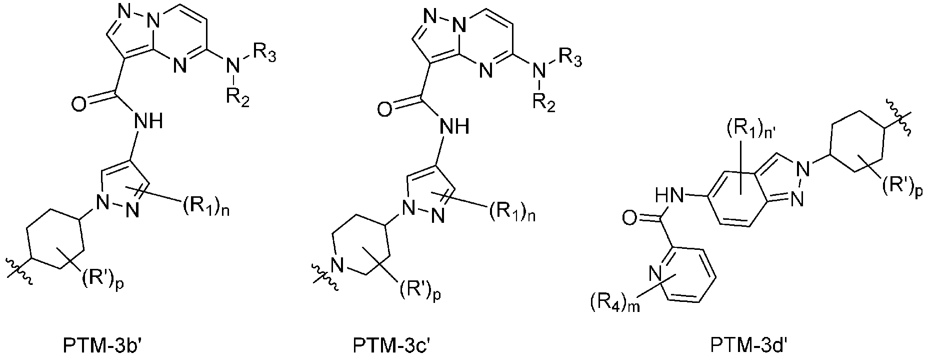 wherein,
n' is 0, 1, 2 or 3;
each R' is independently hydrogen, deuterium, hydroxy, amino, cyano, halogen, C₁-C₆ alkyl or -O(C₁-C₆ alkyl), wherein the alkyl is optionally substituted with 1-3 groups independently selected from hydroxy, halogen, cyano and amino;
p is 0, 1, 2, 3, 4, 5, 6, 7, 8 or 9;
the R₁, R₂, R₃, R₄, m and n are as defined and described in PTM-1, PTM-2' and PTM-3 in claim 1;
preferably, the PTM has a structure of the following formula: 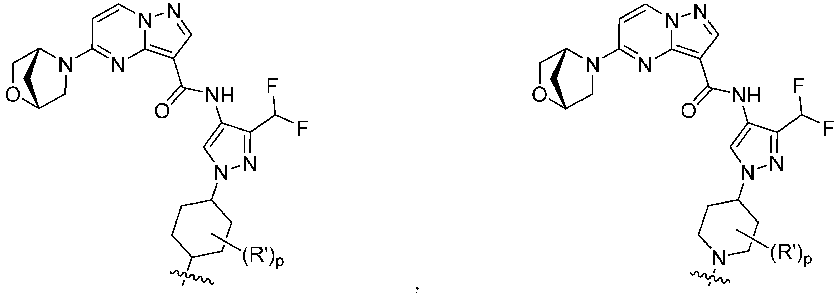 or 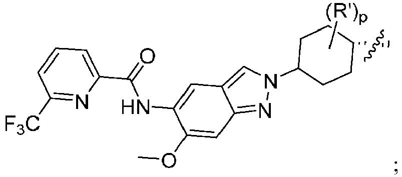
or, the PTM has a structure of the following formula: 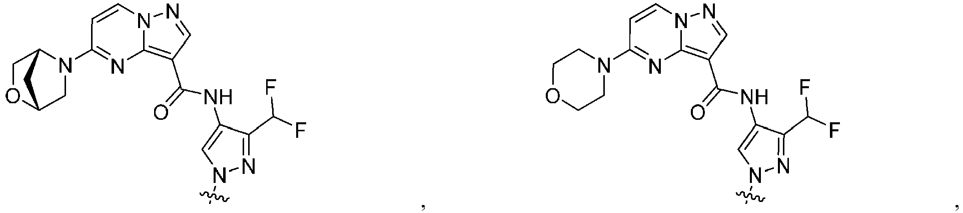 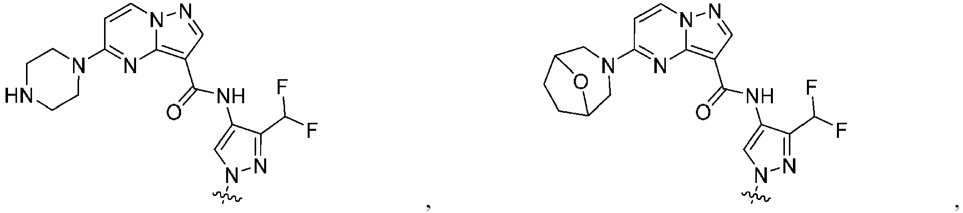 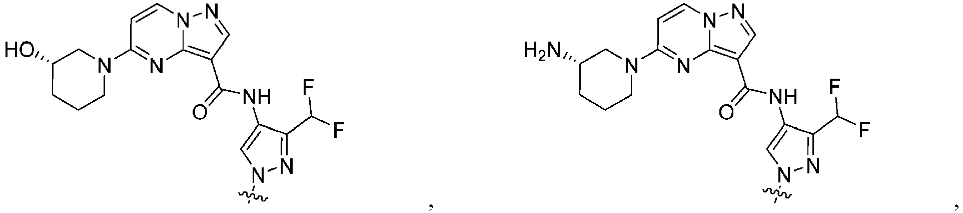 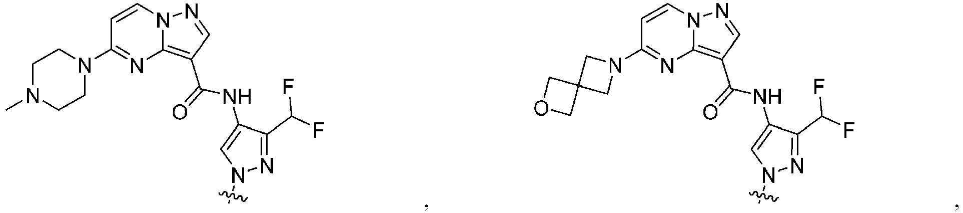 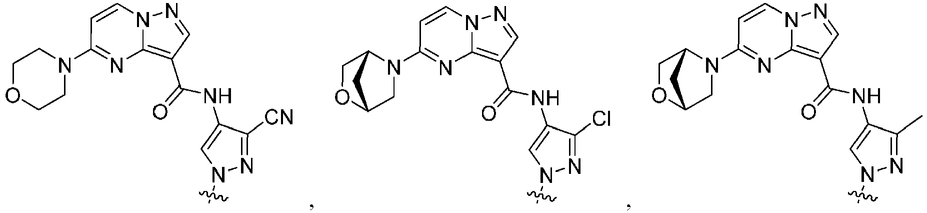 or

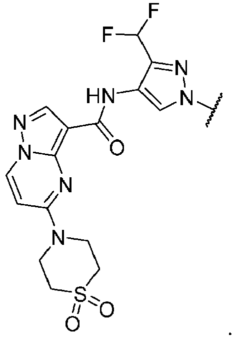

8. The compound of formula I, the stereoisomer, enantiomer, diastereomer, deuteride, hydrate, solvate or prodrug thereof or the pharmaceutically acceptable salt thereof according to claim 1, wherein 1 or 2 of Q₁, Q₂, Q₃, Q₄ and Q₅ in the ULM-1 are N, and the rest are each independently CR₃".

9. The compound of formula I, the stereoisomer, enantiomer, diastereomer, deuteride, hydrate, solvate or prodrug thereof or the pharmaceutically acceptable salt thereof according to claim 1, wherein Q₁, Q₂, Q₃, Q₄ and Q₅ in the ULM-1 are each independently CR₃"; and/or, Y" in the ULM-1 is N;
and/or, each R₁" in the ULM-1 is independently hydrogen, deuterium, -F, -Cl or C₁-C₆ alkyl, wherein the alkyl is optionally substituted with 1-3 halogens; preferably, R₁" is hydrogen;
and/or, R₂" in the ULM-1 is hydrogen or C₁-C₆ alkyl, wherein the alkyl is optionally substituted with 1-3 halogens; preferably, R₂" is hydrogen.

10. The compound of formula I, the stereoisomer, enantiomer, diastereomer, deuteride, hydrate, solvate or prodrug thereof or the pharmaceutically acceptable salt thereof according to claim 1, wherein each R₃" in the ULM-1 is independently hydrogen, deuterium, halogen, -O(C₁-C₆ alkyl) or C₁-C₆ alkyl, wherein the alkyl is optionally substituted with 1-3 halogens; preferably, each R₃" is independently hydrogen, deuterium, F, Cl, methyl, methoxy, ethoxy, trifluoromethoxy, 2-hydroxypropan-2-yl or trifluoromethyl.

11. The compound of formula I, the stereoisomer, enantiomer, diastereomer, deuteride, hydrate, solvate or prodrug thereof or the pharmaceutically acceptable salt thereof according to claim 1, wherein the ULM is selected from 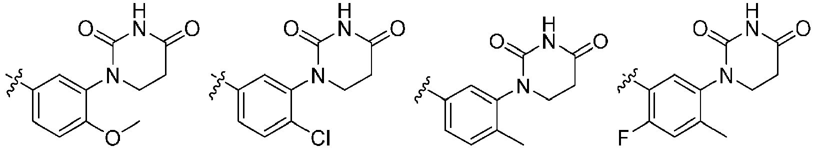 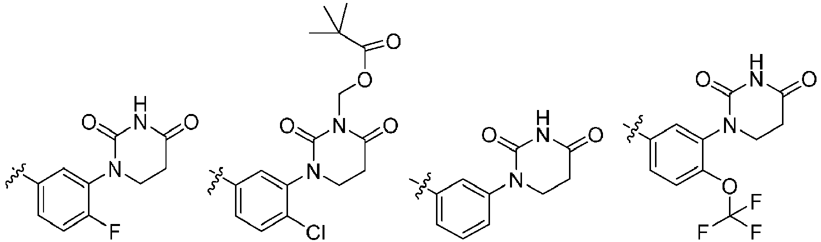 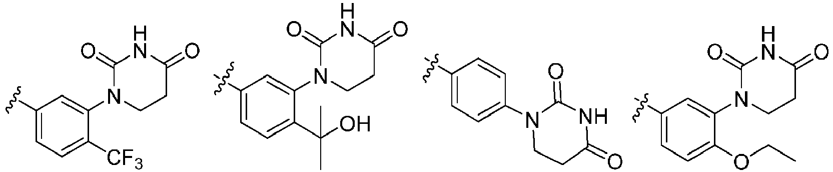 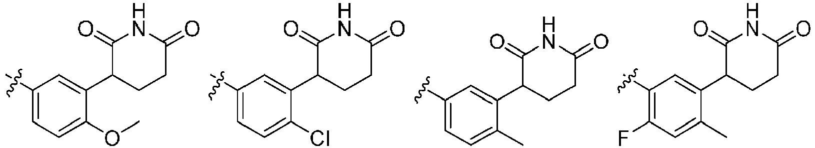 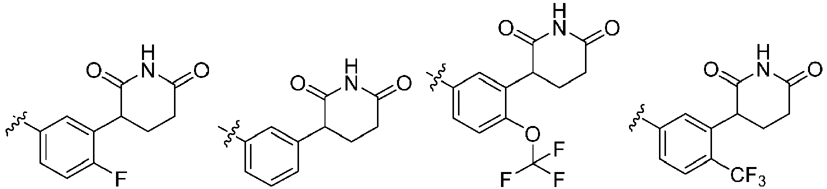 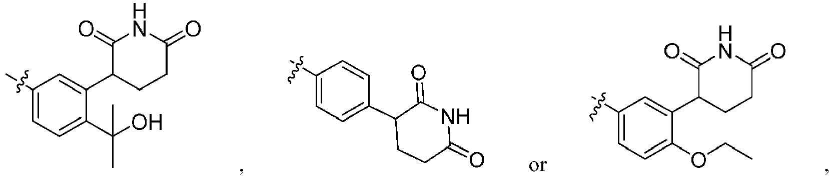 preferably 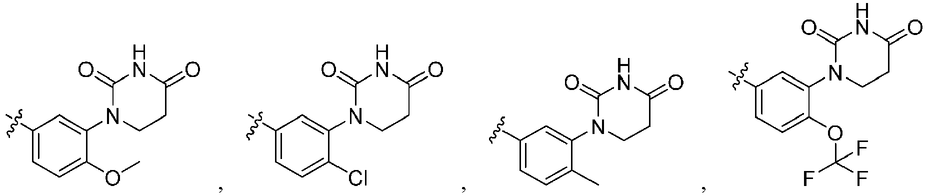 or 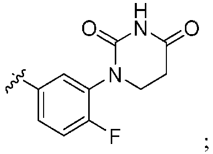 and/or, the L is a bond.

12. The compound of formula I, the stereoisomer, enantiomer, diastereomer, deuteride, hydrate, solvate or prodrug thereof or the pharmaceutically acceptable salt thereof according to claim 1, wherein the L is -(CH₂)ⱼ-, wherein 1 or more methylene groups in the (CH₂)ⱼ- are optionally substituted with substituents selected from -NR^{3'}-, -O-, -S-, -S(O)-, -S(O)NR^{3'}-, -NR^{3'}S(O)-, -S(O)₂-, -S(O)₂NR^{3'}-, -NR^{3'}S(O)₂-, -NR^{4'}S(O)₂NR^{3'}-, - CR^{1'}R^{2'}-, -C(O)-, -C(O)O-, -OC(O)-, -NR^{3'}C(O)O-, -OC(O)NR^{3'}-, -C(O)NR^{3'}-, -NR^{3'}C(O)-, -NR^{4'}C(O)NR^{3'}-, - P(O)-, -P(O)O-, -OP(O)-, -OP(O)O-, ethenylene, ethynylene, C₃-C₁₂ cycloalkylene, 3-12 membered heterocycloalkylene containing 1 or more heteroatoms selected from N, O or S, 6-10 membered arylene or 5-10 membered heteroarylene, wherein the ethenylene, cycloalkylene, heterocycloalkylene, arylene and heteroarylene are each independently optionally substituted with 1 or more substituents selected from halogen, -OR^{3'}, -NR^{3'}R^{4'}, oxo, nitro, cyano, C₁-C₆ alkyl, -S(C₁-C₆ alkyl), C₃-C₁₀ cycloalkyl, 3-10 membered heterocycloalkyl, 6-10 membered aryl, 5-10 membered heteroaryl, -C(O)R^{1'}, -C(O)OR^{3'}, -OC(O)R^{1'}, - C(O)NR^{3'}, -NR^{3'}C(O)R^{1'}, -S(O)R^{1'}, -S(O)NR^{3'}, -S(O)₂R^{1'}, -S(O)₂NR^{3'}, -NR^{3'}S(O)₂R^{1'}, -NR^{4'}S(O)₂NR^{3'}, - OC(O)NR^{3'} and -NR^{4'}C(O)NR^{3'}, wherein the alkyl, cycloalkyl, heterocycloalkyl, aryl and heteroaryl are each independently optionally substituted with 1 or more substituents selected from halogen, -OH, -NR^{3'}R^{4'}, oxo, nitro, cyano and C₁-C₆ alkyl, C₃-C₁₀ cycloalkyl, 3-10 membered heterocycloalkyl, 6-10 membered aryl and 5-10 membered heteroaryl; R^{1'} and R^{2'} are each independently halogen, -OH, -NR^{3'}R^{4'}, C₁-C₆ alkyl, C₁-C₆ chloroalkyl, hydroxy C₁-C₆ alkyl, -O(C₁-C₆ alkyl), -NH(C₁-C₆ alkyl), -NH(C₁-C₆ alkyl), C₃-C₁₀ cycloalkyl, - O(C₃-C₁₀ cycloalkyl), -NH(C₃-C₁₀ cycloalkyl), 3-10 membered heterocycloalkyl, -0(3-10 membered heterocycloalkyl), -NH(3-10 membered heterocycloalkyl), 6-10 membered aryl, -0(6-10 membered aryl), - NH(6-10 membered aryl), 5-10 membered heteroaryl, -0(5-10 membered heteroaryl) or -NH(5-10 membered heteroaryl); R^{3'} and R^{4'} are each independently hydrogen, deuterium, C₁-C₆ alkyl, C₃-C₁₀ cycloalkyl, 3-10 membered heterocycloalkyl, 6-10 membered aryl or 5-10 membered heteroaryl; j is 1, 2, 3, 4, 5, 6, 7, 8, 9, 10, 11, 12, 13, 14, 15, 16, 17, 18, 19, 20, 21, 22, 23, 24 or 25.

13. The compound of formula I, and/or the stereoisomer, enantiomer, diastereomer, deuteride, hydrate, solvate and/or prodrug thereof and/or the pharmaceutically acceptable salt thereof according to claim 12, wherein the L is -(CH₂)ⱼ-, wherein 1, 2, 3 or 4 methylene groups in the -(CH₂)ⱼ- are optionally replaced by groups selected from -NH-, -NCH₃-, -NCH₂CH₃-, -O-, -C(CH₃)₂-, -CHF-, -CHCF₃-, -C(O)-, -C(O)O-, -OC(O)-, -C(O)NH-, - C(O)NCH₃-, -NHC(O)-, -NCH₃C(O)-, ethenylene, ethynylene, cyclopropylene, cyclobutylene, cyclopentylene, cyclohexylene, oxiranylidene, oxetanylidene, oxolanylidene, oxanylidene, aziridinylidene, azetidinylidene, azolidinylidene, piperidinylidene, piperazinylidene, morpholinylidene, homomorpholinylidene, phenylene, pyrrolylidene, thienylidene, furylidene, imidazolylidene, pyrazolylidene, triazolylidene, tetrazolylidene, oxazolylidene, isoxazolylidene, thiazolylidene, isothiazolylidene, pyridinylidene, pyrimidinylidene, pyridazinylidene, pyrazinylidene, 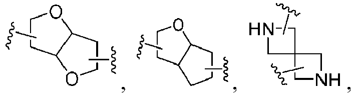 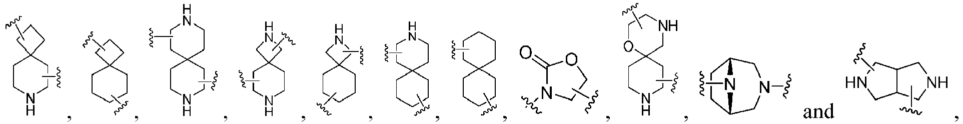 wherein the group for replacement is optionally substituted with 1 or more substituents selected from halogen, oxo, -NR^{3'}R^{4'}, -OR^{3'} and C₁-C₄ alkyl, wherein the alkyl is optionally substituted with 1 or more substituents selected from halogen, -OH and -NH₂; R^{3'} and R^{4'} are each independently hydrogen, deuterium or C₁-C₄ alkyl; j is 2, 3, 4, 5, 6, 7 or 8.

14. The compound of formula I, the stereoisomer, enantiomer, diastereomer, deuteride, hydrate, solvate and/or prodrug thereof and/or the pharmaceutically acceptable salt thereof according to claim 12, wherein the L is - (CH₂)ⱼ-, wherein 1, 2 or 3 methylene groups in the -(CH₂)ⱼ- are optionally replaced by groups selected from - O-, -NH-, -NCH₃-, -NCH₂CH₃-, -C(O)-, -C(O)NH-, -NHC(O)-, -NCH₃C(O)-, -C(O)NCH₃-, 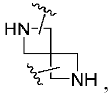 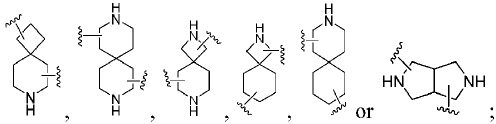 j is 2, 3, 4, 5, 6, 7 or 8.

15. The compound of formula I, the stereoisomer, enantiomer, diastereomer, deuteride, hydrate, solvate and/or prodrug thereof and/or the pharmaceutically acceptable salt thereof according to claim 12, wherein the L is - (CH₂)ⱼ₋₁-C(O)-, wherein the methylene group in the -(CH₂)ⱼ₋₁-C(O)- is as defined in claim 11 and optionally substituted with 1 or more groups, and j is as defined in claim 20.

16. The compound of formula I, the stereoisomer, enantiomer, diastereomer, deuteride, hydrate, solvate and/or prodrug thereof and/or the pharmaceutically acceptable salt thereof according to claim 12, wherein the L is 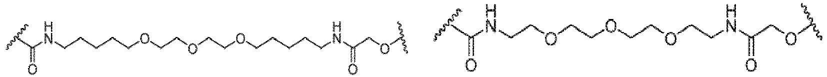 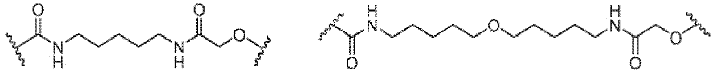 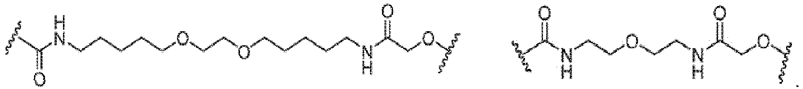 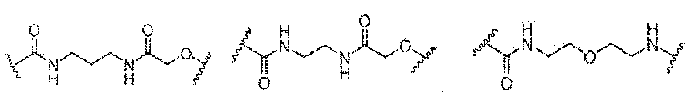 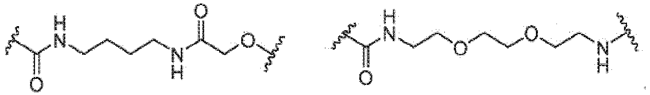 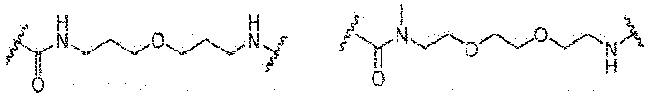 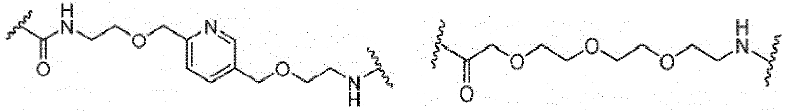 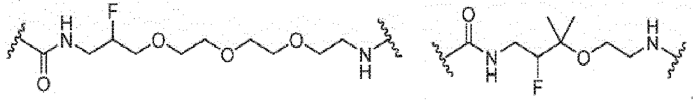 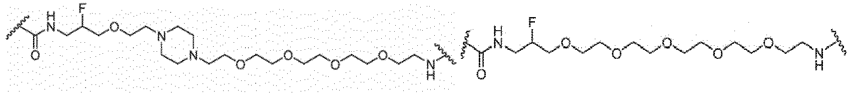 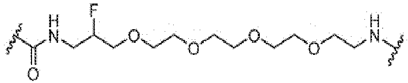 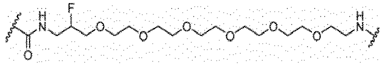 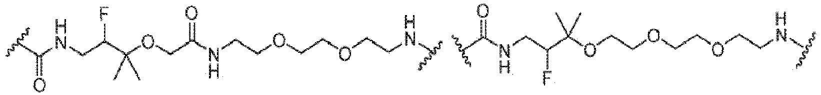 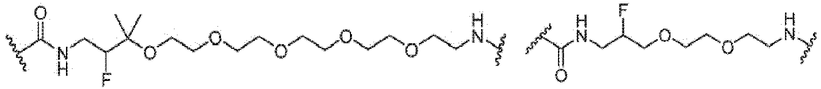 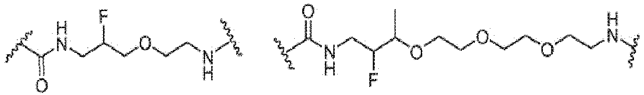 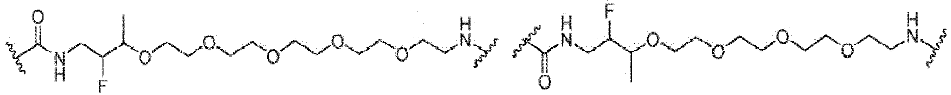 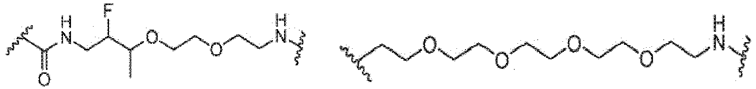 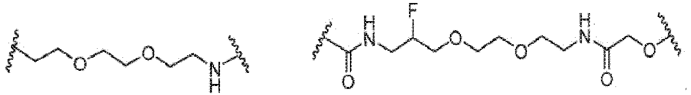 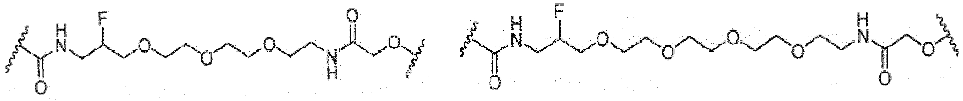 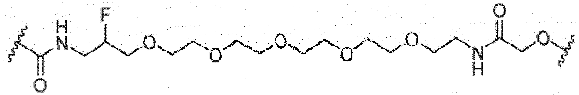 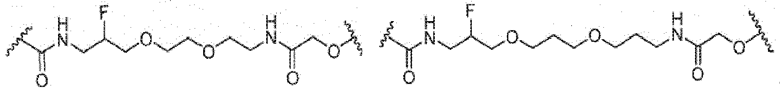 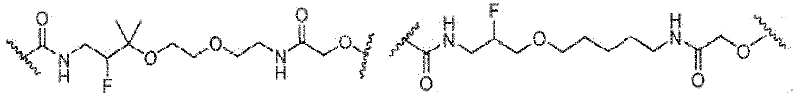 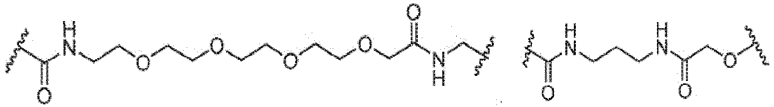 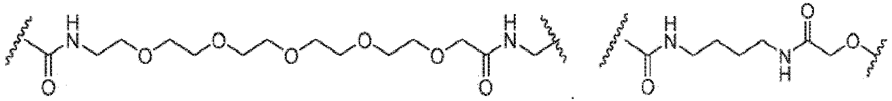 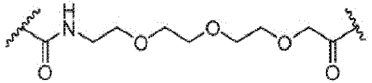 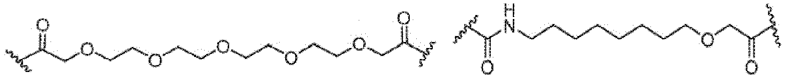 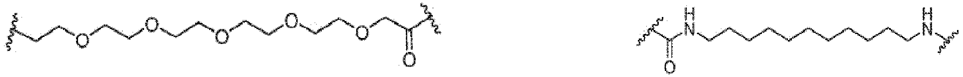 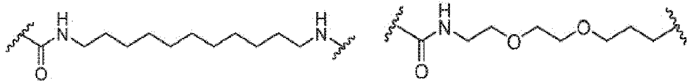 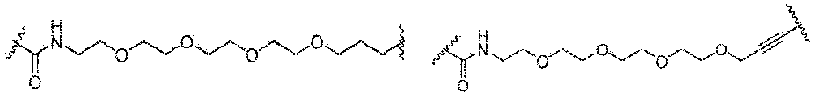 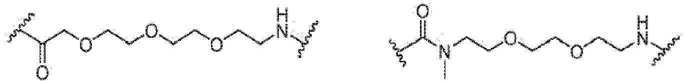 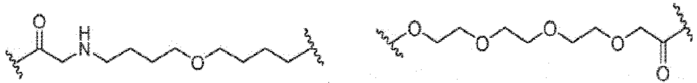 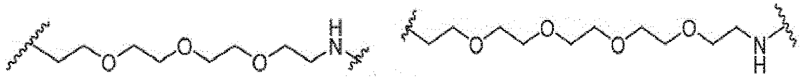 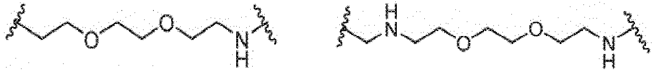 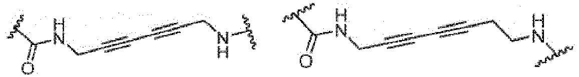 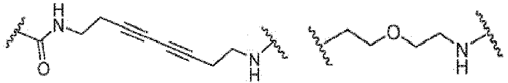 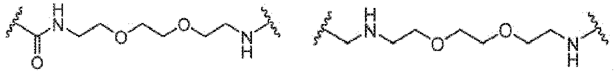 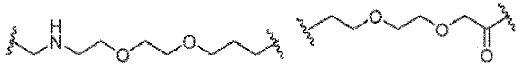 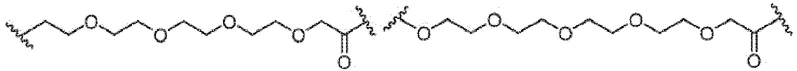 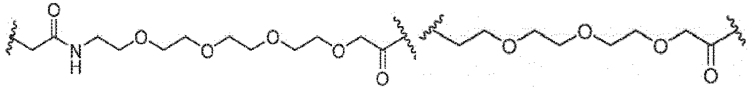 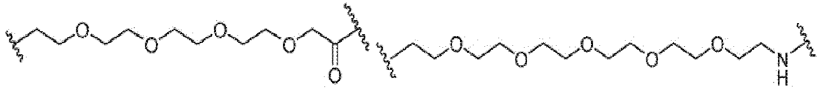 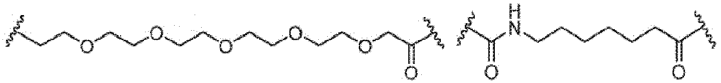 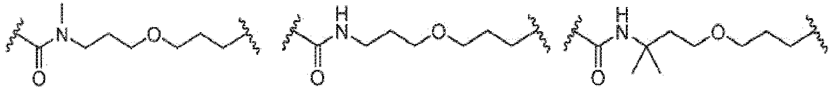 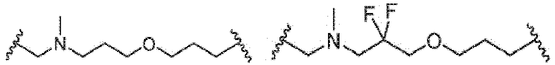 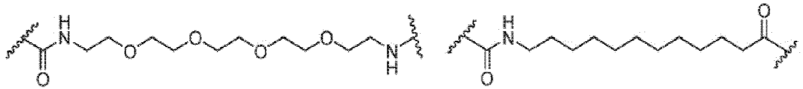 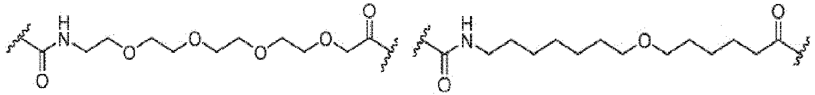 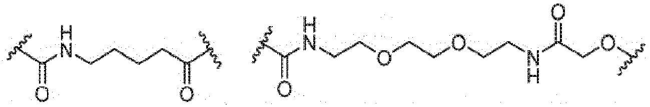 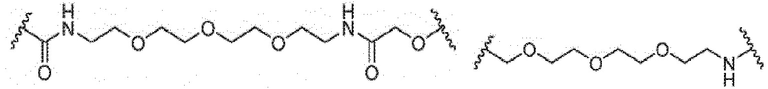 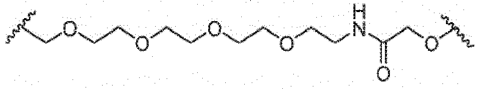 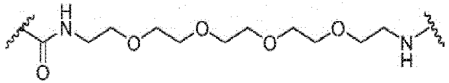 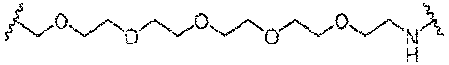 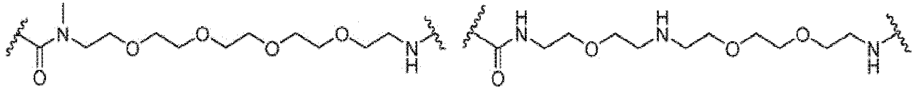 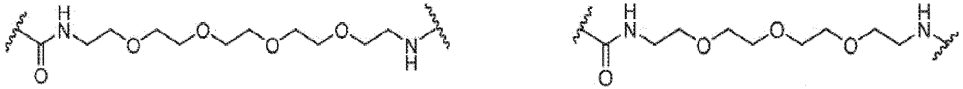 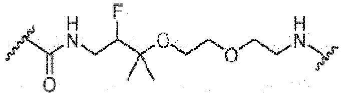 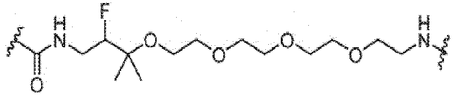 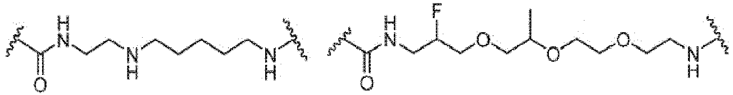 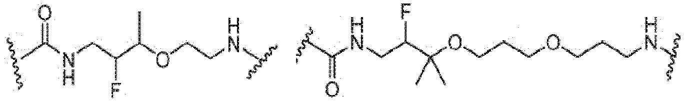 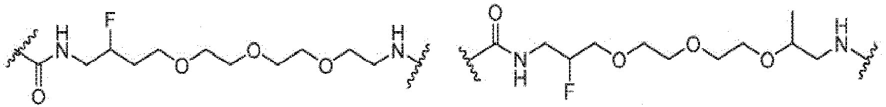 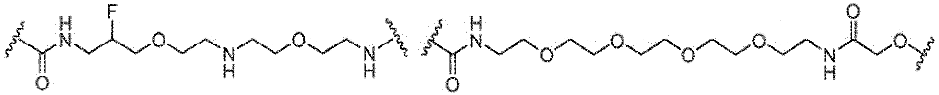 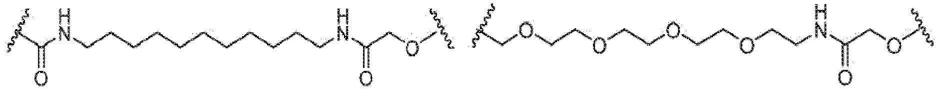 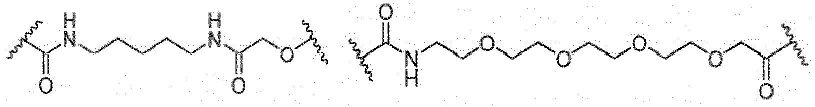 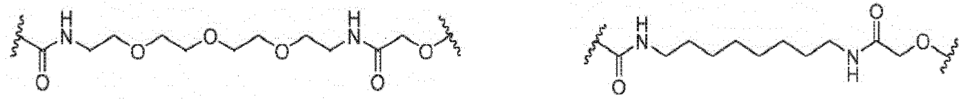 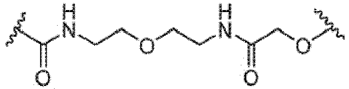 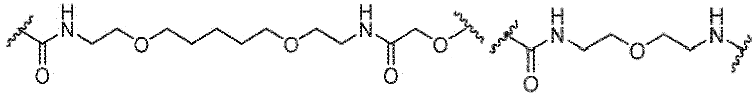 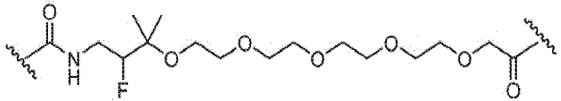 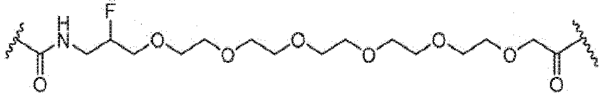 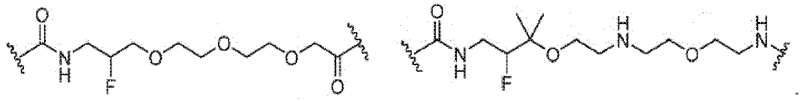 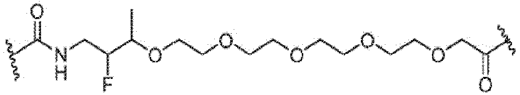 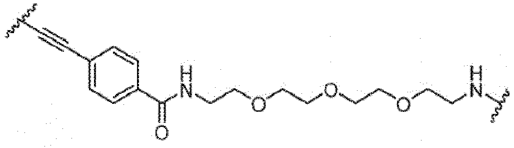 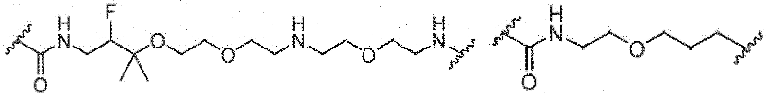 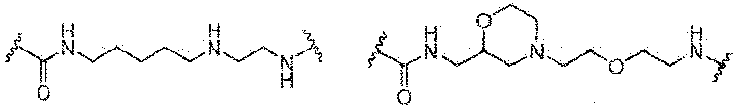 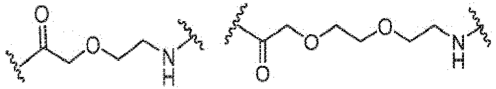 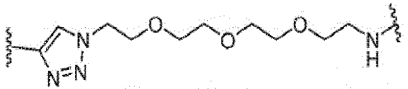 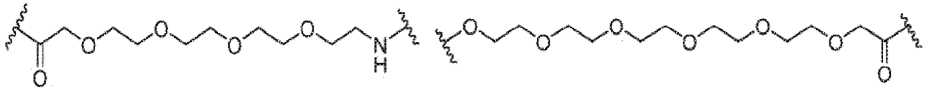 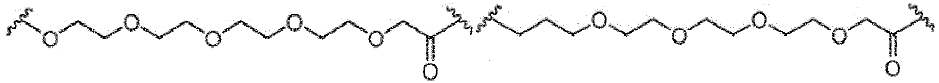 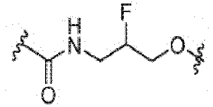 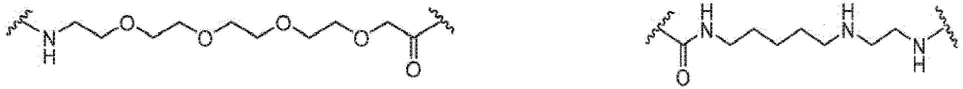 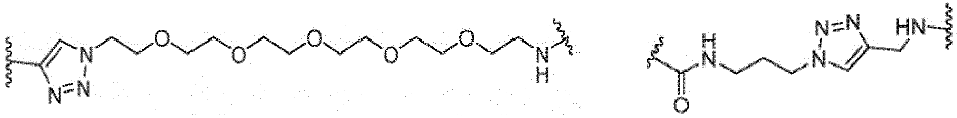 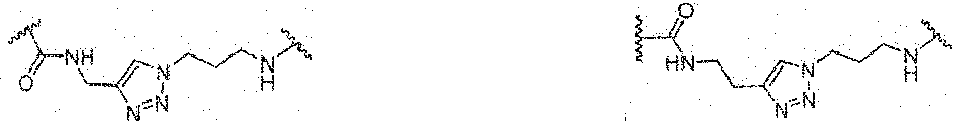 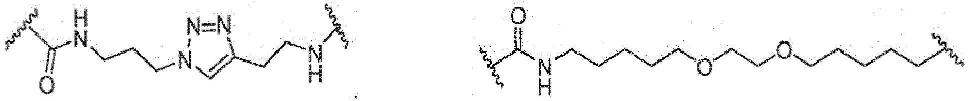 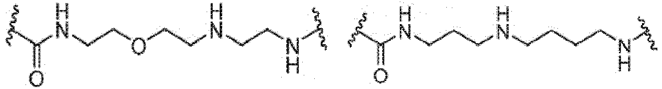 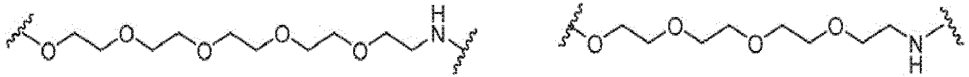 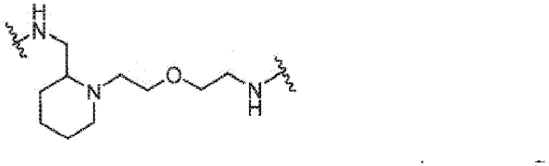 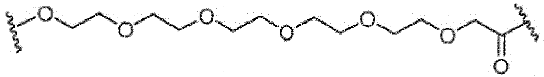 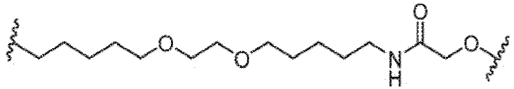 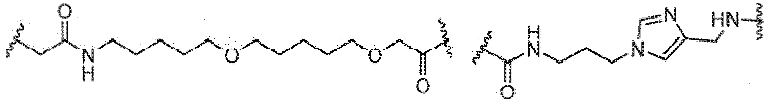 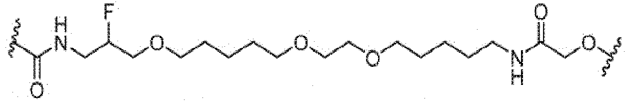 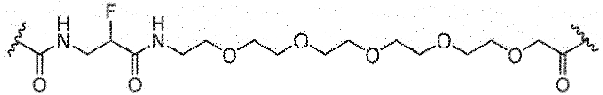 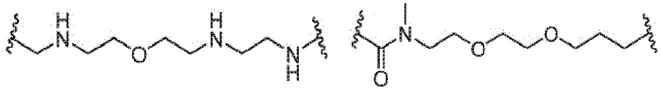 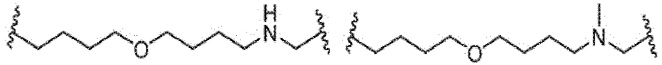 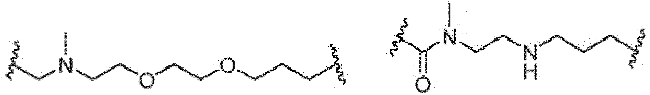 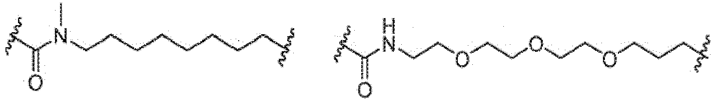 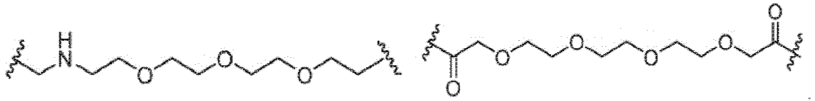 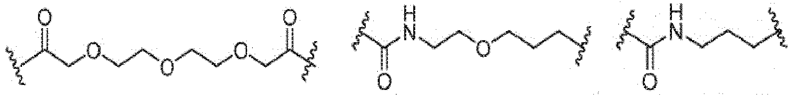 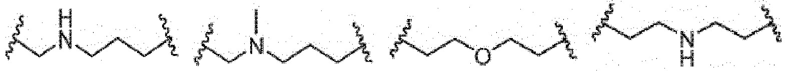 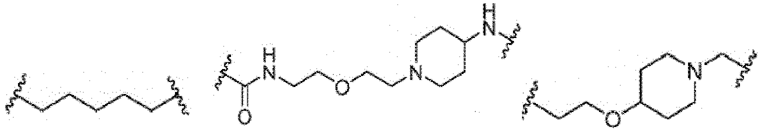 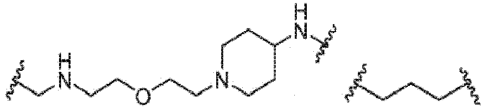 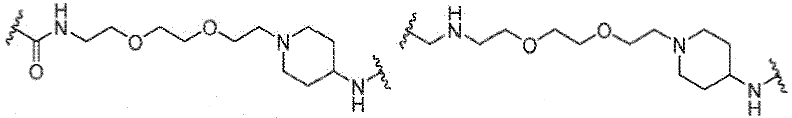 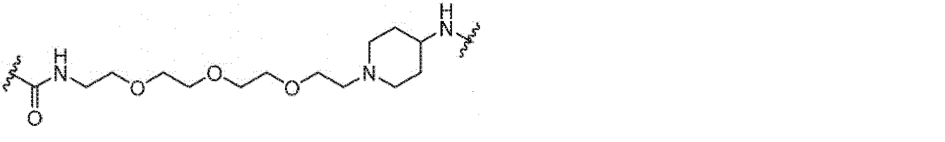 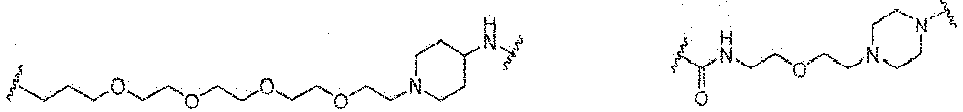 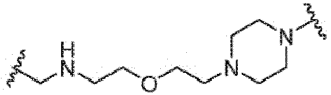 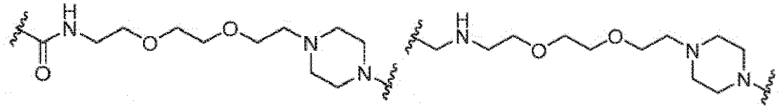 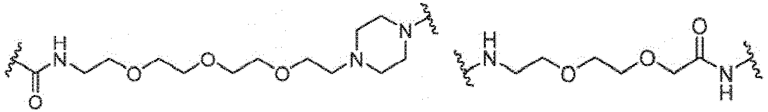 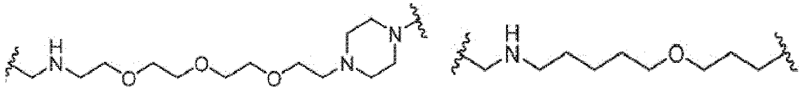 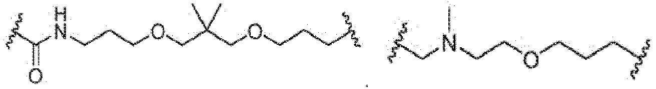 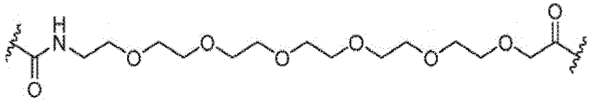 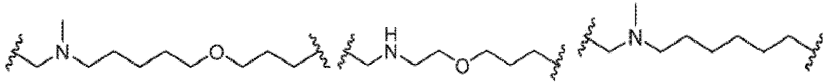 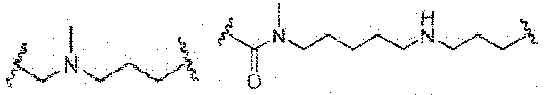 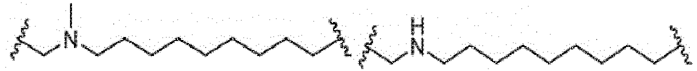 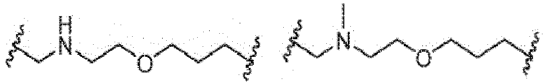 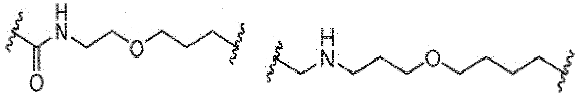 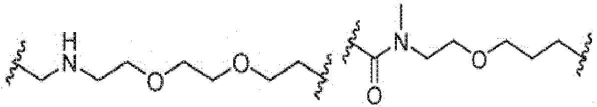 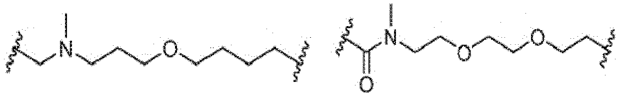 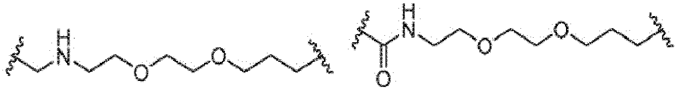 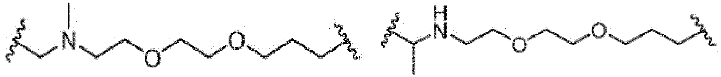 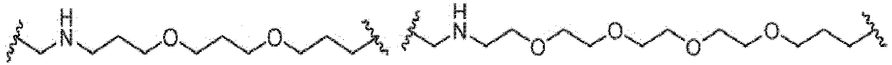 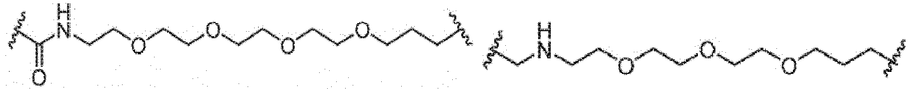 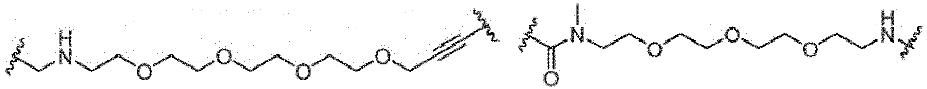 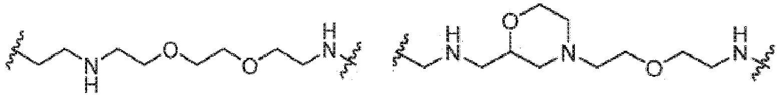 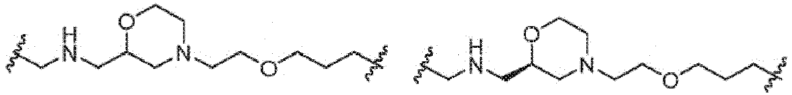 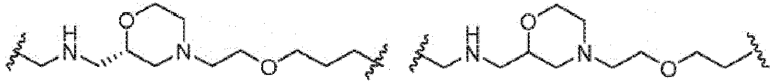 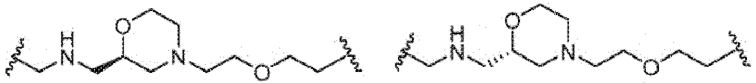 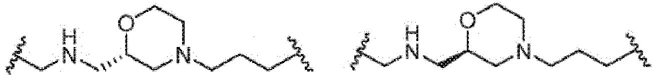 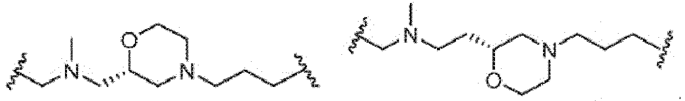 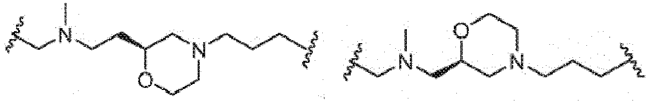 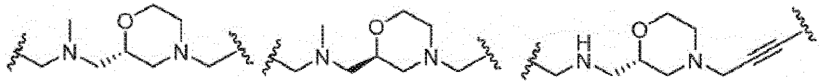 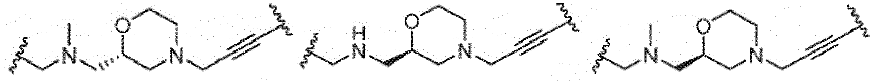 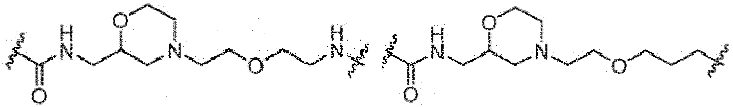 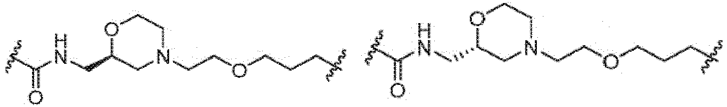 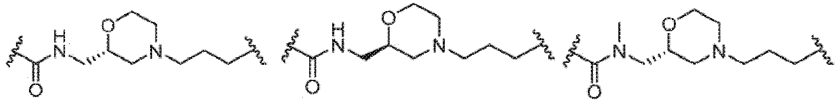 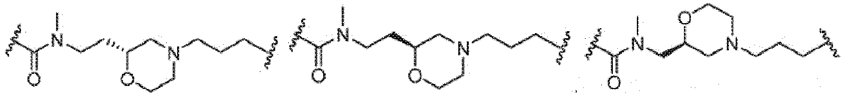 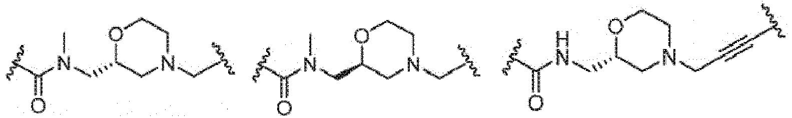 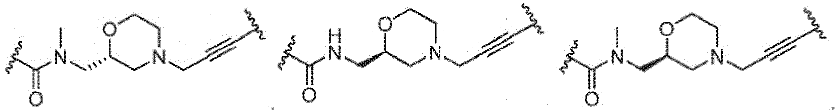 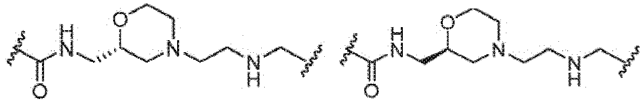 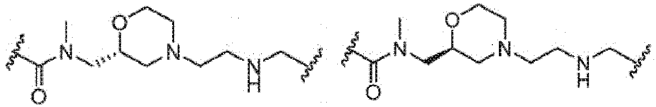 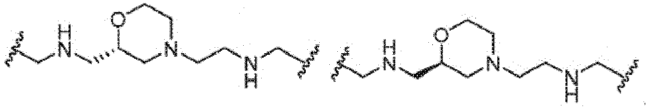 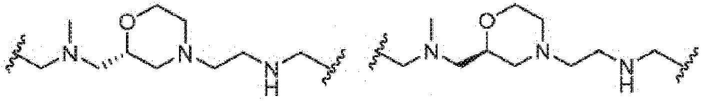 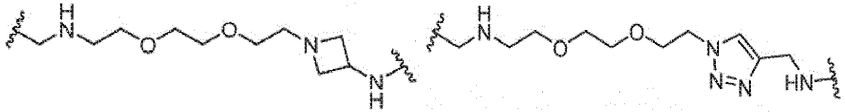 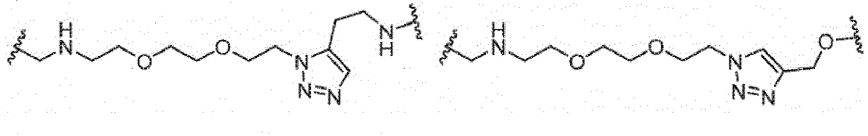 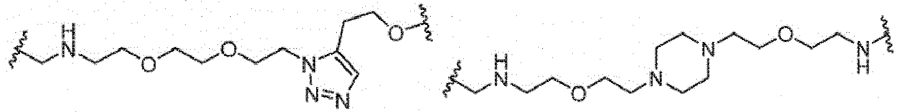 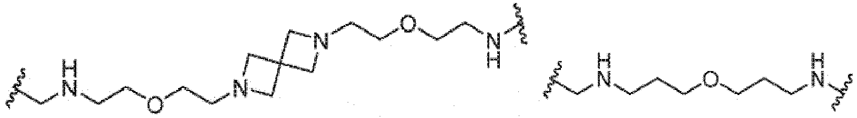 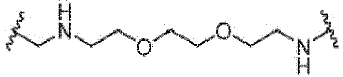 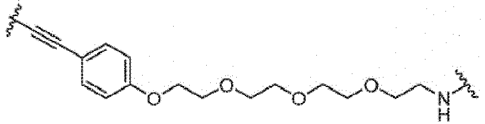 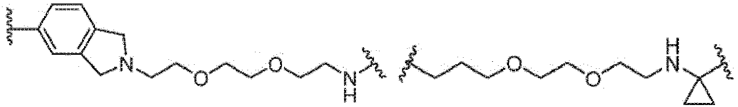 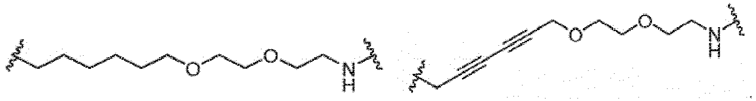 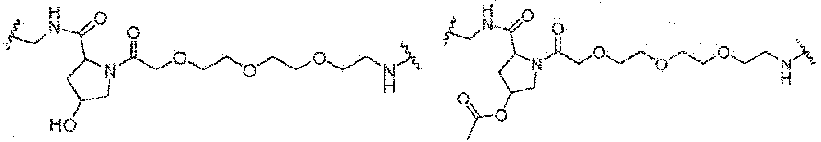 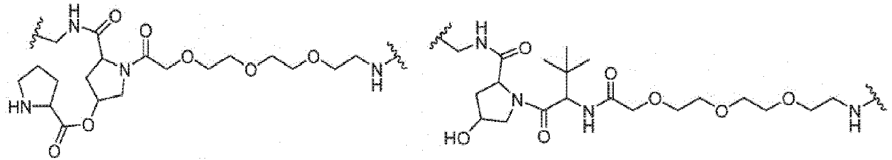 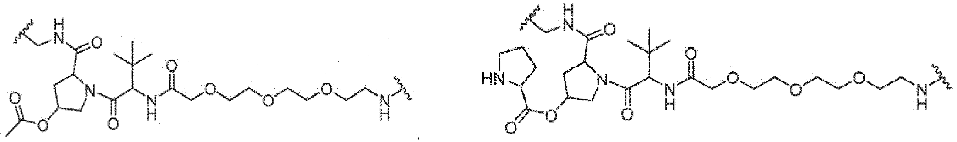 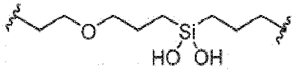 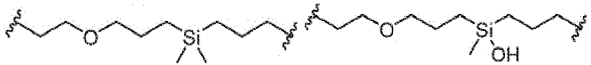 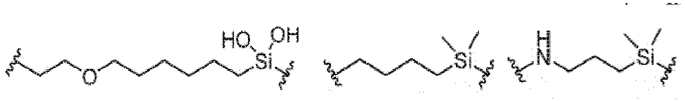 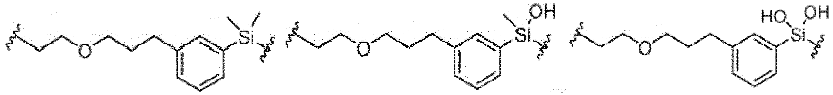 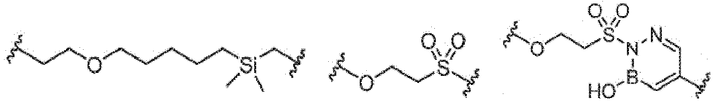 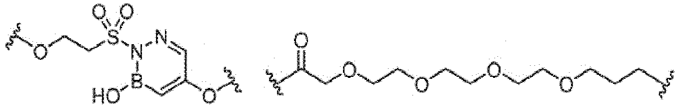 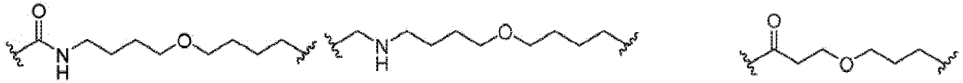 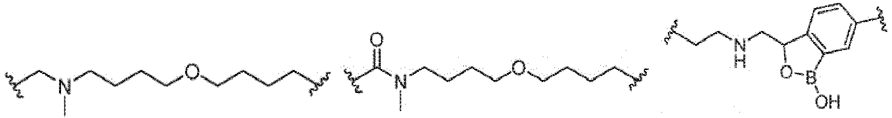 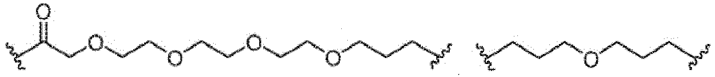 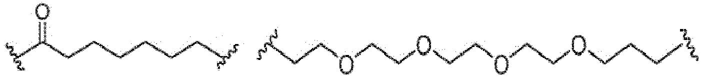 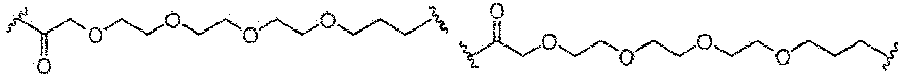 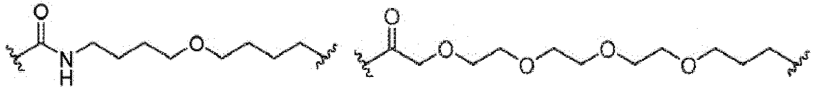 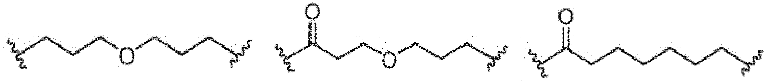 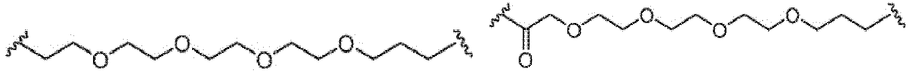 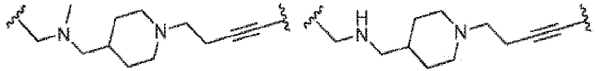 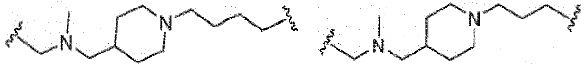 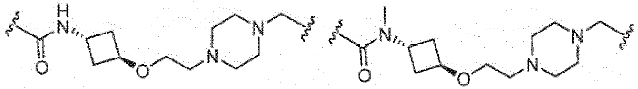 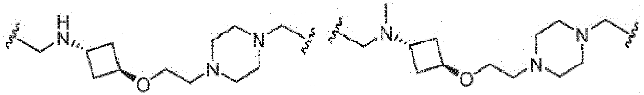 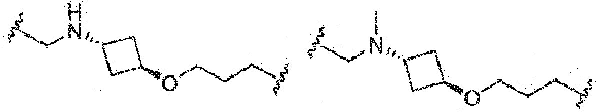 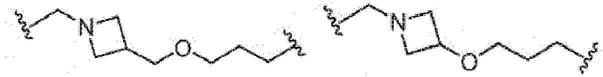 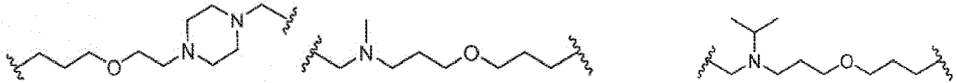 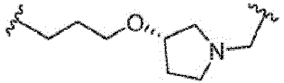 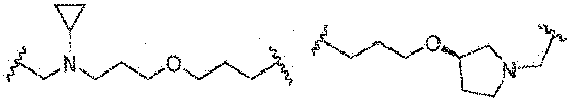 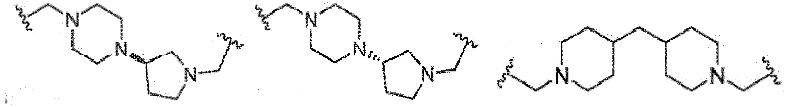 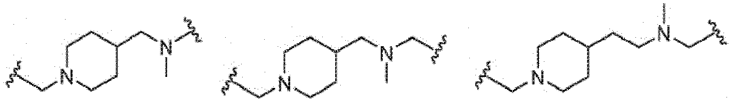 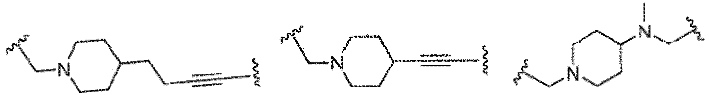 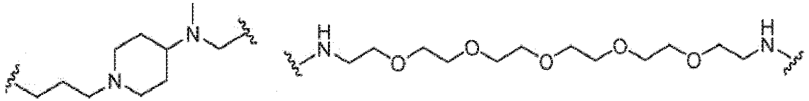 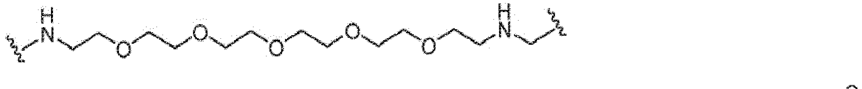 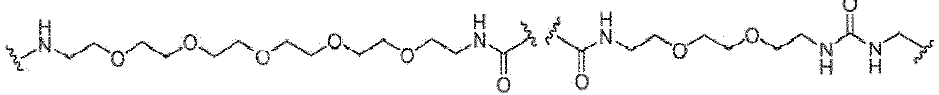 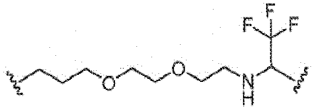 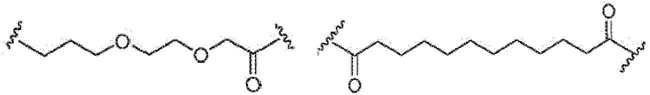 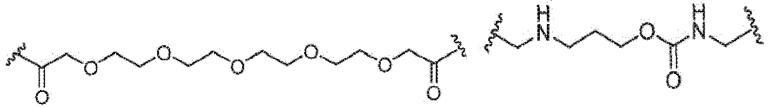 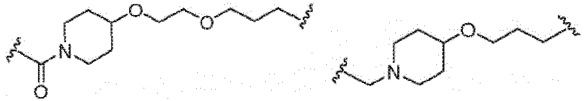 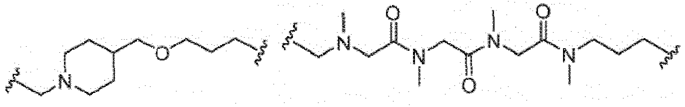 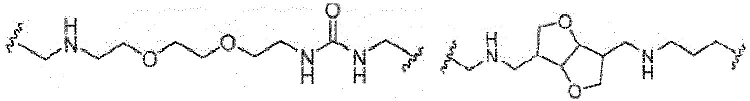 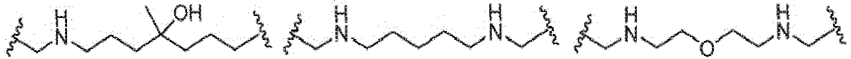 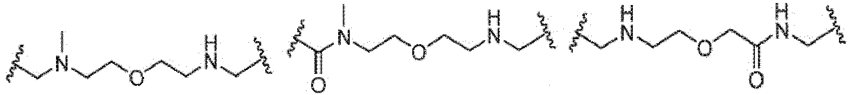 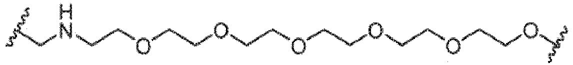 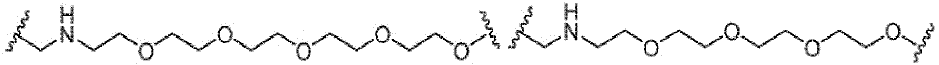 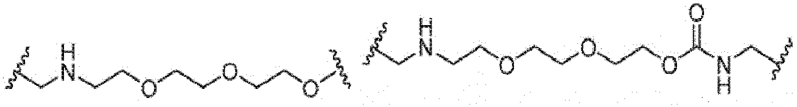 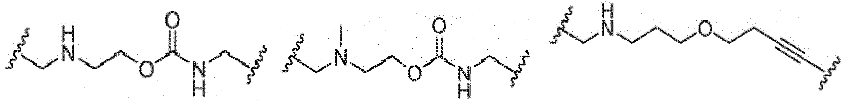 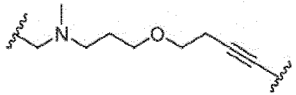 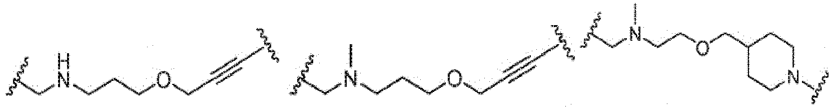 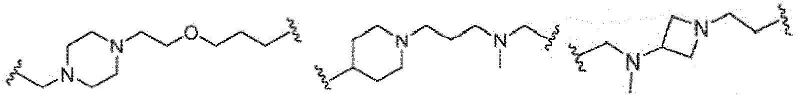 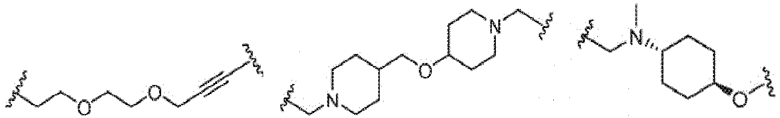 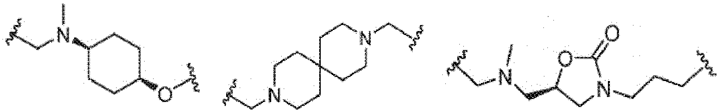 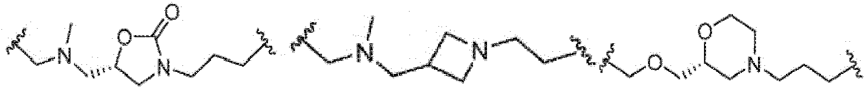 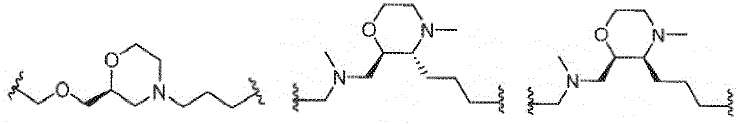 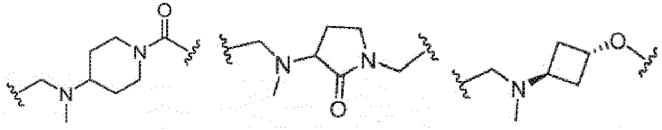 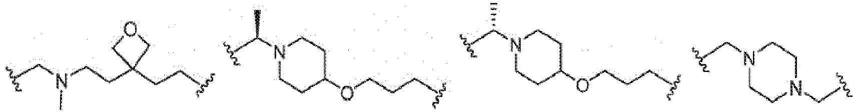 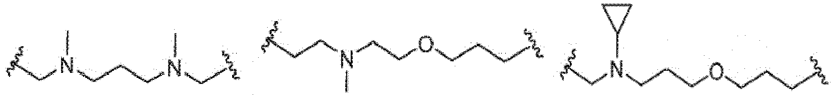 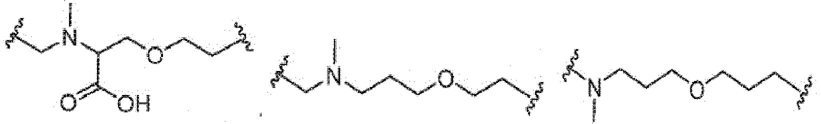 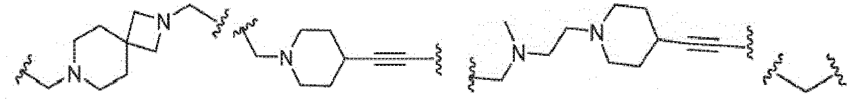 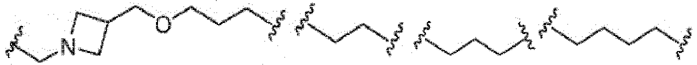 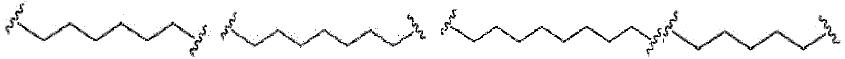 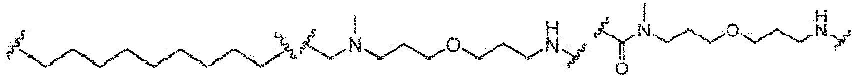 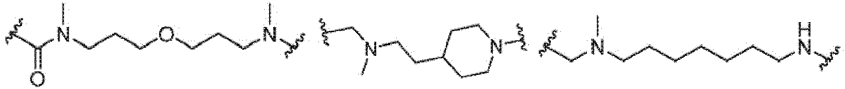 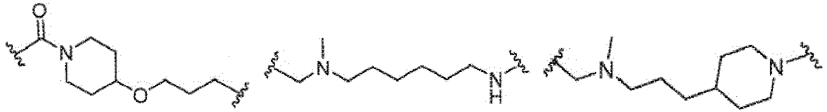 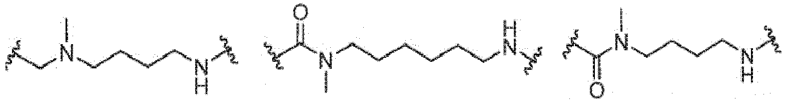 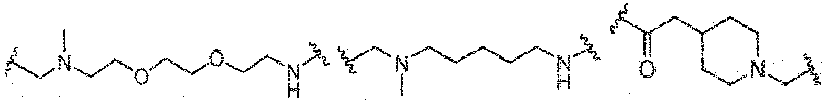 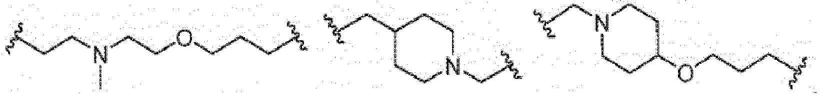 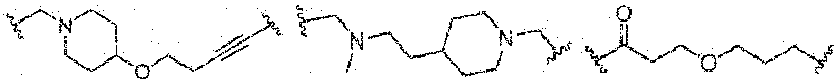 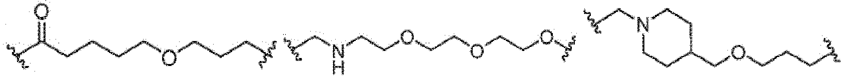 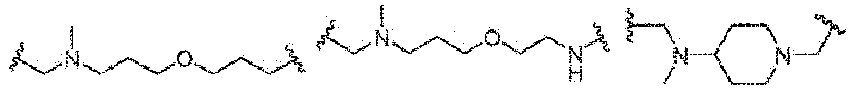 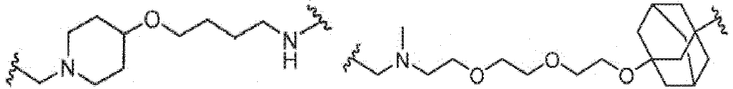 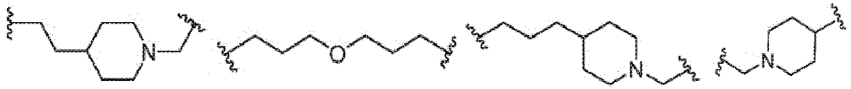 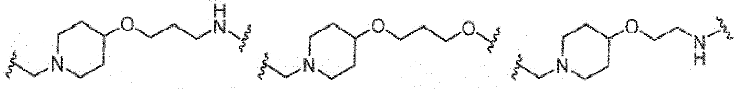 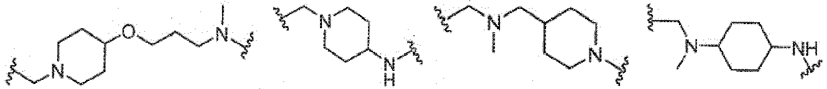 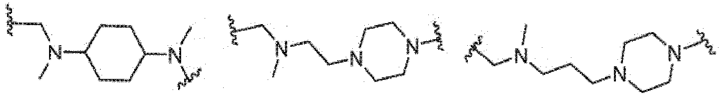 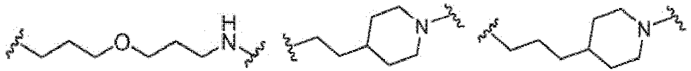 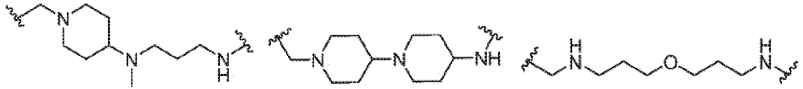 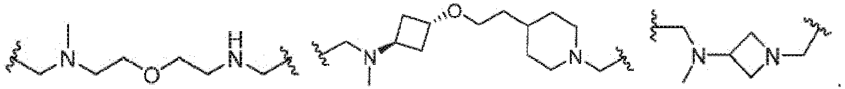 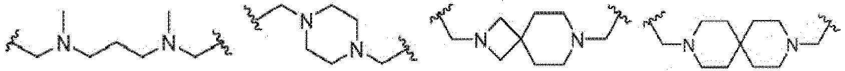 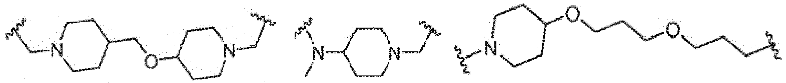 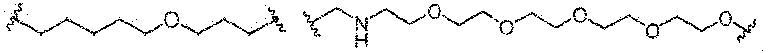 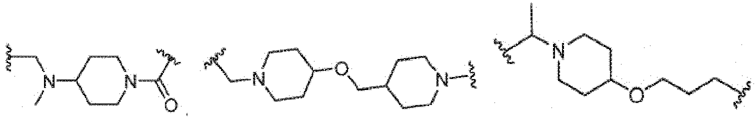 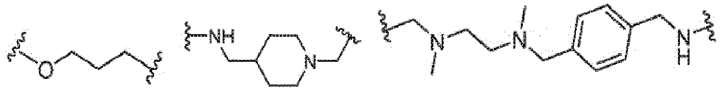 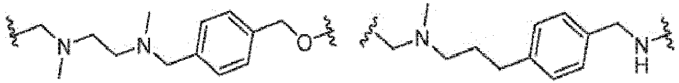 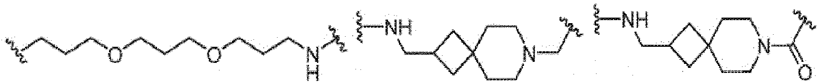 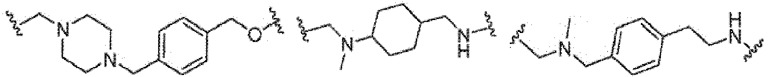 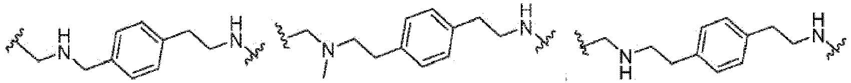 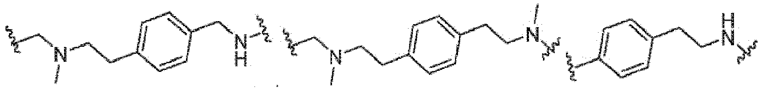 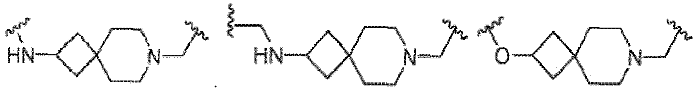 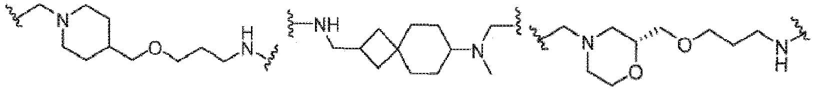 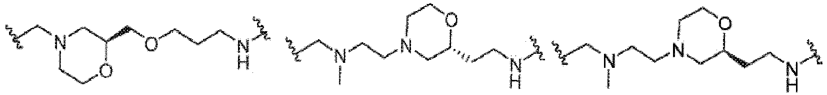 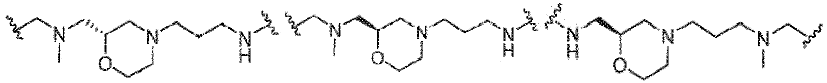 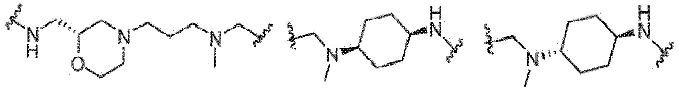 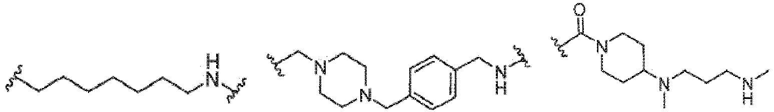 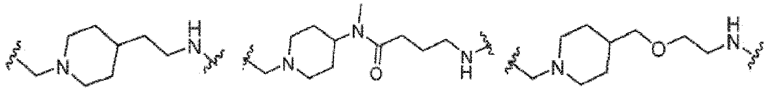 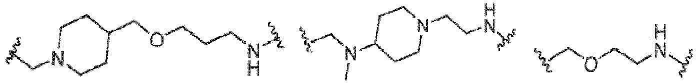 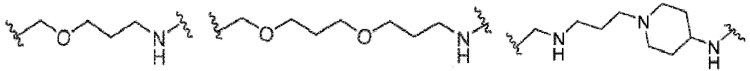 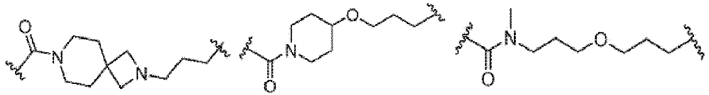 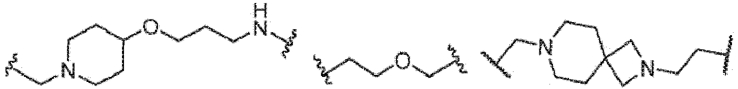 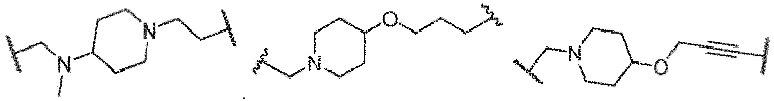 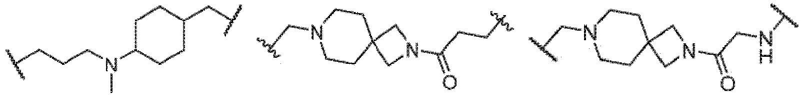 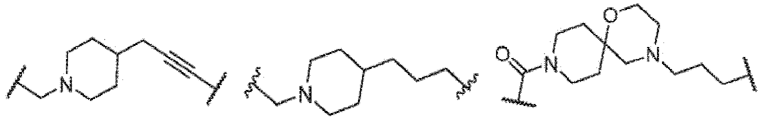 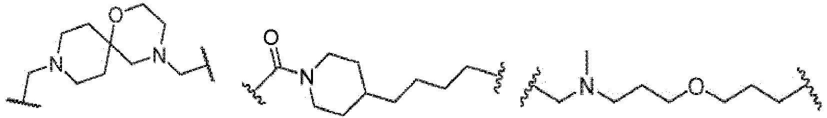 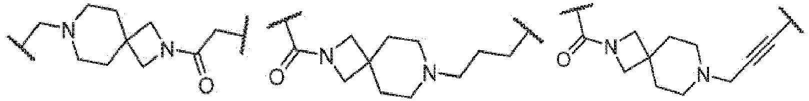 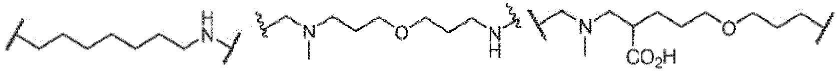 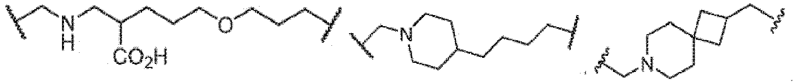 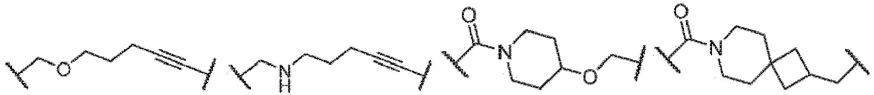 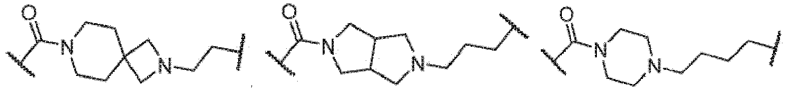 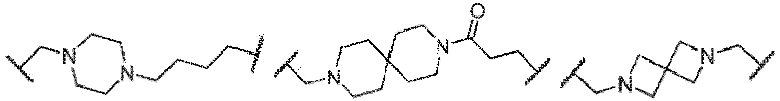 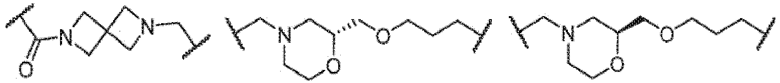 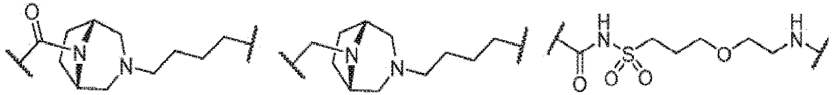 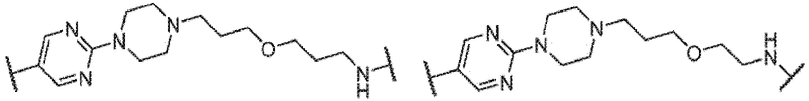 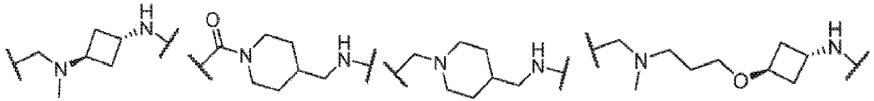 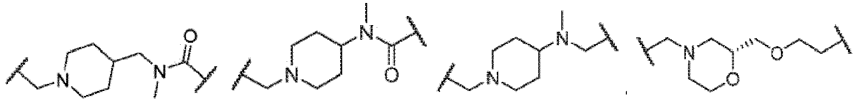 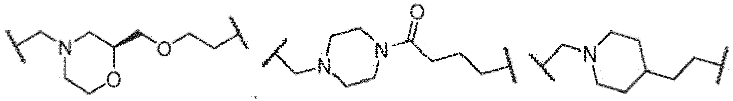 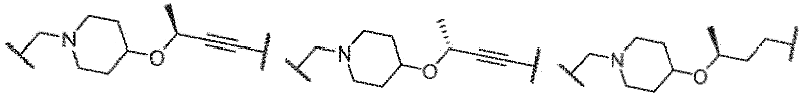 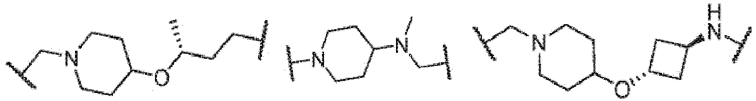 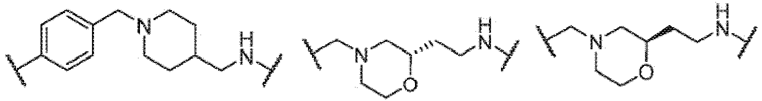 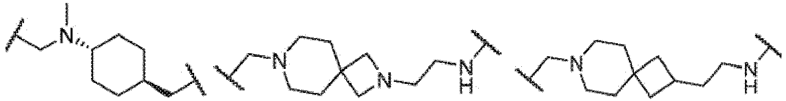 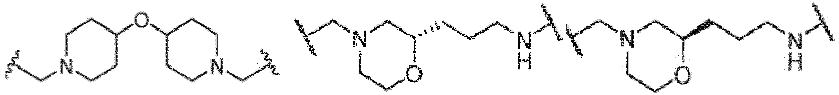 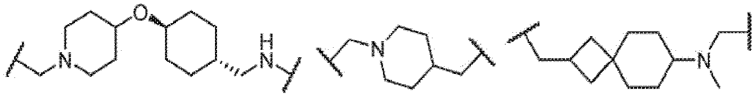 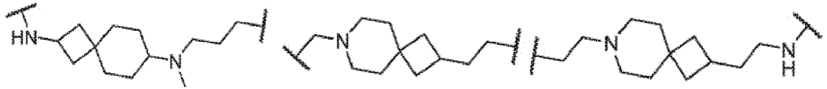 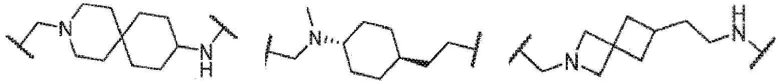 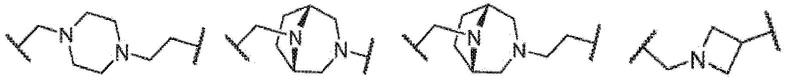 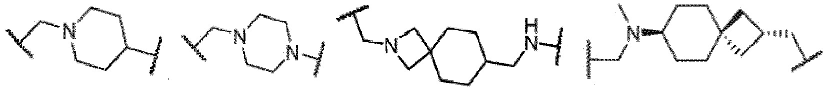 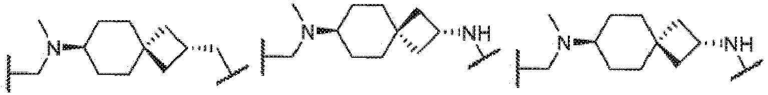 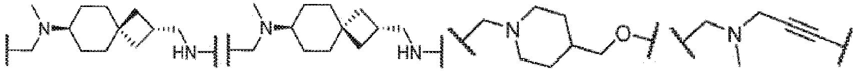 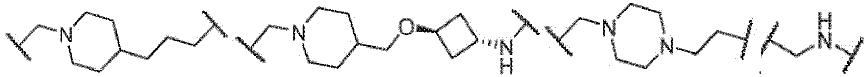 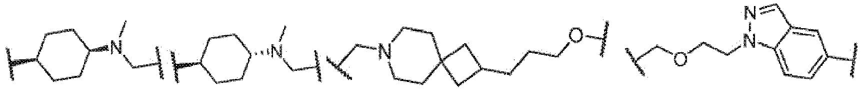 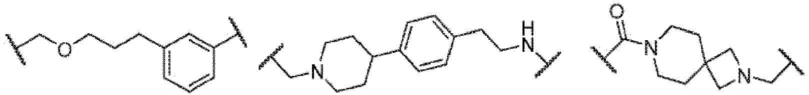 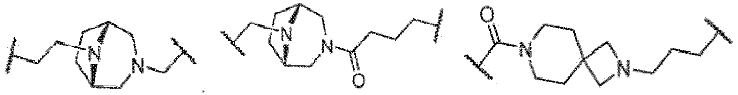 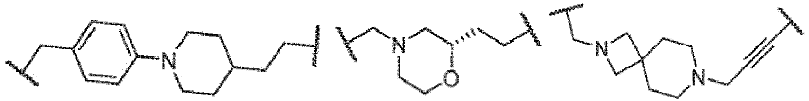 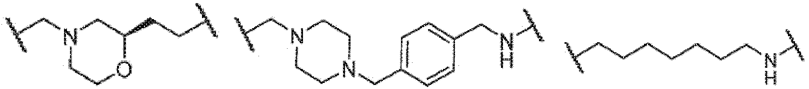 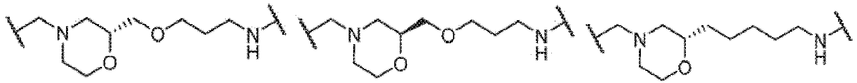 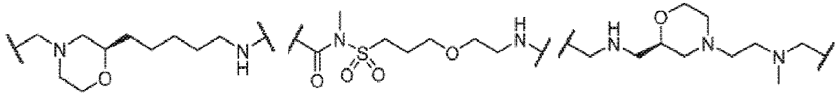 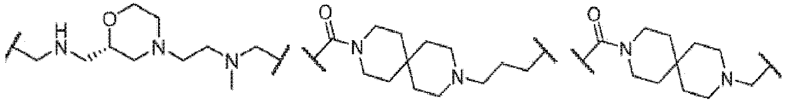 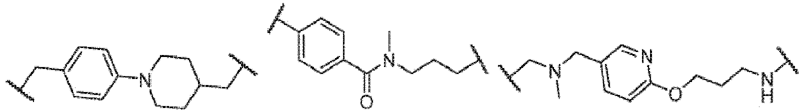 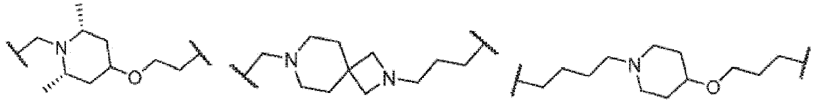 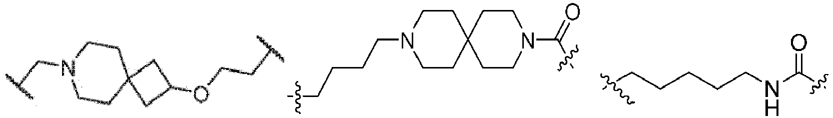 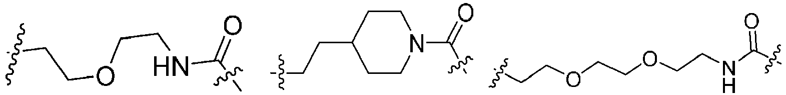 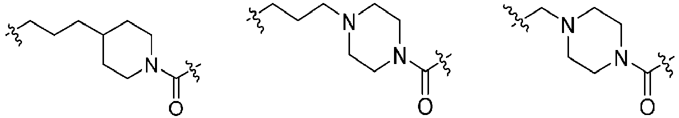 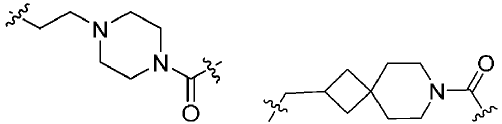 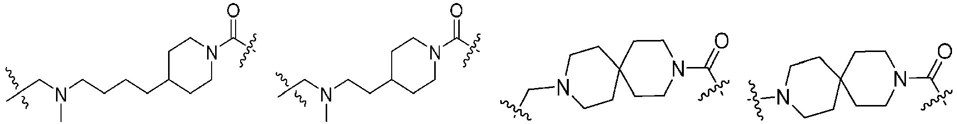 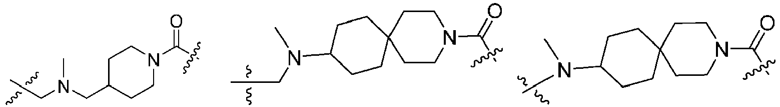 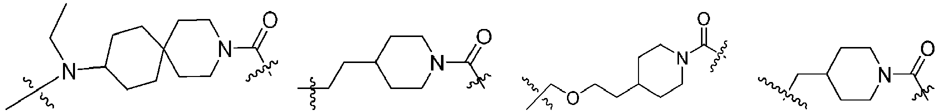 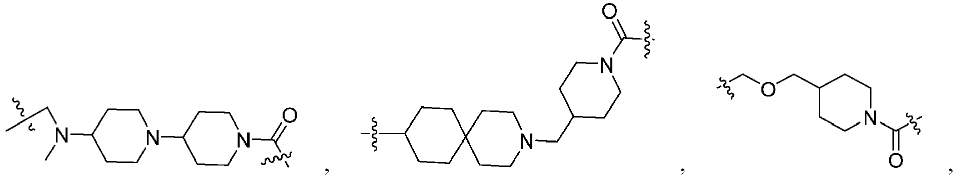 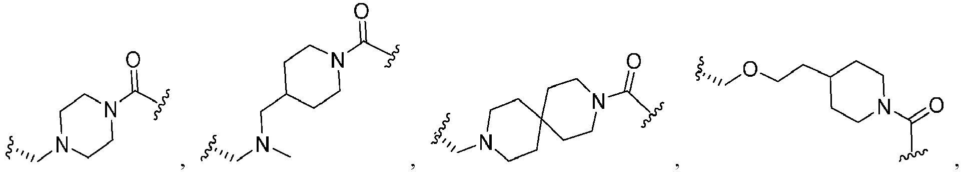 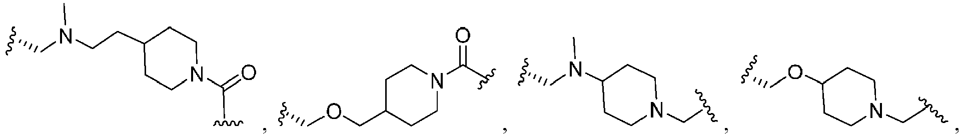 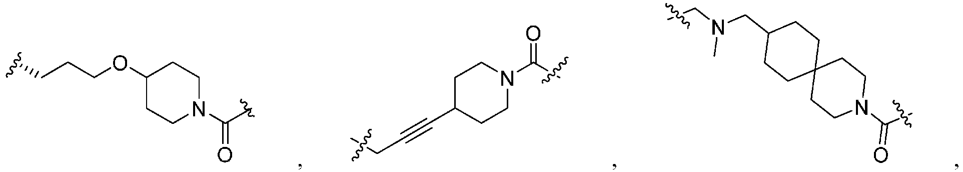 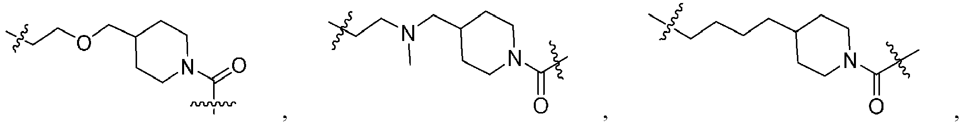 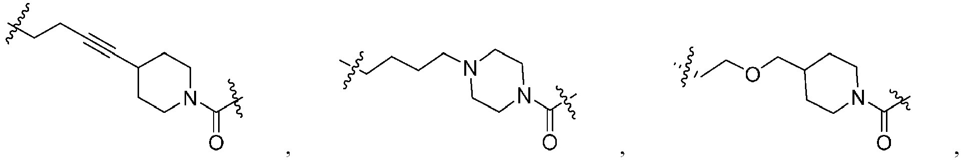 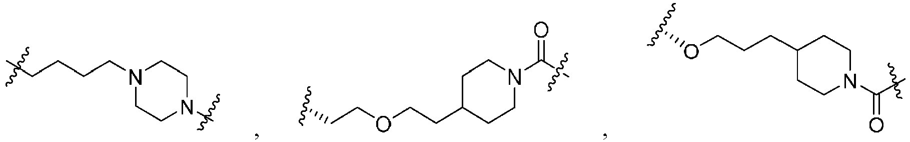 or

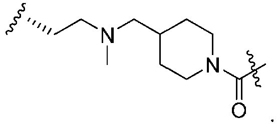

17. The compound of formula I, the stereoisomer, enantiomer, diastereomer, deuteride, hydrate, solvate or prodrug thereof or the pharmaceutically acceptable salt thereof according to claim 12, wherein the L is LA, 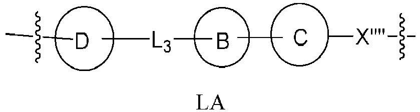 wherein in the LA:
ring D is absent, 6-10 membered aryl or 5-10 membered heteroaryl or is C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
ring B is absent or is C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
ring C is absent, C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
X"" is a bond, -NH-, -NCH₃-, -O-, -C(CH₃)₂-, -S-, -C=C-, -C≡C-, -CHF-, -CHCF₃-, -C(O)-, -S(O)-, -S(O)₂-, - C(O)O-, -OC(O)-, -C(O)NH-, -C(O)NCH₃-, -NHC(O)-, -(CH₂)ₜ- or -NCH₃C(O)-; one or more methylene groups in the -(CH₂)ₜ- are optionally replaced by groups selected from -O-, -NH-, -C≡C-, -N(C₁-C₆ alkyl)- and -C(O)-, and t is 0, 1, 2, 3, 4, 5, 6 or 7;
L₃ is -(CH₂)ₖ-, wherein one or more methylene groups in the L₃ are optionally replaced by groups selected from -O-, -NH-, -N(C₁-C₆ alkyl)-, -N(C₁-C₆ haloalkyl)-, -N(C₁-C₆ hydroxyalkyl)-, -C≡C-, -C(O)- or -N(C₃-C₈ cycloalkyl)-, and k is 0, 1, 2, 3, 4, 5, 6, 7, 8, 9 or 10.

18. The compound of formula I, the stereoisomer, enantiomer, diastereomer, deuteride, hydrate, solvate or prodrug thereof or the pharmaceutically acceptable salt thereof according to claim 17, wherein the L is LA, and in the LA, ring D and ring B are absent, and ring C is 7-11 membered spiroheterocycloalkylene containing 1 or 2 nitrogen heteroatoms; X"" is -C(O)-; L₃ is -(CH₂)ₖ-; k is 1, 2, 3, 4 or 5.

19. The compound of formula I, the stereoisomer, enantiomer, diastereomer, deuteride, hydrate, solvate or prodrug thereof or the pharmaceutically acceptable salt thereof according to claim 18, wherein the L is selected from 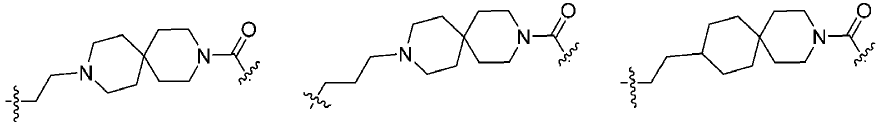 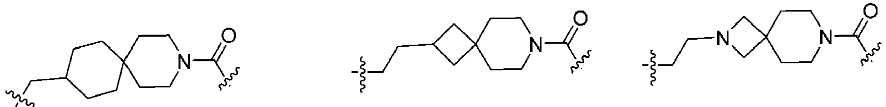 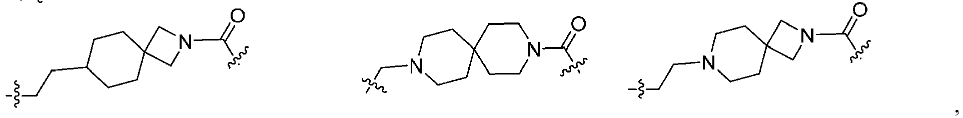 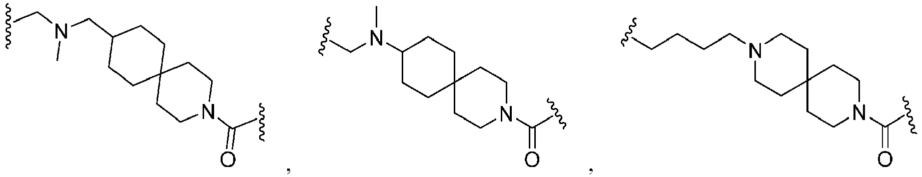 or

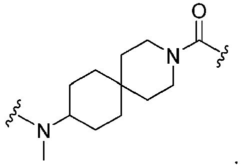

20. The compound of formula I, the stereoisomer, enantiomer, diastereomer, deuteride, hydrate, solvate or prodrug thereof or the pharmaceutically acceptable salt thereof according to claim 17, wherein the L is LA, and in the LA, ring D and ring B are absent, and ring C is 4-7 membered heterocycloalkyl containing 1 or 2 nitrogen heteroatoms; X"" is -C(O)-, a bond or -CH₂-; L₃ is -(CH₂)ₖ-; k is 1, 2, 3, 4 or 5.

21. The compound of formula I, the stereoisomer, enantiomer, diastereomer, deuteride, hydrate, solvate or prodrug thereof or the pharmaceutically acceptable salt thereof according to claim 20, wherein the L is selected from

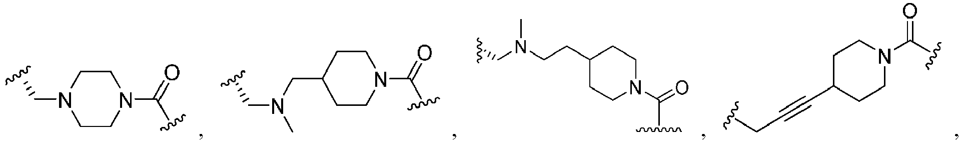

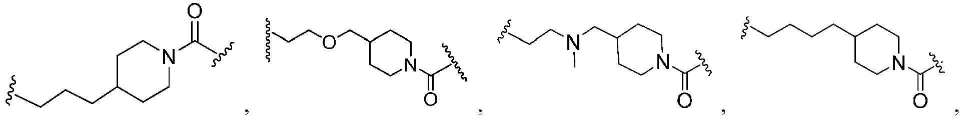

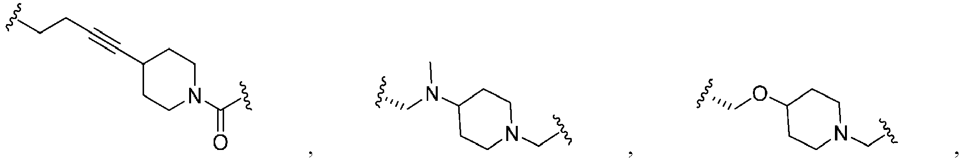

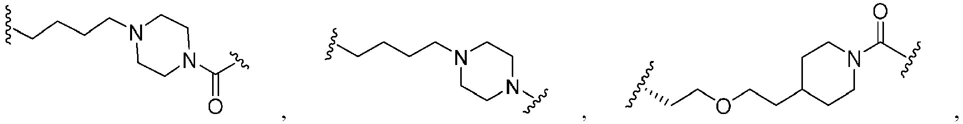

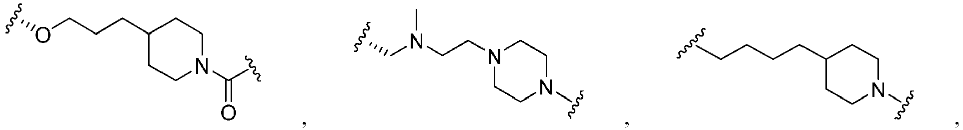

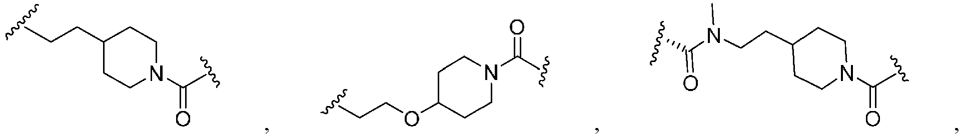

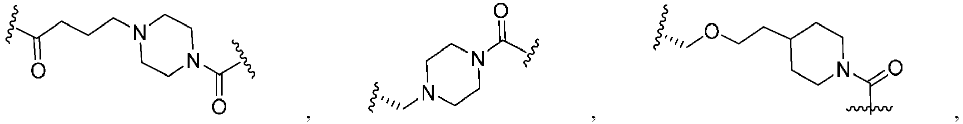

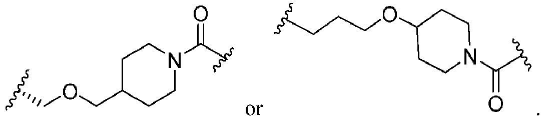

22. The compound of formula I, the stereoisomer, enantiomer, diastereomer, deuteride, hydrate, solvate or prodrug thereof or the pharmaceutically acceptable salt thereof according to claim 17, wherein the L is LA, and in the LA, ring D is C₄-C₇ membered cycloalkyl or 4-7 membered heterocycloalkyl, and ring B and ring C are absent or 5-11 membered heterocycloalkylene containing 1 or 2 nitrogen heteroatoms; X"" is -C(O)- or -(CH₂)ₜ-, and t is 0, 1, 2, 3, 4 or 5; L₃ is -(CH₂)ₖ-, and k is 1, 2, 3, 4 or 5.

23. The compound of formula 1, the stereoisomer, enantiomer, diastereomer, deuteride, hydrate, solvate or prodrug thereof or the pharmaceutically acceptable salt thereof according to claim 22, wherein the L is selected from 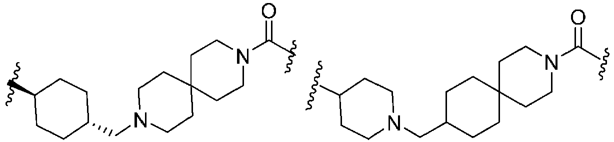 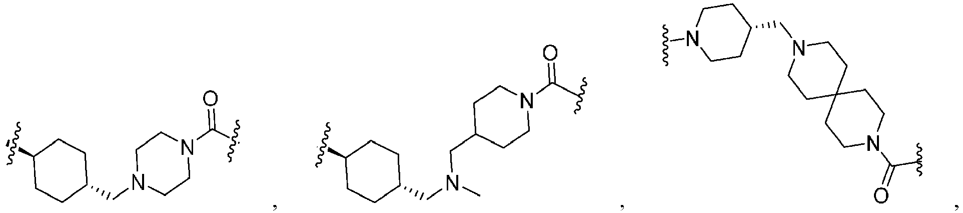 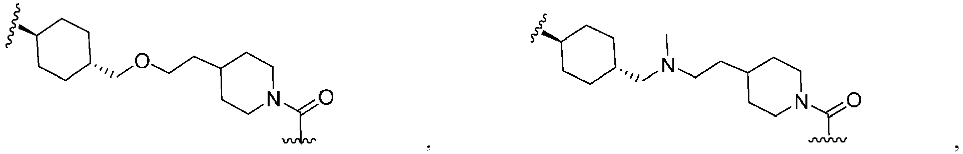 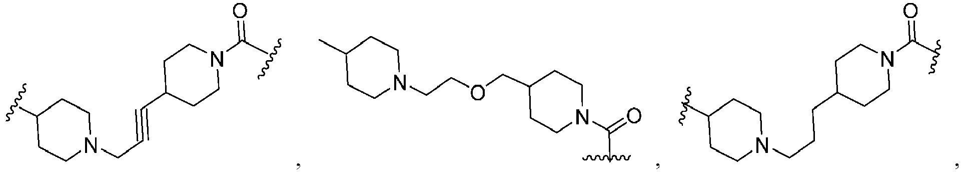 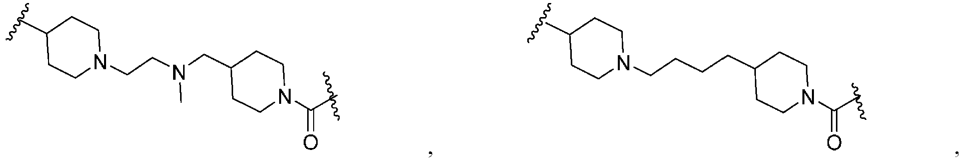 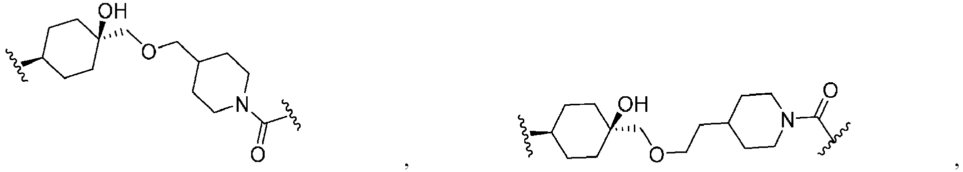 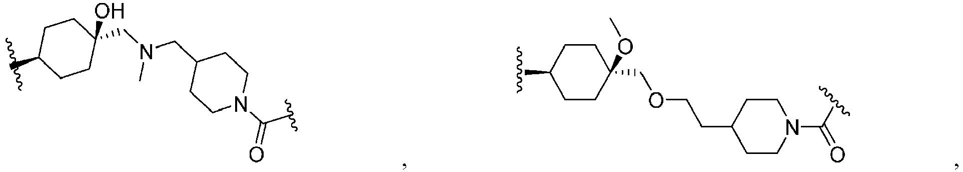 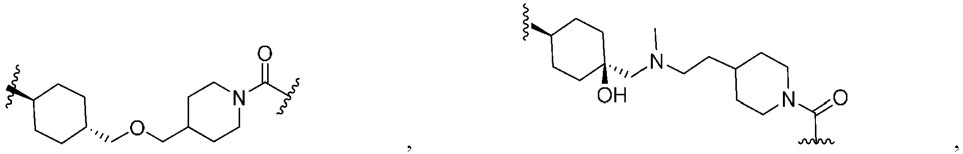 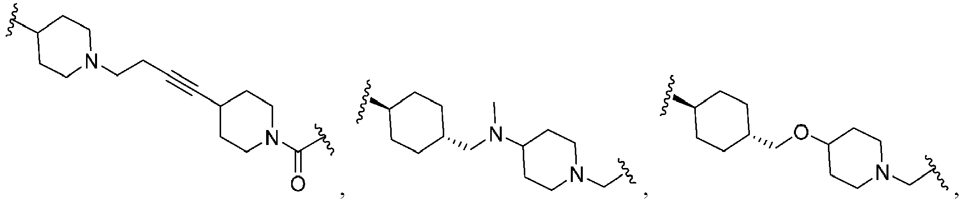 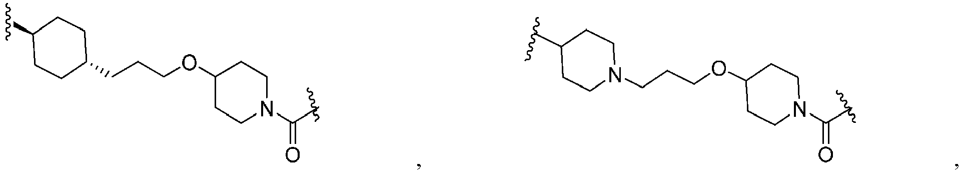 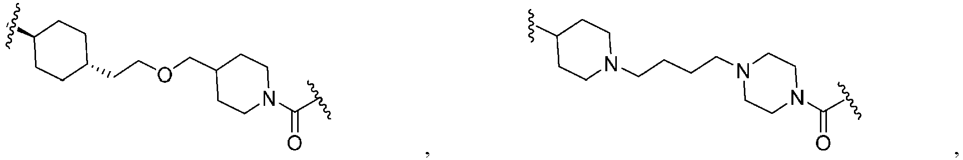 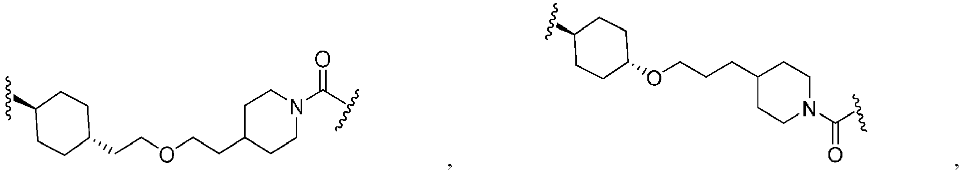 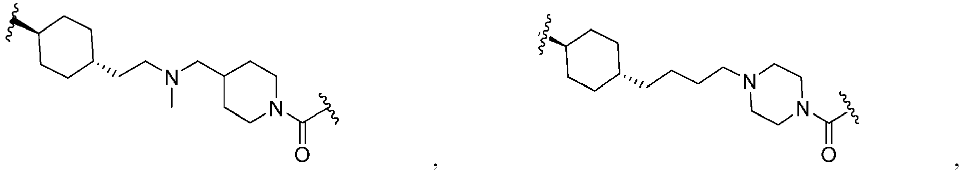 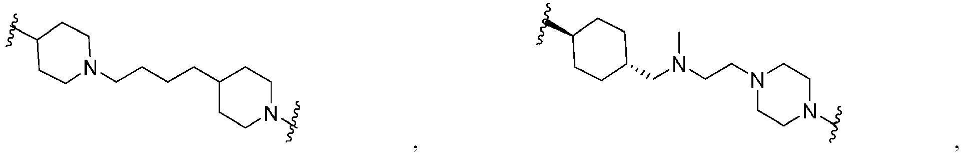 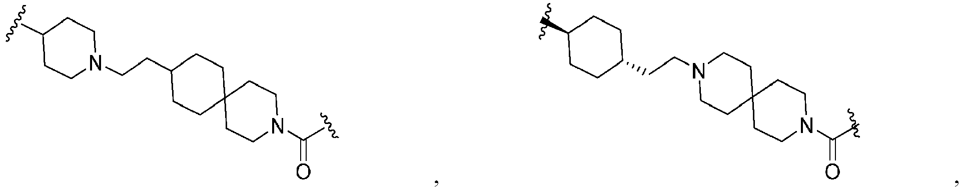 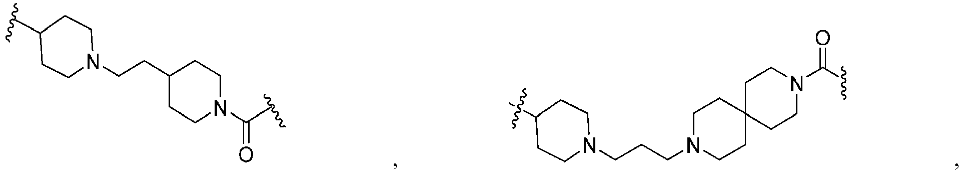 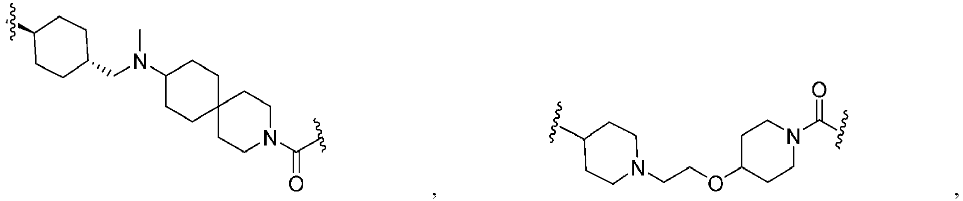 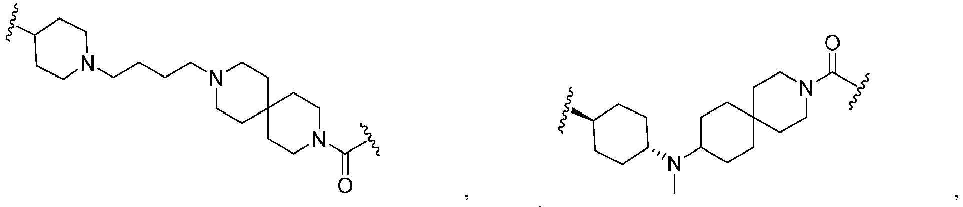 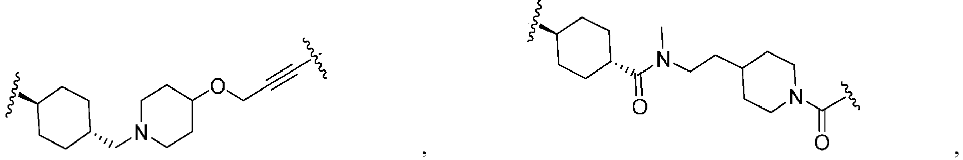 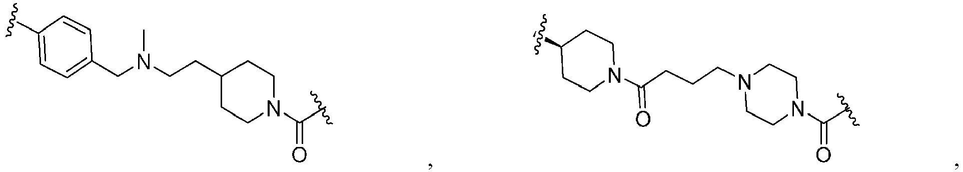 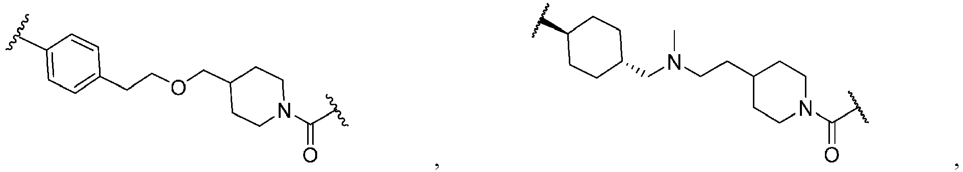 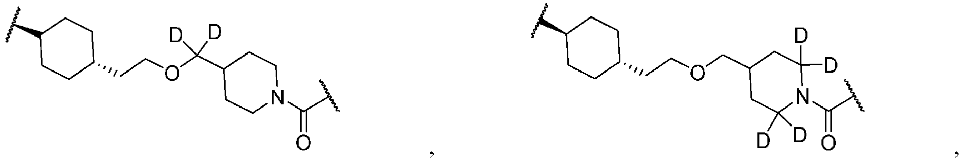 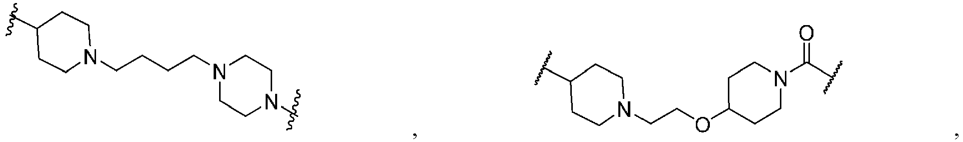 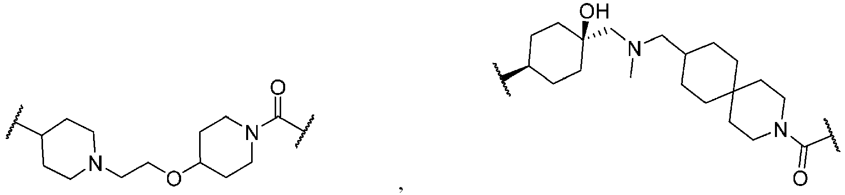 and 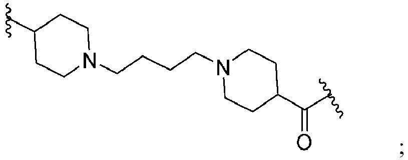 preferably

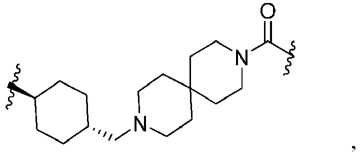

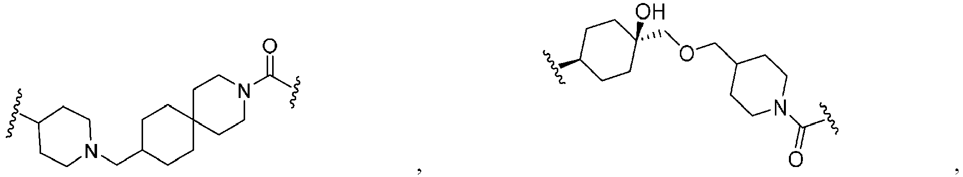

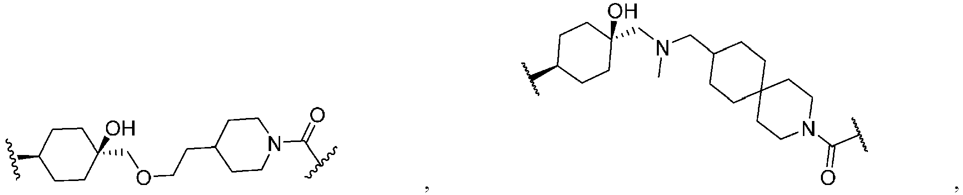

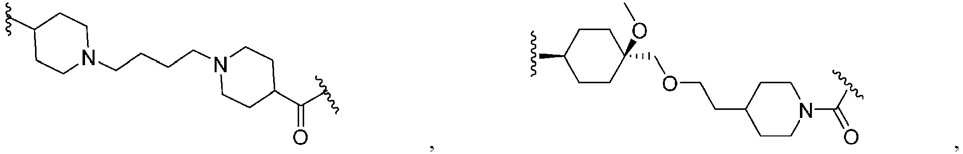

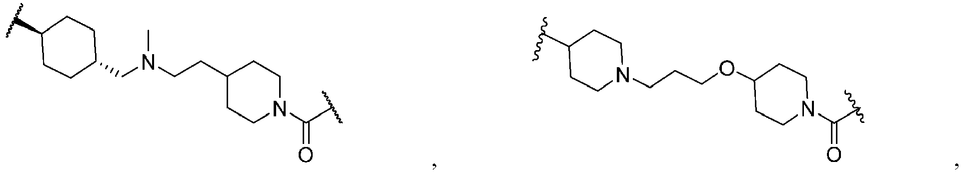

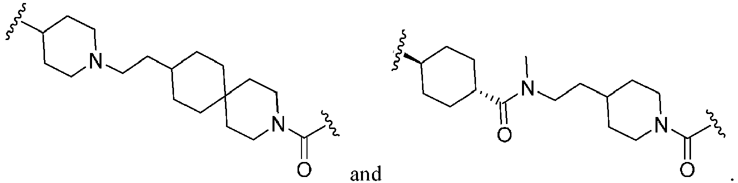

24. The compound of formula I, the stereoisomer, enantiomer, diastereomer, deuteride, hydrate, solvate or prodrug thereof or the pharmaceutically acceptable salt thereof according to claim 1, wherein the L is the LA, 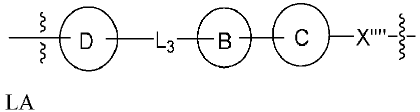 wherein in the LA:
ring D is absent or is C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from deuterium, halogen, oxo, cyano, amino, hydroxy, C₁-C₆ alkyl, C₁-C₆ haloalkyl, C₁-C₆ hydroxyalkyl or -O-(C₁-C₆ alkyl);
ring B is absent or is C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from deuterium, halogen, oxo, cyano, amino, hydroxy, C₁-C₆ alkyl, C₁-C₆ haloalkyl. Ci-C₆ hydroxyalkyl or -O-(C₁-C₆ alkyl);
ring C is absent, C₃-C₁₂ cycloalkylene or 3-12 membered heterocycloalkylene containing 1-2 heteroatoms selected from N, O or S, wherein the cycloalkylene and heterocycloalkylene are optionally substituted with substituents selected from deuterium, halogen, oxo, cyano, amino, hydroxy, C₁-C₆ alkyl, C₁-C₆ haloalkyl, Ci-C₆ hydroxyalkyl or -O-(C₁-C₆ alkyl);
X"" is a bond, -NH-, -NCH₃-, -O-, -C(CH₃)₂-, -S-, -C=C-, -C≡C-, -CHF-, -CHCF₃-, -C(O)-, -S(O)-, -S(O)₂-,-C(O)O-, -OC(O)-, -C(O)NH-, -C(O)NCH₃-, -NHC(O)- or -NCH₃C(O)-;
L₃ is -(CH₂)ₖ-, wherein one or two methylene groups in the L₃ are optionally replaced by groups selected from deuterium, -O-, -NH-, -N(C₁-C₆ alkyl)-, -N(C₁-C₆ haloalkyl)-, -C≡C-, -C(O)-, -N(C₁-C₆ hydroxyalkyl)- or - N(C₃-C₈ cycloalkyl)-, and k is 0, 1, 2, 3, 4, 5, 6, 7, 8, 9 or 10;
and/or, when ring A" is 5-11 membered bicyclic heteroaryl containing 1, 2 or 3 heteroatoms selected from N, O or S, ring D is 6-10 membered aryl, 5-10 membered heteroaryl, C₃-C₁₂ cycloalkylene or 3-12 membered heterocycloalkylene containing 1-2 heteroatoms selected from N, O or S, wherein the cycloalkylene and heterocycloalkylene are substituted with substituents selected from halogen, oxo, cyano, amino, hydroxy, Ci-C₆ alkyl, C₁-C₆ haloalkyl, C₁-C₆ hydroxyalkyl and -O-(C₁-C₆ alkyl).

25. The compound of formula I, the stereoisomer, enantiomer, diastereomer, deuteride, hydrate, solvate or prodrug thereof or the pharmaceutically acceptable salt thereof according to claim 1, wherein when ring A" is 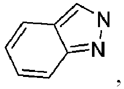 the ring D is C₃-C₁₂ cycloalkylene or is 3-12 membered heterocycloalkylene containing 1-2 heteroatoms selected from N, O or S, wherein the cycloalkylene and heterocycloalkylene are independently substituted with 1, 2 or 3 hydroxy groups or with 1, 2 or 3 -O-(C₁-C₆ alkyl) groups.

26. The compound of formula I, the stereoisomer, enantiomer, diastereomer, deuteride, hydrate, solvate or prodrug thereof or the pharmaceutically acceptable salt thereof according to claim 1, wherein
the L is LA, 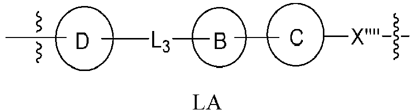
wherein the LA is scheme 1, scheme 2, scheme 3 or scheme 4;
scheme 1:
ring D is C₃-C₁₂ cycloalkylene or is 3-12 membered heterocycloalkylene containing 1-2 N heteroatoms, wherein the cycloalkylene and heterocycloalkylene are optionally substituted with substituents selected from halogen, hydroxy or -O-(C₁-C₆ alkyl);
ring B is C₃-C₁₂ cycloalkylene or is 3-12 membered heterocycloalkylene containing 1-2 heteroatoms selected from N, O or S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl or -O-(C₁-C₆ alkyl);
ring C is absent;
X"" is -C(O)-;
L₃ is -(CH₂)ₖ-, wherein one or two methylene groups in the L₃ are replaced by groups selected from -O-, -NH-, -N(C₁-C₆ alkyl)-, -N(C₁-C₆ haloalkyl)-, -C(O)-, -N(C₁-C₆ hydroxyalkyl)- or -N(C₃-C₈ cycloalkyl)-, and k is 0, 1, 2, 3, 4, 5, 6, 7, 8, 9 or 10;
scheme 2:
ring D is C₃-C₁₂ cycloalkylene or is 3-12 membered heterocycloalkylene containing 1-2 N heteroatoms,
wherein the cycloalkylene and heterocycloalkylene are optionally substituted with substituents selected from halogen, hydroxy or methoxy;
ring B is 7-11 membered spiroheterocycloalkylene or fused heterocycloalkylene containing 1 or 2 nitrogen heteroatoms;
ring C is absent;
X"" is -C(O)-;
L₃ is -(CH₂)ₖ-, wherein k is 1, 2, 3, 4, 5, 6, 7, 8, 9 or 10;
scheme 3:
ring D is C₃-C₁₂ cycloalkylene or is 3-12 membered heterocycloalkylene containing 1-2 N heteroatoms,
wherein the cycloalkylene and heterocycloalkylene are optionally substituted with substituents selected from halogen, hydroxy or methoxy;
ring B is 3-12 membered heterocycloalkylene containing 1-2 heteroatoms selected from N, O or S;
ring C is absent;
X"" is -C(O)-;
L₃ is -(CH₂)ₖ-, wherein k is 1, 2, 3 or 4;
scheme 4:
ring D is cyclohexylene or is 6-membered heterocycloalkylene containing 1-2 N heteroatoms, wherein the cyclohexylene and 6-membered heterocycloalkylene are optionally substituted with substituents selected from halogen, hydroxy or methoxy;
ring B is 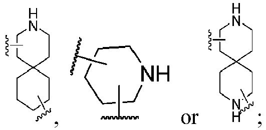
ring C is absent;
X"" is -C(O)-;
L₃ is -(CH₂)ₖ-, wherein one or two methylene groups in the L₃ are replaced by groups selected from -O-, -NH-, -N(C₁-C₆ alkyl)-, -N(C₁-C₆ haloalkyl)-, -C(O)-, -N(C₁-C₆ hydroxyalkyl)- or -N(C₃-C₈ cycloalkyl)-, and k is 1, 2, 3 or 4.

27. The compound of formula I, the stereoisomer, enantiomer, diastereomer, deuteride, hydrate, solvate or prodrug thereof or the pharmaceutically acceptable salt thereof according to claim 1, wherein
in the LA, ring D is 6-10 membered aryl, 4-7 membered saturated monocyclic heterocycloalkylene containing 1 or 2 nitrogen heteroatoms, or 7-11 membered spiroheterocycloalkylene or fused heterocycloalkylene containing 1 or 2 nitrogen heteroatoms;
and/or, ring C is 4-11 membered saturated or partially unsaturated spirocycloalkylene, 4-11 membered saturated or partially unsaturated fused cycloalkylene, 8-10 membered bicyclic saturated or partially unsaturated cycloalkylene, 4-7 membered saturated or partially unsaturated heterocycloalkylene having 1-2 heteroatoms independently selected from nitrogen, oxygen or sulfur, 4-11 membered saturated or partially unsaturated spiroheterocycloalkylene having 1-2 heteroatoms independently selected from nitrogen, oxygen or sulfur, 4-11 membered saturated and/or partially unsaturated fused heterocycloalkylene having 1-2 heteroatoms independently selected from nitrogen, oxygen or sulfur, 8-10 membered bicyclic saturated and/or partially unsaturated heterocycloalkylene having 1-2 heteroatoms independently selected from nitrogen, oxygen or sulfur, wherein the cycloalkyl and heterocycloalkyl are optionally substituted with substituents selected from deuterium, halogen, oxo, cyano, amino, hydroxy, C₁-C₆ alkyl, C₁-C₆ haloalkyl, C₁-C₆ hydroxyalkyl and -O-(C₁-C₆ alkyl);
and/or, in the LA, L₃ is -(CH₂)ₖ-, wherein one or two methylene groups in the L₃ are optionally replaced by groups selected from deuterium, -C(O)-, -C≡C-, -O-, -NH-, -NCH₃- or -NCH₂CH₃-; k is 1, 2, 3 or 4.

28. The compound of formula I, the stereoisomer, enantiomer, diastereomer, deuteride, hydrate, solvate or prodrug thereof or the pharmaceutically acceptable salt thereof according to claim 1, wherein the compound of formula I is 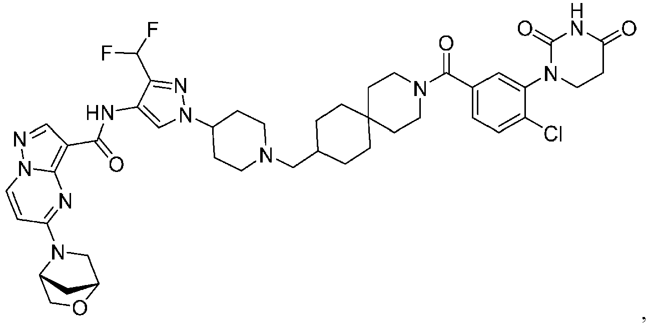 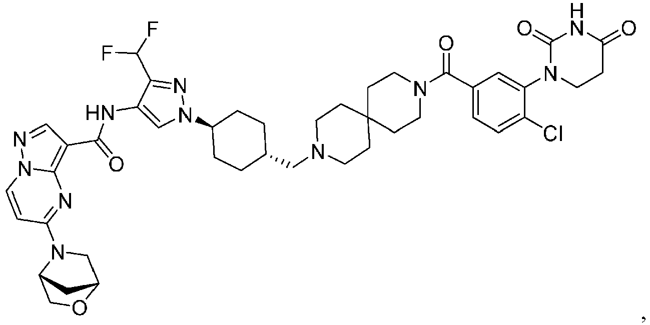 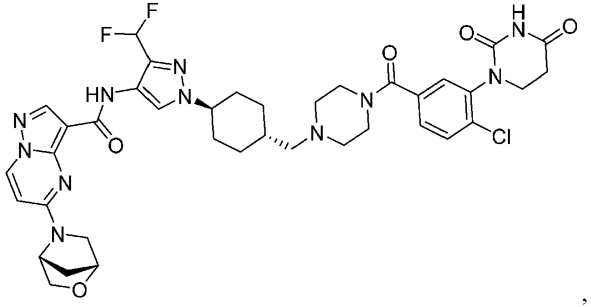 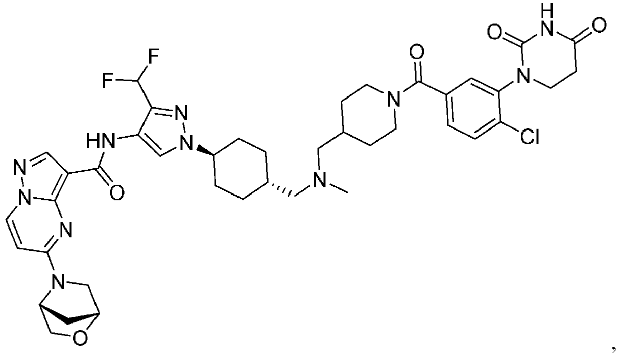 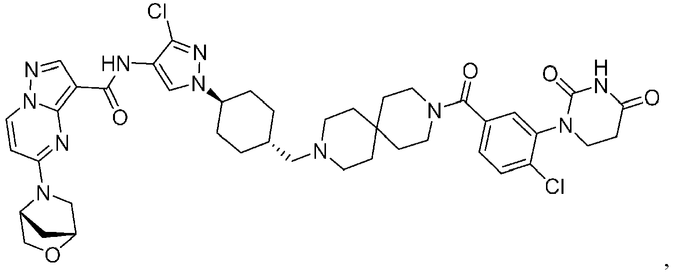 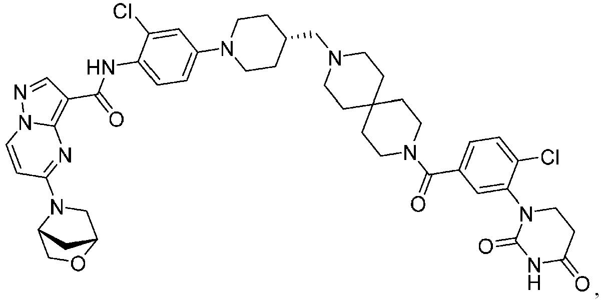 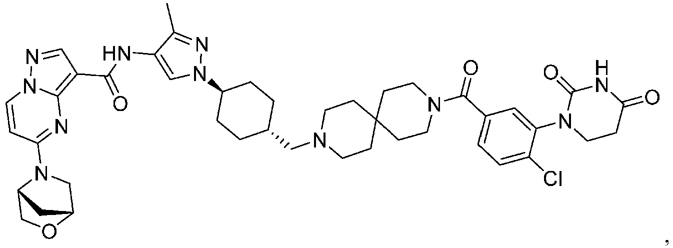 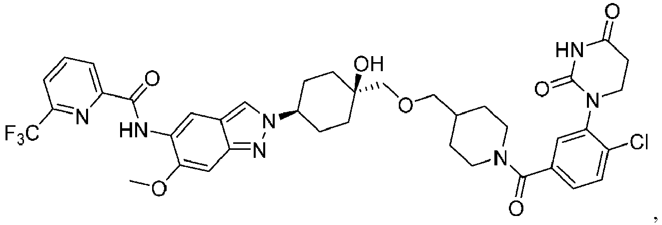 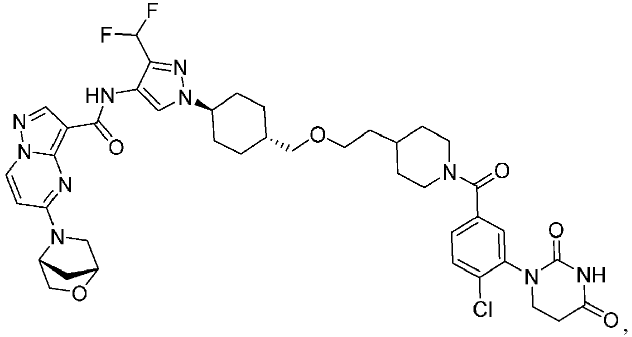 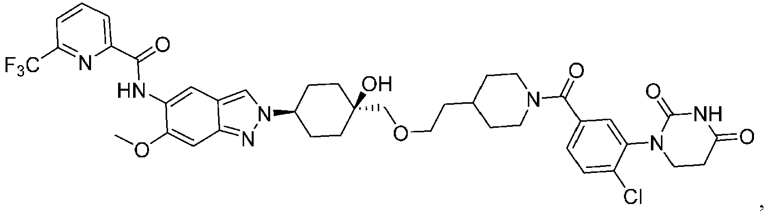 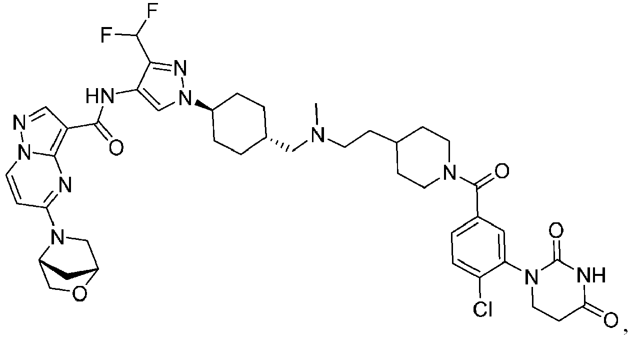 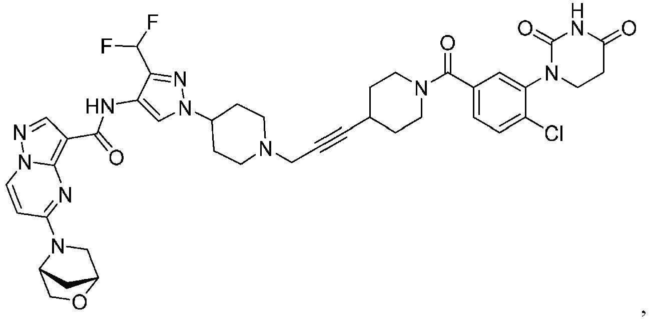 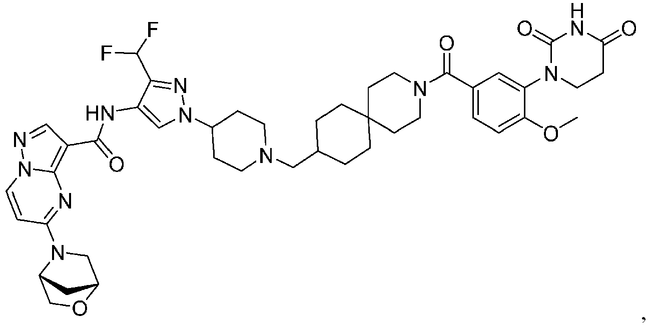 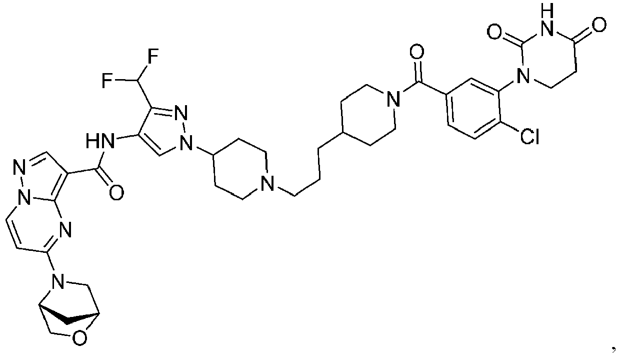 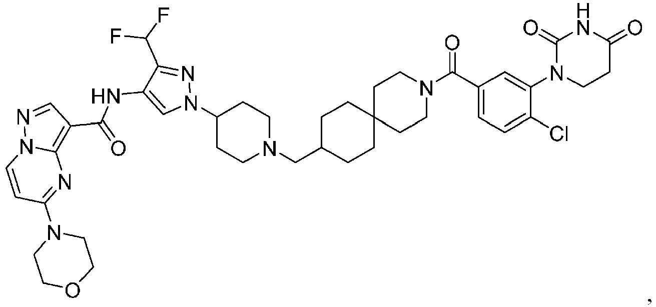 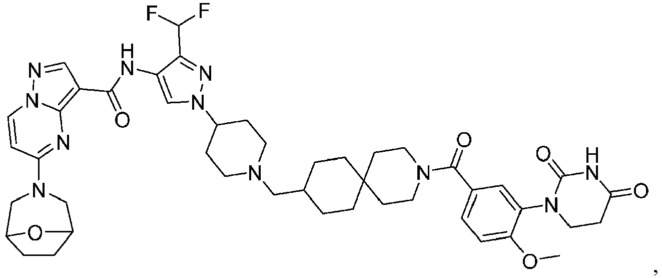 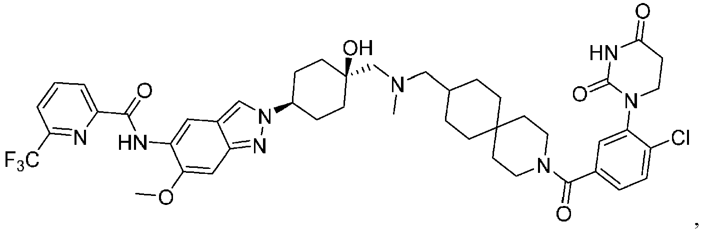 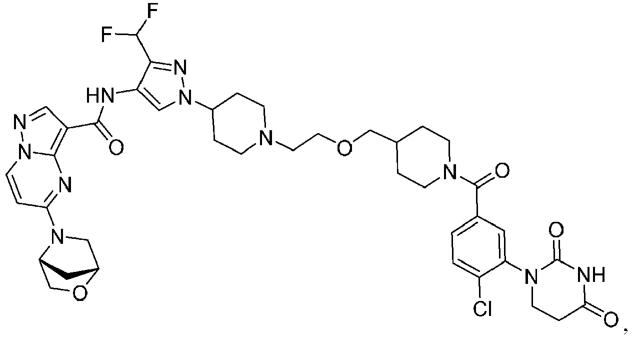 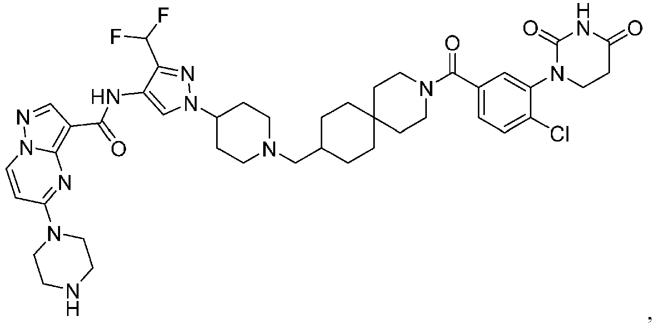 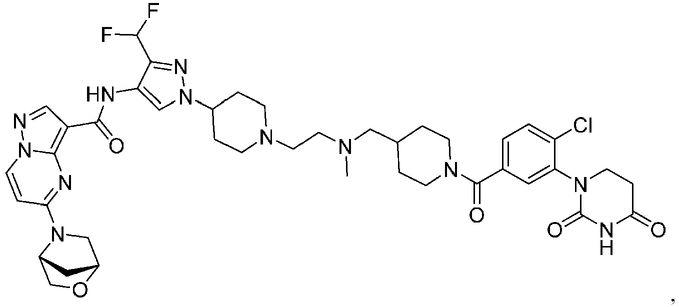 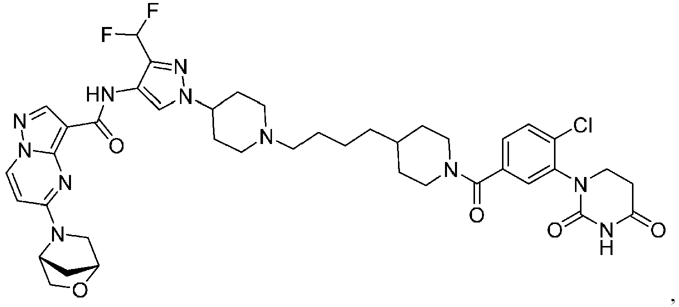 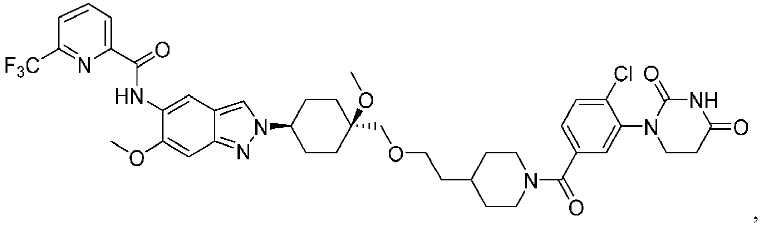 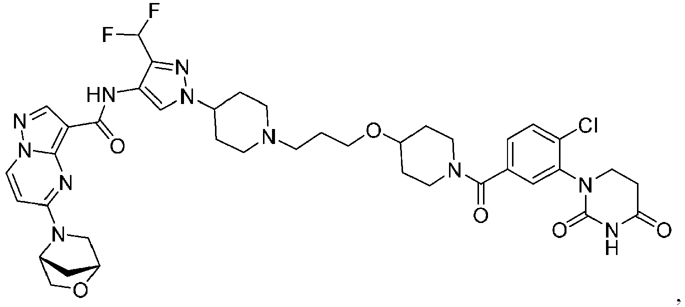 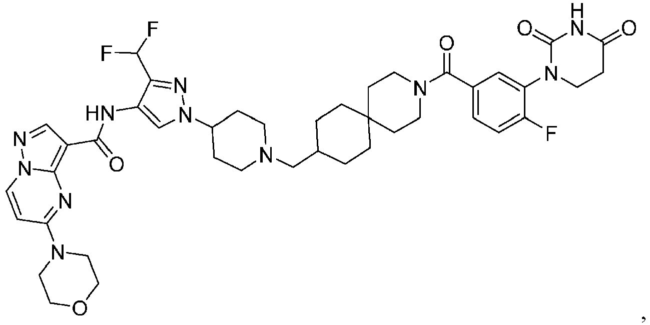 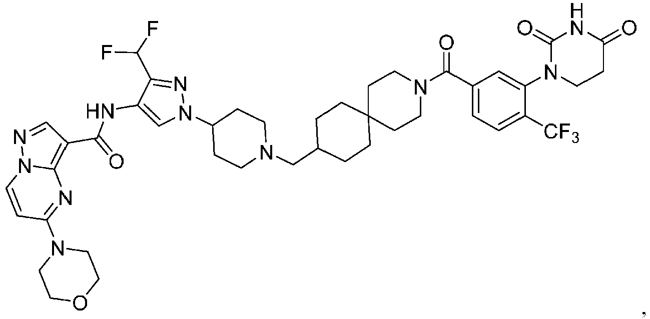 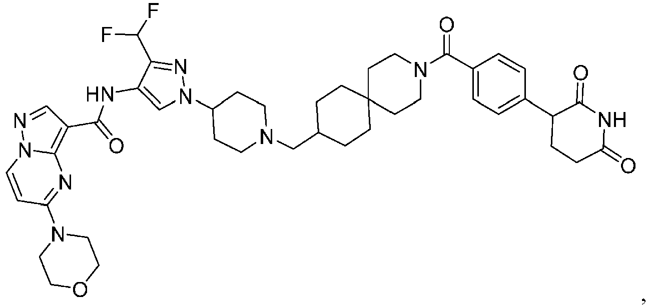 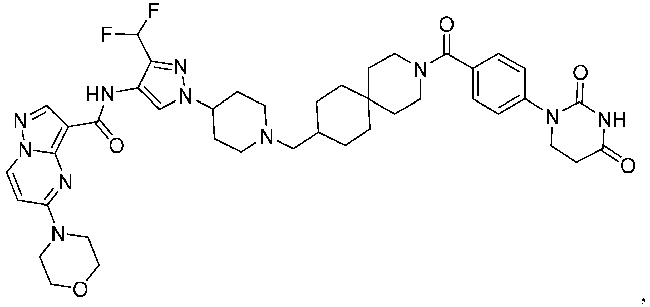 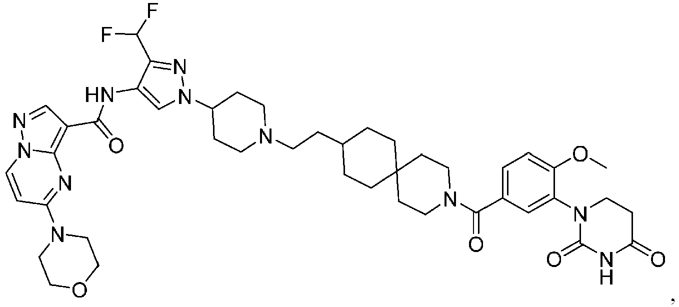 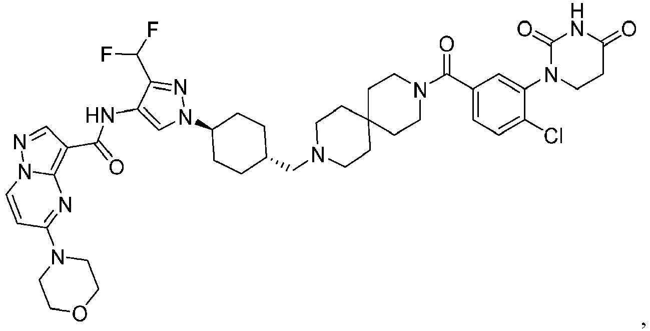 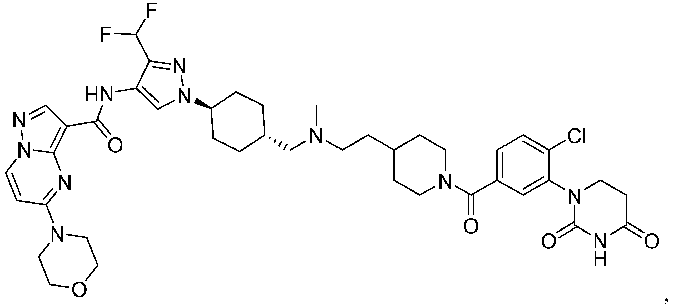 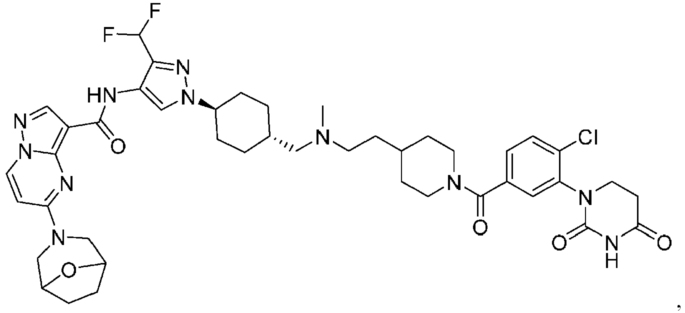 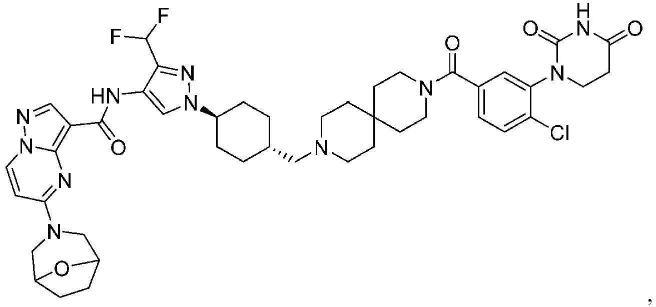 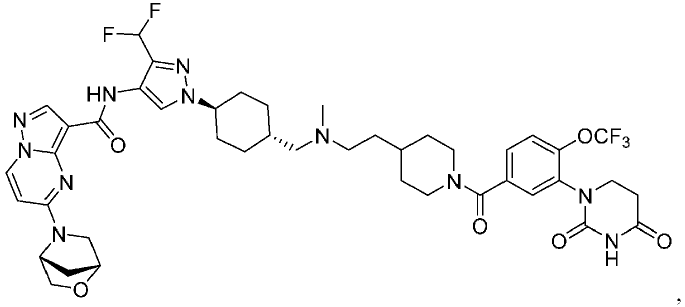 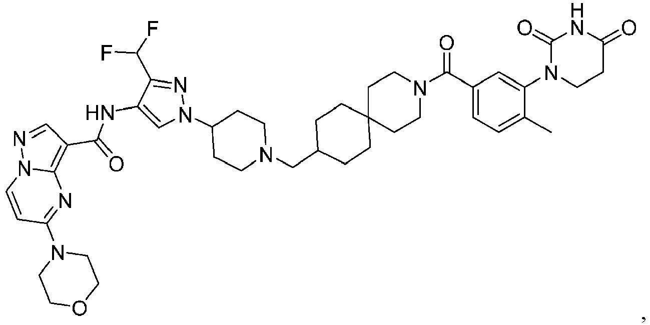 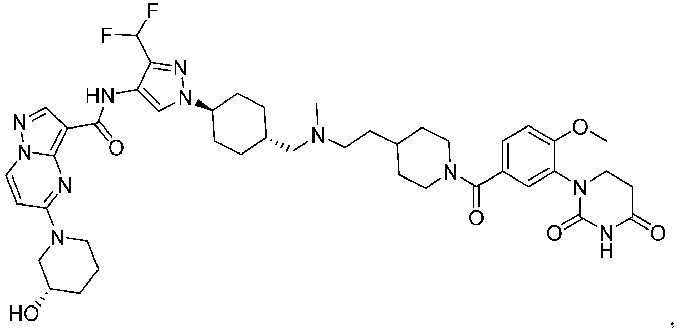 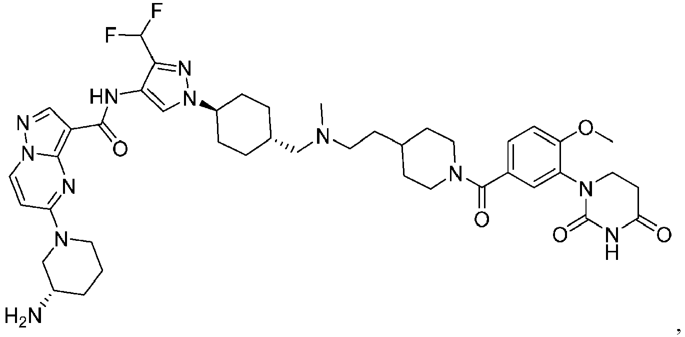 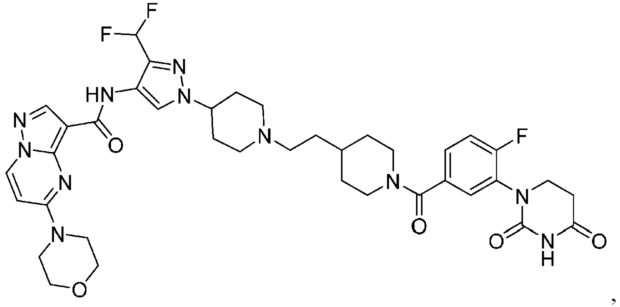 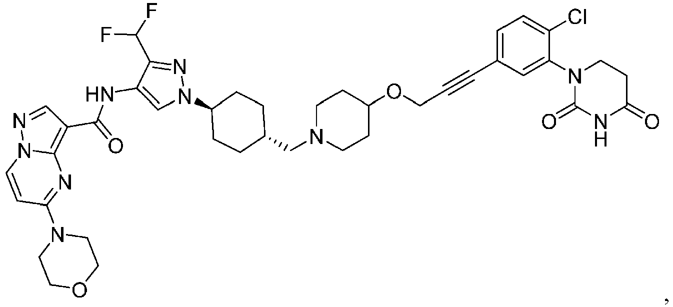 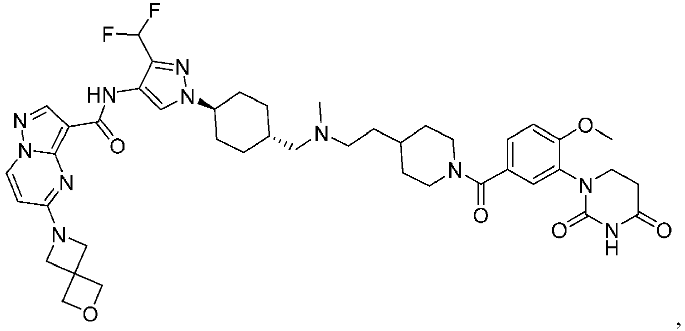 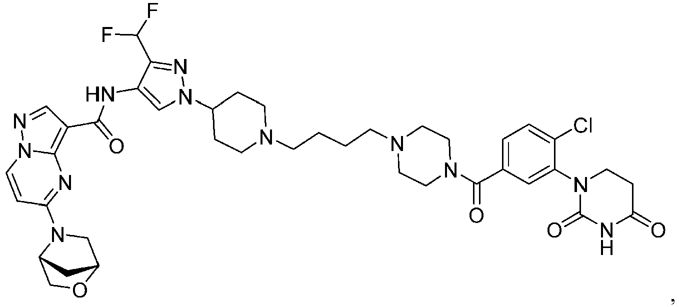 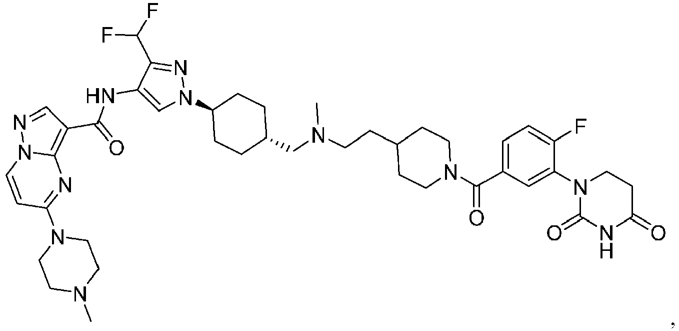 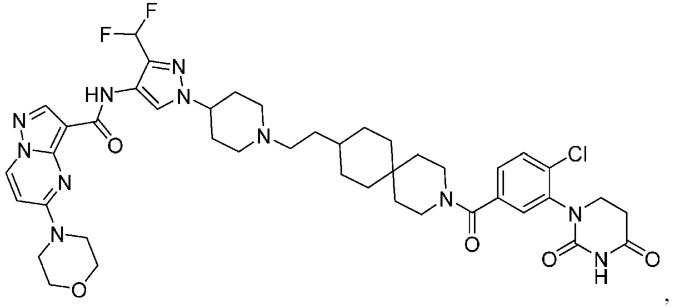 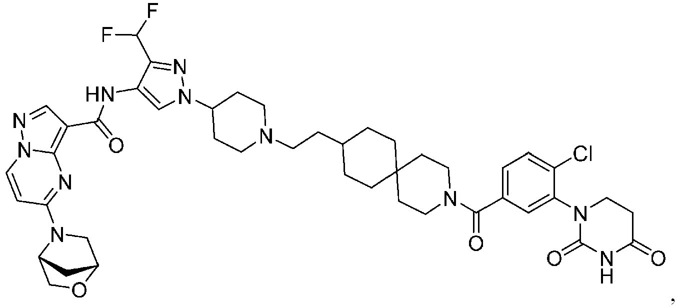 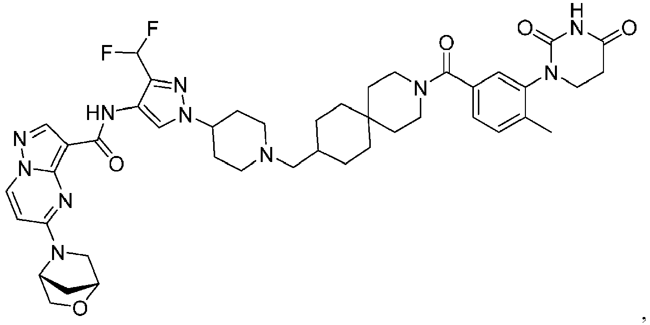 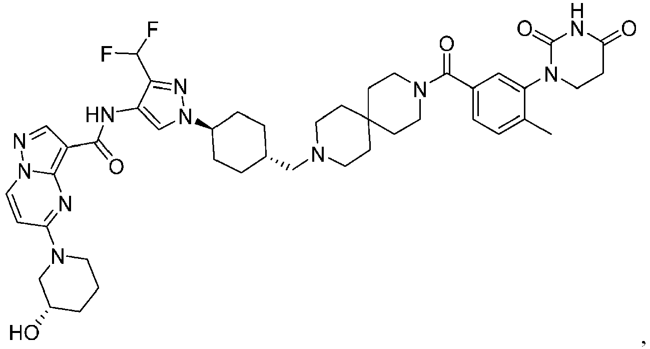 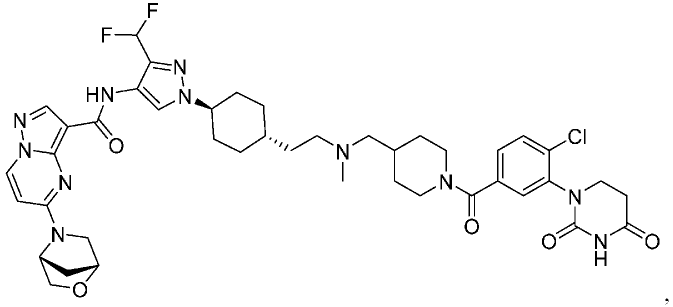 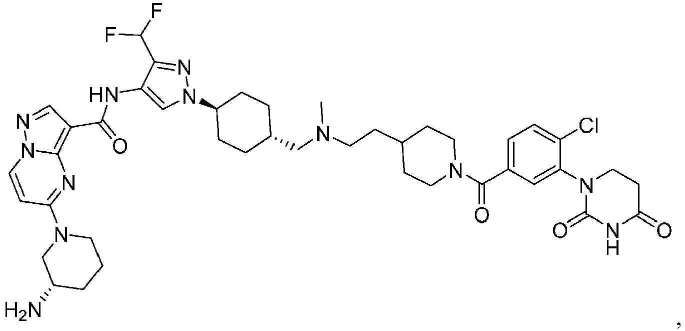 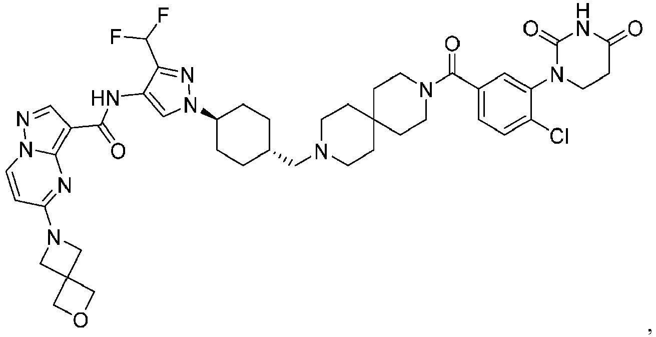 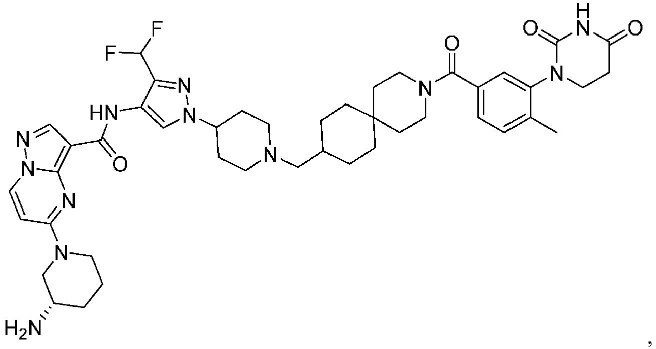 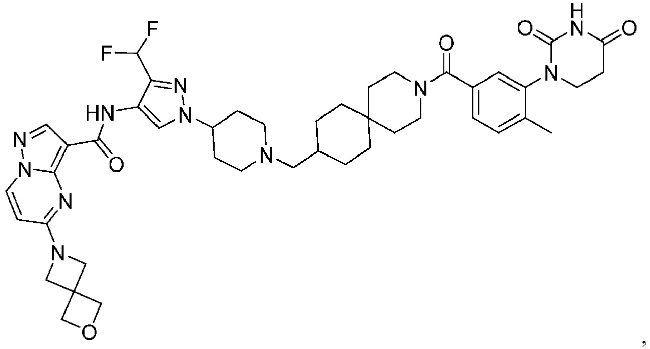 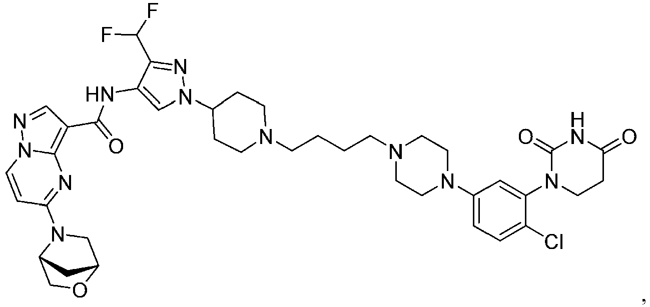 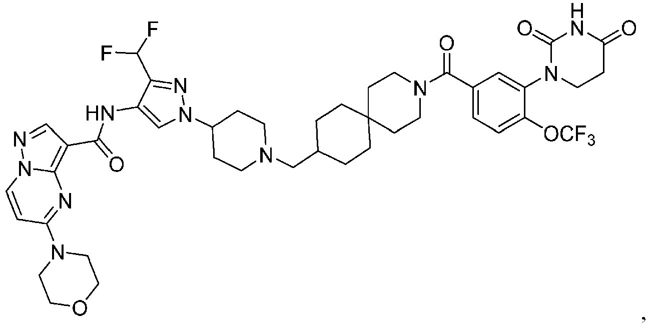 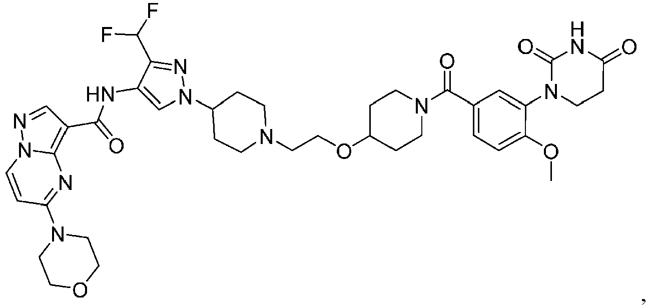 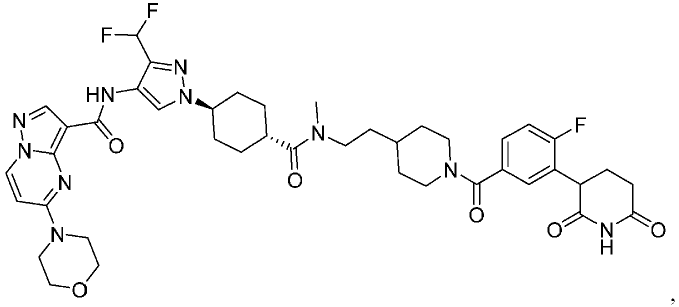 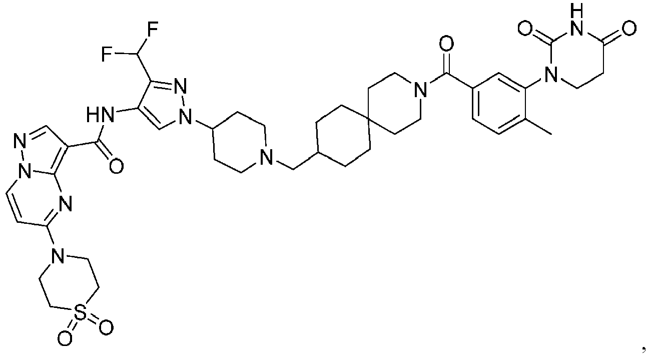 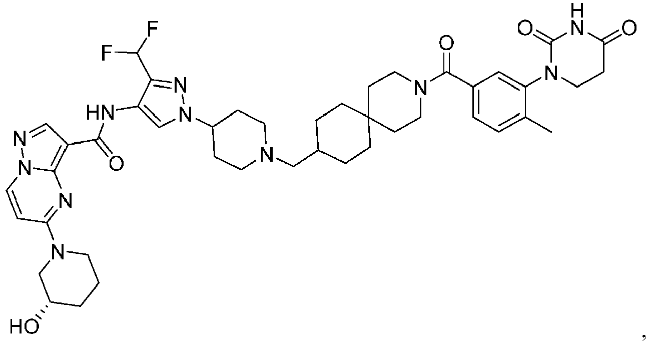 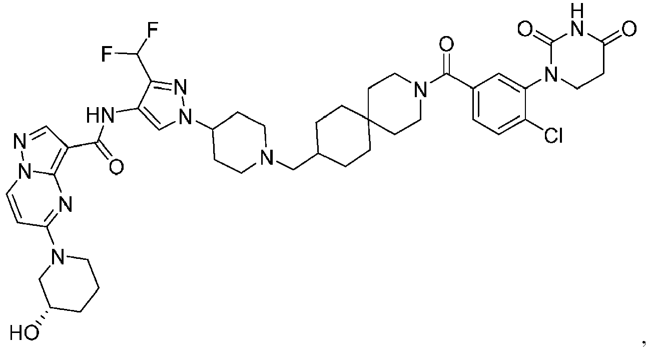 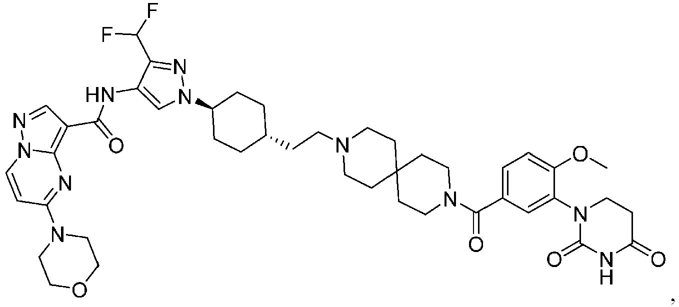 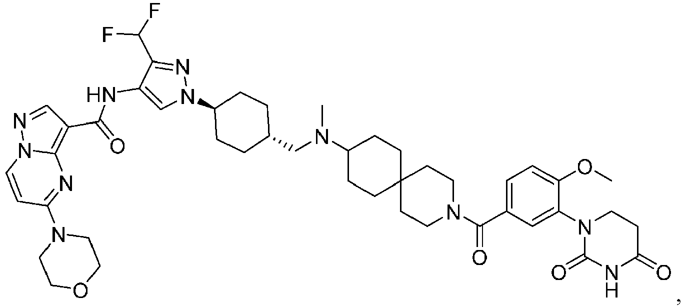 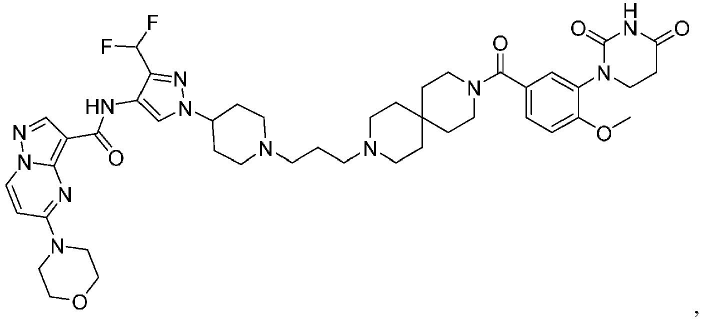 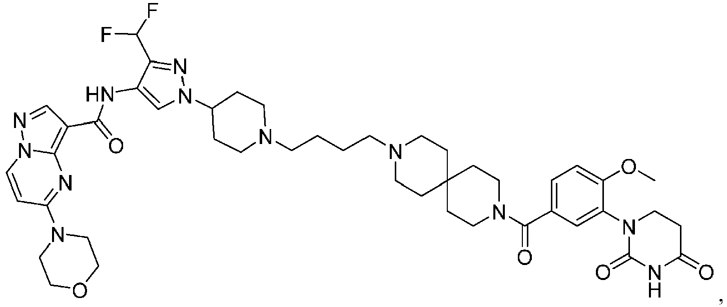 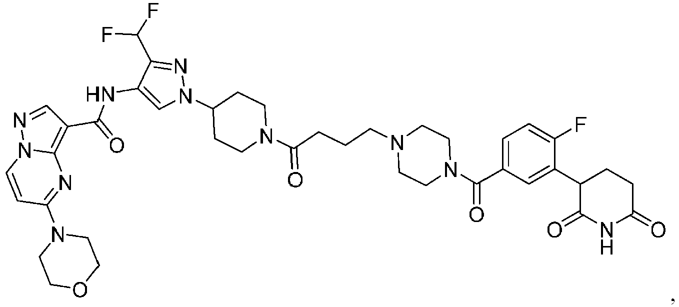 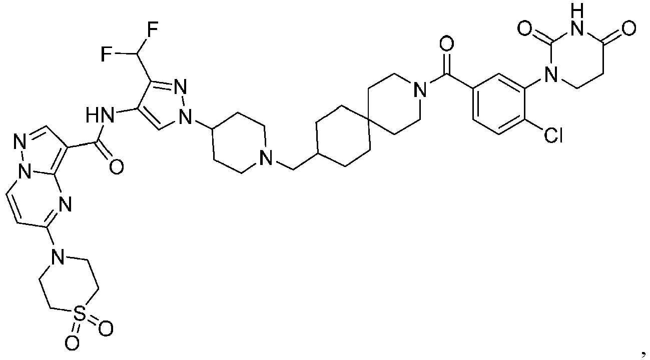 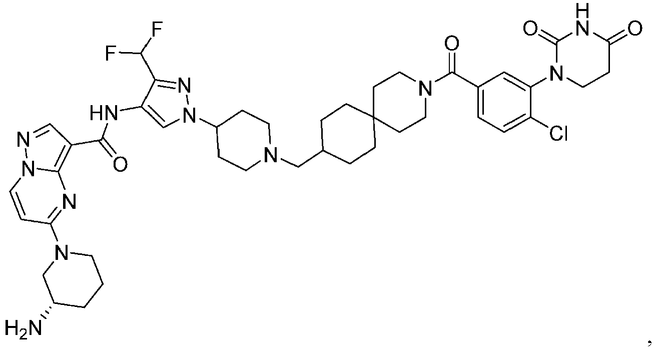 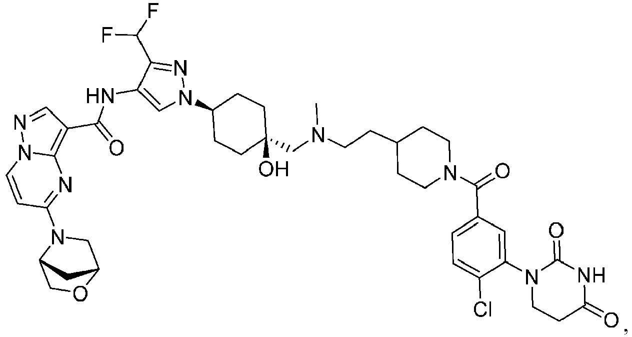 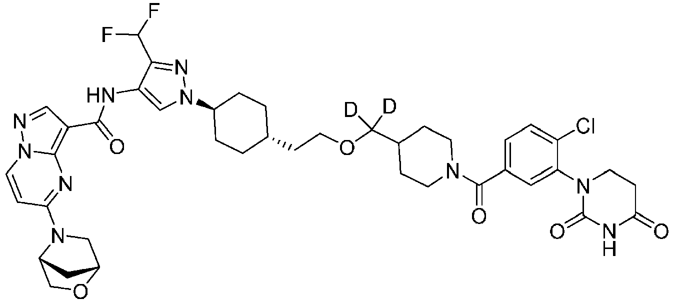 or

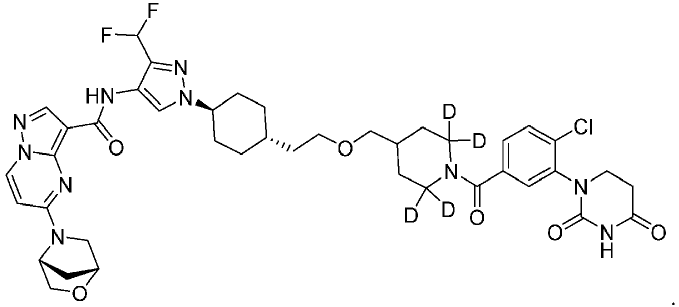

29. A pharmaceutical composition, comprising the compound of formula I, or the stereoisomer, enantiomer, diastereomer, deuteride, hydrate, solvate or prodrug thereof or the pharmaceutically acceptable salt thereof according to any of claims 1-28, and a pharmaceutically acceptable carrier, diluent or excipient.

30. Use of the compound of formula I, the stereoisomer, enantiomer, diastereomer, deuteride, hydrate, solvate or prodrug thereof or the pharmaceutically acceptable salt thereof according to any of claims 1-28 in the preparation of a medicament for treating and/or preventing IRAK4-mediated diseases or disorders, or diseases or disorders mediated by TLRs (except TLR3R) or the IL-1α or IL-1β receptor family.

31. Use of the compound of formula I, the stereoisomer, enantiomer, diastereomer, deuteride, hydrate, solvate or prodrug thereof or the pharmaceutically acceptable salt thereof according to any of claims 1-28 in the preparation of a medicament for treating and/or preventing disorders or diseases including cancer, neurodegenerative disorders, viral diseases, autoimmune diseases, inflammatory diseases, genetic disorders, hormone-associated diseases, metabolic disorders, diseases associated with organ transplantation, immunodeficiency disorders, destructive bone diseases, proliferative disorders, infectious diseases, conditions associated with cell death, thrombin-induced platelet aggregation, liver diseases, pathological immune conditions involving T cell activation, cardiovascular disorders or CNS.

32. Use of the compound of formula I, the stereoisomer, enantiomer, diastereomer, deuteride, hydrate, solvate or prodrug thereof or the pharmaceutically acceptable salt thereof according to any of claims 1-28 in the preparation of a medicament for treating and/or preventing disorders or diseases including brain cancer, kidney cancer, liver cancer, adrenal cancer, bladder cancer, breast cancer, stomach cancer, ovarian cancer, colon cancer, rectal cancer, prostate cancer, pancreatic cancer, lung cancer, vaginal cancer, cervical cancer, testicular cancer, genitourinary tract cancer, esophageal cancer, laryngeal cancer, skin cancer, bone cancer, thyroid cancer, sarcoma, glioblastoma, neuroblastoma, multiple myeloma, gastrointestinal cancer, neck or head tumor, epidermal hyperplasia, psoriasis, prostate hyperplasia, adenoma, adenocarcinoma, keratoacanthoma, squamous cell carcinoma, large cell carcinoma, non-small cell lung cancer, lymphoma, Hodgkin's and non-Hodgkin's, breast cancer, follicular carcinoma, undifferentiated carcinoma, papillary tumor, seminoma, melanoma, ABC DLBCL, Hodgkin's lymphoma, primary cutaneous T-cell lymphoma, chronic lymphocytic leukemia, smoldering indolent multiple myeloma, leukemia, diffuse large B-cell lymphoma (DLBCL), chronic lymphocytic leukemia (CLL), chronic lymphocytic lymphoma, primary exudative lymphoma, Burkitt's lymphoma/leukemia, acute lymphocytic leukemia, B-cell prolymphocytic leukemia, lymphoplasmacytic lymphoma, Waldenstroms's macroglobulinemia (WM), splenic marginal zone lymphoma, multiple myeloma, or plasmacytoma or intravascular large B-cell lymphoma; neurodegenerative disease caused by Alzheimer's disease, Parkinson's disease, amyotrophic lateral sclerosis, Huntington's disease, cerebral ischemia or traumatic injury, glutamate neurotoxicity, hypoxia, epilepsy, diabetes therapy, metabolic syndrome, obesity, organ transplantation or graft-versus-host disease; eye diseases, such as eye allergy, conjunctivitis, dry eye or spring conjunctivitis, diseases affecting the nose, including allergic rhinitis; autoimmune hematological disorders, such as hemolytic anemia, aplastic anemia, pure red blood cell anemia and idiopathic thrombocytopenia, systemic lupus erythematosus, rheumatoid arthritis, polychondritis, scleroderma, dermatomyositis, polymyositis, chronic active hepatitis, myasthenia gravis, Stephen-Johnson syndrome, idiopathic stomatitis diarrhea, autoimmune inflammatory bowel disease, irritable bowel syndrome, celiac disease, tooth root periostitis, lung hyaline membrane disease, nephropathy, glomerular disease, alcoholic liver disease, multiple sclerosis, endocrine ophthalmopathy, Grave's disease, sarcomatosis, dry eye, spring conjunctival keratitis, interstitial pulmonary fibrosis, psoriatic arthritis, systemic juvenile idiopathic arthritis, nephritis, vasculitis, interstitial cystitis, diverticulitis, glomerulonephritis, chronic granulomatous disease, adenomyosis, leptospirosis nephropathy, glaucoma, retinal disease, aging, headache, pain, complex regional pain syndrome, cardiac hypertrophy, muscle atrophy, catabolism, obesity, slow fetal growth, hypercholesterolemia, heart disease, chronic heart failure, mesothelioma, anhidrotic ectodermal dysplasia, Behcet's disease, pigment incontinence, Paget's disease, pancreatitis, hereditary periodic fever syndrome, asthma, acute lung injury, acute respiratory distress syndrome, eosinophilia, allergic reaction, systemic allergic reaction, sinusitis, eye allergy, silica-induced disease, COPD, lung disease, cystic fibrosis, acid-induced lung injury, pulmonary hypertension, polyneuropathy, cataract, muscle inflammation combined with systemic sclerosis, inclusion body myositis, myasthenia gravis, thyroiditis, Addison's disease, lichen planus, type 1 diabetes, type 2 diabetes, appendicitis, atopic dermatitis, asthma, allergy, bronchiolitis, bronchitis, bursitis, cervicitis, cholangitis, cholecystitis, chronic transplant rejection, colitis, conjunctivitis, cystitis, lacrimal gland inflammation, dermatitis, dermatomyositis, polymyositis, encephalitis, endocarditis, Endometritis, enteritis, enterocolitis, upper ankle inflammation, epididymitis, fasciitis, fibrous tissue inflammation, gastritis, gastroenteritis, Henoch-Schonlein purpura, hepatitis, suppurative sweat inflammation, immunoglobulin A nephropathy, interstitial lung disease, laryngitis, mastitis, meningitis, myelitis, myocarditis, myositis, nephritis, liver fibrosis, renal fibrosis, alcoholic fatty liver, non-alcoholic fatty liver, heart fibrosis, psoriasis, Crohn's disease, inflammatory bowel disease, oophoritis, orchitis, osteitis, otitis, pancreatitis, mumps, pericarditis, peritonitis, pharyngitis, pleurisy, phlebitis, local pneumonia, pneumonia, polymyositis, proctitis, prostatitis, pyelonephritis, rhinitis, salpingitis, sinusitis, stomatitis, articular membrane inflammation, tendinitis, tonsillitis, ulcerative colitis, uveitis, vaginitis, vasculitis, vulvitis, alopecia areata, erythema multiforme, dermatitis herpetiformis, sclerosis, vitiligo, hypersensitivity vasculitis, urticaria, bullous pemphigoid, pemphigus vulgaris, deciduous pemphigus, paraneoplastic pemphigus, acquired bullous epidermal laxity, acute and chronic gout, chronic gouty arthritis, psoriasis, psoriatic arthritis, rheumatoid arthritis, juvenile rheumatoid arthritis, cryopyrin-associated periodic syndrome or osteoarthritis.

33. Use of a substance A in the preparation of an IRAK4 protein degrader, wherein the substance A comprises the compound of formula I, the stereoisomer, enantiomer, diastereomer, deuteride, hydrate, solvate, metabolite or prodrug thereof or the pharmaceutically acceptable salt thereof according to any of claims 1-28, or the pharmaceutical composition according to claim 29.

34. A compound of formula VII or VIII, a stereoisomer, enantiomer, diastereomer or deuteride thereof or a salt thereof: 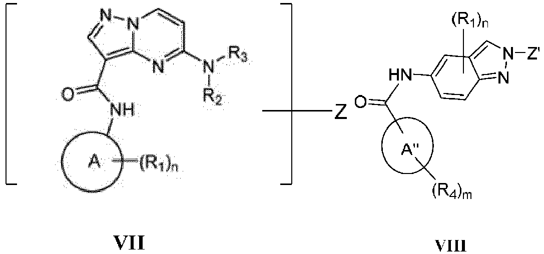
wherein Z is 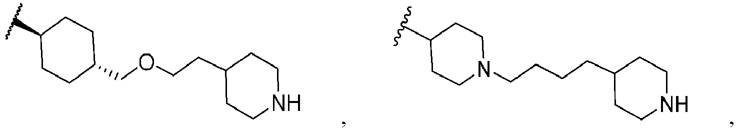 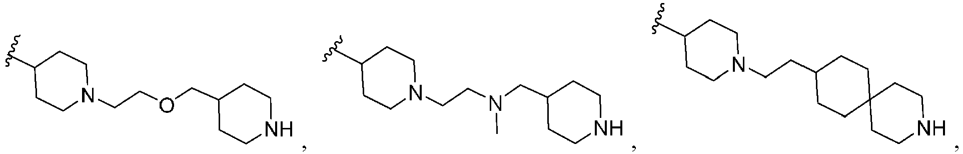 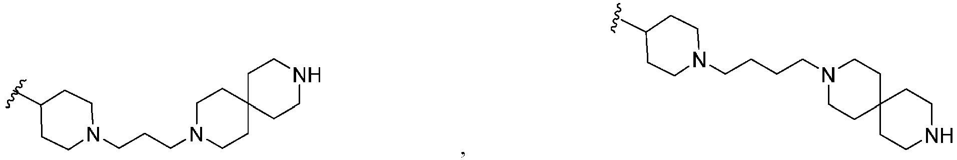 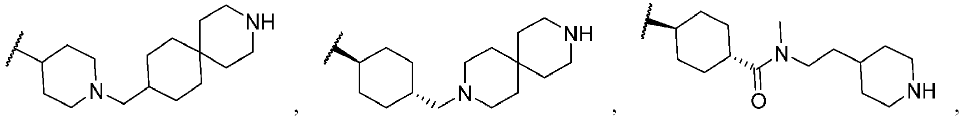 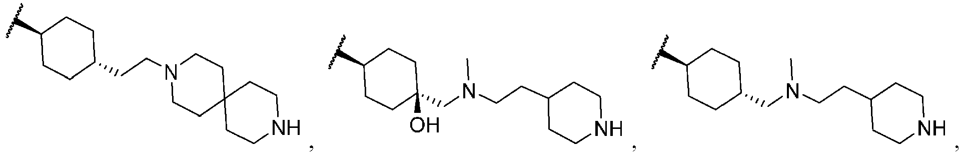 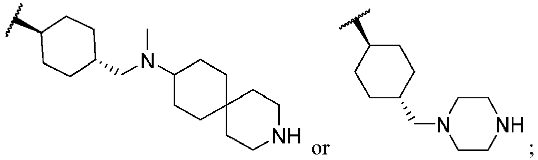
Z' is 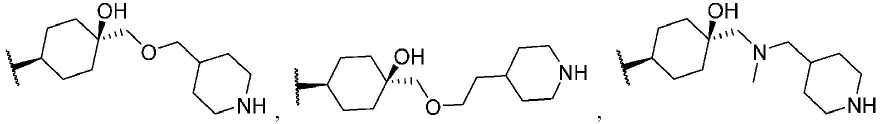 or 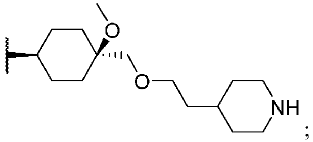
wherein m, n, A, A', R₁, R₂, R₃ and R₄ are as defined in any of claims 1-28; 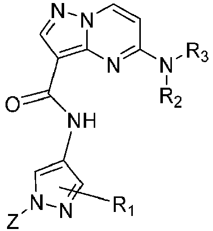
preferably, the compound of formula VII is a compound of formula VII-1;
more preferably, the compound of formula VII is any of the following compounds: 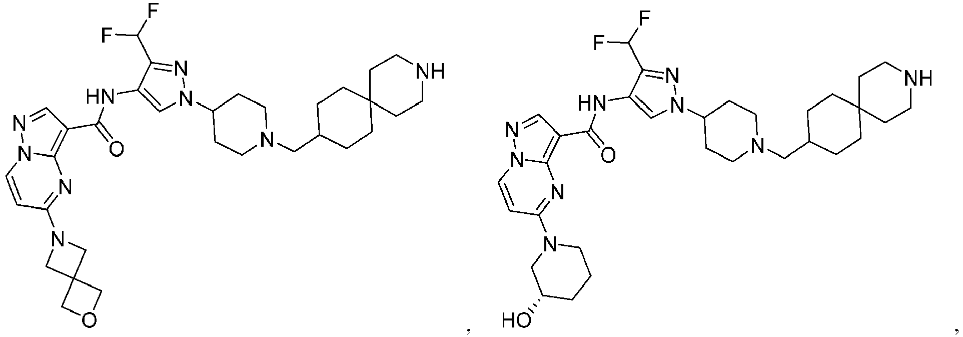 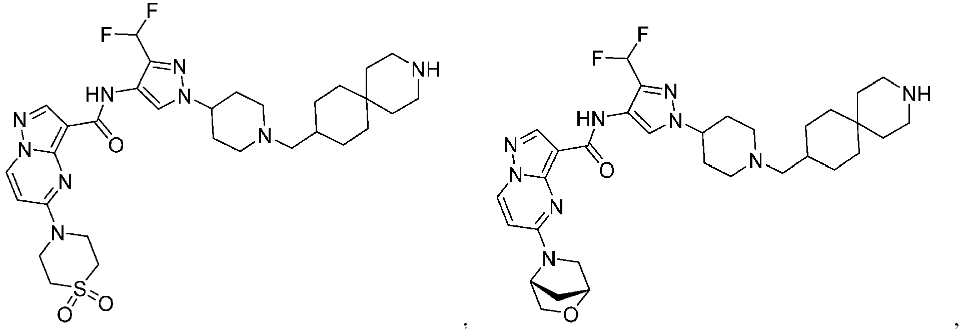 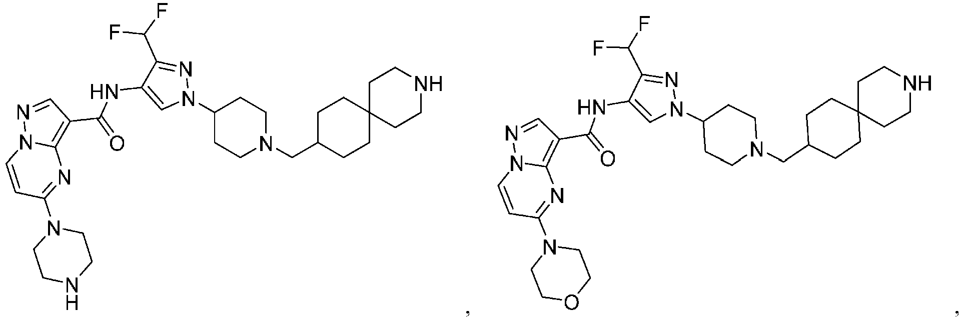 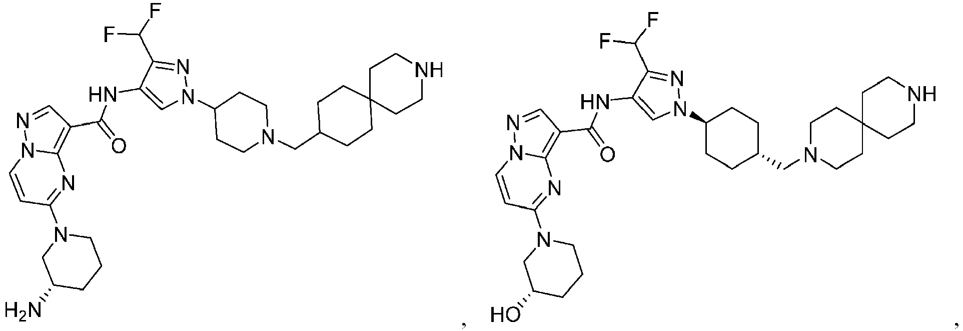 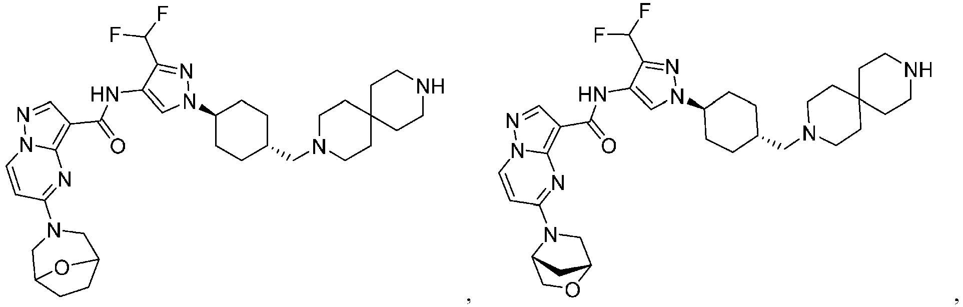 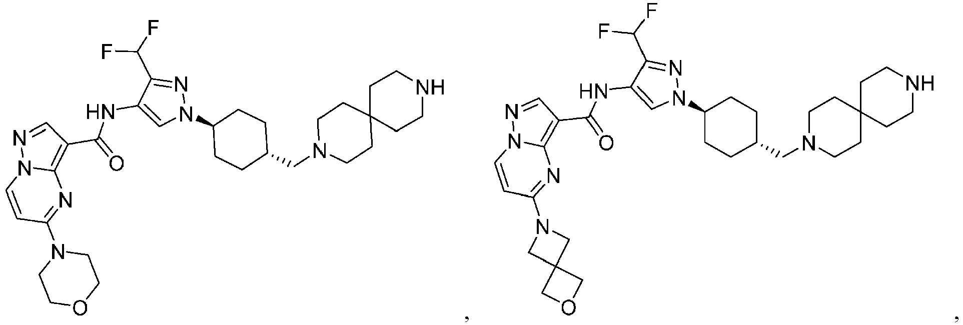 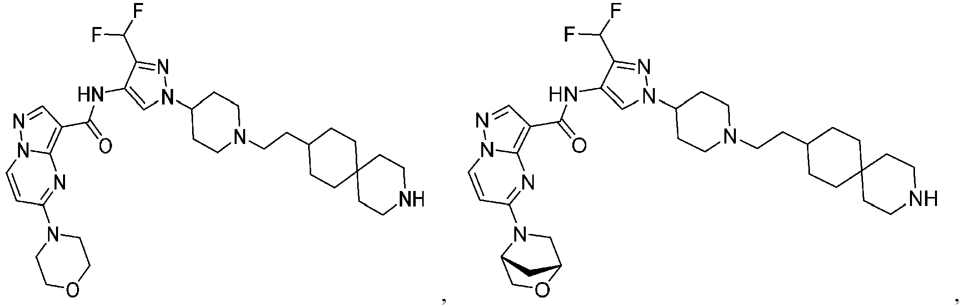 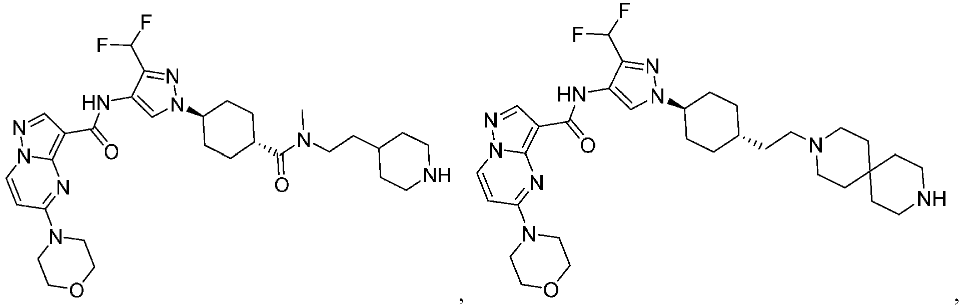 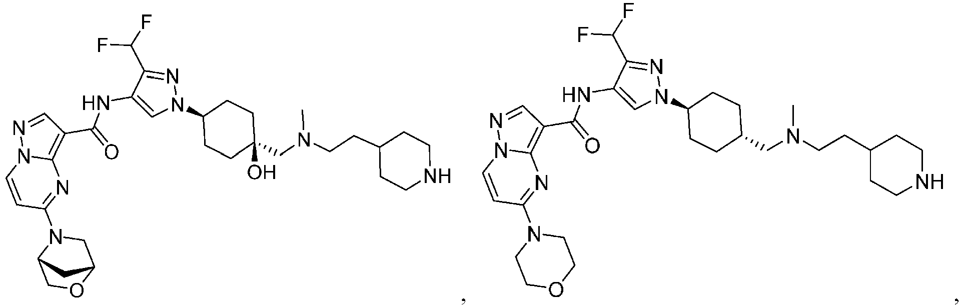 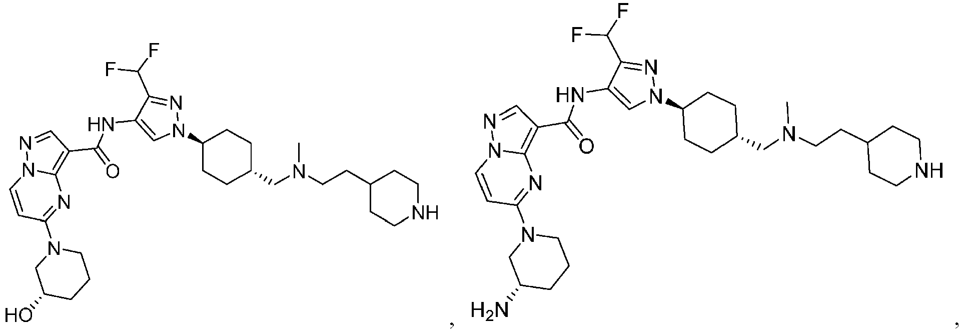 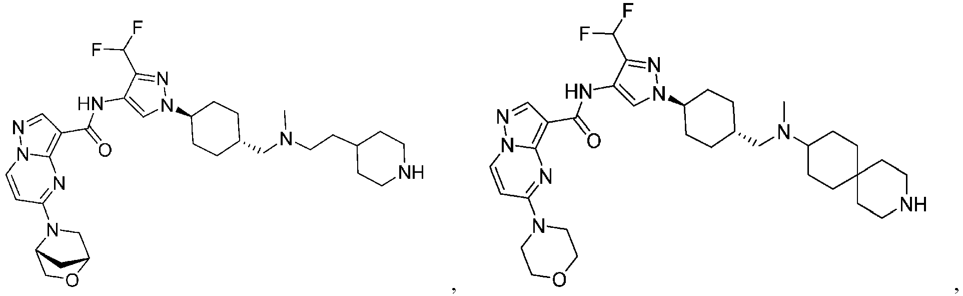 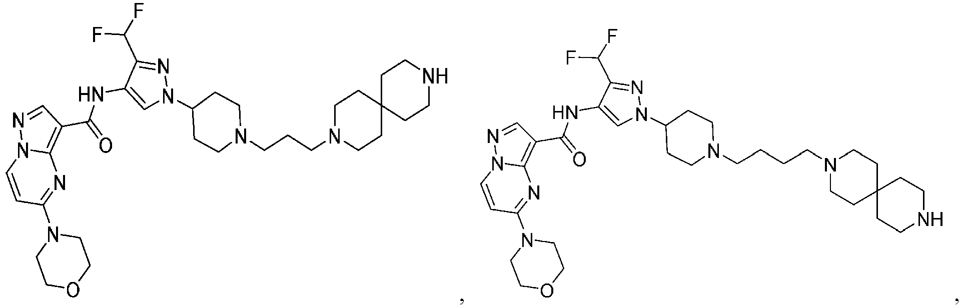 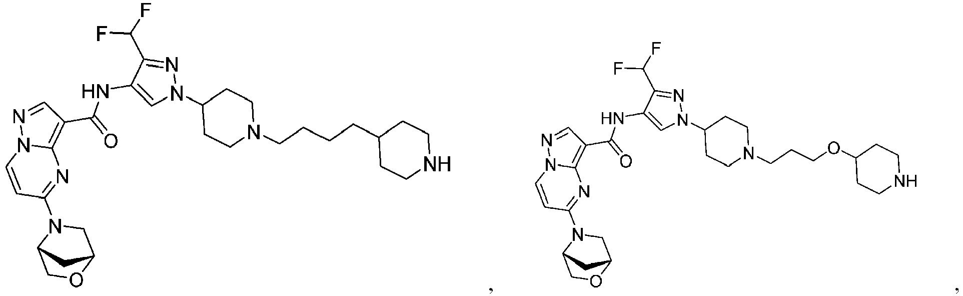 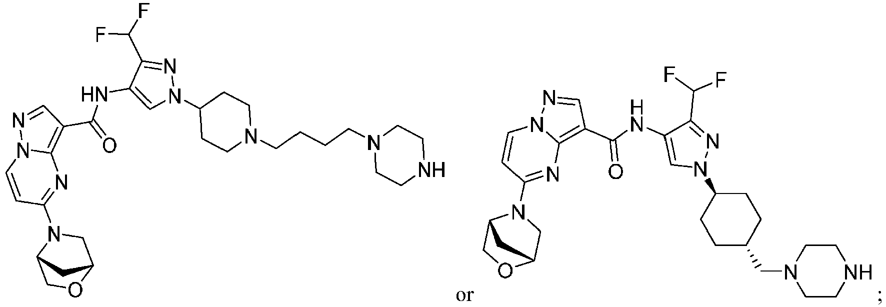
and/or, the compound of formula VIII is any of the following compounds:
